(19) Europäisches Patentamt
European Patent Office
Office européen des brevets

(11) EP 4 397 661 A1

(12) EUROPEAN PATENT APPLICATION
published in accordance with Art. 153(4) EPC

(43) Date of publication:
**10.07.2024 Bulletin 2024/28**

(21) Application number: **22863588.4**

(22) Date of filing: **01.09.2022**

(51) International Patent Classification (IPC):
***C07D 487/04*** *(2006.01)* ***C07K 5/083*** *(2006.01)*
***A61K 31/5377*** *(2006.01)* ***A61P 35/00*** *(2006.01)*

(52) Cooperative Patent Classification (CPC):
**A61K 31/5377; A61P 35/00; C07D 487/04; C07K 5/0804**

(86) International application number:
**PCT/CN2022/116529**

(87) International publication number:
**WO 2023/030453 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**01.09.2021 CN 202111000638**
**20.10.2021 CN 202111220461**
**06.01.2022 CN 202210003537**
**26.01.2022 CN 202210091103**
**13.05.2022 CN 202210508260**
**11.08.2022 CN 202210950054**

(71) Applicant: **Xizang Haisco Pharmaceutical Co., Ltd.**
**Lhoka, Tibet 856099 (CN)**

(72) Inventors:
- **ZHANG, Chen**
**Chengdu, Sichuan 611130 (CN)**
- **LIAO, Yuting**
**Chengdu, Sichuan 611130 (CN)**
- **LU, Yonghua**
**Chengdu, Sichuan 611130 (CN)**
- **ZHAO, Junbin**
**Chengdu, Sichuan 611130 (CN)**
- **ZOU, Sijia**
**Chengdu, Sichuan 611130 (CN)**
- **YU, Yan**
**Chengdu, Sichuan 611130 (CN)**
- **TANG, Pingming**
**Chengdu, Sichuan 611130 (CN)**
- **GAO, Qiu**
**Chengdu, Sichuan 611130 (CN)**
- **CHENG, Xinfan**
**Chengdu, Sichuan 611130 (CN)**
- **YE, Fei**
**Chengdu, Sichuan 611130 (CN)**
- **LI, Yao**
**Chengdu, Sichuan 611130 (CN)**
- **NI, Jia**
**Chengdu, Sichuan 611130 (CN)**
- **YAN, Pangke**
**Chengdu, Sichuan 611130 (CN)**

(74) Representative: **IP TRUST SERVICES**
**Europole - Le Grenat**
**3, avenue Doyen Louis Weil**
**38000 Grenoble (FR)**

# (54) COMPOUND FOR DEGRADATION OF BCL-2 FAMILY PROTEINS AND MEDICAL APPLICATION THEREOF

(57) A compound of general formula (I) or a stereoisomer, a deuterated compound, a solvate, a prodrug, a metabolite, a pharmaceutically acceptable salt, or a co-crystal thereof, an intermediate thereof, and a use thereof in Bcl-2 family proteinsrelated diseases such as cancer. B-L-K (I)



Processed by Luminess, 75001 PARIS (FR)

## Description

### Technical Field

The present invention relates to a compound of general formula (I) or a stereoisomer, deuterated compound, solvate, prodrug, metabolite, pharmaceutically acceptable salt, or co-crystal thereof, and an intermediate thereof and a preparation method therefor, as well as the use thereof in Bcl-2 family proteins-related diseases such as cancer.

### Background Art

Apoptosis is an autonomous and orderly death process controlled by genes for most cells at certain developmental stages in the organism. It plays an important role in tissue evolution, organ development and the maintenance of the body's stability. Anti-apoptotic effects are considered a key feature in malignant tumours. Therefore, specifically targeting the anti-apoptotic pathways shows potential for cancer treatment applications. The Bcl-2 protein family consists of both pro-apoptotic and anti-apoptotic proteins, which can regulate the intrinsic apoptotic pathways of cancer cells. Bcl-2 protein family members Bcl-2, Bcl-xL and Mcl-1 have been identified as anti-tumour targets. Inhibition of these proteins can promote BaxBak oligomerization and ultimately induce mitochondrial outer membrane permeabilization, leading to release of cytochrome C and activation of caspases, which subsequently execute cancer cell apoptosis.

PROTAC (proteolysis targeting chimera) molecules are a class of bifunctional compounds that can simultaneously bind targeting proteins and E3 ubiquitin ligases. Such compounds can be recognized by proteasomes of cells, causing the degradation of targeting proteins, and can effectively reduce the content of targeting proteins in the cells. By introducing a ligand capable of binding to various targeting proteins into the PROTAC molecules, it is possible to apply the PROTAC technology to the treatment of various diseases, and this technology has attracted extensive attention in recent years.

Therefore, it is necessary to develop a novel Bcl-xL/Bcl-2 inhibitor and a PROTAC drug of E3 ubiquitin ligase for the treatment of apoptosis-related tumour diseases.

### Summary of the Invention

An objective of the present invention is to provide a compound with a novel structure, good efficacy, high bioavailability and higher safety that can inhibit and degrade Bcl-2 family proteins (e.g., Bcl-xL or Bcl-2), for use in the treatment of a disease related to Bcl-2 family proteins (e.g., Bcl-xL or Bcl-2), such as cancer.

The present invention provides a compound or a stereoisomer, deuterated compound, solvate, prodrug, metabolite, pharmaceutically acceptable salt, or co-crystal thereof, wherein the compound is selected from a compound of general formula (I),

B-L-K (I);

in certain embodiments, L is selected from a bond or -$C_{1-50}$ hydrocarbyl-, wherein the hydrocarbyl has 0 to 20 methylene units optionally further replaced by -Ak- or -Cy-;

in certain embodiments, L is selected from a bond or -$C_{1-20}$ hydrocarbyl-, wherein the hydrocarbyl has 0 to 20 methylene units optionally further replaced by -Ak- or -Cy-;

in certain embodiments, L is selected from a bond or -$C_{1-10}$ hydrocarbyl-, wherein the hydrocarbyl has 0 to 10 (such as 0, 1, 2, 3, 4, 5, 6, 7, 8, 9 or 10) methylene units optionally further replaced by -Ak- or -Cy-;

in certain embodiments, L is selected from $C_{1-20}$ alkylene, wherein the alkylene is optionally further substituted with 0 to 4 substituents selected from H, halogen, OH, =O, CN, $NH_2$, $C_{1-6}$ alkyl, $C_{1-6}$ alkoxy, halogen-substituted $C_{1-6}$ alkyl, hydroxyl-substituted $C_{1-6}$ alkyl, or cyano-substituted $C_{1-6}$ alkyl, the alkylene has 0 to 5 (such as 0, 1, 2, 3, 4 or 5) methylene units optionally further replaced by O, S, NH, or $N(CH_3)$, and the alkylene chain does not contain a chemical bond selected from N-O, O-O orN-S;

in certain embodiments, L is selected from $C_{1-12}$ alkylene, wherein the alkylene is optionally further substituted with 0 to 4 substituents selected from H, halogen, OH, =O, CN, $NH_2$, $C_{1-6}$ alkyl, $C_{1-6}$ alkoxy, halogen-substituted $C_{1-6}$ alkyl, hydroxyl-substituted $C_{1-6}$ alkyl, or cyano-substituted $C_{1-6}$ alkyl, the alkylene has 0 to 5 (such as 0, 1, 2, 3, 4 or 5) methylene units optionally further replaced by O, S, NH, or $N(CH_3)$, and the alkylene chain does not contain a chemical bond selected from N-O, O-O orN-S;

in certain embodiments, L is selected from $C_{1-12}$ alkylene, wherein the alkylene is optionally further substituted with 0 to 4 substituents selected from H, F, OH, =O, CN, $NH_2$, methyl, or methoxy, the alkylene has 0 to 5 (such as 0, 1, 2, 3, 4 or 5) methylene units optionally further replaced by O, S, NH, or $N(CH_3)$, and the alkylene chain does not

contain a chemical bond selected from N-O, O-O or N-S and optionally contains 0 to 4 (such as 0, 1, 2, 3 or 4) structural units selected from $-OCH_2CH_2-$, $-SCH_2CH_2-$, $-NHCH_2CH_2-$, or $-N(CH_3)CH_2CH_2-$;

in certain embodiments, L is selected from $-C(=O)C_{1-7}$ alkylene;

in certain embodiments, each -Ak- is independently selected from Ak1, Ak2, Ak3, Ak4, Ak5, Ak6, Ak7, Ak8, or Ak9;

in certain embodiments, each -Ak- is independently selected from $-(CH_2)_q-$, - $(CH_2)_q$-O-, -O-$(CH_2)_q$-, $-(CH_2)_q-NR^L-$, $-NR^L-(CH_2)_q-$, $-(CH_2)_q-NR^LC(=O)-$, - $NR^L(CH_2)_qC(=O)-$, $-(CH_2)_q-C(=O)NR^L-$, -C(=O)-, $-C(=O)-(CH_2)_q-NR^L-$, $-(C{\equiv}C)_q-$, -CH=CH-, $-Si(R^L)_2-$, $-Si(OH)(R^L)-$, $-Si(OH)_2-$, $-P(=O)(OR^L)-$, $-P(=O)(R^L)-$, -S-, - S(=O)-, $-S(=O)_2-$ or a bond, wherein the $-CH_2-$ is optionally further substituted with 0 to 2 (such as 0, 1 or 2) substituents selected from H, halogen, OH, CN, $NH_2$, $C_{1-6}$ alkyl, $C_{1-6}$ alkoxy, halogen-substituted $C_{1-6}$ alkyl, hydroxyl-substituted $C_{1-6}$ alkyl or cyano-substituted $C_{1-6}$ alkyl;

in certain embodiments, each -Cy- is independently selected from Cy1, Cy2, Cy3, Cy4 or Cy5;

in certain embodiments, each -Cy- is independently selected from a bond, a 4-to 8-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 (such as 0, 1, 2, 3 or 4) substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 (such as 1, 2, 3 or 4) heteroatoms selected from O, S or N, and is optionally further substituted with 0, 1 or 2 =O when the heteroatom is selected from S;

in certain embodiments, each Cy1, Cy2, Cy3, Cy4 or Cy5 is independently selected from a bond, a 4- to 7-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 (such as 0, 1, 2, 3 or 4) substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 (such as 1, 2, 3 or 4) heteroatoms selected from O, S or N, and is optionally further substituted with 0, 1 or 2 =O when the heteroatom is selected from S;

in certain embodiments, L is selected from -Cy1-Ak1-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3 -Cy4-Ak3 -Ak4-Ak5 -, -Cy1-Ak1-Cy2-Ak2-Ak3-Cy3- Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, - Cy1-Cy2-Cy3-Cy4-Ak1-Ak2-Ak3-Ak4-Ak5-, -Cy1-Ak1-Cy2-Cy3-Cy4-Ak2-Ak3- Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, -Cy1-Ak1-Ak2-Cy2-Cy3-Cy4-Ak3-Ak4-Ak5-, -Cy1-Cyl-Ak 1 -Ak2-Cy3 -Cy4-Ak3 -Ak4-Ak5 -, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, -Ak1-Ak2-Ak3-Ak4-Ak5-Cy1- Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, - Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, - Ak 1 -Cy 1 -Cy2-Cy 3 - Ak2-Ak3-Ak4-Ak5-Cy4-, -Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, -Ak1-Ak2-Cy1-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, -Ak1-Ak2-Ak3-Ak4-Cy1-Cy2-Cy3-Ak5-Cy4-, -Ak1-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Ak1-Ak2-Ak3-Ak4-Ak5-Ak6-Ak7-, -Ak1-Ak2-Ak3-Ak4-Ak5-Ak6-Ak7-Ak8-, or -Ak1-Ak2-Ak3-Ak4-Ak5-Ak6-Ak7-Ak8-Ak9;

in certain embodiments, L is selected from a bond, -Ak1-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Cy1-, -Cy1-Ak1-, -Cy1-Ak1-Ak2-, -Cy1-Ak1-Ak2-Ak3-, -Cy1-Ak1-Ak2-Ak3-Ak4-, -Cy1-Cy2-, -Cy1-Ak1-Cy2-, -Cy1-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Ak2-Cy3-, -Cy1-Ak1-Ak2-Cy3-Ak3-, -Cy1-Cy2-Cy3-, -Cy 1- Ak 1-Cy2-Cy3 -, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, - Cy1-Ak1-Cy2-Ak2-Cy3-Ak3-, -Cy 1 -Cy2-Cy 3 - Ak3 - Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cy1-Ak1-Cy2-Ak2-Cy3-Cy4-, -Ak1-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Cy1-Cy2-Cy3-Ak3-Ak4-Ak5-, - Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-Ak2-Ak3-Ak4-, -Ak1-Cy2-Ak2-Ak3-, -Ak1-Ak2-Ak3-Ak4-Ak5 -, -Ak1-Ak2-Ak3-Ak4-Ak5-Ak6-, or -Ak1-Ak2-Ak3-Ak4-Ak5-Ak6-Ak7-;

in certain embodiments, L is selected from a bond or a group shown in Table B-1, wherein the left side of the group is linked to B;

in certain embodiments, L is selected from a bond, -C(=O)$C_{1-8}$ alkylene-, or - $CH_2$-4- to 10-membered nitrogen-containing heterocycle, wherein the nitrogen-containing heterocycle is optionally substituted with 1 to 4 substituents selected from F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl or $C_{1-4}$ alkoxy, and the nitrogen-containing heterocycle contains 1 to 4 (such as 1, 2, 3 or 4) heteroatoms selected from O, S or N;

in certain embodiments, L is selected from a bond, -C(=O)$C_{2-5}$ alkylene-, or - $CH_2$-4- to 10-membered nitrogen-containing heterocycle, wherein the nitrogen-containing heterocycle is optionally substituted with 1 to 4 substituents selected from F, Cl, Br, I, OH, $NH_2$, COOH, CN, =O, methyl, or $CF_3$, and the nitrogen-containing heterocycle contains 1 to 4 (such as 1, 2, 3 or 4) heteroatoms selected from O, S or N;

in certain embodiments, Ak1, Ak2, Ak3, Ak4, Ak5, Ak6, Ak7, Ak8 and Ak9 are each independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)_q-NR^LC(=O)-$, $-(CH_2)_q-C(=O)NR^L-$, -C(=O)-, $-C(=O)-(CH_2)_q-NR^L-$, $-(C\equiv C)_q-$ or a bond, wherein the $-CH_2-$ is optionally further substituted with 0 to 2 (such as 0, 1 or 2) substituents selected from H, halogen, OH, CN, $NH_2$, $C_{1-4}$ alkyl, $C_{1-4}$ alkoxy, halogen-substituted $C_{1-4}$ alkyl, hydroxyl-substituted $C_{1-4}$ alkyl or cyano-substituted $C_{1-4}$ alkyl;

in certain embodiments, Ak1, Ak2, Ak3, Ak4, Ak5, Ak6, Ak7, Ak8 and Ak9 are each independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)_q-NR^LC(=O)-$, $-(CH_2)_q-C(=O)NR^L-$, -C(=O)-, $-C(=O)-(CH_2)_q-NR^L-$, $-(C\equiv C)_q-$, or a bond, wherein the $-CH_2-$ is optionally further substituted with 0 to 2 substituents selected from H, F, Cl, Br, I, OH, CN, $NH_2$, $CF_3$, hydroxymethyl, $C_{1-4}$ alkyl, or $C_{1-4}$ alkoxy;

in certain embodiments, Ak1, Ak2, Ak3, Ak4, Ak5, Ak6, Ak7, Ak8 and Ak9 are each independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)_q-NR^LC(=O)-$, $-(CH_2)_q-C(=O)NR^L-$, -C(=O)-, $-C(=O)-(CH_2)_q-NR^L-$, $-(C\equiv C)_q-$, or a bond, wherein the $-CH_2-$ is optionally further substituted with 0 to 2 substituents selected from H, F, Cl, Br, I, OH, CN, $NH_2$, $CF_3$, hydroxymethyl, methyl, ethyl, methoxy or ethoxy;

in certain embodiments, Ak1, Ak2, Ak3, Ak4, Ak5, Ak6, Ak7, Ak8 and Ak9 are each independently selected from a bond, -O-, $-OCH_2-$, $-CH_2O-$, $-OCH_2CH_2-$, $- CH_2CH_2O-$, $-C\equiv C-$, $-C(CH_3)_2-$, $-CH_2-$, $-CH_2CH_2-$, $-CH_2CH_2CH_2-$, $-N(CH_3)-$, -NH-, $-CH_2N(CH_3)-$, $-CH_2NH-$, $-NHCH_2-$, $-CH_2CH_2N(CH_3)-$, $-CH_2CH_2NH-$, $- NHCH_2CH_2-$, -C(=O)-, $-C(=O)CH_2NH-$, $-CH_2C(=O)NH-$, -C(=O)NH- or - NHC(=O)-;

in certain embodiments, each $R^L$ is independently selected from H, $C_{1-6}$ alkyl, 3- to 7-membered heterocyclyl, 3- to 7-membered cycloalkyl, phenyl or 5- to 6-membered heteroaryl;

in certain embodiments, each $R^L$ is independently selected from H or $C_{1-6}$ alkyl;

in certain embodiments, each $R^L$ is independently selected from H or $C_{1-4}$ alkyl;

in certain embodiments, each $R^L$ is independently selected from H, methyl or ethyl;

in certain embodiments, each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, azacyclopentyl, azacyclohexenyl, piperidyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclopropyl-fused-piperidyl, cyclobutyl-fused-azetidinyl, cyclobutyl-fused-azacyclopentyl, cyclobutyl-fused-azacyclohexyl, cyclobutyl-fused-piperidyl, cyclopentyl-fused-azetidinyl, cyclopentyl-fused-azacyclopentyl, cyclopentyl-fused-azacyclohexyl, cyclopentyl-fused-piperidyl, cyclohexyl-fused-azetidinyl, cyclohexyl-fused-azacyclopentyl, cyclohexyl-fused-azacyclohexyl, cyclohexyl-fused-piperidyl, azetidinyl-fused-azetidinyl, azetidinyl-fused-azacyclopentyl, azetidinyl-fused-azacyclohexyl, azetidinyl-fused-piperidyl, azacyclopentyl-fused-azetidinyl, azacyclopentyl-fused-azacyclopentyl, azacyclopentyl-fused-azacyclohexyl, azacyclopentyl-fused-piperidyl, azacyclohexyl-fused-azetidinyl, azacyclohexyl-fused-azacyclopentyl, azacyclohexyl-fused-azacyclohexyl, azacyclohexyl-fused-piperidyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidyl, cyclopentyl-spiro-piperidyl, cyclohexyl-spiro-piperidyl, azetidinyl-spiro-piperidyl, azacyclopentyl-spiro-piperidyl, azacyclohexyl-spiro-piperidyl,

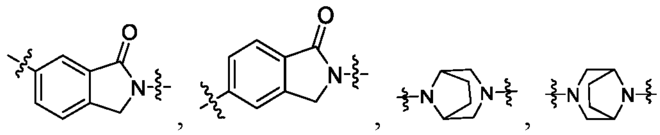
, , , ,

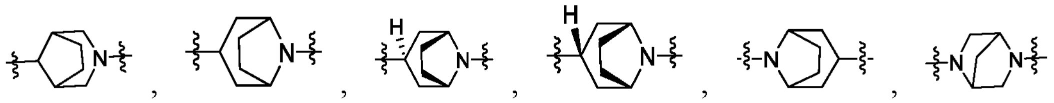
, , , , , ,

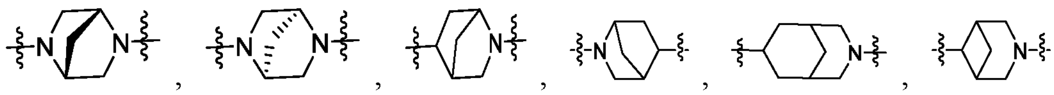
, , , , , ,

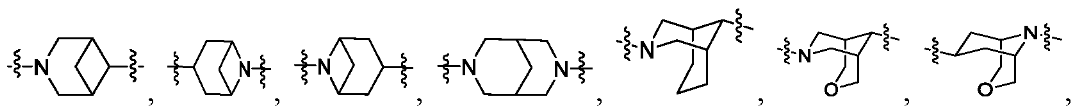
, , , , , , ,

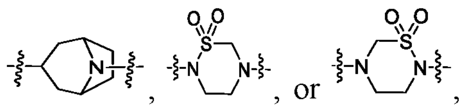
, , or ,

which, when substituted, is optionally further substituted with 0 to 4 (such as 0, 1, 2, 3 or 4) substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl, or $C_{1-4}$ alkoxy;

in certain embodiments, each Cy1, Cy2, Cy3, Cy4 or Cy5 is independently selected from a bond or one of the following substituted or unsubstituted groups:

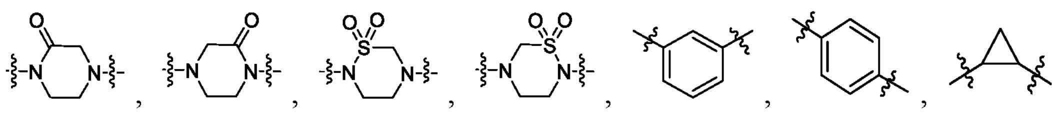
, , , , , , ,

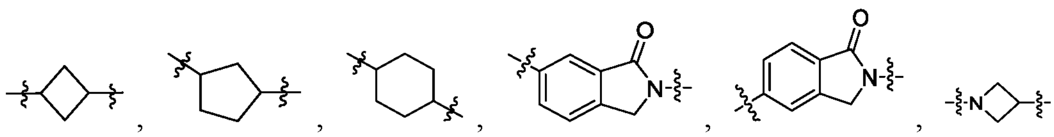
, , , , , ,

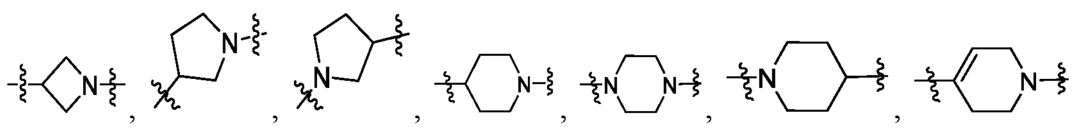
, , , , , , ,

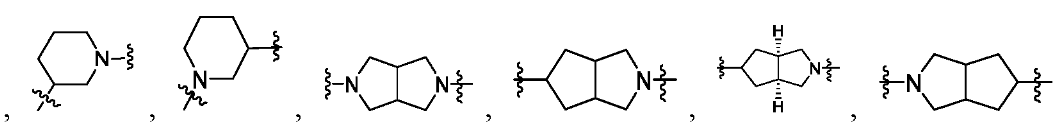
, , , , , , ,

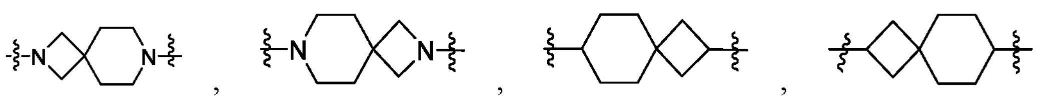
, , , ,

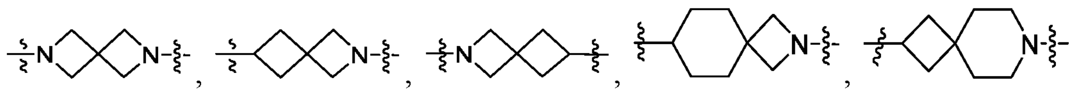
, , , ,

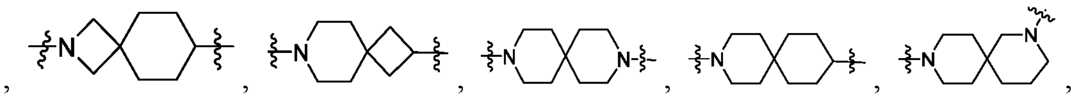
, , , , ,

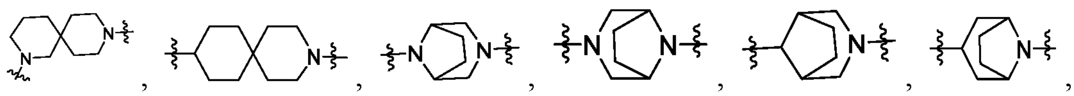
, , , , , ,

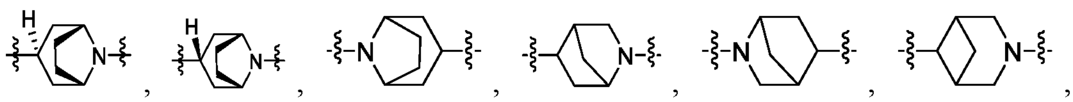
, , , , , ,

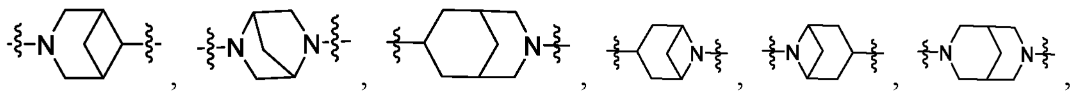
, , , , , ,

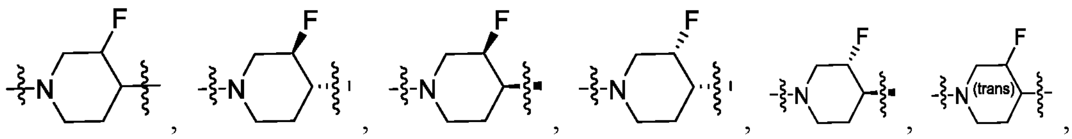
, , , , , ,

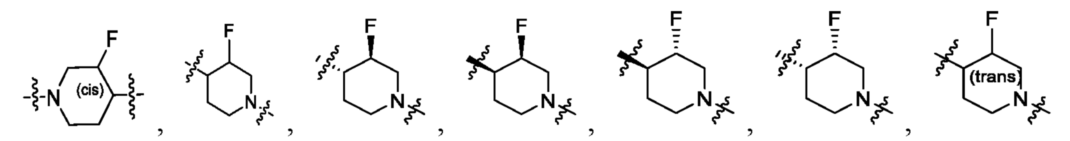
, , , , , ,

or

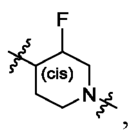
,

which, when substituted, is optionally further substituted with 0 to 4 (such as 0, 1, 2, 3 or 4) substituents selected from H, F, $CF_3$, methyl, =O, hydroxymethyl, COOH, CN or $NH_2$;
in certain embodiments, K is selected from

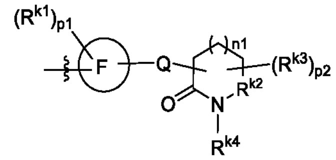
,

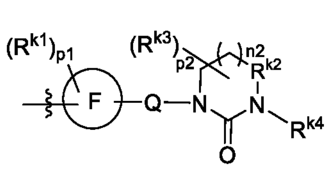, 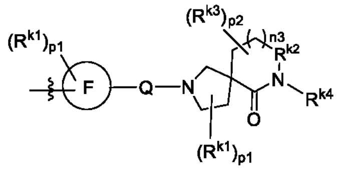, 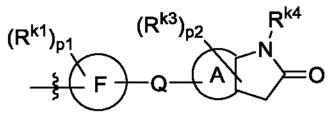,

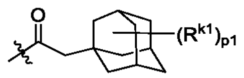, 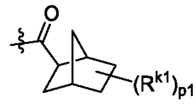, 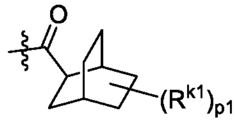, 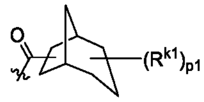,

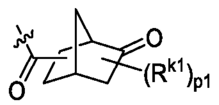, 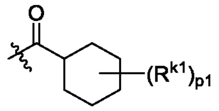, 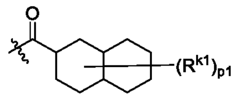, 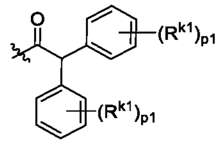,

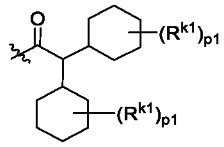, 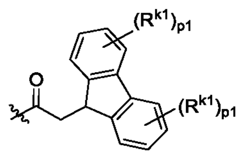, 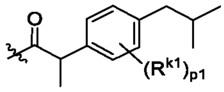, 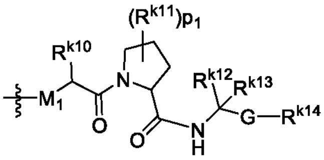,

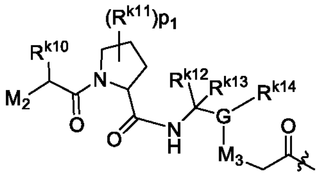 or 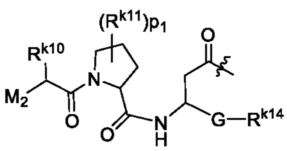;

in certain embodiments, K is selected from

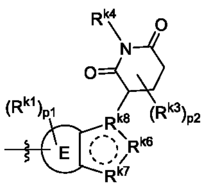,

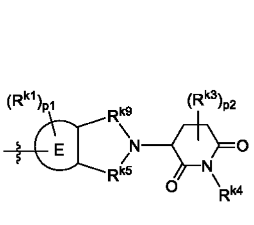, 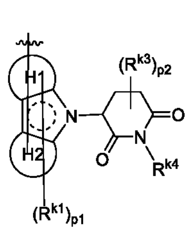, 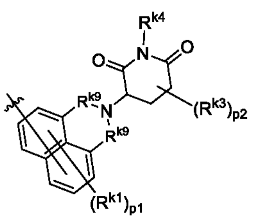, 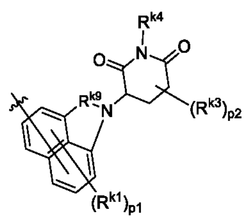,

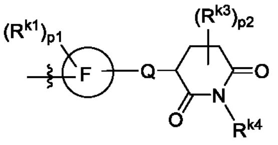, 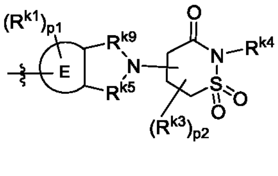, 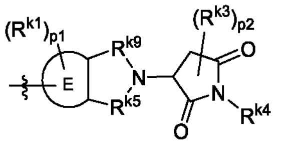,

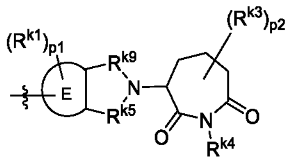 , 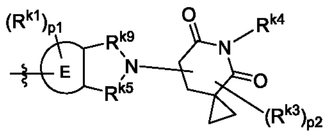 , 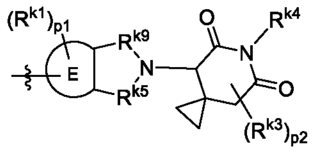 ,

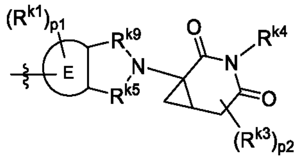 , 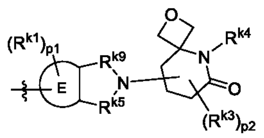 , 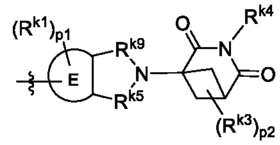 ,

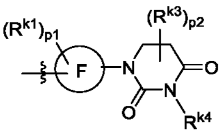 , 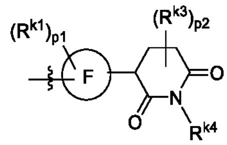 , 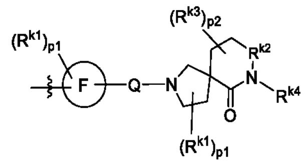 ,

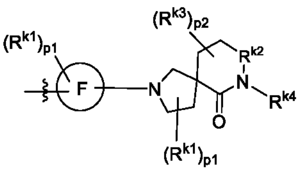 , 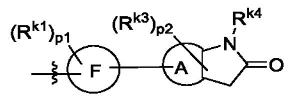 , 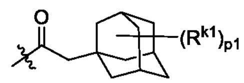 ,

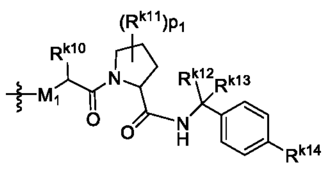 , 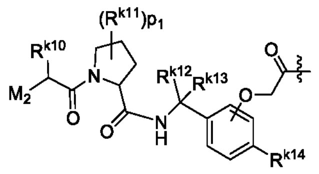 or 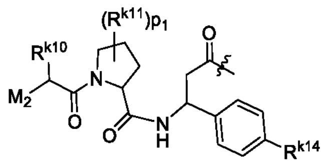 ,

wherein

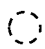

represents a ring selected from an aromatic ring or a non-aromatic ring;
in certain embodiments, K is selected from

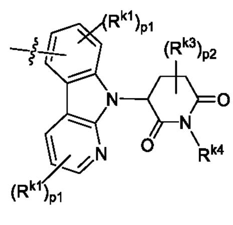 ,

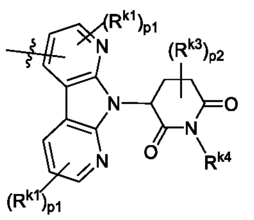,
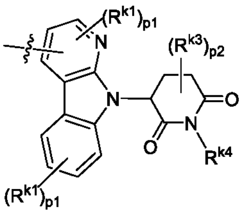,
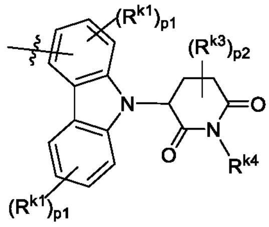,
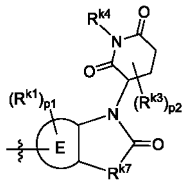,
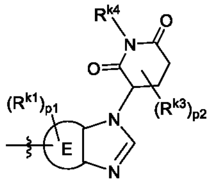,
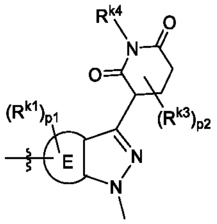,
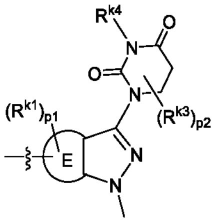,
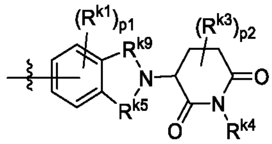,
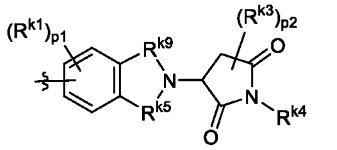,
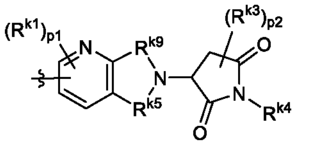,
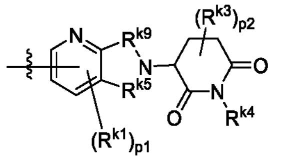,
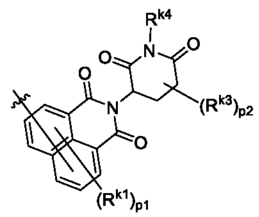,
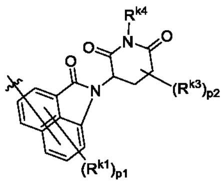,
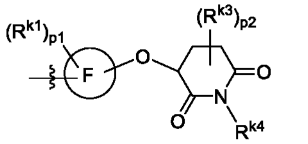,
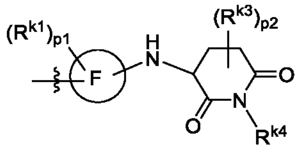,
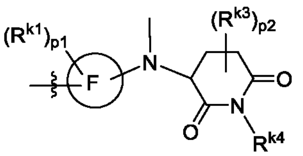,
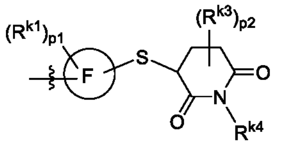,
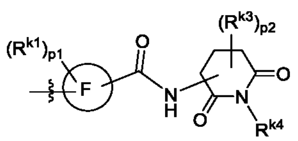,
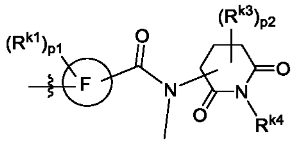,
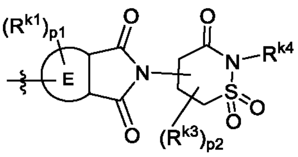,
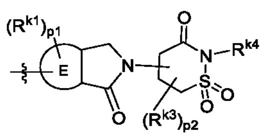,
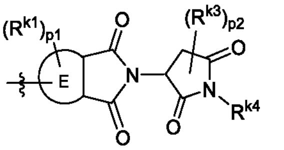,
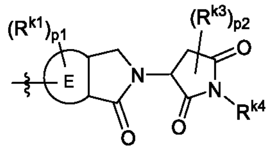,

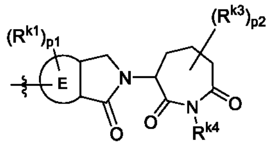, 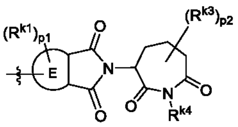, 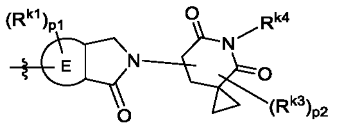,

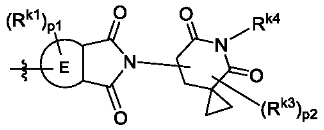, 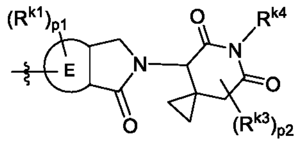, 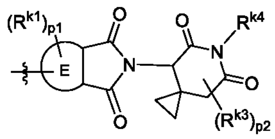,

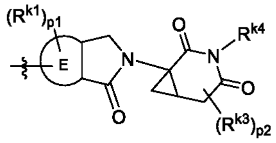, 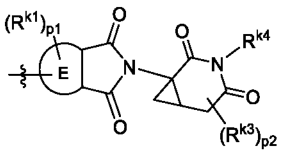, 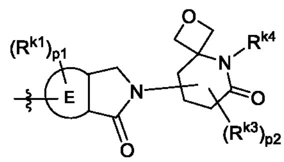,

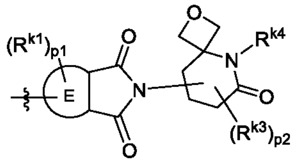, 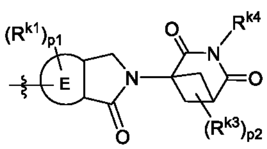, 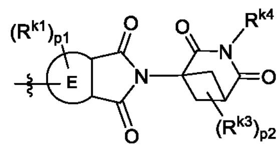,

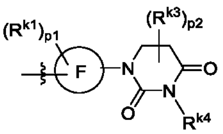, 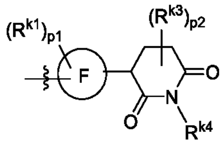, 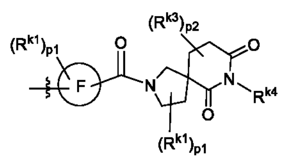,

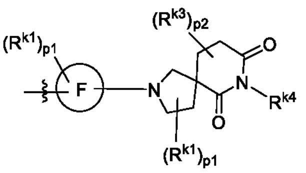, 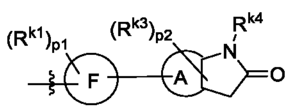, 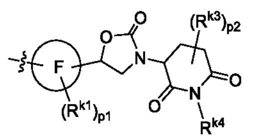,

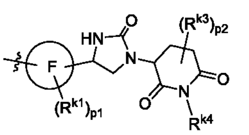, 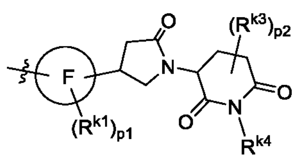, 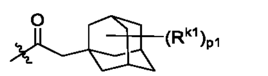,

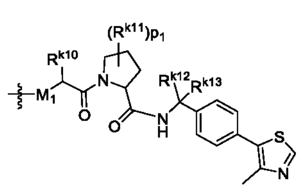, 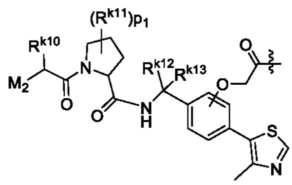, 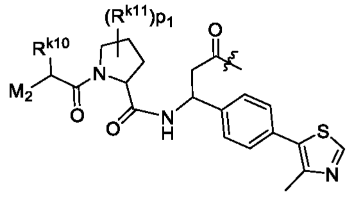,

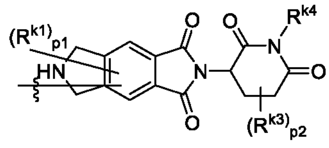 , 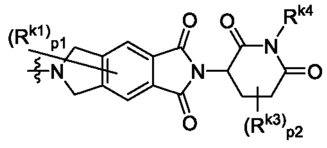 , 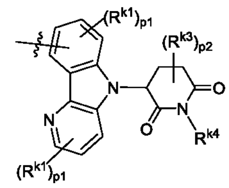

or

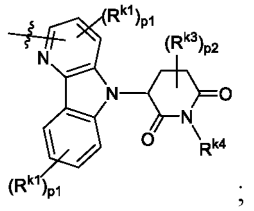 ;

in certain embodiments, each Q is independently selected from a bond, -O-, - S-, $-CH_2-$, $-NR^q-$, -CO-, $-NR^qCO-$, $-CONR^q-$ or 3- to 12-membered heterocyclyl, wherein the heterocyclyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;

in certain embodiments, each Q is independently selected from -O-, -S-, - $CH_2$-, $-NR^q-$, -CO-, $-NR^qCO-$, $-CONR^q-$ or 4- to 7-membered heterocyclyl, wherein the heterocyclyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;

in certain embodiments, $R^q$ is selected from H or $C_{1-6}$ alkyl;

in certain embodiments, $R^q$ is selected from H or $C_{1-4}$ alkyl;

in certain embodiments, $R^q$ is selected from H, methyl or ethyl;

in certain embodiments, each E is independently selected from $C_{3-10}$ carbocyclyl, $C_{6-10}$ aryl, 3- to 12-membered heterocyclyl or 5- to 12-membered heteroaryl, wherein the heterocycle or heteroaryl contains 1 to 4 (such as 1, 2, 3 or 4) heteroatoms selected from O, S or N;

in certain embodiments, each E is independently selected from $C_{3-8}$ carbocycle, a benzene ring, 4- to 7-membered heterocycle, 8- to 12-membered heterocycle, 7- to 12-membered heteroaryl or 5- to 6-membered heteroaryl, wherein the heterocycle or heteroaryl contains 1 to 4 (such as 1, 2, 3 or 4) heteroatoms selected from O, S or N;

in certain embodiments, each E is independently selected from phenyl, pyridyl, pyridazinyl, pyrazinyl, pyrimidyl, pyrrolyl, pyrazolyl, imidazolyl, thiazolyl, furyl, thienyl, oxazolyl, indolinyl, isoindolinyl, 1,2,3,4-tetrahydroquinolyl or 1,2,3,4-tetrahydroisoquinolinyl;

in certain embodiments, each E is independently selected from phenyl, pyridyl, pyridazinyl, pyrazinyl, pyrimidyl, pyrrolyl, pyrazolyl, imidazolyl, thiazolyl, furyl, thienyl or oxazolyl;

in certain embodiments, each E is independently selected from phenyl, pyridyl, pyridazinyl, pyrazinyl or pyrimidyl;

in certain embodiments, each E is independently selected from a benzene ring or a pyridine ring;

in certain embodiments, A is selected from $C_{3-10}$ carbocyclyl, $C_{6-10}$ aryl, 3- to 10-membered heterocyclyl or 5- to 10-membered heteroaryl, wherein the heterocycle or heteroaryl contains 1 to 4 (such as 1, 2, 3 or 4) heteroatoms selected from O, S or N;

in certain embodiments, each A, H1 or H2 is independently selected from $C_{3-8}$ carbocycle, a benzene ring, 4- to 7-membered heterocycle or a 5- to 6-membered heteroaryl, wherein the heterocycle or heteroaryl contains 1 to 4 (such as 1, 2, 3 or 4) heteroatoms selected from O, S or N;

in certain embodiments, each A, H1 or H2 is independently selected from phenyl, pyridyl, pyridazinyl, pyrazinyl, pyrimidyl, pyrrolyl, pyrazolyl, imidazolyl, thiazolyl, furyl, thienyl or oxazolyl;

in certain embodiments, each A, H1 or H2 is independently selected from phenyl or pyridyl;

in certain embodiments, each F (ring F) is independently selected from $C_{3-20}$ carbocyclyl, $C_{6-20}$ aryl, 3- to 20-membered heterocyclyl or 5- to 20-membered heteroaryl, wherein the heterocyclyl or heteroaryl contains 1 to 4 (such as 1, 2, 3 or 4) heteroatoms selected from O, S or N;

in certain embodiments, each F is independently selected from 3- to 7-membered monocycloalkyl, 4- to 10-membered fused cycloalkyl, 5- to 12-membered spiro cycloalkyl, 5- to 10-membered bridged cycloalkyl, 4- to 7-membered mono-heterocyclic ring, 4- to 10-membered fused-heterocyclic ring, 5-to 12-membered spiro-heterocyclic ring, 5- to 10-membered bridged-heterocyclic ring, $C_{6-14}$ aryl or 5- to 10-membered heteroaryl, wherein the mono-heterocyclic ring, fused-heterocyclic ring, spiro-heterocyclic ring, bridged-heterocyclic ring or heteroaryl contains 1 to 4 (such as 1, 2, 3 or 4) heteroatoms selected from O, S or N;

in certain embodiments, each F is independently selected from cyclopropyl, cyclobutyl, cyclopentyl, cyclohexyl, bicyclo[1.1.1]pentanyl, 6,7-dihydro-5H-cyclopenta[c]pyridyl, 2,3-dihydro-1H-indenyl, phenyl, naphthyl, anthryl, phenanthryl, azetidinyl, azacyclopentyl, piperidyl, morpholinyl, pyridyl, pyrimidyl, pyridazinyl, pyrazinyl, triazinyl, pyrrolyl, pyrazolyl, imidazolyl, triazolyl, oxazolyl, furyl, thienyl, thiazolyl, benzoimidazolyl, benzopyrazolyl, benzothiazolyl, benzothienyl, benzofuryl, benzopyrrolyl, benzopyridyl, benzopyrazinyl, benzopyrimidyl, benzopyridazinyl, pyrrolopyrrolyl, pyrrolopyridyl, pyrrolopyrimidyl, pyrrolopyridazinyl, pyrrolopyrazinyl, imidazopyrimidyl, imidazopyridyl, imidazopyrazinyl, imidazopyridazinyl, pyrazolopyridyl, pyrazolopyrimidyl, pyrazolopyridazinyl, pyrazolopyrazinyl, pyrimidopyridyl, pyrimidopyrazinyl, pyrimidopyridazinyl, pyrimidopyrimidyl, pyridopyridyl, pyridopyrazinyl, pyridopyridazinyl, pyridazinopyridazinyl, pyridazinopyrazinyl or pyrazinopyrazinyl;

in certain embodiments, each $R^{k2}$ is independently selected from a bond, -CO-, $-SO_2-$, -SO- or $-C(R^{k3})_2-$;

in certain embodiments, each $R^{k2}$ is independently selected from -CO-, $-SO_2-$ or $-C(R^{k3})_2-$;

in certain embodiments, each $R^{k1}$ is independently selected from H, F, Cl, Br, I, OH, =O, $NH_2$, CN, COOH, $CONH_2$, $C_{1-6}$ alkyl or $C_{1-6}$ alkoxy, wherein the alkyl or alkoxy is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy;

in certain embodiments, each $R^{k3}$ is independently selected from H, F, Cl, Br, I, OH, =O, $NH_2$, CN, COOH, $CONH_2$, $C_{1-6}$ alkyl, $C_{1-6}$ alkoxy, $C_{3-8}$ cycloalkyl or 3-to 8-membered heterocyclyl, wherein the alkyl, alkoxy, cycloalkyl or heterocyclyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;

in certain embodiments, $R^{k1}$ and $R^{k3}$ are each independently selected from H, F, Cl, Br, I, OH, =O, $NH_2$, $CF_3$, CN, COOH, $CONH_2$, $C_{1-4}$ alkyl or $C_{1-4}$ alkoxy, wherein the alkyl or alkoxy is optionally further substituted with 0 to 4 (such as 0, 1, 2, 3 or 4) substituents selected from H, F, Cl, Br, I, OH or $NH_2$;

in certain embodiments, $R^{k1}$ and $R^{k3}$ are each independently selected from H, F, Cl, Br, I, OH, =O, $NH_2$, $CF_3$, CN, COOH, $CONH_2$, methyl, ethyl, isopropyl, methoxy, ethoxy or isopropoxy, wherein the methyl, ethyl, isopropyl, methoxy, ethoxy or isopropoxy is optionally further substituted with 0 to 4 (such as 0, 1, 2, 3 or 4) substituents selected from H, F, Cl, Br, I, OH or $NH_2$;

in certain embodiments, two $R^{k3}$ together with the carbon atoms or ring backbones to which they are directly attached form 3- to 8-membered carbocycle or 3- to 8-membered heterocycle, wherein the carbocycle or heterocycle is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 (such as 1, 2, 3 or 4) heteroatoms selected from O, S or N;

in certain embodiments, two $R^{k3}$ together with the carbon atoms or ring backbones to which they are directly attached form 3- to 6-membered carbocycle or 3- to 7-membered heterocycle, wherein the carbocycle or heterocycle is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 (such as 1, 2, 3 or 4) heteroatoms selected from O, S or N;

in certain embodiments, each $R^{k4}$ is independently selected from H, OH, $NH_2$, CN, $CONH_2$, $C_{1-6}$ alkyl, $C_{3-8}$ cycloalkyl or 3- to 8-membered heterocyclyl, wherein the alkyl, cycloalkyl or heterocyclyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;

in certain embodiments, each $R^{k4}$ is independently selected from H, OH, $NH_2$, $CF_3$, CN or $C_{1-4}$ alkyl;

in certain embodiments, each $R^{k5}$ is independently selected from CO, $CH_2$, $SO_2$ or

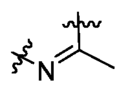;

in certain embodiments, each $R^{k6}$ is independently selected from CO, CH, SO, $SO_2$, $CH_2$ or N;

in certain embodiments, each $R^{k7}$ is independently selected from CO, CH, N, $CH_2$, O, S, $N(CH_3)$ or NH;

in certain embodiments, each $R^{k7}$ is independently selected from $CH_2$, O, $N(CH_3)$ or NH;

in certain embodiments, each $R^{k8}$ is independently selected from C, N or CH;

in certain embodiments, each $R^{k9}$ is independently selected from CO, $SO_2$ or $CH_2$;

in certain embodiments, $M_1$ is selected from a bond, -C(=O)NH-, -NHC(=O)-, $-CH_2-C(=O)NH-$, $-C(=O)CH_2NH-$, or 5- to 6-membered heteroaryl, wherein the heteroaryl is optionally further substituted with 0 to 4 (such as 0, 1, 2, 3 or 4) substituents selected from H, F, Cl, Br, I, OH, =O, $CF_3$, $NH_2$, CN, $C_{1-4}$ alkyl, halogen-substituted $C_{1-4}$ alkyl, hydroxyl-substituted $C_{1-4}$ alkyl, or $C_{1-4}$ alkoxy, and the heteroaryl contains 1 to 4 (such as 1, 2, 3 or 4) heteroatoms

selected from O, S or N;

in certain embodiments, M1 is selected from a bond, -C(=O)NH-, -$CH_2$-C(=O)NH-, -C(=O)$CH_2$NH-, pyrrolyl, pyrazolyl, imidazolyl, triazolyl, oxazolyl, isoxazolyl, furyl, thienyl, or thiazolyl;

in certain embodiments, $M_1$ is selected from a bond, -$CH_2$-C(=O)NH- or - C(=O)$CH_2$NH-;

in certain embodiments, $M_2$ is selected from -NHC(=O)-$C_{1-6}$ alkyl, - NHC(=O)-$C_{3-6}$ cycloalkyl or 4- to 10-membered heterocyclyl, wherein the alkyl, cycloalkyl or heterocyclyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy, and the heterocyclyl contains 1 to 4 heteroatoms selected from O, S or N;

in certain embodiments, $M_2$ is selected from -NHC(=O)-$C_{1-4}$ alkyl, - NHC(=O)-$C_{3-6}$ cycloalkyl or 4- to 10-membered heterocyclyl, wherein the alkyl, cycloalkyl or heterocyclyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy, and the heterocyclyl contains 1 to 4 heteroatoms selected from O, S or N;

in certain embodiments, $M_2$ is selected from -NHC(=O)-$CH_3$, -NHC(=O)-cyclopropyl, -NHC(=O)-cyclobutyl, azetidinyl, azacyclopentyl, benzo-azacyclopentyl or benzo-azacyclohexyl, wherein the cyclopropyl, cyclobutyl, azetidinyl, azacyclopentyl, benzo-azacyclopentyl or benzo-azacyclohexyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy;

in certain embodiments, $M_3$ is selected from -NH- or -O-;

in certain embodiments, $R^{k10}$ is selected from $C_{1-6}$ alkyl, wherein the alkyl is optionally further substituted with 0 to 4 (such as 0, 1, 2, 3 or 4) substituents selected from H, F, Cl, Br, I, =O, OH, $C_{1-6}$ alkyl or $C_{3-6}$ cycloalkyl;

in certain embodiments, $R^{k10}$ is selected from $C_{1-4}$ alkyl, wherein the alkyl is optionally further substituted with 0 to 4 (such as 0, 1, 2, 3 or 4) substituents selected from H, F, Cl, Br, I, =O, OH, $C_{1-4}$ alkyl or $C_{3-6}$ cycloalkyl;

in certain embodiments, $R^{k10}$ is selected from methyl, ethyl, isopropyl, propyl, or tert-butyl, wherein the methyl, ethyl, isopropyl, propyl, or tert-butyl is optionally further substituted with 0 to 4 (such as 0, 1, 2, 3 or 4) substituents selected from H, F, Cl, Br, I, =O, OH, $C_{1-4}$ alkyl or $C_{3-6}$ cycloalkyl;

in certain embodiments, G is selected from 6- to 10-membered aryl or 5- to 10-membered heteroaryl, wherein the aryl or heteroaryl is optionally further substituted with 0 to 4 (for example, 0, 1, 2, 3 or 4) substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 (for example, 1, 2, 3 or 4) heteroatoms selected from N, O or S;

in certain embodiments, each $R^{k11}$ is independently selected from H, F, Cl, Br, I, =O, OH, SH, $C_{1-6}$ alkyl, $C_{1-6}$ alkoxy or $C_{1-6}$ alkylthio or -O-C(=O)-$C_{1-6}$ alkyl, wherein the alkyl, alkoxy or alkylthio is optionally further substituted with 0 to 4 (such as 0, 1, 2, 3 or 4) substituents selected from H, F, Cl, Br, I, OH, $C_{1-4}$ alkyl or $C_{1-4}$ alkoxy;

in certain embodiments, each $R^{k11}$ is independently selected from H, F, Cl, Br, I, =O, OH, SH, $C_{1-4}$ alkyl, $C_{1-4}$ alkoxy, or $C_{1-4}$ alkylthio or -O-C(=O)-$C_{1-4}$ alkyl, wherein the alkyl, alkoxy or alkylthio is optionally further substituted with 0 to 4 (such as 0, 1, 2, 3 or 4) substituents selected from H, F, Cl, Br, I, OH, $C_{1-4}$ alkyl or $C_{1-4}$ alkoxy;

in certain embodiments, each $R^{k11}$ is independently selected from H, F, Cl, Br, I, =O, OH, SH, methyl, ethyl, isopropyl, propyl, methoxy, ethoxy, propoxy, isopropyloxy, methylthio, ethylthio, propylthio or -O-C(=O)-$CH_3$, wherein the methyl, ethyl, isopropyl, propyl, methoxy, ethoxy, propoxy, isopropyloxy, methylthio, ethylthio, or propylthio is optionally further substituted with 0 to 4 (such as 0, 1, 2, 3 or 4) substituents selected from H, F, Cl, Br, I, OH, $C_{1-4}$ alkyl or $C_{1-4}$ alkoxy;

in certain embodiments, $R^{k12}$ and $R^{k13}$ are each independently selected from H, $C_{1-6}$ alkyl or $C_{3-6}$ cycloalkyl, wherein the alkyl or cycloalkyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy;

in certain embodiments, $R^{k12}$ and $R^{k13}$ are each independently selected from H, $C_{1-4}$ alkyl or $C_{3-6}$ cycloalkyl, wherein the alkyl or cycloalkyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy;

in certain embodiments, $R^{k12}$ and $R^{k13}$ are each independently selected from H, methyl, ethyl, isopropyl, propyl, cyclopropyl or cyclobutyl, wherein the methyl, ethyl, isopropyl, propyl, cyclopropyl or cyclobutyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy;

in certain embodiments, $R^{k14}$ is selected from 5- to 6-membered heteroaryl, wherein the heteroaryl is optionally further substituted with 0 to 4 (such as 0, 1, 2, 3 or 4) substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 (such as 1, 2, 3 or 4) heteroatoms selected from N, O or S;

in certain embodiments, K is selected from one of the structural fragments shown in Table K-a;

in certain embodiments, K is selected from one of the following structural fragments:

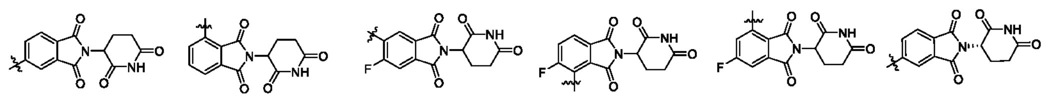

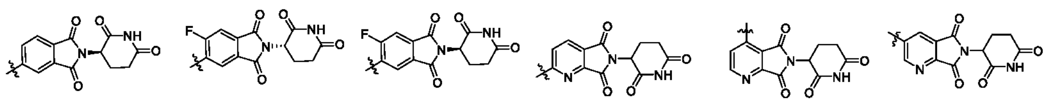

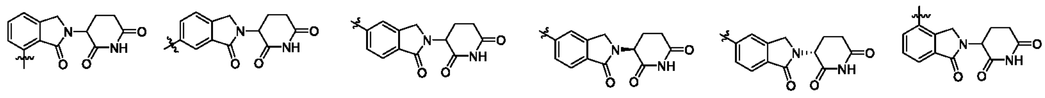

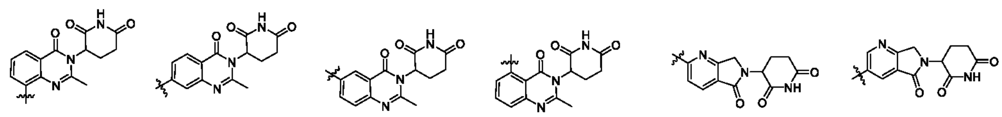

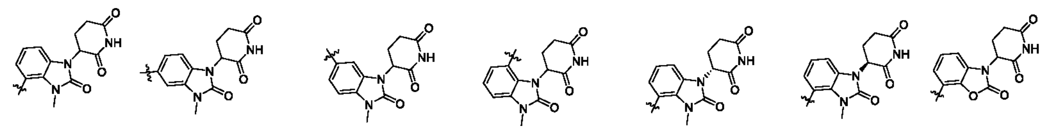

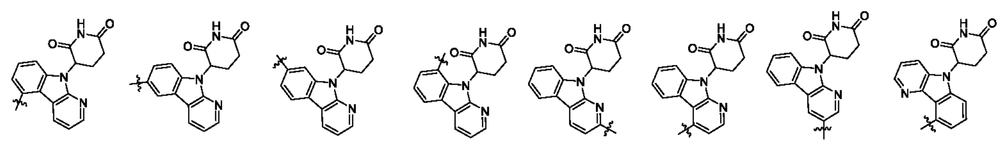

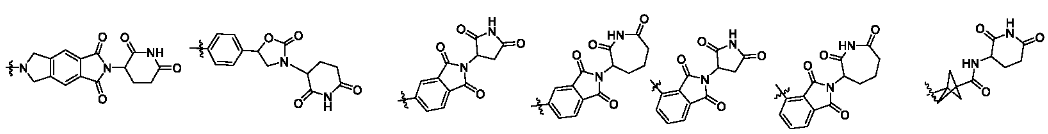

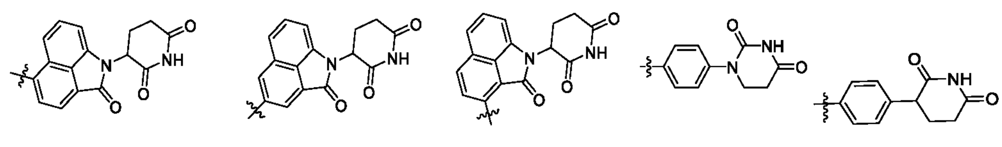

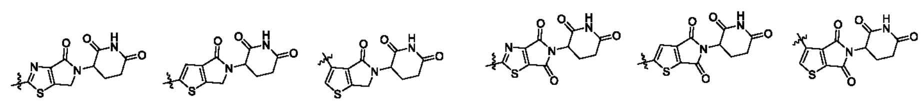

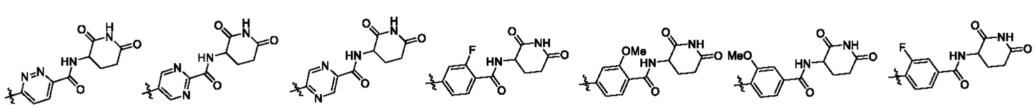

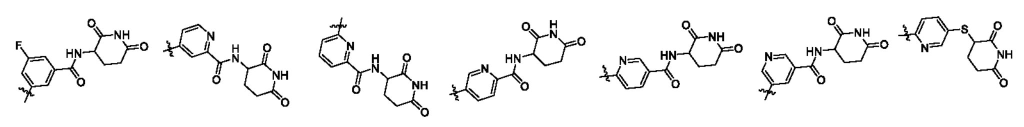

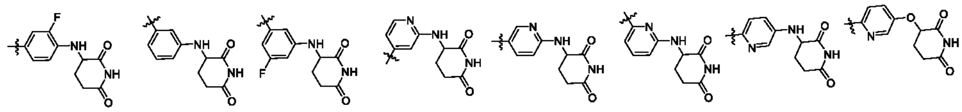

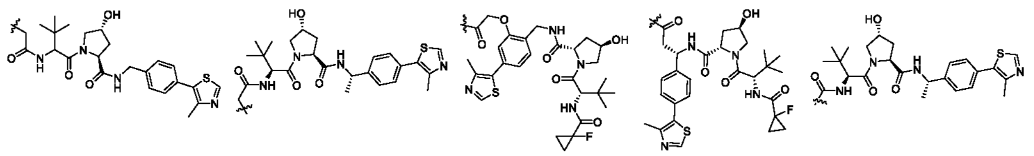

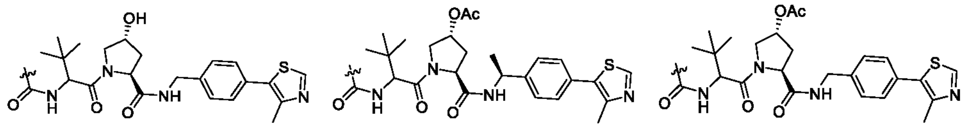
;

in certain embodiments, K is selected from

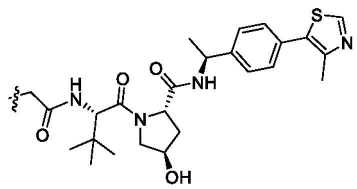
;

in certain embodiments, B is selected from

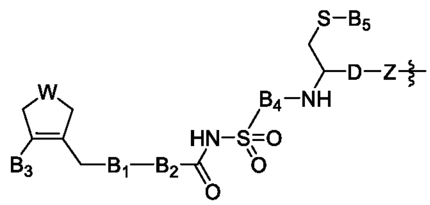
;

in certain embodiments, B is selected from

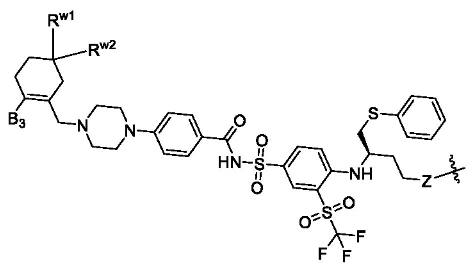
;

in certain embodiments, B is selected from

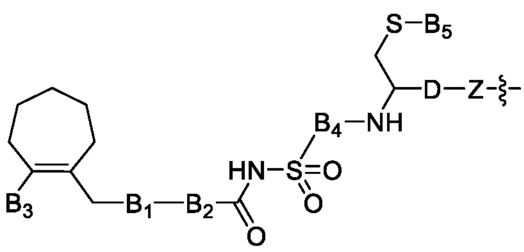
;

in some embodiments,

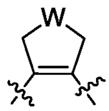

is selected from

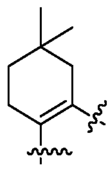
,
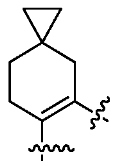
,
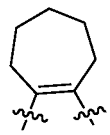
,

or

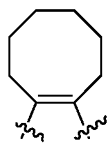
;

in certain embodiments, W is selected from $W_1$ or $W_2$;
in certain embodiments, $W_1$ is selected from $-CR^{w1}R^{w2}-$, $-(CR^{w1}R^{w2})_3-$, $-(CR^{w1}R^{w2})_4-$, $-CH_2CR^{w3}R^{w4}-$, $-CR^{w3}R^{w4}CH_2-$, $-CR^{w1}R^{w2}O-$, $-OCR^{w1}R^{w2}-$, $-CR^{w1}R^{w2}NR^{w5}-$, or $-NR^{w5}CR^{w1}R^{w2}-$;
in certain embodiments, $W_2$ is selected from $-(CR^{w1}R^{w2})_2-$;
in certain embodiments, D is selected from $C_{1-4}$ alkylene;
in certain embodiments, D is selected from ethylene;
in certain embodiments,

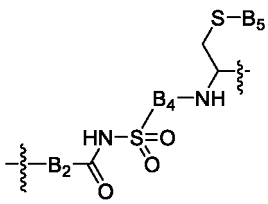

is selected from

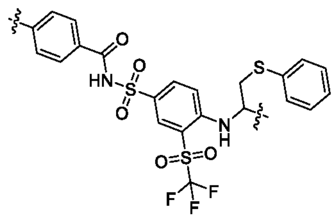

;

in certain embodiments, $B_1$ and Z are each independently selected from a 4- to 7-membered mono-heterocyclic ring, a 5- to 12-membered fused-heterocyclic ring, a 6- to 12-membered spiro-heterocyclic ring, or a 7- to 12-membered bridged-heterocyclic ring, the $B_1$ is optionally further substituted with 0 to 4 $R^{B1}$, and the Z is optionally further substituted with 0 to 4 $R^Q$, wherein the fused-heterocyclic ring, spiro-heterocyclic ring, or bridged-heterocyclic ring contains 1 to 3 heteroatoms selected from O, S or N, and is optionally further substituted with 0, 1 or 2 =O when the heteroatom is selected from S;

in certain embodiments, $B_1$ and Z are each independently selected from azetidinyl, azacyclopentyl, piperazinyl, piperidyl, azacyclohexenyl, azepanyl, 1,4-diazepanyl, cyclobutyl-fused-azetidinyl, cyclobutyl-fused-azacyclopentyl, cyclobutyl-fused-azacyclohexyl, cyclobutyl-fused-piperidyl, cyclopentyl-fused-azetidinyl, cyclopentyl-fused-azacyclopentyl, cyclopentyl-fused-azacyclohexyl, cyclopentyl-fused-piperidyl, cyclohexyl-fused-azetidinyl, cyclohexyl-fused-azacyclopentyl, cyclohexyl-fused-azacyclohexyl, cyclohexyl-fused-piperidyl, azetidinyl-fused-azetidinyl, azacyclopentyl-fused-azetidinyl, azacyclopentyl-fused-azacyclopentyl, azacyclopentyl-fused-azacyclohexyl, azacyclopentyl-fused-piperidyl, azacyclohexyl-fused-azetidinyl, azacyclohexyl-fused-azacyclopentyl, azacyclohexyl-fused-azacyclohexyl, azacyclohexyl-fused-piperidyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidyl, cyclopentyl-spiro-piperidyl, cyclohexyl-spiro-piperidyl, azetidinyl-spiro-piperidyl, azacyclopentyl-spiro-piperidyl, azacyclohexyl-spiro-piperidyl,

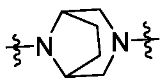 , 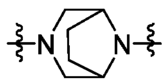 , 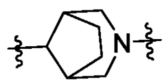 ,

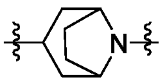 , 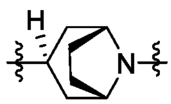 , 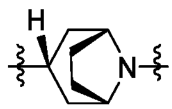 , 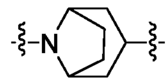 , 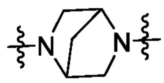 ,

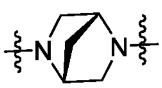 , 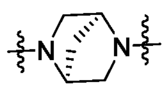 , 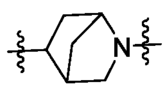 , 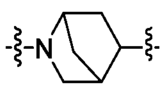 , 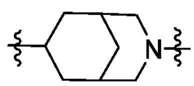 ,

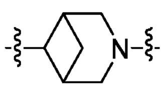 , 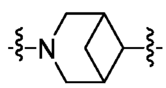 , 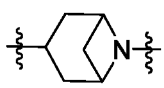 , 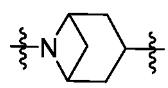 , 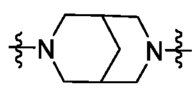 ,

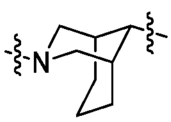 , 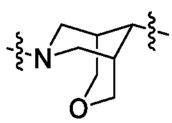 , 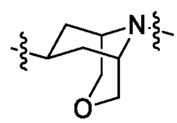 , 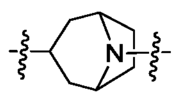 , 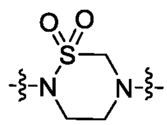 ,

or

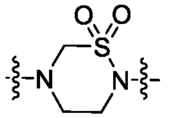,

the $B_1$ is optionally further substituted with 0 to 4 $R^{B1}$, and the Z is optionally further substituted with 0 to 4 $R^Q$; in certain embodiments, $B_1$ and Z are each independently selected from

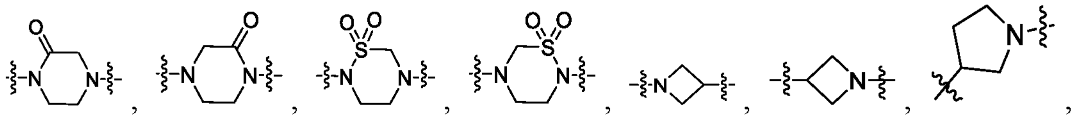, , , , , , ,

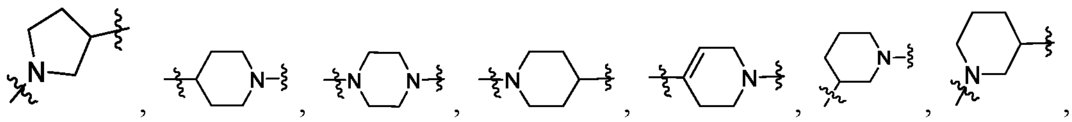, , , , , , ,

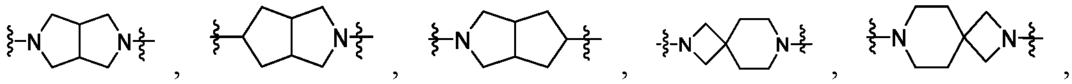, , , , ,

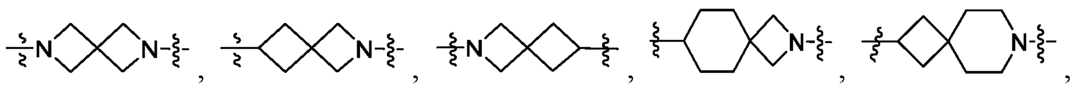, , , , ,

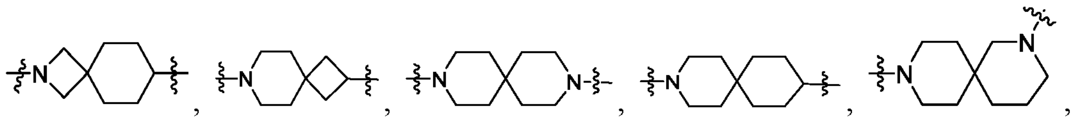, , , , ,

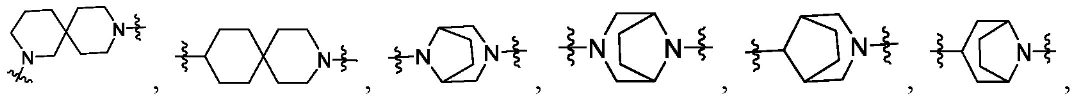, , , , , ,

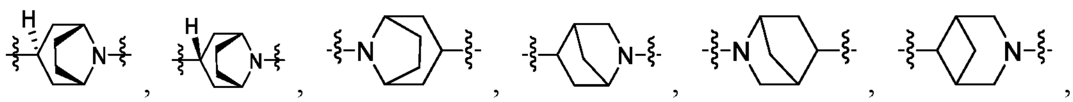, , , , , ,

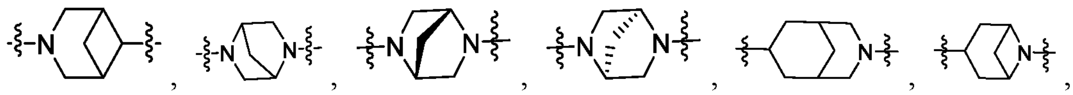, , , , , ,

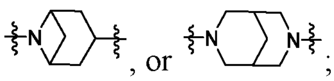, or ;

in certain embodiments, $B_1$ and Z are each independently selected from

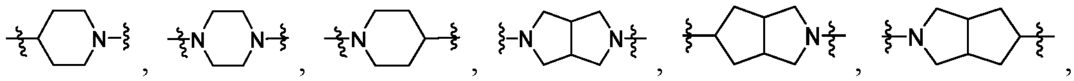, , , , , ,

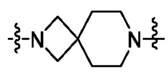, 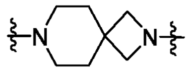, 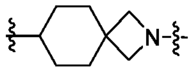, 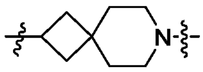, 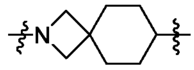,

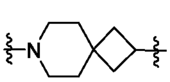, 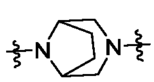, 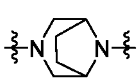, 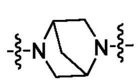, 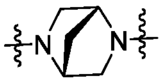, 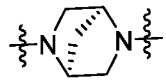, or

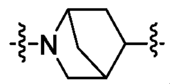;

in certain embodiments, $B_2$, $B_3$, $B_4$, and $B_5$ are each independently selected from $C_{6-10}$ aryl or 5- to 10-membered heteroaryl, the $B_2$ is optionally further substituted with 0 to 4 $R^{B2}$, the $B_3$ is optionally further substituted with 0 to 5 $R^{B3}$, the $B_4$ is optionally further substituted with 0 to 4 $R^{B4}$, and the $B_5$ is optionally further substituted with 0 to 5 $R^{B5}$, wherein the heteroaryl contains 1 to 3 heteroatoms selected from O, S or N;
in certain embodiments, $B_2$ and $B_4$ are each independently selected from phenyl or 5- to 6-membered heteroaryl, $B_3$ and $B_5$ are each independently selected from phenyl, naphthyl, 5- to 6-membered heteroaryl or benzo 5- to 6-membered heteroaryl, the $B_2$ is optionally further substituted with 0 to 4 $R^{B2}$, the $B_3$ is optionally further substituted with 0 to 5 $R^{B3}$, the $B_4$ is optionally further substituted with 0 to 4 $R^{B4}$, and the $B_5$ is optionally further substituted with 0 to 5 $R^{B5}$, wherein the heteroaryl contains 1 to 3 heteroatoms selected from O, S or N;
in certain embodiments, $B_3$ is selected from phenyl, naphthyl, 5- to 6-membered heteroaryl, or benzo 5- to 6-membered heteroaryl and the $B_3$ is optionally further substituted with 0 to 5 $R^{B3}$;
in certain embodiments, $B_3$ is selected from

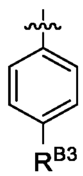 or 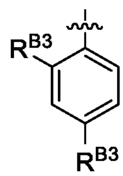;

in certain embodiments, $B_3$ is selected from

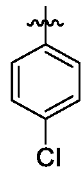 or 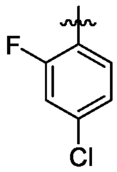;

in certain embodiments, $B_2$ and $B_4$ are each independently selected from phenyl, thienyl, furyl, pyrrolyl, imidazolyl, pyrazolyl, oxazolyl, thiazolyl, pyridyl, pyrimidyl, pyrazinyl, or pyridazinyl, $B_3$ and $B_5$ are each independently selected from phenyl, naphthyl, thienyl, furyl, pyrrolyl, imidazolyl, pyridyl, pyrimidyl, pyrazinyl, pyridazinyl, benzothienyl, benzofuryl, benzopyrrolyl, or benzoimidazolyl, the $B_2$ is optionally further substituted with 0 to 4 $R^{B2}$, the $B_3$ is optionally further substituted with 0 to 5 $R^{B3}$, the $B_4$ is optionally further substituted with 0 to 4 $R^{B4}$, and the $B_5$ is optionally further substituted with 0 to 5 $R^{B5}$;
in certain embodiments, $R^{B1}$, $R^Q$, $R^{B2}$, $R^{B3}$, and $R^{B5}$ are each independently selected from halogen, oxo, OH, CN, $C_{1-4}$ alkyl or $C_{1-4}$ alkoxy, wherein the alkyl or alkoxy is optionally further substituted with 0 to 4 substituents selected from H, halogen, OH, CN, or $C_{1-4}$ alkyl;
in certain embodiments, $R^{B1}$, $R^Q$, $R^{B2}$, $R^{B3}$ and $R^{B5}$ are each independently selected from F, Cl, Br, I, oxo, OH, CN, methyl, ethyl, methoxy or ethoxy, wherein the methyl, ethyl, methoxy or ethoxy is optionally further substituted with 0 to 4 substituents selected from H, halogen, OH, CN or $C_{1-4}$ alkyl;
in certain embodiments, each $R^{B4}$ is independently selected from $-SO_2-C_{1-4}$ alkyl, nitro, halogen, CN, OH, $C_{1-4}$ alkyl or $C_{1-4}$ alkoxy, wherein the alkyl or alkoxy is optionally further substituted with 0 to 4 substituents selected from H, halogen, OH, CN or $C_{1-4}$ alkyl;
in certain embodiments, each $R^{B4}$ is independently selected from $-SO_2$-methyl, $-SO_2$-ethyl, nitro, F, Cl, Br, I, OH, CN, methyl, ethyl, methoxy or ethoxy, wherein the methyl, ethyl, methoxy or ethoxy is optionally further substituted

with 0 to 4 substituents selected from H, halogen, OH, CN or $C_{1-4}$ alkyl;
in certain embodiments, each $R^{B4}$ is independently selected from $-SO_2-CF_3$ or nitro;
in certain embodiments, $R^{w1}$, $R^{w2}$ and $R^{w5}$ are each independently selected from H, halogen, CN, OH, $C_{1-4}$ alkyl or $C_{1-4}$ alkoxy, wherein the alkyl or alkoxy is optionally further substituted with 0 to 4 substituents selected from H, halogen, OH, CN or $C_{1-4}$ alkyl;
in certain embodiments, $R^{w1}$, $R^{w2}$ and $R^{w5}$ are each independently selected from H, F, Cl, Br, I, OH, CN, methyl, ethyl, methoxy or ethoxy, wherein the methyl, ethyl, methoxy or ethoxy is optionally further substituted with 0 to 4 substituents selected from H, halogen, OH, CN or $C_{1-4}$ alkyl;
in certain embodiments, $R^{w1}$ and $R^{w2}$ are directly connected to form $C_{3-6}$ carbocycle or 3- to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;
in certain embodiments, $R^{w1}$ and $R^{w2}$ are directly connected to form cyclopropyl, cyclobutyl, cyclopentyl, cyclohexyl, azetidinyl, azacyclopentyl, azacyclohexyl, oxetanyl, oxacyclopentyl, or oxacyclohexyl, wherein the cyclopropyl, cyclobutyl, cyclopentyl, cyclohexyl, azetidinyl, azacyclopentyl, azacyclohexyl, oxetanyl, oxacyclopentyl, or oxacyclohexyl is optionally further substituted with 0 to 4 substituents selected from H, halogen, OH, CN or $C_{1-4}$ alkyl;
in certain embodiments, $R^{w1}$ and $R^{w2}$ are selected from methyl, or $R^{w1}$ and $R^{w2}$ are directly connected to form, together with the carbon atom to which they are attached, cyclopropyl;
in certain embodiments, $R^{w3}$ and $R^{w4}$ are directly connected to form $C_{3-6}$ carbocycle or 3- to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;
in certain embodiments, $R^{w3}$ and $R^{w4}$ are directly connected to form cyclopropyl, cyclobutyl, cyclopentyl, cyclohexyl, azetidinyl, azacyclopentyl, azacyclohexyl, oxetanyl, oxacyclopentyl, or oxacyclohexyl, wherein the cyclopropyl, cyclobutyl, cyclopentyl, cyclohexyl, azetidinyl, azacyclopentyl, azacyclohexyl, oxetanyl, oxacyclopentyl, or oxacyclohexyl is optionally further substituted with 0 to 4 substituents selected from H, halogen, OH, CN or $C_{1-4}$ alkyl;
in certain embodiments, $R^{B1}$ and $R^{B2}$ are directly connected to form $C_{5-7}$ carbocycle or 5- to 7-membered heterocycle, wherein the carbocycle or heterocycle is optionally further substituted with 0 to 3 $R^c$, and the heterocycle contains 1 to 3 heteroatoms selected from O, S or N;
in certain embodiments, each $R^c$ is independently selected from halogen, OH, CN, =O, $C_{1-4}$ alkyl, $C_{1-4}$ alkoxy, $C_{3-6}$ carbocycle or 3- to 7-membered heterocycle, wherein the alkyl, alkoxy, carbocycle or heterocycle is optionally further substituted with 0 to 4 substituents selected from H, halogen, OH, CN, $C_{1-4}$ alkyl, $C_{1-4}$ alkoxy, $C_{3-6}$ cycloalkyl or 3- to 7-membered heterocycloalkyl, and the heterocycle or heterocycloalkyl contains 1 to 3 heteroatoms selected from O, S or N,
in certain embodiments, each $R^c$ is independently selected from F, Cl, Br, I, OH, CN, =O, methyl, ethyl, methoxy, ethoxy, cyclopropyl, cyclobutyl, cyclopentyl, cyclohexyl, oxetanyl, oxacyclopentyl, oxacyclohexyl, azetidinyl, azacyclopentyl, azacyclohexyl, morpholinyl, or piperazinyl, wherein the methyl, ethyl, methoxy, ethoxy, cyclopropyl, cyclobutyl, cyclopentyl, cyclohexyl, oxetanyl, oxacyclopentyl, oxacyclohexyl, azetidinyl, azacyclopentyl, azacyclohexyl, morpholinyl, or piperazinyl is optionally further substituted with 0 to 4 substituents selected from H, halogen, OH, CN, $C_{1-4}$ alkyl, $C_{1-4}$ alkoxy, $C_{3-6}$ cycloalkyl, or 3- to 7-membered heterocycloalkyl, and the heterocycloalkyl contains 1 to 3 heteroatoms selected from O, S or N;
in certain embodiments, Z is selected from

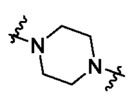, 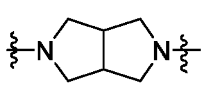,

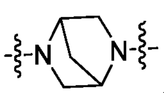, 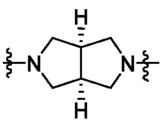, 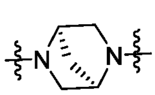 or 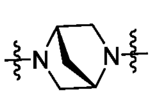;

in certain embodiments, when Z is selected from

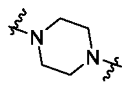,

and $R^{w1}$ and $R^{w2}$ are selected from methyl, $B_3$ is selected from

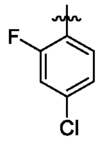;

in some embodiments, the compound of general formula (I) is defined as follows:
when $W_2$ is selected from $-(CR^{w1}R^{w2})_2-$, and $R^{w1}$ and $R^{w2}$ are each independently selected from F, methyl or methoxy, B at least satisfies any one of the following conditions:

1) $B_1$ is not piperazine;
2) Z is not piperazine, piperidine,

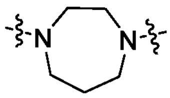, 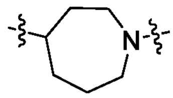, 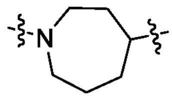,

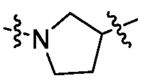, 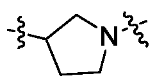, 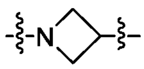, or 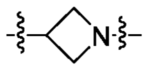;

3) when $B_3$ is selected from phenyl, the phenyl is substituted with 1 to 4 $R^{B3}$ and at least one $R^{B3}$ is not halogen, methyl or trifluoromethyl;
4) when $B_2$ is selected from phenyl, the phenyl is substituted with 1 to 4 $R^{B2}$;
5) $R^{B1}$ and $R^{B2}$ are directly connected to form $C_{5-7}$ carbocycle or 5- to 7-membered heterocycle, wherein the carbocycle or heterocycle is optionally further substituted with 0 to 3 $R^c$, and the heterocycle contains 1 to 3 heteroatoms selected from O, S or N;
6) when $B_5$ is selected from phenyl, the phenyl is substituted with 1 to 4 $R^{B5}$;

in some embodiments, the compound of general formula (I) is defined as follows:
when $W_2$ is selected from $-(CR^{w1}R^{w2})_2-$, and $R^{w1}$ and $R^{w2}$ are each independently selected from F, methyl or methoxy, B at least satisfies any one of the following conditions:

1) $B_1$ is not piperazine;
2) Z is not piperazine, piperidine,

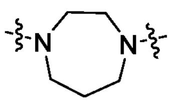, 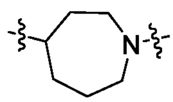, 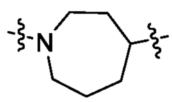,

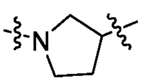, 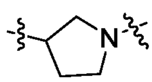, 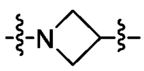, or 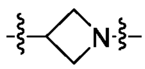;

3) $B_3$ is selected from phenyl substituted with 1 $R^{B3}$, when $R^{B3}$ is at the para-position of the phenyl, $R^{B3}$ is not halogen, methyl or trifluoromethyl;
4) when $B_2$ is selected from phenyl, the phenyl is substituted with 1 to 4 $R^{B2}$;
5) $R^{B1}$ and $R^{B2}$ are directly connected to form $C_{5-7}$ carbocycle or 5- to 7-membered heterocycle, wherein the carbocycle or heterocycle is optionally further substituted with 0 to 3 $R^c$, and the heterocycle contains 1 to 3 heteroatoms selected from O, S or N;
6) when $B_5$ is selected from phenyl, the phenyl is substituted with 1 to 4 $R^{B5}$;

optionally, 1 to 20 H of the compound of general formula (I) are replaced by 1 to 20 deuterium;
in certain embodiments, B is selected from one of the structural fragments shown in Table B-a;

in certain embodiments, each q is independently selected from 0, 1, 2, 3, 4, 5 or 6;
in certain embodiments, each q is independently selected from 0, 1, 2, 3 or 4;
in certain embodiments, each q is independently selected from 0, 1, 2 or 3;
in certain embodiments, each q is independently selected from 0, 1 or 2;
in certain embodiments, n1, n2 and n3 are each independently selected from 0, 1, 2 or 3;
in certain embodiments, each p1 or p2 is independently selected from 0, 1, 2, 3, 4 or 5;
in certain embodiments, each p1 or p2 is independently selected from 0, 1 or 2.

As a first embodiment of the present invention, provided is the above-mentioned compound of general formula (I) or the stereoisomer, deuterated compound, solvate, prodrug, metabolite, pharmaceutically acceptable salt or co-crystal thereof, wherein

L is selected from a bond or -$C_{1-50}$ hydrocarbyl-, wherein the hydrocarbyl has 0 to 20 methylene units optionally further replaced by -Ak- or -Cy-;

each -Ak- is independently selected from -$(CH_2)_q$-, -$(CH_2)_q$-O-, -O-$(CH_2)_q$-, -$(CH_2)_q$-$NR^L$-, -$NR^L$-$(CH_2)_q$-, -$(CH_2)_q$-$NR^L$C(=O)-, -$NR^L(CH_2)_q$C(=O)-, -$(CH_2)_q$-C(=O)$NR^L$-, -C(=O)-, -C(=O)-$(CH_2)_q$-$NR^L$-, -$(C{\equiv}C)_q$-, -CH=CH-, -$Si(R^L)_2$-, - $Si(OH)(R^L)$-, -$Si(OH)_2$-, -P(=O)($OR^L$)-, -P(=O)($R^L$)-, -S-, -S(=O)-, -$S(=O)_2$- or a bond, wherein the -$CH_2$- is optionally further substituted with 0 to 2 substituents selected from H, halogen, OH, CN, $NH_2$, $C_{1-6}$ alkyl, $C_{1-6}$ alkoxy, halogen-substituted $C_{1-6}$ alkyl, hydroxyl-substituted $C_{1-6}$ alkyl, or cyano-substituted $C_{1-6}$ alkyl;
each q is independently selected from 0, 1, 2, 3, 4, 5 or 6;
each $R^L$ is independently selected from H, $C_{1-6}$ alkyl, 3- to 7-membered heterocyclyl, 3- to 7-membered cycloalkyl, phenyl or 5- to 6-membered heteroaryl;
each -Cy- is independently selected from a bond, a 4- to 8-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally further substituted with 0, 1 or 2 =O when the heteroatom is selected from S; B is selected from

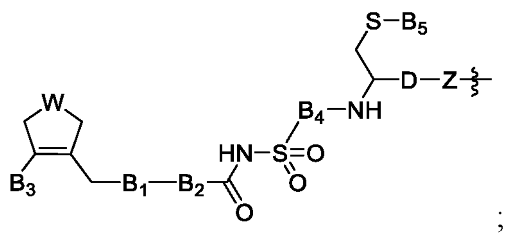
;

W is selected from $W_1$ or $W_2$;
$W_1$ is selected from -$CR^{w1}R^{w2}$-, -$(CR^{w1}R^{w2})_3$-, -$(CR^{w1}R^{w2})_4$-, -$CH_2CR^{w3}R^{w4}$-, - $CR^{w3}R^{w4}CH_2$-, -$CR^{w1}R^{w2}$O-, -O$CR^{w1}R^{w2}$-, -$CR^{ww1}R^{w2}NR^{w5}$-, or -$NR^{w5}CR^{w1}R^{w2}$-;
$W_2$ is selected from -$(CR^{w1}R^{w2})_2$-;
D is selected from $C_{1-4}$ alkylene;
$B_1$ and Z are each independently selected from a 4- to 7-membered mono-heterocyclic ring, a 5- to 12-membered fused-heterocyclic ring, a 6- to 12-membered spiro-heterocyclic ring, or a 7- to 12-membered bridged-heterocyclic ring, the $B_1$ is optionally further substituted with 0 to 4 $R^{B1}$, and the Z is optionally further substituted with 0 to 4 $R^Q$, wherein the fused-heterocyclic ring, spiro-heterocyclic ring, or bridged-heterocyclic ring contains 1 to 3 heteroatoms selected from O, S or N, and is optionally further substituted with 0, 1 or 2 =O when the heteroatom is selected from S;
$B_2$, $B_3$, $B_4$, and $B_5$ are each independently selected from $C_{6-10}$ aryl or 5- to 10-membered heteroaryl, the $B_2$ is optionally further substituted with 0 to 4 $R^{B2}$, the $B_3$ is optionally further substituted with 0 to 5 $R^{B3}$, the $B_4$ is optionally further substituted with 0 to 4 $R^{B4}$, and the $B_5$ is optionally further substituted with 0 to 5 $R^{B5}$, wherein the heteroaryl contains 1 to 3 heteroatoms selected from O, S or N;
$R^{B1}$, $R^Q$, $R^{B2}$, $R^{B3}$, and $R^{B5}$ are each independently selected from halogen, oxo, OH, CN, $C_{1-4}$ alkyl or $C_{1-4}$ alkoxy, wherein the alkyl or alkoxy is optionally further substituted with 0 to 4 substituents selected from H, halogen, OH,

CN, or $C_{1-4}$ alkyl;

each $R^{B4}$ is independently selected from -$SO_2$-$C_{1-4}$ alkyl, nitro, halogen, CN, OH, $C_{1-4}$ alkyl or $C_{1-4}$ alkoxy, wherein the alkyl or alkoxy is optionally further substituted with 0 to 4 substituents selected from H, halogen, OH, CN or $C_{1-4}$ alkyl;

$R^{w1}$, $R^{w2}$ and $R^{w5}$ are each independently selected from H, halogen, CN, OH, $C_{1-4}$ alkyl or $C_{1-4}$ alkoxy, wherein the alkyl or alkoxy is optionally further substituted with 0 to 4 substituents selected from H, halogen, OH, CN or $C_{1-4}$ alkyl; alternatively, $R^{w1}$ and $R^{w2}$ are directly connected to form $C_{3-6}$ carbocycle or 3-to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;

$R^{w3}$ and $R^{w4}$ are directly connected to form $C_{3-6}$ carbocycle or 3- to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;

alternatively, $R^{B1}$ and $R^{B2}$ are directly connected to form $C_{5-7}$ carbocycle or 5-to 7-membered heterocycle, wherein the carbocycle or heterocycle is optionally further substituted with 0 to 3 $R^c$, and the heterocycle contains 1 to 3 heteroatoms selected from O, S or N;

each $R^c$ is independently selected from halogen, OH, CN, =O, $C_{1-4}$ alkyl, $C_{1-4}$ alkoxy, $C_{3-6}$ carbocycle or 3- to 7-membered heterocycle, wherein the alkyl, alkoxy, carbocycle or heterocycle is optionally further substituted with 0 to 4 substituents selected from H, halogen, OH, CN, $C_{1-4}$ alkyl, $C_{1-4}$ alkoxy, $C_{3-6}$ cycloalkyl or 3- to 7-membered heterocycloalkyl, and the heterocycle or heterocycloalkyl contains 1 to 3 heteroatoms selected from O, S or N, provided that when $W_2$ is selected from -$(CR^{w1}R^{w2})_2$-, and $R^{w1}$ and $R^{w2}$ are each independently selected from F, methyl or methoxy, B at least satisfies any one of the following conditions:

1) $B_1$ is not piperazine;

2) Z is not piperazine, piperidine,

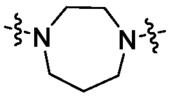,
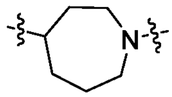,
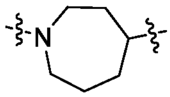,

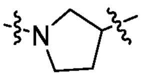,
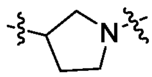,
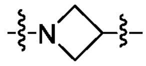, or
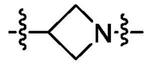;

3) $B_3$ is selected from phenyl substituted with 1 $R^{B3}$, when $R^{B3}$ is at the para-position of the phenyl, $R^{B3}$ is not halogen, methyl or trifluoromethyl;

4) when $B_2$ is selected from phenyl, the phenyl is substituted with 1 to 4 $R^{B2}$;

5) $R^{B1}$ and $R^{B2}$ are directly connected to form $C_{5-7}$ carbocycle or 5- to 7-membered heterocycle, wherein the carbocycle or heterocycle is optionally further substituted with 0 to 3 $R^c$, and the heterocycle contains 1 to 3 heteroatoms selected from O, S or N;

6) when $B_5$ is selected from phenyl, the phenyl is substituted with 1 to 4 $R^{B5}$; K is selected from

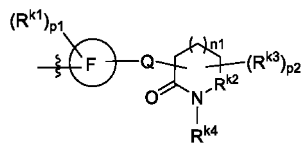,
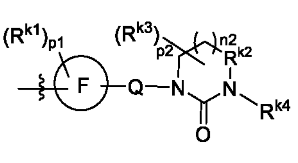,

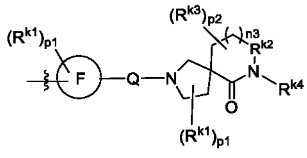,
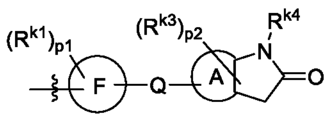,
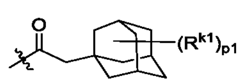,

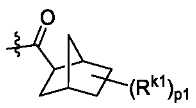 , 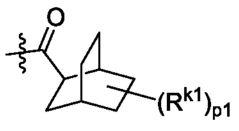 , 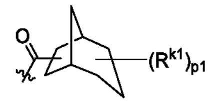 , 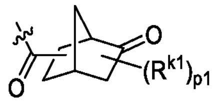 ,

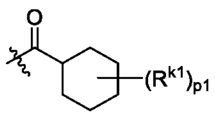 , 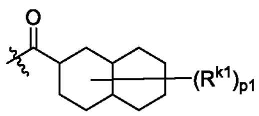 , 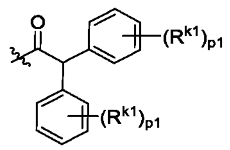 , 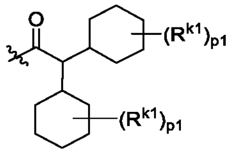 ,

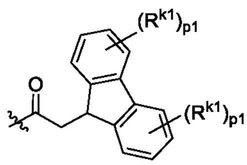 , 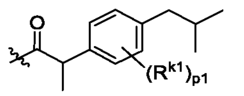 , 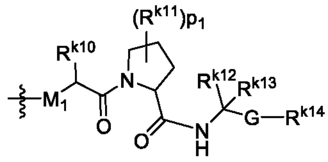 ,

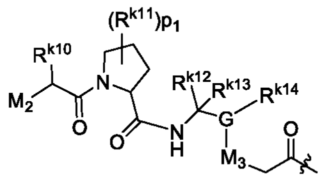 or 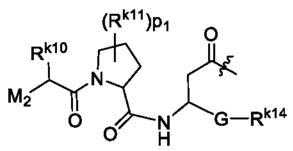 ;

each Q is independently selected from a bond, -O-, -S-, $-CH_2-$, $-NR^q-$, -CO-, - $NR^qCO-$, $-CONR^q-$ or 3- to 12-membered heterocyclyl, wherein the heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

$R^q$ is selected from H or $C_{1-6}$ alkyl;

A is selected from $C_{3-10}$ carbocyclyl, $C_{6-10}$ aryl, 3- to 10-membered heterocyclyl or 5- to 10-membered heteroaryl, wherein the heterocycle or heteroaryl contains 1 to 4 heteroatoms selected from O, S or N;

each F is independently selected from $C_{3-20}$ carbocyclyl, $C_{6-20}$ aryl, 3- to 20-membered heterocyclyl or 5- to 20-membered heteroaryl, wherein the heterocyclyl or heteroaryl contains 1 to 4 heteroatoms selected from O, S or N;

each $R^{k2}$ is independently selected from a bond, -CO-, $-SO_2-$, -SO- or - $C(R^{k3})_2-$;

each $R^{k1}$ is independently selected from H, F, Cl, Br, I, OH, =O, $NH_2$, CN, COOH, $CONH_2$, $C_{1-6}$ alkyl or $C_{1-6}$ alkoxy, wherein the alkyl or alkoxy is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy;

each $R^{k3}$ is independently selected from H, F, Cl, Br, I, OH, =O, $NH_2$, CN, COOH, $CONH_2$, $C_{1-6}$ alkyl, $C_{1-6}$ alkoxy, $C_{3-8}$ cycloalkyl or 3- to 8-membered heterocyclyl, wherein the alkyl, alkoxy, cycloalkyl or heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

or two $R^{k3}$ together with the carbon atoms or ring backbones to which they are directly attached form 3- to 8-membered carbocycle or 3- to 8-membered heterocycle, wherein the carbocycle or heterocycle is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;

each $R^{k4}$ is independently selected from H, OH, $NH_2$, CN, $CONH_2$, $C_{1-6}$ alkyl, $C_{3-8}$ cycloalkyl or 3- to 8-membered heterocyclyl, wherein the alkyl, cycloalkyl or heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

$M_1$ is selected from a bond, -C(=O)NH-, -NHC(=O)-, $-CH_2-C(=O)NH-$, - $C(=O)CH_2NH-$, or 5- to 6-membered heteroaryl, wherein the heteroaryl is optionally further substituted with 0 to 4 substituents selected from H, F, Cl, Br, I, OH, =O, $CF_3$, $NH_2$, CN, $C_{1-4}$ alkyl, halogen-substituted $C_{1-4}$ alkyl, hydroxyl-substituted $C_{1-4}$ alkyl or $C_{1-4}$ alkoxy, and the heteroaryl contains 1 to 4 heteroatoms selected from O, S or N;

$M_2$ is selected from $-NHC(=O)-C_{1-6}$ alkyl, $-NHC(=O)-C_{3-6}$ cycloalkyl or 4- to 10-membered heterocyclyl, wherein the

alkyl, cycloalkyl or heterocyclyl is optionally further substituted with 0 to 4 substituents selected from H, F, Cl, Br, I, =O, OH, $NH_2$, $C_{1-4}$ alkyl or $C_{1-4}$ alkoxy, and the heterocyclyl contains 1 to 4 heteroatoms selected from O, S or N;
$M_3$ is selected from -NH- or -O-;
$R^{k10}$ is selected from $C_{1-6}$ alkyl, wherein the alkyl is optionally further substituted with 0 to 4 substituents selected from H, F, Cl, Br, I, =O, OH, $C_{1-6}$ alkyl or $C_{3-6}$ cycloalkyl;
each $R^{k11}$ is independently selected from H, F, Cl, Br, I, =O, OH, SH, $C_{1-6}$ alkyl, $C_{1-6}$ alkoxy or $C_{1-6}$ alkylthio or -O-C(=O)-$C_{1-6}$ alkyl, wherein the alkyl, alkoxy or alkylthio is optionally further substituted with 0 to 4 substituents selected from H, F, Cl, Br, I, OH, $C_{1-4}$ alkyl or $C_{1-4}$ alkoxy;
$R^{k12}$ and $R^{k13}$ are each independently selected from H, $C_{1-6}$ alkyl or $C_{3-6}$ cycloalkyl, wherein the alkyl or cycloalkyl is optionally further substituted with 0 to 4 substituents selected from H, F, Cl, Br, I, =O, OH, $NH_2$, $C_{1-4}$ alkyl or $C_{1-4}$ alkoxy;
$R^{k14}$ is selected from 5- to 6-membered heteroaryl, wherein the heteroaryl is optionally further substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 heteroatoms selected from N, O or S;
G is selected from 6- to 10-membered aryl or 5- to 10-membered heteroaryl, wherein the aryl or heteroaryl is optionally further substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 heteroatoms selected from N, O or S;
optionally, 1 to 20 H of the compound of general formula (I) are replaced by 1 to 20 deuterium;
n1, n2 and n3 are each independently selected from 0, 1, 2 or 3;
each p1 or p2 is independently selected from 0, 1, 2, 3, 4 or 5.

As a second embodiment of the present invention, provided is the above-mentioned compound of general formula (I) or the stereoisomer, deuterated compound, solvate, prodrug, metabolite, pharmaceutically acceptable salt or co-crystal thereof, wherein

L is selected from -Cy1-Ak1-Cy2-Ak2-Cy3-Ak3-Cy4-Ak4-Cy5-Ak5-, -Cy1-Cy2-Cy3 -Cy4-Ak 1 -Ak2-Ak3 - Ak4-Ak5 -, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, - Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, -Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, - Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, - Ak 1 - Ak2-Cy 1 -Cy2-Cy 3 - Ak3 - Ak4-Ak5 -Cy4-, - Ak 1 - Ak2-Ak3 -Cy 1 - Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, -Ak1-Ak2-Ak3-Ak4-Cy1-Cy2-Cy3-Ak5-Cy4-, -Ak1-, -Ak1-Ak2-, -Ak1-Ak2-Ak3-, -Ak1-Ak2-Ak3-Ak4-, - Ak1-Ak2-Ak3-Ak4-Ak5-, -Ak1-Ak2-Ak3-Ak4-Ak5-Ak6-, -Ak1-Ak2-Ak3-Ak4-Ak5-Ak6-Ak7-, -Ak1-Ak2-Ak3-Ak4-Ak5-Ak6-Ak7-Ak8-, or -Ak1-Ak2-Ak3-Ak4-Ak5-Ak6-Ak7-Ak8-Ak9;
Ak1, Ak2, Ak3, Ak4, Ak5, Ak6, Ak7, Ak8 and Ak9 are each independently selected from -$(CH_2)_q$-, -$(CH_2)_q$-O-, -O-$(CH_2)_q$-, -$(CH_2)_q$-$NR^L$-, -$NR^L$-$(CH_2)_q$-, - $(CH_2)$q-$NR^L$C(=O)-, -$(CH_2)_q$-C(=O)$NR^L$-, -C(=O)-, -C(=O)-$(CH_2)_q$-$NR^L$-, - $(C{\equiv}C)_q$- or a bond, wherein the -$CH_2$- is optionally further substituted with 0 to 2 substituents selected from H, halogen, OH, CN, $NH_2$, $C_{1-4}$ alkyl, $C_{1-4}$ alkoxy, halogen-substituted $C_{1-4}$ alkyl, hydroxyl-substituted $C_{1-4}$ alkyl or cyano-substituted $C_{1-4}$ alkyl;
each Cy1, Cy2, Cy3, Cy4 or Cy5 is independently selected from a bond, a 4-to 7-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring

contains 1 to 4 heteroatoms selected from O, S or N, and is optionally further substituted with 0, 1 or 2 =O when the heteroatom is selected from S;
each q is independently selected from 0, 1, 2, 3 or 4;
each $R^L$ is independently selected from H or $C_{1-6}$ alkyl;
the definitions of other groups are the same as those in the first embodiment of the present invention.

As a third embodiment of the present invention, provided is the above-mentioned compound of general formula (I) or the stereoisomer, deuterated compound, solvate, prodrug, metabolite, pharmaceutically acceptable salt or co-crystal thereof, wherein

Ak1, Ak2, Ak3, Ak4, Ak5, Ak6, Ak7, Ak8 and Ak9 are each independently selected from $-(CH_2)_q-$, $-(CH_2)_q-O-$, $-O-(CH_2)_q-$, $-(CH_2)_q-NR^L-$, $-NR^L-(CH_2)_q-$, - $(CH_2)q-NR^LC(=O)-$, $-(CH_2)_q-C(=O)NR^L-$, -C(=O)-, $-C(=O)-(CH_2)_q-NR^L-$, - $(C\equiv C)_q-$, or a bond, wherein the $-CH_2-$ is optionally further substituted with 0 to 2 substituents selected from H, F, Cl, Br, I, OH, CN, $NH_2$, $CF_3$, hydroxymethyl, $C_{1-4}$ alkyl, or $C_{1-4}$ alkoxy;
each Cy1, Cy2, Cy3, Cy4 or Cy5 is independently selected from a bond, a 4-to 7-membered nitrogen-containing mono-heterocyclic ring, a 4- to 10-membered nitrogen-containing fused-heterocyclic ring, a 5- to 12-membered nitrogen-containing spiro-heterocyclic ring, a 7- to 10-membered nitrogen-containing bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally further substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused-heterocyclic ring, bridged-heterocyclic ring, spiro-heterocyclic ring or heteroaryl contains 1 to 4 heteroatoms selected from O, S or N, and is optionally further substituted with 0, 1 or 2 =O when the heteroatom is selected from S;
each $R^L$ is independently selected from H or $C_{1-4}$ alkyl;
K is selected from

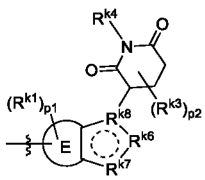 , 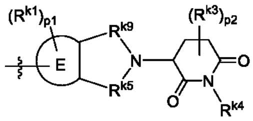 , 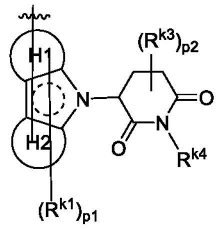 ,

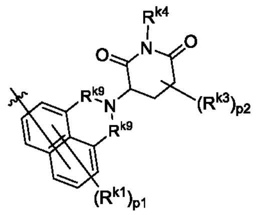 , 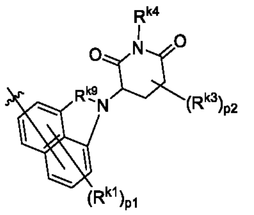 , 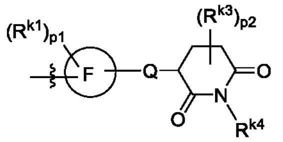 ,

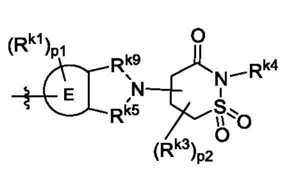 , 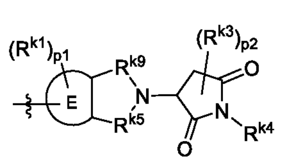 , 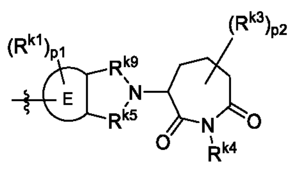 ,

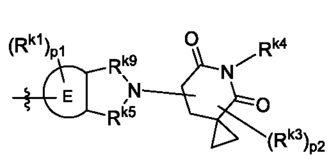 , 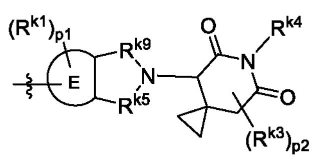 , 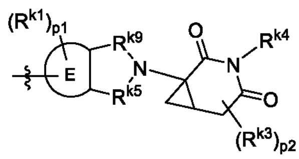 ,

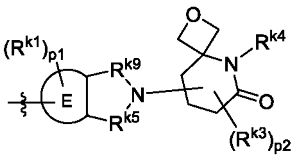, 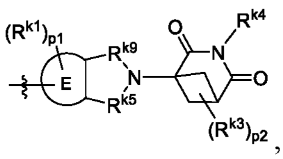, 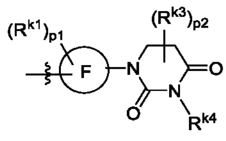, 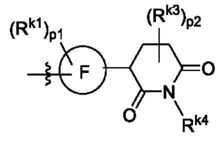,

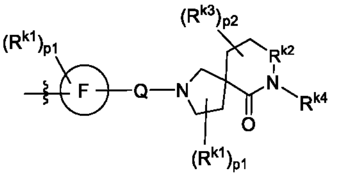, 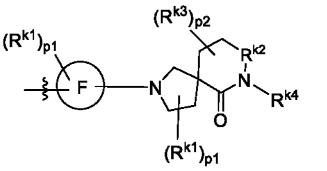, 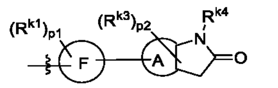,

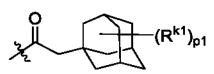, 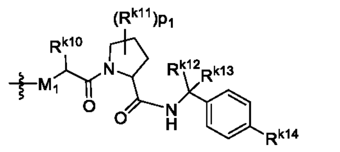, 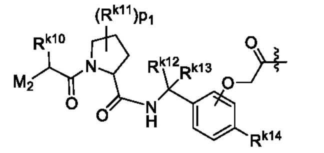

or

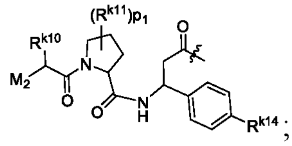;

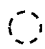 ]

represents a ring selected from an aromatic ring or a non-aromatic ring;

$M_2$ is selected from -NHC(=O)-$C_{1-4}$ alkyl, -NHC(=O)-$C_{3-6}$ cycloalkyl or 4- to 10-membered heterocyclyl, wherein the alkyl, cycloalkyl or heterocyclyl is optionally further substituted with 0 to 4 substituents selected from H, F, Cl, Br, I, =O, OH, $NH_2$, $C_{1-4}$ alkyl or $C_{1-4}$ alkoxy, and the heterocyclyl contains 1 to 4 heteroatoms selected from O, S or N;

$R^{k10}$ is selected from $C_{1-4}$ alkyl, wherein the alkyl is optionally further substituted with 0 to 4 substituents selected from H, F, Cl, Br, I, =O, OH, $C_{1-4}$ alkyl or $C_{3-6}$ cycloalkyl;

each $R^{k11}$ is independently selected from H, F, Cl, Br, I, =O, OH, SH, $C_{1-4}$ alkyl, $C_{1-4}$ alkoxy or $C_{1-4}$ alkylthio or -O-C(=O)-$C_{1-4}$ alkyl, wherein the alkyl, alkoxy or alkylthio is optionally further substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, $C_{1-4}$ alkyl or $C_{3-6}$ cycloalkyl, wherein the alkyl or cycloalkyl is optionally further substituted with 0 to 4 substituents selected from H, F, Cl, Br, I, =O, OH, $NH_2$, $C_{1-4}$ alkyl or $C_{1-4}$ alkoxy;

each Q is independently selected from -O-, -S-, -$CH_2$-, -$NR^q$-, -CO-, -$NR^q$CO-, -CO$NR^q$- or 4- to 7-membered heterocyclyl, wherein the heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

$R^q$ is selected from H or $C_{1-4}$ alkyl;

$R^{k1}$ and $R^{k3}$ are each independently selected from H, F, Cl, Br, I, OH, =O, $NH_2$, $CF_3$, CN, COOH, $CONH_2$, $C_{1-4}$ alkyl or $C_{1-4}$ alkoxy, wherein the alkyl or alkoxy is optionally further substituted with 0 to 4 substituents selected from H, F, Cl, Br, I, OH, or $NH_2$;

or two $R^{k3}$ together with the carbon atoms or ring backbones to which they are directly attached form 3- to 6-membered carbocycle or 3- to 7-membered heterocycle, wherein the carbocycle or heterocycle is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;

each $R^{k4}$ is independently selected from H, OH, $NH_2$, $CF_3$, CN or $C_{1-4}$ alkyl;

each $R^{k5}$ is independently selected from CO, $CH_2$, $SO_2$ or

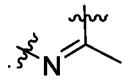;

each $R^{k6}$ is independently selected from CO, CH, SO, $SO_2$, $CH_2$ or N;
each $R^{k7}$ is independently selected from CO, CH, N, $CH_2$, O, S, $N(CH_3)$ or NH;
each $R^{k8}$ is independently selected from C, N or CH;
each $R^{k9}$ is independently selected from CO, $CH_2$ or $SO_2$;
each A, H1 or H2 is independently selected from $C_{3-8}$ carbocycle, a benzene ring, 4- to 7-membered heterocycle or 5- to 6-membered heteroaryl, wherein the heterocycle or heteroaryl contains 1 to 4 heteroatoms selected from O, S or N;
each E is independently selected from $C_{3-8}$ carbocycle, a benzene ring, 4- to 7-membered heterocycle, 8- to 12-membered heterocycle, 7- to 12-membered heteroaryl or 5- to 6-membered heteroaryl, wherein the heterocycle or heteroaryl contains 1 to 4 heteroatoms selected from O, S or N;
each F is independently selected from 3- to 7-membered monocycloalkyl, 4-to 10-membered fused cycloalkyl, 5- to 12-membered spiro cycloalkyl, 5- to 10-membered bridged cycloalkyl, a 4- to 7-membered mono-heterocyclic ring, a 4- to 10-membered fused-heterocyclic ring, a 5- to 12-membered spiro-heterocyclic ring, a 5- to 10-membered bridged-heterocyclic ring, $C_{6-14}$ aryl or 5- to 10-membered heteroaryl, wherein the mono-heterocyclic ring, fused-heterocyclic ring, spiro-heterocyclic ring, bridged-heterocyclic ring or heteroaryl contains 1 to 4 heteroatoms selected from O, S or N;
the definitions of other groups are the same as those in either the first or second embodiment of the present invention.

As a fourth embodiment of the present invention, provided is the above-mentioned compound of general formula (I) or the stereoisomer, deuterated compound, solvate, prodrug, metabolite, pharmaceutically acceptable salt or co-crystal thereof, wherein

Ak1, Ak2, Ak3, Ak4, Ak5, Ak6, Ak7, Ak8 and Ak9 are each independently selected from $-(CH_2)_q-$, $-(CH_2)_q-O-$, $-O-(CH_2)_q-$, $-(CH_2)_q-NR^L-$, $-NR^L-(CH_2)_q-$, - $(CH_2)q-NR^LC(=O)-$, $-(CH_2)_q-C(=O)NR^L-$, $-C(=O)-$, $-C(=O)-(CH_2)_q-NR^L-$, - $(C\equiv C)_q-$, or a bond, wherein the $-CH_2-$ is optionally further substituted with 0 to 2 substituents selected from H, F, Cl, Br, I, OH, CN, $NH_2$, $CF_3$, hydroxymethyl, methyl, ethyl, methoxy or ethoxy;
$R^L$ is selected from H, methyl or ethyl;
each q is independently selected from 0, 1, 2 or 3;
each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, azacyclopentyl, azacyclohexenyl, piperidyl, morpholinyl, piperazine, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclopropyl-fused-piperidyl, cyclobutyl-fused-azetidinyl, cyclobutyl-fused-azacyclopentyl, cyclobutyl-fused-azacyclohexyl, cyclobutyl-fused-piperidyl, cyclopentyl-fused-azetidinyl, cyclopentyl-fused-azacyclopentyl, cyclopentyl-fused-azacyclohexyl, cyclopentyl-fused-piperidyl, cyclohexyl-fused-azetidinyl, cyclohexyl-fused-azacyclopentyl, cyclohexyl-fused-azacyclohexyl, cyclohexyl-fused-piperidyl, azetidinyl-fused-azetidinyl, azetidinyl-fused-azacyclopentyl, azetidinyl-fused-azacyclohexyl, azetidinyl-fused-piperidyl, azacyclopentyl-fused-azetidinyl, azacyclopentyl-fused-azacyclopentyl, azacyclopentyl-fused-azacyclohexyl, azacyclopentyl-fused-piperidyl, azacyclohexyl-fused-azetidinyl, azacyclohexyl-fused-azacyclopentyl, azacyclohexyl-fused-azacyclohexyl, azacyclohexyl-fused-piperidyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidyl, cyclopentyl-spiro-piperidyl, cyclohexyl-spiro-piperidyl, azetidinyl-spiro-piperidyl, azacyclopentyl-spiro-piperidyl, azacyclohexyl-spiro-piperidyl,

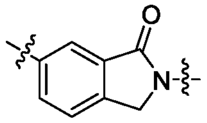, 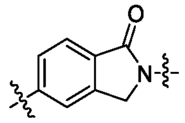,

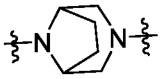, 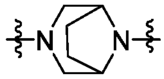, 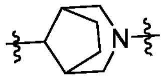, 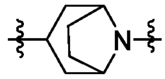, 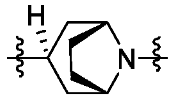,

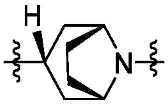, 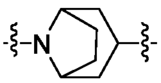, 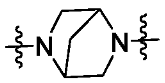, 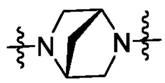, 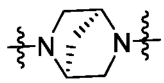, 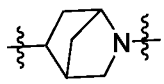

, 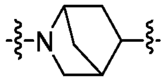, 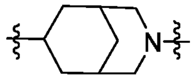, 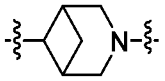, 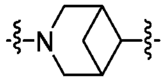, 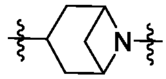,

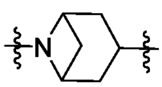, 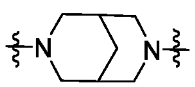, 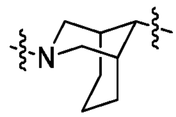, 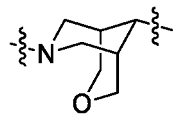, 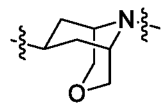,

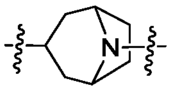, 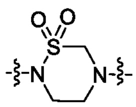, or 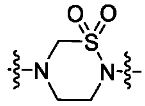,

which, when substituted, is optionally further substituted with 0 to 4 substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl, or $C_{1-4}$ alkoxy;

D is selected from ethylene;

$B_1$ and Z are each independently selected from azetidinyl, azacyclopentyl, piperazinyl, piperidyl, azacyclohexenyl, azepanyl, 1,4-diazepanyl, cyclobutyl-fused-azetidinyl, cyclobutyl-fused-azacyclopentyl, cyclobutyl-fused-azacyclohexyl, cyclobutyl-fused-piperidyl, cyclopentyl-fused-azetidinyl, cyclopentyl-fused-azacyclopentyl, cyclopentyl-fused-azacyclohexyl, cyclopentyl-fused-piperidyl, cyclohexyl-fused-azetidinyl, cyclohexyl-fused-azacyclopentyl, cyclohexyl-fused-azacyclohexyl, cyclohexyl-fused-piperidyl, azetidinyl-fused-azetidinyl, azacyclopentyl-fused-azetidinyl, azacyclopentyl-fused-azacyclopentyl, azacyclopentyl-fused-azacyclohexyl, azacyclopentyl-fused-piperidyl, azacyclohexyl-fused-azetidinyl, azacyclohexyl-fused-azacyclopentyl, azacyclohexyl-fused-azacyclohexyl, azacyclohexyl-fused-piperidyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidyl, cyclopentyl-spiro-piperidyl, cyclohexyl-spiro-piperidyl, azetidinyl-spiro-piperidyl, azacyclopentyl-spiro-piperidyl, azacyclohexyl-spiro-piperidyl,

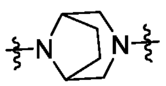, 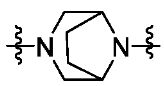, 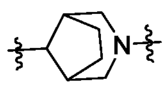,

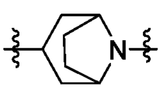, 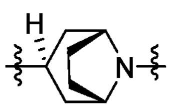, 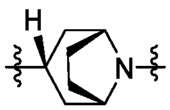, 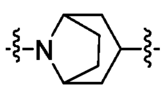, 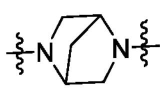,

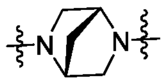, 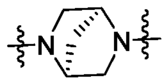, 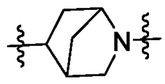, 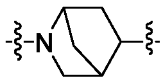, 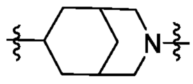,

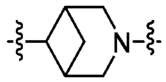, 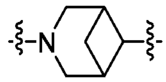, 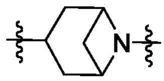, 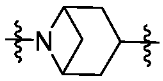, 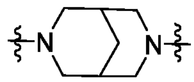,

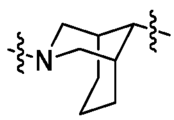, 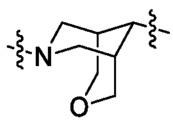, 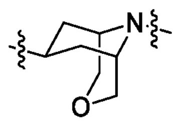, 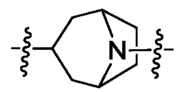, 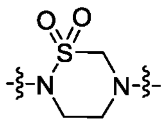, or

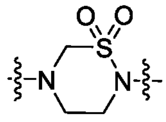,

the $B_1$ is optionally further substituted with 0 to 4 $R^{B1}$, and the Z is optionally further substituted with 0 to 4 $R^Q$;
$B_2$ and $B_4$ are each independently selected from phenyl or 5- to 6-membered heteroaryl, $B_3$ and $B_5$ are each independently selected from phenyl, naphthyl, 5- to 6-membered heteroaryl or benzo 5- to 6-membered heteroaryl, the $B_2$ is optionally further substituted with 0 to 4 $R^{B2}$, the $B_3$ is optionally further substituted with 0 to 5 $R^{B3}$, the $B_4$ is optionally further substituted with 0 to 4 $R^{B4}$, and the $B_5$ is optionally further substituted with 0 to 5 $R^{B5}$, wherein the heteroaryl contains 1 to 3 heteroatoms selected from O, S or N;
$R^{B1}$, $R^Q$, $R^{B2}$, $R^{B3}$ and $R^{B5}$ are each independently selected from F, Cl, Br, I, oxo, OH, CN, methyl, ethyl, methoxy or ethoxy, wherein the methyl, ethyl, methoxy or ethoxy is optionally further substituted with 0 to 4 substituents selected from H, halogen, OH, CN or $C_{1-4}$ alkyl;
each $R^{B4}$ is independently selected from $-SO_2$-methyl, $-SO_2$-ethyl, nitro, F, Cl, Br, I, OH, CN, methyl, ethyl, methoxy or ethoxy, wherein the methyl, ethyl, methoxy or ethoxy is optionally further substituted with 0 to 4 substituents selected from H, halogen, OH, CN or $C_{1-4}$ alkyl;
$R^{w1}$, $R^{w2}$ and $R^{w5}$ are each independently selected from H, F, Cl, Br, I, OH, CN, methyl, ethyl, methoxy or ethoxy, wherein the methyl, ethyl, methoxy or ethoxy is optionally further substituted with 0 to 4 substituents selected from H, halogen, OH, CN or $C_{1-4}$ alkyl;
alternatively, $R^{w1}$ and $R^{w2}$ are directly connected to form $C_{3-6}$ carbocycle or 3-to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;
$R^{w3}$ and $R^{w4}$ are directly connected to form $C_{3-6}$ carbocycle or 3- to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;
alternatively, $R^{B1}$ and $R^{B2}$ are directly connected to form $C_{5-7}$ carbocycle or 5-to 7-membered heterocycle, wherein the carbocycle or heterocycle is optionally further substituted with 0 to 3 $R^c$, and the heterocycle contains 1 to 3 heteroatoms selected from O, S or N;
each $R^c$ is independently selected from F, Cl, Br, I, OH, CN, =O, methyl, ethyl, methoxy, ethoxy, cyclopropyl, cyclobutyl, cyclopentyl, cyclohexyl, oxetanyl, oxacyclopentyl, oxacyclohexyl, azetidinyl, azacyclopentyl, azacyclohexyl, morpholinyl, or piperazinyl, wherein the methyl, ethyl, methoxy, ethoxy, cyclopropyl, cyclobutyl, cyclopentyl, cyclohexyl, oxetanyl, oxacyclopentyl, oxacyclohexyl, azetidinyl, azacyclopentyl, azacyclohexyl, morpholinyl, or piperazinyl is optionally further substituted with 0 to 4 substituents selected from H, halogen, OH, CN, $C_{1-4}$ alkyl, $C_{1-4}$ alkoxy, $C_{3-6}$ cycloalkyl, or 3- to 7-membered heterocycloalkyl, and the heterocycloalkyl contains 1 to 3 heteroatoms selected from O, S or N;
K is selected from

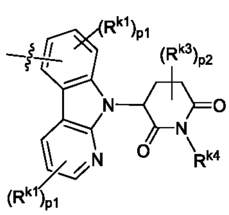 , 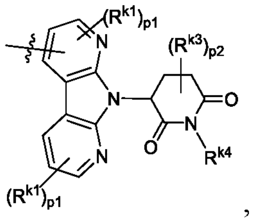 , 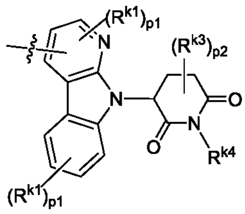 ,

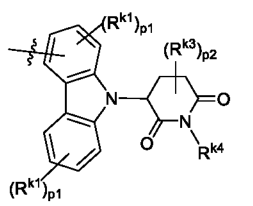 , 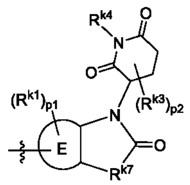 , 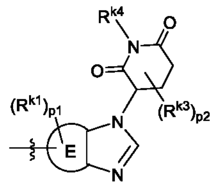 , 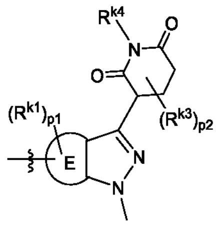 ,

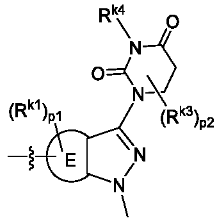 , 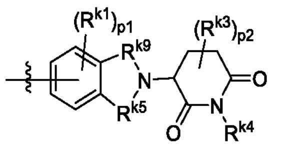 , 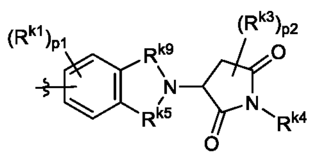 ,

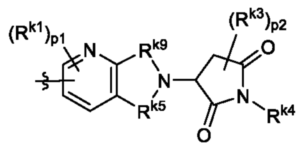 , 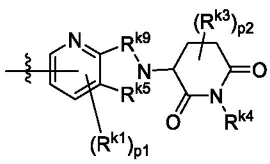 , 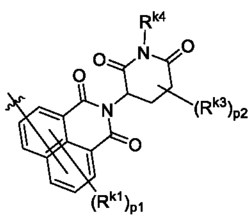 ,

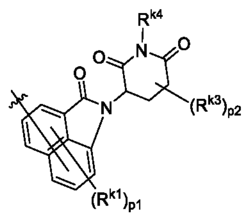 , 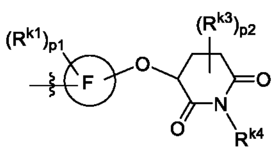 , 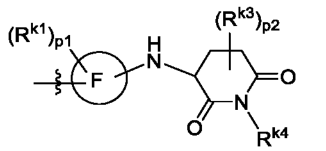 ,

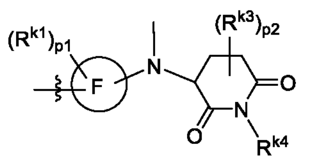 , 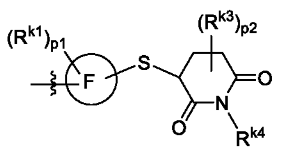 , 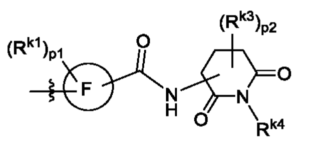 ,

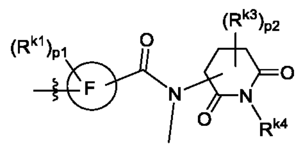 , 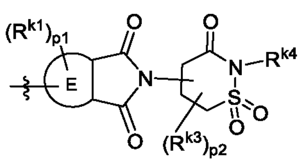 , 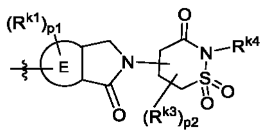 ,

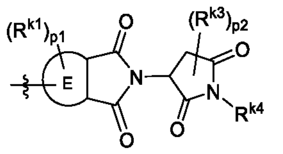 , 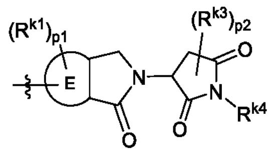 , 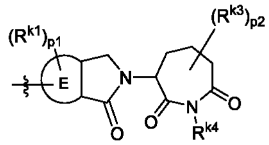 ,

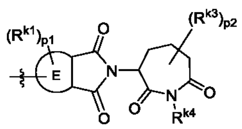 , 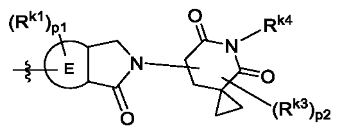 , 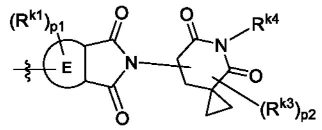 ,

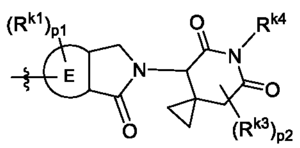 , 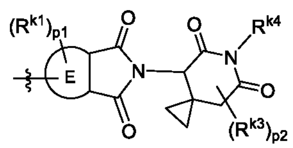 , 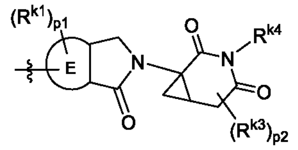 ,

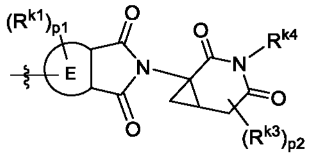 , 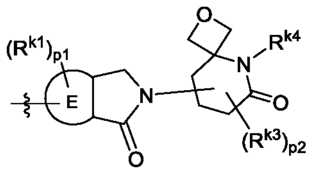 , 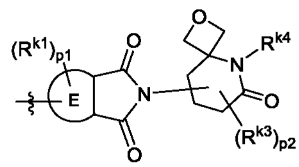 ,

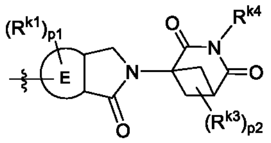 , 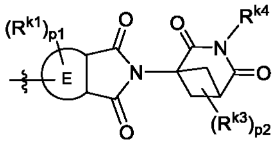 , 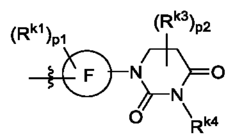 , 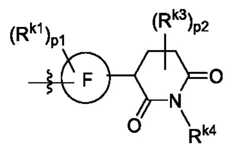 ,

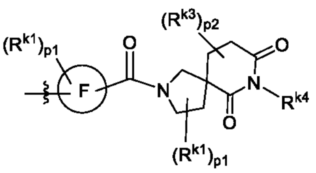 , 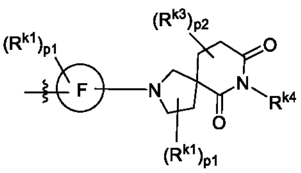 , 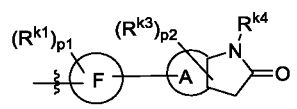 ,

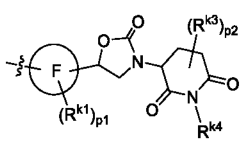 , 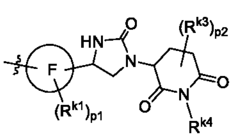 , 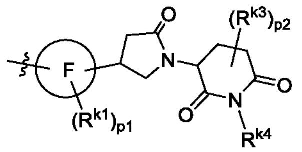 ,

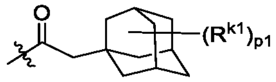 , 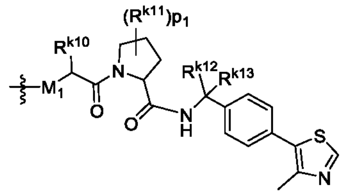 , 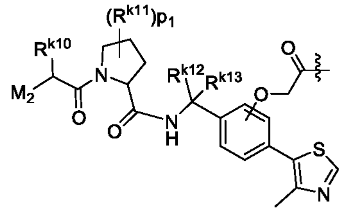 ,

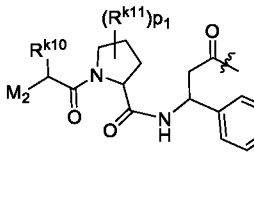, 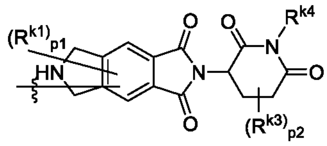, 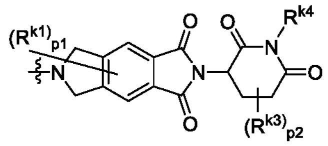,

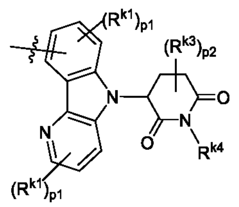 or 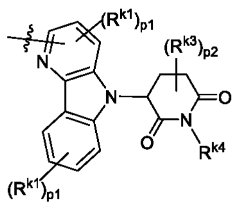;

M1 is selected from a bond, -C(=O)NH-, $-CH_2-C(=O)NH-$, $-C(=O)CH_2NH-$, pyrrolyl, pyrazolyl, imidazolyl, triazolyl, oxazolyl, isoxazolyl, furyl, thienyl, or thiazolyl;

$M_2$ is selected from $-NHC(=O)-CH_3$, -NHC(=O)-cyclopropyl, -NHC(=O)-cyclobutyl, azetidinyl, azacyclopentyl, benzo-azacyclopentyl or benzo-azacyclohexyl, wherein the cyclopropyl, cyclobutyl, azetidinyl, azacyclopentyl, benzo-azacyclopentyl or benzo-azacyclohexyl is optionally further substituted with 0 to 4 substituents selected from H, F, Cl, Br, I, =O, OH, $NH_2$, $C_{1-4}$ alkyl or $C_{1-4}$ alkoxy;

$R^{k10}$ is selected from methyl, ethyl, isopropyl, propyl or tert-butyl, wherein the methyl, ethyl, isopropyl, propyl or tert-butyl is optionally further substituted with 0 to 4 substituents selected from H, F, Cl, Br, I, =O, OH, $C_{1-4}$ alkyl or $C_{3-6}$ cycloalkyl;

each $R^{k11}$ is independently selected from H, F, Cl, Br, I, =O, OH, SH, methyl, ethyl, isopropyl, propyl, methoxy, ethoxy, propoxy, isopropyloxy, methylthio, ethylthio, propylthio or $-O-C(=O)-CH_3$, wherein the methyl, ethyl, isopropyl, propyl, methoxy, ethoxy, propoxy, isopropyloxy, methylthio, ethylthio, or propylthio is optionally further substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, methyl, ethyl, isopropyl, propyl, cyclopropyl or cyclobutyl, wherein the methyl, ethyl, isopropyl, propyl, cyclopropyl or cyclobutyl is optionally further substituted with 0 to 4 substituents selected from H, F, Cl, Br, I, =O, OH, $NH_2$, $C_{1-4}$ alkyl or $C_{1-4}$ alkoxy;

each E (ring E) is independently selected from phenyl, pyridyl, pyridazinyl, pyrazinyl, pyrimidyl, pyrrolyl, pyrazolyl, imidazolyl, thiazolyl, furyl, thienyl or oxazolyl;

each A is independently selected from phenyl, pyridyl, pyridazinyl, pyrazinyl, pyrimidyl, pyrrolyl, pyrazolyl, imidazolyl, thiazolyl, furyl, thienyl or oxazolyl;

each F is independently selected from cyclopropyl, cyclobutyl, cyclopentyl, cyclohexyl, bicyclo[1.1.1]pentanyl, 6,7-dihydro-5H-cyclopenta[c]pyridyl, 2,3-dihydro-1H-indenyl, phenyl, naphthyl, anthryl, phenanthryl, azetidinyl, azacyclopentyl, piperidyl, morpholinyl, pyridyl, pyrimidyl, pyridazinyl, pyrazinyl, triazinyl, pyrrolyl, pyrazolyl, imidazolyl, triazolyl, oxazolyl, furyl, thienyl, thiazolyl, benzoimidazolyl, benzopyrazolyl, benzothiazolyl, benzothienyl, benzofuryl, benzopyrrolyl, benzopyridyl, benzopyrazinyl, benzopyrimidyl, benzopyridazinyl, pyrrolopyrrolyl, pyrrolopyridyl, pyrrolopyrimidyl, pyrrolopyridazinyl, pyrrolopyrazinyl, imidazopyrimidyl, imidazopyridyl, imidazopyrazinyl, imidazopyridazinyl, pyrazolopyridyl, pyrazolopyrimidyl, pyrazolopyridazinyl, pyrazolopyrazinyl, pyrimidopyridyl, pyrimidopyrazinyl, pyrimidopyridazinyl, pyrimidopyrimidyl, pyridopyridyl, pyridopyrazinyl, pyridopyridazinyl, pyridazinopyridazinyl, pyridazinopyrazinyl or pyrazinopyrazinyl;

each $R^{k7}$ is independently selected from $CH_2$, O, $N(CH_3)$ or NH;

each p1 or p2 is independently selected from 0, 1 or 2;

the definitions of other groups are the same as those in any one of the first, second and third embodiments of the present invention.

As a fifth embodiment of the present invention, provided is the above-mentioned compound of general formula (I) or the stereoisomer, deuterated compound, solvate, prodrug, metabolite, pharmaceutically acceptable salt or co-crystal thereof, wherein

Ak1, Ak2, Ak3, Ak4, Ak5, Ak6, Ak7, Ak8 and Ak9 are each independently selected from a bond, -O-, $-OCH_2-$, $-CH_2O-$, $-OCH_2CH_2-$, $-CH_2CH_2O-$, -C≡C-, $-C(CH_3)_2-$, $-CH_2-$, $-CH_2CH_2-$, $-CH_2CH_2CH_2-$, $-N(CH_3)-$, -NH-, $-CH_2N(CH_3)-$, $-CH_2NH-$, $-NHCH_2-$, $-CH_2CH_2N(CH_3)-$, $-CH_2CH_2NH-$, $-NHCH_2CH_2-$, -C(=O)-, $-C(=O)CH_2NH-$, $-CH_2C(=O)NH-$, -C(=O)NH- or -NHC(=O)-;

each Cy1, Cy2, Cy3, Cy4 or Cy5 is independently selected from a bond or one of the following substituted or unsubstituted groups

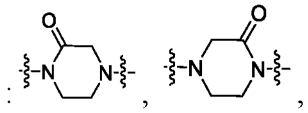

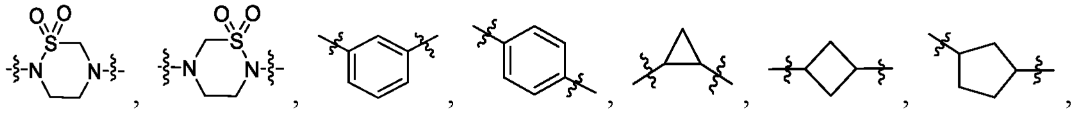

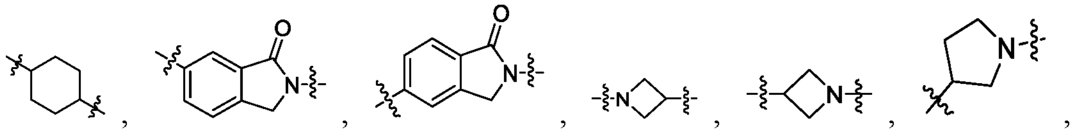

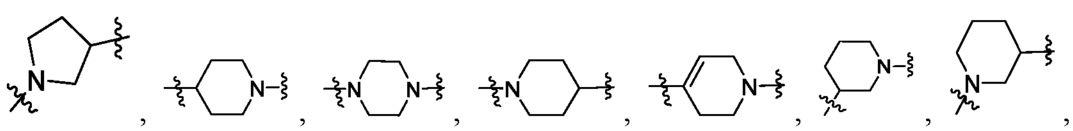

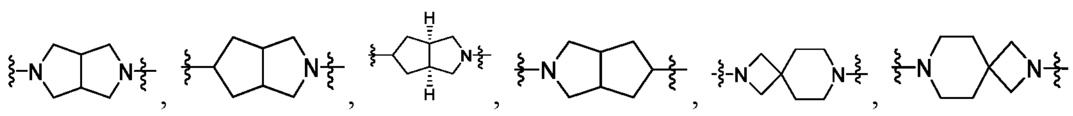

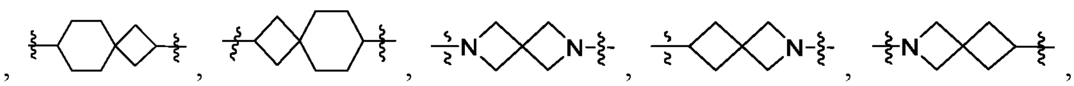

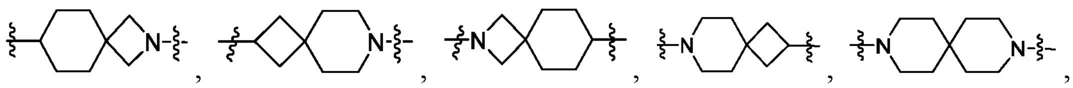

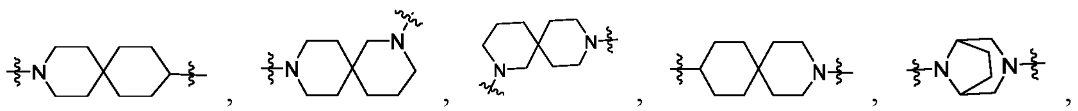

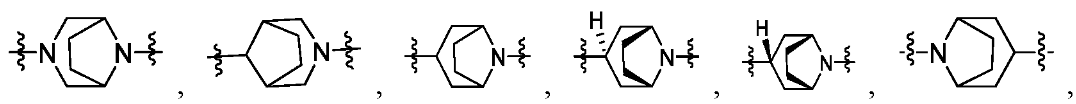

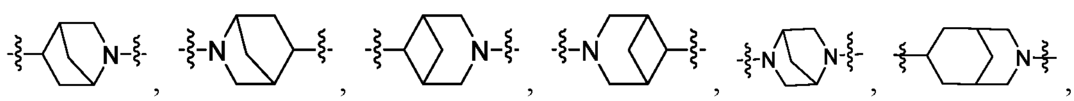

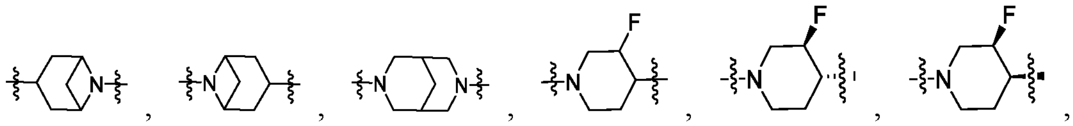

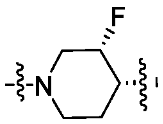, 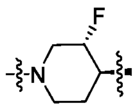, 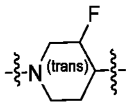, 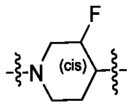, 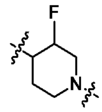, 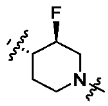, 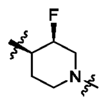,

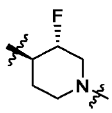, 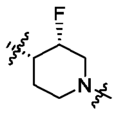, 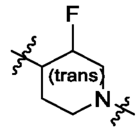 or 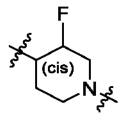,

which, when substituted, is optionally further substituted with 0 to 4 substituents selected from H, F, $CF_3$, methyl, =O, hydroxymethyl, COOH, CN or $NH_2$;

B is selected from one of the following structural fragments (Table B-a),

| | |
|---|---|
| 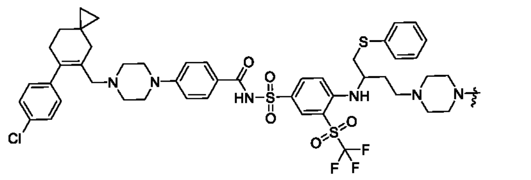 | 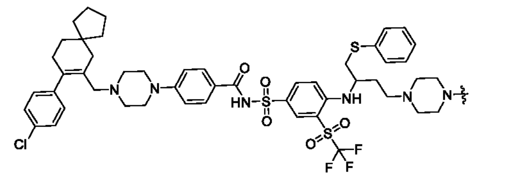 |
| 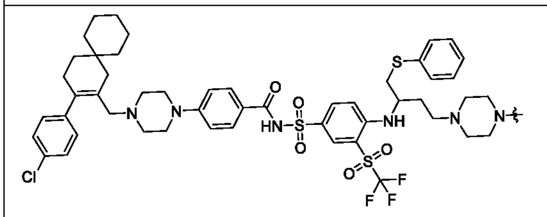 | 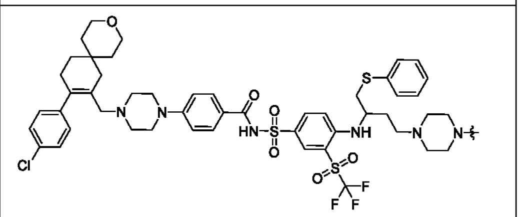 |
| 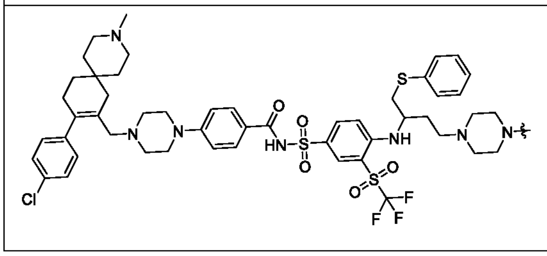 | 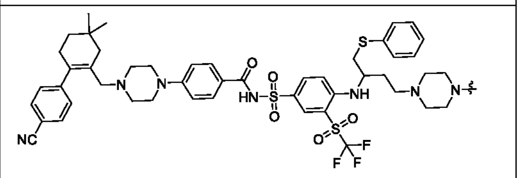 |

| | |
|---|---|
| 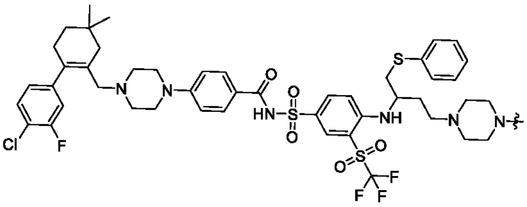 | 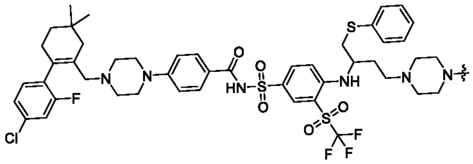 |
| 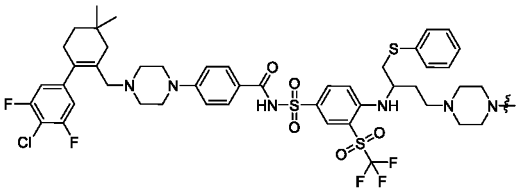 | 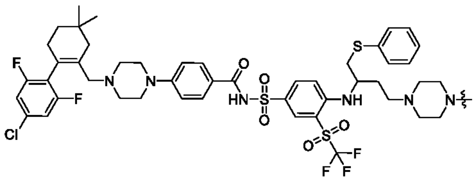 |
| 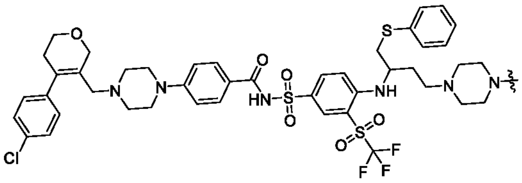 | 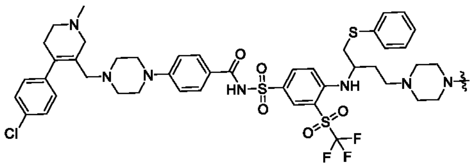 |
| 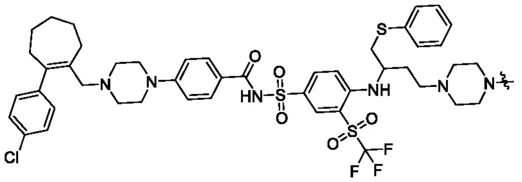 | 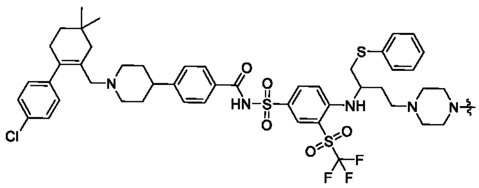 |
| 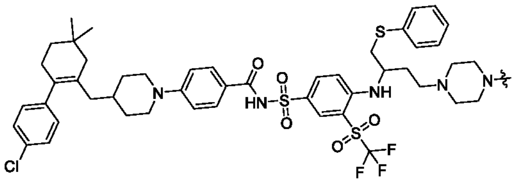 | 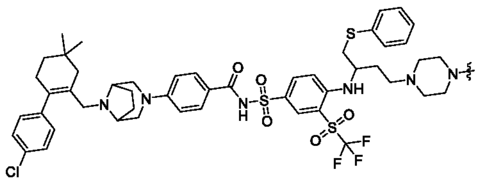 |
| 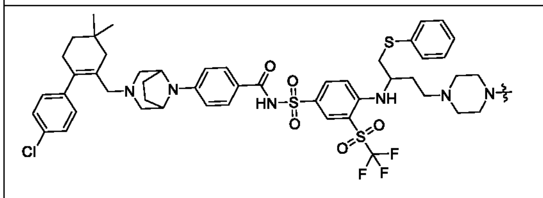 | 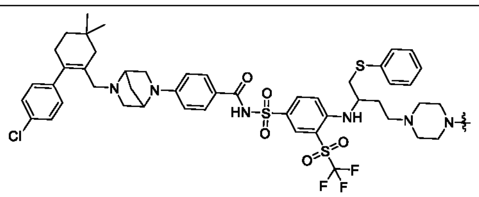 |
| 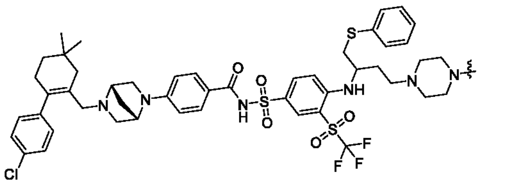 | 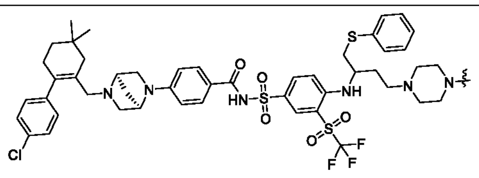 |
| 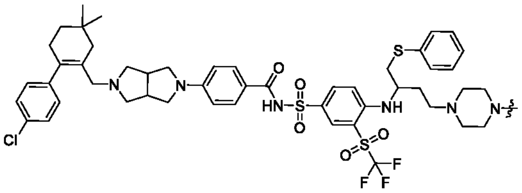 | 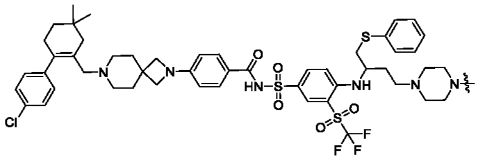 |

| | |
|---|---|
| 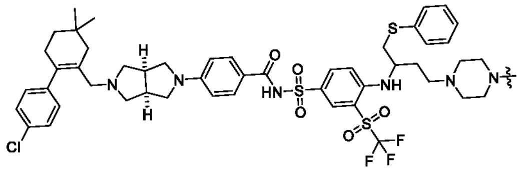 | 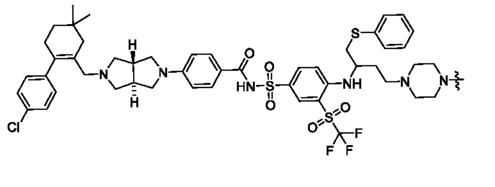 |
| 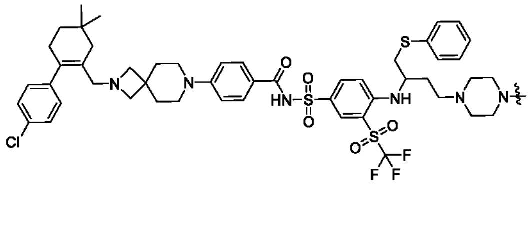 | 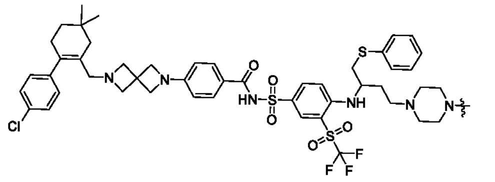 |
| 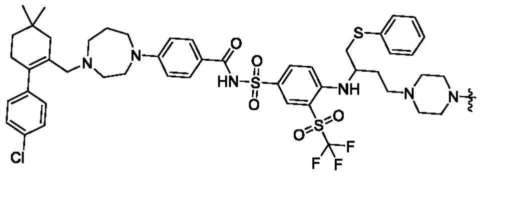 | 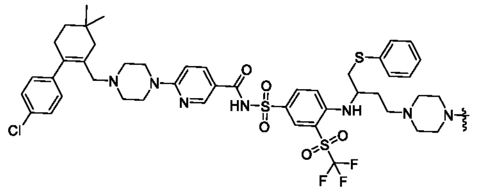 |
| 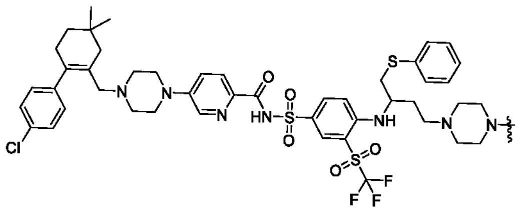 | 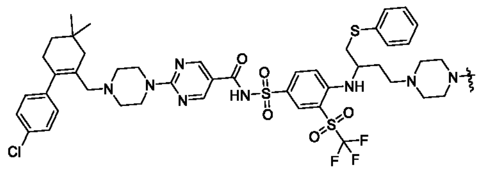 |
| 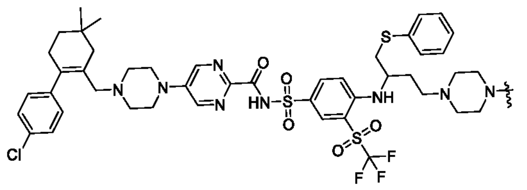 | 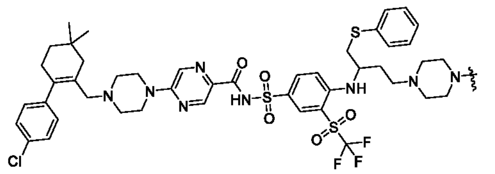 |
| 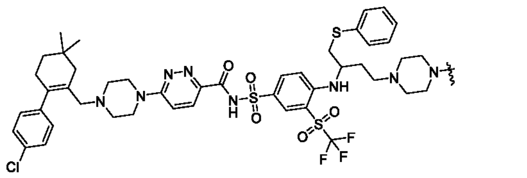 | 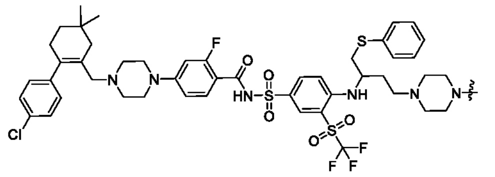 |
| 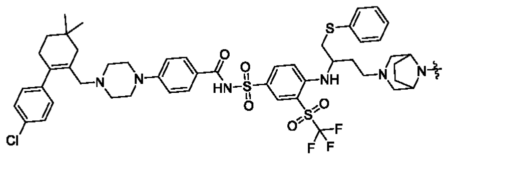 | 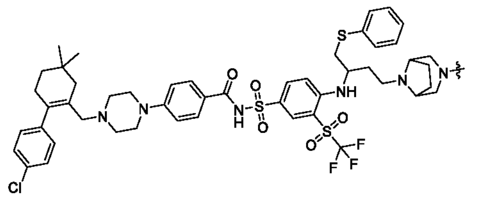 |
| 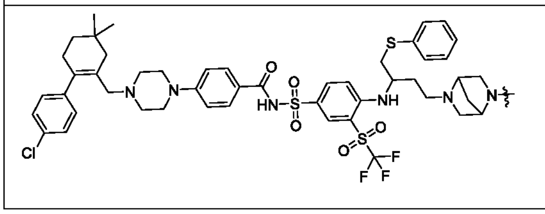 | 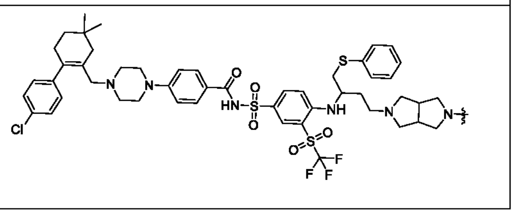 |

| | |
|---|---|
| 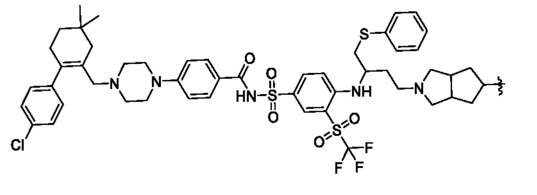 | 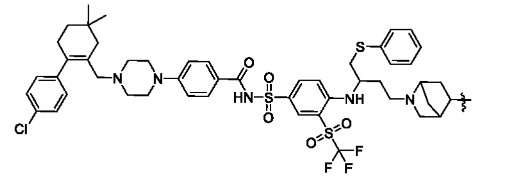 |
| 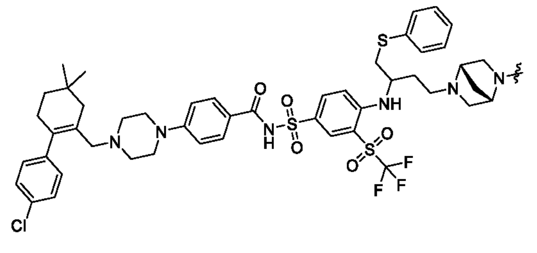 | 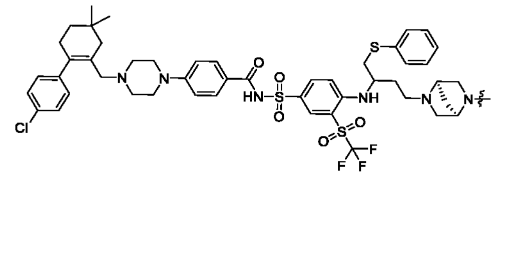 |
| 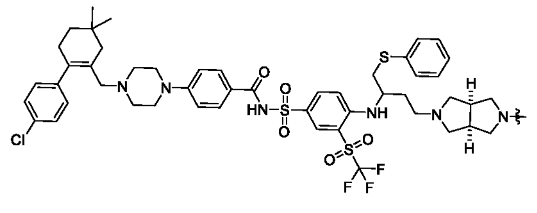 | 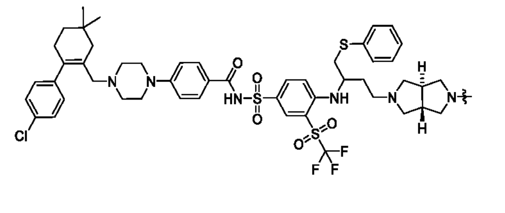 |
| 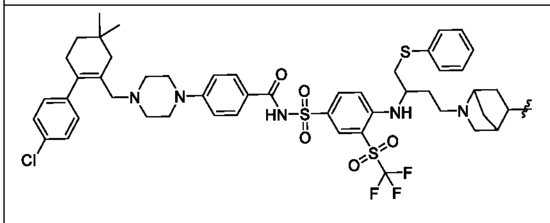 | 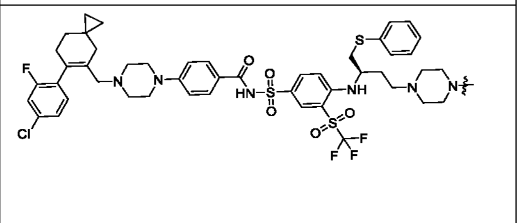 |
| 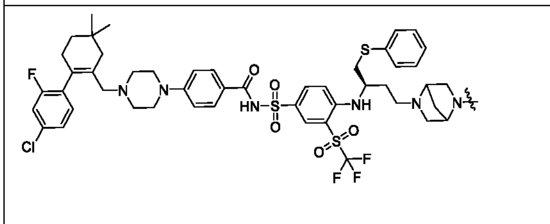 | 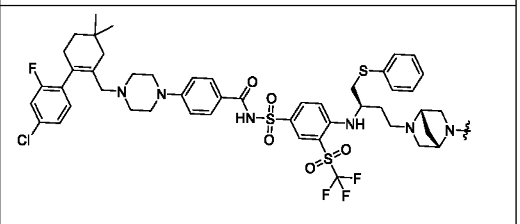 |
| 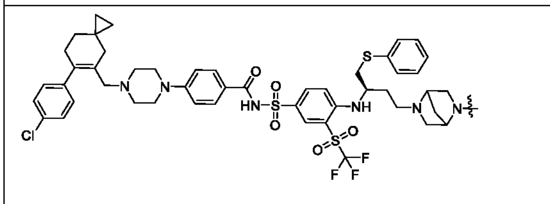 | 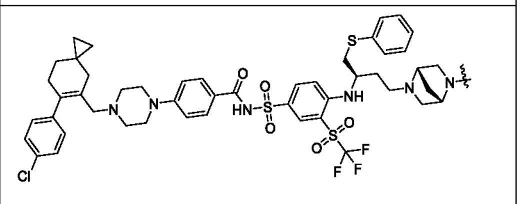 |
| 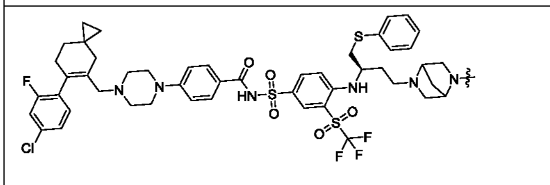 | 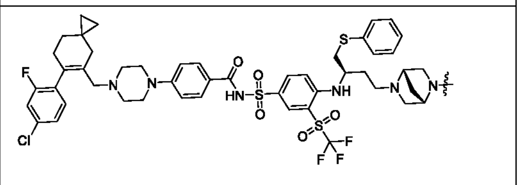 |
| 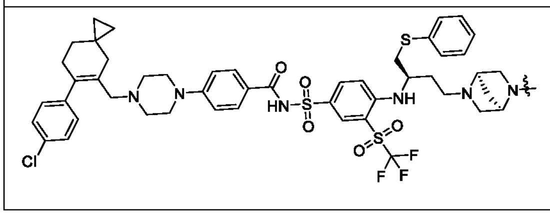 | 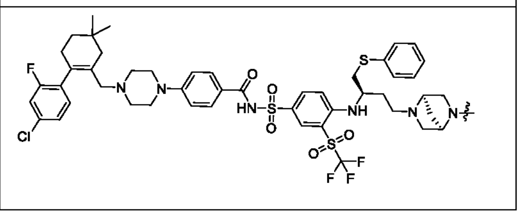 |

| | |
|---|---|
| 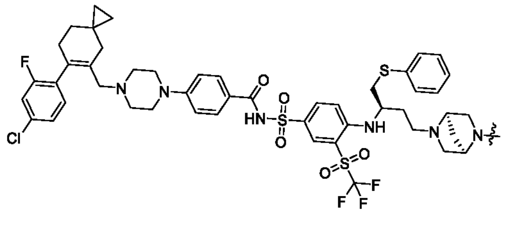 | 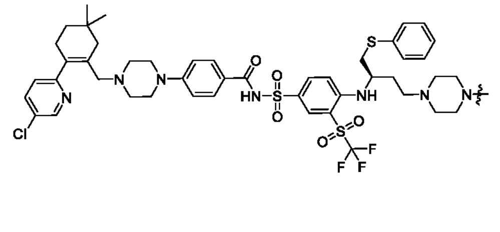 |
| 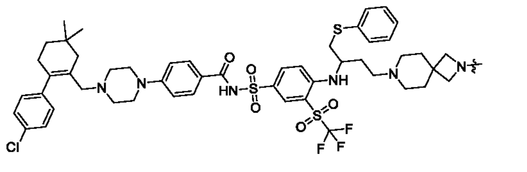 | 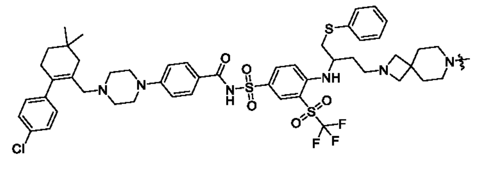 |
| 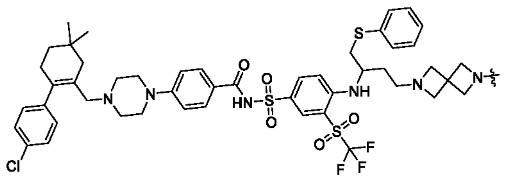 | 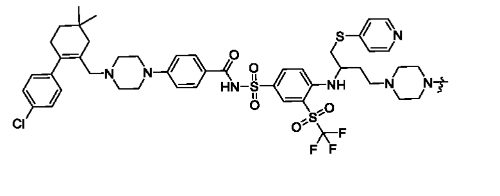 |
| 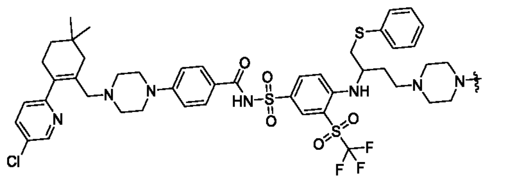 | 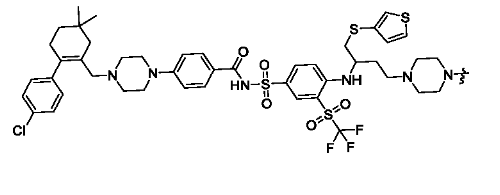 |
| 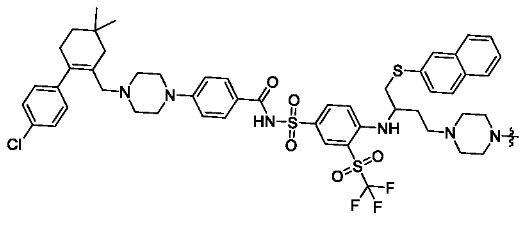 | 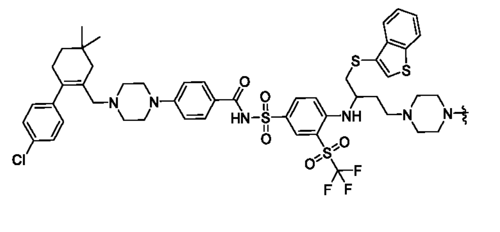 |
| 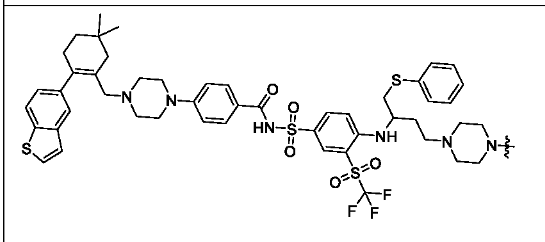 | 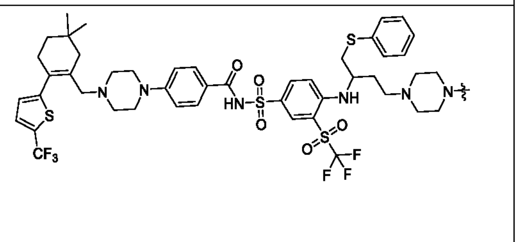 |
| 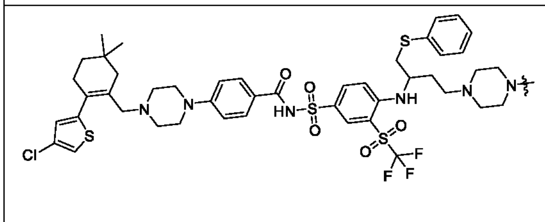 | 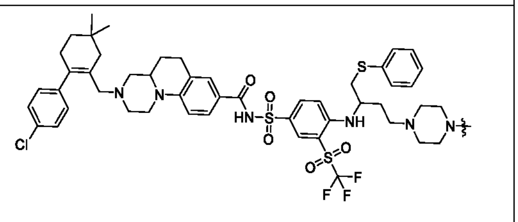 |
| 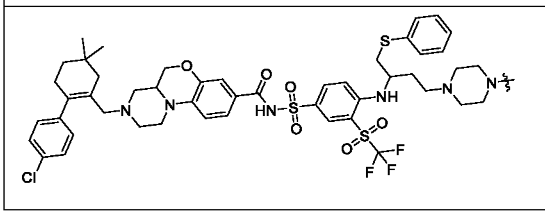 | 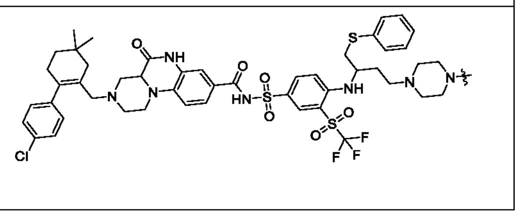 |

| | |
|---|---|
| 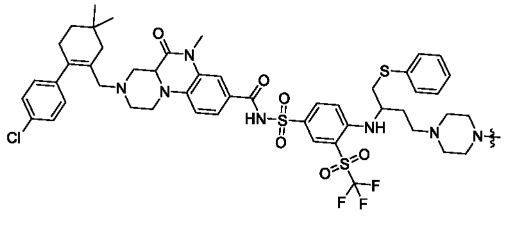 | 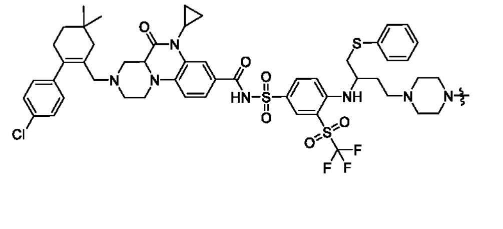 |
| 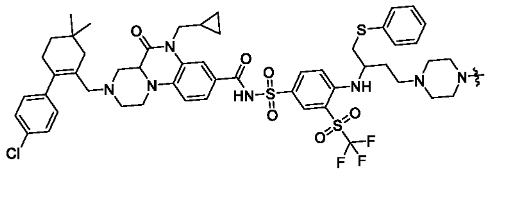 | 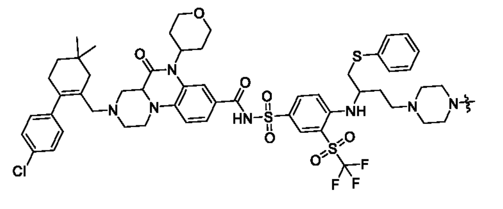 |
| 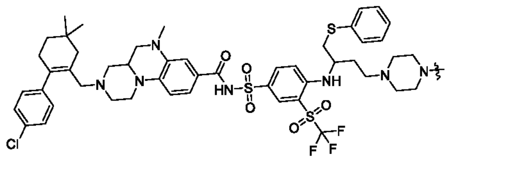 | 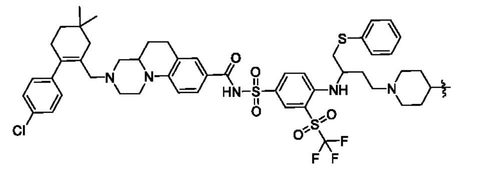 |
| 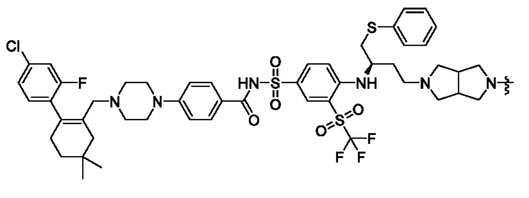 | 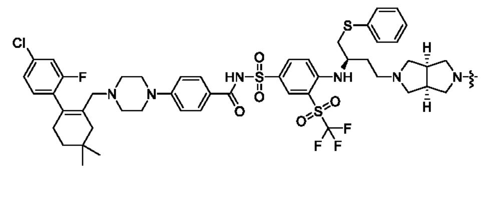 |
| 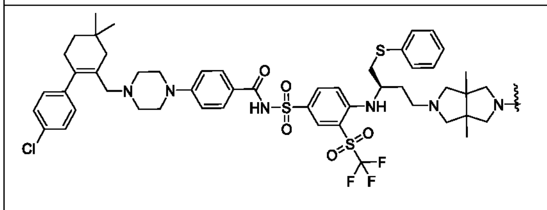 | 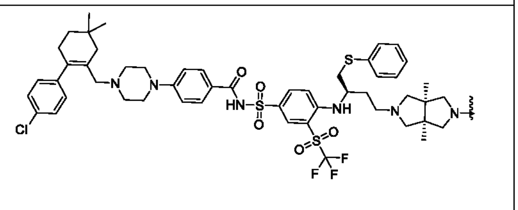 |
| 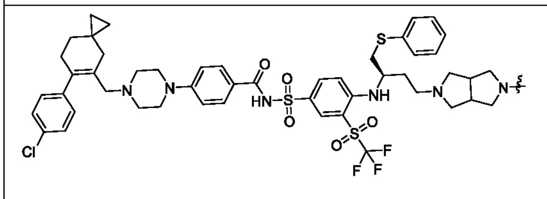 | 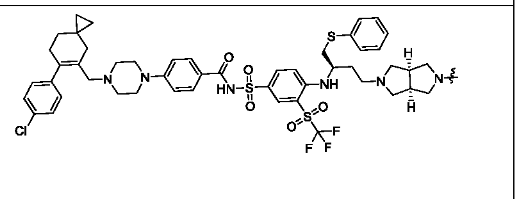 |
| 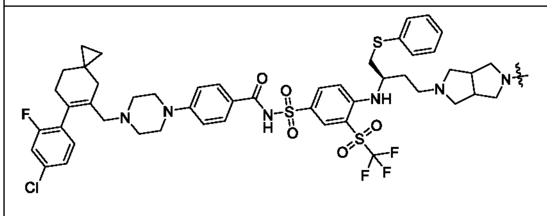 | 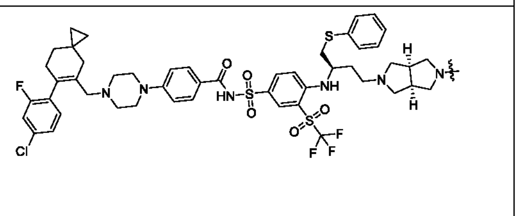 |
| 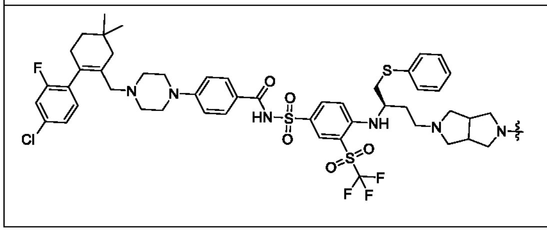 | 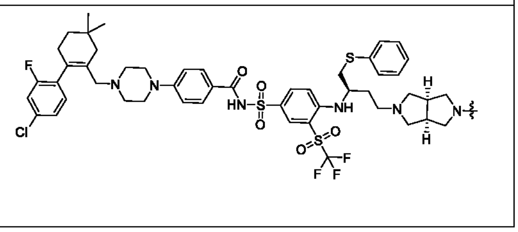 |

| | |
|---|---|
| 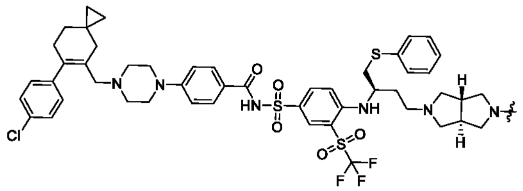 | 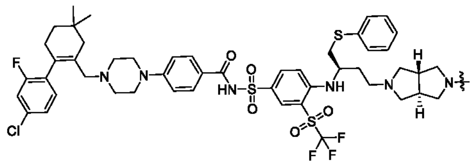 |
| 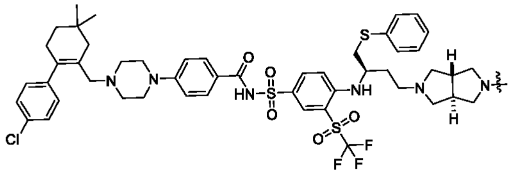 | 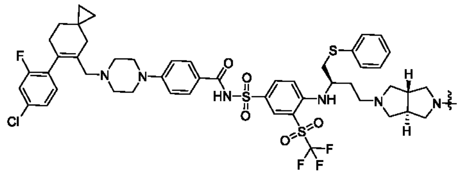 |
| 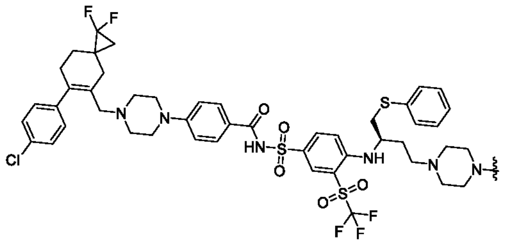 | 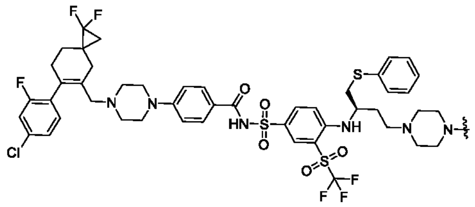 |
| 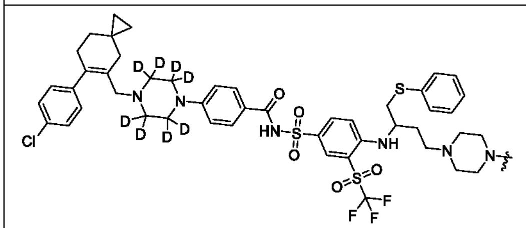 | 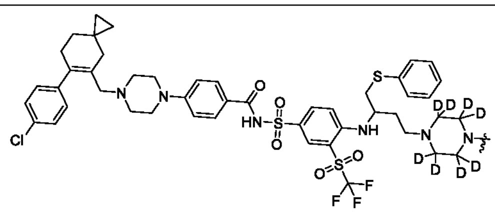 |
| 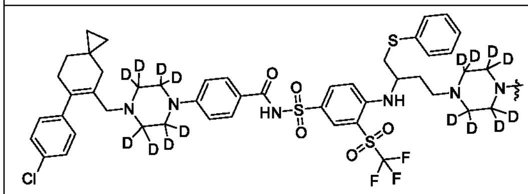 | 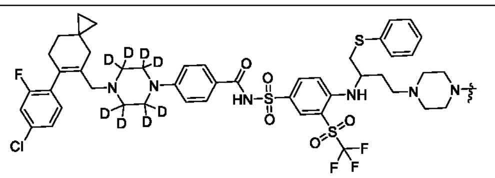 |
| 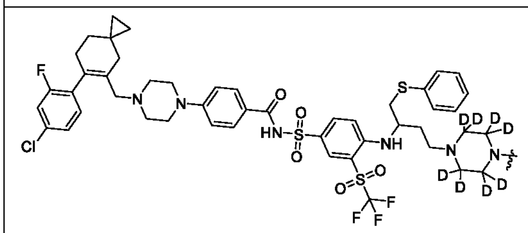 | 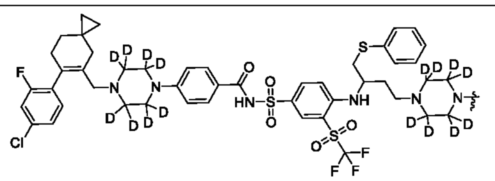 |
| 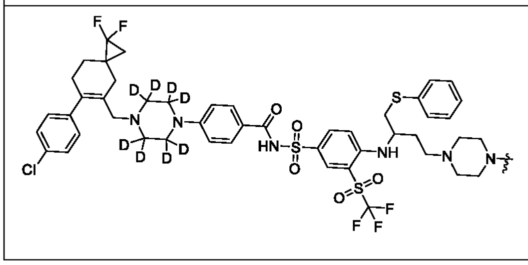 | 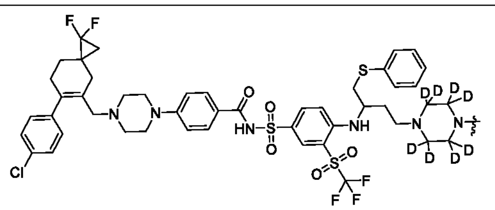 |

| | |
|---|---|
| 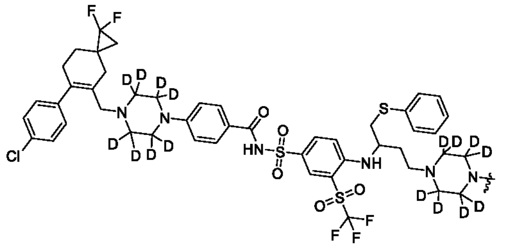 | 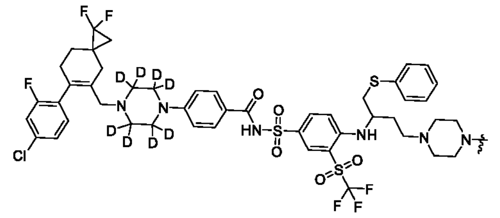 |
| 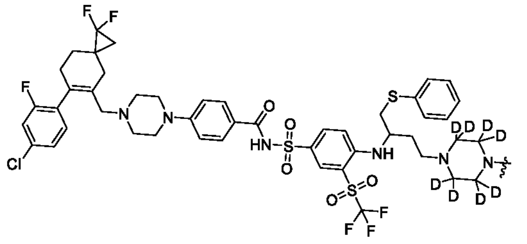 | 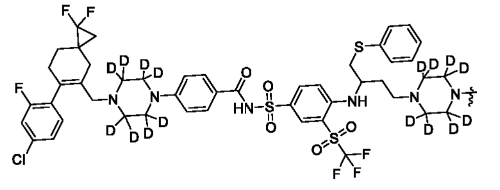 |
| 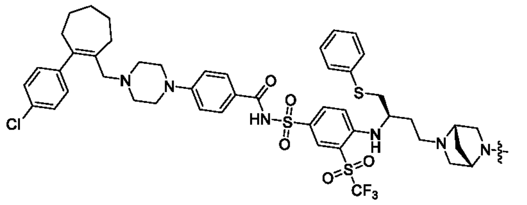 | 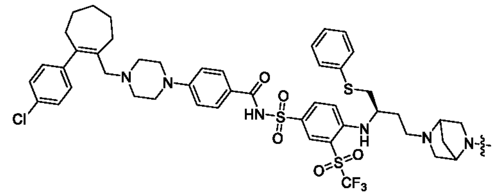 |
| 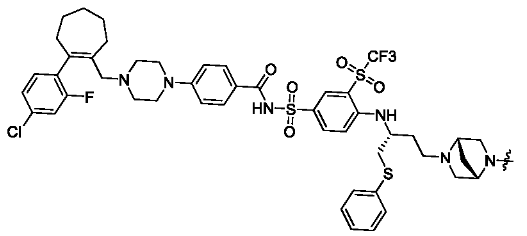 | 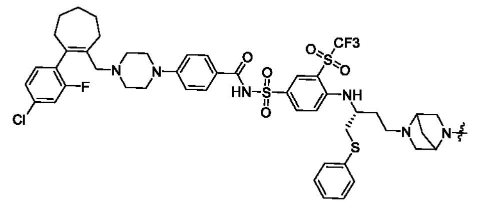 |
| 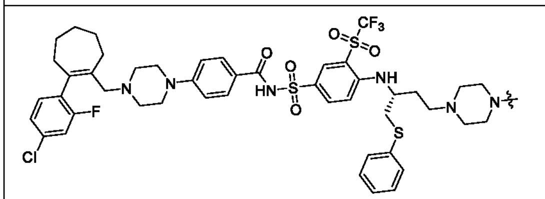 | 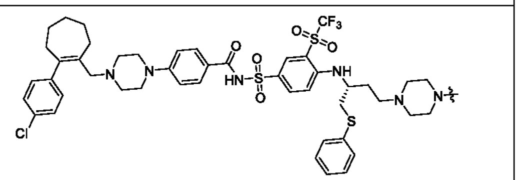 |
| 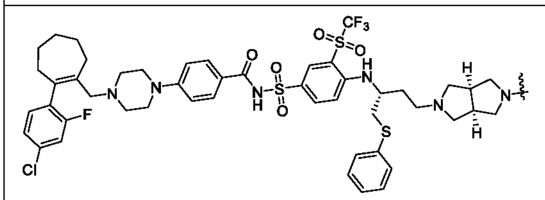 | 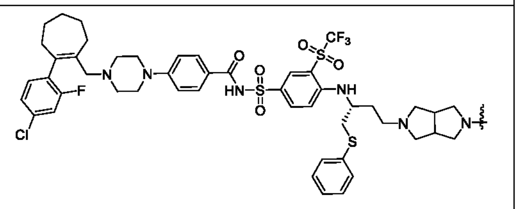 |
| 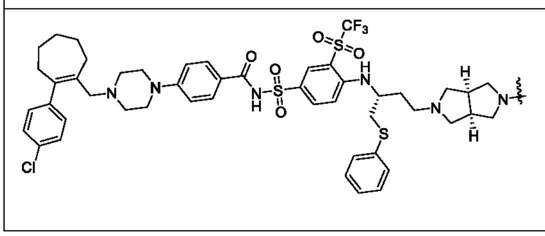 | 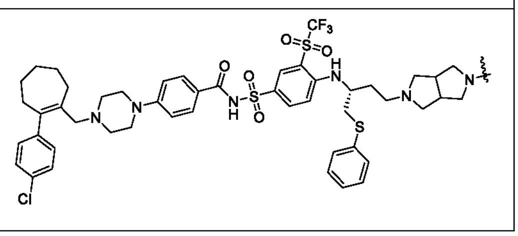 |

| | |
|---|---|
| 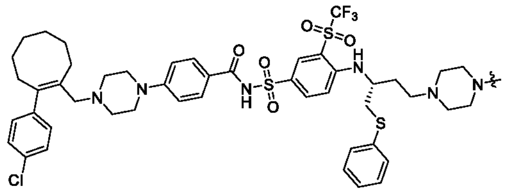 | |

K is selected from one of the following structural fragments (Table K-a):

| | | | |
|---|---|---|---|
| 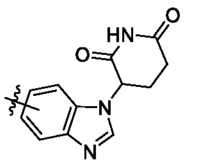 | 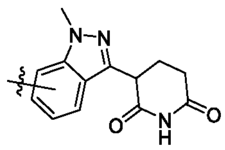 | 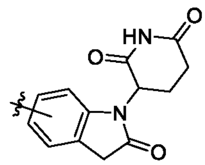 | 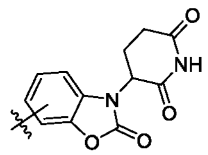 |
| 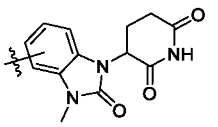 | 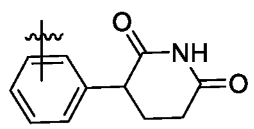 | 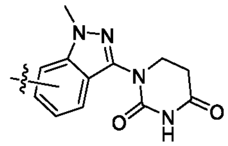 | |
| 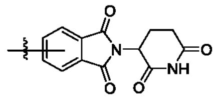 | 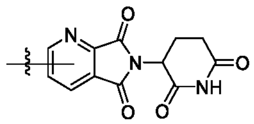 | 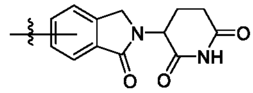 | 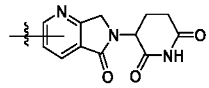 |
| 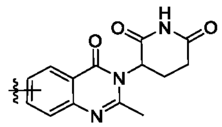 | 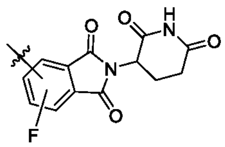 | 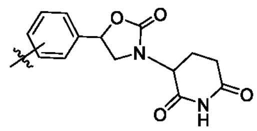 | 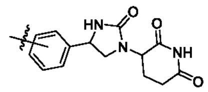 |
| 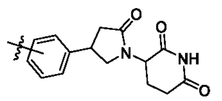 | 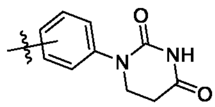 | 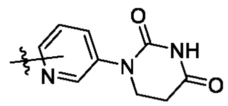 | 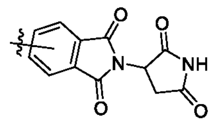 |
| 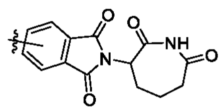 | 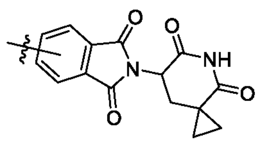 | 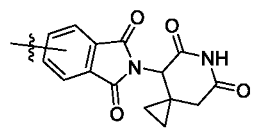 | 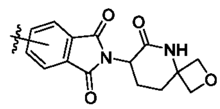 |
| 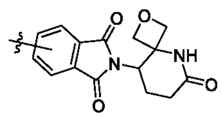 | 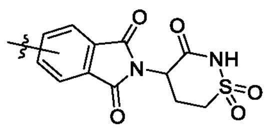 | 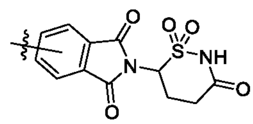 | 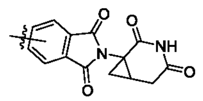 |
| 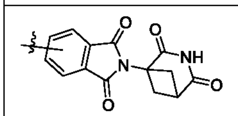 | 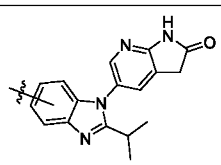 | 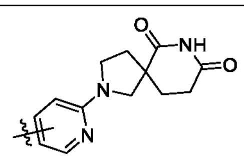 | 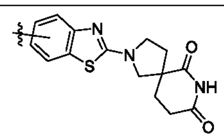 |

| | | | |
|---|---|---|---|
| 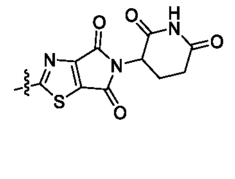 | 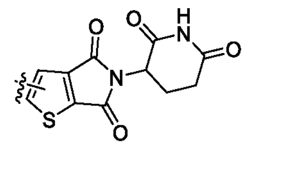 | 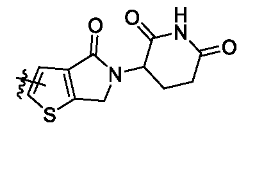 | 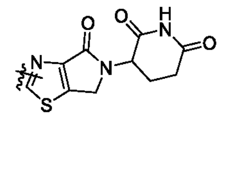 |
| 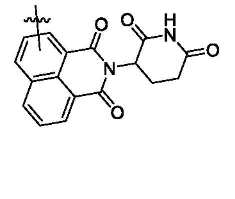 | 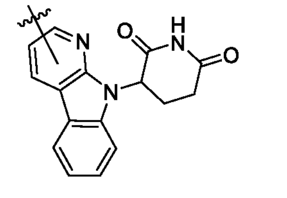 | 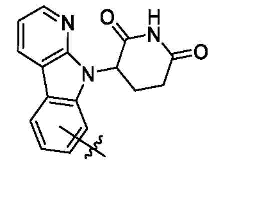 | 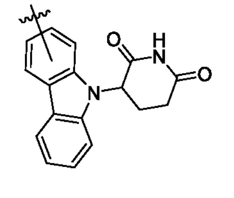 |
| 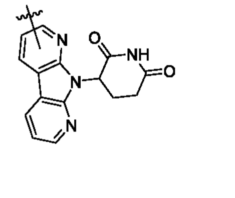 | 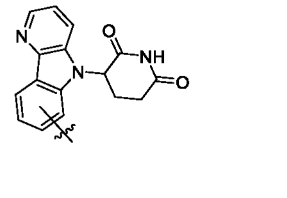 | 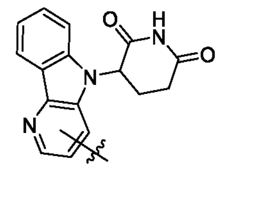 | 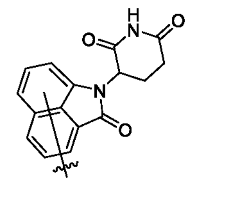 |
| 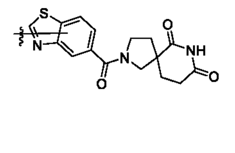 | 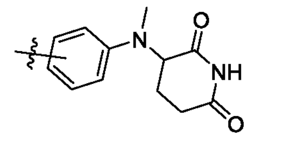 | 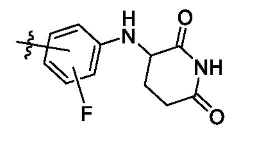 | |
| 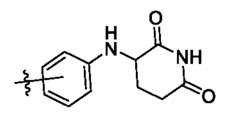 | 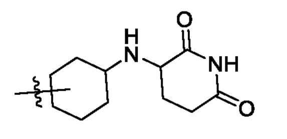 | 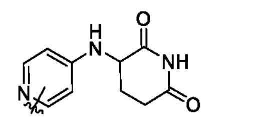 | 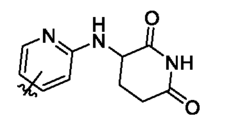 |
| 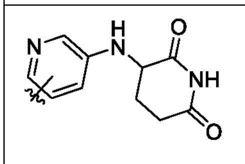 | 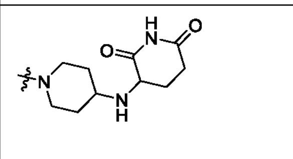 | 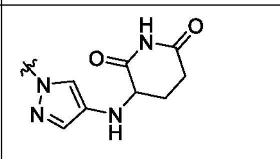 | 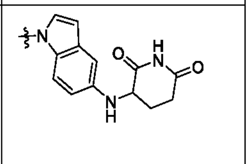 |
| 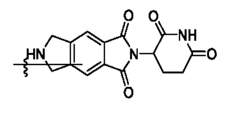 | 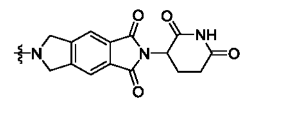 | | |
| 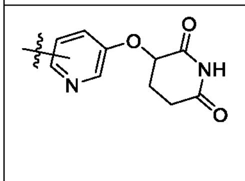 | 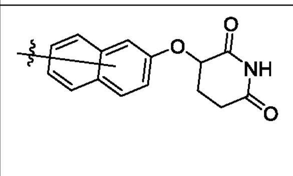 | 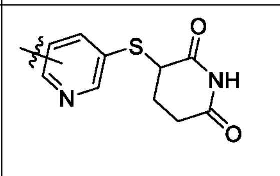 | 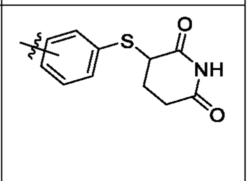 |
| 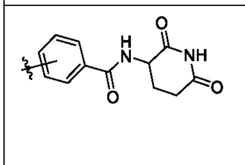 | 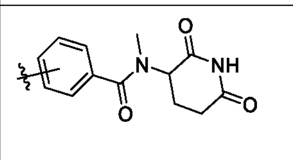 | 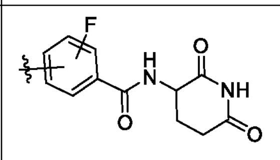 | 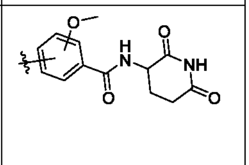 |
| 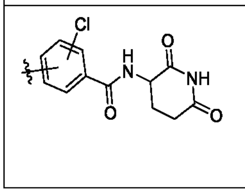 | 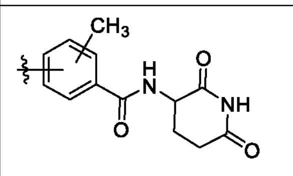 | 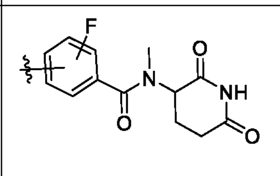 | 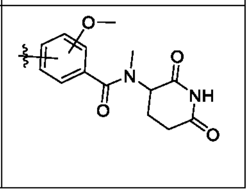 |

| | | | |
|---|---|---|---|
| 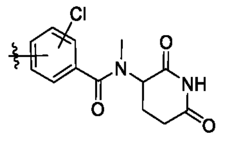 | 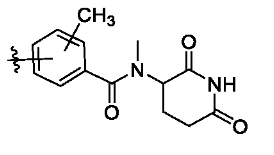 | 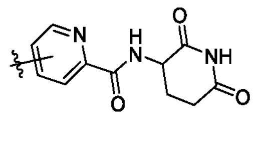 | 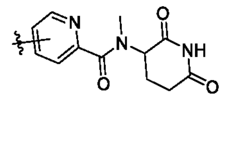 |
| 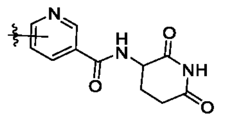 | 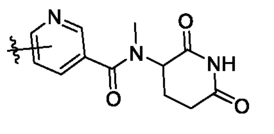 | 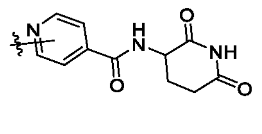 | 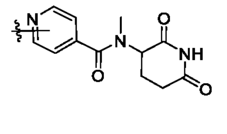 |
| 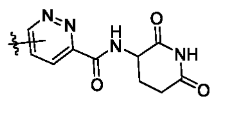 | 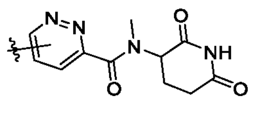 | 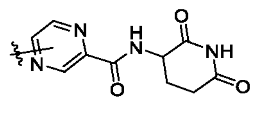 | 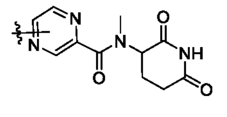 |
| 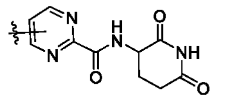 | 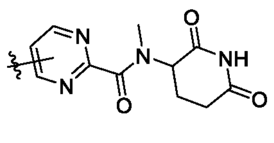 | 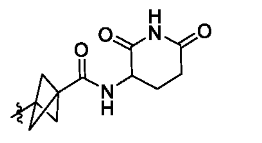 | 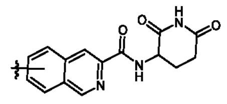 |
| 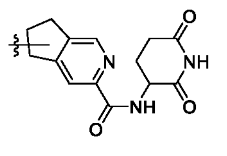 | 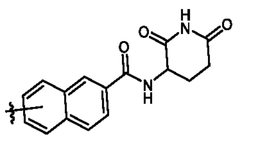 | 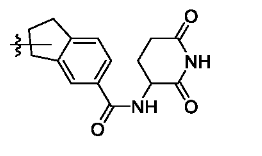 | 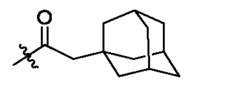 |

| | | |
|---|---|---|
| 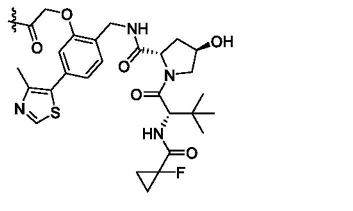 | 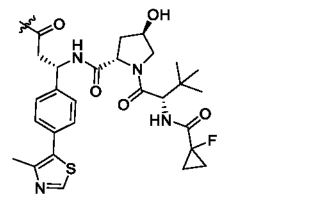 | 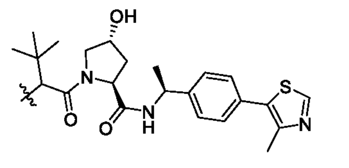 |
| 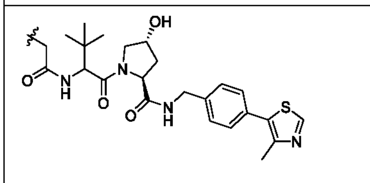 | 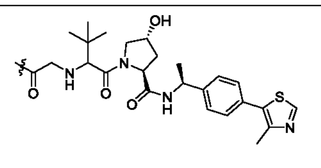 | 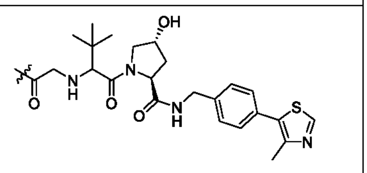 |
| 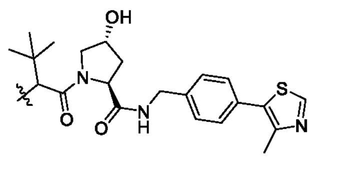 | 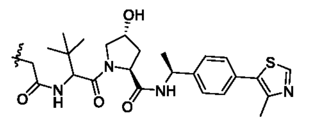 | 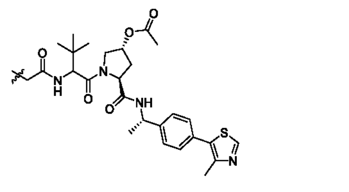 |
| 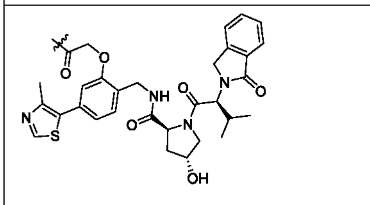 | 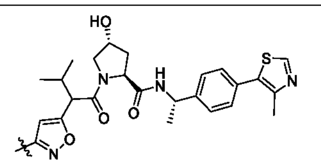 | 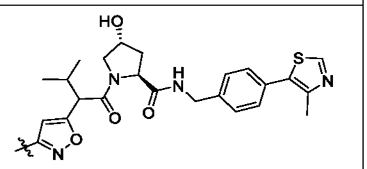 |
| 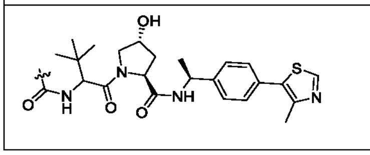 | 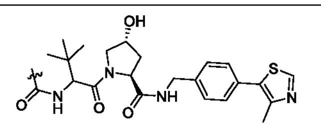 | 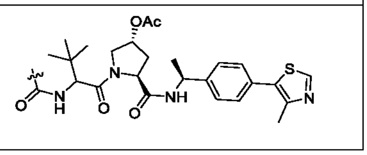 |

| | | |
|---|---|---|
| 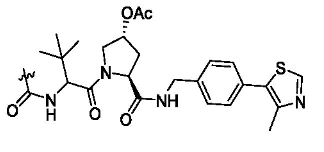 | | |

;

the definitions of other groups are the same as those in any one of the first, second, third and fourth embodiment of the present invention.

As a sixth embodiment of the present invention, provided is the above-mentioned compound of general formula (I) or the stereoisomer, deuterated compound, solvate, prodrug, metabolite, pharmaceutically acceptable salt or co-crystal thereof, wherein

L is selected from a bond, -Ak1-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Cy1-, - Cy1-Ak1-, -Cy1-Ak1-Ak2-, -Cy1-Ak1-Ak2-Ak3-, -Cy1-Ak1-Ak2-Ak3-Ak4-, - Cy1-Cy2-, -Cy1-Ak1-Cy2-, -Cy1-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Ak2-Cy3-, -Cy1-Ak1-Ak2-Cy3-Ak3-, -Cy1-Cy2-Cy3-, -Cy1-Ak1-Cy2-Cy3-, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cy1-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cy1-Ak1-Cy2-Ak2-Cy3-Cy4-, -Ak1-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Cy1-Cy2-Cy3-Ak3-Ak4-Ak5-, -Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-Ak2-Ak3-Ak4-, -Ak1-Cy2-Ak2-Ak3-, -Ak1-Ak2-Ak3-Ak4-Ak5 -, -Ak1-Ak2-Ak3-Ak4-Ak5-Ak6-, or -Ak1-Ak2-Ak3-Ak4-Ak5-Ak6-Ak7-;

the definitions of other groups are the same as those in any one of the first, second, third, fourth and fifth embodiments of the present invention.

As a seventh embodiment of the present invention, provided is the above-mentioned compound of general formula (I) or the stereoisomer, deuterated compound, solvate, prodrug, metabolite, pharmaceutically acceptable salt or co-crystal thereof, wherein

L is selected from a bond or a group shown in Table B-1, wherein the left side of the group is linked to B;

the definitions of other groups are the same as those in any one of the first, second, third, fourth, fifth and sixth embodiments of the present invention.

As an eighth embodiment of the present invention, provided is the above-mentioned compound of general formula (I) or the stereoisomer, deuterated compound, solvate, prodrug, metabolite, pharmaceutically acceptable salt or co-crystal thereof, wherein K is selected from one of the following structural fragments:

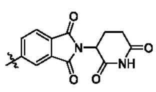 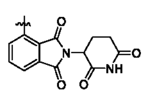 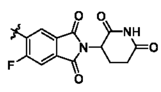 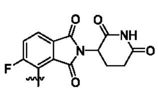 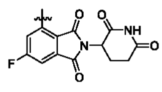 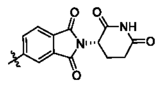

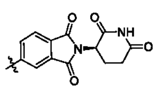 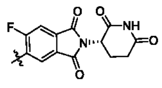 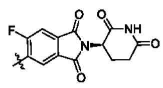 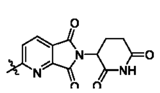 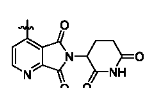 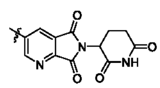

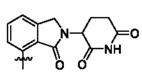 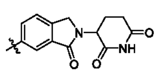 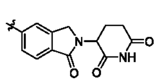 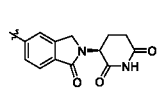 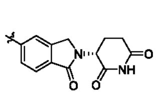 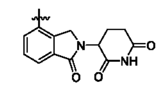

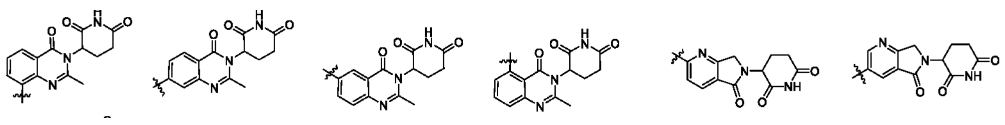

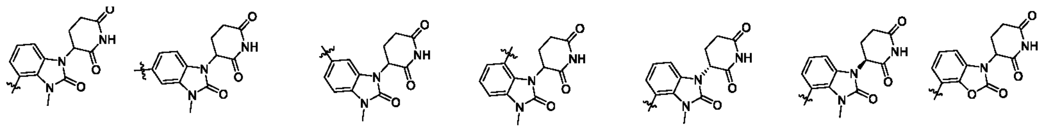

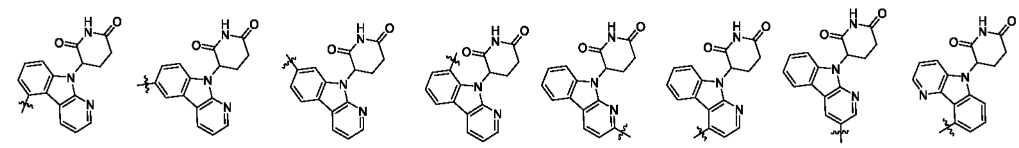

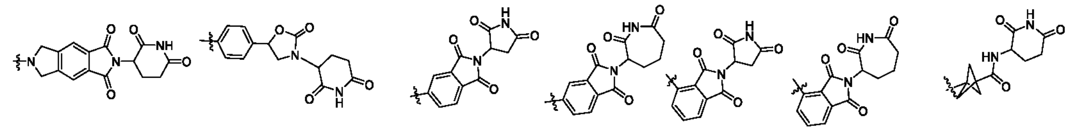

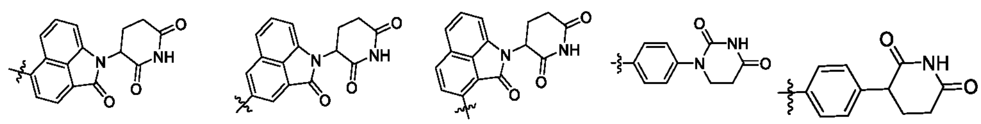

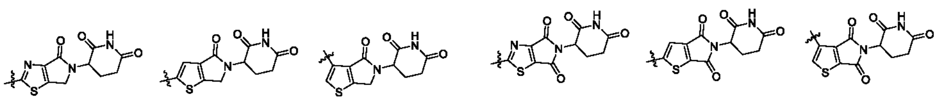

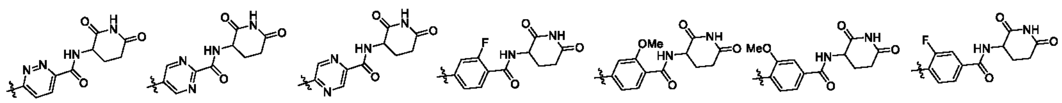

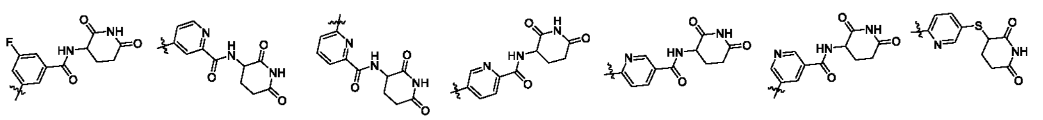

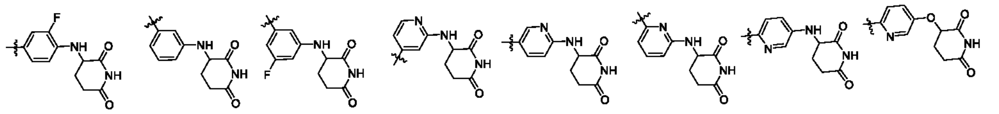

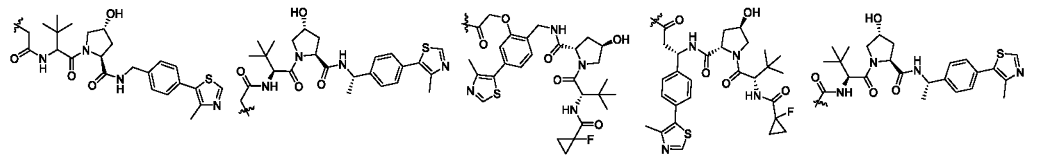

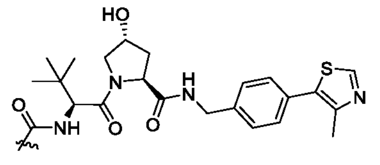
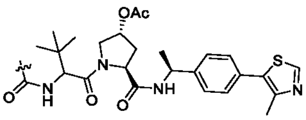
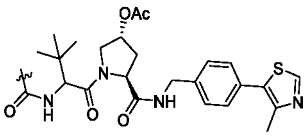
;

the definitions of other groups are the same as those in any one of the first, second, third, fourth, fifth, sixth and seventh embodiments of the present invention.

As a ninth embodiment of the present invention, provided is the above-mentioned compound of general formula (I) or the stereoisomer, deuterated compound, solvate, prodrug, metabolite, pharmaceutically acceptable salt or co-crystal thereof, wherein the compound of general formula (I) is selected from the compound of general formula (Ia)

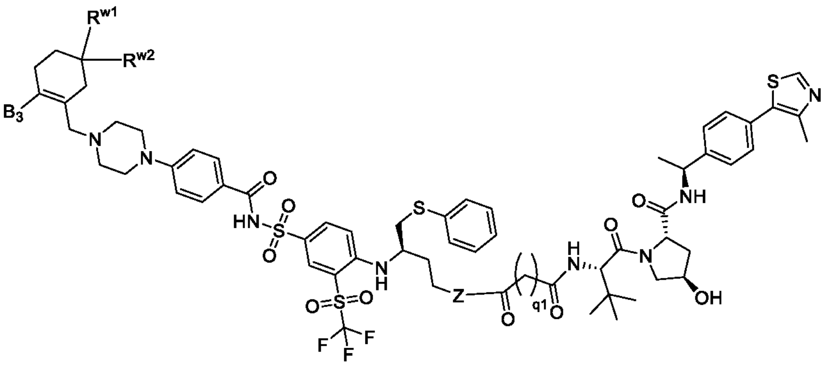
(Ia)

q1 is selected from 1, 2, 3, 4, 5, 6, 7 or 8;

$B_3$ is selected from

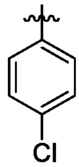
or
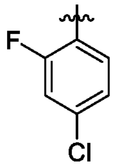
;

$R^{w1}$ and $R^{w2}$ are selected from methyl;

or $R^{w1}$ and $R^{w2}$ are directly connected to form, together with the carbon atom to which they are attached, cyclopropyl;

Z is selected from

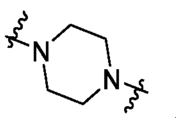
,
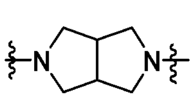
,
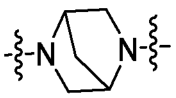
,

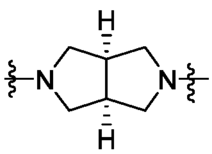
,
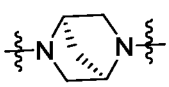
, or
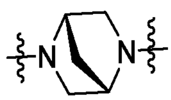
;

when Z is selected from

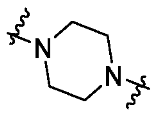,

and $R^{w1}$ and $R^{w2}$ are selected from methyl, $B_3$ is selected from

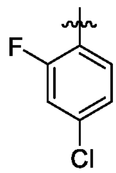.

As a tenth embodiment of the present invention, provided is the above-mentioned compound of general formula (I) or the stereoisomer, deuterated compound, solvate, prodrug, metabolite, pharmaceutically acceptable salt or co-crystal thereof, wherein the compound of general formula (I) is selected from the compound of general formula (Ib),

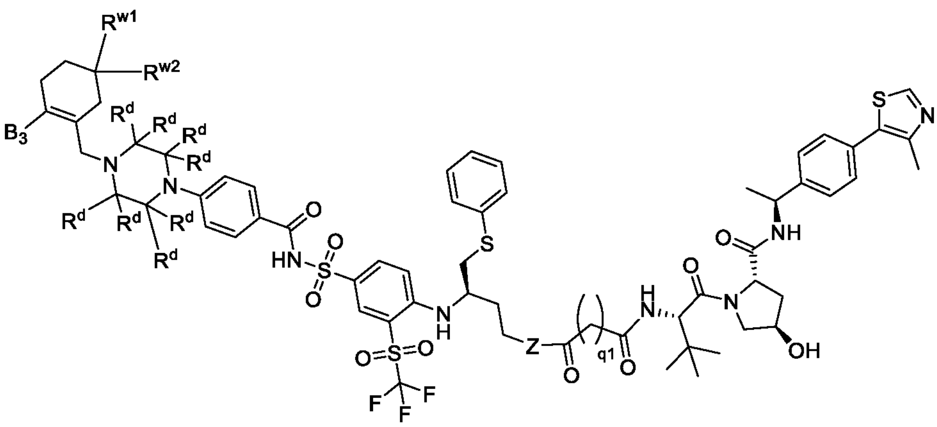

(Ib)

q1 is selected from 1, 2, 3, 4, 5, 6, 7 or 8;

$B_3$ is selected from

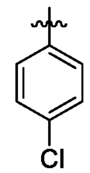 or 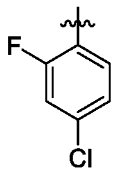;

$R^{w1}$ and $R^{w2}$ are selected from methyl;

or $R^{w1}$ and $R^{w2}$ are directly connected to form, together with the carbon atom to which they are attached, cyclopropyl;

Z is selected from

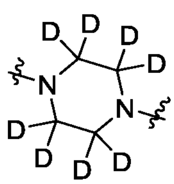, 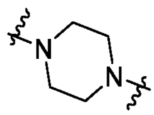, 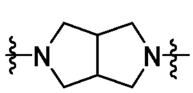, 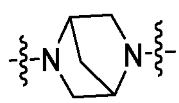,

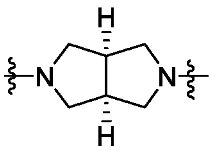, 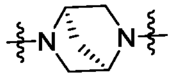, or 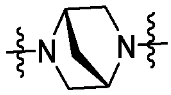;

each $R^d$ is independently selected from H or deuterium;

the compound of general formula (Ib) has at least one deuterium.

The present invention relates to a compound as described below or a stereoisomer, deuterated compound, solvate, prodrug, metabolite, pharmaceutically acceptable salt, or co-crystal thereof, wherein the compound is selected from one of the structures shown in Table P-1:

Table P-1

| |
|---|
| 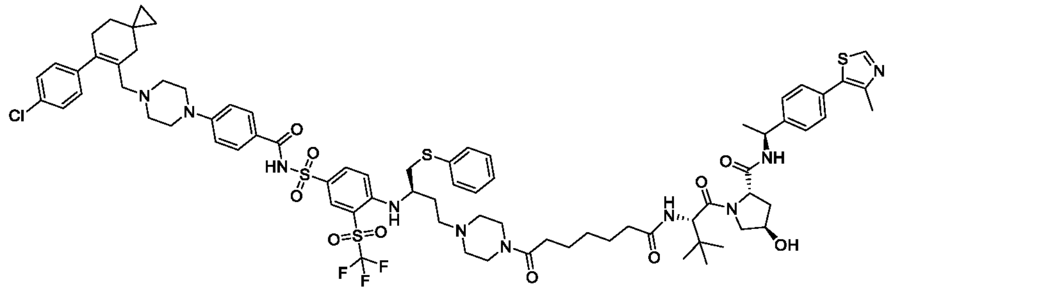 |
| 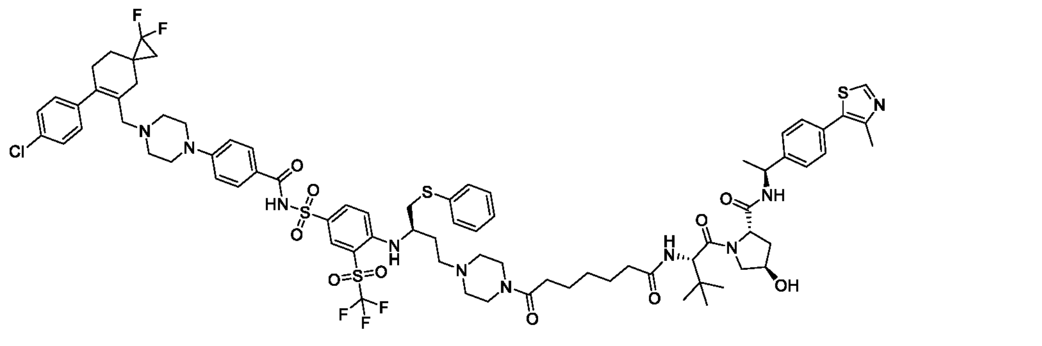 |
| 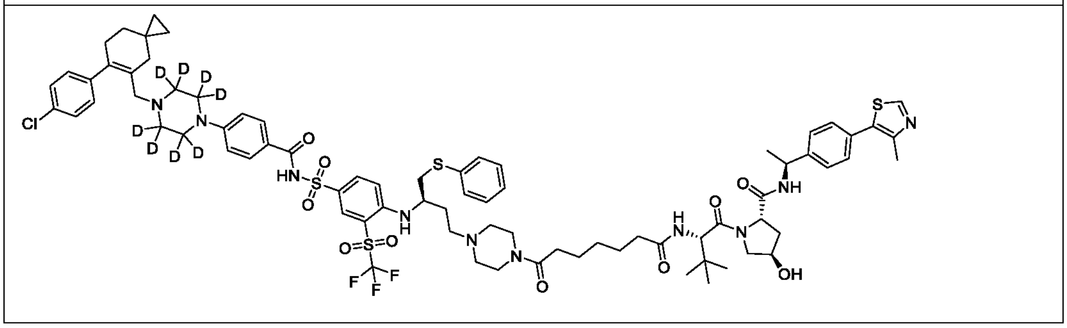 |

| |
|---|
| 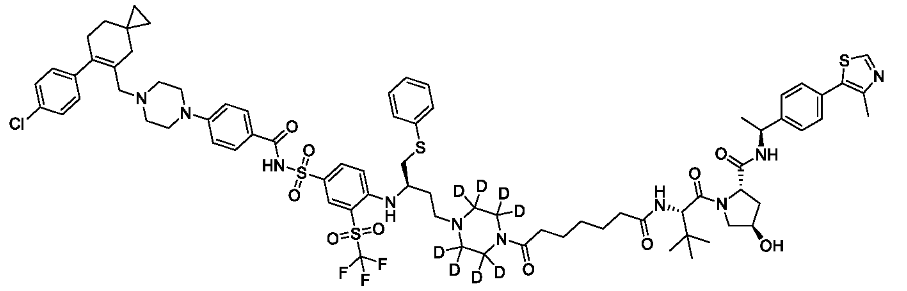 |
| 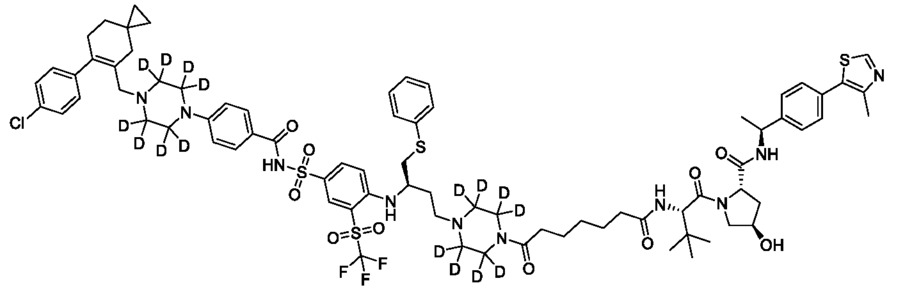 |
| 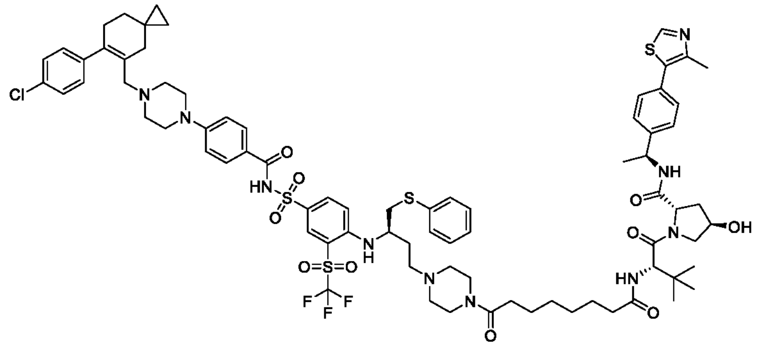 |
| 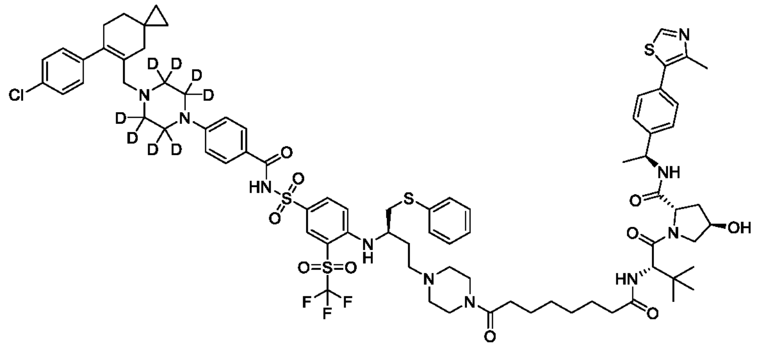 |
| 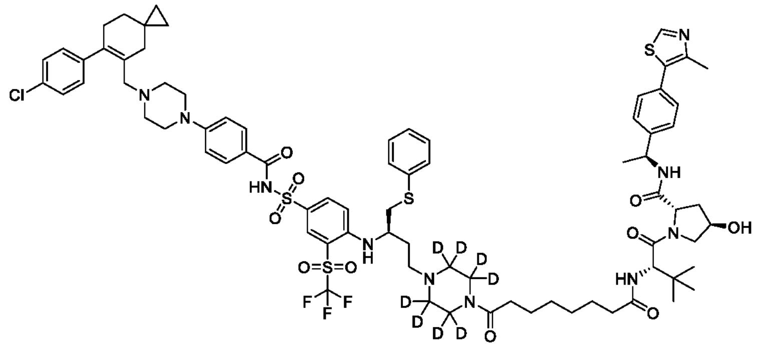 |

| |
|---|
| 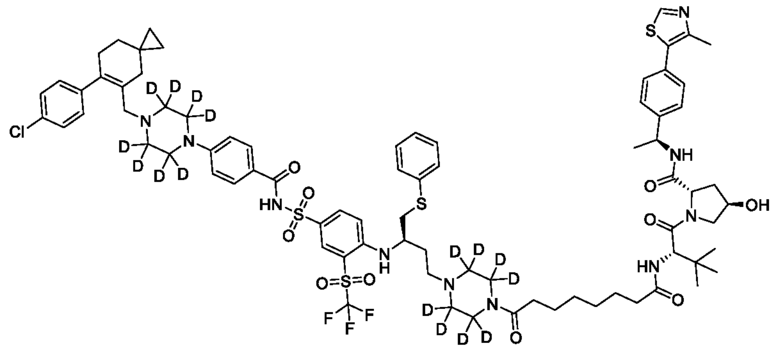 |
| 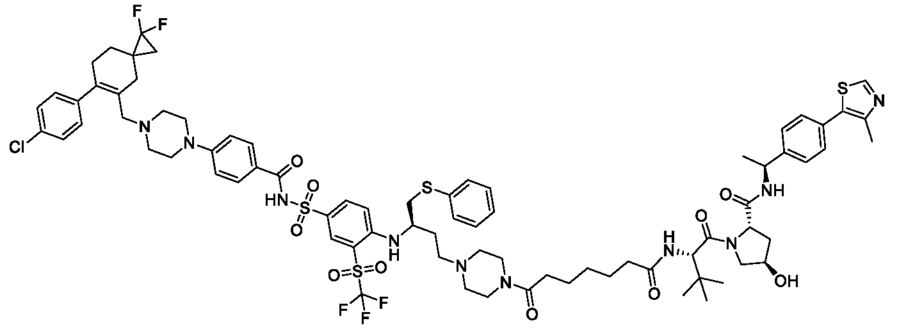 |
| 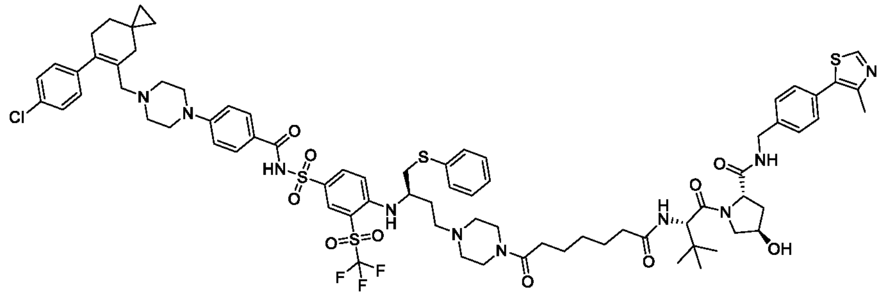 |
| 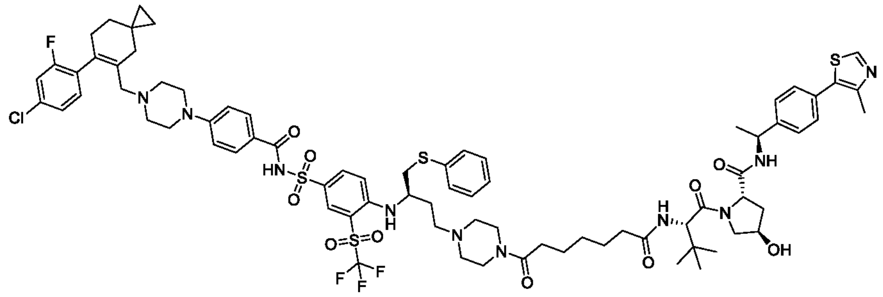 |
| 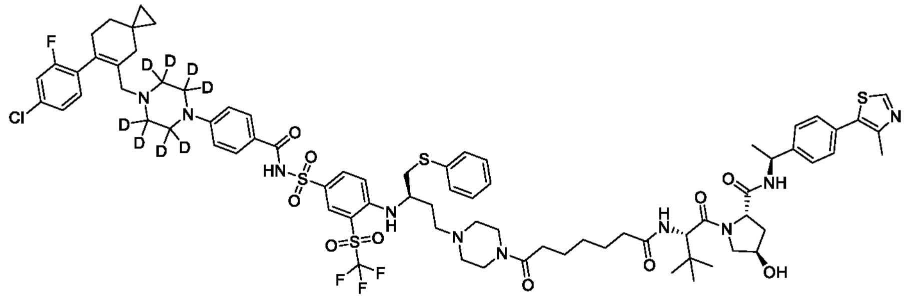 |

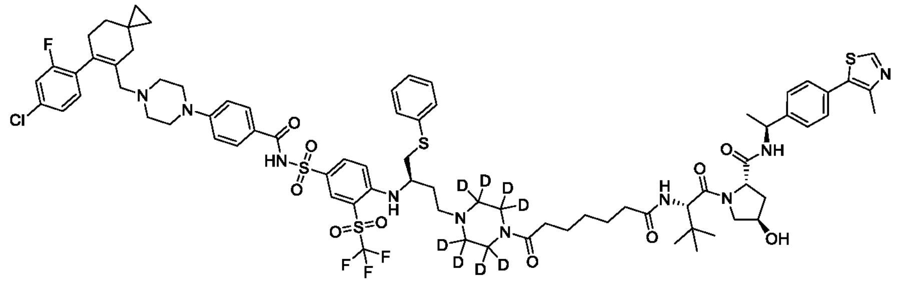

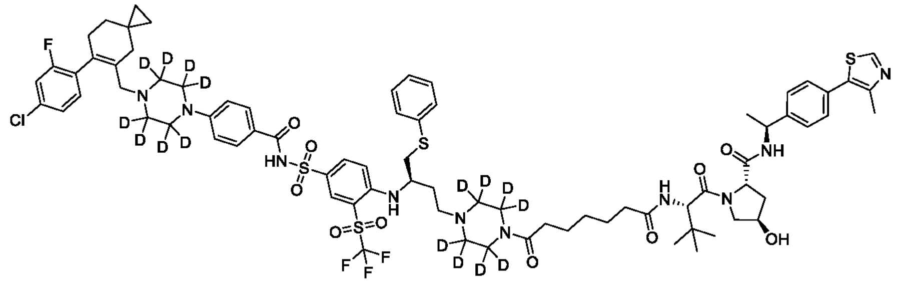

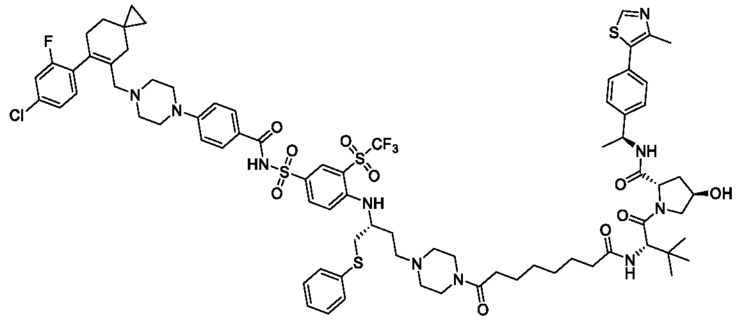

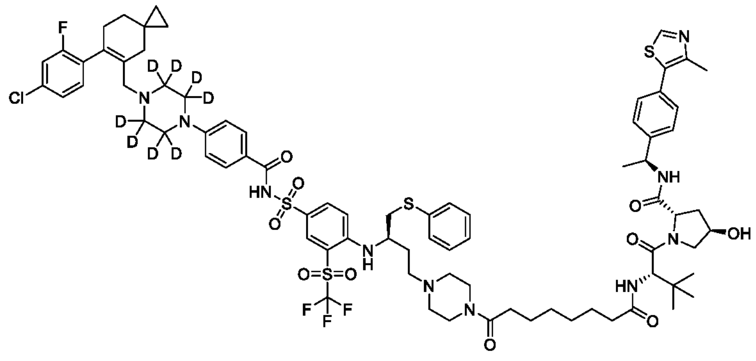

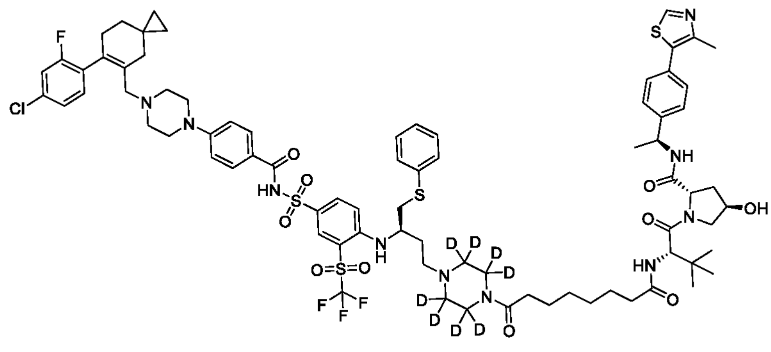

| |
|---|
| 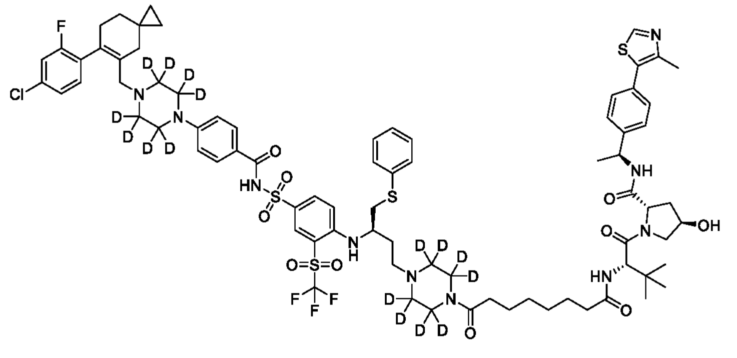 |
| 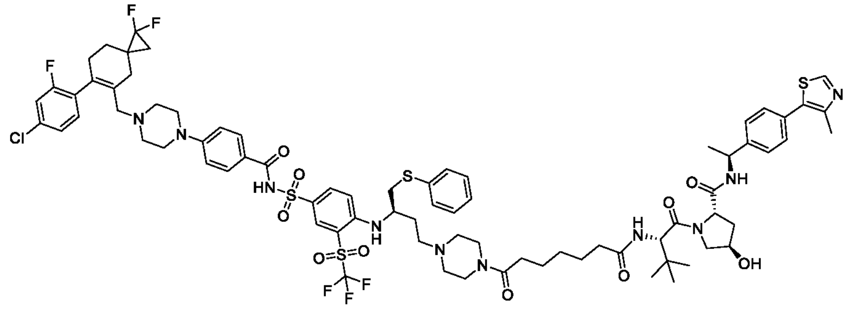 |
| 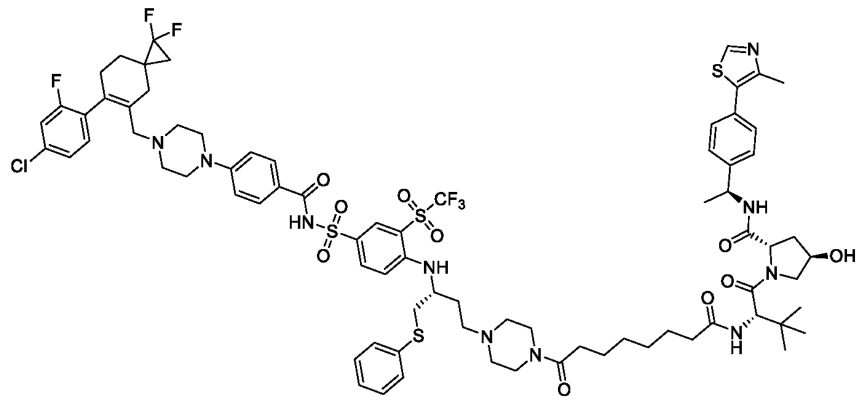 |
| 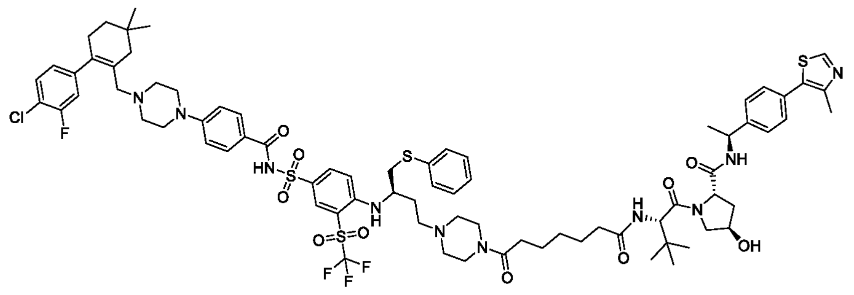 |
| 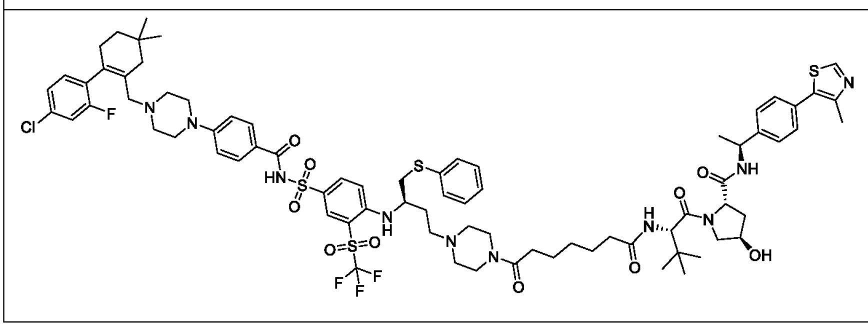 |

| |
|---|
| 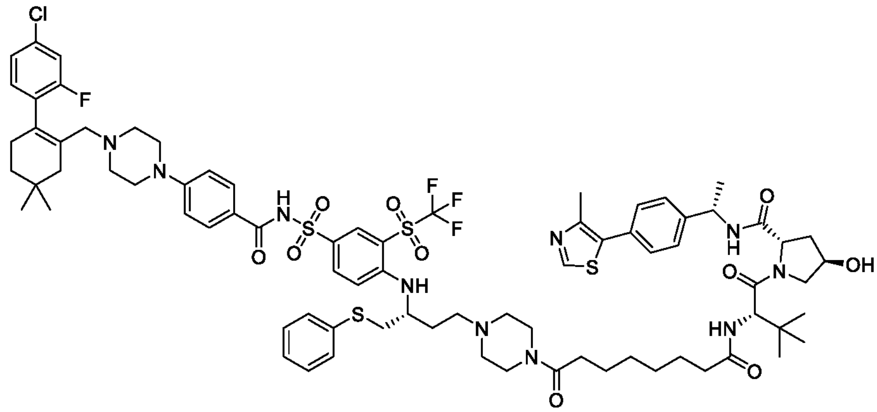 |
| 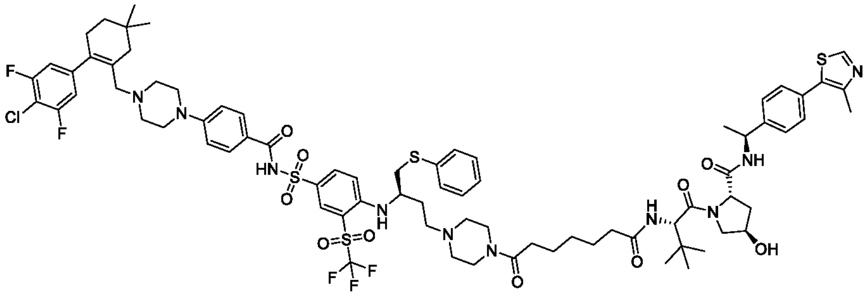 |
| 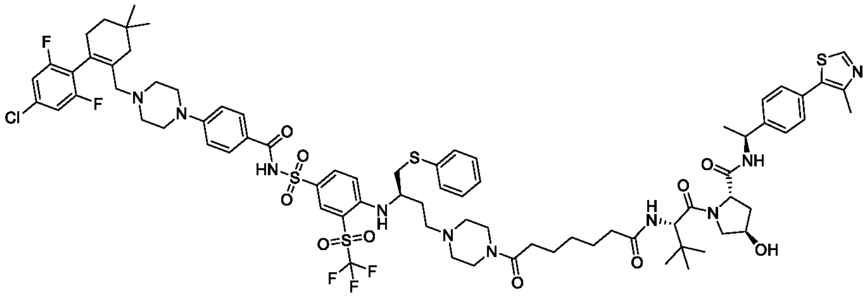 |
| 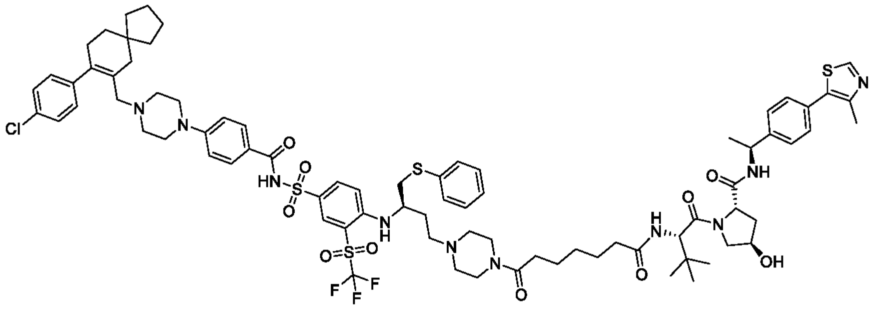 |
| 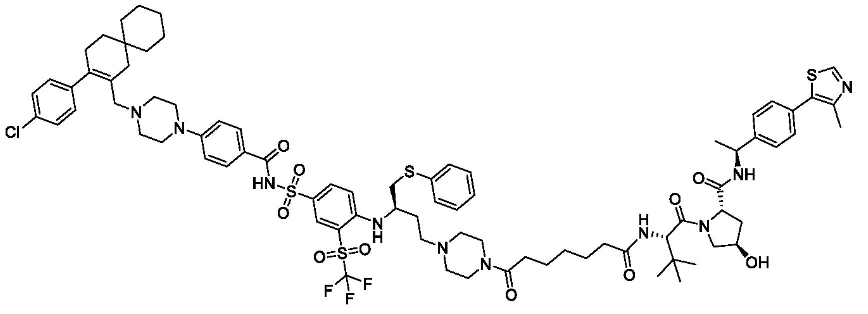 |

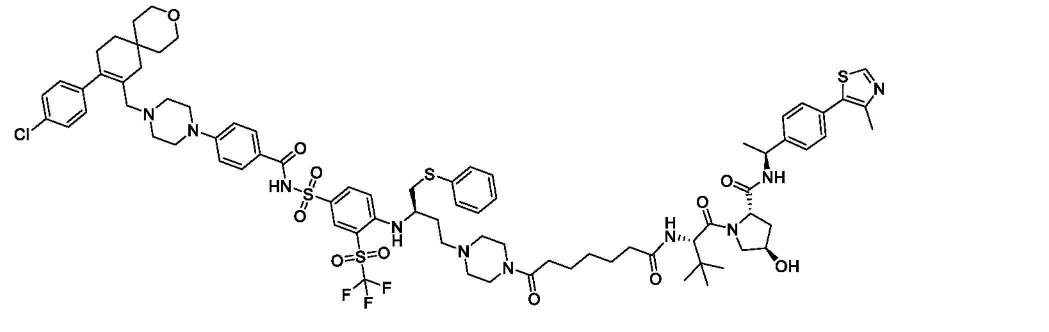

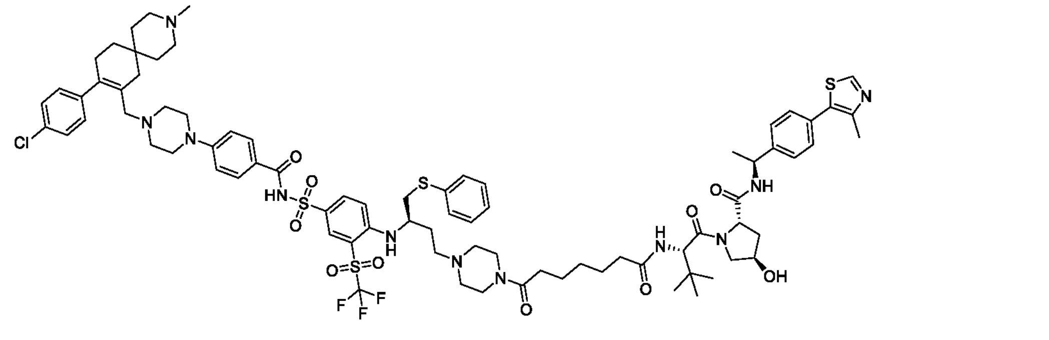

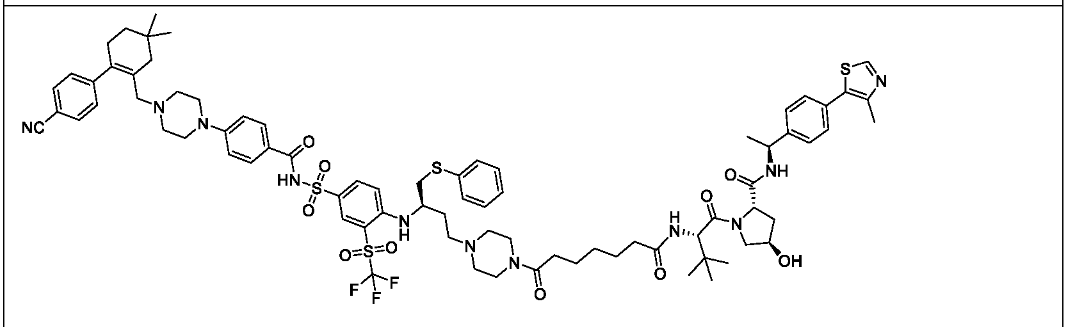

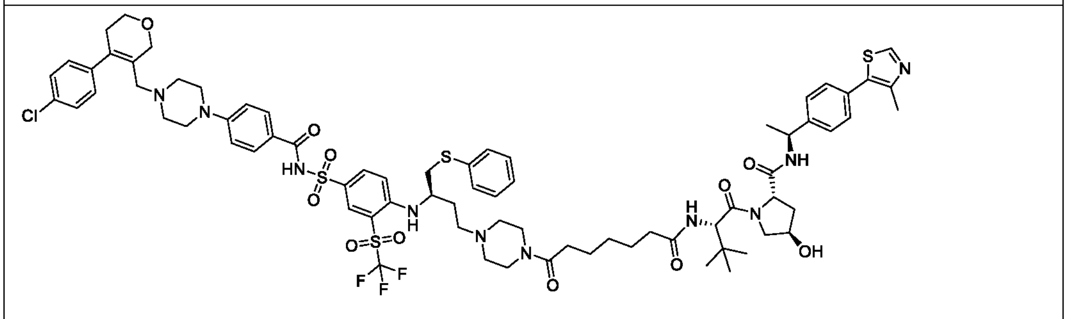

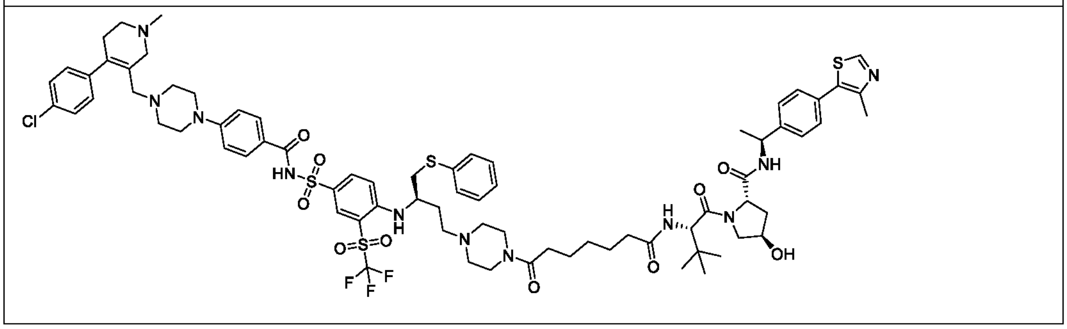

| |
|---|
| 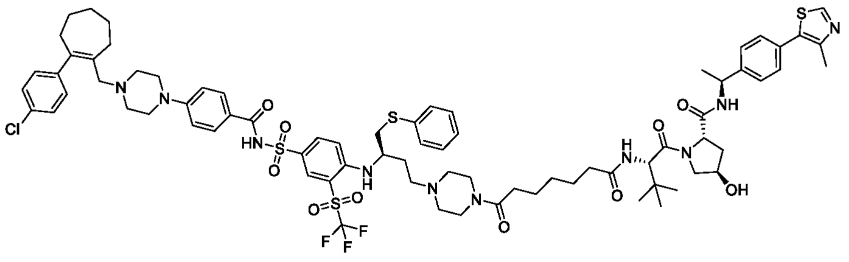 |
| 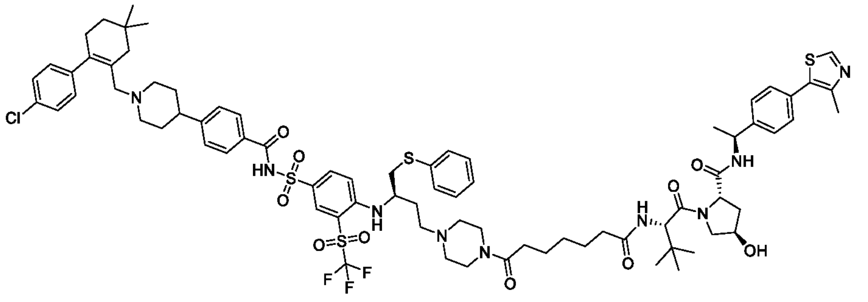 |
| 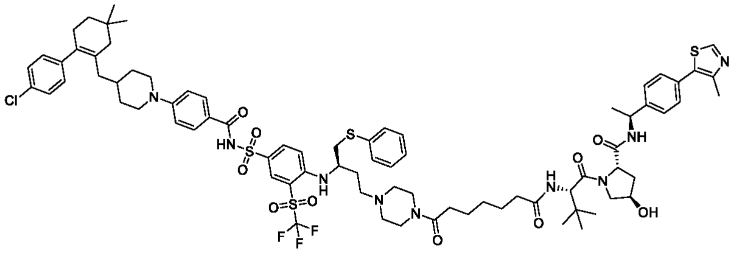 |
| 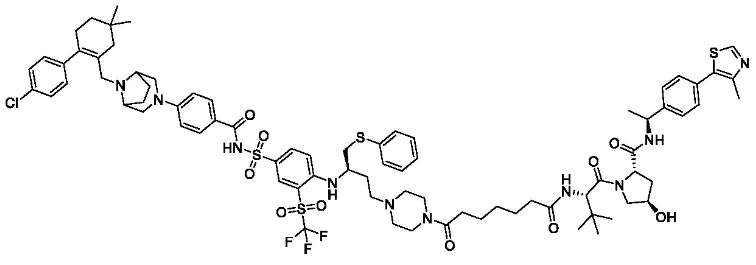 |
| 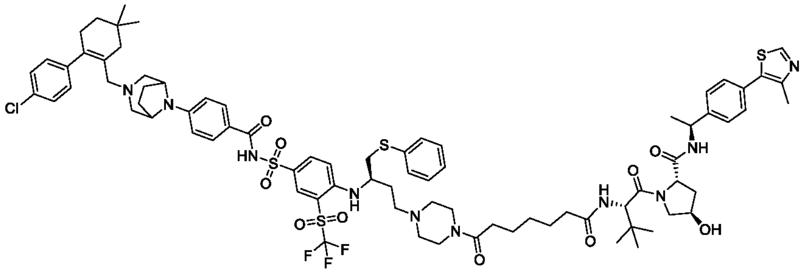 |
| 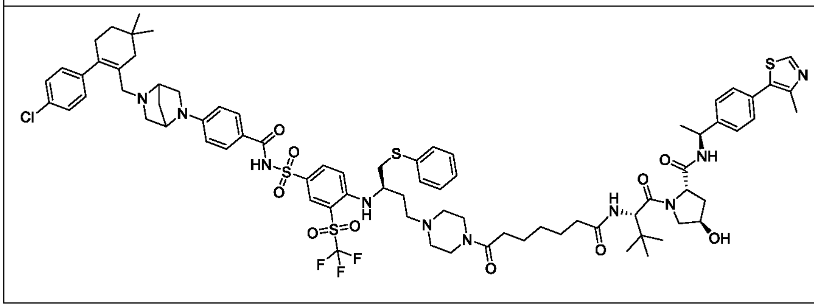 |

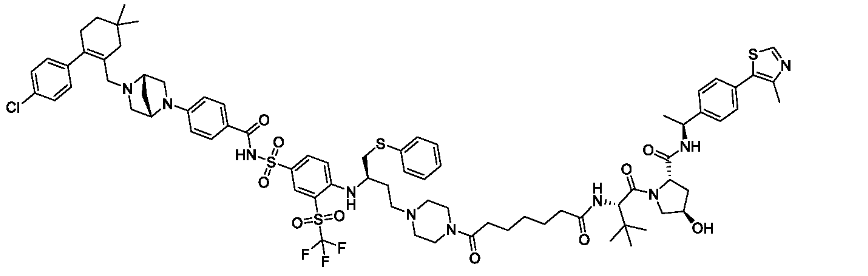

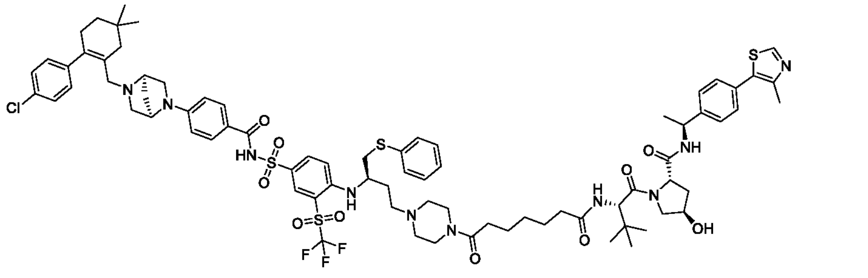

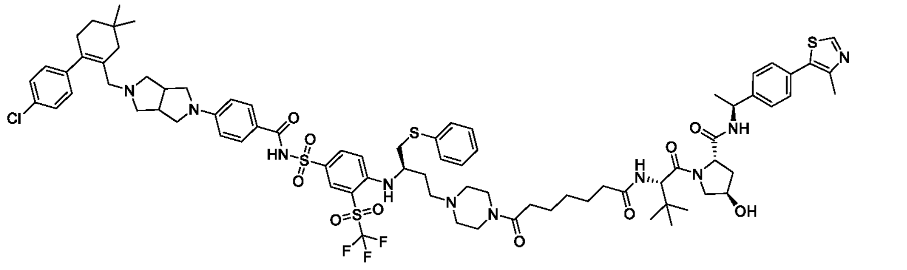

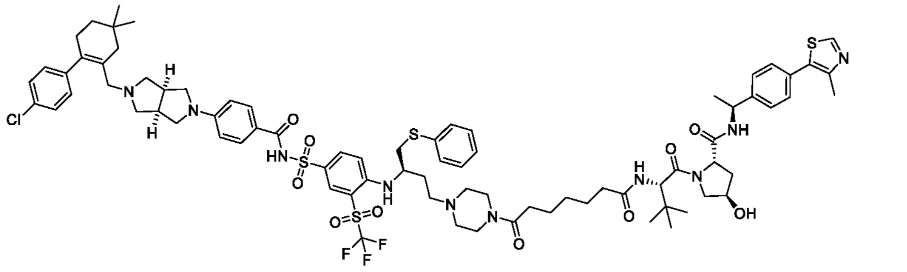

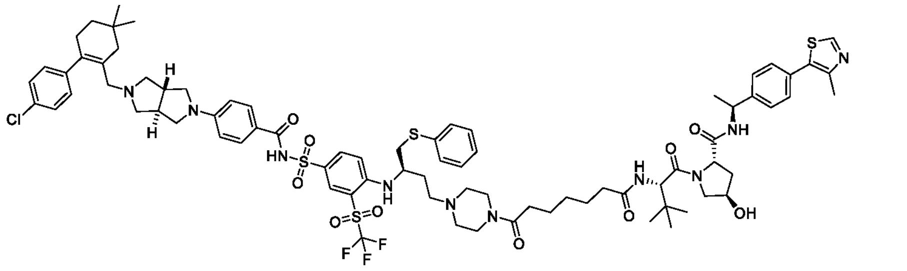

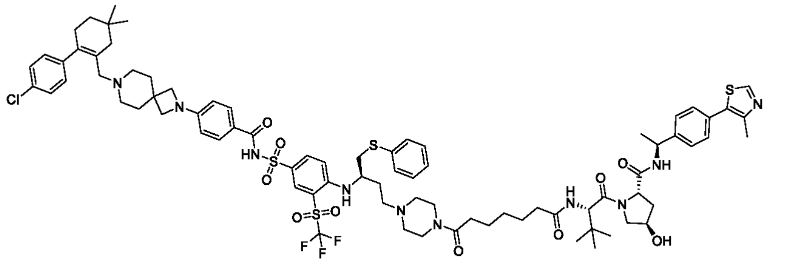

| |
|---|
| 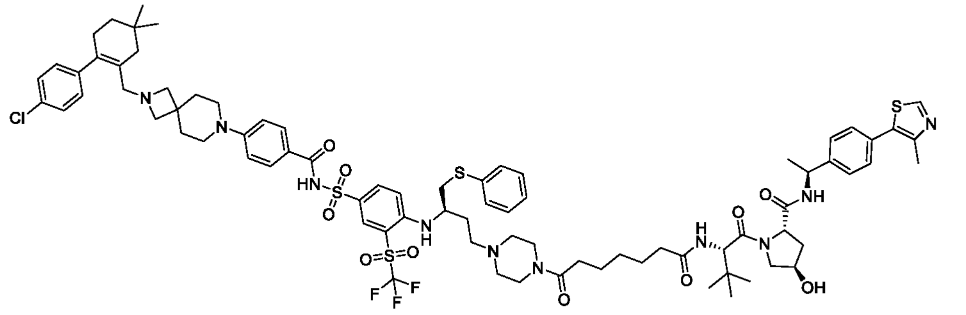 |
| 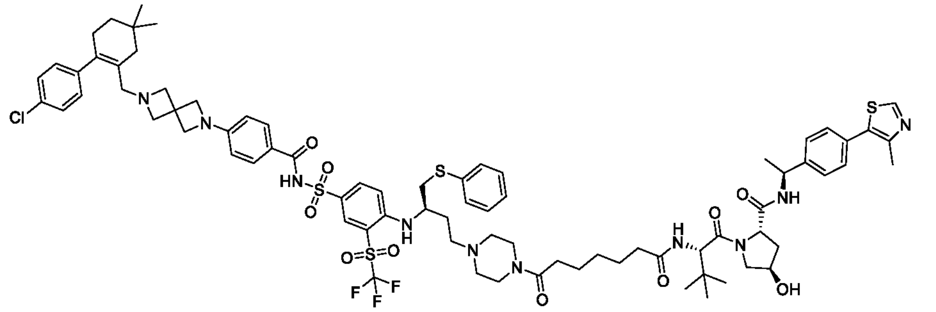 |
| 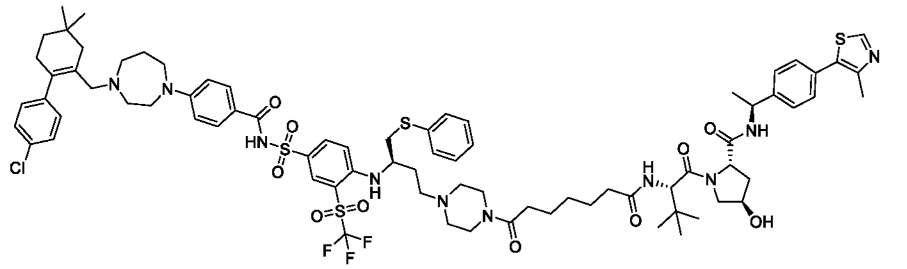 |
| 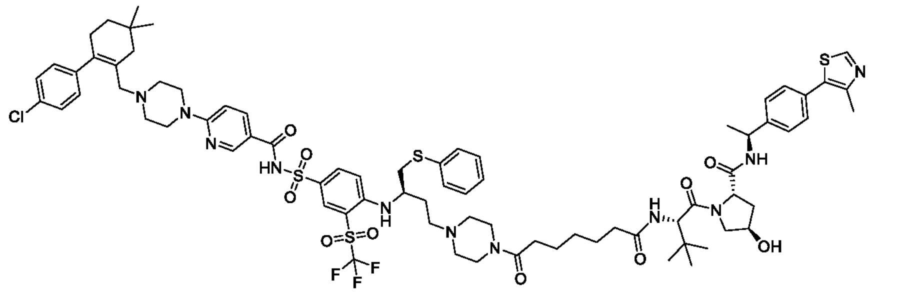 |
| 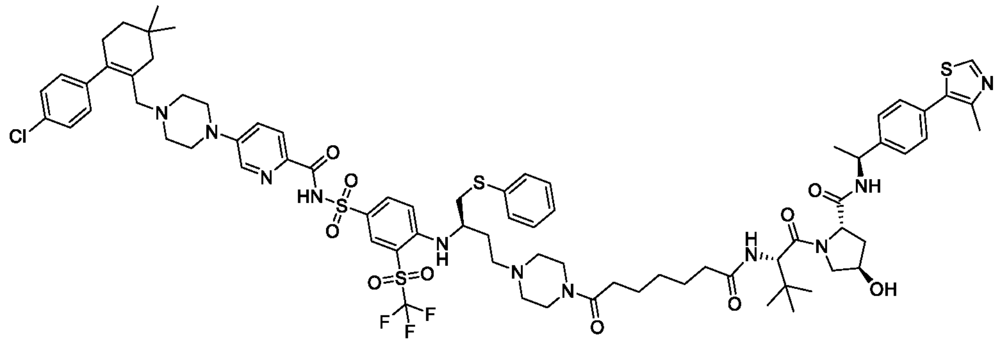 |

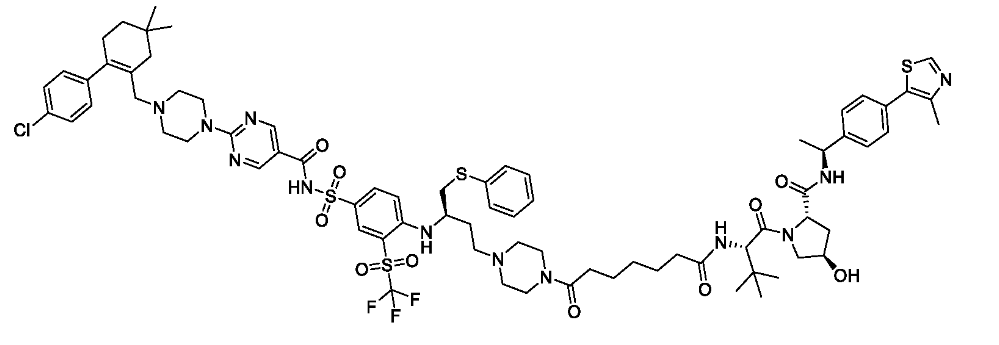

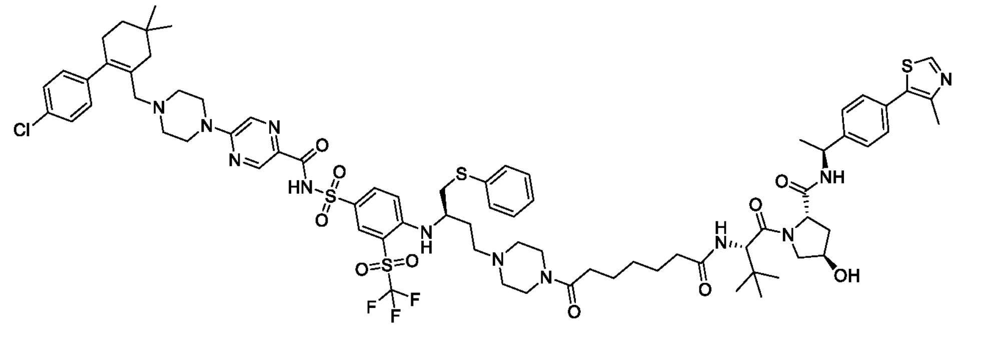

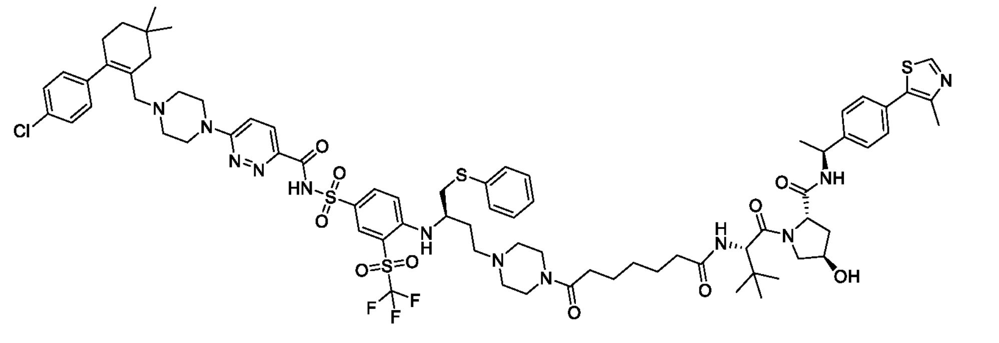

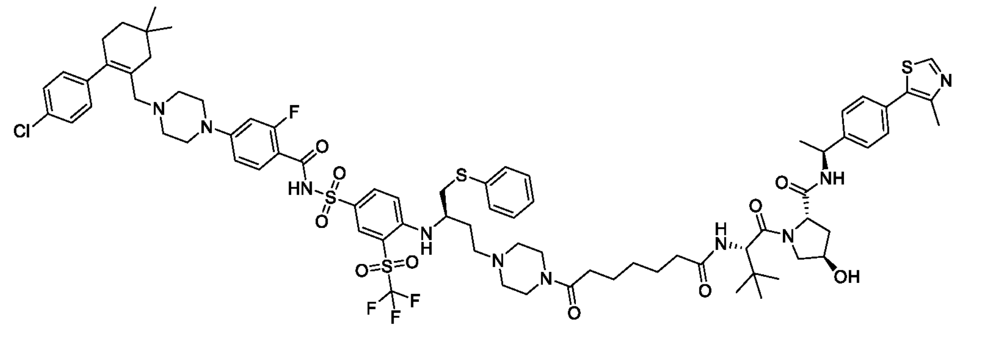

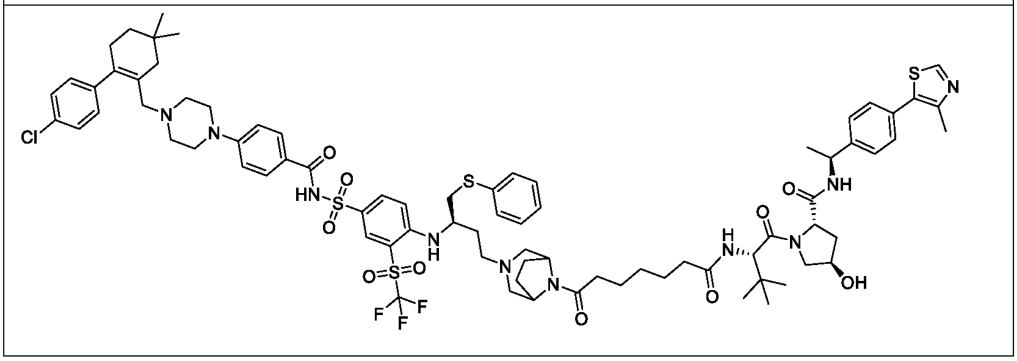

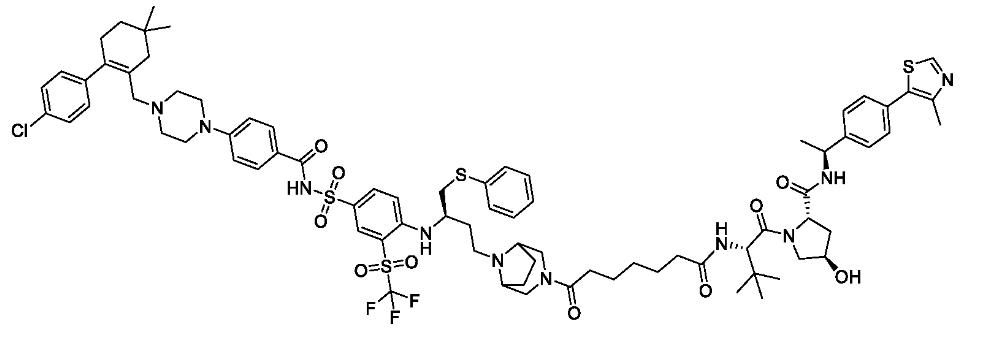

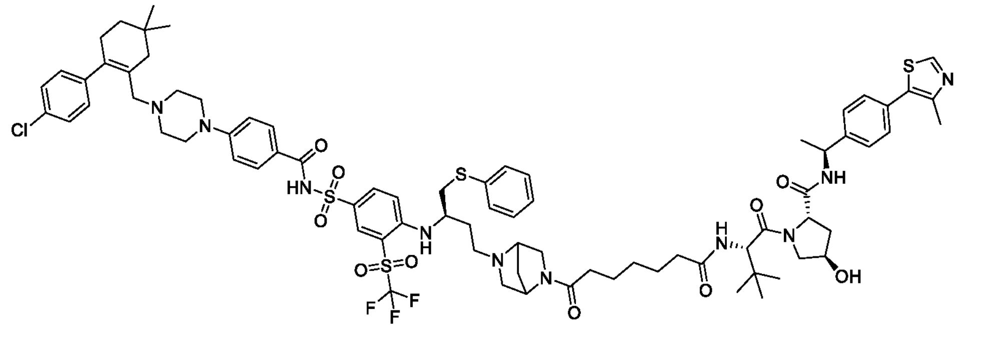

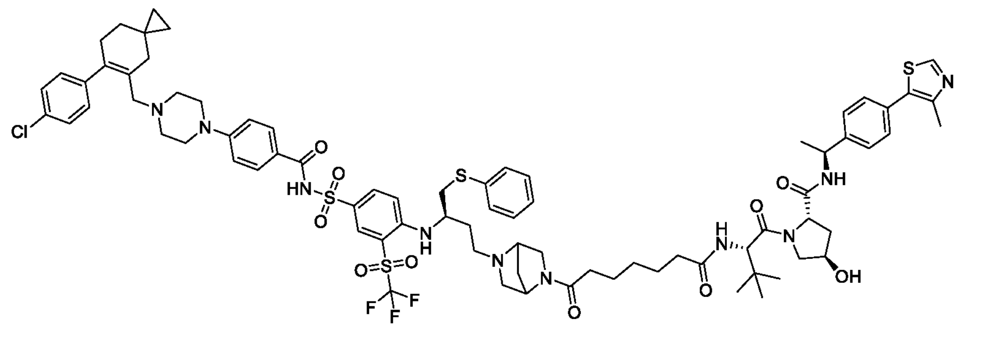

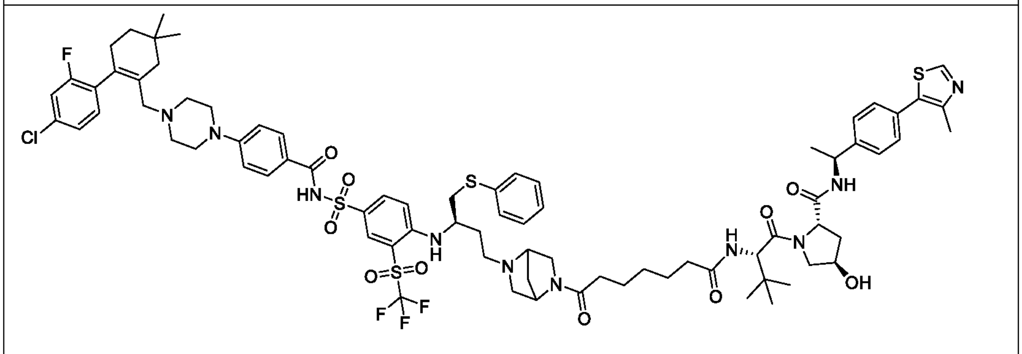

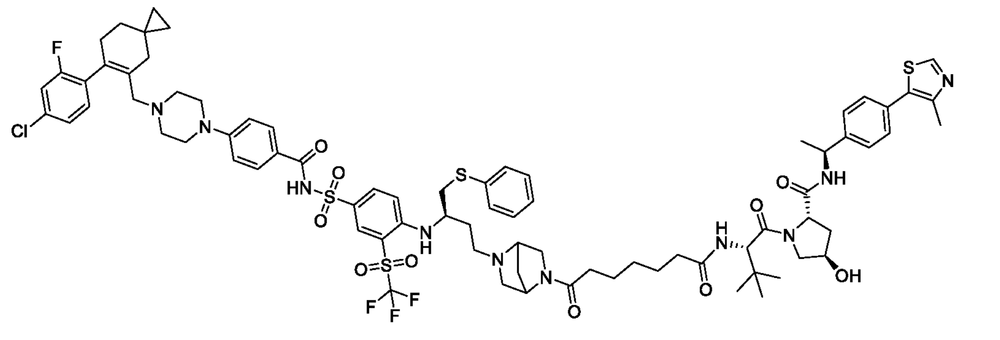

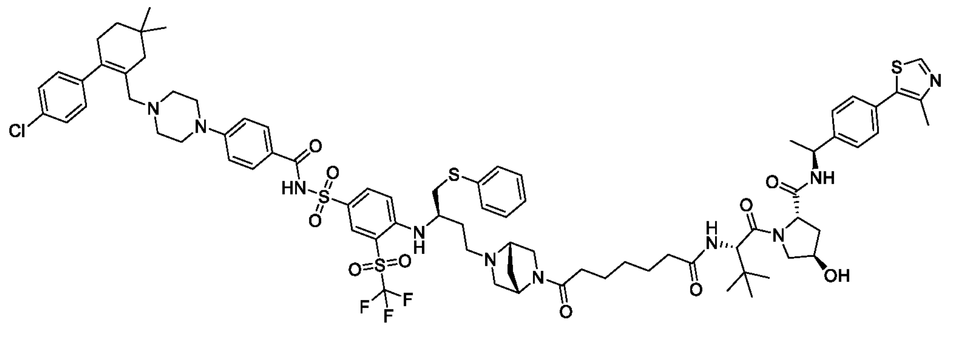

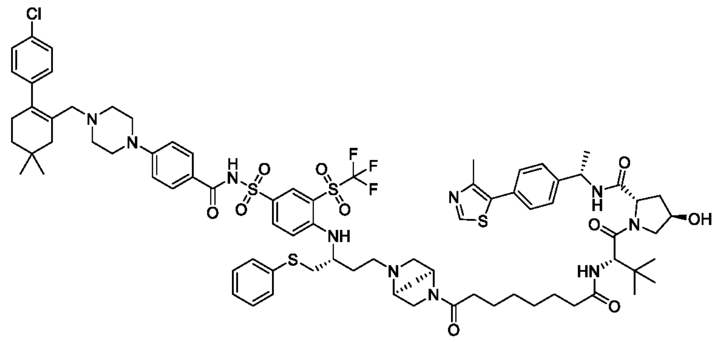

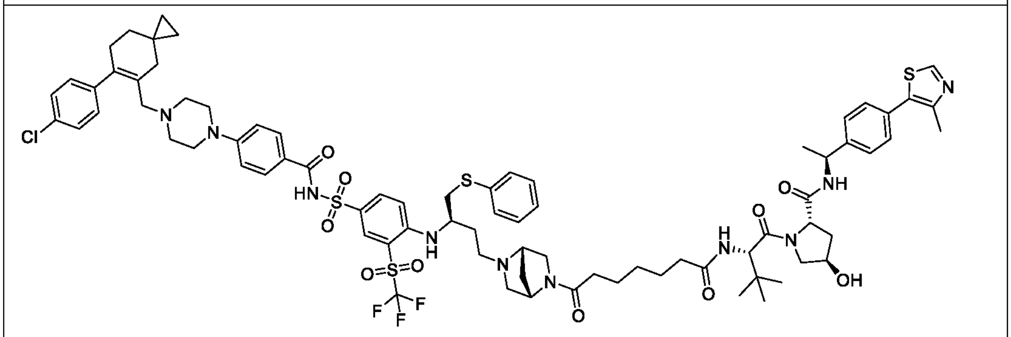

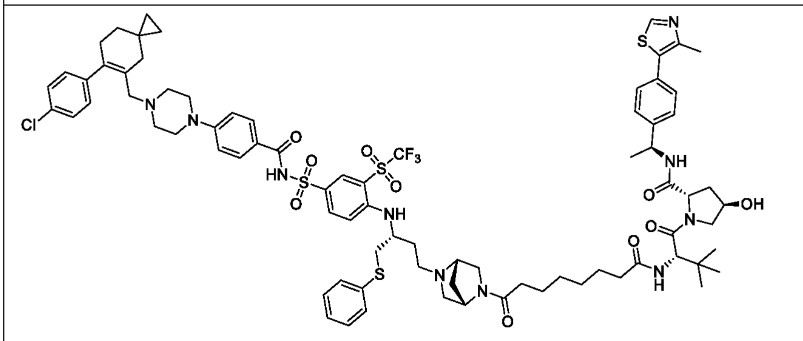

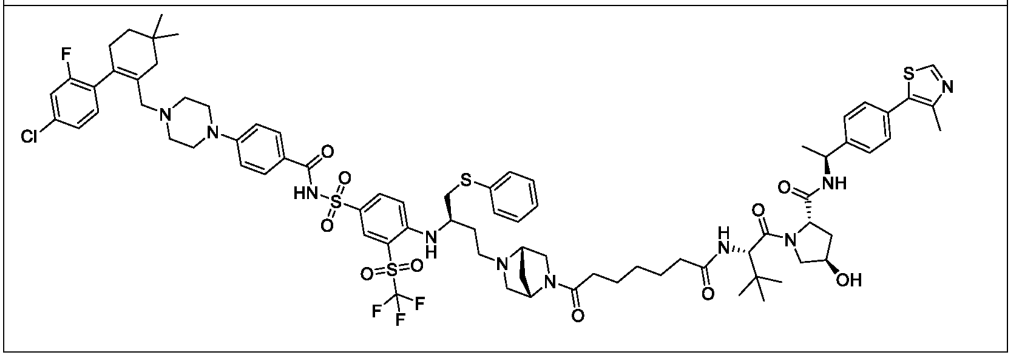

| |
|---|
| 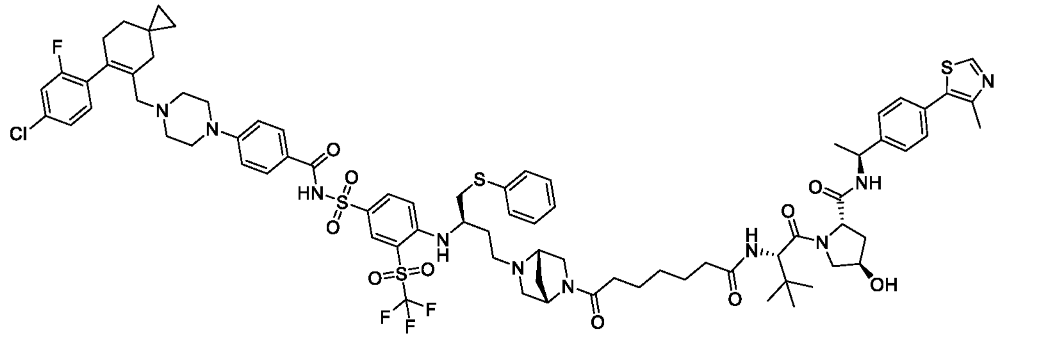 |
| 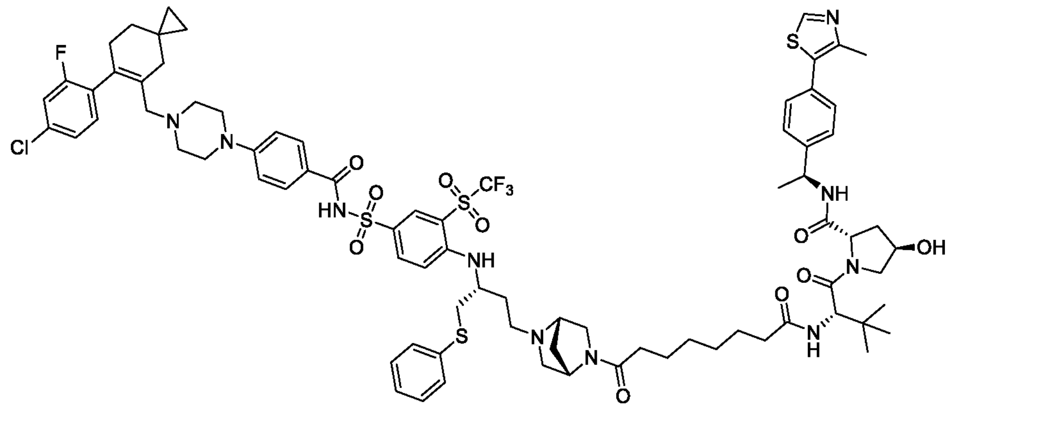 |
| 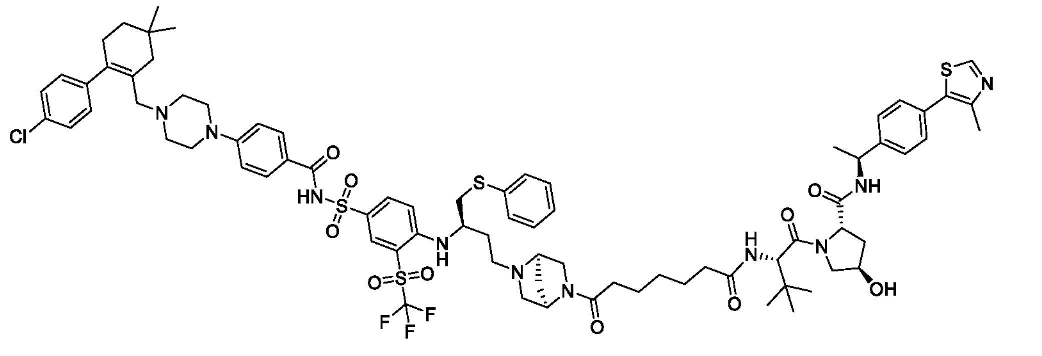 |
| 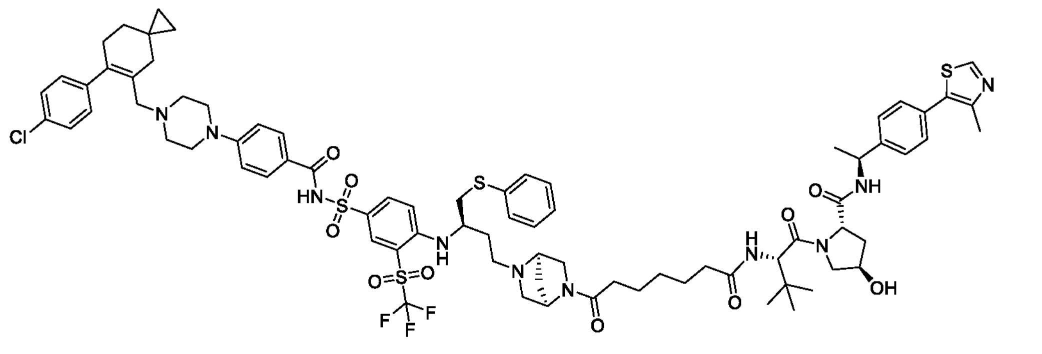 |

| |
|---|
| 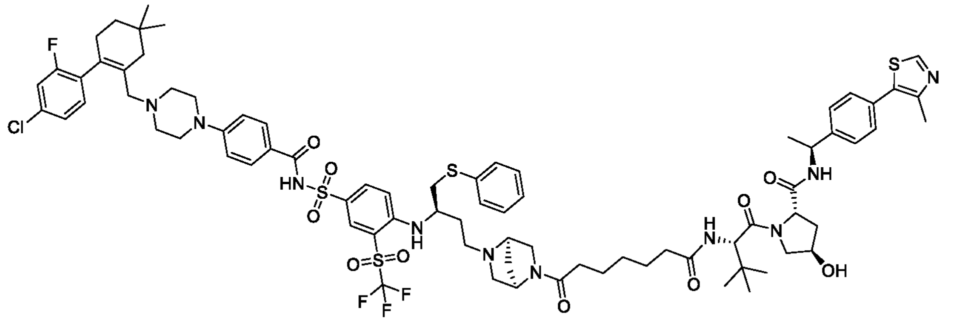 |
| 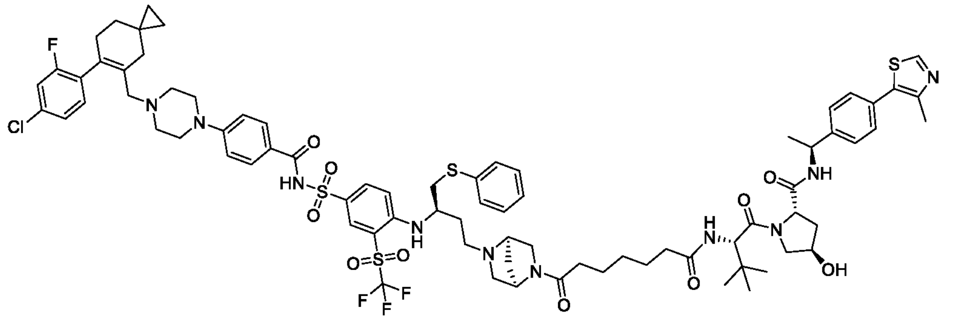 |
| 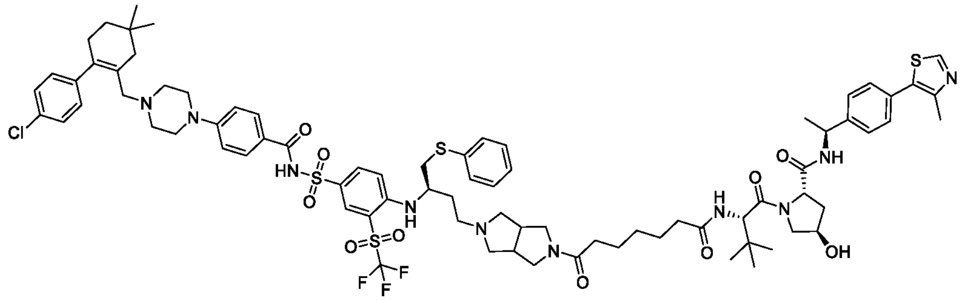 |
| 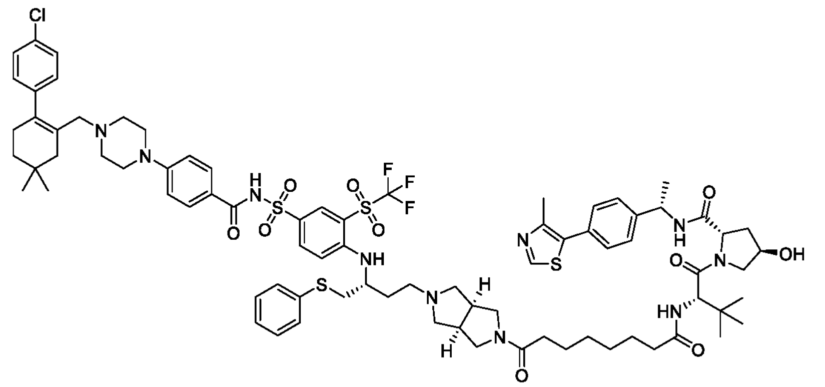 |
| 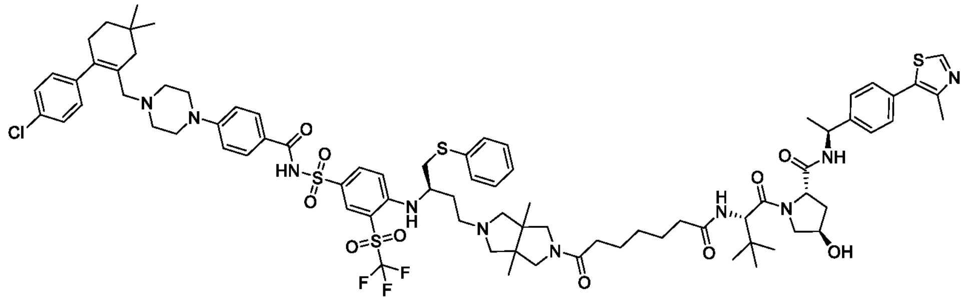 |

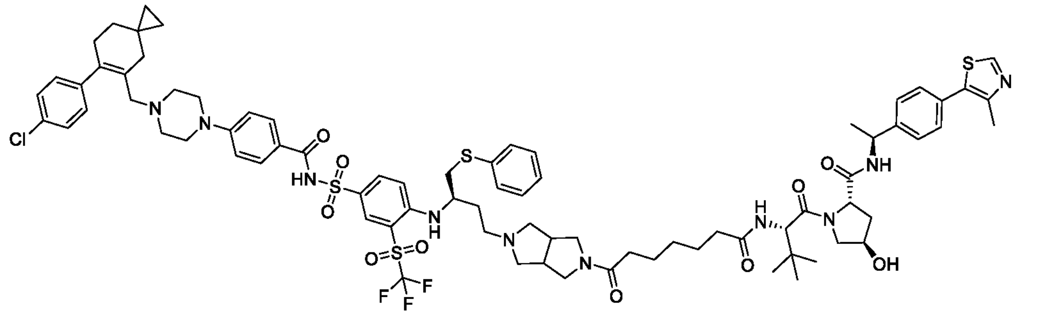

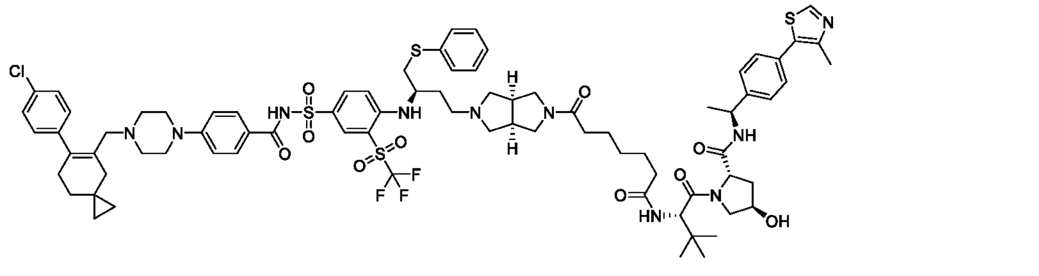

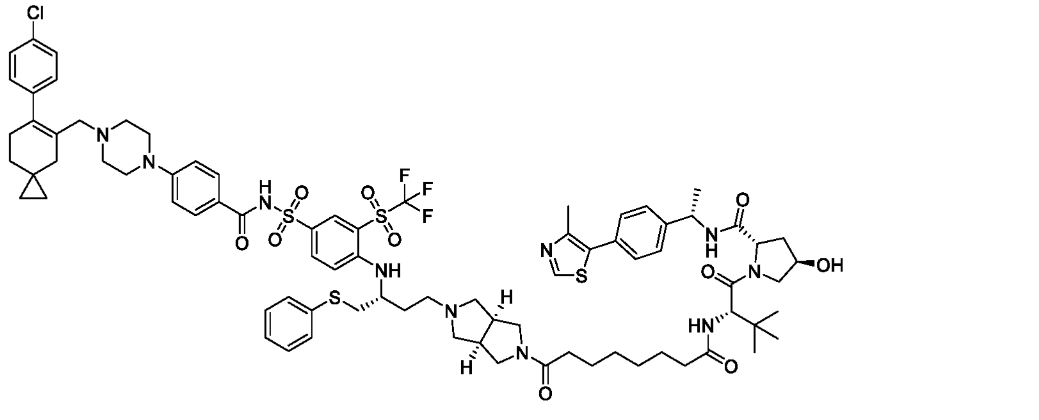

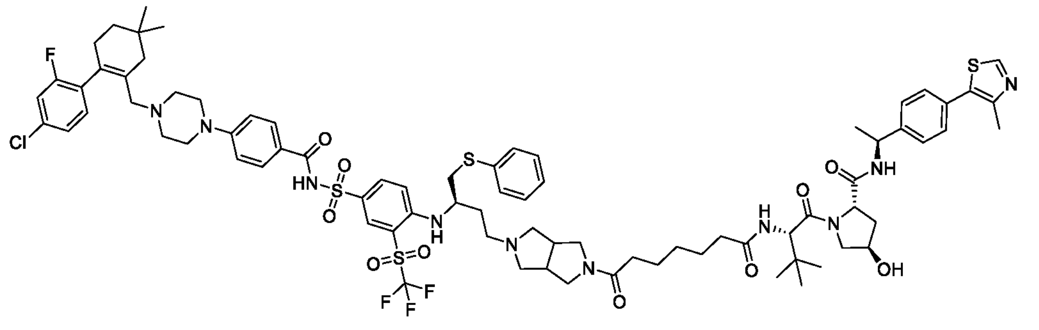

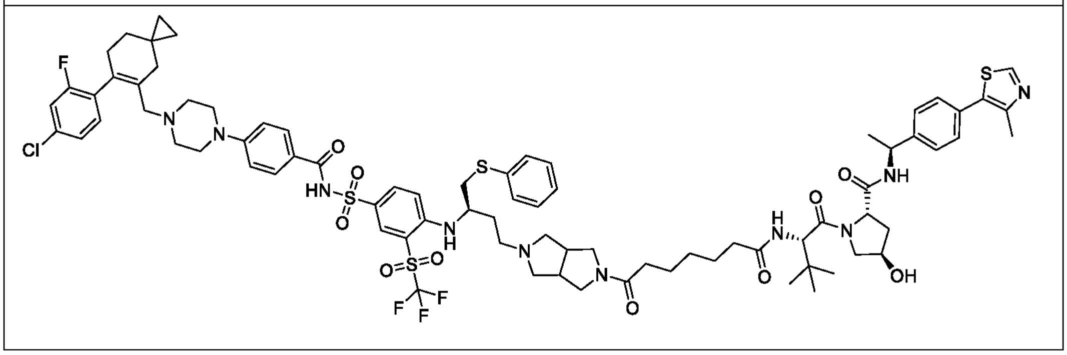

| |
|---|
| 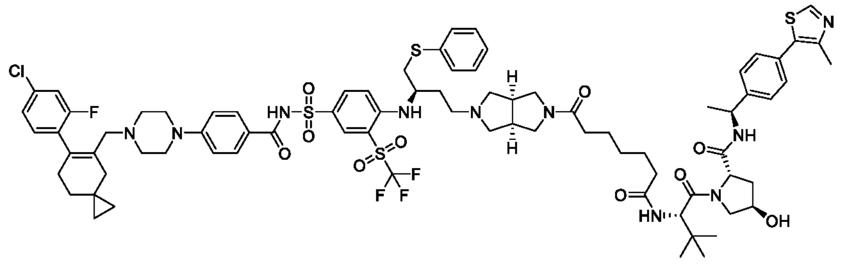 |
| 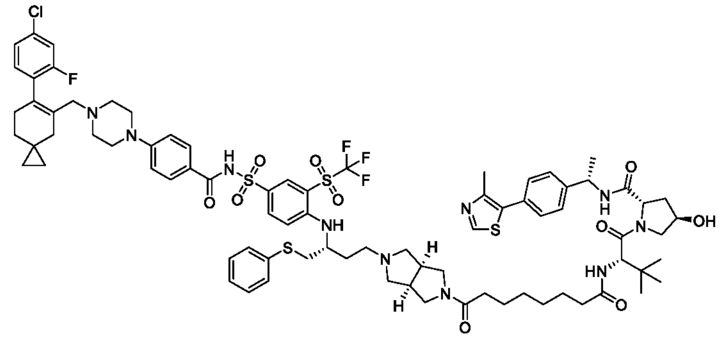 |
| 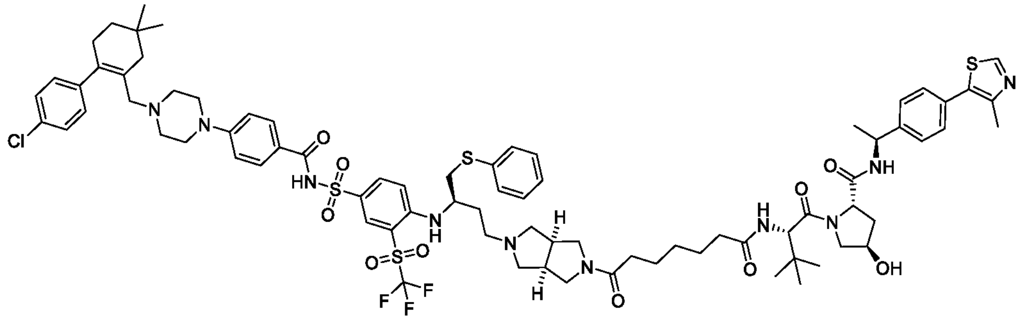 |
| 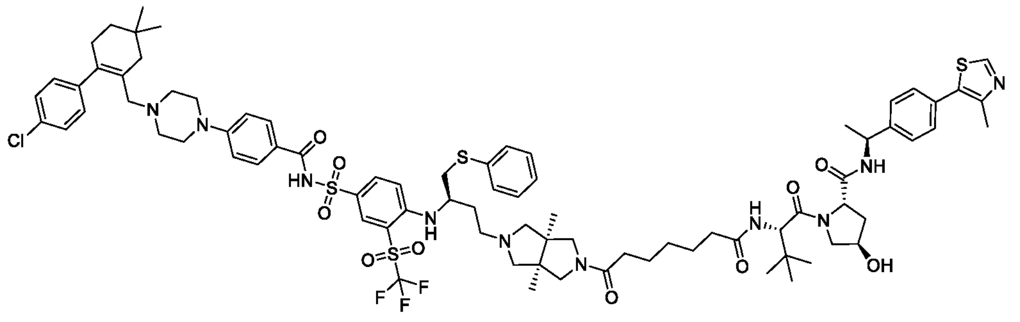 |
| 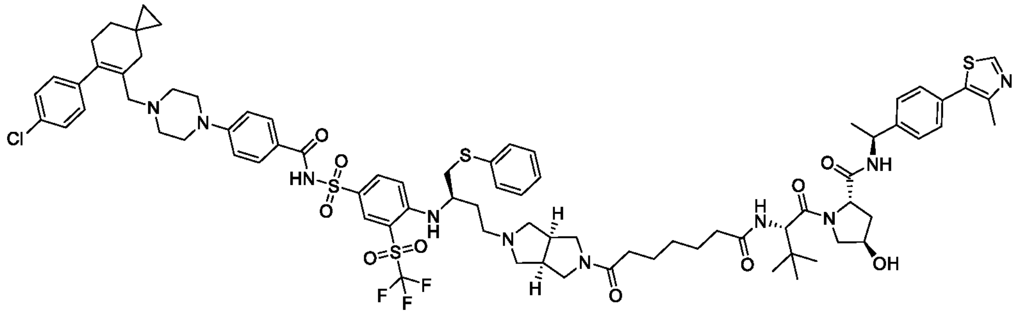 |

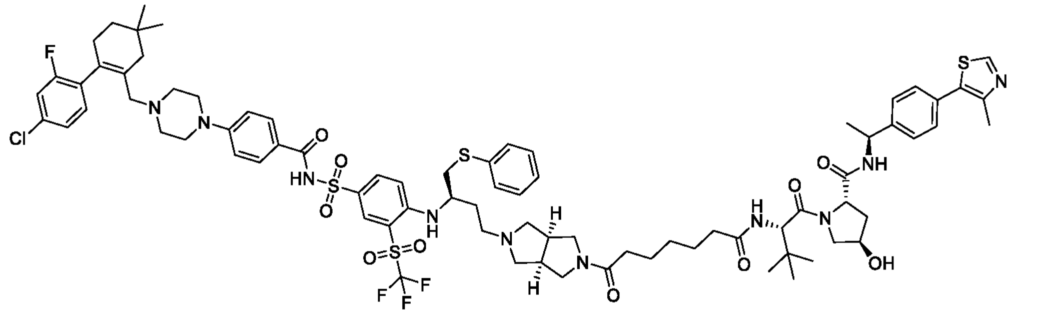

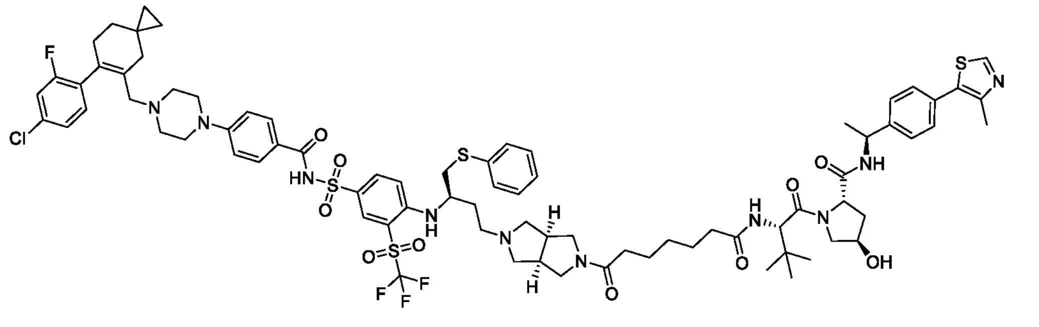

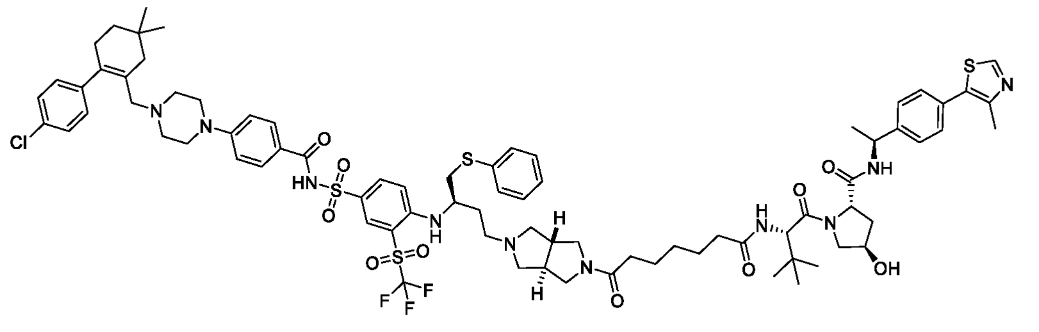

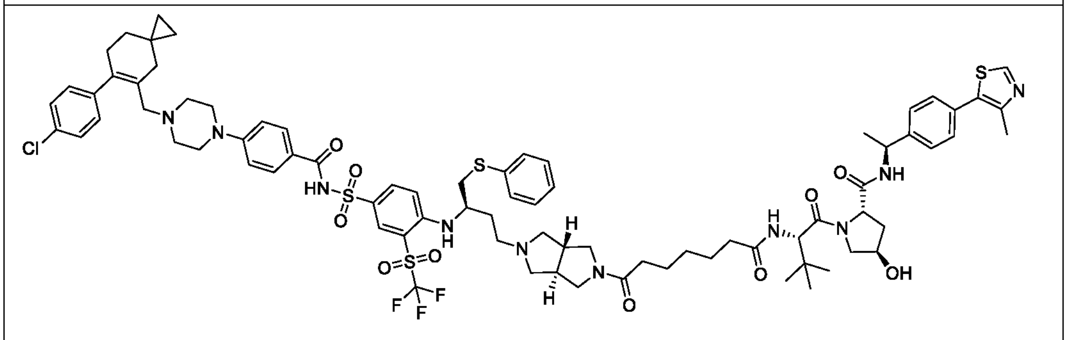

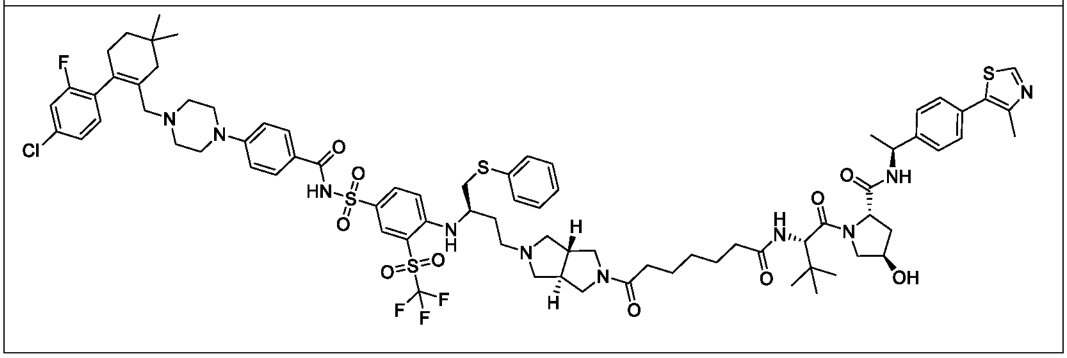

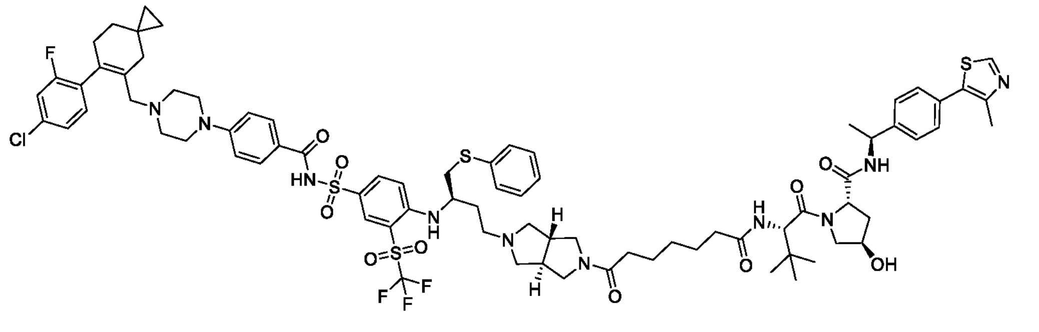

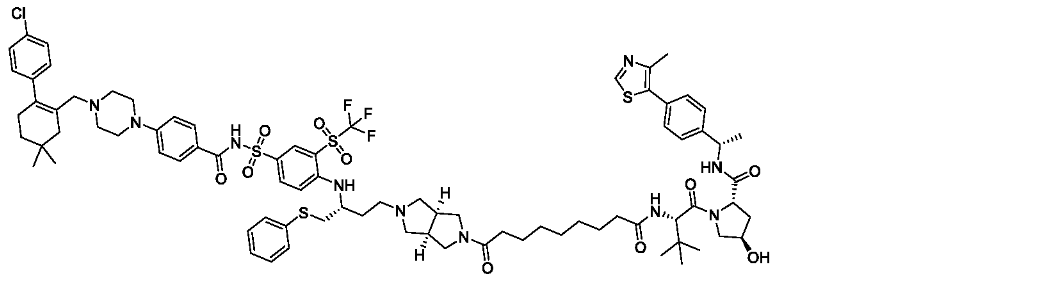

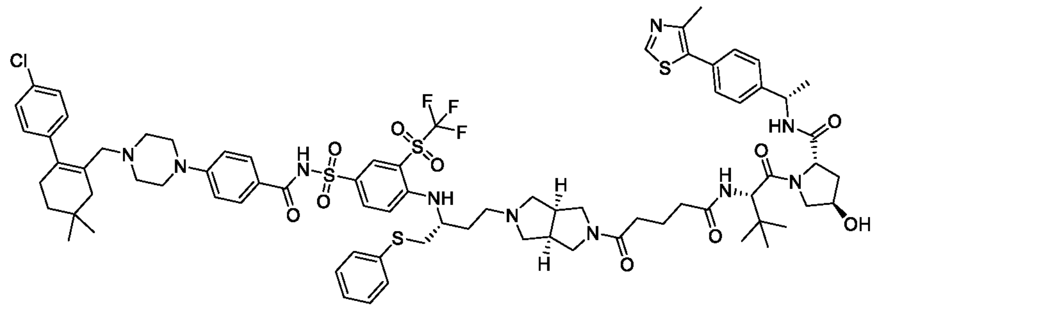

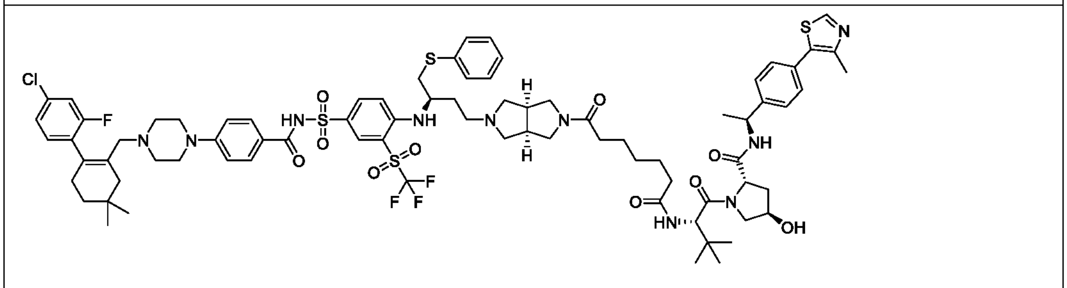

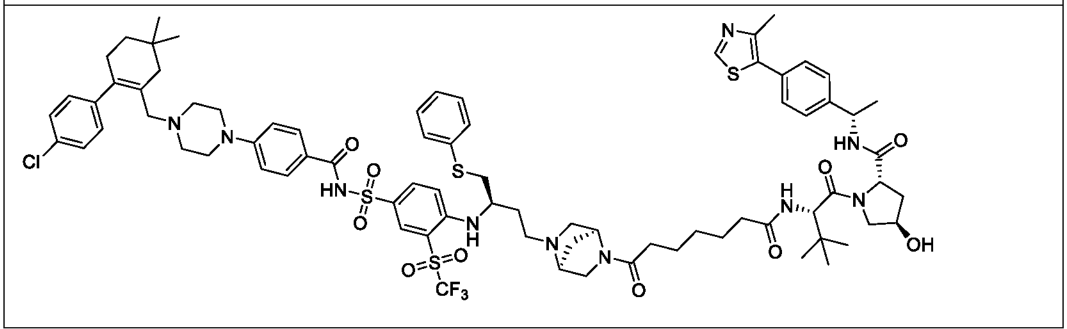

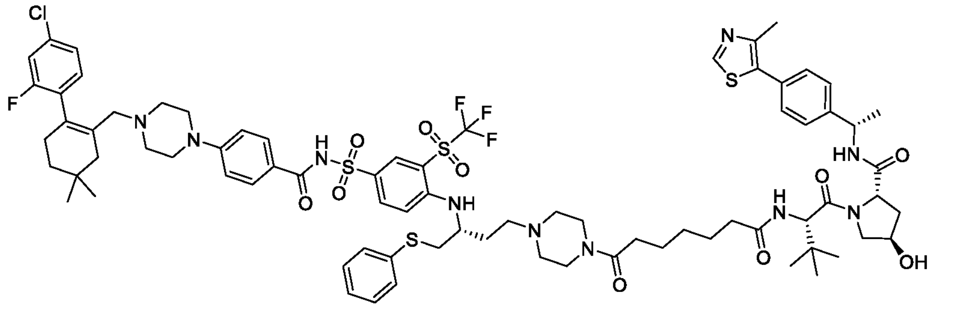

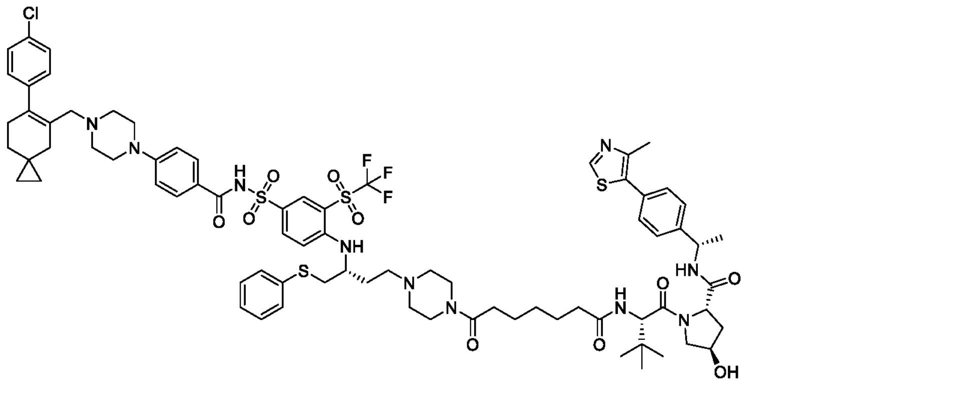

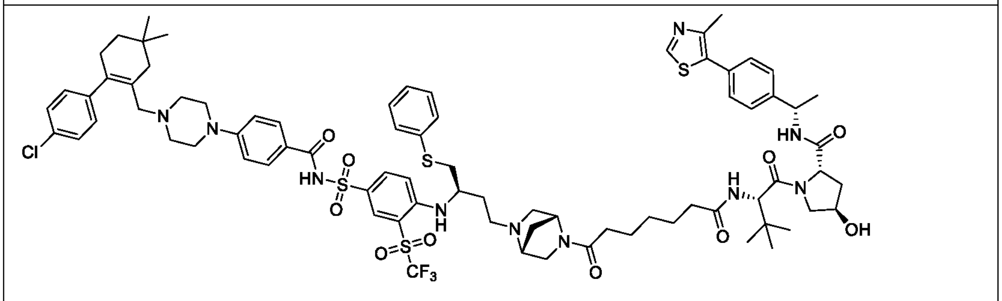

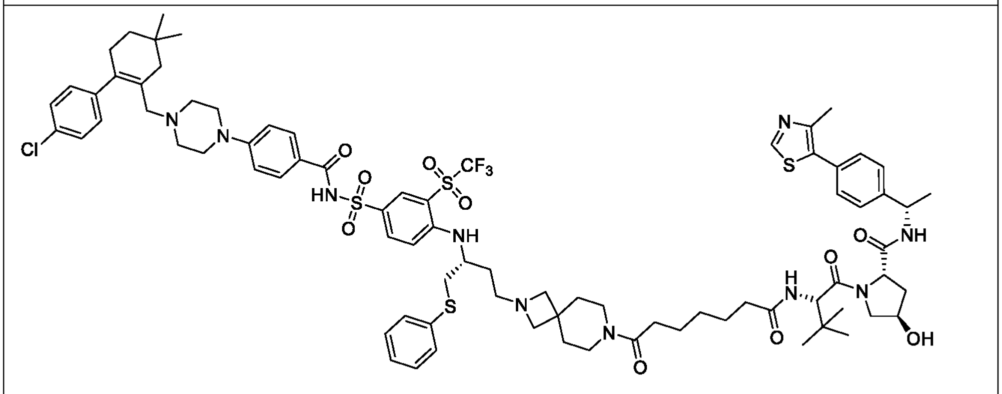

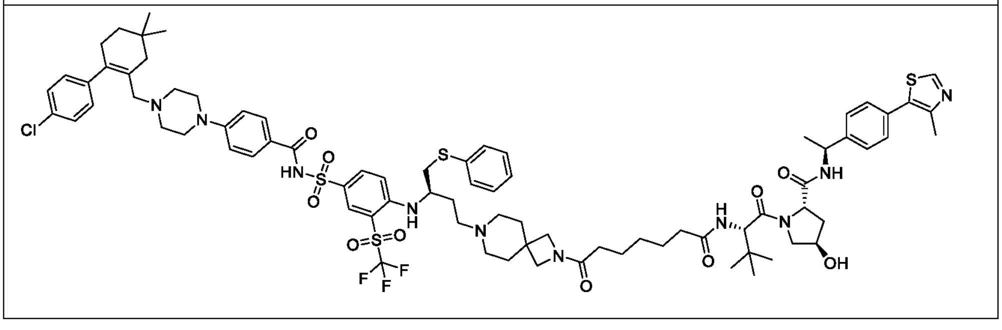

| |
|---|
| 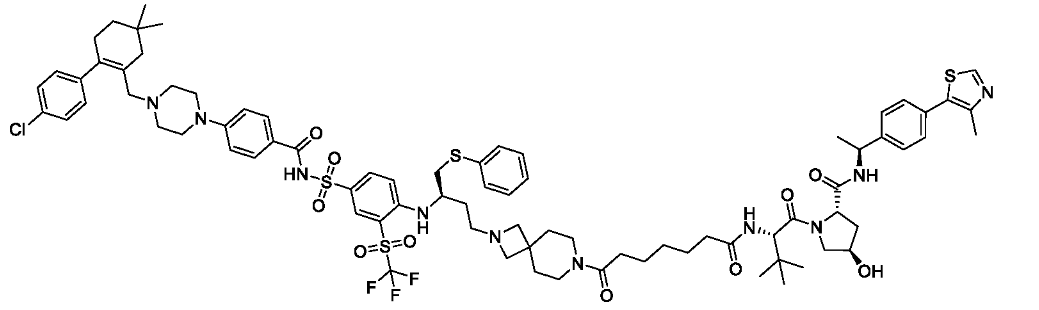 |
| 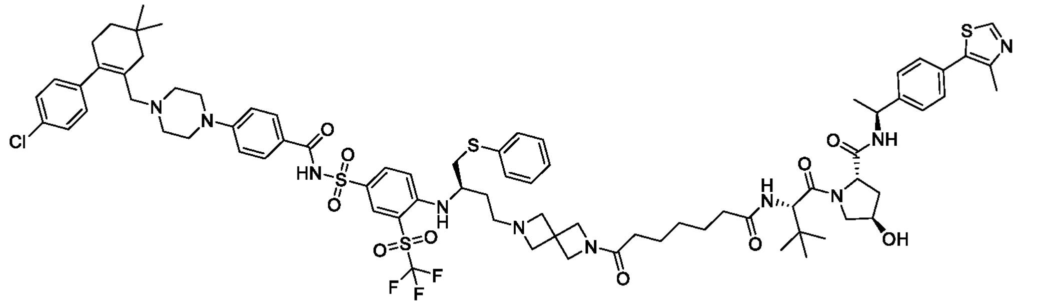 |
| 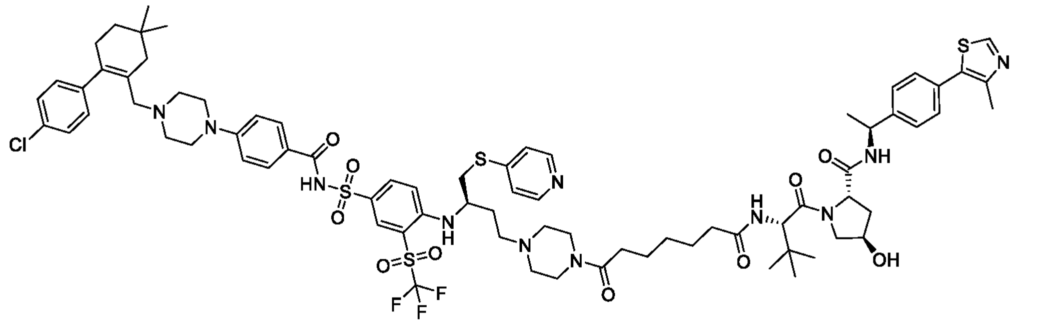 |
| 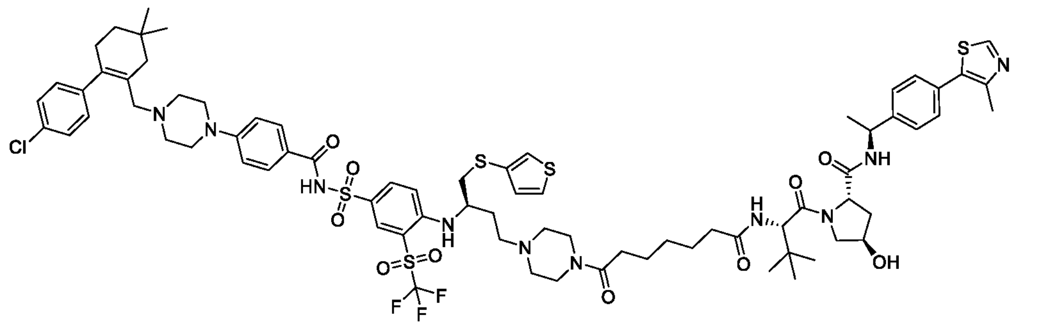 |
| 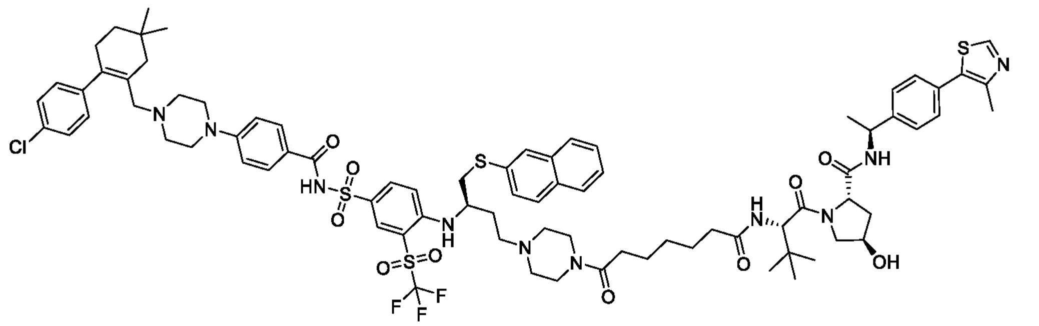 |

| |
|---|
| 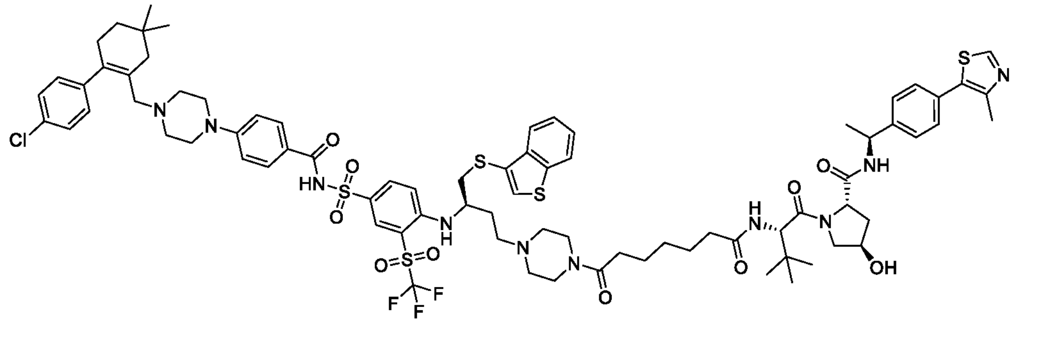 |
| 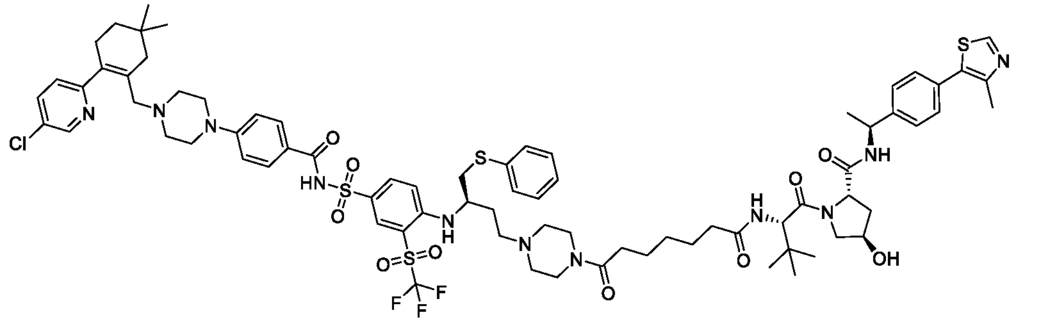 |
| 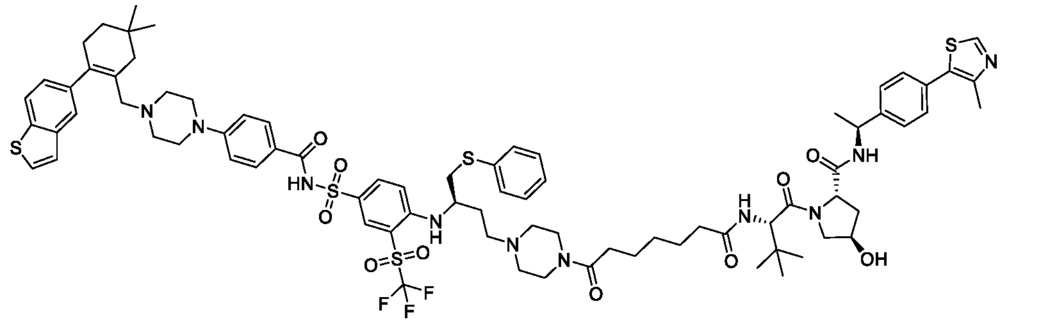 |
| 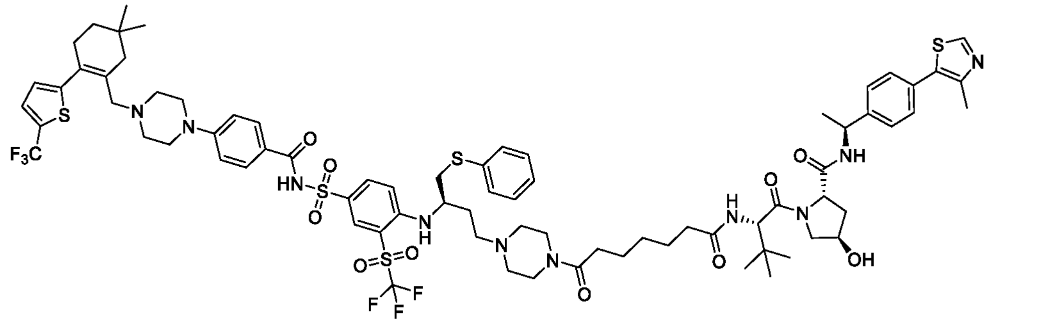 |
| 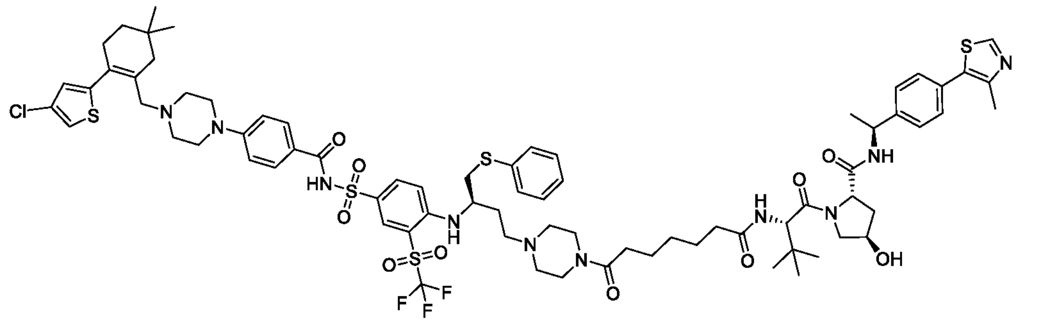 |

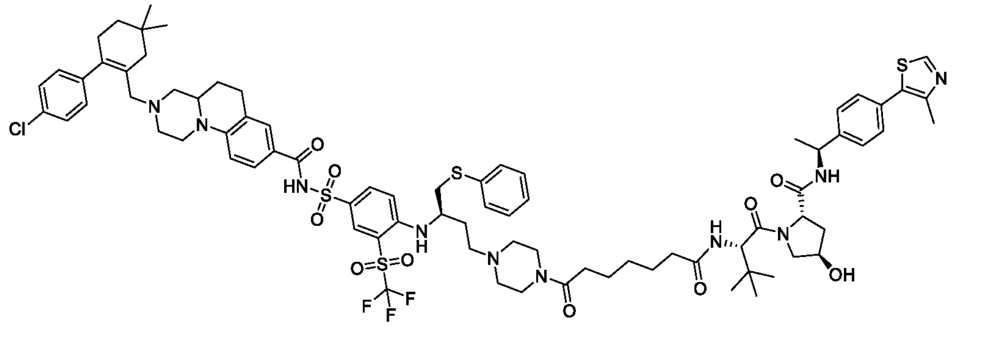

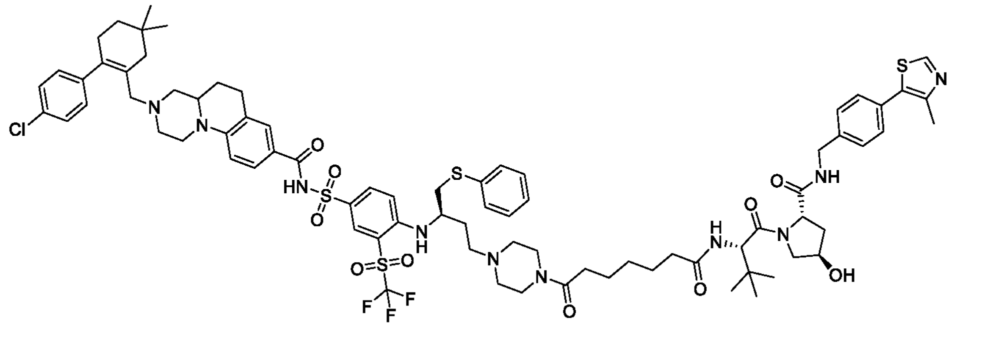

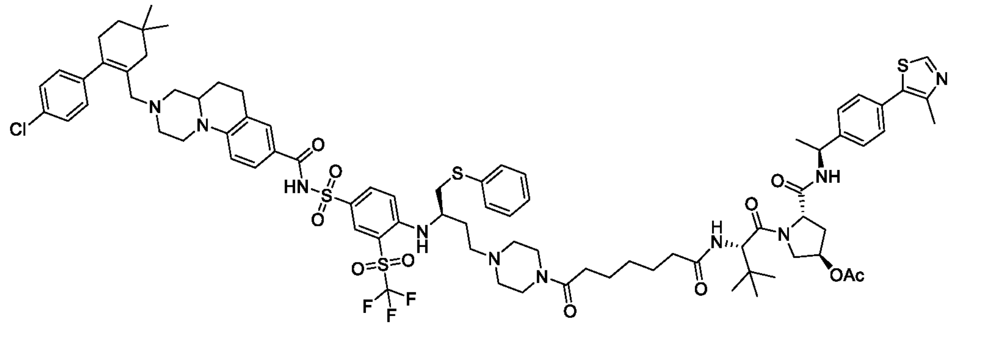

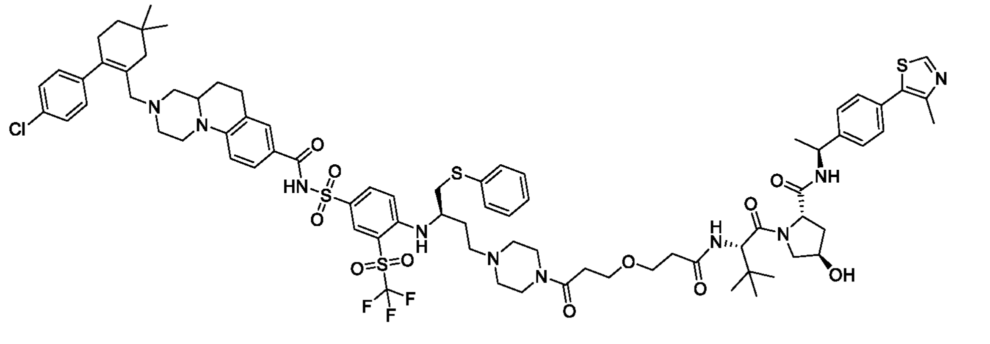

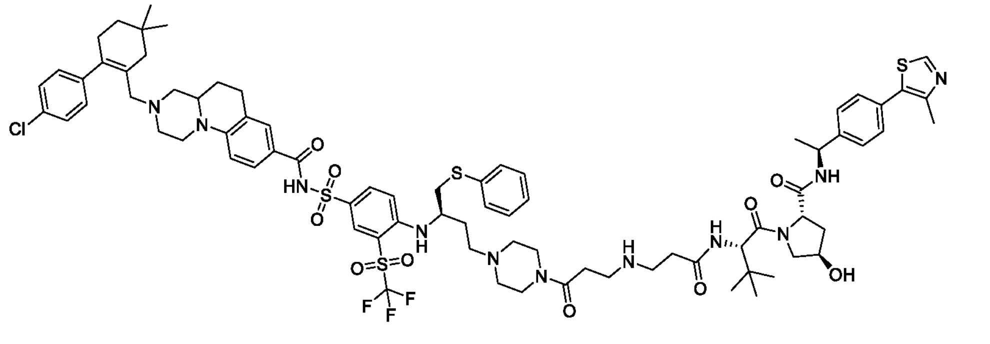

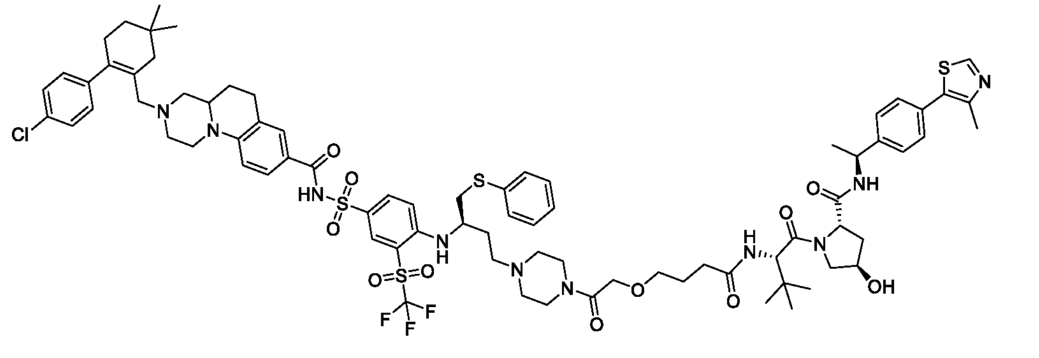

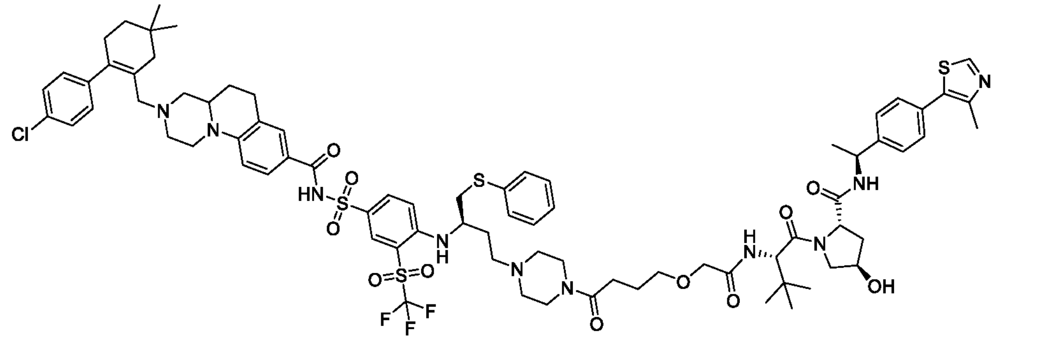

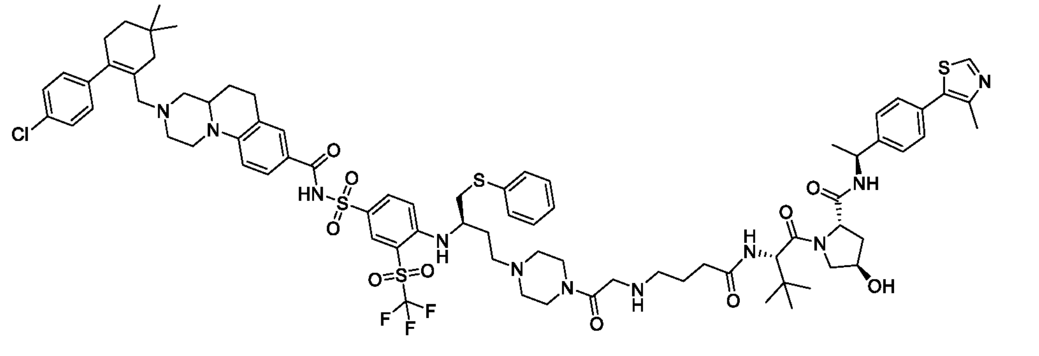

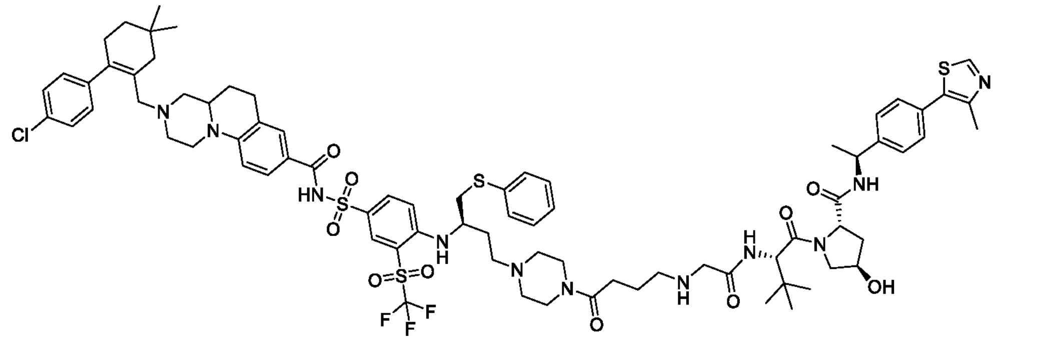

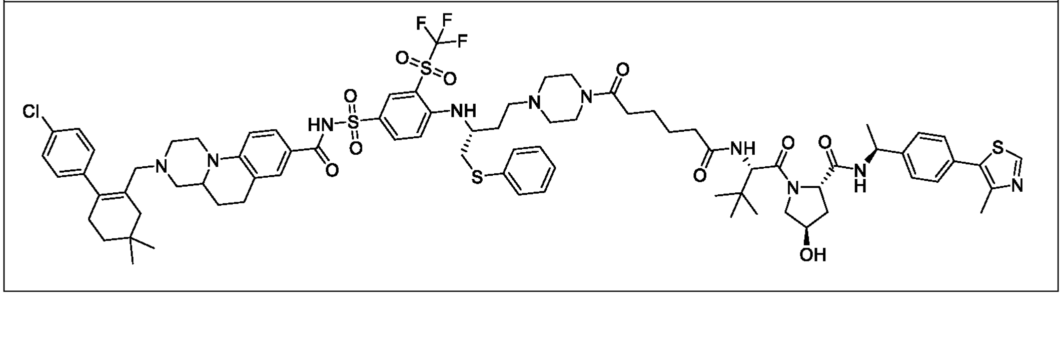

| |
|---|
| 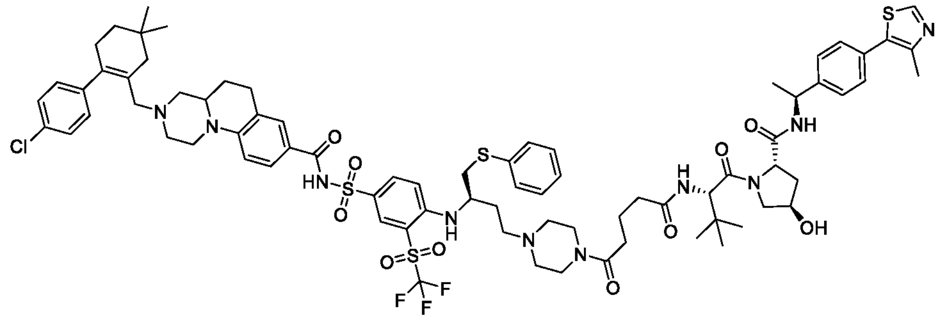 |
| 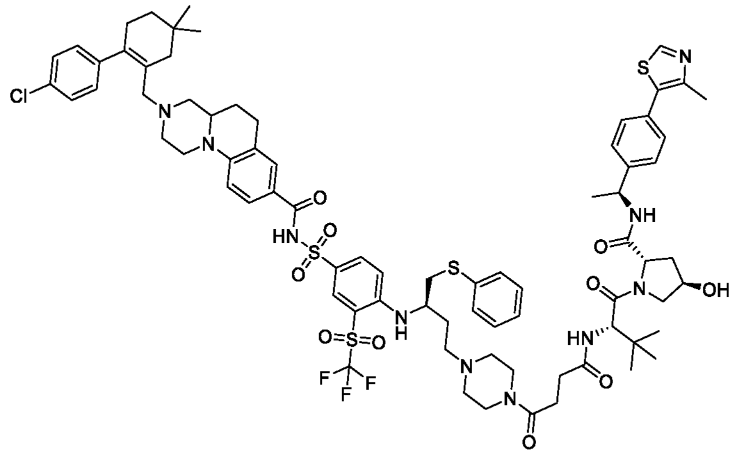 |
| 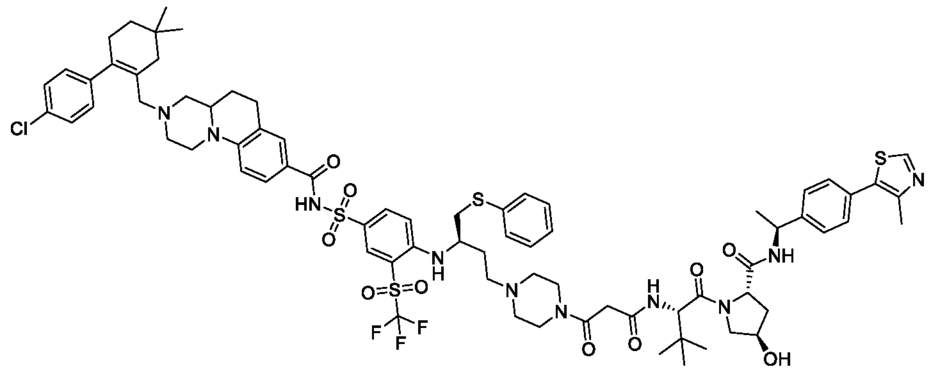 |
| 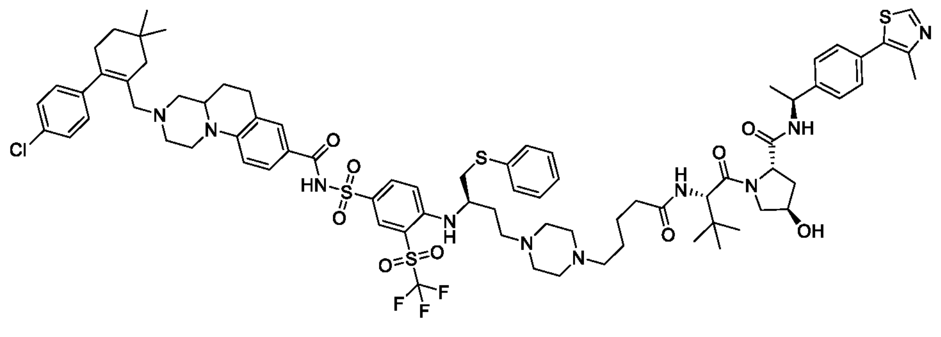 |

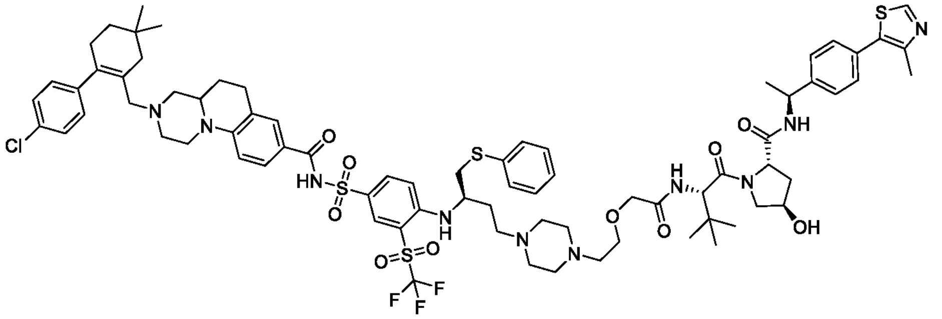

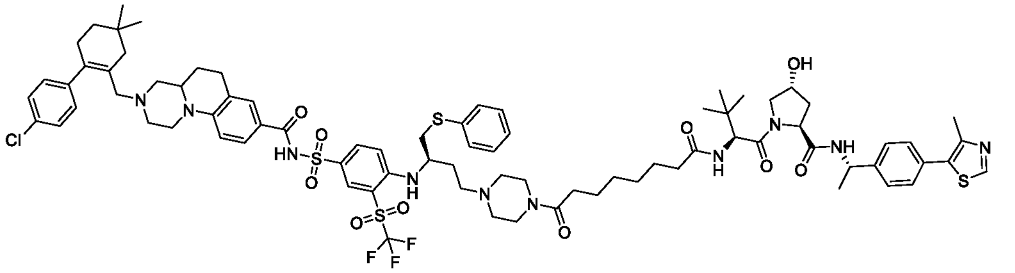

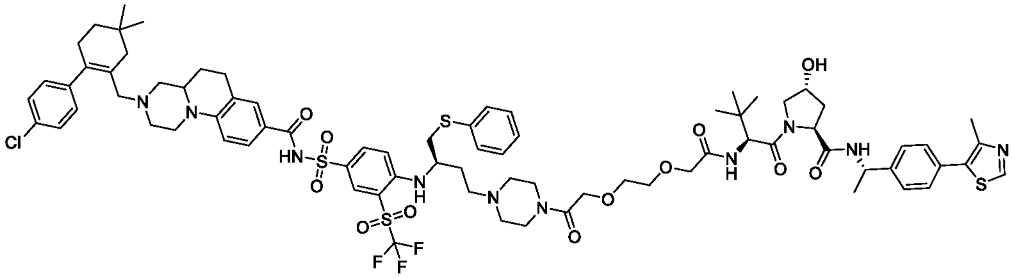

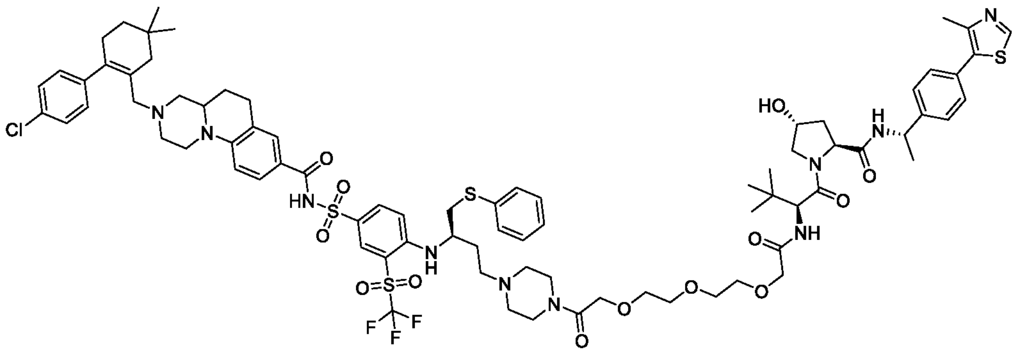

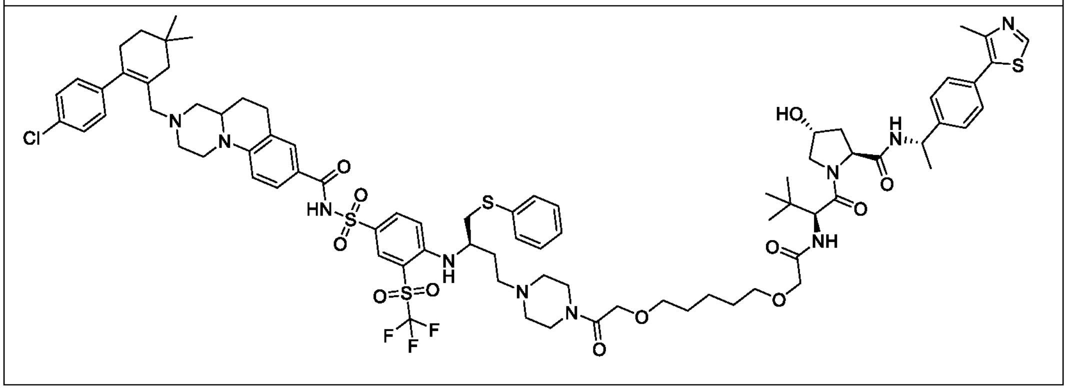

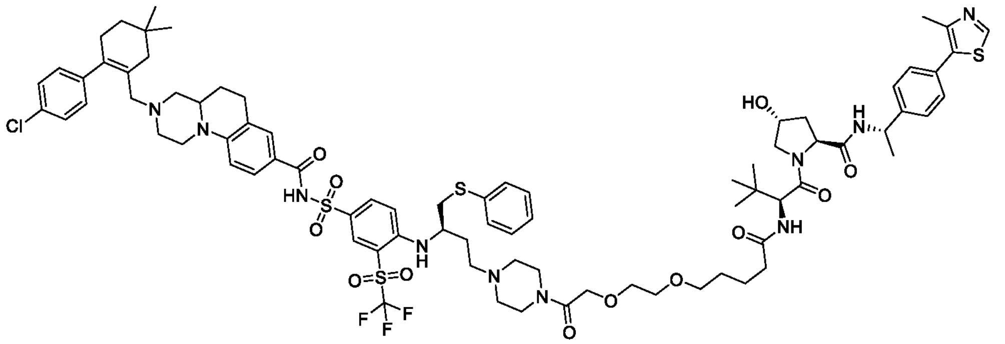

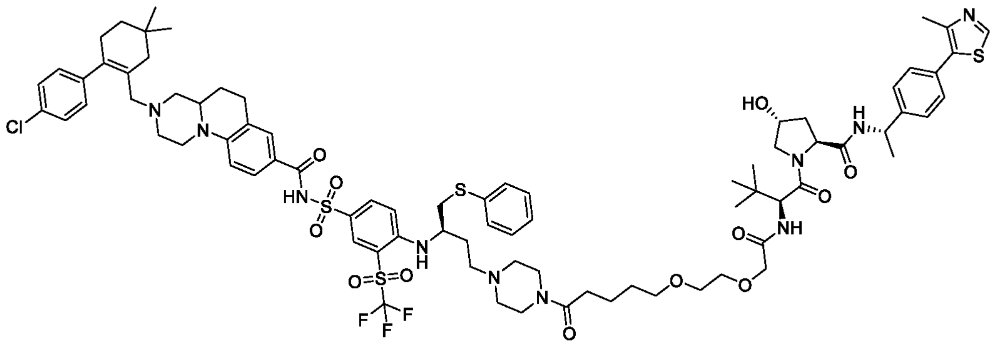

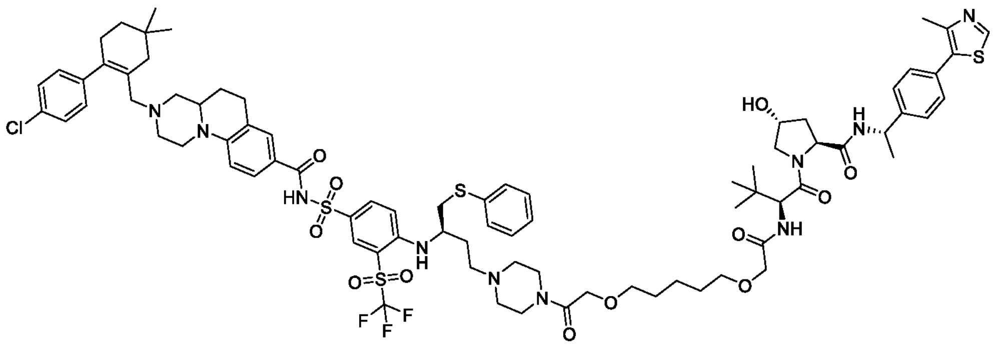

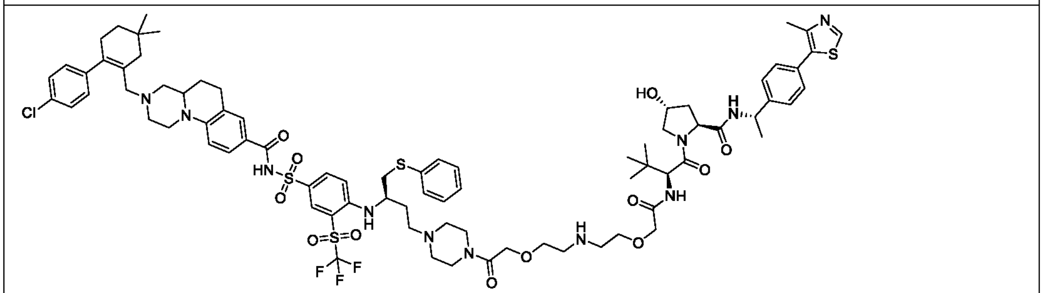

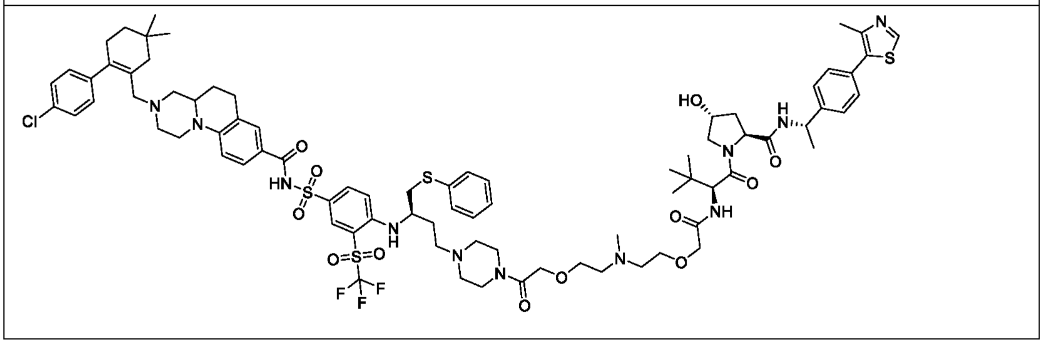

| |
|---|
| 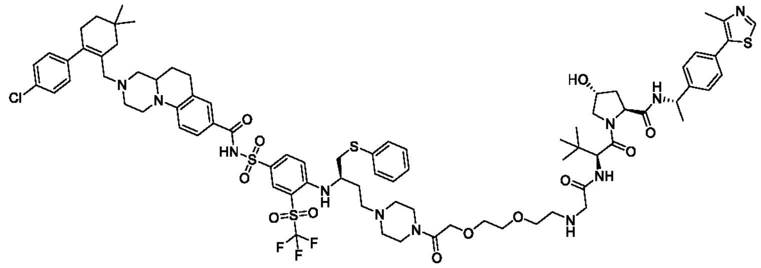 |
| 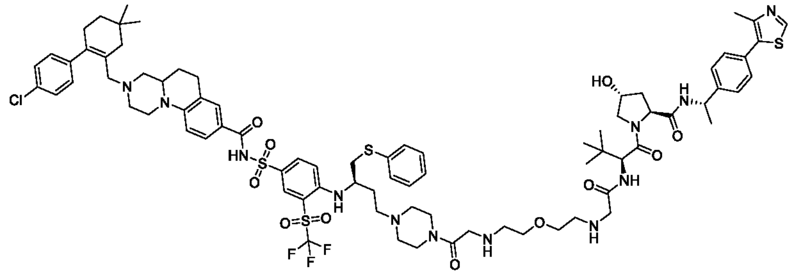 |
| 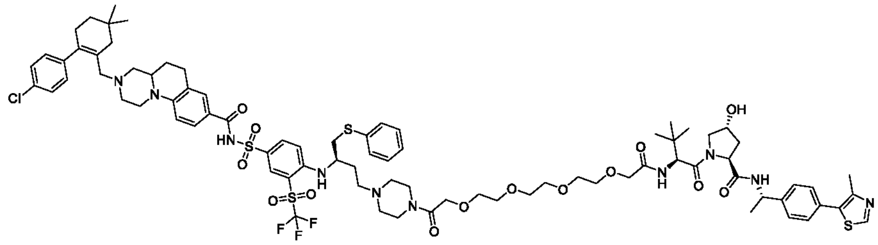 |
| 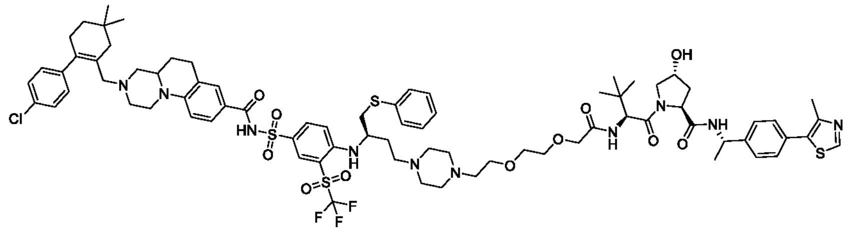 |
| 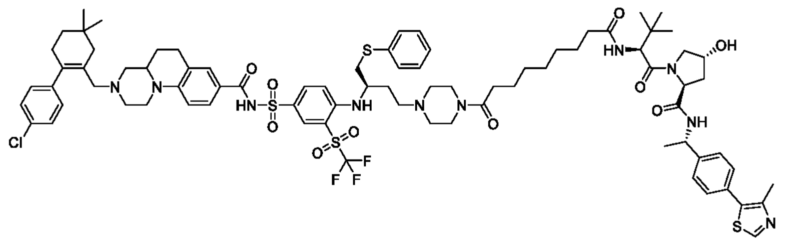 |
| 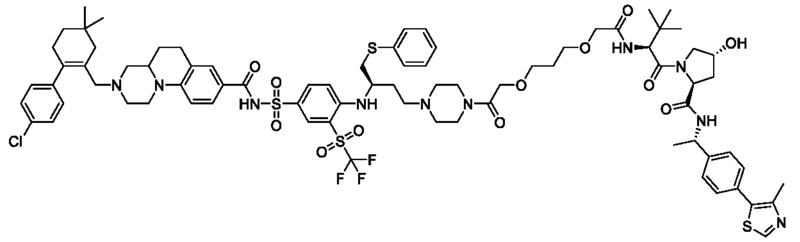 |

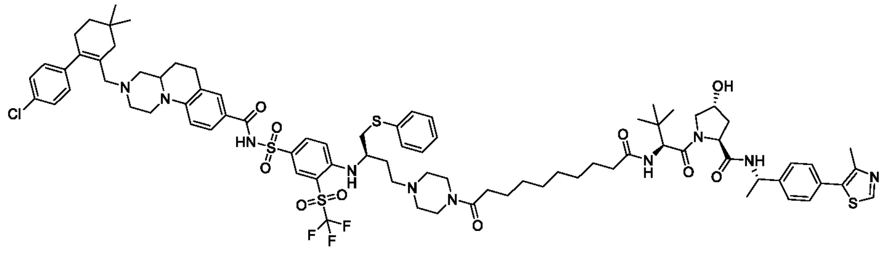

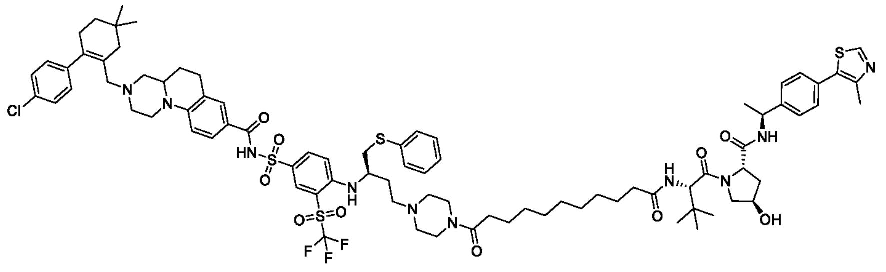

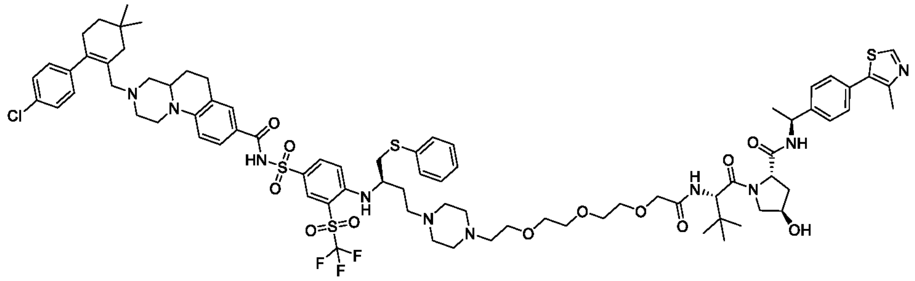

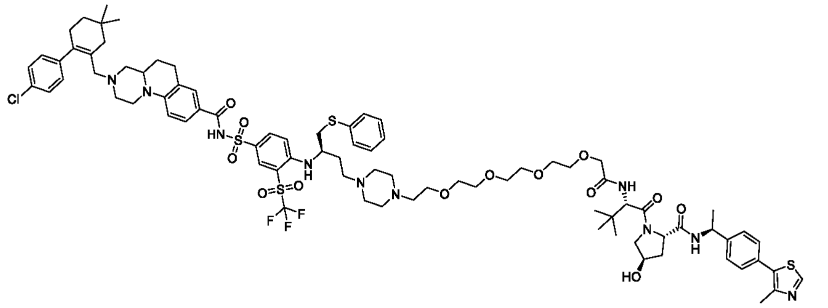

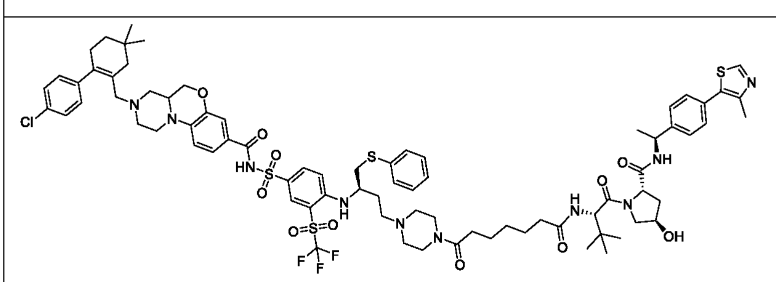

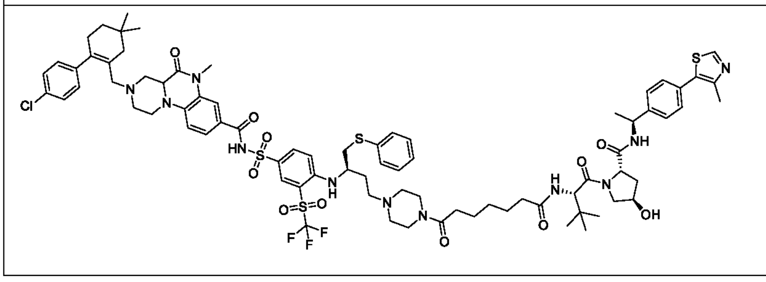

| |
|---|
| 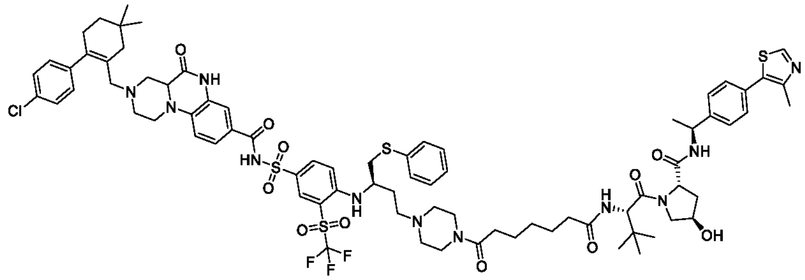 |
| 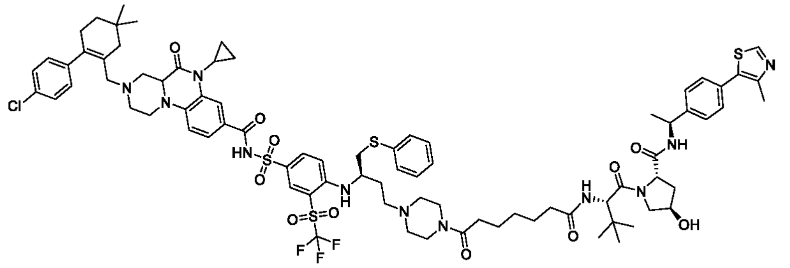 |
| 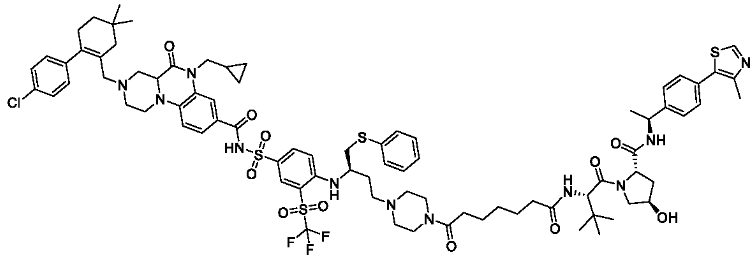 |
| 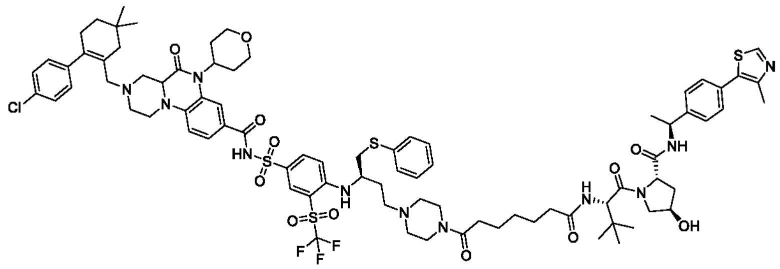 |
| 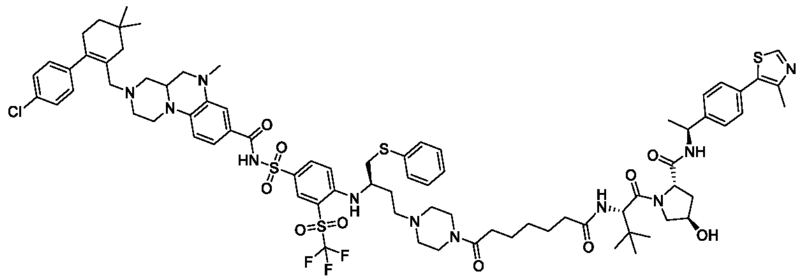 |
| 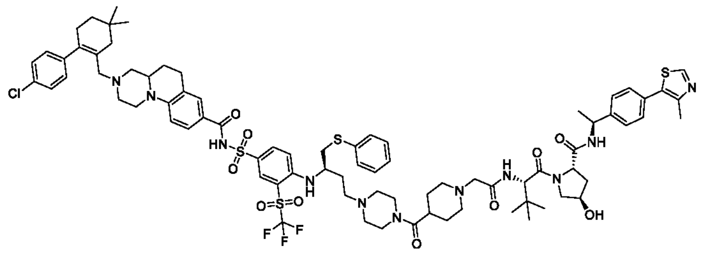 |

| |
|---|
| 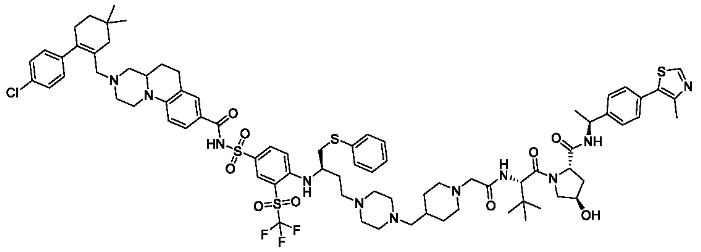 |
| 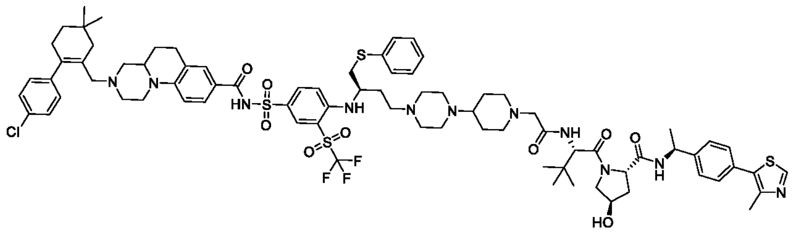 |
| 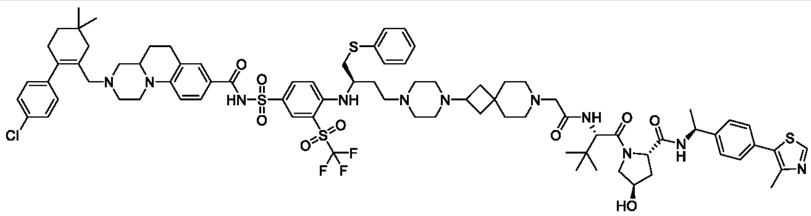 |
| 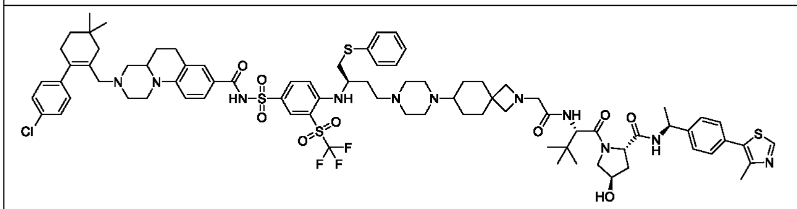 |
| 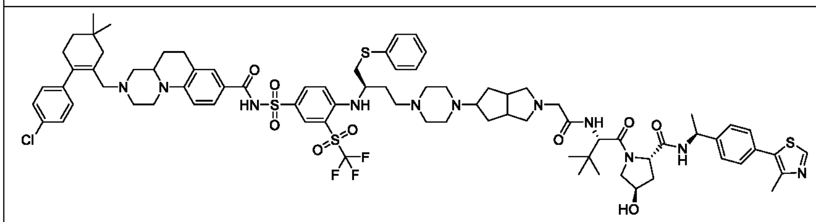 |
| 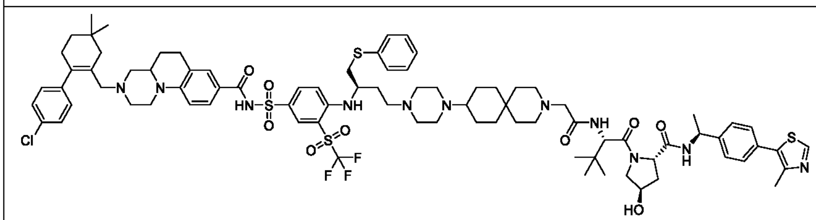 |
| 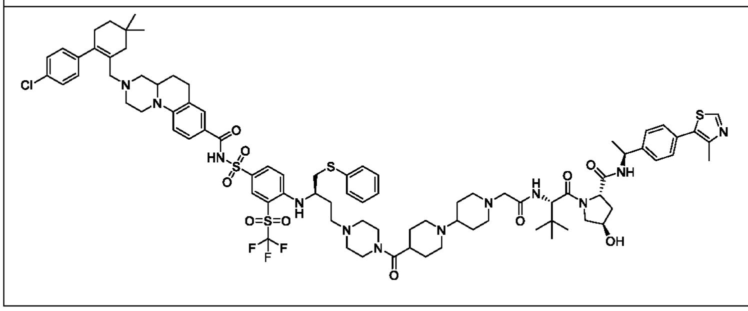 |

| |
|---|
| 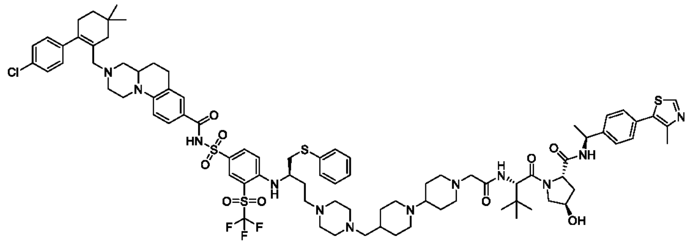 |
| 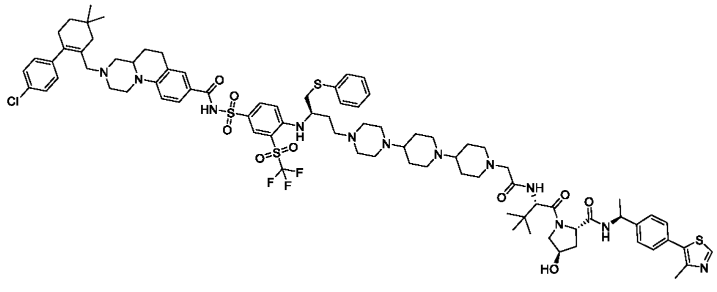 |
| 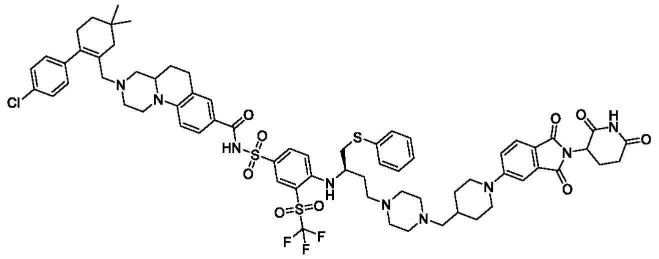 |
| 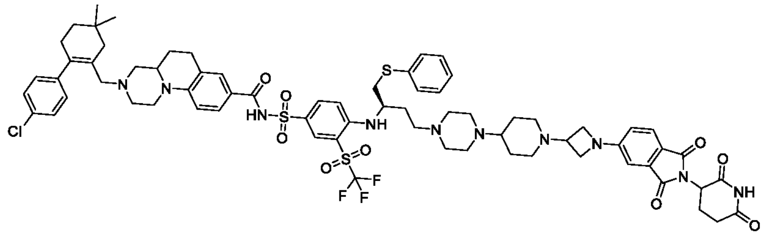 |
| 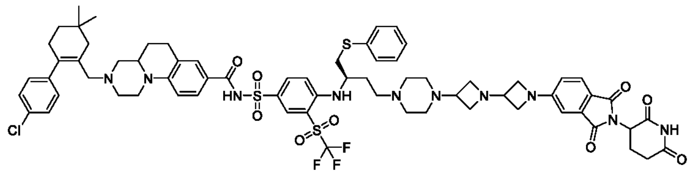 |
| 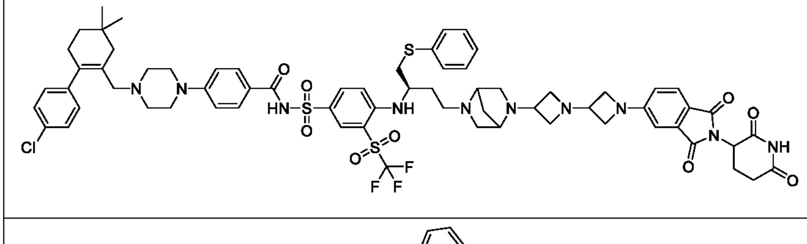 |
| 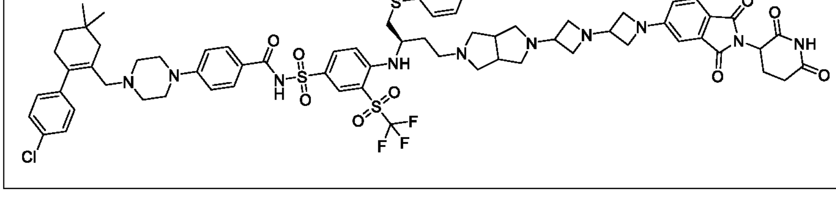 |

| |
|---|
| 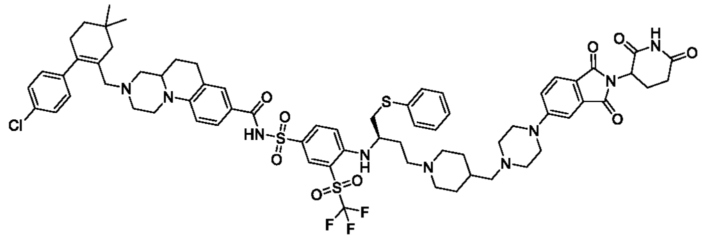 |
| 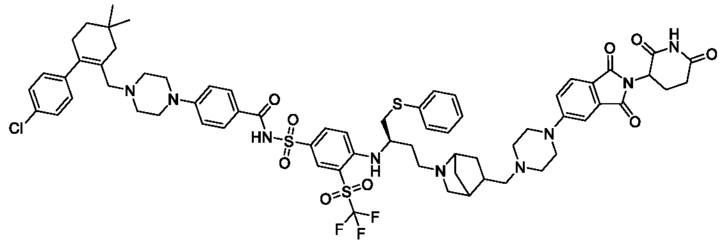 |
| 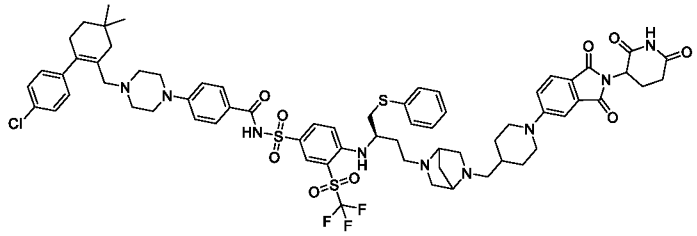 |
| 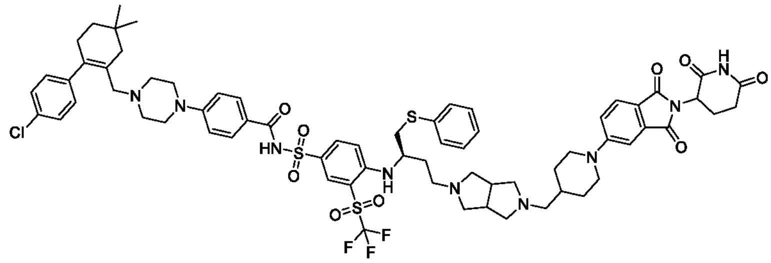 |
| 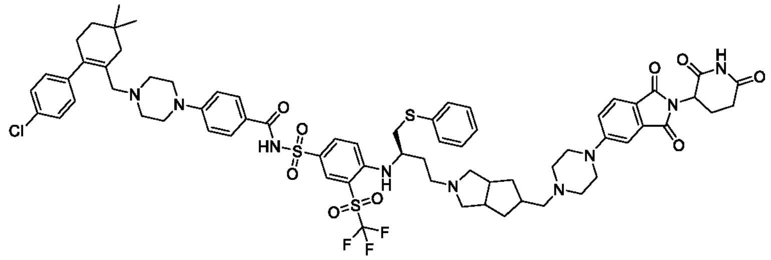 |
| 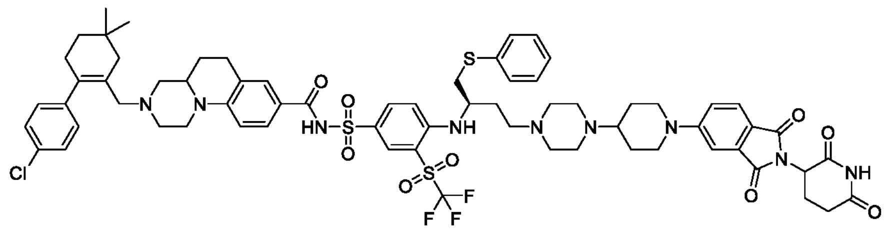 |

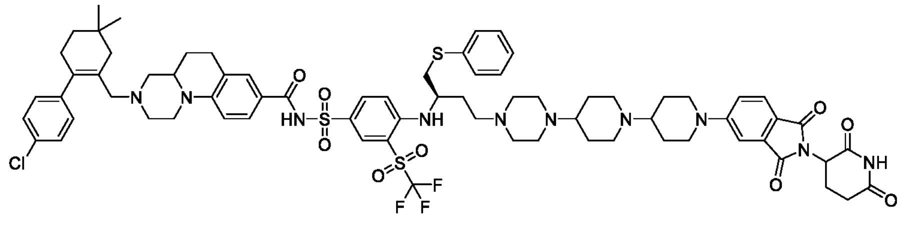

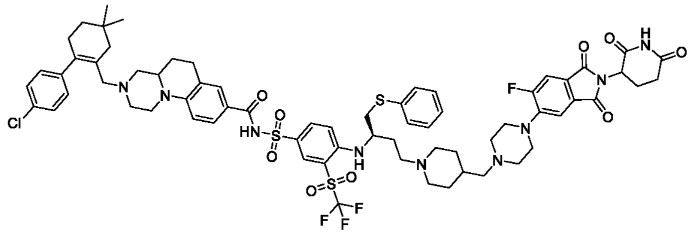

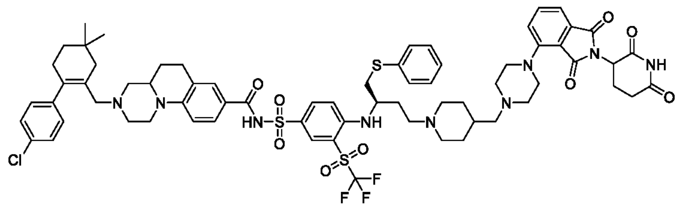

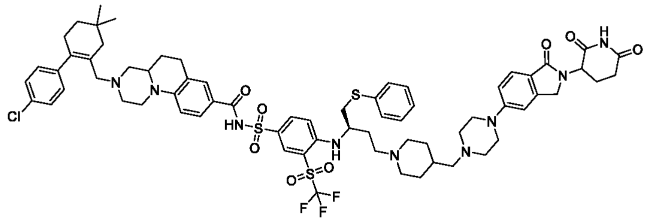

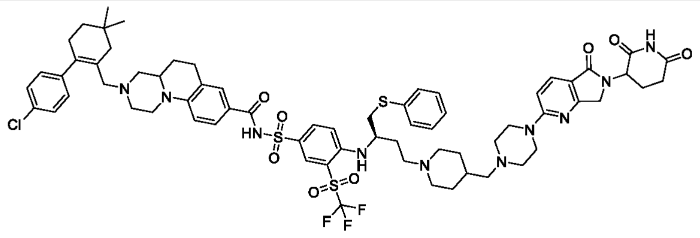

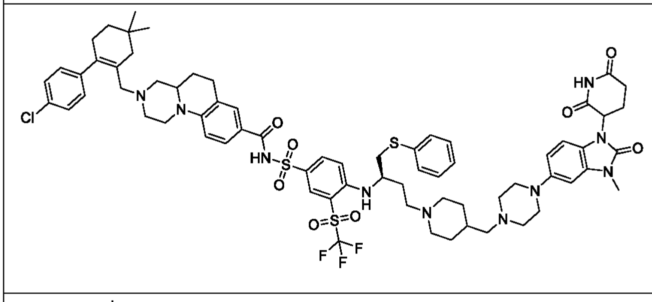

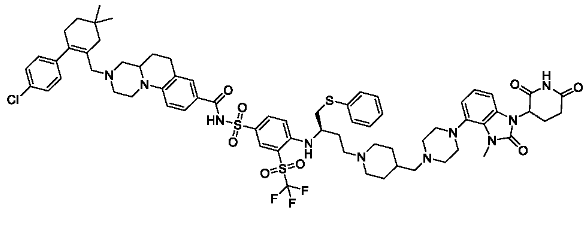

| |
|---|
| 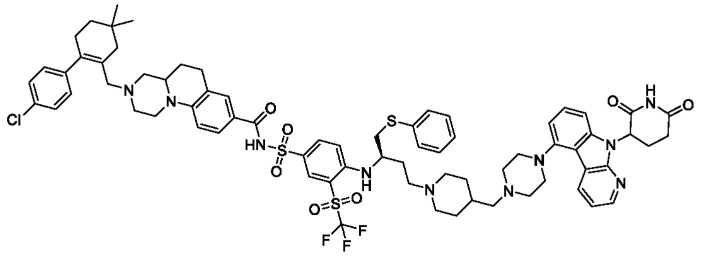 |
| 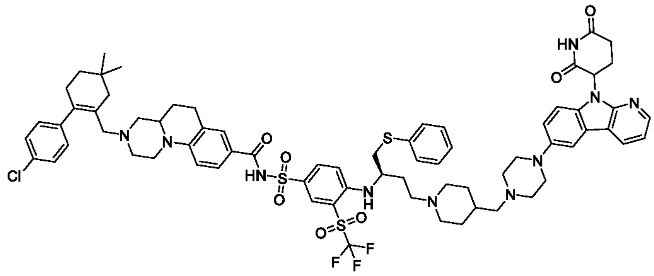 |
| 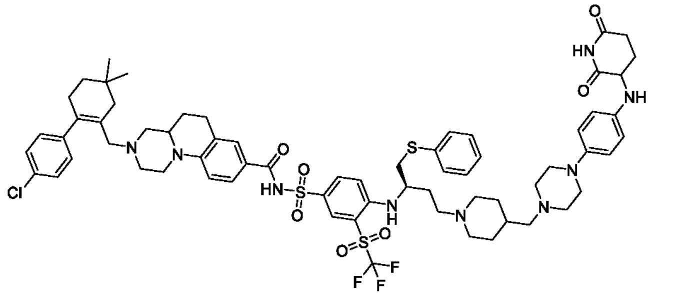 |
| 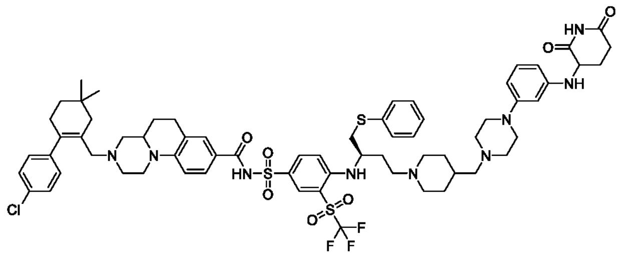 |
| 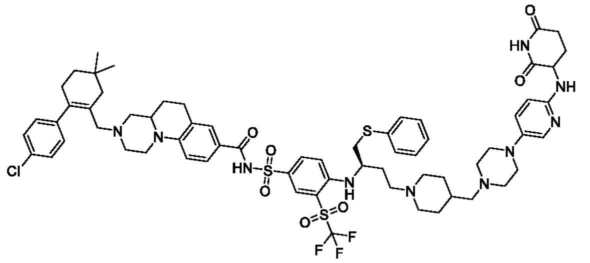 |
| 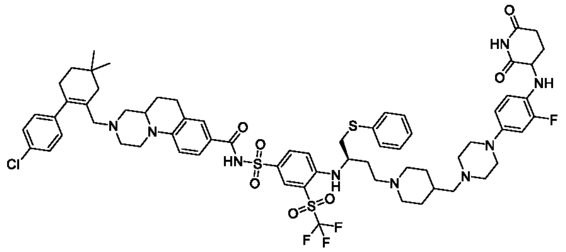 |

| |
|---|
| 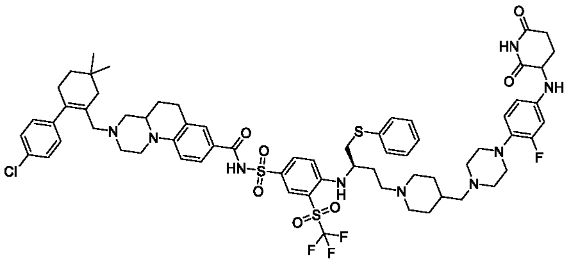 |
| 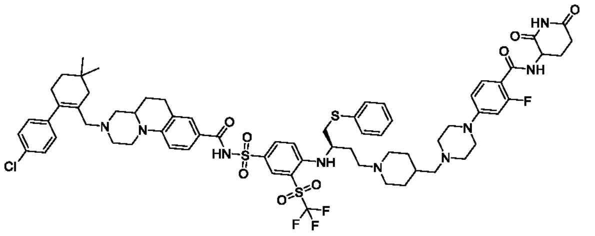 |
| 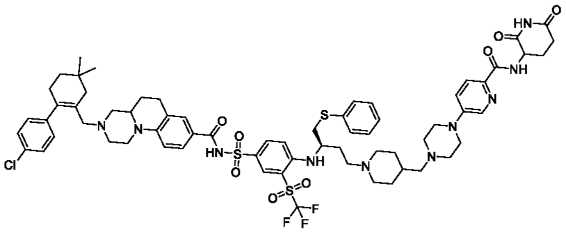 |
| 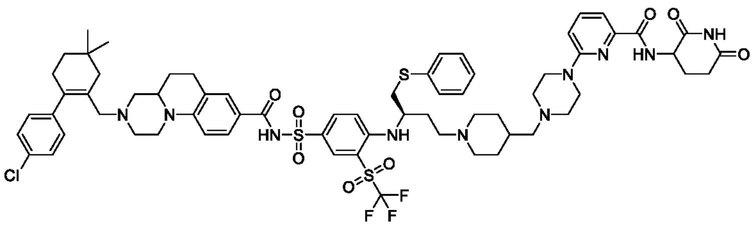 |
| 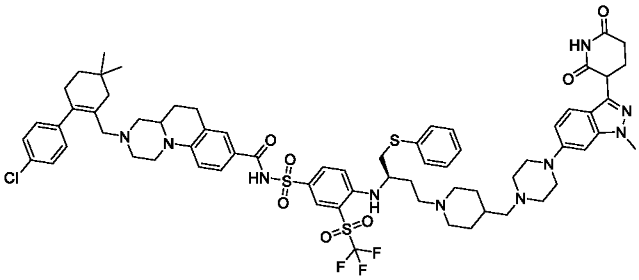 |
| 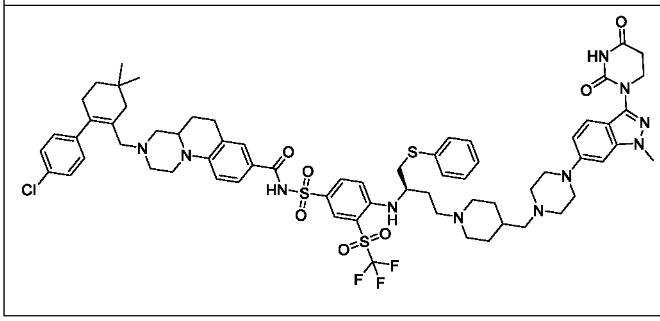 |

| |
|---|
| 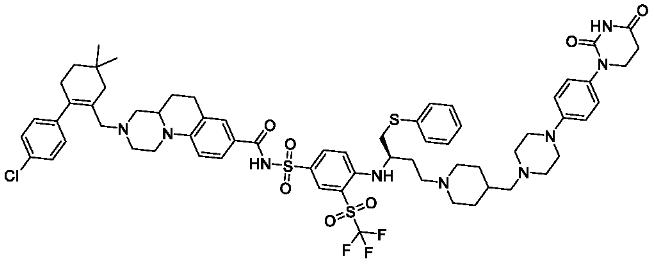 |
| 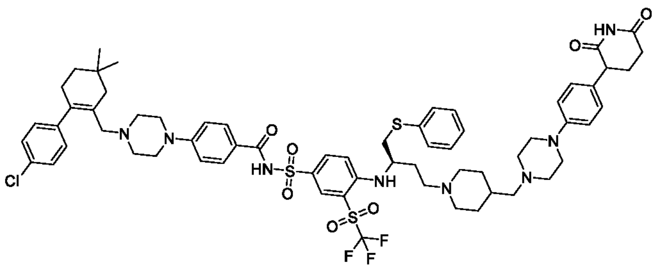 |
| 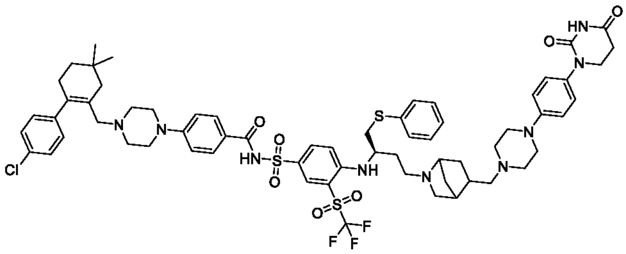 |
| 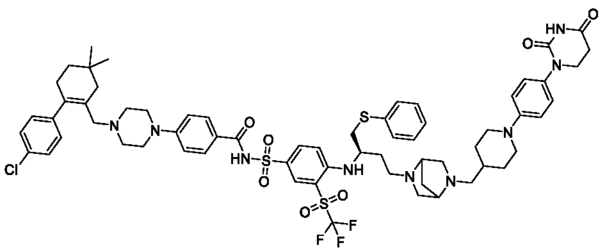 |
| 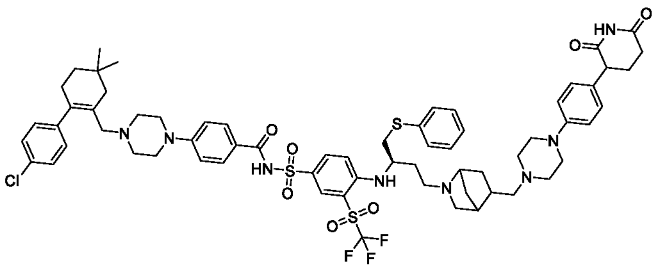 |
| 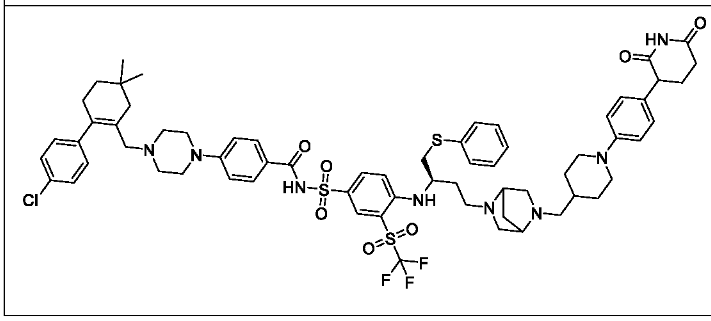 |

| |
|---|
| 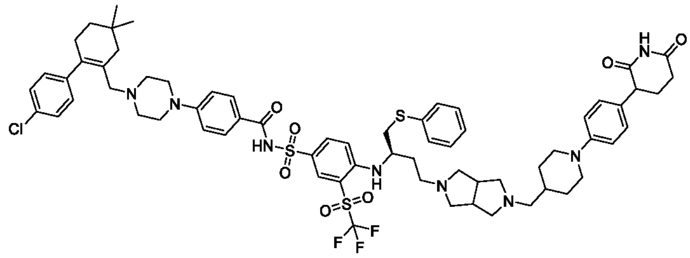 |
| 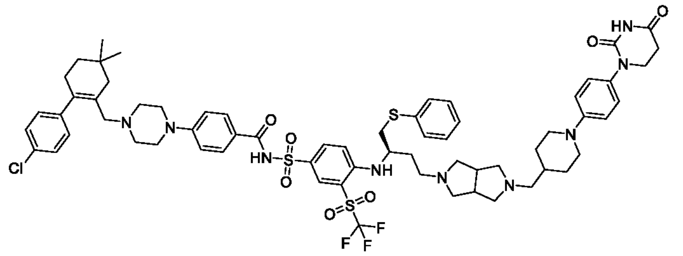 |
| 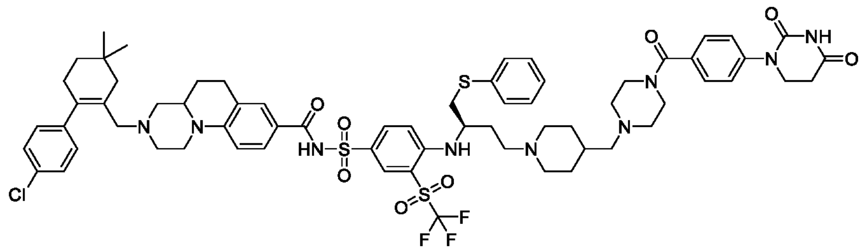 |
| 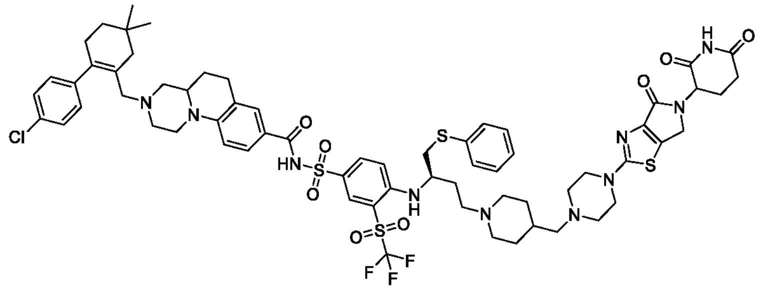 |
| 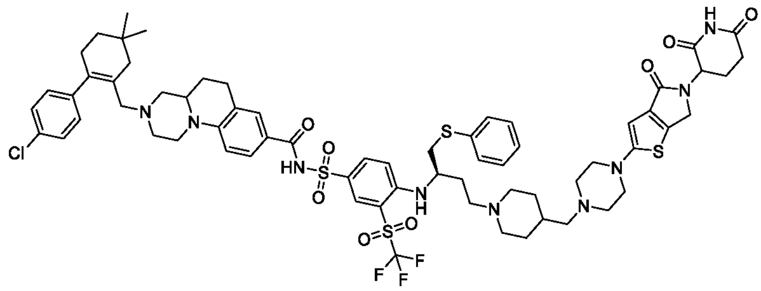 |
| 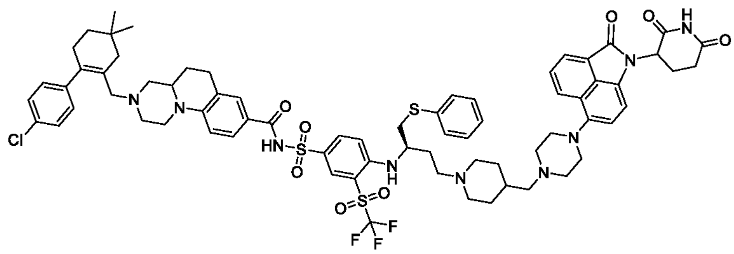 |

| |
|---|
| 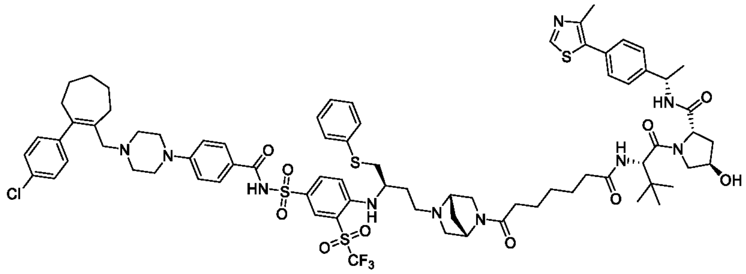 |
| 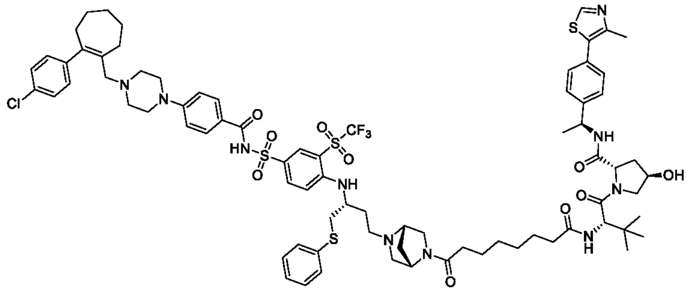 |
| 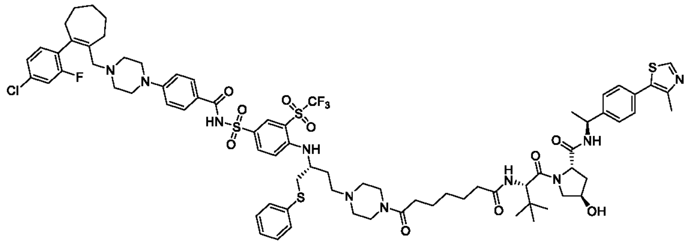 |
| 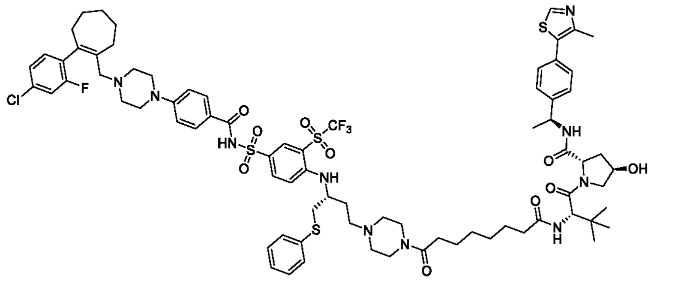 |
| 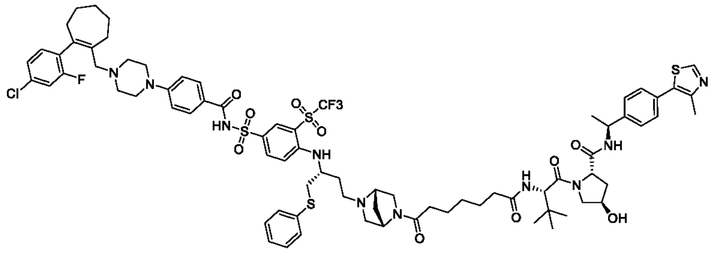 |
| 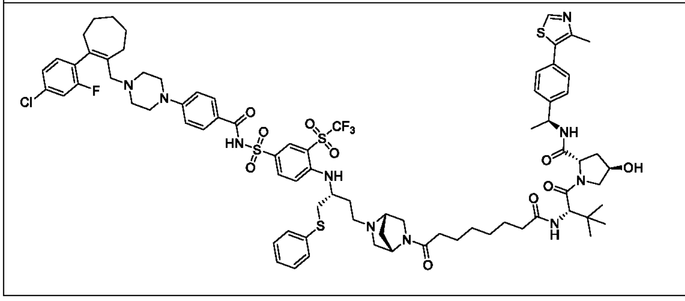 |

| |
|---|
| 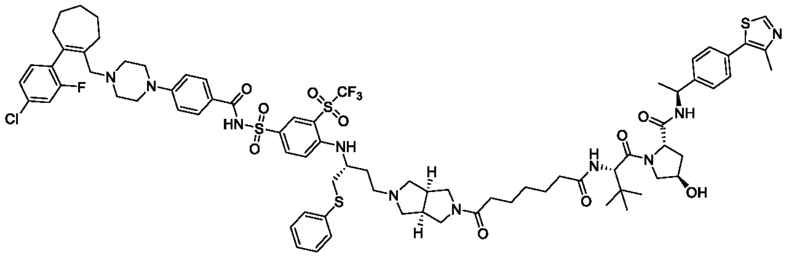 |
| 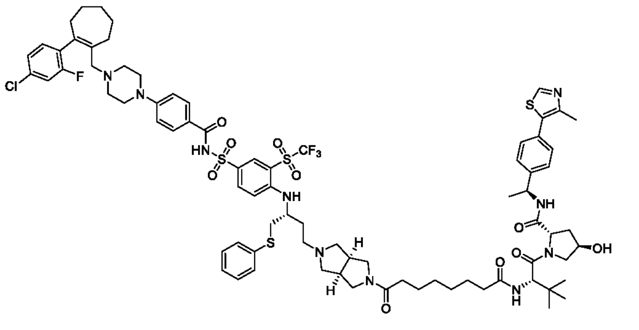 |
| 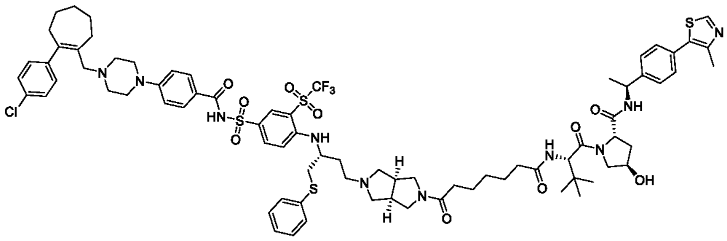 |
| 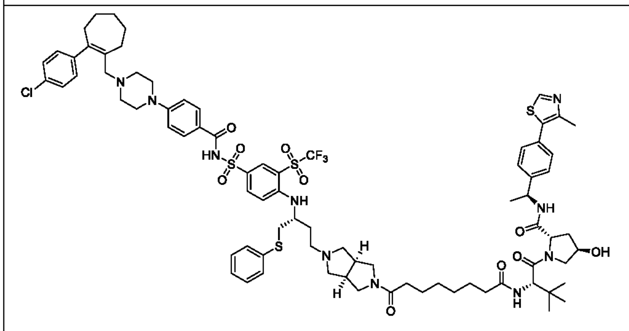 |
| 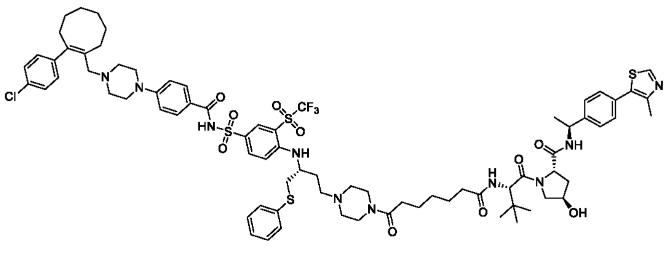 |
| 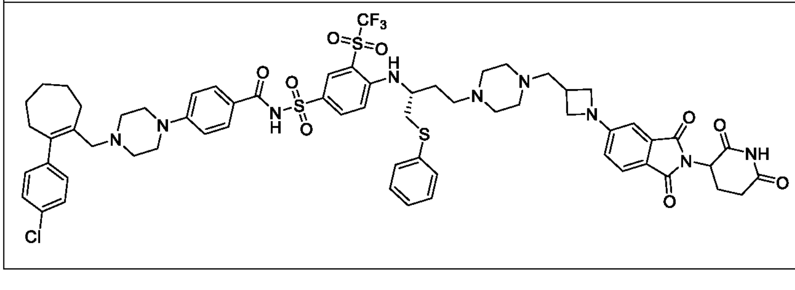 |

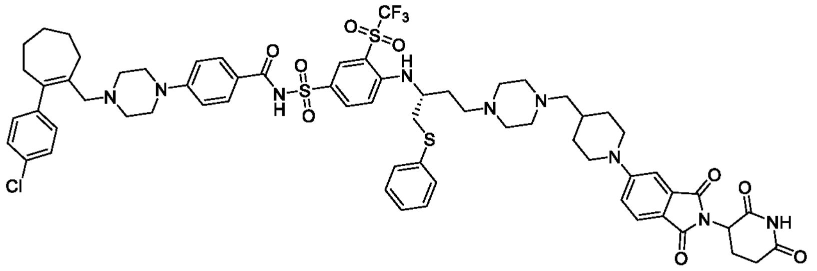

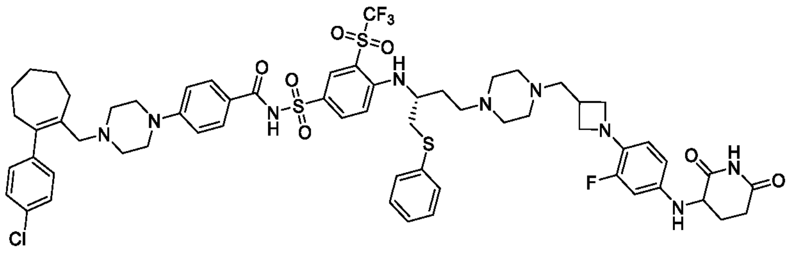

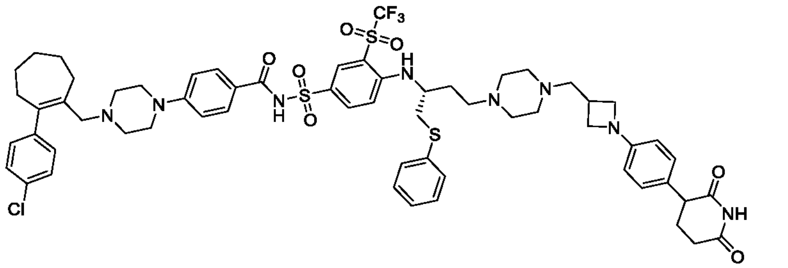

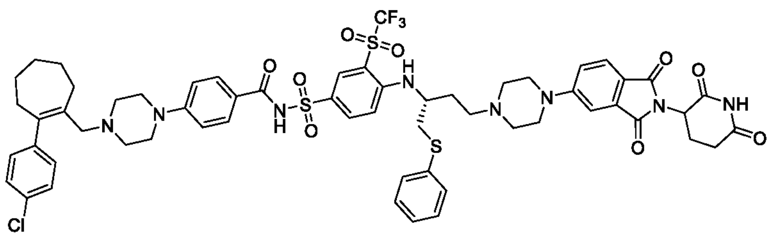

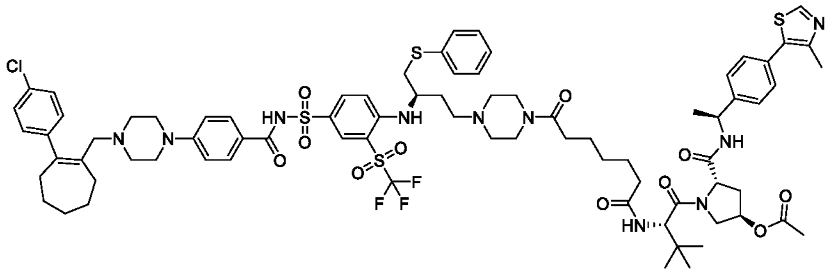

Table B-1 L group

| | | | |
|---|---|---|---|
| 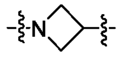 | 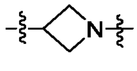 | 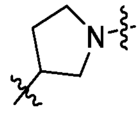 | 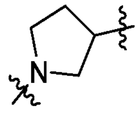 |
| 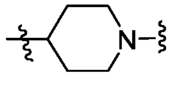 | 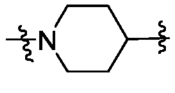 | 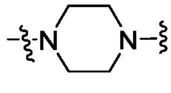 | 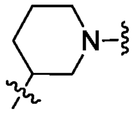 |

| | | | |
|---|---|---|---|
| 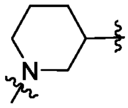 | 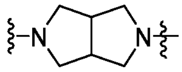 | 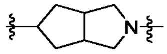 | 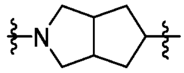 |
| 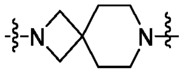 | 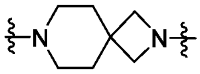 | 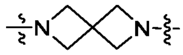 | 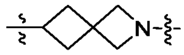 |
| 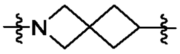 | 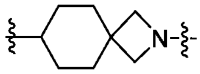 | 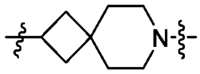 | 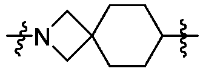 |
| 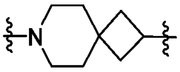 | 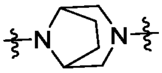 | 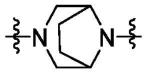 | 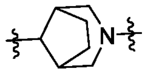 |
| 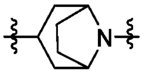 | 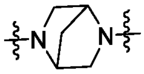 | 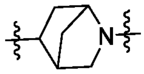 | 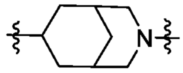 |
| 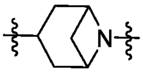 | 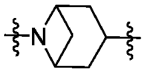 | 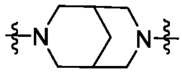 | 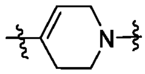 |
| 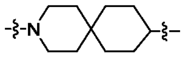 | 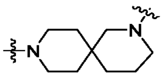 | 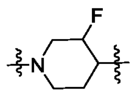 | 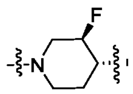 |
| 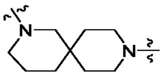 | 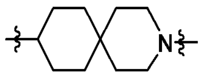 | | |
| 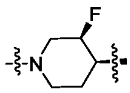 | 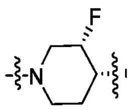 | 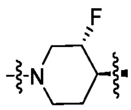 | 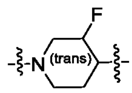 |
| 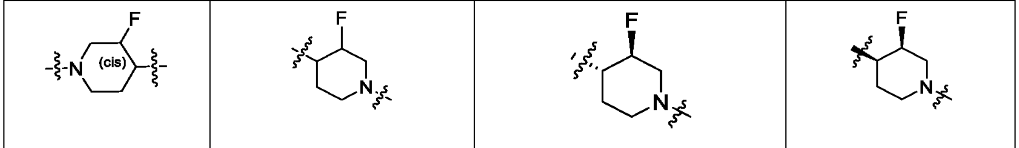 | | | |
| 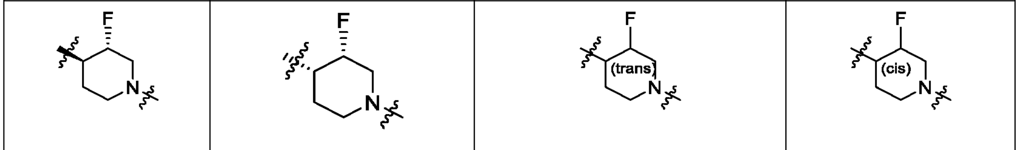 | | | |
| | 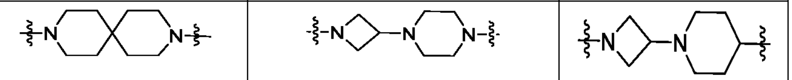 | | |
| 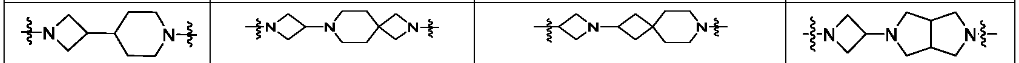 | | | |
| 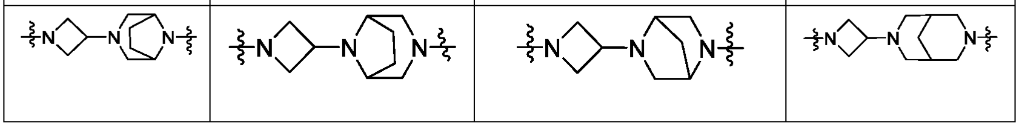 | | | |

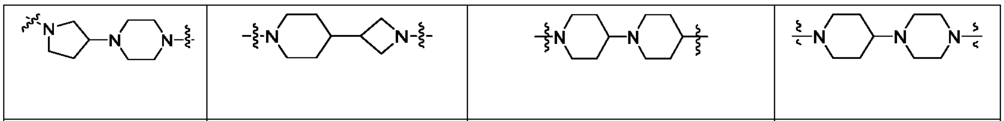
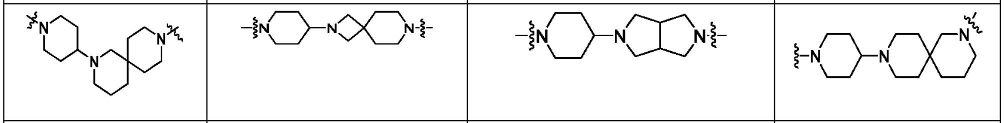
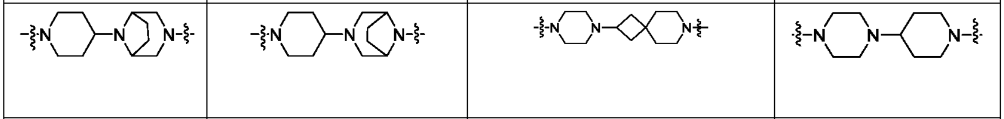
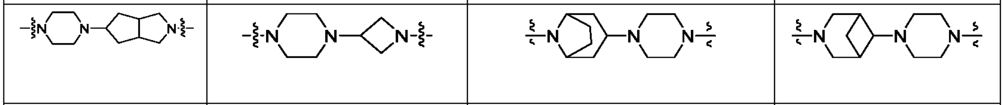
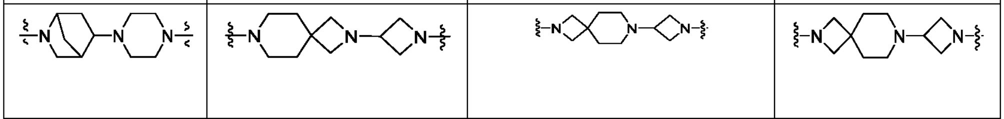
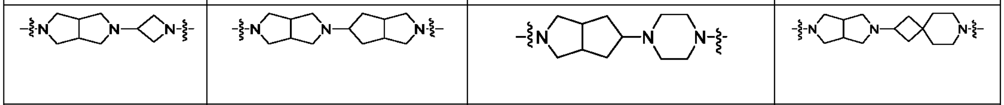
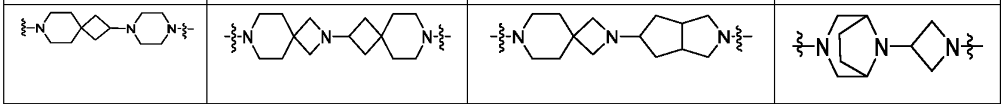
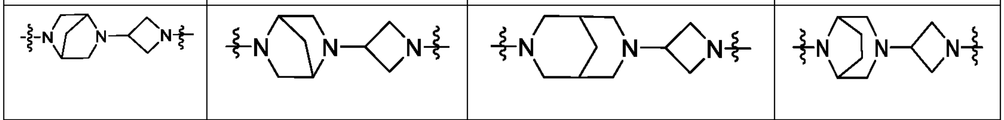
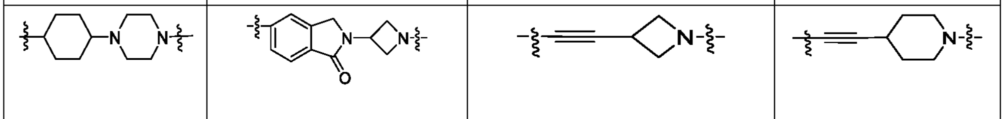
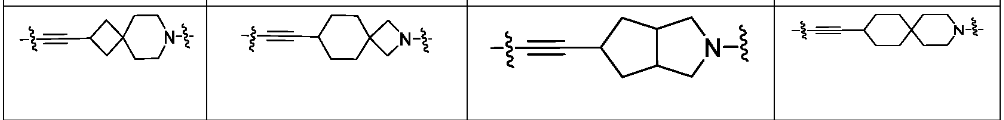
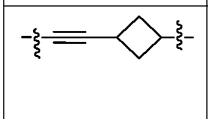
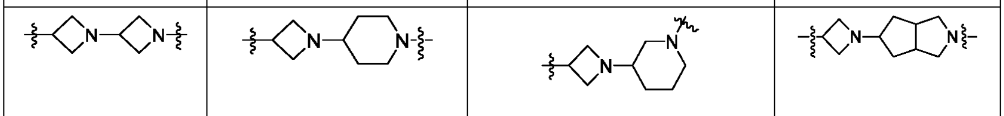
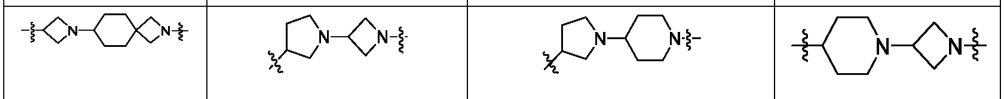
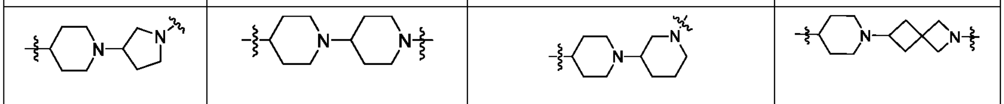
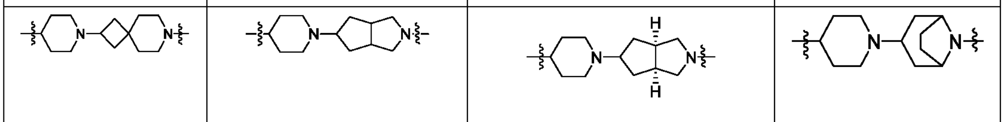
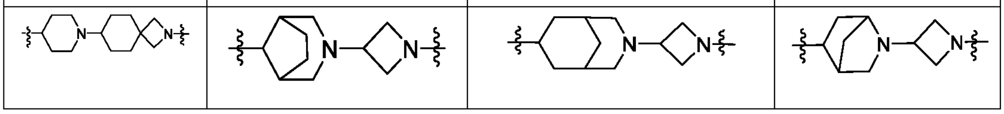

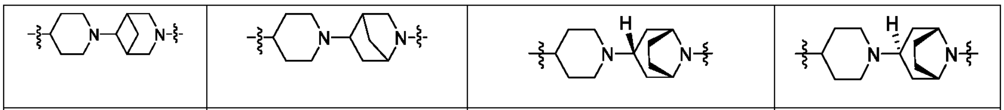

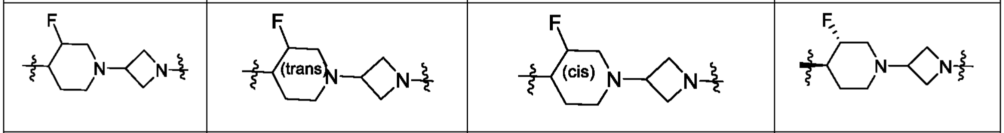

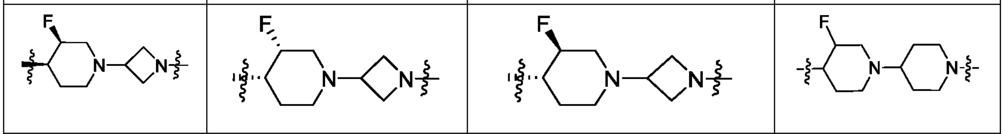

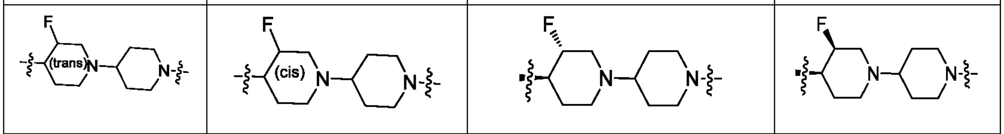

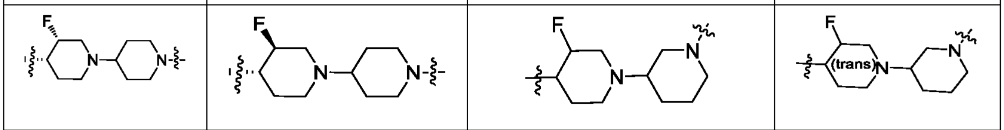

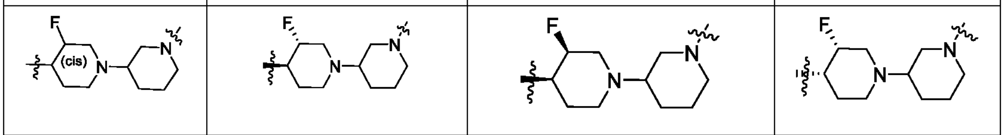

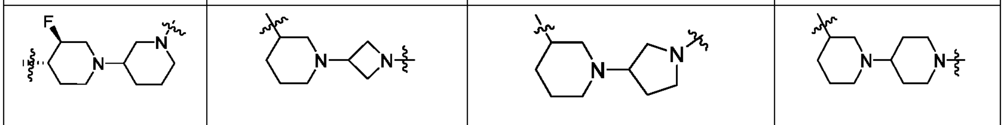

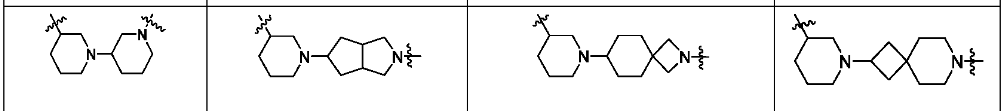

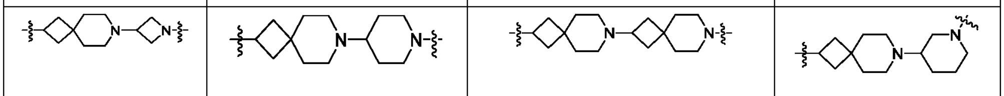

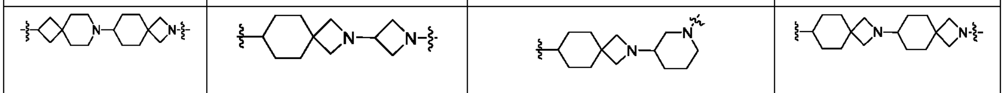

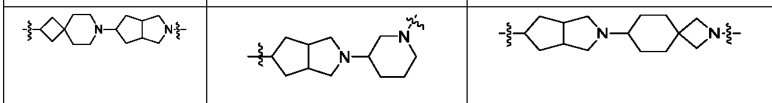

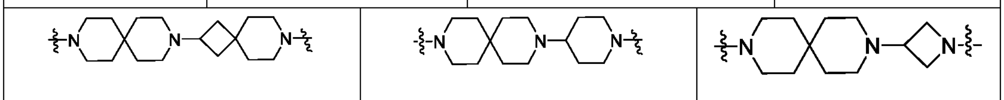

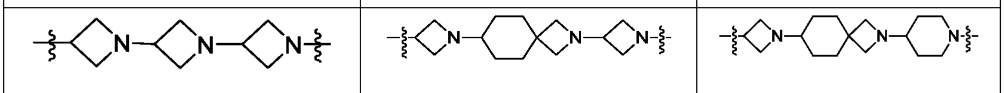

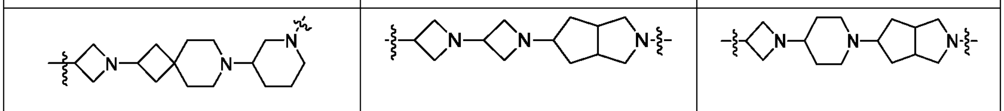

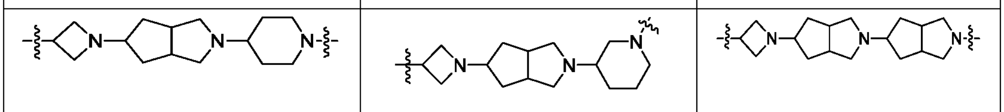

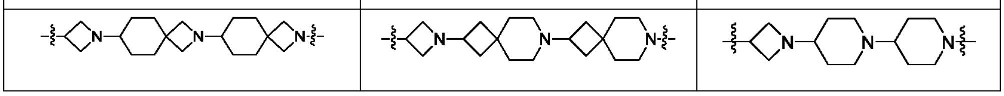

| | | |
|---|---|---|
| 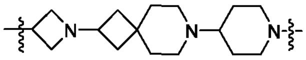 | 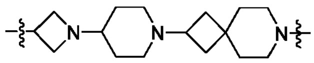 | 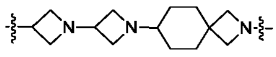 |
| 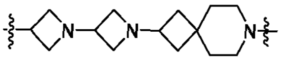 | 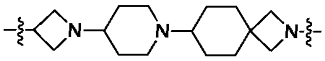 | 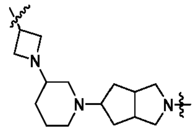 |
| 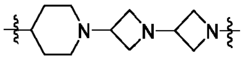 | 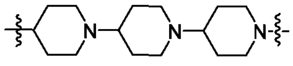 | 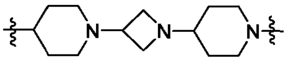 |
| 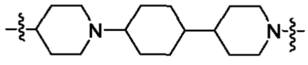 | 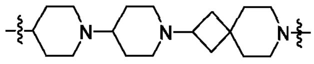 | 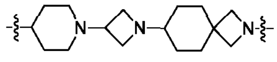 |
| 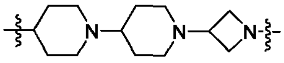 | 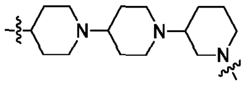 | 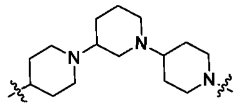 |
| 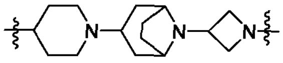 | 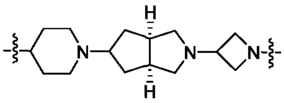 | 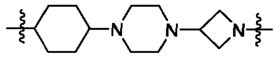 |
| 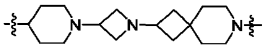 | 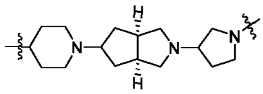 | 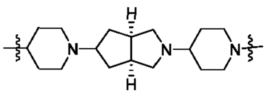 |
| 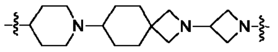 | 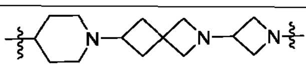 | 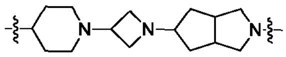 |
| 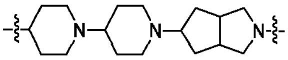 | 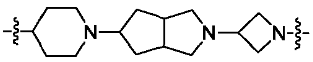 | 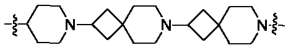 |
| 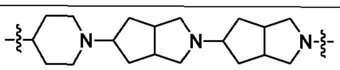 | 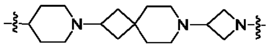 | 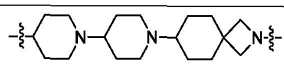 |
| 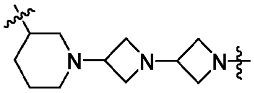 | 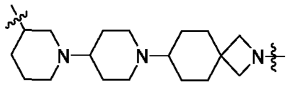 | 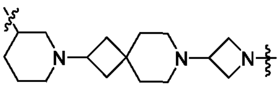 |
| 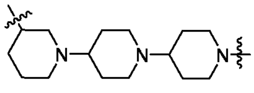 | 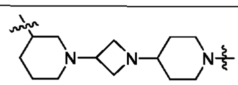 | 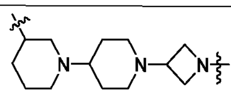 |
| 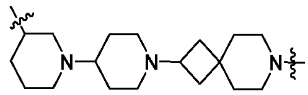 | 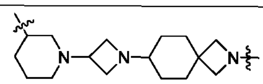 | 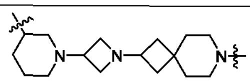 |
| 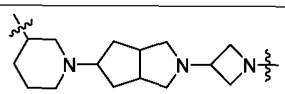 | | |
| | | |
| | | |

| | | |
|---|---|---|
| 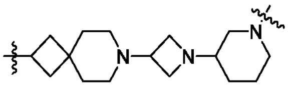 | 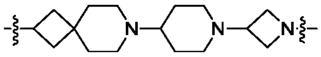 | |
| 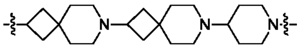 | 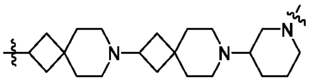 | 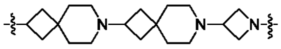 |
| 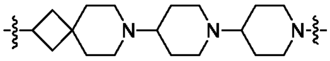 | 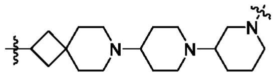 | 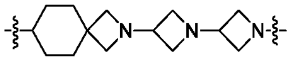 |
| 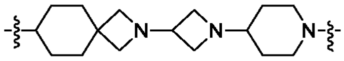 | 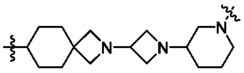 | 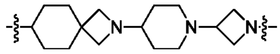 |
| 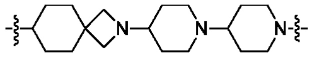 | 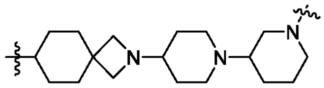 | 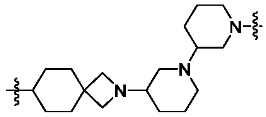 |
| 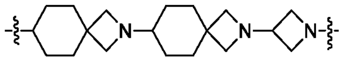 | 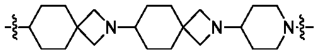 | 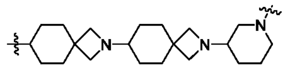 |
| 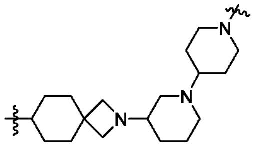 | 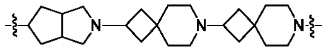 | |
| 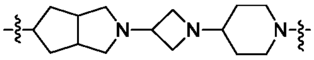 | 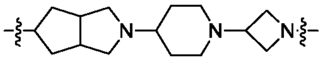 | 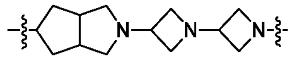 |
| 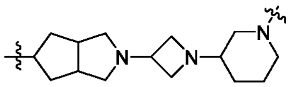 | 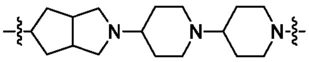 | 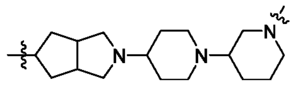 |
| 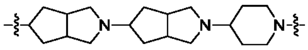 | 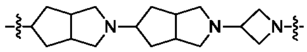 | 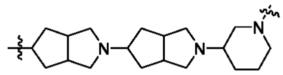 |
| 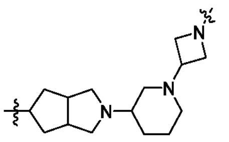 | 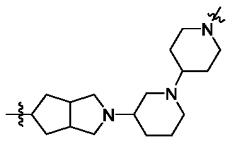 | 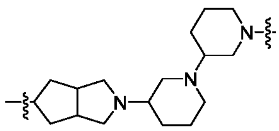 |
| 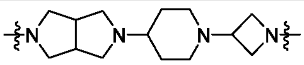 | 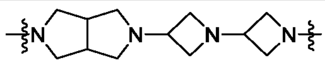 | 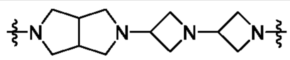 |
| 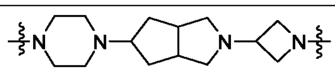 | 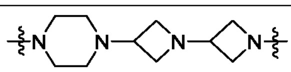 | 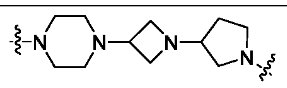 |
| 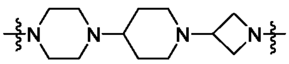 | 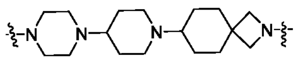 | 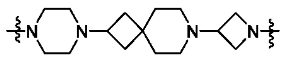 |
| | | |

| | | |
|---|---|---|
| 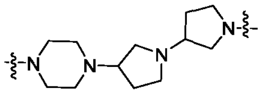 | 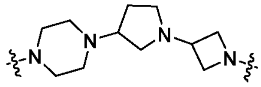 | 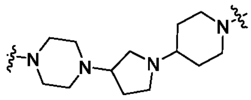 |
| 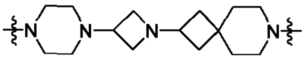 | 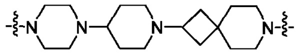 | 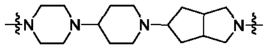 |
| 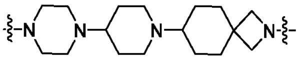 | 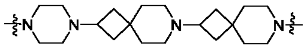 | 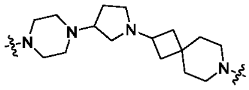 |
| 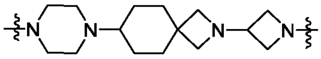 | 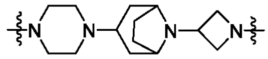 | 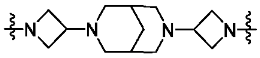 |
| 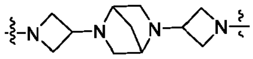 | 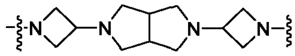 | 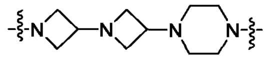 |
| 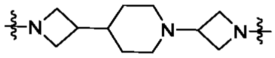 | 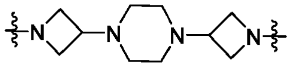 | 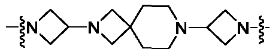 |
| 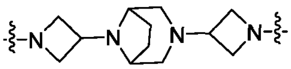 | 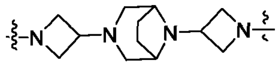 | 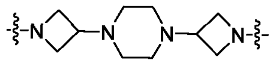 |
| 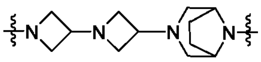 | 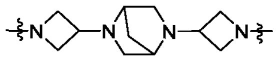 | 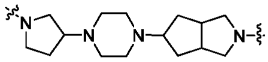 |
| 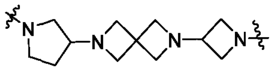 | 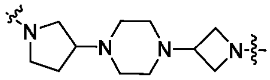 | |
| 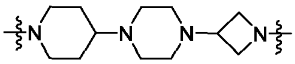 | 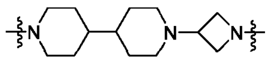 | 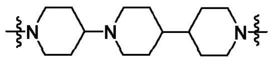 |
| 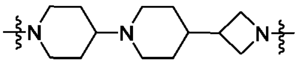 | 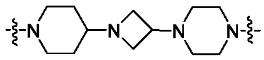 | 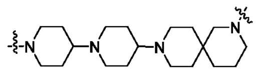 |
| 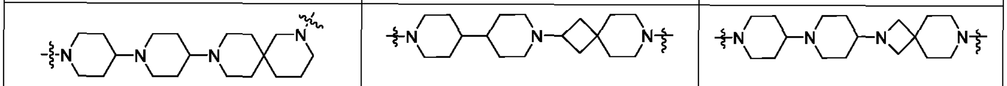 | | |
| 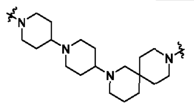 | | |
| 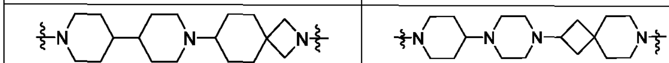 | | |
| 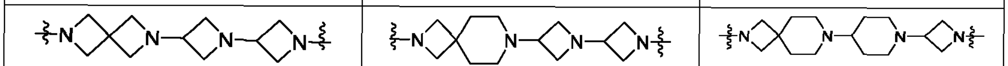 | | |
| 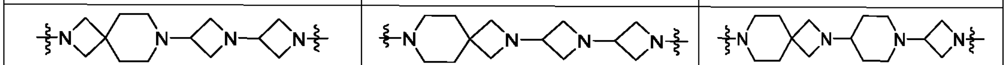 | | |
| 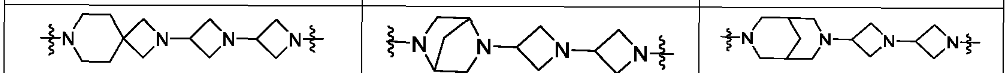 | | |
| 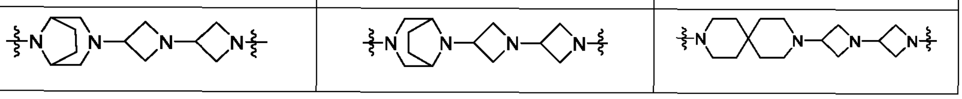 | | |

| | | | |
|---|---|---|---|
| 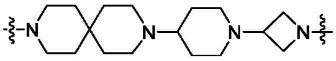 | 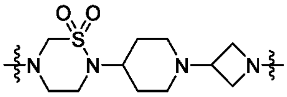 | 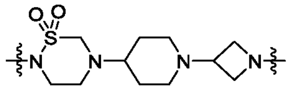 | |
| 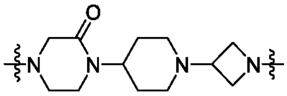 | 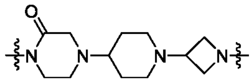 | | |
| 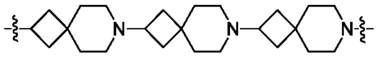 | 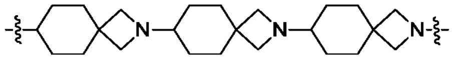 | | |
| 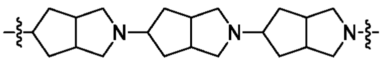 | 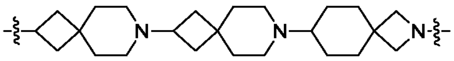 | | |
| 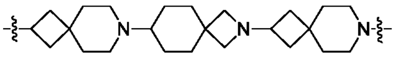 | 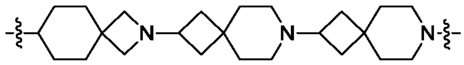 | | |
| 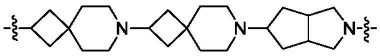 | 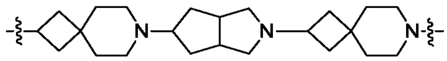 | | |
| 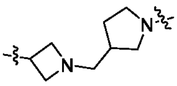 | 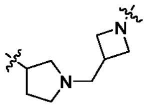 | 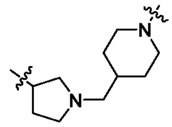 | |
| 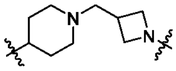 | 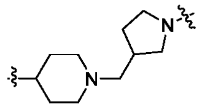 | 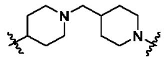 | 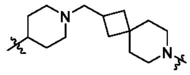 |
| 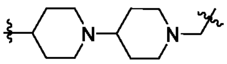 | 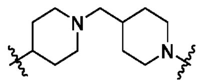 | 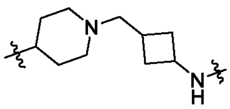 | 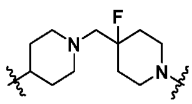 |
| 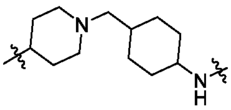 | 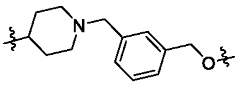 | 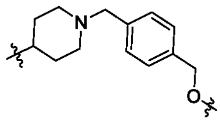 | 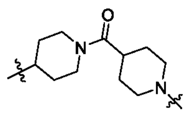 |
| 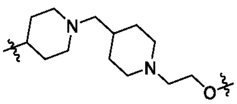 | 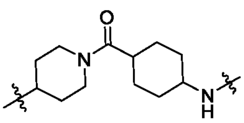 | 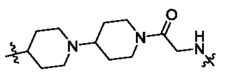 | 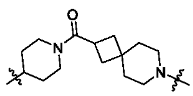 |
| 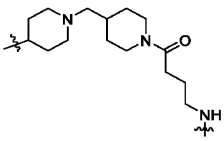 | 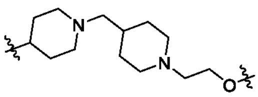 | 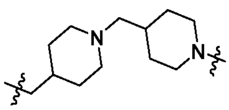 | 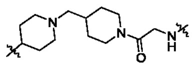 |
| 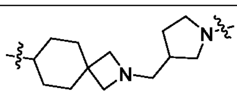 | 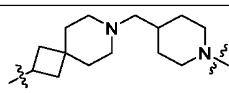 | 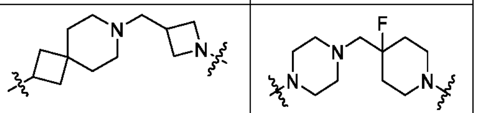 | |
| 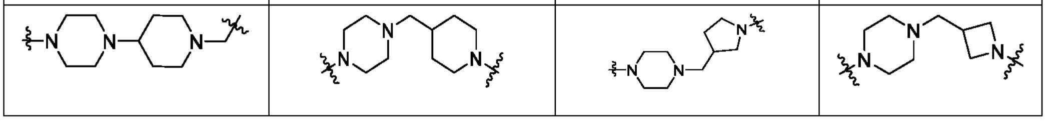 | | | |

| | | | |
|---|---|---|---|
| 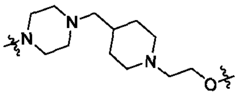 | 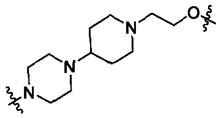 | 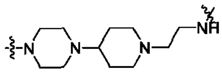 | 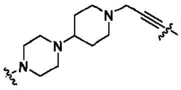 |
| 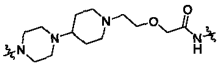 | 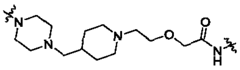 | 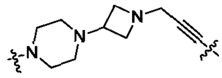 | |
| 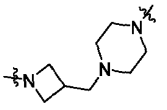 | 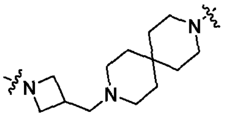 | 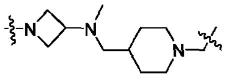 | 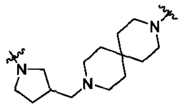 |
| 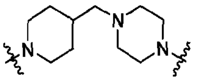 | 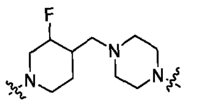 | 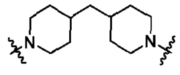 | 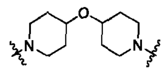 |
| 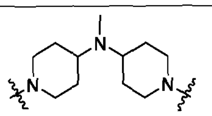 | 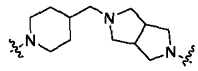 | 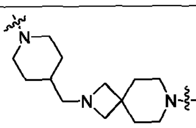 | 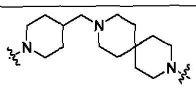 |
| 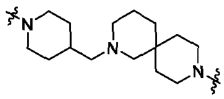 | 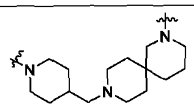 | 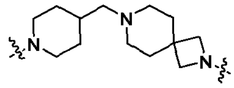 | |
| 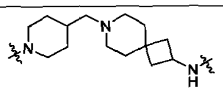 | 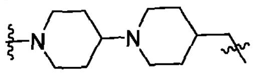 | 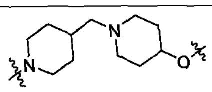 | 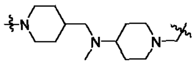 |
| 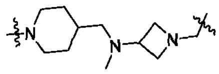 | 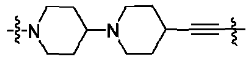 | 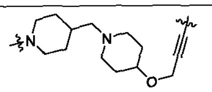 | 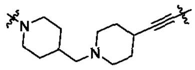 |
| 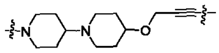 | 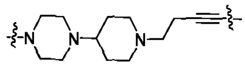 | 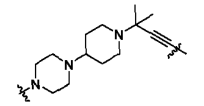 | 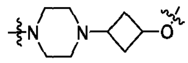 |
| 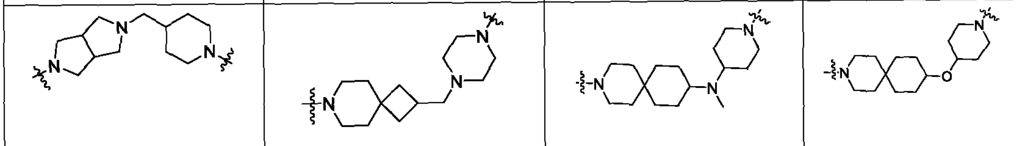 | | | |
| 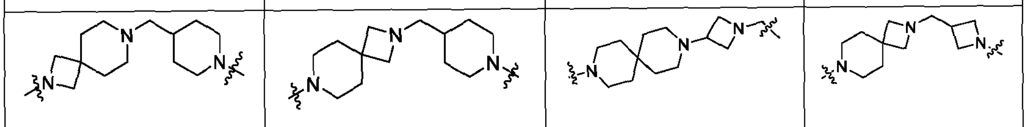 | | | |
| 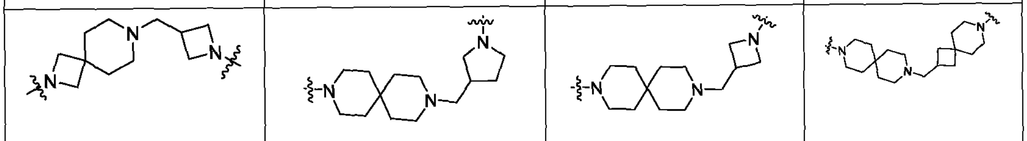 | | | |
| 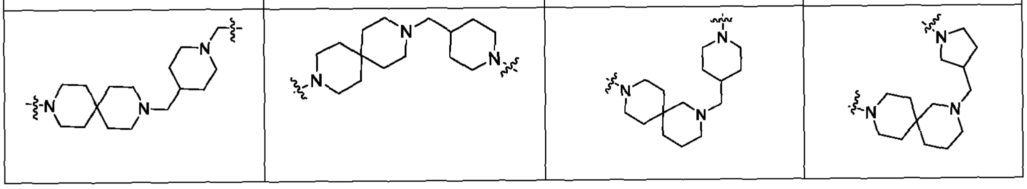 | | | |

| | | | |
|---|---|---|---|
| 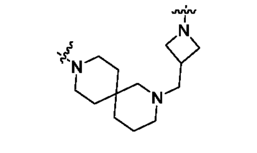 | 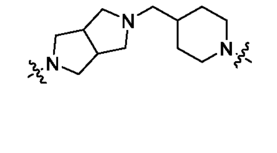 | | |
| | 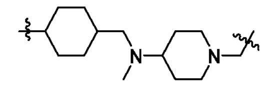 | 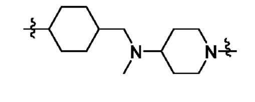 | 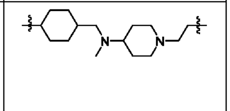 |
| 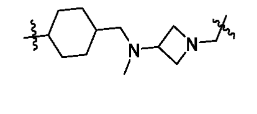 | 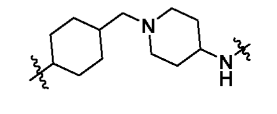 | 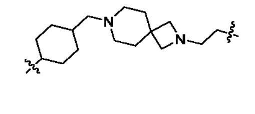 | 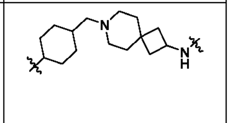 |
| 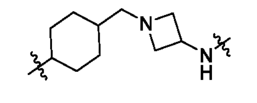 | 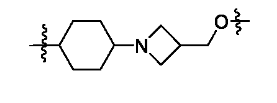 | 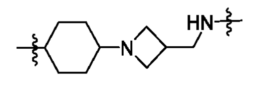 | 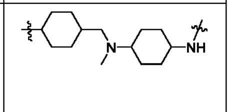 |
| 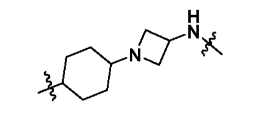 | 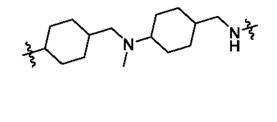 | 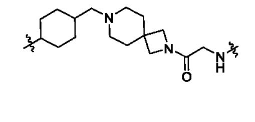 | 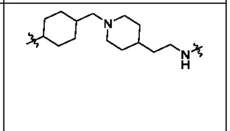 |
| 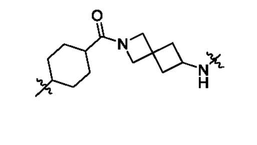 | 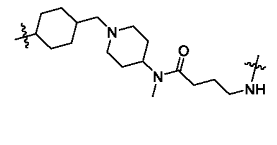 | 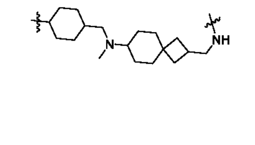 | 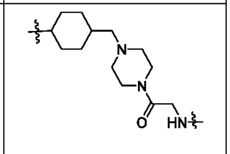 |
| 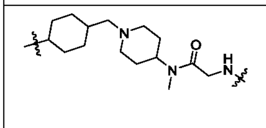 | 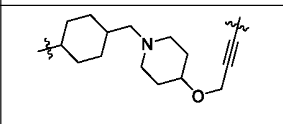 | 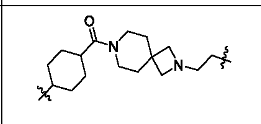 | |
| 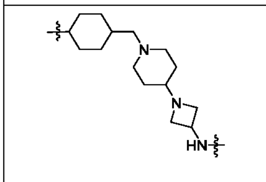 | 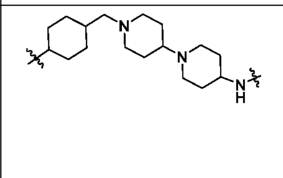 | 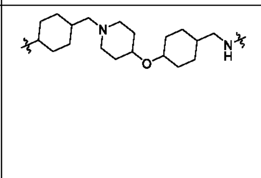 | |
| 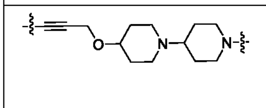 | 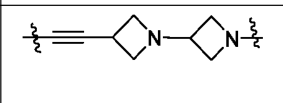 | 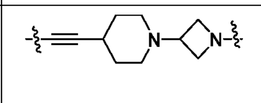 | 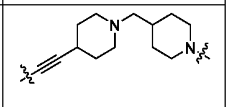 |
| 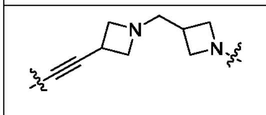 | 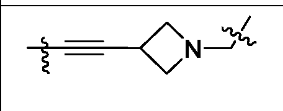 | | |
| 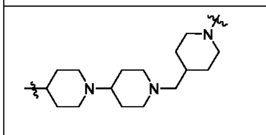 | 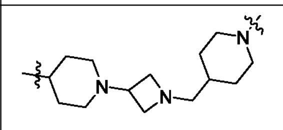 | 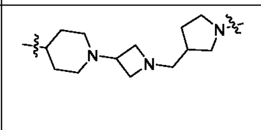 | 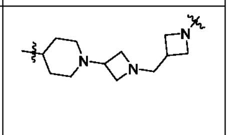 |
| 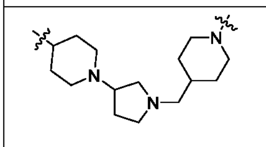 | 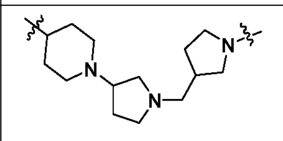 | 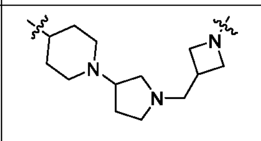 | |
| | | | |

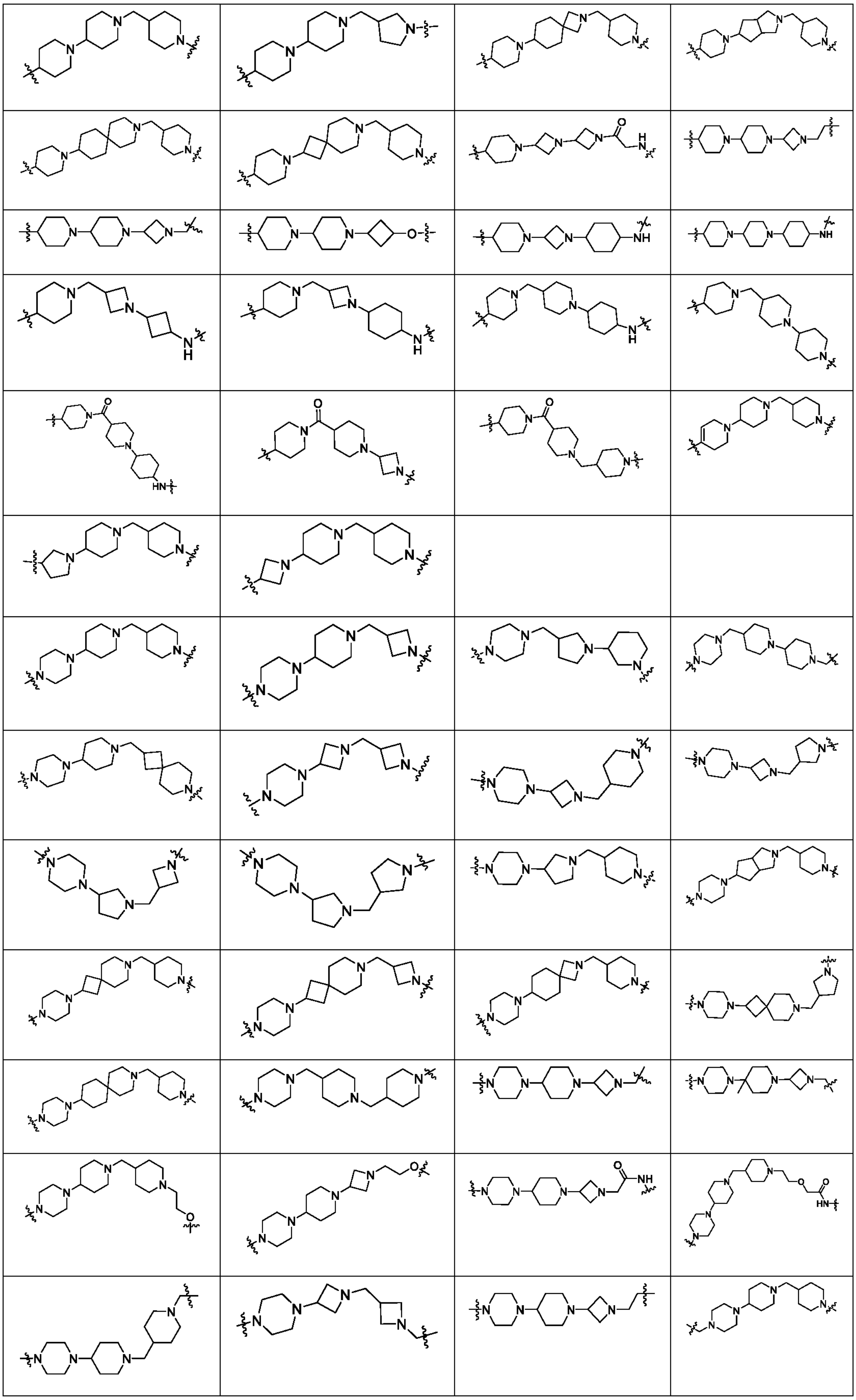

| | | | |
|---|---|---|---|
| 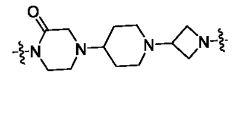 | 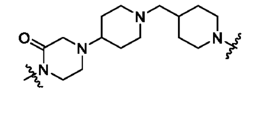 | 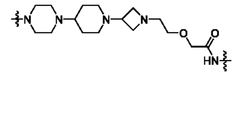 | 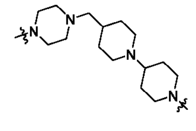 |
| 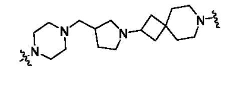 | 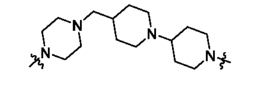 | 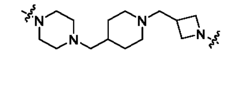 | |
| 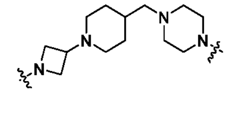 | 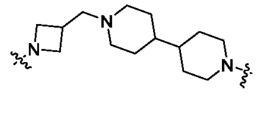 | 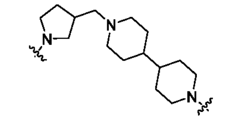 | 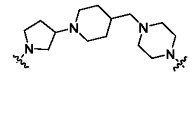 |
| 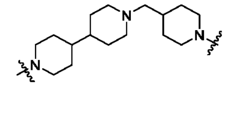 | | 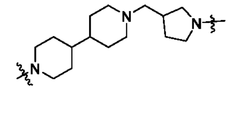 | 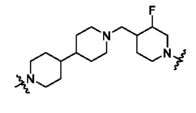 |
| 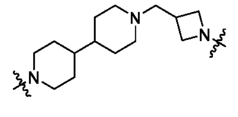 | 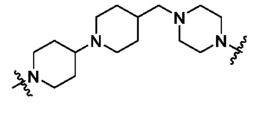 | 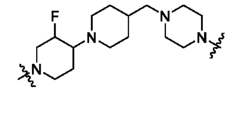 | 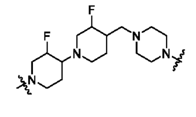 |
| 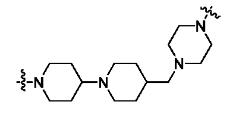 | 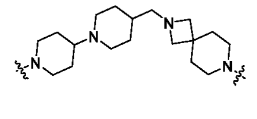 | 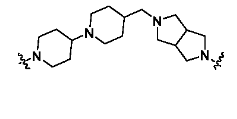 | 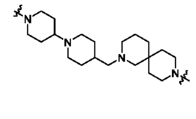 |
| 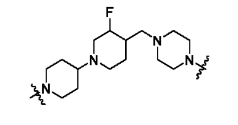 | 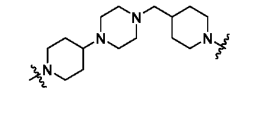 | 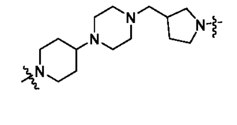 | 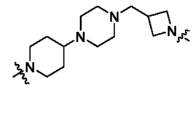 |
| 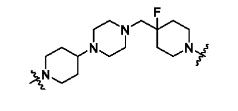 | 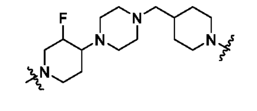 | 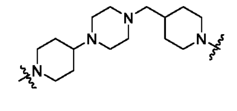 | 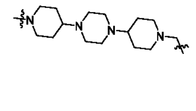 |
| 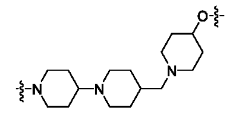 | 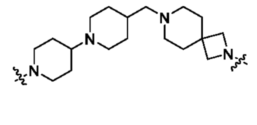 | 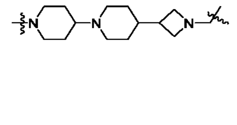 | 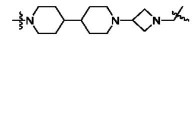 |
| 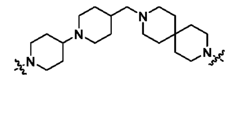 | 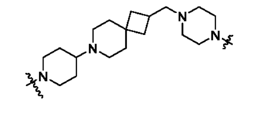 | 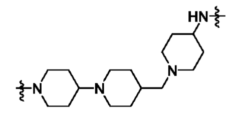 | 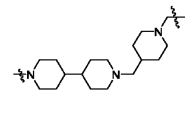 |
| 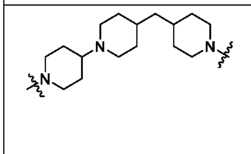 | 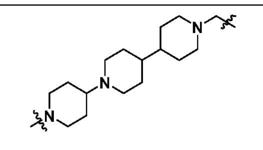 | | |
| | | | |
| | | | |

| | | | |
|---|---|---|---|
| 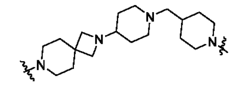 | 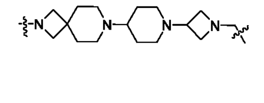 | 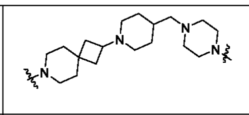 | 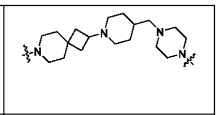 |
| 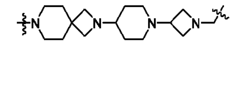 | 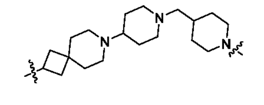 | 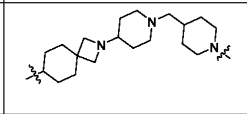 | 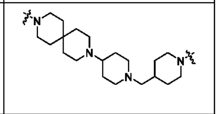 |
| 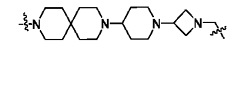 | 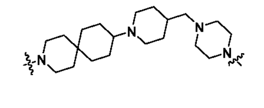 | 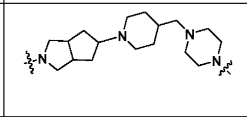 | 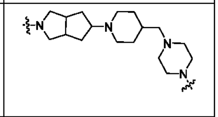 |
| 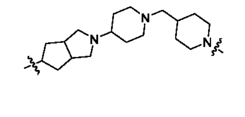 | 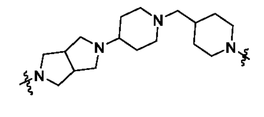 | 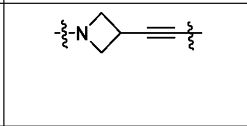 | |
| 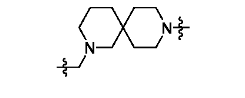 | 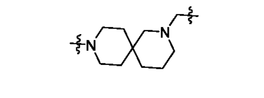 | 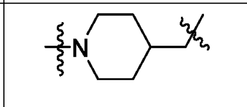 | 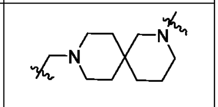 |
| 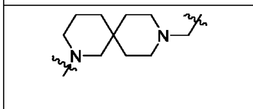 | 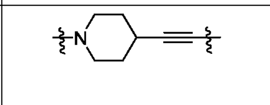 | 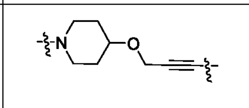 | 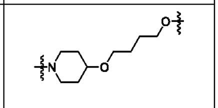 |
| 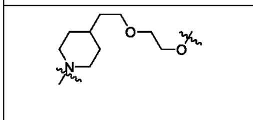 | 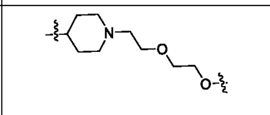 | 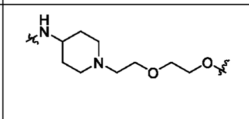 | 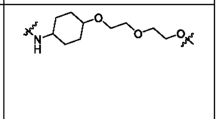 |
| 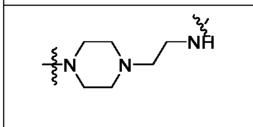 | 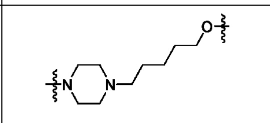 | 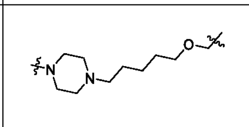 | 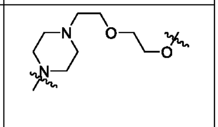 |
| 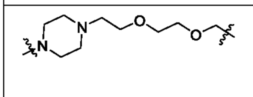 | 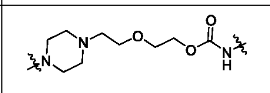 | 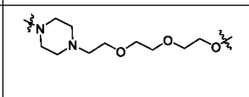 | 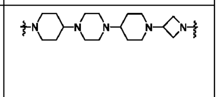 |
| 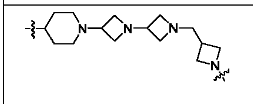 | 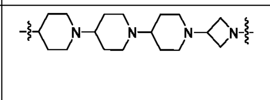 | 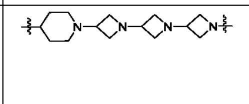 | 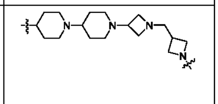 |
| 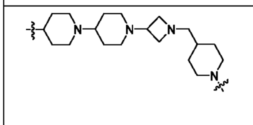 | | | |
| | | | |
| | | | |
| | | | |
| | | | |
| | | | |

| | | | |
|---|---|---|---|
| 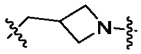 | | | |
| 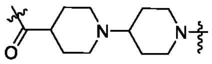 | 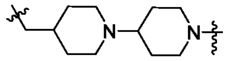 | 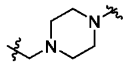 | 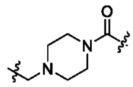 |
| $-(CH_2)-$ | $-(CH_2)_2-$ | $-(CH_2)_3-$ | $-(CH_2)_4-$ |
| $-(CH_2)_5-$ | $-(CH_2)_6-$ | $-(CH_2)_7-$ | $-(CH_2)_8-$ |
| $-(CH_2)_9-$ | $-(CH_2)O-$ | $-(CH_2)_2O-$ | $-(CH_2)_3O-$ |
| $-(CH_2)_4O-$ | $-(CH_2)_5O-$ | $-(CH_2)_6O-$ | $-(CH_2)_7O-$ |
| $-(CH_2)_8O-$ | $-(CH_2)OCH_2-$ | $-(CH_2)_2OCH_2-$ | $-(CH_2)_3OCH_2-$ |
| $-(CH_2)_4OCH_2-$ | $-(CH_2)_5OCH_2-$ | $-(CH_2)_6OCH_2-$ | $-(CH_2)_7OCH_2-$ |
| $-(CH_2)_8OCH_2-$ | $-(CH_2)OCH_2CH_2-$ | $-(CH_2)_2OCH_2CH_2-$ | $-(CH_2)_3OCH_2CH_2-$ |
| $-(CH_2)_4OCH_2CH_2-$ | $-(CH_2)_5OCH_2CH_2-$ | $-(CH_2)_6OCH_2CH_2-$ | $-(CH_2)_7OCH_2CH_2-$ |
| $-(CH_2)_8OCH_2CH_2-$ | $-(CH_2CH_2O)_2-$ | $-(CH_2CH_2O)_3-$ | $-(CH_2CH_2O)_4-$ |
| $-(CH_2CH_2O)_5-$ | $-(CH_2CH_2O)_6-$ | $-(CH_2CH_2O)_7-$ | $-(CH_2CH_2O)_8-$ |
| $-(CH_2CH_2O)_2O-$ | $-(CH_2CH_2O)_3O-$ | $-(CH_2CH_2O)_4O-$ | |
| $-(CH_2)NH-$ | $-(CH_2)_2NH-$ | $-(CH_2)_3NH-$ | $-(CH_2)_4NH-$ |
| $-(CH_2)_5NH-$ | $-(CH_2)_6NH-$ | $-(CH_2)_7NH-$ | $-(CH_2)_8NH-$ |
| $-(CH_2)NHCH_2-$ | $-(CH_2)_2NHCH_2-$ | $-(CH_2)_3NHCH_2-$ | $-(CH_2)_4NHCH_2-$ |
| $-(CH_2)_5NHCH_2-$ | $-(CH_2)_6NHCH_2-$ | $-(CH_2)_7NHCH_2-$ | $-(CH_2)_8NHCH_2-$ |
| $-(CH_2)NHCH_2CH_2-$ | $-(CH_2)_2NHCH_2CH_2-$ | $-(CH_2)_3NHCH_2CH_2-$ | $-(CH_2)_4NHCH_2CH_2-$ |
| $-(CH_2)_5NHCH_2CH_2-$ | $-(CH_2)_6NHCH_2CH_2-$ | $-(CH_2)_7NHCH_2CH_2-$ | $-(CH_2)_8NHCH_2CH_2-$ |
| $-(CH_2)N(CH_3)-$ | $-(CH_2)_2N(CH_3)-$ | $-(CH_2)_3N(CH_3)-$ | $-(CH_2)_4N(CH_3)-$ |
| $-(CH_2)_5N(CH_3)-$ | $-(CH_2)_6N(CH_3)-$ | $-(CH_2)_7NH(CH_3)-$ | $-(CH_2)_8N(CH_3)-$ |
| $-(CH_2)N(CH_3)CH_2-$ | $-(CH_2)_2N(CH_3)CH_2-$ | $-(CH_2)_3N(CH_3)CH_2-$ | $-(CH_2)_4N(CH_3)CH_2-$ |
| $-(CH_2)_5N(CH_3)CH_2-$ | $-(CH_2)_6N(CH_3)CH_2-$ | $-(CH_2)_7N(CH_3)CH_2-$ | $-(CH_2)_8N(CH_3)CH_2-$ |
| $-C(=O)-$ | $-C(=O)CH_2-$ | $-C(=O)CH_2CH_2-$ | $-C(=O)(CH_2)_3-$ |
| $-C(=O)(CH_2)_4-$ | $-C(=O)(CH_2)_5-$ | $-C(=O)(CH_2)_6-$ | $-C(=O)(CH_2)_7-$ |
| $-C(=O)(CH_2)_8-$ | $-C(=O)(CH_2)_9-$ | $-C(=O)CH_2CH_2O-$ | $-C(=O)CH_2CH_2OCH_2-$ |
| $-C(=O)CH_2CH_2O(CH_2)_2-$ | $-C(=O)CH_2CH_2O(CH_2)_3-$ | $-C(=O)(CH_2)_2O(CH_2)_4-$ | $-C(=O)CH_2O-$ |
| $-C(=O)CH_2OCH_2-$ | $-C(=O)CH_2O(CH_2)_2-$ | $-C(=O)CH_2O(CH_2)_3-$ | $-C(=O)CH_2O(CH_2)_4-$ |
| $-C(=O)(CH_2)_2O-$ | $-C(=O)(CH_2)_3O-$ | $-C(=O)(CH_2)_4O-$ | $-C(=O)(CH_2)_5O-$ |
| $-C(=O)(CH_2)_6O-$ | $-C(=O)(CH_2)_2OCH_2-$ | $-C(=O)(CH_2)_3OCH_2-$ | |

| | | |
|---|---|---|
| $-C(=O)(CH_2)_4OCH_2-$ | $-C(=O)(CH_2)_5OCH_2-$ | $-C(=O)(CH_2)_6OCH_2-$ |
| $-C(=O)CH_2CH_2NH-$ | $-C(=O)(CH_2)_2NHCH_2-$ | $-C(=O)(CH_2)_2NH(CH_2)_2-$ |
| $-C(=O)(CH_2)_2NH(CH_2)_3-$ | $-C(=O)(CH_2)_2NH(CH_2)_4-$ | $-C(=O)CH_2NH-$ |
| $-C(=O)CH_2NHCH_2-$ | $-C(=O)CH_2NH(CH_2)_2-$ | $-C(=O)CH_2NH(CH_2)_3-$ |
| $-C(=O)CH_2NH(CH_2)_4-$ | $-C(=O)(CH_2)_2NH-$ | $-C(=O)(CH_2)_3NH-$ |
| $-C(=O)(CH_2)_4NH-$ | $-C(=O)(CH_2)_5NH-$ | $-C(=O)(CH_2)_6NH-$ |
| $-C(=O)(CH_2)_2NHCH_2-$ | $-C(=O)(CH_2)_3NHCH_2-$ | $-C(=O)(CH_2)_4NHCH_2-$ |
| $-C(=O)(CH_2)_5NHCH_2-$ | $-C(=O)(CH_2)_6NHCH_2-$ | $-C(=O)CH_2O(CH_2)_2O-$ |
| $-C(=O)CH_2(O(CH_2)_2)_2O-$ | $-C(=O)CH_2(O(CH_2)_2)_3O-$ | $-C(O)CH_2(O(CH_2)_2)_4O-$ |
| $-C(=O)CH_2O(CH_2)_2-$ | $-C(=O)CH_2(O(CH_2)_2)_2-$ | $-C(=O)CH_2(O(CH_2)_2)_3-$ |
| $-C(O)CH_2(O(CH_2)_2)_4-$ | $-C(=O)(CH_2)_2O(CH_2)_2-$ | $-C(O)(CH_2)_2(O(CH_2)_2)_2-$ |
| $-C(O)(CH_2)_2(O(CH_2)_2)_3-$ | | $-C(=O)CH_2OCH_2O-$ |
| $-C(=O)CH_2O(CH_2)_2O-$ | $-C(=O)CH_2O(CH_2)_3O-$ | $-C(=O)CH_2O(CH_2)_4O-$ |
| $-C(=O)CH_2O(CH_2)_5O-$ | $-C(=O)CH_2O(CH_2)_6O-$ | $-C(=O)CH_2O(CH_2)_7O-$ |

| | |
|---|---|
| $-C(=O)CH_2O(CH_2)_6OCH_2-$ | $-C(=O)CH_2O(CH_2)_5OCH_2-$ |
| $-C(=O)CH_2O(CH_2)_4OCH_2-$ | $-C(=O)CH_2O(CH_2)_3OCH_2-$ |
| $-C(=O)CH_2O(CH_2)_2OCH_2-$ | $-C(=O)CH_2O(CH_2)_2O(CH_2)_2-$ |
| $-C(=O)CH_2O(CH_2)_2O(CH_2)_3-$ | $-C(=O)CH_2O(CH_2)_2O(CH_2)_4-$ |
| $-C(=O)(CH_2)OCH_2CH_2O-$ | $-C(=O)(CH_2)_2OCH_2CH_2O-$ |
| $-C(=O)(CH_2)_3OCH_2CH_2O-$ | $-C(=O)(CH_2)_4OCH_2CH_2O-$ |
| $-C(=O)CH_2OCH_2CH_2NHCH_2CH_2O-$ | $-C(=O)CH_2OCH_2CH_2N(CH_3)CH_2CH_2O-$ |
| $-C(=O)CH_2OCH_2CH_2OCH_2CH_2NH-$ | $-C(=O)CH_2OCH_2CH_2OCH_2CH_2N(CH_3)-$ |
| $-C(=O)CH_2NHCH_2CH_2OCH_2CH_2O-$ | $-C(=O)CH_2N(CH_3)CH_2CH_2OCH_2CH_2O-$ |
| $-C(=O)CH_2NHCH_2CH_2OCH_2CH_2NH-$ | $-C(=O)CH_2N(CH_3)CH_2CH_2OCH_2CH_2NH-$ |
| $-C(=O)CH_2OCH_2CH_2OCH_2CH_2CH_2-$ | $-C(=O)CH_2OCH_2CH_2NHCH_2CH_2CH_2-$ |

The present invention relates to a pharmaceutical composition, comprising the above-mentioned compound or the stereoisomer, deuterated compound, solvate, prodrug, metabolite, pharmaceutically acceptable salt or co-crystal thereof according to the present invention, and a pharmaceutically acceptable carrier.

The present invention relates to the use of the above-mentioned compound or the stereoisomer, deuterated compound, solvate, prodrug, metabolite, pharmaceutically acceptable salt or co-crystal thereof according to the present invention in the preparation of a medicament for treating a disease related to Bcl-2 family protein activity or expression level.

The present invention relates to the use of the above-mentioned compound or the stereoisomer, deuterated

compound, solvate, prodrug, metabolite, pharmaceutically acceptable salt or co-crystal thereof according to the present invention in the preparation of a medicament for treating a disease related to the inhibition or degradation of Bcl-2 family proteins.

The present invention relates to the use of the above-mentioned compound or the stereoisomer, deuterated compound, solvate, prodrug, metabolite, pharmaceutically acceptable salt or co-crystal thereof according to the present invention, wherein the disease is selected from cancer.

The present invention relates to a pharmaceutical composition or pharmaceutical preparation comprising a therapeutically effective amount of the compound, or the stereoisomer, deuterated compound, solvate, prodrug, metabolite, pharmaceutically acceptable salt or co-crystal thereof according to the present invention, and a pharmaceutically acceptable excipient. The pharmaceutical composition can be in a unit preparation form (the amount of the active drug in the unit preparation is also referred to as the "preparation specification").

The present invention further provides a method for treating a disease in a mammal, the method comprising administering to the mammal a therapeutically effective amount of the compound, or the stereoisomer, deuterated compound, solvate, prodrug, metabolite, pharmaceutically acceptable salt or co-crystal thereof or the pharmaceutical composition according to the present invention. In some embodiments, the mammal according to the present invention comprises humans.

The term "effective amount" or "therapeutically effective amount" according to the present application refers to a sufficient amount of the compound disclosed in the present application that is administered to ameliorate, to some extent, one or more symptoms of a disease or condition (such as cancer) being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are limited to 1-1500 mg, 1-1000 mg, 1-900 mg, 1-800 mg, 1-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 80-1500 mg, 80-1000 mg, and 80-800 mg;

in some embodiments, the pharmaceutical composition comprises the compound, or the stereoisomer, deuterated compound, solvate, prodrug, metabolite, pharmaceutically acceptable salt or co-crystal thereof according to the present invention in an amount including but not limited to 1-1500 mg, 1-1000 mg, 20-800 mg, 40-800 mg, 40-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg, 840 mg, and 1000 mg.

The present invention further provides a method for treating a disease in a mammal, the method comprising administering to a subject a therapeutically effective amount of the compound, or the stereoisomer, deuterated compound, solvate, prodrug, metabolite, pharmaceutically acceptable salt, or co-crystal thereof according to the present invention, wherein the therapeutically effective amount is preferably 1-1500 mg, and the disease is preferably cancer.

The present invention further provides a method for treating a disease in a mammal, the method comprising administering to a subject a medicament, i.e., the compound, or the stereoisomer, deuterated compound, solvate, prodrug, metabolite, pharmaceutically acceptable salt, or co-crystal thereof according to the present invention in a daily dose of 1-1500 mg/day, wherein the daily dose can be a single dose or divided doses; in some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 10 mg/day, 20 mg/day, 25 mg/day, 50 mg/day, 80 mg/day, 100 mg/day, 125 mg/day, 150 mg/day, 160 mg/day, 200 mg/day, 300 mg/day, 320 mg/day, 400 mg/day, 480 mg/day, 600 mg/day, 640 mg/day, 800 mg/day, 1000 mg/day, or 1500 mg/day.

The present invention relates to a kit, wherein the kit can comprise a composition in the form of a single dose or multiple doses and comprises the compound, or the stereoisomer, deuterated compound, solvate, prodrug, metabolite, pharmaceutically acceptable salt, or co-crystal thereof according to the present invention, and the amount of the compound or the stereoisomer, deuterated compound, solvate, prodrug, metabolite, pharmaceutically acceptable salt, or

co-crystal thereof according to the present invention is identical to the amount of same in the above-mentioned pharmaceutical composition.

In the present invention, the amount of the compound, or the stereoisomer, deuterated compound, solvate, prodrug, metabolite, pharmaceutically acceptable salt or co-crystal thereof according to the present invention is calculated in the form of a free base in each case.

Unless stated to the contrary, the terms used in the description and claims have the following meanings.

The carbon, hydrogen, oxygen, sulphur, nitrogen or F, Cl, Br, I involved in the groups and compounds of the present invention all comprise their isotopes, and the carbon, hydrogen, oxygen, sulphur or nitrogen involved in the groups and compounds of the present invention is optionally further substituted with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen), tritium (T, also known as superheavy hydrogen), the isotopes of oxygen comprise $^{16}O$, $^{17}O$ and $^{18}O$, the isotopes of sulphur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"Halogen" refers to F, Cl, Br or I.

"Halogen-substituted" refers to F, Cl, Br or I substitution, including but not limited to a substitution with 1 to 10 substituents selected from F, Cl, Br or I, a substitution with 1 to 6 substituents selected from F, Cl, Br or I, or a substitution with 1 to 4 substituents selected from F, Cl, Br or I. "Halogen-substituted" is referred to simply as "halo".

"Alkyl" refers to a substituted or unsubstituted linear or branched saturated aliphatic hydrocarbyl group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. The definition of the "alkyl" herein is consistent with this definition. Alkyl can be monovalent, divalent, trivalent or tetravalent.

"Hydrocarbyl" refers to a substituted or unsubstituted linear or branched saturated or unsaturated group consisting of carbon and hydrogen atoms. Hydrocarbyl can be monovalent, divalent, trivalent or tetravalent.

"Heteroalkyl" refers to a substituted or unsubstituted alkyl group in which one or more (including but not limited to 2, 3, 4, 5 or 6) carbon atoms are replaced by heteroatoms (including but not limited to N, O or S). Non-limiting examples include $-X(CH_2)v-X(CH_2)v-X(CH_2)v-H$ (v is an integer from 1 to 5; each X is independently selected from a bond or a heteroatom, which includes but is not limited to N, O or S; at least one X is selected from a heteroatom; and N or S in the heteroatom can be oxidized to various oxidation states). Heteroalkyl can be monovalent, divalent, trivalent or tetravalent.

"Alkylene" refers to a substituted or unsubstituted linear or branched divalent saturated hydrocarbyl group, including $-(CH_2)_v-$ (v is an integer from 1 to 10), and examples of alkylene include, but are not limited to, methylene, ethylene, propylene, butylene, *etc.*

"Heteroalkylene" refers to a substituted or unsubstituted alkylene group in which one or more (including but not limited to 2, 3, 4, 5 or 6) carbon atoms are replaced by heteroatoms (including but not limited to N, O or S). Non-limiting examples include $-X(CH_2)v-X(CH_2)v-X(CH_2)v-$, wherein v is an integer from 1 to 5, each X is independently selected from a bond, N, O or S, and at least one X is selected from N, O or S.

"Cycloalkyl" refers to a substituted or unsubstituted saturated carbocyclic hydrocarbyl group, usually having from 3 to 10 carbon atoms, and non-limiting examples include cyclopropyl, cyclobutyl, cyclopentyl, cyclohexyl, cycloheptyl, etc. The "cycloalkyl" herein is as defined above. Cycloalkyl can be monovalent, divalent, trivalent or tetravalent.

"Heterocycloalkyl" refers to a substituted or unsubstituted saturated heteroatom-containing cyclic hydrocarbyl group, including but not limited to 3 to 10 atoms, 3 to 8 atoms, or 1 to 3 heteroatoms selected from N, O or S. N and S selectively substituted in the heterocycloalkyl ring can be oxidized to various oxidation states. Heterocycloalkyl can be connected to a heteroatom or a carbon atom; heterocycloalkyl can be connected to an aromatic ring or a non-aromatic ring; and heterocycloalkyl can be connected to a bridged ring or a spiro ring. Non-limiting examples include oxiranyl, azacyclopropyl, oxetanyl, azetidinyl, tetrahydrofuryl, tetrahydro-2*H*-pyranyl, dioxolanyl, dioxanyl, pyrrolidinyl, piperidyl, imidazolidinyl, oxazolidinyl, oxazinanyl, morpholinyl, hexahydropyrimidyl or piperazinyl. Heterocycloalkyl can be monovalent, divalent, trivalent or tetravalent.

"Alkenyl" refers to a substituted or unsubstituted linear or branched unsaturated hydrocarbyl group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The definition of the "alkenyl" herein is consistent with this definition. Alkenyl can be monovalent, divalent, trivalent or tetravalent.

"Alkynyl" refers to a substituted or unsubstituted linear or branched monovalent unsaturated hydrocarbyl group,

having at least 1, usually 1, 2 or 3 carbon-carbon triple bonds, with a main chain including but not limited to 2 to 10 carbon atoms, 2 to 6 carbon atoms, or 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, etc. Alkynyl can be monovalent, divalent, trivalent or tetravalent.

"Alkoxy" refers to a substituted or unsubstituted -O-alkyl group. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system. Carbocyclyl can be connected to an aromatic ring or a non-aromatic ring, wherein the aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring,

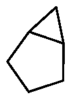,

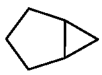, 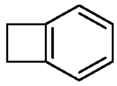, 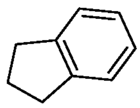 or 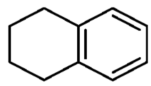.

"Carbocyclyl" or "carbocycle" can be monovalent, divalent, trivalent or tetravalent.

"Heterocyclyl" or "heterocycle" refers to a substituted or unsubstituted saturated or unsaturated aromatic ring or non-aromatic ring, wherein the aromatic ring or non-aromatic ring can be 3- to 8-membered monocyclic ring, 4- to 12-membered bicyclic ring or 10- to 15-membered tricyclic ring system, and contains one or more (including but not limited to 2, 3, 4 or 5) heteroatoms selected from N, O or S, and the selectively substituted N and S in the heterocyclyl ring can be oxidized to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, azacyclopropyl, oxetanyl, azetidinyl, 1,3-dioxolanyl, 1,4-dioxolanyl, 1,3-dioxanyl, azacycloheptyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

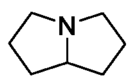,

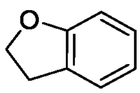, 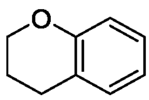, 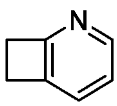, 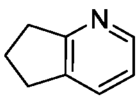, 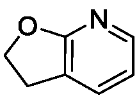, 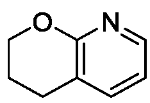, 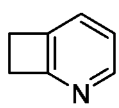

, 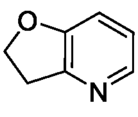, 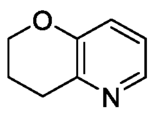, 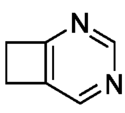, 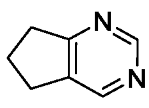, 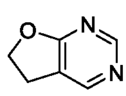 or 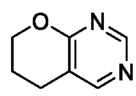.

"Heterocyclyl" or "heterocycle" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not

limited to 5 to 20, 6 to 14, 6 to 12, or 6 to 10, wherein one or more rings may contain 0 or more (including but not limited to 1, 2, 3 or 4) double bonds, and can optionally contain 0 to 5 heteroatoms selected from N, O or $S(=O)_n$.

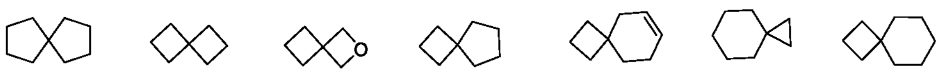

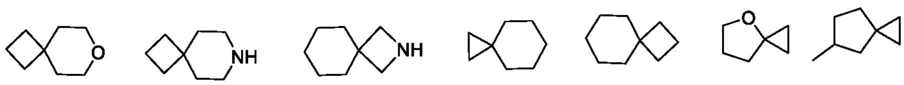

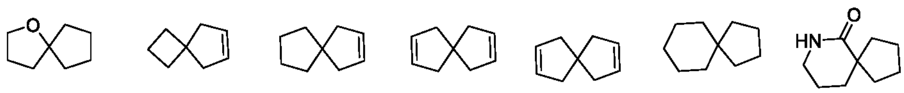

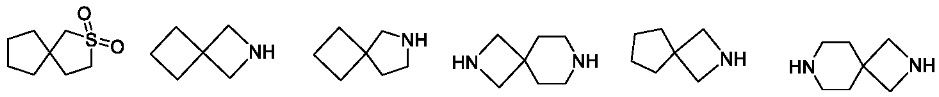

"Spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes but is not limited to 5 to 20, 5 to 14, 5 to 12, or 5 to 10. Non-limiting examples include:

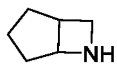 ,

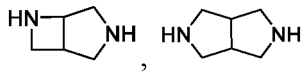 , ,

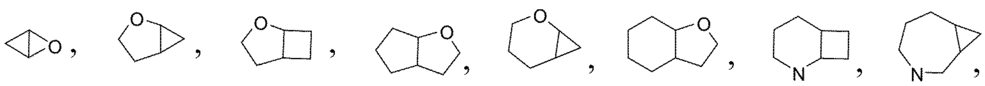 , , , , , , , ,

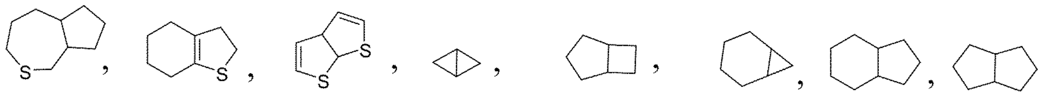 , , , , , , , ,

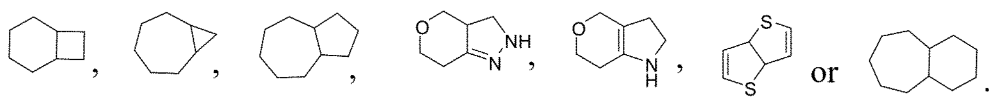 , , , , , or .

"Fused ring" or "fused ring group" can be monovalent, divalent, trivalent or tetravalent.

"Bridged ring" or "bridged ring group" refers to a substituted or unsubstituted polycyclic group containing any two atoms that are not directly connected, and may contain 0 or more double bonds. Any ring in the fused ring system may contain 0 to 5 groups selected from heteroatoms or groups containing heteroatoms (including but not limited to N, S(=O)n or O, wherein n is 0, 1 or 2). The number of ring atoms includes but is not limited to 5 to 20, 5 to 14, 5 to 12 or 5 to 10. Non-limiting examples include

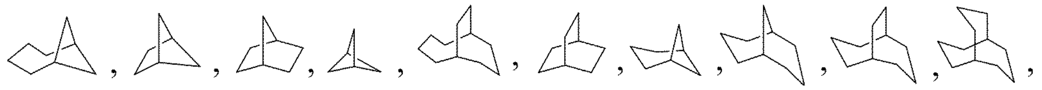
, , , , , , , , , ,

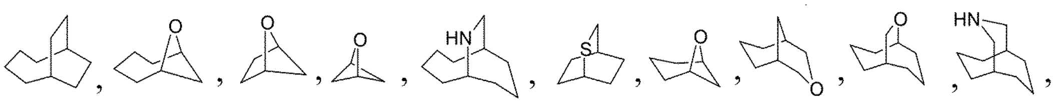
, , , , , , , , , ,

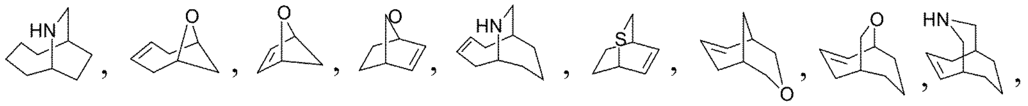
, , , , , , , , ,

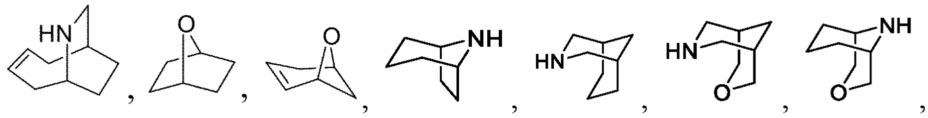
, , , , , , , ,

cubane or adamantane. "Bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms. The definition of the "carbospiro ring", "spiro ring carbocyclyl", "spirocarbocyclyl" or "carbospiro ring group" herein is consistent with that of a spiro ring.

"Carbo-fused ring", "fused ring carbocyclyl", "fused carbocyclyl" or "carbo-fused ring group" refers to a "fused ring" with a ring system consisting only of carbon atoms. The definition of the "carbo-fused ring", "fused ring carbocyclyl", "fused carbocyclyl" or "carbo-fused ring group" herein is consistent with that of a fused ring.

"Carbo-bridged ring", "bridged ring carbocyclyl", "bridged carbocyclyl" or "carbo-bridged ring group" refers to a "bridged ring" with a ring system consisting only of carbon atoms. The definition of the "carbo-bridged ring", "bridged ring carbocyclyl", "bridged carbocyclyl" or "carbo-bridged ring group" herein is consistent with that of a bridged ring.

"Mono-heterocyclic ring", "monocyclic heterocyclyl" or "mono-heterocyclic ring group" refers to "heterocyclyl" or "heterocycle" with a monocyclic system. The definition of the "heterocyclyl", "monocyclic heterocyclyl" or "mono-heterocyclic ring group" herein is consistent with that of heterocycle.

"Fused-heterocyclic ring", "fused-heterocyclic ring group", "fused ring heterocyclyl" or "fused-heterocyclic ring group" refers to a "fused ring" containing a heteroatom. The definition of the "fused-heterocyclic ring", "fused-heterocyclic ring group", "fused ring heterocyclyl" or "fused-heterocyclic ring group" herein is consistent with that of a fused ring.

"Spiro-heterocyclic ring", "spiro-heterocyclic ring group", "spiro ring heterocyclyl" or "spiro-heterocyclic ring group" refers to a "spiro ring" containing a heteroatom. The definition of the "spiro-heterocyclic ring", "spiro-heterocyclic ring group", "spiro ring heterocyclyl" or "spiro-heterocyclic ring group" herein is consistent with that of a spiro ring.

"Bridged-heterocyclic ring", "bridged-heterocyclic ring group", "bridged ring heterocyclyl" or "bridged-heterocyclic ring group" refers to a "bridged ring" containing a heteroatom. The definition of the "bridged-heterocyclic ring", "bridged-heterocyclic ring group", "bridged ring heterocyclyl" or "bridged-heterocyclic ring group" herein is consistent with that of a bridged ring.

"Aryl" or "aromatic ring" refers to a substituted or unsubstituted aromatic hydrocarbyl group with a monocyclic ring or a fused ring, wherein the number of ring atoms in the aromatic ring includes but is not limited to 6 to 18, 6 to 12 or 6 to 10 carbon atoms. The aryl ring can be fused to a saturated or unsaturated carbocycle or heterocycle, wherein the ring connected to the parent structure is an aryl ring. Non-limiting examples include a benzene ring, a naphthalene ring, or

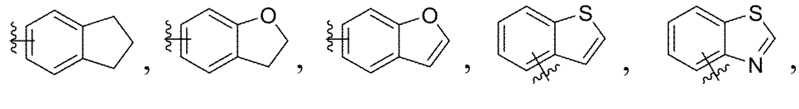
, , , , ,

and "aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbyl group containing 1 to 5 heteroatoms or groups containing heteroatoms (including but not limited to N, O or S(=O)n, wherein n is 0, 1 or 2), wherein the number of ring atoms in the heteroaromatic ring includes but is not limited to 5-15, 5-10 or 5-6.

Non-limiting examples of heteroaryl include, but are not limited to pyridyl, furyl, thienyl, pyridyl, pyranyl, N-alkylpyrrolyl, pyrimidyl, pyrazinyl, pyridazinyl, imidazolyl, benzopyrazole, benzoimidazole, benzopyridine, pyrrolopyridine, etc. The heteroaryl ring may be fused to a saturated or unsaturated carbocycle or heterocycle, wherein the ring connected to the parent structure is a heteroaryl ring. Non-limiting examples include

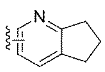, 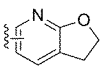, 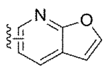, 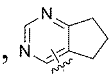, 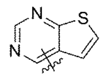, 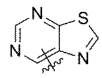, 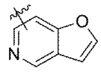 and 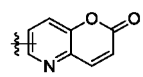.

The definition of the "heteroaryl" herein is consistent with this definition. Heteroaryl can be monovalent, divalent, trivalent or tetravalent. When divalent, trivalent or tetravalent, the point of connection is on the heteroaryl ring.

"5-membered ring fused 5-membered heteroaromatic ring" refers to a 5 fused 5-membered fused heteroaromatic ring, wherein at least one of the two fused rings contains at least one heteroatom (including but not limited to O, S or N), and the entire group is aromatic. Non-limiting examples include a pyrrolopyrrole ring, a pyrazolopyrrole ring, a pyrazolopyrazole ring, a pyrrolofuran ring, a pyrazolofuran ring, a pyrrolothiophene ring and a pyrazolothiophene ring.

"5 fused 6-membered heteroaromatic ring" refers to a 5 fused 6-membered fused heteroaromatic ring, wherein at least one of the two fused rings contains at least one heteroatom (including but not limited to O, S or N), and the entire group is aromatic. Non-limiting examples include a benzo 5-membered heteroaryl and 6-membered heteroaromatic ring fused 5-membered heteroaromatic ring.

"Substitution" or "substituted" refers to a substitution with 1 or more (including, but not limited to 2, 3, 4 or 5) substituents including, but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl-$R^a$, $OR^d$ or $-(CH_2)_m$-alkynyl-$R^a$ (wherein m and n are 0, 1 or 2), arylthio, thiocarbonyl, silyl, $-NR^bR^c$, etc., wherein $R^b$ and $R^c$ are independently selected from H, hydroxyl, amino, carbonyl, alkyl, alkoxy, cycloalkyl, heterocyclyl, aryl, heteroaryl, sulphonyl, or trifluoromethylsulphonyl. Alternatively, $R^b$ and $R^c$ can form a five-or six-membered cycloalkyl or heterocyclyl.

"Containing 1 to 5 heteroatoms selected from O, S or N" means containing 1, 2, 3, 4 or 5 heteroatoms selected from O, S or N.

"Substituted with 0 to X substituents selected from ..." means substituted with 0, 1, 2, 3 ... X substituents selected from ..., wherein X is selected from any integer between 1 and 10. For example, "substituted with 0 to 4 substituents selected from ..." means substituted with 0, 1, 2, 3 or 4 substituents selected from ... For example, "substituted with 0 to 5 substituents selected from ..." means substituted with 0, 1, 2, 3, 4 or 5 substituents selected from ... For example, "bridged-heterocyclic ring is optionally further substituted with 0 to 4 substituents selected from H or F" means that the bridged-heterocyclic ring is optionally further substituted with 0, 1, 2, 3 or 4 substituents selected from H or F.

An X- to Y-membered ring (X is selected from an integer less than Y and greater than or equal to 3, and Y is selected from any integer between 4 and 12) includes X+1-, X+2-, X+3-, X+4-, ..., Y-membered rings. Rings include heterocycle, carbocycle, an aromatic ring, aryl, heteroaryl, cycloalkyl, a mono-heterocyclic ring, a fused-heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound according to the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated compounds, solvates, prodrugs, metabolites, pharmaceutically acceptable salts or co-crystals thereof and other chemical components, wherein "other chemical components" refer to pharmaceutically acceptable carriers, excipients and/or one or more other therapeutic agents.

The term "preparation specification" refers to the weight of the active drug contained in each vial, tablet or other unit preparation.

"Carrier" refers to a material that does not cause significant irritation to an organism and does not eliminate the biological activity and characteristics of a compound administered.

"Animal" is meant to include mammals, such as humans, companion animals, zoo animals, and domestic animals, preferably humans, horses, or dogs.

The term "stereoisomer" refers to an isomer produced as a result of different spatial arrangement of atoms in molecules, including cis-trans isomers, enantiomers and conformational isomers.

"Tautomer" refers to a functional group isomer produced by the rapid movement of an atom in two positions in a molecule, such as keto-enol isomerization and amide-imino alcohol isomerization.

"$IC_{50}$" refers to the concentration of a medicament or inhibitor required to inhibit half of a given biological process (or a component of the process such as an enzyme, a receptor and a cell).

## Brief Description of the Drawings

Figure 1 shows the results of growth inhibition of compound 6 on MOLT-4 xenograft tumour models in nude mice (****P < 0.0001 versus the vehicle control group, two-way ANOVA and then Dunnnett's test).

## Detailed Description of Embodiments

The technical solutions of the present invention will be described in detail below in conjunction with examples, but the protection scope of the present invention includes but is not limited thereto.

To achieve the objectives of the present invention, according to organic synthesis techniques known to those skilled in the art, and starting from commercially available chemicals and/or compounds described in chemical documents, the prepared compounds, "commercially available chemicals", for use in the reactions described herein are obtained from standard commercial sources, including Shanghai Aladdin Bio-Chem Technology Co., Ltd., Shanghai Macklin Biochemical Co., Ltd., Sigma-Aldrich, Alfa Aesar (China) Chemical Co., Ltd., Tokyo Chemical Industry (Shanghai) Co., Ltd., Energy Chemical Co., Ltd., Shanghai Titan Scientific Co., Ltd., Kelong Chemical Co., Ltd., J&K Scientific and the like.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3 527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups", John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalogue are optionally prepared by custom chemical synthesis plants, wherein many of standard chemical supply plants (such as those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

The technical solutions of the present invention will be described in detail by the following examples, but the scope of protection of the present invention includes but is not limited thereto.

The compounds used in the reactions described herein are prepared according to organic synthesis techniques known to those skilled in the art, and starting from commercially available chemicals and(or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

The structures of the compounds are determined by nuclear magnetic resonance (NMR) or (and) mass spectrometry (MS). The NMR shift (δ) is given in the unit of $10^{-6}$ (ppm). NMR is determined with (Bruker Avance III 400 and Bruker Avance 300) nuclear magnetic resonance instrument; the solvents for determination are deuterated dimethyl sulphoxide (DMSO-$d_6$), deuterated chloroform ($CDCl_3$) and deuterated methanol ($CD_3OD$); and the internal standard is tetramethylsilane (TMS).

MS is determined with Agilent 6120B (ESI) and Agilent 6120B (APCI);

HPLC is determined with Agilent 1260DAD high pressure liquid chromatograph (Zorbax SB-C18 100 × 4.6 mm, 3.5 μM);

Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;

and for the column chromatography, Yantai Huanghai silica gel of 200-300 mesh silica gel is generally used as a carrier. SEM:

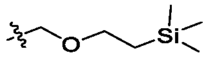

; THP:

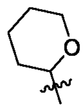

;

Boc: tert-butoxycarbonyl; Ms:

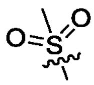

;

TBS:

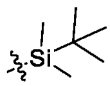

;

MTBE: methyl tert-butyl ether; Bn:

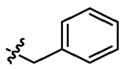

;

DIPEA: N,N-diisopropylethylamine; DMAc: N,N-dimethylacetamide; DMSO: dimethyl sulphoxide; DCM: dichloromethane; Cbz:

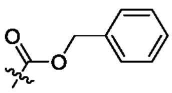

;

NMP: N-methylpyrrolidone; Troc: 2,2,2-trichloroethoxycarbonyl; DMAP: 4-dimethylaminopyridine;
EDCI: CAS 25952-53-8; HOBT: CAS: 2592-95-2; HATU: 148893-10-1;
intermediate 1: 7-(((S)-1-((2S,4R)-4-hydroxy-2-(((S)-1-(4-(4-methylthiazol-5-yl)phenyl)ethyl)carbamoyl)pyrrolidin-1-yl)-3,3-dimethyl-1-oxobutan-2-yl)amino)-7-oxoheptanoic acid (see WO 2019144117 for the synthetic method);
intermediate 2: 2,2,2-tri chloroethyl (R)-4-(4-(phenylthio)-3-((4-aminosulphonyl-2-((trifluoromethyl)sulphonyl)phenyl)amino)butyl)piperazine-1-carboxylate (see WO 2017184995 for the synthetic method);

intermediate 3: 8-(((S)-1-((2S,4R)-4-hydroxy-2-(((S)-1-(4-(4-methylthiazol-5-yl)phenyl)ethyl)carbamoyl)pyrrolidin-1-yl)-3,3-dimethyl-1-oxobutan-2-yl)amino)-8-oxocaprylic acid (see WO 2020163823 of the synthetic method).

**Example 1:**

(2S,4R)-1-((2S)-2-(7-(3-((R)-3-((4-(N-(4-(4-((4'-chloro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl) amino)-4-(phenylthio)butyl)-3,8-diazabicyclo [3.2.1]octan-8-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 1)**

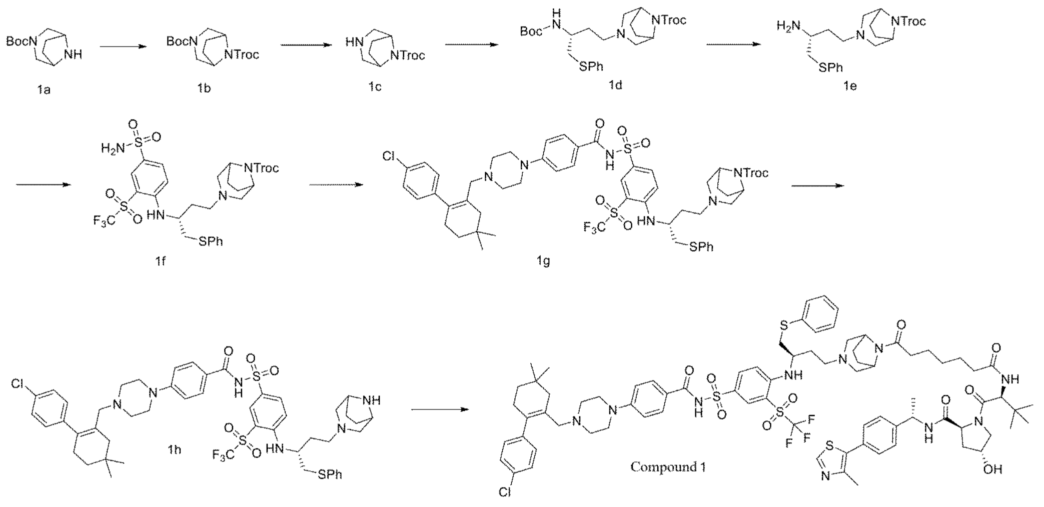
1a 1b 1c 1d 1e 1f 1g 1h Compound 1

Step 1: Preparation of 1b

1a (0.85 g, 4.0 mmol) was dissolved in 20 mL of dichloromethane and triethylamine (0.51 g, 5.0 mmol) was added. The mixture was cooled to 0°C and 2,2,2-trichloroethyl chloroformate (1.0 g, 4.7 mmol) was slowly added dropwise. Then the mixture was slowly warmed to room temperature and reacted for 2 h. After the reaction was completed, 50 mL of dichloromethane and 50 mL of water were added. The organic phase was separated, dried over anhydrous sodium sulphate and concentrated under reduced pressure to afford crude 1b (1.5 g).

Step 2: Preparation of 1c

Crude 1b (1.5 g) was added to 50 mL of 2 mol/L ethyl acetate hydrogen chloride solution and the mixture was reacted at room temperature for 3 h. The reaction system was filtered and 50 mL of dichloromethane was added to the filter cake. Saturated sodium bicarbonate solution (50 mL) was added and the mixture was stirred until the solid was completely dissolved. The organic phase was separated, dried over anhydrous sodium sulphate and concentrated under reduced pressure to afford crude 1c (0.9 g).

LCMS m/z = 287.0 $[M+1]^+$.

Step 3: Preparation of 1d

The above-mentioned crude 1c (0.9 g) was added to 50 mL of 1,2-dichloroethane. Tert-butyl (R)-(4-oxo-1-(phenylthio)butan-2-yl)carbamate (see Nature Communications, 2020, 11, 1996 for the synthetic method) (0.95 g, 3.2 mmol) was added and the mixture was reacted at room temperature for 1 h. Sodium triacetoxyborohydride (0.72 g, 3.4 mmol) was added and the resulting mixture was reacted at room temperature for 12 h. Dichloromethane (50 mL) was added to dilute the reaction solution. Saturated sodium bicarbonate solution was used to adjust the reaction mixture to pH 9.0. The organic phase was separated, dried over anhydrous sodium sulphate and concentrated under reduced pressure. The resulting crude was separated and purified by chromatographic column on silica gel (petroleum ether : ethyl acetate (v/v) = 1 : 1) to afford 1d (1.6 g, total yield over three steps from compound 1a: 71%).

LCMS m/z = 566.1 $[M+1]^+$.

Step 4: Preparation of 1e

1d (1.6 g, 2.83 mmol) was added to 50 mL of 2 mol/L ethyl acetate hydrogen chloride solution and the mixture was reacted at room temperature for 3 h. The reaction system was filtered and 50 mL of dichloromethane was added to the filter cake. Saturated sodium bicarbonate solution (50 mL) was added and the mixture was stirred until the solid was completely dissolved. The organic phase was separated, dried over anhydrous sodium sulphate and concentrated under reduced pressure to afford crude 1e (1.3 g).

LCMS m/z = 466.0 $[M+1]^+$.

Step 5: Preparation of 1f

The above-mentioned crude 1e (1.3 g) was added to 30 mL of acetonitrile. Triethylamine (4.0 g, 4.0 mmol) and 4-fluoro-3-((trifluoromethyl)sulphonyl) benzenesulphonamide (1.0 g, 3.3 mmol) were added and the mixture was refluxed and reacted at 85°C for 3 h. The reaction mixture was cooled to room temperature. The reaction solution was concentrated under reduced pressure and the resulting crude was separated and purified by chromatographic column on silica gel (petroleum ether : ethyl acetate (v/v) = 2 : 1) to afford 1f (1.8 g, yield over two steps from compound 1d: 84%).

LCMS m/z = 753.0 $[M+1]^+$.

Step 6: Preparation of 1g

1f (1.8 g, 2.4 mmol) was added to 100 mL of dichloromethane. 4-(4-((4'-chloro-4,4-dimethyl-3,4, 5,6-tetrahydro-[1,1'-biphenyl]-2-yl)methyl)piperazin-1-yl)benzoic acid (see WO 2017101851 for the synthetic method) (1.31 g, 3.0 mmol), DMAP (0.6 g, 4.9 mmol) and EDCI (0.96 g, 5.0 mmol) were sequentially added and the mixture was reacted at room temperature for 12 h. To the reaction system was added 100 mL of water. The organic phase was separated, dried over anhydrous sodium sulphate and concentrated under reduced pressure and the resulting crude was separated and purified by chromatographic column on silica gel (ethyl acetate) to afford 1g (2.0 g, yield: 71.0%).

LCMS m/z = 588.1 $[M/2+1]^+$.

Step 7: Preparation of 1h

1g (2.0 g, 1.7 mmol) was added to 40 mL of tetrahydrofuran. Zinc powder (6.5 g, 100.0 mmol) and 1.2 mL of acetic acid were sequentially added and the mixture was reacted at room temperature for 5 h. The reaction system was filtered and the filtrate was concentrated under reduced pressure. The residue was then diluted with 100 mL of dichloromethane and then 50 mL of saturated sodium bicarbonate solution was added. The organic phase was separated, dried over anhydrous sodium sulphate and concentrated under reduced pressure to afford crude 1h (1.5 g).

LCMS m/z = 500.3 $[M/2+1]^+$.

Step 8: Preparation of compound 1

The above-mentioned crude 1h (1.5 g) was added to 30 mL of DMF. Intermediate 1 (0.96 g, 1.6 mmol), triethylamine (0.6 g, 4.9 mmol) and HATU (0.78 g, 2.1 mmol) were sequentially added and the mixture was reacted at room temperature for 12 h. To the reaction system was added water (200 mL), and a solid was precipitated. The system was filtered and the filter cake was passed through Pre-HPLC (instrument and preparative column: using Glison GX-281 preparative liquid phase chromatographic instrument, preparative column model: Sunfire C18, 5 $\mu$m, inner diameter $\times$ length = 30 mm $\times$ 150 mm). Preparation method: the crude was dissolved with methanol and dimethyl sulphoxide, and filtered with a 0.45 $\mu$m filter membrane, to prepare into a sample liquid. Mobile phase system: acetonitrile/water (containing 0.1% TFA). Gradient elution method: gradient elution with acetonitrile from 5% to 60% (elution time: 15 min). To the preparative solution were added 100 mL of dichloromethane and 60 mL of saturated sodium bicarbonate solution and the mixture was stirred for 1 h. The organic phase was separated, dried over anhydrous sodium sulphate and concentrated under reduced pressure to afford compound 1 (0.7 g, yield over two steps from compound 1g: 26%).

$^1$H NMR (400 MHz, $CDCl_3$) $\delta$ 8.59 (s, 1H), 8.28 (s, 1H), 8.04 (d, 1H), 7.69 - 7.59 (m, 2H), 7.44 - 7.08 (m, 12H), 6.96 - 6.87 (m, 2H), 6.85 - 6.76 (m, 1H), 6.68 (d, 2H), 6.61 - 6.49 (m, 1H), 6.32 - 6.17 (m, 1H), 5.07 - 4.95 (m, 1H), 4.70 - 4.58 (m, 1H), 4.56 - 4.36 (m, 3H), 4.08 - 3.70 (m, 3H), 3.56 - 3.45 (m, 1H), 3.28 - 3.12 (m, 4H), 3.10 - 3.00 (m, 1H), 2.98 - 2.85 (m, 1H), 2.84 - 2.74 (m, 2H), 2.68 - 2.49 (m, 2H), 2.48 - 1.86 (m, 23H), 1.78 - 1.15 (m, 15H), 1.05 - 0.80 (m, 15H).

LCMS m/z = 523.5 $[M/3+1]^+$.

**Example 2:**

(2S,4R)-1-((2S)-2-(7-(5-((R)-3-((4-(N-(4-(4-((4'-chloro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)-2,5-diazabicyclo [2.2.1]heptan-2-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 2)**

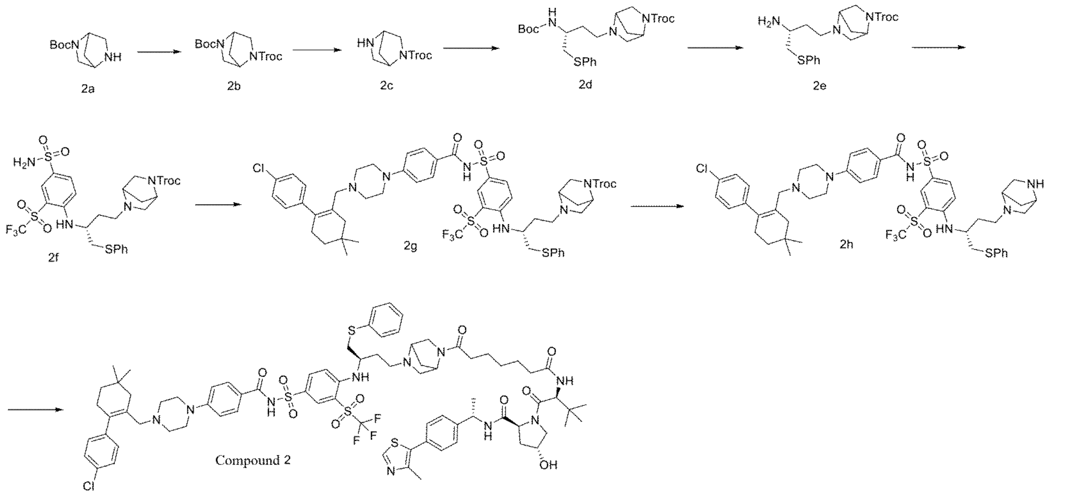

2a 2b 2c 2d 2e 2f 2g 2h Compound 2

Step 1: Preparation of 2b

2a (0.8 g, 4.0 mmol) was dissolved in 20 mL of dichloromethane and triethylamine (0.51 g, 5.0 mmol) was added. The mixture was cooled to 0°C and 2,2,2-trichloroethyl chloroformate (1.0 g, 4.7 mmol) was slowly added dropwise. Then the mixture was slowly warmed to room temperature and reacted for 2 h. After the reaction was completed, the reaction solution was diluted with dichloromethane (50 mL) and then 50 mL of water was added. The organic phase was separated, dried over anhydrous sodium sulphate and concentrated under reduced pressure to afford crude 2b (1.4 g).

Step 2: Preparation of 2c

The above-mentioned crude 2b (1.4 g) was added to 50 mL of 2 mol/L ethyl acetate hydrogen chloride solution and the mixture was reacted at room temperature for 3 h. The reaction system was filtered and 50 mL of DCM was added to the filter cake. Saturated sodium bicarbonate solution (50 mL) was added and the mixture was stirred until the solid was completely dissolved. The organic phase was separated, dried over anhydrous sodium sulphate and concentrated under reduced pressure to afford crude 2c (0.85 g).
LCMS m/z = 273.0 $[M+1]^+$.

Step 3: Preparation of 2d

The above-mentioned crude 2c (0.85 g) was added to 50 mL of 1,2-dichloroethane. Tert-butyl (R)-(4-oxo-1-(phenylthio)butan-2-yl)carbamate (0.95 g, 3.2 mmol) was added and the mixture was reacted at room temperature for 1 h. Sodium triacetoxyborohydride (0.72 g, 3.4 mmol) was added and the resulting mixture was reacted at room temperature for 12 h. DCM (50 mL) was added to dilute the reaction solution. Saturated sodium bicarbonate solution was used to adjust the reaction mixture to pH 9.0. The organic phase was separated, dried over anhydrous sodium sulphate and concentrated under reduced pressure. The resulting crude was separated and purified by chromatographic column on silica gel (petroleum ether : ethyl acetate (v/v) = 1 : 1) to afford 2d (1.3 g, total yield over three steps from compound 2a: 59%).
LCMS m/z = 552.2 $[M+1]^+$.

Step 4: Preparation of 2e

2d (1.3 g, 2.36 mmol) was added to 50 mL of 2 mol/L ethyl acetate hydrogen chloride solution and the mixture was reacted at room temperature for 3 h. The reaction system was filtered and 50 mL of DCM was added to the filter cake. Saturated sodium bicarbonate solution (50 mL) was added and the mixture was stirred until the solid was completely dissolved. The organic phase was separated, dried over anhydrous sodium sulphate and concentrated under reduced pressure to afford crude 2e (1.0 g).
LCMS m/z = 452.0 $[M+1]^+$.

Step 5: Preparation of 2f

The above-mentioned crude 2e (1.0 g) was added to 30 mL of acetonitrile. Triethylamine (4.0 g, 4.0 mmol) and 4-fluoro-3-((trifluoromethyl)sulphonyl) benzenesulphonamide (0.76 g, 2.6 mmol) were added and the mixture was refluxed and reacted at 85°C for 3 h. The reaction mixture was cooled to room temperature. The reaction solution was concentrated under reduced pressure and the resulting crude was separated and purified by chromatographic column on silica gel (petroleum ether : ethyl acetate (v/v) = 2 : 1) to afford 2f (1.5 g, yield over two steps from compound 2d: 86%).
LCMS m/z = 739.0 $[M +1]^+$.

Step 6: Preparation of 2g

2f (1.5 g, 2.0 mmol) was added to 100 mL of dichloromethane. 4-(4-((4'-chloro-4,4-dimethyl-3,4, 5,6-tetrahydro-[1,1'-biphenyl]-2-yl)methyl)piperazin-1-yl)benzoic acid (0.87 g, 2.0 mmol), DMAP (0.5 g, 4.1 mmol) and EDCI (0.76 g, 4.0 mmol) were sequentially added and the mixture was reacted at room temperature for 12 h. To the reaction system was added 100 mL of water. The organic phase was separated, dried over anhydrous sodium sulphate and concentrated under reduced pressure and the resulting crude was separated and purified by chromatographic column on silica gel (ethyl acetate) to afford 2g (1.6 g, yield: 69%).
LCMS m/z = 581.2 $[M/2+1]^+$.

Step 7: Preparation of 2h

2g (1.6 g, 1.38 mmol) was added to 40 mL of tetrahydrofuran. Zinc powder (6.5 g, 100.0 mmol) and 1.2 mL of acetic acid were sequentially added and the mixture was reacted at room temperature for 5 h. The reaction system was filtered and the filtrate was concentrated under reduced pressure. The residue was then diluted with 100 mL of DCM and then 50 mL of saturated sodium bicarbonate solution was added. The organic phase was separated, dried over anhydrous sodium sulphate and concentrated under reduced pressure to afford crude 2h (1.1 g).
LCMS m/z = 493.2 $[M/2+1]^+$.

Step 8: Preparation of compound 2

The above-mentioned crude 2h (1.1 g) was added to 30 mL of DMF. Intermediate 1 (0.96 g, 1.6 mmol), triethylamine (0.6 g, 4.9 mmol) and HATU (0.78 g, 2.1 mmol) were sequentially added and the mixture was reacted at room temperature for 12 h. To the reaction system was added water (200 mL), and a solid was precipitated. The system was filtered and the filter cake was passed through Pre-HPLC (instrument and preparative column: using Glison GX-281 preparative liquid phase chromatographic instrument, preparative column model: Sunfire C18, 5 $\mu$m, inner diameter $\times$ length = 30 mm $\times$ 150 mm). Preparation method: the crude was dissolved with methanol and dimethyl sulphoxide, and filtered with a 0.45 $\mu$m filter membrane, to prepare into a sample liquid. Mobile phase system: acetonitrile/water (containing 0.1% TFA). Gradient elution method: gradient elution with acetonitrile from 5% to 60% (elution time: 15 min). To the preparative solution were added 100 mL of dichloromethane and 60 mL of saturated sodium bicarbonate solution and the mixture was stirred for 1 h. The organic phase was separated, dried over anhydrous sodium sulphate and concentrated under reduced pressure to afford compound 2 (0.05 g, yield over two steps from compound 2g: 2%).
$^1$H NMR (400 MHz, $CDCl_3$) $\delta$ 8.66 (s, 1H), 8.46 - 8.34 (m, 1H), 8.12 - 7.92 (m, 1H), 7.83 - 7.63 (m, 2H), 7.44 - 7.05 (m, 13H), 7.04 - 6.93 (m, 2H), 6.90 - 6.65 (m, 3H), 6.48 - 6.30 (m, 1H), 5.16 - 4.94 (m, 1H), 4.86 - 4.42 (m, 4H), 4.15 - 3.97 (m, 2H), 3.70 - 2.95 (m, 10H), 2.94 - 1.95 (m, 24H), 1.62 - 1.20 (m, 14H), 1.10 - 0.95 (m, 15H).
LCMS m/z = 518.9 $[M/3+1]^+$.

**Example 3:**

(2S,4R)-1-((S)-2-(7-(4-((R)-3-((4-(N-(4-(1-((4'-chloro-4,4-dimethyl-3,4,5,6-tetrahydro-[1,1'-biphenyl]-2-yl)methyl)piperidin-4-yl)benzoyl)sulphamoyl)-2-((trifluoromethyl)sulphonyl)phenyl)amino)-4-(phenylthio)butyl)piperazin-1-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 3**)

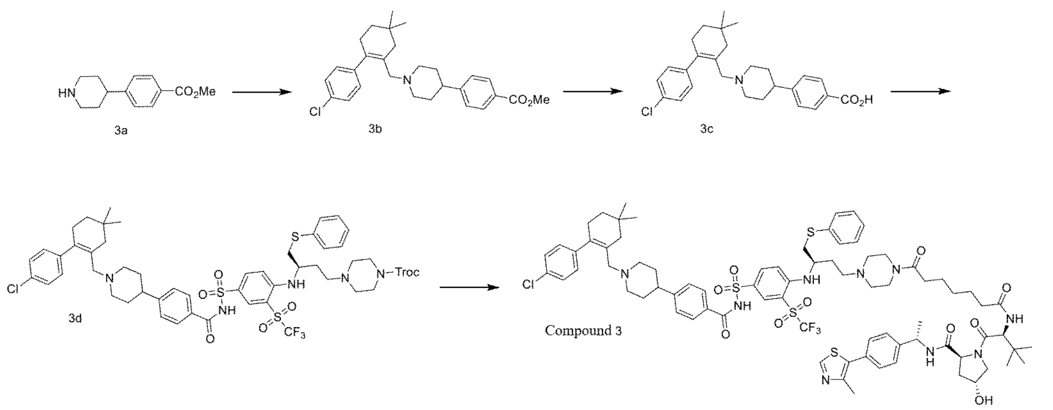

3a 3b 3c 3d Compound 3

Step 1: Preparation of 3b

4'-chloro-4,4-dimethyl-3,4,5,6-tetrahydro-[1,1'-biphenyl]-2-carbaldehyde (see WO 2020041406 for the synthetic method) (350 mg, 1.41 mmol) was dissolved in 15 mL of tetrahydrofuran. 3a (see WO 2020212530 for the synthetic method) (309 mg, 1.41 mmol) and 1 mL of tetraisopropyl titanate were sequentially added and the mixture was stirred at room temperature for 4 h. Then sodium triacetoxyborohydride (1.49 g, 7.03 mmol) was added and the resulting mixture was reacted at room temperature for 16 h. To the reaction solution was slowly added 20 mL of saturated sodium bicarbonate solution. The resulting mixture was extracted with ethyl acetate (60 mL × 2). The organic phase was washed with 50 mL of saturated sodium chloride solution, dried over anhydrous sodium sulphate and concentrated under reduced pressure and the resulting crude was separated and purified by silica gel column chromatography (ethyl acetate/petroleum ether (v/v) = 0 : 1-1 : 1) to afford 3b (400 mg, yield: 63%).

LCMS m/z = 452.3 $[M+1]^+$.

Step 2: Preparation of 3c

3b (0.40 g, 0.88 mmol) was dissolved in 10 mL of methanol. Water (1 mL) and sodium hydroxide (0.15 g, 3.75 mmol) were added and the mixture was reacted at 80°C for 10 h. The reaction system was cooled to room temperature and concentrated under reduced pressure. The residue was dissolved in 10 mL of water and washed with 20 mL of methyl tert-butyl ether. The aqueous phase was separated and adjusted to pH 6 with 1 mol/L hydrochloric acid, extracted with ethyl acetate (100 mL × 2), dried over anhydrous sodium sulphate and concentrated under reduced pressure to afford crude 3c (0.20 g).

LCMS m/z = 438.2 $[M+1]^+$.

Step 3: Preparation of 3d

The above-mentioned crude 3c (200 mg) was dissolved in 15 mL of DCM. Intermediate 2 (330 mg, 0.45 mmol), DMAP (110 mg, 0.9 mmol) and EDCI (180 mg, 0.95 mmol) were sequentially added and the mixture was reacted at room temperature for 16 h. To the reaction system was slowly added 30 mL of water and the mixture was extracted with DCM (60 mL × 2). The organic phase was washed with 50 mL of water, dried over anhydrous sodium sulphate and concentrated under reduced pressure and the resulting crude was separated and purified by silica gel column chromatography (dichloromethane/methanol (v/v) = 9 : 1) to afford 3d (260 mg, yield over two steps from compound 3b: 26%).

LCMS m/z = 574.5 $[M/2+1]^+$.

Step 4: Preparation of compound 3

3d (260 mg, 0.23 mmol) was dissolved in 20 mL of tetrahydrofuran. Acetic acid (0.6 mL) and zinc powder (960 mg, 14.8 mmol) were sequentially added and the mixture was reacted at room temperature for 16 h. The reaction system was filtered and to the filtrate was added water (10 mL). The mixture was then extracted with 20 mL of ethyl acetate. The organic phase was separated, dried over anhydrous sodium sulphate and concentrated under reduced pressure to afford a crude (250 mg). The above-mentioned crude (250 mg) was dissolved in 10 mL of DCM. Triethylamine (0.2 mL), intermediate 1 (154 mg, 0.26 mmol) and HATU (142 mg, 0.37 mmol) were sequentially added and the mixture was reacted at room temperature for 1 h. To the reaction system was added 20 mL of water and the mixture was extracted with 20 mL of ethyl acetate. The organic phase was washed with 20 mL of water, dried over anhydrous sodium sulphate and concentrated under reduced pressure and the resulting crude was passed through Pre-HPLC (instrument and preparative column: using Glison GX-281 preparative liquid phase chromatographic instrument, preparative column model: Sunfire C18, 5 $\mu$m, inner diameter $\times$ length = 30 mm $\times$ 150 mm). Preparation method: the crude was dissolved with methanol and dimethyl sulphoxide, and filtered with a 0.45 $\mu$m filter membrane, to prepare into a sample liquid. Mobile phase system: acetonitrile/water (containing 0.1% TFA). Gradient elution method: gradient elution with acetonitrile from 5% to 60% (elution time: 15 min). To the preparative solution were added 100 mL of dichloromethane and 60 mL of saturated sodium bicarbonate solution and the mixture was stirred for 1 h. The organic phase was separated, dried over anhydrous sodium sulphate and concentrated under reduced pressure to afford compound 3 (40 mg, yield from compound 3d: 11%).

$^1$H NMR (400 MHz, $CDCl_3$) $\delta$ 8.66 (s, 1H), 8.32 (s, 1H), 8.05 - 7.97 (m, 1H), 7.95 - 7.84 (m, 2H), 7.64 - 7.55 (m, 1H), 7.48 - 7.12 (m, 12H), 7.09 - 6.96 (m, 3H), 6.86 - 6.78 (m, 1H), 6.65 - 6.57 (m, 1H), 6.34 - 6.22 (m, 1H), 5.16 - 5.03 (m, 1H), 4.71 - 4.61 (m, 1H), 4.50 - 4.39 (m, 2H), 4.12 - 3.96 (m, 1H), 3.96 - 3.79 (m, 1H), 3.70 - 3.45 (m, 2H), 3.40 - 2.88 (m, 9H), 2.58 - 1.98 (m, 23H), 1.77 - 1.38 (m, 14H), 1.36 - 1.18 (m, 2H), 1.08 - 0.82 (m, 15H).

LCMS m/z = 770.8 $[M/2+1]^+$.

**Example 4:**

(2S,4R)-1-((S)-2-(7-(4-((R)-3-((4-(N-(4-(4-((4'-cyano-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)piperazin-1-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 4**) trifluoroacetate

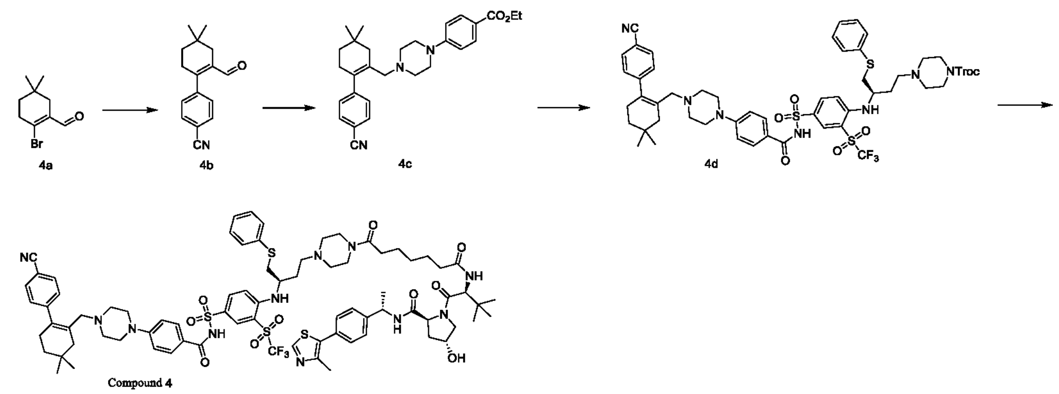

4a 4b 4c 4d

Compound 4

Step 1: Preparation of 4b

4a (see WO 2020041406 for the synthetic method) (1.00 g, 4.61 mmol) and (4-cyanophenyl)boronic acid (1.01 g, 6.87 mmol) were dissolved in 20 mL of dioxane and 2 mL of water. Potassium acetate (1.36 g, 13.8 mmol) and $Pd(dppf)Cl_2$ (0.33 g, 0.45 mmol) were sequentially added and the mixture was reacted at 90°C for 4 h. The reaction solution was cooled to room temperature and to the reaction solution was slowly added 100 mL of water. The mixture was extracted with ethyl acetate (60 mL $\times$ 2), dried over anhydrous sodium sulphate and concentrated under reduced pressure and the resulting crude was separated and purified by silica gel column chromatography (ethyl acetate/petroleum ether (v/v) = 0 : 1-1 : 10) to afford 4b (0.95 g, yield: 86%).

LCMS m/z = 240.3 $[M+1]^+$.

Step 2: Preparation of 4c

4b (510 mg, 2.13 mmol) was dissolved in 15 mL of tetrahydrofuran. Ethyl 4-(piperazin-1-yl)benzoate (500 mg, 2.13 mmol) was added, followed by 1 mL of tetraisopropyl titanate. The mixture was stirred at room temperature for 4 h and then sodium triacetoxyborohydride (1.35 g, 6.37 mmol) was added. The resulting mixture was reacted at room temperature for 16 h. To the reaction solution was slowly added 20 mL of saturated aqueous sodium bicarbonate solution. The resulting mixture was extracted with 60 mL of ethyl acetate twice and the organic phase was washed with 50 mL of saturated sodium chloride, dried over anhydrous sodium sulphate and concentrated under reduced pressure. The resulting crude was separated and purified by silica gel column chromatography (ethyl acetate/petroleum ether (v/v) = 0 : 1-1 : 1) to afford 4c (550 mg, yield: 56%).
LCMS m/z = 458.3 $[M+1]^+$.

Step 3: Preparation of 4d

4c (0.55 g, 1.20 mmol) was dissolved in 20 mL of methanol. Water (2 mL) was added, followed by sodium hydroxide (0.24 g, 6.0 mmol) and the mixture was stirred at 80°C for 10 h. The reaction solution was cooled to room temperature and concentrated under reduced pressure. The residue was dissolved with 10 mL of water and then extracted with 20 mL of methyl tert-butyl ether to remove the impurities. The aqueous phase was separated, adjusted to pH 6 with 1 mol/L hydrochloric acid, extracted with ethyl acetate (100 mL $\times$ 2), dried over anhydrous sodium sulphate and concentrated under reduced pressure to afford a crude (0.35 g). The above-mentioned crude (350 mg) was dissolved in 20 mL of DCM. Intermediate 2 (590 mg, 0.81 mmol), DMAP (200 mg, 1.64 mmol) and EDCI (310 mg, 1.62 mmol) were sequentially added and the mixture was reacted at room temperature for 16 h. To the reaction system was slowly added 30 mL of water and the mixture was extracted with 60 mL of DCM twice. The organic phase was washed with 50 mL of water, dried over anhydrous sodium sulphate and concentrated under reduced pressure and the resulting crude was separated and purified by silica gel column chromatography (dichloromethane/methanol (v/v) = 9 : 1) to afford 4d (310 mg, yield: 23%).

Step 4: Preparation of trifluoroacetate of compound 4

4d (310 mg, 0.27 mmol) was dissolved in 20 mL of tetrahydrofuran. Acetic acid (0.6 mL) and zinc powder (960 mg, 14.8 mmol) were sequentially added and the mixture was reacted at room temperature for 16 h. The reaction system was filtered and to the filtrate was added water (20 mL). The mixture was then extracted with 20 mL of ethyl acetate. The organic phase was separated, dried over anhydrous sodium sulphate and concentrated under reduced pressure to afford a crude (250 mg). The above-mentioned crude (250 mg) was dissolved in 10 mL of DCM. Triethylamine (0.2 mL), intermediate 1 (150 mg, 0.26 mmol) and HATU (142 mg, 0.37 mmol) were sequentially added and the mixture was reacted at room temperature for 1 h. To the reaction system was added 20 mL of water and the mixture was extracted with 20 mL of dichloromethane. The organic phase was washed with 20 mL of water, dried over anhydrous sodium sulphate and concentrated under reduced pressure and the resulting crude was passed through Pre-HPLC (instrument and preparative column: using Glison GX-281 preparative liquid phase chromatographic instrument, preparative column model: Sunfire C18, 5 $\mu$m, inner diameter $\times$ length = 30 mm $\times$ 150 mm). Preparation method: the crude was dissolved with methanol and dimethyl sulphoxide, and filtered with a 0.45 $\mu$m filter membrane, to prepare into a sample liquid. Mobile phase system: acetonitrile/water (containing 0.1% TFA). Gradient elution method: gradient elution with acetonitrile from 5% to 60% (elution time: 15 min). Lyophilisation was performed to afford the trifluoroacetate of compound 4 (20 mg).
$^1$H NMR (400 MHz, $CDCl_3$) $\delta$ 8.66 (s, 1H), 8.30 (s, 1H), 8.13 - 8.00 (m, 1H), 7.90 - 7.75 (m, 2H), 7.70 - 7.52 (m, 3H), 7.40 - 7.28 (m, 6H), 7.26 - 7.12 (m, 5H), 6.94 - 6.80 (m, 1H), 6.75 - 6.55 (m, 3H), 6.35 - 6.15 (m, 1H), 5.15 - 4.99 (m, 1H), 4.70 - 4.58 (m, 1H), 4.47 - 4.33 (m, 2H), 4.09 - 3.99 (m, 1H), 3.98 - 3.82 (m, 1H), 3.68 - 3.40 (m, 2H), 3.36 - 2.93 (m, 9H), 2.80 - 2.66 (m, 2H), 2.50 (s, 3H), 2.40 - 1.97 (m, 21H), 1.65 - 1.42 (m, 10H), 1.32 - 1.20 (m, 2H), 1.10 - 0.90 (m, 15H).
LCMS m/z = 767.4 $[M/2 +1]^+$.

**Example 5:**

(2S,4R)-1-((S)-2-(7-(4-((R)-3-((4-(N-(4-(4-((4-(4-chlorophenyl)-5,6-dihydro-2H-pyran-3-yl)methyl)piperazin-1-yl)benzoyl)sulphamoyl)-2-((trifluoromethyl) sulphonyl)phenyl)amino)-4-(phenylthio)butyl)piperazin-1-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide(**compound 5**)

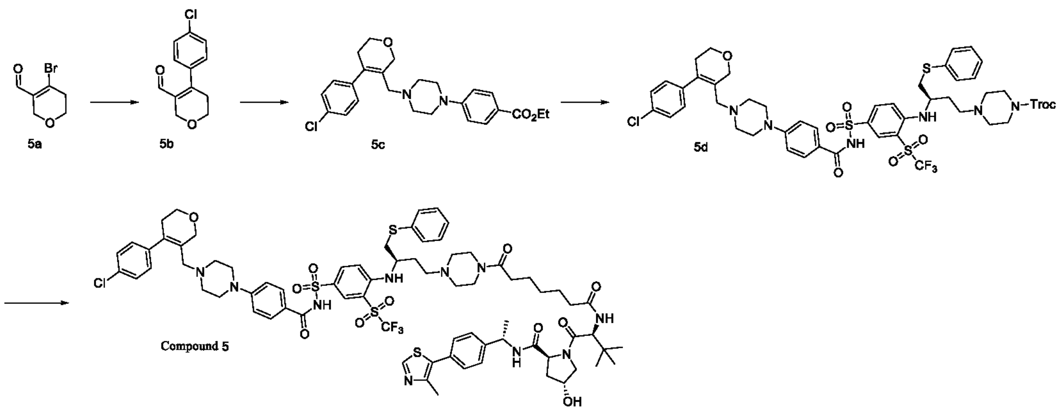

5a 5b 5c 5d Compound 5

Step 1: Preparation of 5b

Under nitrogen protection, 5a (see European Journal of Medicinal Chemistry, 2018, 149, 79 - 89 for the synthetic method) (2 g, 10.47 mmol) and (4-chlorophenyl)boronic acid (2.47 g, 15.80 mmol) were dissolved in 20 mL of dioxane and 2 mL of water. Potassium acetate (3.11 g, 31.69 mmol) and Pd(dppf)$Cl_2$ (0.2 g, 0.27 mmol) were sequentially added and the mixture was reacted at 90°C for 4 h. The reaction solution was cooled to room temperature and to the reaction solution was slowly added 100 mL of water. The mixture was extracted with ethyl acetate (60 mL × 2), dried over anhydrous sodium sulphate and concentrated under reduced pressure and the resulting crude was separated and purified by silica gel column chromatography (ethyl acetate/petroleum ether (v/v) = 0 : 1-1 : 10) to afford 5b (1.76 g, yield: 75%).

$^1$H NMR (400 MHz, $CDCl_3$) δ 9.51 (s, 1H), 7.46 - 7.36 (m, 2H), 7.26 - 7.18 (m, 2H), 4.50 - 4.40 (m, 2H), 3.92 (t, 2H), 2.67 - 2.59 (m, 2H).

Step 2: Preparation of 5c

5b (1.67 g, 7.50 mmol) was dissolved in 15 mL of tetrahydrofuran. Ethyl 4-(piperazin-1-yl)benzoate (1.64 g, 7.00 mmol) and 5 mL of tetraisopropyl titanate were sequentially added and the mixture was stirred at room temperature for 4 h. Then sodium triacetoxyborohydride (3.17 g, 14.96 mmol) was added and the resulting mixture was reacted at room temperature for 16 h. To the reaction solution was slowly added 20 mL of saturated sodium bicarbonate solution. The resulting mixture was extracted with ethyl acetate (60 mL × 2). The organic phase was washed with 50 mL of saturated sodium chloride solution, dried over anhydrous sodium sulphate and concentrated under reduced pressure and the resulting crude was separated and purified by silica gel column chromatography (ethyl acetate/petroleum ether (v/v) = 0 : 1-1 : 1) to afford 5c (2.01 g, yield: 61%).

Step 3: Preparation of 5d

5c (0.40 g, 0.91 mmol) was dissolved in 10 mL of methanol. Water (1 mL) and sodium hydroxide (0.15 g, 3.75 mmol) were added and the mixture was reacted at 80°C for 10 h. The reaction solution was cooled to room temperature and concentrated under reduced pressure. The residue was dissolved with 10 mL of water and then extracted with 20 mL of methyl tert-butyl ether to remove the impurities. The aqueous phase was separated, adjusted to pH 6 with 1 mol/L hydrochloric acid, extracted with ethyl acetate (100 mL × 2), dried over anhydrous sodium sulphate and concentrated under reduced pressure to afford a crude (0.20 g). The above-mentioned crude (200 mg) was dissolved in 15 mL of DCM. Intermediate 2 (330 mg, 0.45 mmol), DMAP (110 mg, 0.9 mmol) and EDCI (180 mg, 0.94 mmol) were sequentially added and the mixture was reacted at room temperature for 16 h. To the reaction system was slowly added 30 mL of water and the mixture was extracted with DCM (60 mL × 2). The organic phase was washed with 50 mL of water, dried over anhydrous sodium sulphate and concentrated under reduced pressure and the resulting crude was separated and purified by silica gel column chromatography (dichloromethane/methanol (v/v) = 9 : 1) to afford 5d (260 mg, yield: 25%).

Step 4: Preparation of compound 5

5d (260 mg, 0.23 mmol) was dissolved in 20 mL of tetrahydrofuran. Acetic acid (0.6 mL) and zinc powder (960 mg, 14.8 mmol) were sequentially added and the mixture was reacted at room temperature for 16 h. The reaction system was filtered and to the filtrate was added water (10 mL). The mixture was then extracted with 20 mL of ethyl acetate. The organic phase was separated, dried over anhydrous sodium sulphate and concentrated under reduced pressure to

afford a crude (250 mg). The above-mentioned crude (250 mg) was dissolved in 10 mL of DCM. Triethylamine (0.2 mL), intermediate 1 (154 mg, 0.27 mmol) and HATU (142 mg, 0.37 mmol) were sequentially added and the mixture was reacted at room temperature for 1 h. To the reaction system was added 20 mL of water and the mixture was extracted with 20 mL of ethyl acetate. The organic phase was washed with 20 mL of water, dried over anhydrous sodium sulphate and concentrated under reduced pressure and the resulting crude was passed through Pre-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude was dissolved with methanol and dimethyl sulphoxide, and filtered with a 0.45 μm filter membrane, to prepare into a sample liquid. Mobile phase system: acetonitrile/water (containing 0.1% TFA). Gradient elution method: gradient elution with acetonitrile from 5% to 60% (elution time: 15 min). To the preparative solution were added 100 mL of dichloromethane and 60 mL of saturated sodium bicarbonate solution and the mixture was stirred for 1 h. The organic phase was separated, dried over anhydrous sodium sulphate and concentrated under reduced pressure to afford compound 5 (40 mg, yield: 11%).

$^1$H NMR (400 MHz, $CDCl_3$) δ 8.68 (s, 1H), 8.35 (d, 1H), 8.09 (dd, 1H), 7.67 (d, 2H), 7.50 - 7.20 (m, 12H), 7.15 - 7.01 (m, 3H), 6.83 - 6.60 (m, 3H), 6.30 - 6.20 (m, 1H), 5.14 - 5.02 (m, 1H), 4.78 - 4.69 (m, 1H), 4.62 - 4.56 (m, 1H), 4.52 - 4.45 (m, 1H), 4.33 - 4.24 (m, 2H), 4.18 - 4.04 (m, 1H), 4.00 - 3.80 (m, 3H), 3.76 - 3.54 (m, 2H), 3.53 - 3.20 (m, 7H), 3.17 - 2.90 (m, 4H), 2.60 - 2.02 (m, 21H), 1.70 - 1.40 (m, 9H), 1.37 - 1.15 (m, 2H), 1.07 - 1.00 (m, 9H).

LCMS m/z = 758.5 $[M/2 +1]^+$.

**Example 6:**

(2S,4R)-1-((S)-2-(7-(4-((R)-3-((4-(N-(4-(4-((2-(4-chlorophenyl)cyclohept-1-en-1-yl)methyl)piperazin-1-yl)benzoyl)sulphamoyl)-2-((trifluoromethyl)sulphonyl) phenyl)amino)-4-(phenylthio)butyl)piperazin-1-yl)-7-oxoheptanamido)-3,3-dimethylbutanoyl)-4-hydroxy-N-((S)-1-(4-(4-methylthiazol-5-yl)phenyl)ethyl) pyrrolidine-2-carboxamide (**compound 6**) trifluoroacetate

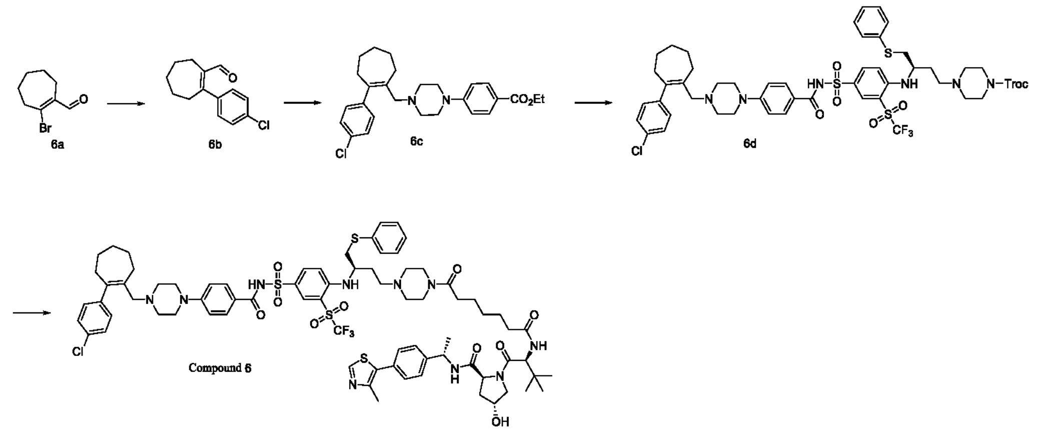
6a
6b
6c
6d
Compound 6

Step 1: Preparation of 6b

Under nitrogen protection, 6a (see WO 2020041406 for the synthetic method) (2 g, 9.85 mmol) and (4-chlorophenyl)boronic acid (2.47 g, 15.80 mmol) were sequentially dissolved in 20 mL of dioxane and 2 mL of water. Potassium acetate (3.11 g, 31.69 mmol) and Pd(dppf)$Cl_2$ (0.2 g, 0.27 mmol) were sequentially added and the mixture was reacted at 90°C for 4 h. The reaction solution was cooled to room temperature and to the reaction solution was slowly added 100 mL of water. The mixture was extracted with ethyl acetate (60 mL × 2), dried over anhydrous sodium sulphate and concentrated under reduced pressure and the resulting crude was separated and purified by silica gel column chromatography (ethyl acetate/petroleum ether (v/v) = 0 : 1-1 : 10) to afford 6b (2.0 g, yield: 87%).

$^1$H NMR (400 MHz, $CDCl_3$) δ 9.39 (s, 1H), 7.38 - 7.30 (m, 2H), 7.18 - 7.10 (m, 2H), 2.78 - 2.68 (m, 2H), 2.66 - 2.56 (m, 2H), 1.95 - 1.80 (m, 2H), 1.78 - 1.65 (m, 2H), 1.60 - 1.47 (m, 2H).

Step 2: Preparation of 6c

6b (1.75 g, 7.52 mmol) was dissolved in 15 mL of tetrahydrofuran. Ethyl 4-(piperazin-1-yl)benzoate (1.64 g, 7.00 mmol) and 5 mL of tetraisopropyl titanate were sequentially added and the mixture was stirred at room temperature for 4 h. Then sodium triacetoxyborohydride (3.17 g, 14.96 mmol) was added and the resulting mixture was reacted at room temperature for 16 h. To the reaction solution was slowly added 20 mL of saturated sodium bicarbonate solution.

The resulting mixture was extracted with ethyl acetate (60 mL × 2). The organic phase was washed with 50 mL of saturated sodium chloride solution, dried over anhydrous sodium sulphate and concentrated under reduced pressure and the resulting crude was separated and purified by silica gel column chromatography (ethyl acetate/petroleum ether (v/v) = 0 : 1-1 : 1) to afford 6c (2.0 g, yield: 63%).

$^1$H NMR (400 MHz, $CDCl_3$) δ 7.93 - 7.86 (m, 2H), 7.30 - 7.20 (m, 2H), 7.03 - 6.94 (m, 2H), 6.86 - 6.74 (m, 2H), 4.31 (q, 2H), 3.33 - 3.19 (m, 4H), 2.81 (s, 2H), 2.55 - 2.26 (m, 8H), 1.90 - 1.75 (m, 2H), 1.67 - 1.48 (m, 4H), 1.36 (t, 3H).

Step 3: Preparation of 6d

6c (0.40 g, 0.88 mmol) was dissolved in 10 mL of methanol. Water (1 mL) and sodium hydroxide (0.15 g, 3.75 mmol) were added and the mixture was reacted at 80°C for 10 h. The reaction solution was cooled to room temperature and concentrated under reduced pressure. The residue was dissolved with 10 mL of water and then extracted with 20 mL of methyl tert-butyl ether to remove the impurities. The aqueous phase was separated, adjusted to pH 6 with 1 mol/L hydrochloric acid, extracted with ethyl acetate (100 mL × 2), dried over anhydrous sodium sulphate and concentrated under reduced pressure to afford a crude (0.20 g). The above-mentioned crude (200 mg) was dissolved in 15 mL of DCM. Intermediate 2 (330 mg, 0.45 mmol), DMAP (110 mg, 0.9 mmol) and EDCI (180 mg, 0.94 mmol) were sequentially added and the mixture was reacted at room temperature for 16 h. To the reaction system was slowly added 30 mL of water and the mixture was extracted with DCM (60 mL × 2). The organic phase was washed with 50 mL of water, dried over anhydrous sodium sulphate and concentrated under reduced pressure and the resulting crude was separated and purified by silica gel column chromatography (dichloromethane/methanol (v/v) = 9 : 1) to afford 6d (260 mg, yield: 26%).

LCMS m/z = 568.2 $[M/2+1]^+$.

Step 4: Preparation of trifluoroacetate of compound 6

6d (260 mg, 0.23 mmol) was dissolved in 20 mL of tetrahydrofuran. Acetic acid (0.6 mL) and zinc powder (960 mg, 14.8 mmol) were sequentially added and the mixture was reacted at room temperature for 16 h. The reaction system was filtered and to the filtrate was added water (10 mL). The mixture was then extracted with 20 mL of ethyl acetate. The organic phase was separated, dried over anhydrous sodium sulphate and concentrated under reduced pressure to afford a crude (250 mg). The above-mentioned crude (250 mg) was dissolved in 10 mL of DCM. Triethylamine (0.2 mL), intermediate 1 (154 mg, 0.27 mmol) and HATU (142 mg, 0.37 mmol) were sequentially added and the mixture was reacted at room temperature for 1 h. To the reaction system was added 20 mL of water and the mixture was extracted with 20 mL of ethyl acetate. The organic phase was washed with 20 mL of water, dried over anhydrous sodium sulphate and concentrated under reduced pressure and the resulting crude was passed through Pre-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude was dissolved with methanol and dimethyl sulphoxide, and filtered with a 0.45 μm filter membrane, to prepare into a sample liquid. Mobile phase system: acetonitrile/water (containing 0.1% TFA). Gradient elution method: gradient elution with acetonitrile from 5% to 60% (elution time: 15 min). Lyophilisation was performed to afford the trifluoroacetate of compound 6 (20 mg).

$^1$H NMR (400 MHz, $CDCl_3$) δ 8.67 (s, 1H), 8.43 - 8.30 (m, 1H), 8.15 - 8.05 (m, 1H), 7.74 - 7.62 (m, 2H), 7.47 - 7.22 (m, 12H), 7.16 - 7.05 (m, 1H), 7.02 - 6.92 (m, 2H), 6.84 - 6.71 (m, 2H), 6.70 - 6.55 (m, 1H), 6.34 - 6.18 (m, 1H), 5.14 - 5.00 (m, 1H), 4.80 - 4.67 (m, 1H), 4.64 - 4.55 (m, 1H), 4.55 - 4.43 (m, 1H), 4.16 - 4.04 (m, 1H), 4.00 - 3.84 (m, 1H), 3.75 - 3.55 (m, 2H), 3.54 - 3.20 (m, 7H), 3.17 - 2.85 (m, 4H), 2.65 - 2.00 (m, 24H), 1.90 - 1.75 (m, 2H), 1.66 - 1.50 (m, 9H), 1.50 - 1.42 (m, 3H), 1.40 - 1.25 (m, 2H), 1.04 (s, 9H).

LCMS m/z = 764.4 $[M/2+1]^+$.

**Synthetic method of free-form compound 6:**

After the reaction in step 4, the system was quenched with water and extracted with ethyl acetate. The organic phase was washed with water, dried over anhydrous sodium sulphate and concentrated under reduced pressure. The resulting crude was subjected to preparative liquid phase chromatography to afford free-form compound 6.

Preparative liquid phase chromatography method:

instrument and preparative column: using SHIMADZU LC-20AP preparative liquid phase chromatographic instrument, preparative column model: Phenomenex C18. Preparation method: the crude was dissolved with acetonitrile and water to prepare into a sample liquid. Mobile phase system: acetonitrile/water (containing 10 mmol/L ammonium bicarbonate). Gradient elution method: gradient elution with acetonitrile from 50% to 70% (elution time: 20 min).

Nuclear magnetic resonance of free-form compound 6

$^1H$ NMR (400 MHz, $CDCl_3$) δ 8.67 (s, 1H), 8.40 - 8.31 (m, 1H), 8.13 - 8.04 (m, 1H), 7.78 - 7.63 (m, 2H), 7.46 - 7.18 (m, 12H), 7.12 - 7.02 (m, 1H), 7.02 - 6.92 (m, 2H), 6.80 - 6.68 (m, 2H), 6.67 - 6.56 (m, 1H), 6.43 - 6.31 (m, 1H), 5.13 - 4.99 (m, 1H), 4.78 - 4.67 (m, 1H), 4.66 - 4.55 (m, 1H), 4.54 - 4.44 (m, 1H), 4.14 - 4.02 (m, 1H), 3.98 - 3.82 (m, 1H), 3.71 - 3.54 (m, 2H), 3.50 - 3.18 (m, 7H), 3.16 - 2.85 (m, 4H), 2.59 - 1.96 (m, 24H), 1.90 - 1.75 (m, 2H), 1.75 - 1.50 (m, 9H), 1.50 - 1.40 (m, 3H), 1.40 - 1.16 (m, 2H), 1.04 (s, 9H).
LCMS m/z = 764.4 $[M/2+1]^+$.

**Example 7:**

6-(4-((4'-chloro-4,4-dimethyl-3,4,5,6-tetrahydro-[1,1'-biphenyl]-2-yl)methyl) piperazin-1-yl)-N-((4-(((R)-4-(4-(7-(((S)-1-((2S,4R)-4-hydroxy-2-(((S)-1-(4-(4-methylthiazol-5-yl)phenyl)ethyl)carbamoyl)pyrrolidin-1-yl)-3,3-dimethyl-1-oxobutan-2-yl)amino)-7-oxoheptanoyl)piperazin-1-yl)-1-(phenylthio)butan-2-yl)amino)-3-((trifluoromethyl)sulphonyl)phenyl)sulphonyl)nicotinamide (**compound 7**)

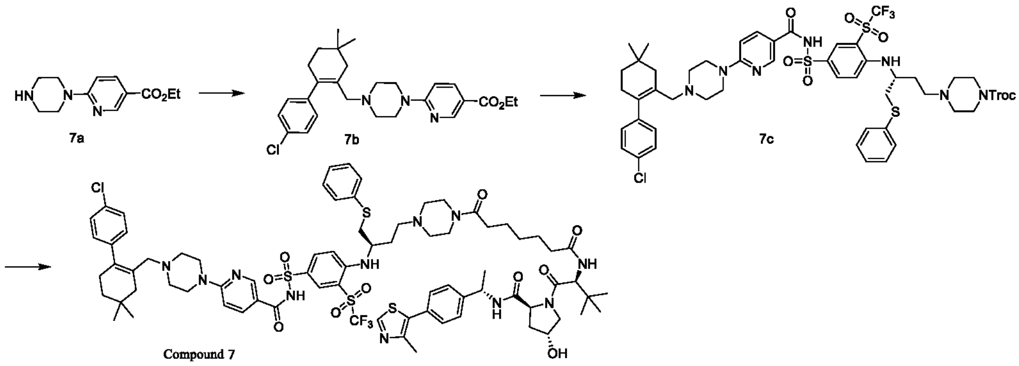

7a 7b 7c

Compound 7

Step 1: Preparation of 7b

4'-chloro-4,4-dimethyl-3,4,5,6-tetrahydro-[1,1'-biphenyl]-2-carbaldehyde (370 mg, 1.49 mmol) was dissolved in 15 mL of tetrahydrofuran. 7a (see CN 105985321 for the synthetic method) (350 mg, 1.49 mmol) and 1 mL of titanium isopropoxide were sequentially added and the mixture was stirred at room temperature for 4 h. Then sodium triacetoxyborohydride (947 mg, 4.47 mmol) was added and the resulting mixture was stirred at room temperature for 16 h. To the reaction solution was slowly added 20 mL of saturated aqueous sodium bicarbonate solution. The resulting mixture was extracted with 60 mL of ethyl acetate twice and the organic phase was washed with 50 mL of saturated sodium chloride solution, dried over anhydrous sodium sulphate and concentrated under reduced pressure. The resulting crude was separated and purified by silica gel column chromatography (ethyl acetate/petroleum ether (v/v) = 0 : 1-1 : 1) to afford 7b (320 mg, yield: 46%).
LCMS m/z = 468.2 $[M+1]^+$.

Step 2: Preparation of 7c

7b (0.320 g, 0.68 mmol) was dissolved in 10 mL of methanol. Water (1 mL) and sodium hydroxide (0.11 g, 2.75 mmol) were added and the mixture was reacted at 80°C for 10 h. The reaction system was cooled to room temperature and concentrated under reduced pressure. The residue was dissolved with 10 mL of water and then extracted with 20 mL of methyl tert-butyl ether to remove the impurities. The resulting mixture was adjusted to pH 6 with 1 mol/L hydrochloric acid, extracted with ethyl acetate (100 mL × 2), dried over anhydrous sodium sulphate and concentrated under reduced pressure to afford a crude (0.23 g). The above-mentioned crude (230 mg) was dissolved in 15 mL of DCM. Intermediate 2 (390 mg, 0.53 mmol), DMAP (200 mg, 1.64 mmol) and EDCI (130 mg, 0.68 mmol) were sequentially added and the mixture was reacted at room temperature for 16 h. To the reaction system was slowly added 30 mL of water and the mixture was extracted with 60 mL of DCM twice. The organic phase was washed with 50 mL of water, dried over anhydrous sodium sulphate and concentrated under reduced pressure and the resulting crude was separated and purified by silica gel column chromatography (dichloromethane/methanol (v/v) = 9 : 1) to afford 7c (260 mg, yield: 33%).
LCMS m/z = 575.8 $[1/2M+1]^+$.

Step 3: Preparation of compound 7

7c (260 mg, 0.23 mmol) was dissolved in 20 mL of tetrahydrofuran. Acetic acid (0.6 mL) and zinc powder (960 mg, 14.8 mmol) were sequentially added and the mixture was reacted at room temperature for 16 h. The reaction system was filtered and to the filtrate was added water (10 mL). The mixture was then extracted with 20 mL of ethyl acetate. The organic phase was separated, dried over anhydrous sodium sulphate and concentrated under reduced pressure to afford a crude (190 mg). The above-mentioned crude (190 mg) was dissolved in 10 mL of DCM. Triethylamine (0.2 mL), intermediate 1 (120 mg, 0.21 mmol) and HATU (110 mg, 0.29 mmol) were sequentially added and the mixture was reacted at room temperature for 1 h. To the reaction system was added 20 mL of water and the mixture was extracted with 20 mL of ethyl acetate. The organic phase was washed with 20 mL of water, dried over anhydrous sodium sulphate and concentrated under reduced pressure and the resulting crude was passed through Pre-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude was dissolved with methanol and dimethyl sulphoxide, and filtered with a 0.45 μm filter membrane, to prepare into a sample liquid. Mobile phase system: acetonitrile/water (containing 0.1% TFA). Gradient elution method: gradient elution with acetonitrile from 5% to 60% (elution time: 15 min). To the preparative solution were added 100 mL of DCM and 60 mL of saturated sodium bicarbonate solution and the mixture was stirred for 1 h. The organic phase was separated, dried over anhydrous sodium sulphate and concentrated under reduced pressure to afford compound 7 (60 mg, yield: 17%).

$^1H$ NMR (400 MHz, $CDCl_3$) δ 8.74 - 8.62 (m, 2H), 8.38 - 8.32 (m, 1H), 8.08 - 7.99 (m, 1H), 7.93 - 7.84 (m, 1H), 7.53 - 7.20 (m, 12H), 7.11 - 7.00 (m, 1H), 6.99 - 6.92 (m, 2H), 6.67 - 6.58 (m, 1H), 6.53 - 6.44 (m, 1H), 6.44 - 6.35 (m, 1H), 5.15 - 5.02 (m, 1H), 4.80 - 4.68 (m, 1H), 4.65 - 4.55 (m, 1H), 4.53 - 4.45 (m, 1H), 4.20 - 4.05 (m, 1H), 3.97 - 3.80 (m, 1H), 3.75 - 3.53 (m, 6H), 3.45 - 3.20 (m, 3H), 3.18 - 2.80 (m, 4H), 2.55 - 1.95 (m, 24H), 1.75 - 1.40 (m, 10H), 1.40 - 1.20 (m, 2H), 1.04 (s, 9H), 1.00 - 0.90 (m, 6H).

LCMS m/z = 771.8 $[M/2 +1]^+$.

**Example 8:**

cis-(2S,4R)-1-((2S)-2-(7-(5-((R)-3-((4-(N-(4-(4-((4'-chloro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)hexahydropyrrolo [3,4-c]pyrrol-2(1H)-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 8**)

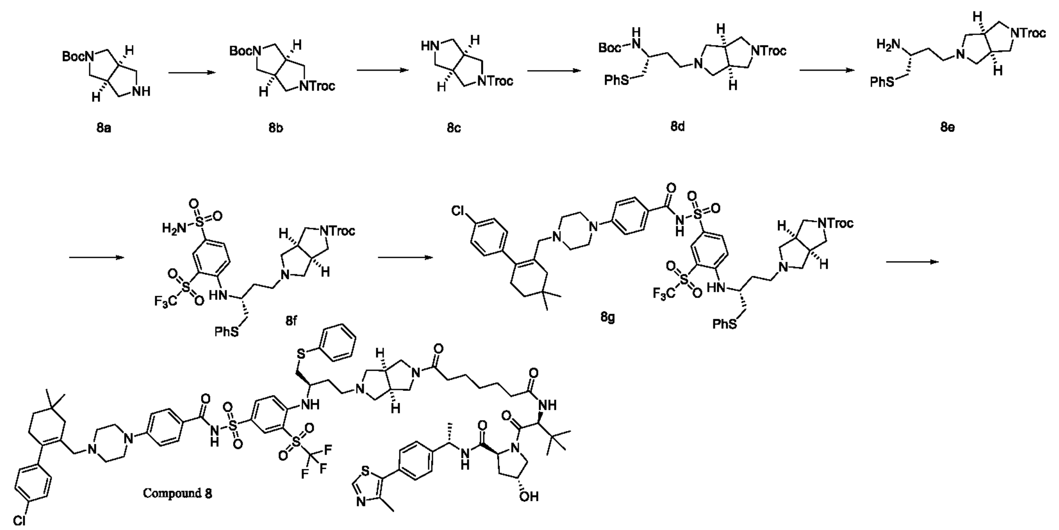
8a 8b 8c 8d 8e
8f 8g
Compound 8

Step 1: Preparation of 8b

8a (1.88 g, 8.86 mmol) was dissolved in 10 mL of dichloromethane and triethylamine (0.98 g, 9.68 mmol) was added. The mixture was cooled to 0°C and 2,2,2-trichloroethyl chloroformate (2.06 g, 9.72 mmol) was slowly added dropwise. Then the mixture was slowly warmed to room temperature and reacted for 2 h. After the reaction was completed, the reaction solution was diluted with 50 mL of dichloromethane and then 50 mL of water was added. The organic phase

was separated, dried over anhydrous sodium sulphate and concentrated under reduced pressure and the resulting crude was separated and purified by silica gel column chromatography (petroleum ether/ethyl acetate (v/v) =10 : 1-2 : 1) to afford 8b (2.4 g, yield: 70%).

Step 2: Preparation of 8c

8b (0.7 g, 1.81 mmol) was dissolved in 2 mL of dichloromethane. Trifluoroacetic acid (1 mL) was added and the mixture was reacted at room temperature for 3 h. The reaction solution was concentrated under reduced pressure. To the residue were added 20 mL of dichloromethane and 10 mL of water. The mixture was adjusted to pH 9 with saturated sodium bicarbonate solution. The organic phase was separated, dried over anhydrous sodium sulphate and concentrated under reduced pressure to afford crude 8c (0.5 g).
LCMS m/z = 287.0 $[M+1]^+$.

Step 3: Preparation of 8d

The above-mentioned crude 8c (0.5 g) was added to 10 mL of 1,2-dichloroethane. Tert-butyl (R)-(4-oxo-1-(phenylthio)butan-2-yl)carbamate (1.03 g, 3.49 mmol) and glacial acetic acid (0.1 mL) were added and the mixture was reacted at room temperature for 0.5 h. Sodium triacetoxyborohydride (0.74 g, 3.49 mmol) was added and the resulting mixture was reacted at room temperature for 19 h. Dichloromethane (50 mL) was added to dilute the reaction solution. Saturated sodium bicarbonate solution was used to adjust the reaction mixture to pH 9. The organic phase was separated, dried over anhydrous sodium sulphate and concentrated under reduced pressure. The resulting crude was separated and purified by chromatographic column on silica gel (dichloromethane : methanol (v/v) = 10 : 1) to afford 8d (0.64 g, yield over two steps from compound 8b: 62%).
LCMS m/z = 566.1 $[M+1]^+$.

Step 4: Preparation of trifluoroacetate of 8e

8d (0.64 g, 1.13 mmol) was dissolved in 3 mL of dichloromethane. Trifluoroacetic acid (1 mL) was added and the mixture was reacted at room temperature for 3 h. The reaction solution was concentrated under reduced pressure to afford crude trifluoroacetate of 8e (0.6 g).

Step 5: Preparation of 8f

The above-mentioned crude trifluoroacetate of 8e (0.64 g) was added to 4 mL of acetonitrile. Triethylamine (0.78 mL, 5.61 mmol) and 4-fluoro-3-((trifluoromethyl)sulphonyl)benzenesulphonamide (0.35 g, 1.14 mmol) were added and the mixture was refluxed and reacted at 80°C for 3 h. The reaction mixture was cooled to room temperature. The reaction solution was concentrated under reduced pressure and the resulting crude was separated and purified by chromatographic column on silica gel (dichloromethane : methanol (v/v) = 10 : 1) to afford 8f (0.88 g, yield over two steps from compound 8d: 97%).
LCMS m/z = 753.0 $[M+1]^+$.

Step 6: Preparation of 8g

8f (0.88 g, 1.17 mmol) was added to 10 mL of dichloromethane. 4-(4-((4'-chloro-4,4-dimethyl-3,4,5,6-tetrahydro-[1,1'-biphenyl]-2-yl)methyl)piperazin-1-yl)benzoic acid (0.51 g, 1.16 mmol), DMAP (0.29 g, 2.37 mmol) and EDCI (0.45 g, 2.35 mmol) were sequentially added and the mixture was reacted at room temperature for 12 h. To the reaction system was added 20 mL of water. The organic phase was separated, dried over anhydrous sodium sulphate and concentrated under reduced pressure and the resulting crude was separated and purified by chromatographic column on silica gel (dichloromethane : methanol (v/v) = 10 : 1) to afford 8g (0.85 g, yield: 62%).
LCMS m/z = 588.2 $[M/2+1]^+$.

Step 7: Preparation of compound 8

8g (0.44 g, 0.37 mmol) was added to 30 mL of tetrahydrofuran. Zinc powder (1.26 g, 19.27 mmol) and 0.78 mL of acetic acid were sequentially added and the mixture was reacted at room temperature for 5 h. The reaction system was filtered and the filtrate was concentrated under reduced pressure. The residue was then diluted with 100 mL of DCM and then 50 mL of saturated sodium bicarbonate solution was added. The organic phase was separated, dried over anhydrous sodium sulphate and concentrated under reduced pressure to afford a crude (0.11 g). The above-mentioned crude (0.11 g) was added to 8 mL of DCM. Intermediate 1 (65 mg, 0.11 mmol), triethylamine (0.15 mL, 1.08 mmol) and HATU (63 mg, 0.166 mmol) were sequentially added and the mixture was reacted at room temperature for 2 h. To the reaction system was added 20 mL of water and the mixture was extracted with 30 mL of DCM twice. The organic phase was separated, dried over anhydrous sodium sulphate and concentrated under reduced pressure and the resulting crude was passed through Pre-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude was dissolved with methanol and dimethyl sulphoxide, and filtered with a 0.45 μm filter membrane, to prepare into a sample liquid. Mobile phase system: acetonitrile/water (containing 0.1% TFA). Gradient elution method: gradient elution with acetonitrile from 5% to 60% (elution time: 15 min). The preparative solution was lyophilised and 2 mL of ethyl acetate and 1 mL of saturated sodium bicarbonate solution were added to the lyophilised solid. The mixture was stirred for 1 min and the organic phase was separated, dried over anhydrous sodium sulphate and concentrated under reduced pressure to afford compound 8 (18 mg, yield: 10%).

$^1H$ NMR (400 MHz, $CDCl_3$) δ 8.76 - 8.62 (m, 1H), 8.54 - 8.38 (m, 1H), 8.06 - 7.68 (m, 3H), 7.43 - 6.90 (m, 15H), 6.87 - 6.64 (m, 3H), 6.56 - 6.34 (m, 1H), 5.15 - 4.87 (m, 1H), 4.85 - 4.65 (m, 2H), 4.56 - 4.44 (m, 1H), 4.22 - 3.84 (m, 2H), 3.75 - 3.41 (m, 3H), 3.40 - 2.95 (m, 8H), 2.95 - 2.55 (m, 6H), 2.55 - 1.95 (m, 22H), 1.76 - 1.40 (m, 8H), 1.40 - 1.20 (m, 4H), 1.15 - 0.90 (m, 15H).

LCMS m/z = 784.5 $[M/2+1]^+$.

**Example 9:**

(2S,4R)-1-((S)-2-(7-(2-((R)-3-((4-(N-(4-(4-((4'-chloro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)-2,7-diazaspiro [3.5]nonan-7-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 9**) trifluoroacetate

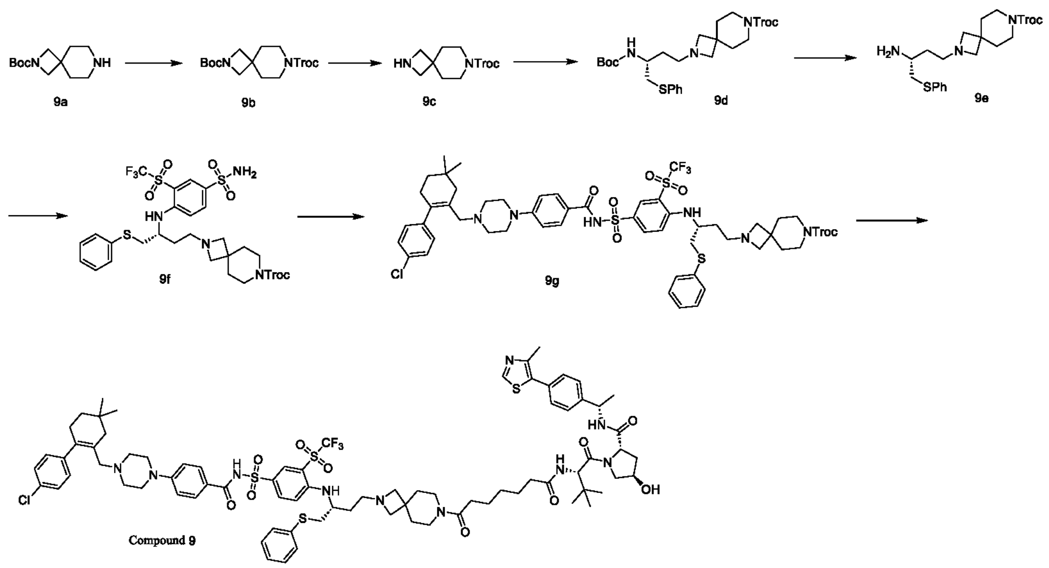
9a 9b 9c 9d 9e 9f 9g Compound 9

Step 1: Preparation of 9b

9a (5.00 g, 22.09 mmol) was dissolved in 25 mL of dichloromethane and triethylamine (2.46 g, 24.30 mmol) was added. The mixture was cooled to 0°C and 2,2,2-trichloroethyl chloroformate (5.15 g, 24.31 mmol) was slowly added dropwise. Then the mixture was slowly warmed to room temperature and reacted for 3 h. After the reaction was completed, the reaction solution was diluted with dichloromethane (50 mL) and then 50 mL of water was added. The organic phase was separated, dried over anhydrous sodium sulphate and concentrated under reduced pressure to afford crude 9b (8.00 g).

Step 2: Preparation of 9c

The above-mentioned crude 9b (8.00 g) was dissolved in 60 mL of dichloromethane. Trifluoroacetic acid (20

mL) was added and the mixture was reacted at room temperature for 3 h. The reaction solution was concentrated under reduced pressure and the residue was dissolved in 50 mL of dichloromethane. The mixture was adjusted to pH 8 with saturated aqueous sodium bicarbonate solution. Liquid separation was performed. The aqueous phase was extracted with 100 mL of dichloromethane twice, and the organic phases were combined and dried over anhydrous sodium sulphate. The reaction system was filtered and 50 mL of dichloromethane was added to the filter cake. Saturated sodium bicarbonate solution (50 mL) was added and the mixture was stirred until the solid was completely dissolved. The organic phase was separated, dried over anhydrous sodium sulphate and concentrated under reduced pressure to afford crude 9c (3.50 g).

LCMS m/z = 301.1 $[M+1]^+$.

Step 3: Preparation of 9d

The above-mentioned crude 9c (0.51 g) was dissolved in 20 mL of DCM. The mixture was cooled to 0°C. Tert-butyl (R)-(4-oxo-1-(phenylthio)butan-2-yl)carbamate (0.50 g, 1.69 mmol) and triethylamine (0.68 g, 6.72 mmol) were added and the mixture was reacted at 0°C for 15 min. Sodium triacetoxyborohydride (0.54 g, 2.55 mmol) was added and the resulting mixture was reacted at room temperature for 12 h. The reaction solution was concentrated under reduced pressure and the resulting crude was separated and purified by chromatographic column on silica gel (dichloromethane : methanol (v/v) = 20 : 1) to afford 9d (0.60 g, yield: 61%).

LCMS m/z = 580.2 $[M+1]^+$.

Step 4: Preparation of 9e

9d (0.60 g, 1.03 mmol) was dissolved in 6 mL of dichloromethane. Trifluoroacetic acid (2 mL) was added and the mixture was reacted at room temperature for 3 h. The reaction solution was concentrated under reduced pressure to afford crude 9e (0.5 g).

LCMS m/z = 480.1 $[M+1]^+$.

Step 5: Preparation of 9f

The above-mentioned crude 9e (0.5 g) was added to 10 mL of acetonitrile. DIPEA (0.54 g, 4.18 mmol) and 4-fluoro-3-((trifluoromethyl)sulphonyl) benzenesulphonamide (0.32 g, 1.04 mmol) were added and the mixture was refluxed and reacted at 85°C for 12 h. The reaction mixture was cooled to room temperature. The reaction solution was concentrated under reduced pressure and the resulting crude was separated and purified by chromatographic column on silica gel (dichloromethane : methanol (v/v) = 10 : 1) to afford 9f (0.2 g, yield over two steps from compound 9d: 25%).

LCMS m/z = 767.0 $[M+1]^+$.

Step 6: Preparation of 9g

9f (0.18 g, 0.23 mmol) was added to 6 mL of DCM. 4-(4-((4'-chloro-4,4-dimethyl-3,4,5,6-tetrahydro-[1,1'-biphenyl]-2-yl)methyl)piperazin-1-yl)benzoic acid (0.10 g, 0.23 mmol), DMAP (56 mg, 0.46 mmol) and EDCI (88 mg, 0.46 mmol) were sequentially added and the mixture was reacted at 40°C for 12 h. The reaction mixture was cooled to room temperature. To the reaction system was added 5 mL of water. Liquid separation was performed. The aqueous phase was extracted with 5 mL of DCM twice. The organic phases were combined, dried over anhydrous sodium sulphate and concentrated under reduced pressure and the crude was separated and purified by chromatographic column on silica gel (dichloromethane : methanol (v/v) = 10 : 1) to afford 9g (0.2 g, yield: 73%).

LCMS m/z = 1189.0 $[M+1]^+$.

Step 7: Preparation of trifluoroacetate of compound 9

9g (0.15 g, 0.13 mmol) was added to 12 mL of tetrahydrofuran. Zinc powder (0.56 g, 8.56 mmol) and 0.36 mL of acetic acid were sequentially added and the mixture was reacted at room temperature for 12 h. To the reaction solution was added 5 mL of water to quench the reaction. The mixture was filtered over diatomaceous earth and the filtrate was adjusted to pH 8 with saturated aqueous sodium bicarbonate solution and extracted with 6 mL of dichloromethane twice. The organic phases were combined, dried over anhydrous sodium sulphate and concentrated under reduced pressure. The residue was diluted with 100 mL of dichloromethane and 50 mL of saturated aqueous sodium bicarbonate solution was added. The organic phase was separated, dried over anhydrous sodium sulphate and concentrated under reduced pressure and the resulting crude was separated and purified by chromatographic column on silica gel (dichloromethane : methanol : triethylamine (v/v/v) = 100 : 10 : 1) to afford a crude (0.06 g). The above-mentioned crude (0.06 g) was added

to 3 mL of dichloromethane. Intermediate 1 (0.042 g, 0.071 mmol), triethylamine (0.03 g, 0.3 mmol) and HATU (0.027 g, 0.071 mmol) were sequentially added and the mixture was reacted at room temperature for 2 h. To the reaction solution was added 5 mL of water and liquid separation was performed. The aqueous phase was extracted with 6 mL of dichloromethane twice. The organic phases were combined, dried over anhydrous sodium sulphate and concentrated under reduced pressure and the resulting crude was passed through Pre-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude was dissolved with methanol and dimethyl sulphoxide, and filtered with a 0.45 μm filter membrane, to prepare into a sample liquid. Mobile phase system: acetonitrile/water (containing 0.1% TFA). Gradient elution method: gradient elution with acetonitrile from 5% to 60% (elution time: 15 min). Lyophilisation was performed to afford the trifluoroacetate of compound 9 (0.02 g).

$^1$H NMR (400 MHz, $CDCl_3$) δ 8.66 (s, 1H), 8.30 (s, 1H), 8.15 - 7.97 (m, 1H), 7.90 - 7.70 (m, 2H), 7.67 - 7.50 (m, 1H), 7.43 - 7.26 (m, 7H), 7.25 - 7.12 (m, 4H), 7.05 - 6.86 (m, 3H), 6.80 - 6.60 (m, 3H), 6.28 - 6.05 (m, 1H), 5.16 - 5.03 (m, 1H), 4.74 - 4.60 (m, 1H), 4.51 - 4.36 (m, 2H), 4.16 - 4.05 (m, 1H), 3.94 - 3.80 (m, 1H), 3.58 - 3.47 (m, 1H), 3.45 - 2.70 (m, 16H), 2.60 - 2.45 (m, 5H), 2.40 - 2.15 (m, 10H), 2.12 - 1.95 (m, 6H), 1.70 - 1.40 (m, 13H), 1.38 - 1.25 (m, 2H), 1.10 - 0.90 (m, 15H).

LCMS m/z = 791.6 $[M/2+1]^+$.

**Example 10:**

(2S,4R)-1-((S)-2-(7-((1R,4R)-5-((R)-3-((4-(N-(4-(4-((4'-chloro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)-2,5-diazabicyclo [2.2.1]heptan-2-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 10**)

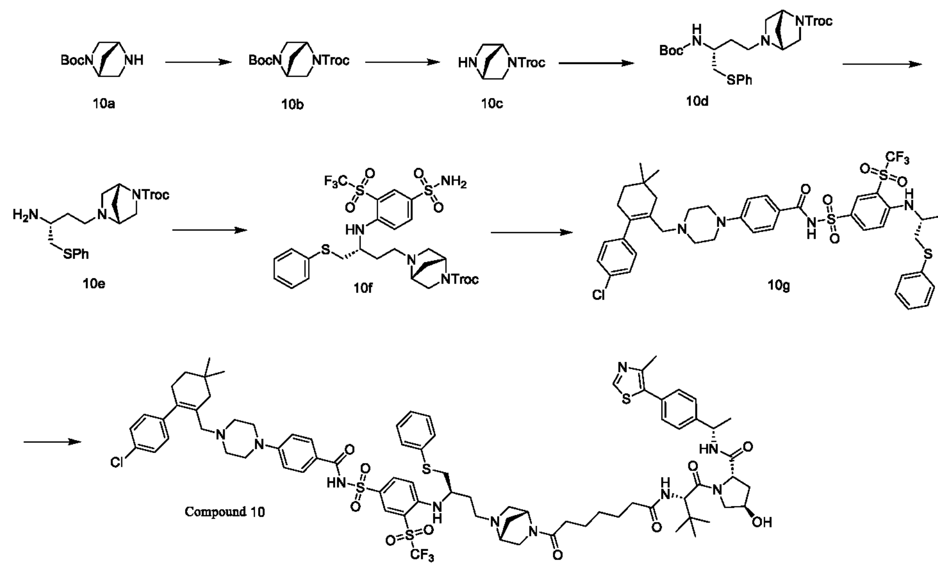
10a 10b 10c 10d 10e 10f 10g Compound 10

Step 1: Preparation of 10b

10a (1.90 g, 9.58 mmol) was dissolved in 10 mL of dichloromethane and triethylamine (1.07 g, 10.57 mmol) was added. The mixture was cooled to 0°C and 2,2,2-trichloroethyl chloroformate (2.24 g, 10.57 mmol) was slowly added dropwise. Then the mixture was slowly warmed to room temperature and reacted for 3 h. After the reaction was completed, the reaction was quenched with 15 mL of water and liquid separation was performed. The aqueous phase was extracted with 15 mL of dichloromethane. The organic phases were combined, dried over anhydrous sodium sulphate and concentrated under reduced pressure to afford crude 10b (3.25 g).

Step 2: Preparation of 10c

The above-mentioned crude 10b (3.25 g) was dissolved in 20 mL of DCM. Trifluoroacetic acid (7 mL) was added and the mixture was reacted at room temperature for 3 h. The reaction solution was concentrated under reduced

pressure and the residue was dissolved with 50 mL of DCM and adjusted to pH 8 with saturated aqueous sodium bicarbonate solution. Liquid separation was performed. The aqueous phase was extracted with 100 mL of DCM twice and the organic phases were combined, dried over anhydrous sodium sulphate and concentrated under reduced pressure to afford crude 10c (2.30 g).

Step 3: Preparation of 10d

The above-mentioned crude 10c (0.93 g) was dissolved in 20 mL of DCM. The mixture was cooled to 0°C. Tert-butyl (R)-(4-oxo-1-(phenylthio)butan-2-yl)carbamate (1.00 g, 3.38 mmol) and triethylamine (1.37 g, 13.54 mmol) were added and the mixture was reacted at 0°C for 15 min. Sodium triacetoxyborohydride (1.08 g, 5.10 mmol) was added and the resulting mixture was reacted at room temperature for 12 h. The reaction solution was concentrated under reduced pressure and the resulting crude was separated and purified by chromatographic column on silica gel (dichloromethane : methanol (v/v) = 20 : 1) to afford 10d (1.20 g, yield: 64%).

LCMS m/z = 552.1 $[M+1]^+$.

Step 4: Preparation of trifluoroacetate of 10e

10d (1.20 g, 2.18 mmol) was dissolved in 9 mL of DCM. Trifluoroacetic acid (3 mL) was added and the mixture was reacted at room temperature for 3 h. The reaction solution was concentrated under reduced pressure to afford crude trifluoroacetate of 10e (1.48 g).

Step 5: Preparation of 10f

The above-mentioned crude trifluoroacetate of 10e (1.48 g) was added to 10 mL of acetonitrile. DIPEA (1.40 g, 10.83 mmol) and 4-fluoro-3-((trifluoromethyl)sulphonyl)benzenesulphonamide (0.67 g, 2.18 mmol) were added and the mixture was refluxed and reacted at 85°C for 12 h. The reaction mixture was cooled to room temperature. The reaction solution was concentrated under reduced pressure and the resulting crude was separated and purified by chromatographic column on silica gel (dichloromethane : methanol (v/v) = 10 : 1) to afford 10f (1.46 g, yield over two steps from compound 10d: 91%).

LCMS m/z = 739.0 $[M+1]^+$.

Step 6: Preparation of 10g

10f (0.66 g, 0.89 mmol) was added to 6 mL of DCM. 4-(4-((4'-chloro-4,4-dimethyl-3,4,5,6-tetrahydro-[1,1'-biphenyl]-2-yl)methyl)piperazin-1-yl)benzoic acid (0.39 g, 0.90 mmol), DMAP (0.22 g, 1.81 mmol) and EDCI (0.34 g, 1.78 mmol) were sequentially added and the mixture was reacted at 35°C for 12 h. The reaction mixture was cooled to room temperature. To the reaction system were added 5 mL of water and 3 mL of DCM. Liquid separation was performed. The aqueous phase was extracted with 5 mL of DCM twice. The organic phases were combined, dried over anhydrous sodium sulphate and concentrated under reduced pressure and the crude was separated and purified by chromatographic column on silica gel (dichloromethane : methanol (v/v) = 10 : 1) to afford 10g (0.77 g, yield: 75%).

LCMS m/z = 581.2 $[M/2+1]^+$.

Step 7: **Preparation of** compound 10

10g (0.77 g, 0.66 mmol) was added to 60 mL of tetrahydrofuran. Zinc powder (2.28 g, 34.85 mmol) and 1.42 mL of acetic acid were sequentially added and the mixture was reacted at room temperature for 4 h. To the reaction solution was added 25 mL of water to quench the reaction. The mixture was filtered over diatomaceous earth and the filtrate was adjusted to pH 8 with saturated aqueous sodium bicarbonate solution and extracted with 6 mL of DCM twice. The organic phases were combined, dried over anhydrous sodium sulphate and concentrated under reduced pressure and the resulting crude was separated and purified by chromatographic column on silica gel (dichloromethane : methanol : triethylamine (v/v/v) = 100 : 10 : 1) to afford a crude (0.33 g). The above-mentioned crude (0.33 g) was added to 5 mL of DCM. Intermediate 1 (0.21 g, 0.36 mmol), triethylamine (0.17 g, 1.68 mmol) and HATU (0.19 g, 0.5 mmol) were sequentially added and the mixture was reacted at room temperature for 2 h. To the reaction solution was added 5 mL of water and liquid separation was performed. The aqueous phase was extracted with 6 mL of DCM twice and the organic phases were combined, dried over anhydrous sodium sulphate and concentrated under reduced pressure. The resulting crude was preliminarily separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex

C18). Mobile phase system: acetonitrile/water containing 10 mmol/L $NH_4HCO_3$. Gradient elution method: gradient elution with acetonitrile from 51% to 81% (elution time: 15 min). Lyophilisation was performed to afford compound 10 (0.13 g, yield: 13%).

$^1H$ NMR (400 MHz, $CDCl_3$) $\delta$ 8.66 (s, 1H), 8.41 - 8.32 (m, 1H), 8.14 - 7.92 (m, 1H), 7.80 - 7.62 (m, 2H), 7.44 - 7.26 (m, 10H), 7.26 - 7.10 (m, 3H), 7.04 - 6.92 (m, 2H), 6.84 - 6.70 (m, 2H), 6.68 - 6.53 (m, 1H), 6.40 - 6.20 (m, 1H), 5.15 - 5.00 (m, 1H), 4.82 - 3.80 (m, 6H), 3.67 - 2.95 (m, 10H), 2.93 - 2.55 (m, 4H), 2.55 - 1.88 (m, 20H), 1.85 - 1.40 (m, 12H), 1.38 - 1.20 (m, 2H), 1.10 - 0.90 (m, 15H).

LCMS m/z = 518.8 $[M/3+1]^+$.

**Example 11:**

(2S,4R)-1-((S)-2-(7-(4-((R)-3-((4-(N-(4-(4-((6-(4-chlorophenyl)spiro[2.5]oct-5-en-5-yl)methyl)piperazin-1-yl)benzoyl)sulphamoyl)-2-((trifluoromethyl)sulphonyl) phenyl)amino)-4-(phenylthio)butyl)piperazin-1-yl)-7-oxoheptanamido)-3,3-dimethylbutanoyl)-4-hydroxy-N-((S)-1-(4-(4-methylthiazol-5-yl)phenyl)ethyl) pyrrolidine-2-carboxamide (**compound 11**)

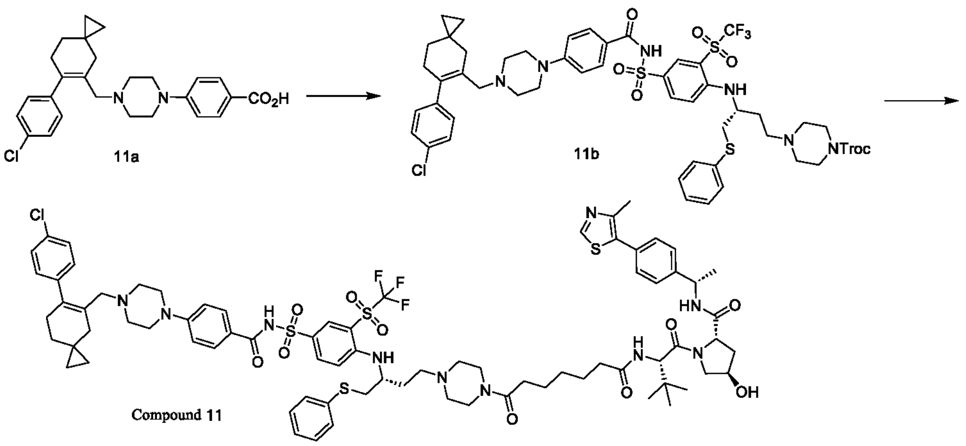

11a

11b

Compound 11

Step 1: Preparation of 11b

11a (see WO 2013185202 for the synthetic method) (350 mg, 0.8 mmol) was dissolved in 15 mL of DCM. Intermediate 2 (583 mg, 0.79 mmol), DMAP (195 mg, 1.6 mmol) and EDCI (306 mg, 1.6 mmol) were sequentially added and the mixture was reacted at room temperature for 16 h. To the reaction system was slowly added 100 mL of water and the mixture was extracted with 60 mL of DCM twice. The organic phase was dried over anhydrous sodium sulphate and concentrated under reduced pressure and the resulting crude was separated and purified by silica gel column chromatography (dichloromethane/methanol (v/v) = 9 : 1) to afford 11b (550 mg, yield: 60%).

LCMS m/z = 574.1 $[M/2 +1]^+$.

Step 2: Preparation of compound 11

11b (260 mg, 0.23 mmol) was dissolved in 20 mL of tetrahydrofuran. Acetic acid (0.6 mL) and zinc powder (960 mg, 14.8 mmol) were sequentially added and the mixture was reacted at room temperature for 16 h. The reaction system was filtered and to the filtrate was added water (10 mL). The mixture was then extracted with 20 mL of ethyl acetate. The organic phase was separated, dried over anhydrous sodium sulphate and concentrated under reduced pressure. The residue was separated and purified by chromatographic column on silica gel (methanol/dichloromethane/triethylamine (v/v) = 19 : 1 : 0-89 : 10 : 1) to afford a crude (160 mg). The above-mentioned crude (160 mg) was dissolved in 15 mL of DCM. Triethylamine (0.22 mL), intermediate 1 (95 mg, 0.17 mmol) and HATU (110 mg, 0.29 mmol) were sequentially added and the mixture was reacted at room temperature for 2 h. To the reaction solution was added 100 mL of water. The mixture was extracted with 50 mL of DCM, dried over anhydrous sodium sulphate and concentrated under reduced pressure. The residue was separated and purified by silica gel column chromatography (methanol/dichloromethane (v/v) = 0 : 1-1 : 9) and the resulting crude was passed through Pre-HPLC (instrument and preparative column: using SHIMADZU LC-20AP preparative liquid phase chromatographic instrument, preparative column model: Phenomenex C18, mobile phase system: acetonitrile/water containing 10 mmol/L $NH_4HCO_3$. Gradient elution method: gradient elution with acetonitrile from 58% to 88% (elution time: 10 min)). Lyophilisation was performed to afford compound 11 (50 mg, yield: 14%).

$^1H$ NMR (400 MHz, $CDCl_3$) $\delta$ 8.67 (s, 1H), 8.37 - 8.30 (m, 1H), 8.09 (dd, 1H), 7.71 (d, 2H), 7.44 - 7.19 (m, 12H), 7.13 - 6.97 (m, 3H), 6.75 (d, 2H), 6.62 (d, 1H), 6.39 (d, 1H), 5.15 - 5.00 (m, 1H), 4.80 - 4.67 (m, 1H), 4.61 (d, 1H), 4.55 - 4.43 (m, 1H), 4.14 - 4.02 (m, 1H), 3.99 - 3.82 (m, 1H), 3.75 - 3.53 (m, 2H), 3.50 - 3.17 (m, 7H), 3.16 - 2.95 (m, 2H), 2.89 (s, 2H), 2.57 - 1.97 (m, 24H), 1.76 - 1.40 (m, 10H), 1.38 - 1.22 (m, 2H), 1.04 (s, 9H), 0.35 (s, 4H).

LCMS m/z = 770.8 $[M/2+1]^+$.

**Example 12:**

(2S,4R)-1-((S)-2-(7-(4-((R)-3-((4-(N-(4-(4-((4'-chloro-2'-fluoro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)piperazin-1-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 12**)

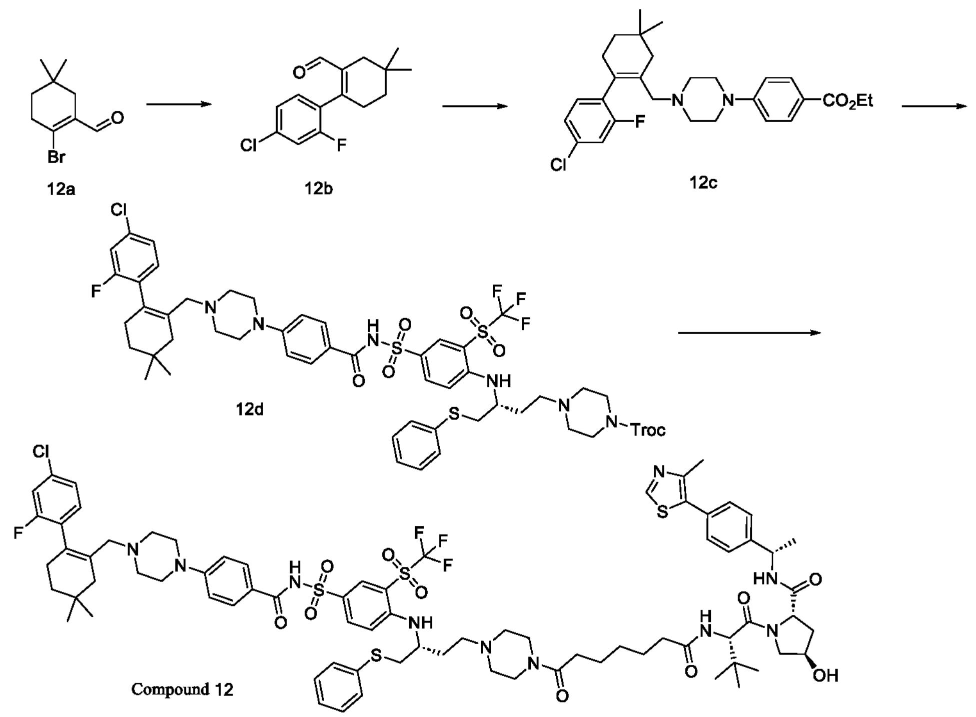
12a
12b
12c
12d
Compound 12

Step 1: Preparation of 12b

Under nitrogen protection, 12a (see WO 2021066873 for the synthetic method) (2 g, 9.21 mmol) and (4-chloro-2-fluorophenyl)boronic acid (2.41 g, 13.82 mmol) were dissolved in 20 mL of dioxane and 2 mL of water. Potassium acetate (2.71 g, 27.61 mmol) and $Pd(dppf)Cl_2$ (0.2 g, 0.27 mmol) were sequentially added and the mixture was reacted at 90°C for 4 h. The reaction solution was cooled to room temperature and to the reaction solution was slowly added 100 mL of water. The mixture was extracted with ethyl acetate (60 mL $\times$ 2), dried over anhydrous sodium sulphate and concentrated under reduced pressure and the resulting crude was separated and purified by silica gel column chromatography (ethyl acetate/petroleum ether (v/v) = 0 : 1-1 : 10) to afford 12b (2.0 g, yield: 81%).

Step 2: Preparation of 12c

12b (2.4 g, 9.0 mmol) was dissolved in 15 mL of DCE. Ethyl 4-(piperazin-1-yl)benzoate (2.10 g, 9.0 mmol) and 5 mL of tetraisopropyl titanate were sequentially added and the mixture was stirred at room temperature for 3 h. Then sodium triacetoxyborohydride (3.81 g, 17.98 mmol) was added and the resulting mixture was reacted at room temperature for 16 h. To the reaction solution was slowly added 20 mL of saturated sodium bicarbonate solution. The resulting mixture

was extracted with ethyl acetate (60 mL × 2). The organic phase was washed with 50 mL of saturated sodium chloride solution, dried over anhydrous sodium sulphate and concentrated under reduced pressure and the resulting crude was separated and purified by silica gel column chromatography (ethyl acetate/petroleum ether (v/v) = 0 : 1-1 : 1) to afford 12c (1.0 g, yield: 23%).

LCMS m/z = 485.2 $[M+1]^+$.

Step 3: Preparation of 12d

12c (1.4 g, 2.89 mmol) was dissolved in 10 mL of methanol. Water (1 mL) and sodium hydroxide (0.35 g, 8.75 mmol) were added and the mixture was reacted at 80°C for 10 h. The reaction solution was cooled to room temperature and concentrated under reduced pressure. The residue was dissolved with 10 mL of water and then washed with 20 mL of methyl tert-butyl ether. The aqueous phase was separated, adjusted to pH 6 with 1 mol/L hydrochloric acid, extracted with ethyl acetate (100 mL × 2), dried over anhydrous sodium sulphate and concentrated under reduced pressure to afford a crude (0.8 g). The above-mentioned crude (0.4 g) was dissolved in 15 mL of DCM. Intermediate 2 (0.64 g, 0.87 mmol), DMAP (320 mg, 2.62 mmol) and EDCI (340 mg, 1.77 mmol) were sequentially added and the mixture was reacted at room temperature for 16 h. To the reaction system was slowly added 30 mL of water and the mixture was extracted with DCM (60 mL × 2). The organic phase was washed with 50 mL of water, dried over anhydrous sodium sulphate and concentrated under reduced pressure and the resulting crude was separated and purified by silica gel column chromatography (dichloromethane/methanol (v/v) = 9 : 1) to afford 12d (0.6 g, yield: 59%).

LCMS m/z = 584.1 $[M/2+1]^+$.

Step 4: Preparation of compound 12

12d (0.5 g, 0.43 mmol) was dissolved in 20 mL of tetrahydrofuran. Acetic acid (1.6 g, 26.64 mmol) and zinc powder (1.6 g, 24.67 mmol) were sequentially added and the mixture was reacted at room temperature for 16 h. The reaction system was filtered and to the filtrate was added water (10 mL). The mixture was then extracted with 20 mL of ethyl acetate. The organic phase was separated, dried over anhydrous sodium sulphate and concentrated under reduced pressure to afford a crude (0.2 g). The above-mentioned crude (0.2 g) was dissolved in 10 mL of DCM. Triethylamine (0.2 mL), intermediate 1 and HATU (0.15 g, 0.4 mmol) were sequentially added and the mixture was reacted at room temperature for 1 h. To the reaction system was added 20 mL of water and the mixture was extracted with 20 mL of ethyl acetate. The organic phase was washed with 20 mL of water, dried over anhydrous sodium sulphate and concentrated under reduced pressure and the resulting crude was passed through Pre-HPLC (instrument and preparative column: using SHIMADZU LC-20AP preparative liquid phase chromatographic instrument, preparative column model: Phenomenex C18). Mobile phase system: acetonitrile/water containing 10 mmol/L $NH_4HCO_3$. Gradient elution method: gradient elution with acetonitrile from 54% to 74% (elution time: 15 min). Lyophilisation was performed to afford compound 12 (80 mg, yield: 12%).

$^1H$ NMR (400 MHz, $CDCl_3$) δ 8.67 (s, 1H), 8.38 - 8.32 (m, 1H), 8.09 (dd, 1H), 7.70 (d, 2H), 7.45 - 7.18 (m, 10H), 7.15 - 6.87 (m, 4H), 6.84 - 6.70 (m, 2H), 6.62 (d, 1H), 6.34 (d, 1H), 5.15 - 5.00 (m, 1H), 4.80 - 4.68 (m, 1H), 4.66 - 4.56 (m, 1H), 4.54 - 4.44 (m, 1H), 4.15 - 4.03 (m, 1H), 3.98 - 3.82 (m, 1H), 3.73 - 3.53 (m, 2H), 3.51 - 3.20 (m, 7H), 3.16 - 2.93 (m, 2H), 2.75 (s, 2H), 2.55 - 1.95 (m, 24H), 1.80 - 1.43 (m, 10H), 1.40 - 1.22 (m, 2H), 1.10 - 0.94 (m, 15H).

LCMS m/z = 520.8 $[M/3+1]^+$.

**Example 13:**

(2S,4R)-1-((S)-2-(7-((1S,4S)-5-((R)-3-((4-(N-(4-(4-((4'-chloro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)-2,5-diazabicyclo [2.2.1]heptan-2-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 13**)

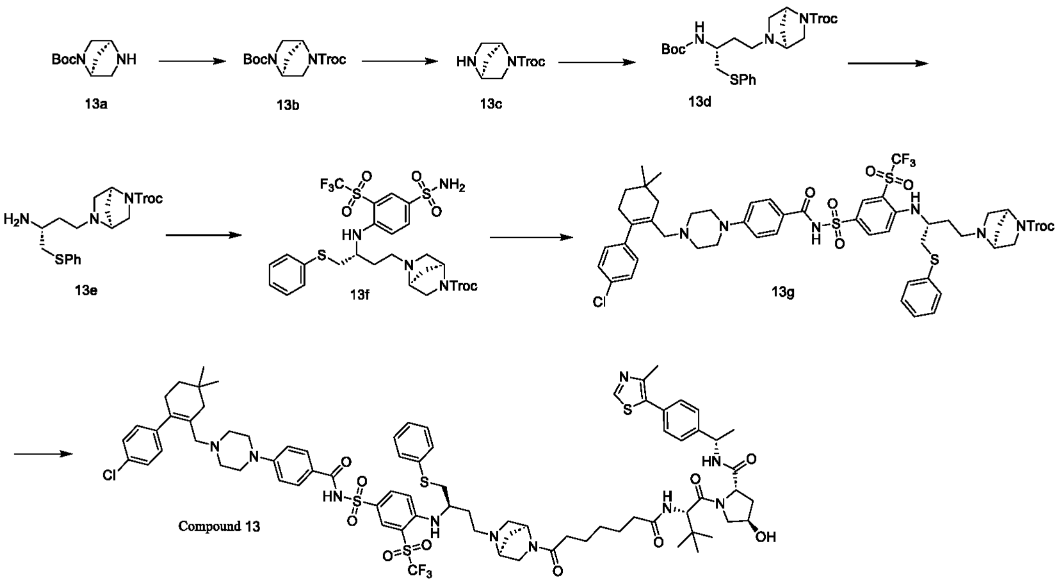

13a 13b 13c 13d 13e 13f 13g

Compound 13

Step 1: Preparation of 13b

13a (1.90 g, 9.58 mmol) was dissolved in 10 mL of DCM and triethylamine (1.07 g, 10.57 mmol) was added. The mixture was cooled to 0°C and 2,2,2-trichloroethyl chloroformate (2.24 g, 10.57 mmol) was slowly added dropwise. Then the mixture was slowly warmed to room temperature and reacted for 3 h. After the reaction was completed, the reaction was quenched with 15 mL of water and liquid separation was performed. The aqueous phase was extracted with 15 mL of DCM. The organic phases were combined, dried over anhydrous sodium sulphate and concentrated under reduced pressure to afford crude 13b (3.25 g).

Step 2: Preparation of 13c

The above-mentioned crude 13b (3.0 g) was dissolved in 20 mL of DCM. Trifluoroacetic acid (7 mL) was added and the mixture was reacted at room temperature for 3 h. The reaction solution was concentrated under reduced pressure and the residue was dissolved with 50 mL of DCM and adjusted to pH 8 with saturated aqueous sodium bicarbonate solution. Liquid separation was performed. The aqueous phase was extracted with 100 mL of DCM twice and the organic phases were combined, dried over anhydrous sodium sulphate and concentrated under reduced pressure to afford crude 13c (2.0 g).

Step 3: Preparation of 13d

The above-mentioned crude 13c (1.00 g) was dissolved in 20 mL of DCM. The mixture was cooled to 0°C. Tert-butyl (R)-(4-oxo-1-(phenylthio)butan-2-yl)carbamate (1.08 g, 3.65 mmol) and triethylamine (1.37 g, 13.54 mmol) were added and the mixture was reacted at 0°C for 15 min. Sodium triacetoxyborohydride (1.08 g, 5.10 mmol) was added and the resulting mixture was reacted at room temperature for 12 h. The reaction solution was concentrated under reduced pressure and the resulting crude was separated and purified by chromatographic column on silica gel (dichloromethane : methanol (v/v) = 20 : 1) to afford 13d (1.2 g, yield: 59%).

Step 4: Preparation of trifluoroacetate of 13e

13d (1.20 g, 2.18 mmol) was dissolved in 9 mL of DCM. Trifluoroacetic acid (3 mL) was added and the mixture was reacted at room temperature for 3 h. The reaction solution was concentrated under reduced pressure to afford crude trifluoroacetate of 13e (1.48 g).

Step 5: Preparation of 13f

The above-mentioned crude trifluoroacetate of 13e (1.48 g) was added to 10 mL of acetonitrile. DIPEA (1.40 g, 10.83 mmol) and 4-fluoro-3-((trifluoromethyl)sulphonyl)benzenesulphonamide (0.67 g, 2.18 mmol) were added and the mixture was refluxed and reacted at 85°C for 12 h. The reaction mixture was cooled to room temperature. The

reaction solution was concentrated under reduced pressure and the resulting crude was separated and purified by chromatographic column on silica gel (dichloromethane : methanol (v/v) = 10 : 1) to afford 13f (1.46 g, yield over two steps from compound 13d: 91%).

LCMS m/z = 739.0 $[M+1]^+$.

Step 6: Preparation of 13g

13f (0.7 g, 0.95 mmol) was added to 6 mL of DCM. 4-(4-((4'-chloro-4,4-dimethyl-3,4,5,6-tetrahydro-[1,1'-biphenyl]-2-yl)methyl)piperazin-1-yl)benzoic acid (0.42 g, 0.97 mmol), DMAP (0.35 g, 2.88 mmol) and EDCI (0.36 g, 1.88 mmol) were sequentially added and the mixture was reacted at 35°C for 12 h. The reaction mixture was cooled to room temperature. To the reaction system were added 5 mL of water and 3 mL of DCM. Liquid separation was performed. The aqueous phase was extracted with 5 mL of DCM twice. The organic phases were combined, dried over anhydrous sodium sulphate and concentrated under reduced pressure and the crude was separated and purified by chromatographic column on silica gel (dichloromethane : methanol (v/v) = 10 : 1) to afford 13g (0.5 g, yield: 45%).

LCMS m/z = 581.2 $[M/2+1]^+$.

Step 7: Preparation of compound 13

13g (0.5 g, 0.43 mmol) was added to 60 mL of tetrahydrofuran. Zinc powder (1.6 g, 24.46 mmol) and 1.6 mL of acetic acid were sequentially added and the mixture was reacted at room temperature for 4 h. To the reaction solution was added 25 mL of water to quench the reaction. The mixture was filtered over diatomaceous earth and the filtrate was adjusted to pH 8 with saturated aqueous sodium bicarbonate solution and extracted with 6 mL of DCM twice. The organic phases were combined, dried over anhydrous sodium sulphate and concentrated under reduced pressure and the resulting crude was separated and purified by chromatographic column on silica gel (dichloromethane : methanol : triethylamine (v/v) = 100 : 10 : 1) to afford a crude (0.2 g). The above-mentioned crude (0.2 g) was added to 5 mL of DCM. Intermediate 1 (0.12 g, 0.21 mmol), triethylamine (0.061 g, 0.6 mmol) and HATU (0.15 g, 0.39 mmol) were sequentially added and the mixture was reacted at room temperature for 2 h. To the reaction solution was added 5 mL of water and liquid separation was performed. The aqueous phase was extracted with 6 mL of DCM twice and the organic phases were combined, dried over anhydrous sodium sulphate and concentrated under reduced pressure. The resulting crude was preliminarily separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water. Gradient elution method: gradient elution with acetonitrile from 60% to 90% (elution time: 15 min). Lyophilisation was performed to afford compound 13 (0.1 g, yield: 15%).

$^1$H NMR (400 MHz, $CDCl_3$) $\delta$ 8.66 (s, 1H), 8.45 - 8.32 (m, 1H), 8.06 - 7.93 (m, 1H), 7.82 - 7.66 (m, 2H), 7.43 - 7.11 (m, 13H), 7.03 - 6.92 (m, 2H), 6.83 - 6.56 (m, 3H), 6.53 - 6.32 (m, 1H), 5.15 - 4.90 (m, 1H), 4.84 - 4.17 (m, 4H), 4.14 - 3.86 (m, 2H), 3.68 - 2.91 (m, 10H), 2.88 - 2.70 (m, 3H), 2.70 - 1.75 (m, 22H), 1.75 - 1.22 (m, 13H), 1.12 - 0.88 (m, 15H).

LCMS m/z = 518.8 $[M/3+1]^+$.

**Example 14:**

cis-(2S,4R)-1-((S)-2-(7-(5-((R)-3-((4-(N-(4-(4-((4'-chloro-2'-fluoro-4,4-dimethyl-3,4,5,6-tetrahydro-[1,1'-biphenyl]-2-yl)methyl)piperazin-1-yl)benzoyl) sulphamoyl)-2-((trifluoromethyl)sulphonyl)phenyl)amino)-4-(phenylthio)butyl) hexahydropyrrolo[3,4-c]pyrrol-2(1H)-yl)-7-oxoheptanamido)-3,3-dimethylbutanoyl)-4-hydroxy-N-((S)-1-(4-(4-methylthiazol-5-yl)phenyl)ethyl) pyrrolidine-2-carboxamide (**compound 14**)

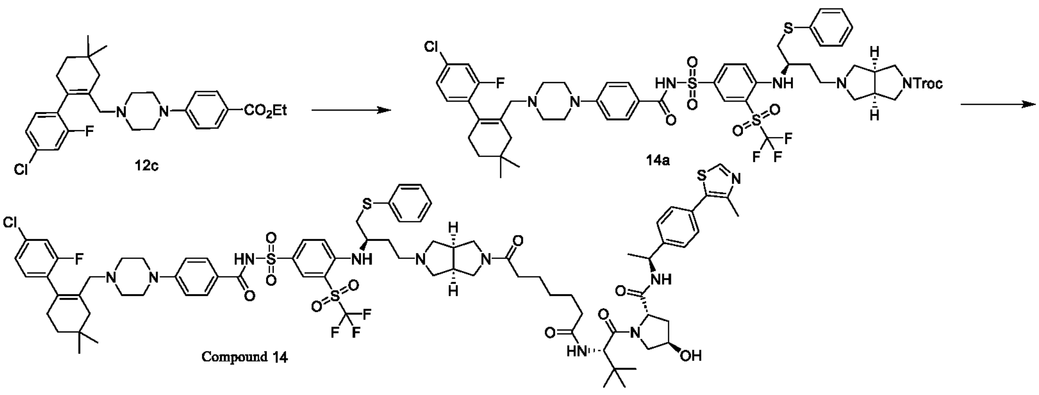

12c

14a

Compound 14

Step 1: Preparation of 14a

12c (1.4 g, 2.89 mmol) was dissolved in 10 mL of methanol. Water (1 mL) and sodium hydroxide (0.35 g, 8.75 mmol) were added and the mixture was reacted at 80°C for 10 h. The reaction solution was cooled to room temperature and concentrated under reduced pressure. The residue was dissolved with 10 mL of water and then washed with 20 mL of methyl tert-butyl ether. The aqueous phase was separated, adjusted to pH 6 with 1 mol/L hydrochloric acid, extracted with ethyl acetate (100 mL × 2), dried over anhydrous sodium sulphate and concentrated under reduced pressure to afford a crude (0.8 g). The above-mentioned crude (240 mg) was dissolved in 15 mL of DCM. 8f (400 mg, 0.53 mmol), DMAP (130 mg, 1.06 mmol) and EDCI (200 mg, 1.04 mmol) were added and the mixture was reacted at room temperature for 16 h. To the reaction system was slowly added 100 mL of water and the mixture was extracted with DCM (100 mL × 2). The organic phase was separated, dried over anhydrous sodium sulphate and concentrated under reduced pressure and the resulting crude was separated and purified by silica gel column chromatography (dichloromethane : methanol (v/v) = 0 : 1-9 : 1) to afford 14a (500 mg, yield: 48%).

LCMS m/z = 597.1 $[M/2 +1]^+$.

Step 2: Preparation of compound 14

14a (500 mg, 0.42 mmol) was dissolved in 40 mL of tetrahydrofuran. Acetic acid (1.2 mL) and zinc powder (1.8 g, 27.75 mmol) were sequentially added and the mixture was reacted at room temperature for 16 h. The reaction system was filtered and to the filtrate were added 10 mL of water and 20 mL of ethyl acetate. The organic phase was separated, dried over anhydrous sodium sulphate and concentrated under reduced pressure. The residue was separated and purified by chromatographic column on silica gel (methanol/dichloromethane/triethylamine (v/v) = 1 : 19 : 0-10 : 89 : 1) to afford a crude (330 mg). The above-mentioned crude (150 mg) was dissolved in 15 mL of DCM. Triethylamine (0.21 mL), intermediate 1 (88 mg, 0.15 mmol) and HATU (86 mg, 0.23 mmol) were sequentially added and the mixture was reacted at room temperature for 2 h. To the reaction system was added 100 mL of water. The mixture was extracted with 50 mL of DCM, dried over anhydrous sodium sulphate and concentrated under reduced pressure. The residue was first separated and purified by silica gel column chromatography (methanol/dichloromethane (v/v) = 0 : 1-1 : 9) and then the resulting crude was passed through Pre-HPLC (instrument and preparative column: using SHIMADZU LC-20AP preparative liquid phase chromatographic instrument, preparative column model: Phenomenex C18). Mobile phase system: acetonitrile/water. Gradient elution method: gradient elution with acetonitrile from 59% to 89% (elution time: 15 min). Lyophilisation was performed to afford compound 14 (35 mg, yield: 12%).

$^1H$ NMR (400 MHz, $CDCl_3$) $\delta$ 8.75 - 8.60 (m, 1H), 8.55 - 8.43 (m, 1H), 7.96 - 7.68 (m, 3H), 7.46 - 7.15 (m, 10H), 7.15 - 6.89 (m, 4H), 6.86 - 6.63 (m, 3H), 6.56 - 6.30 (m, 1H), 5.15 - 4.64 (m, 3H), 4.57 - 4.44 (m, 1H), 4.26 - 3.85 (m, 2H), 3.80 - 3.40 (m, 3H), 3.39 - 2.92 (m, 8H), 2.92 - 1.95 (m, 28H), 1.85 - 1.20 (m, 12H), 1.18 - 0.90 (m, 15H).

LCMS m/z = 793.8 $[M/2+1]^+$.

**Example 15:**

cis-(2S,4R)-1-((S)-2-(5-(5-((R)-3-((4-(N-(4-(4-((4'-chloro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)hexahydropyrrolo[3,4-c]pyrrol-2(1H)-yl)-5-oxopentanamido)-3,3-dimethylbutanoyl)-4-hydroxy-N-((S)-1-(4-(4-methylthiazol-5-yl)phenyl)ethyl)pyrrolidine-2-carboxamide (**compound 15**)

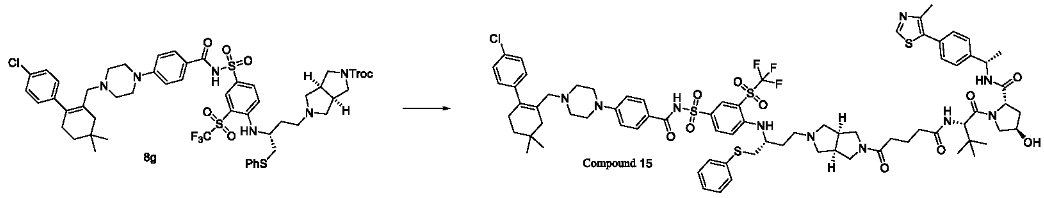

8g (0.44 g, 0.37 mmol) was added to 30 mL of tetrahydrofuran. Zinc powder (1.26 g, 19.27 mmol) and 0.78 mL of acetic acid were sequentially added and the mixture was reacted at room temperature for 5 h. The reaction system was filtered and the filtrate was concentrated under reduced pressure. The residue was then diluted with 100 mL of DCM and then 50 mL of saturated sodium bicarbonate solution was added. The organic phase was separated, dried over anhydrous sodium sulphate and concentrated under reduced pressure to afford a crude (0.11 g). The above-mentioned crude (101 mg) was added to 2 mL of DCM. 5-((S)-1-((2S,4R)-4-hydroxy-2-(((S)-1-(4-(4-methylthiazol-5-yl)phenyl)ethyl)carbamoyl)pyrrolidin-1-yl)-3,3-dimethyl-1-oxobutan-2-yl)amino)-5-oxopentanoic acid (see WO 2020163823 for the synthetic method) (58 mg, 0.104 mmol), triethylamine (0.14 mL, 1.01 mmol) and HATU (57 mg, 0.15 mmol) were sequentially added and the mixture was reacted at room temperature for 2 h. To the reaction system was added 20 mL of water and the mixture was extracted with 20 mL of DCM twice. The organic phase was separated, dried over anhydrous sodium sulphate and concentrated under reduced pressure. Then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water (containing 0.05% $NH_4HCO_3$). Gradient elution method: gradient elution with acetonitrile from 60% to 90% (elution time: 15 min). Lyophilisation was performed to afford compound 15 (35 mg, yield: 7%).

$^1H$ NMR (400 MHz, $CDCl_3$) δ 8.66 (s, 1H), 8.45 (s, 1H), 7.95 - 7.70 (m, 3H), 7.46 - 7.14 (m, 12H), 7.10 - 6.90 (m, 4H), 6.85 - 6.63 (m, 3H), 5.12 - 4.92 (m, 1H), 4.82 - 4.40 (m, 3H), 4.22 - 3.85 (m, 2H), 3.72 - 3.38 (m, 3H), 3.35 - 2.57 (m, 14H), 2.56 - 1.77 (m, 24H), 1.75 - 1.55 (m, 1H), 1.53 - 1.30 (m, 5H), 1.16 - 0.87 (m, 15H).

LCMS m/z = 770.8 $[M/2+1]^+$.

**Example 16:**

cis-(2S,4R)-1-((S)-2-(9-(5-((R)-3-((4-(N-(4-(4-((4'-chloro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)hexahydropyrrolo[3,4-c]pyrrol-2(1H)-yl)-9-oxononanamido)-3,3-dimethylbutanoyl)-4-hydroxy-N-((S)-1-(4-(4-methylthiazol-5 - yl)phenyl)ethyl)pyrrolidine-2-carboxamide (**compound 16**)

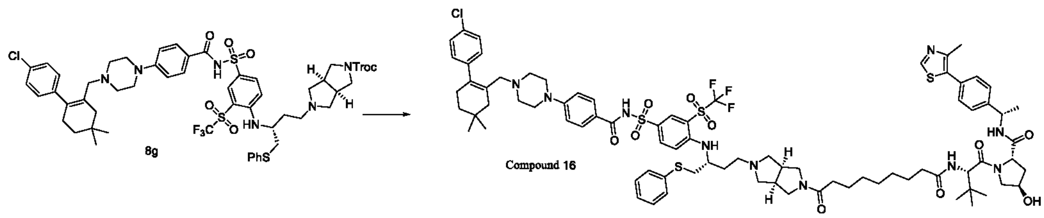

8g (0.44 g, 0.37 mmol) was added to 30 mL of tetrahydrofuran. Zinc powder (1.26 g, 19.27 mmol) and 0.78 mL of acetic acid were sequentially added and the mixture was reacted at room temperature for 5 h. The reaction system was filtered and the filtrate was concentrated under reduced pressure. The residue was then diluted with 100 mL of DCM and then 50 mL of saturated sodium bicarbonate solution was added. The organic phase was separated, dried over anhydrous sodium sulphate and concentrated under reduced pressure to afford a crude (0.11 g). The above-mentioned crude (101 mg) was added to 2 mL of DCM. 9-(((S)-1-((2S,4R)-4-hydroxy-2-(((S)-1-(4-(4-methylthiazol-5-yl)phenyl)ethyl)carbamoyl)pyrrolidin-1-yl)-3,3-dimethyl-1-oxobutan-2-yl)amino)-9-oxononanoic acid (see WO 2020163823 for the synthetic method) (61 mg, 0.099 mmol), triethylamine (0.14 mL, 1.01 mmol) and HATU (57 mg, 0.15 mmol) were sequentially added and the mixture was reacted at room temperature for 2 h. To the reaction system was added 30 mL of water and the mixture was extracted with 20 mL of DCM twice. The organic phase was separated, dried over anhydrous sodium sulphate and concentrated under reduced pressure. Then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water (containing 0.05% $NH_4HCO_3$). Gradient elution method: gradient elution with acetonitrile from 60% to 90% (elution time: 15 min). Lyophilisation was performed to afford compound 16 (85 mg, yield: 16%).

$^1H$ NMR (400 MHz, $CDCl_3$) δ 8.66 (s, 1H), 8.52 - 8.34 (m, 1H), 8.07 - 7.87 (m, 1H), 7.83 - 7.67 (m, 2H), 7.50 - 7.16 (m, 12H), 7.11 - 6.90 (m, 3H), 6.85 - 6.55 (m, 3H), 6.40 - 6.25 (m, 1H), 5.18 - 5.00 (m, 1H), 4.80 - 4.58 (m, 2H), 4.56 - 4.45 (m, 1H), 4.18 - 4.05 (m, 1H), 4.00 - 3.85 (m, 1H), 3.70 - 3.42 (m, 3H), 3.35 - 2.95 (m, 8H), 2.94 - 2.64 (m, 5H), 2.63 - 1.95 (m, 23H), 1.80 - 1.36 (m, 10H), 1.35 - 1.15 (m, 6H), 1.10 - 0.90 (m, 15H).

LCMS m/z = 798.2 $[M/2+1]^+$.

**Example 17:**

cis-(2S,4R)-1-((S)-2-(8-(5-((R)-3-((4-(N-(4-(4-((4'-chloro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)hexahydropyrrolo[3,4-c]pyrrol-2(1H)-yl)-8-oxooctanamido)-3,3-dimethylbutanoyl)-4-hydroxy-N-((S)-1-(4-(4-methylthiazol-5 -yl)phenyl)ethyl)pyrrolidine-2-carboxamide (**compound 17**)

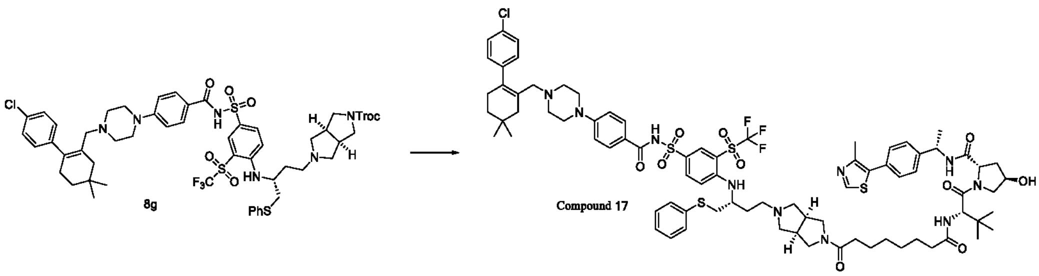

8g (0.30 g, 0.25 mmol) was added to 300 mL of tetrahydrofuran. Zinc powder (1.36 g, 20.8 mmol) and ammonium chloride (0.41 g, 7.66 mmol) were sequentially added and the mixture was reacted at room temperature for 16 h. The reaction system was filtered and to the filtrate were added 10 mL of water and 20 mL of ethyl acetate, followed by extraction. The organic phase was dried over anhydrous sodium sulphate and concentrated under reduced pressure. The resulting crude was separated and purified by chromatographic column on silica gel (dichloromethane : methanol : triethylamine (v/v) = 95 : 5 : 0-89 : 10 : 1) to afford a crude (0.26 g). The above-mentioned crude (120 mg) was added to 15 mL of DCM. Intermediate 3 (75 mg, 0.12 mmol), triethylamine (0.17 mL, 1.2 mmol) and HATU (68 mg, 0.18 mmol) were sequentially added and the mixture was reacted at room temperature for 2 h. To the reaction system was added 100 mL of water. The mixture was extracted with 200 mL of DCM, dried over anhydrous sodium sulphate and concentrated under reduced pressure. The crude was separated and purified by silica gel column chromatography (methanol/dichloromethane (v/v) = 0 : 1-1 : 9) and then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water. Gradient elution method: gradient elution with acetonitrile from 59% to 89% (elution time: 15 min). Lyophilisation was performed to afford compound 17 (90 mg, yield: 49%).

$^1H$ NMR (400 MHz, $CDCl_3$) δ 8.66 (s, 1H), 8.52 - 8.39 (m, 1H), 8.05 - 7.86 (m, 1H), 7.85 - 7.70 (m, 2H), 7.58 - 7.14 (m, 12H), 7.07 - 6.92 (m, 3H), 6.86 - 6.60 (m, 3H), 6.54 - 6.29 (m, 1H), 5.15 - 4.92 (m, 1H), 4.84 - 4.60 (m, 2H), 4.58 - 4.42 (m, 1H), 4.19 - 3.85 (m, 2H), 3.68 - 3.40 (m, 3H), 3.35 - 2.94 (m, 8H), 2.94 - 1.95 (m, 28H), 1.78 - 1.15 (m, 14H), 1.15 - 0.95 (m, 15H).

LCMS m/z = 791.8 $[M/2+1]^+$.

**Example 18:**

cis-(2S,4R)-1-((S)-2-(7-(5-((R)-3-((4-(N-(4-(4-((6-(4-chlorophenyl)spiro[2.5]oct-5-en-5-yl)methyl)piperazin-1-yl)benzoyl)sulphamoyl)-2-((trifluoromethyl)sulphonyl) phenyl)amino)-4-(phenylthio)butyl)hexahydropyrrolo[3,4-c]pyrrol-2(1H)-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 18**)

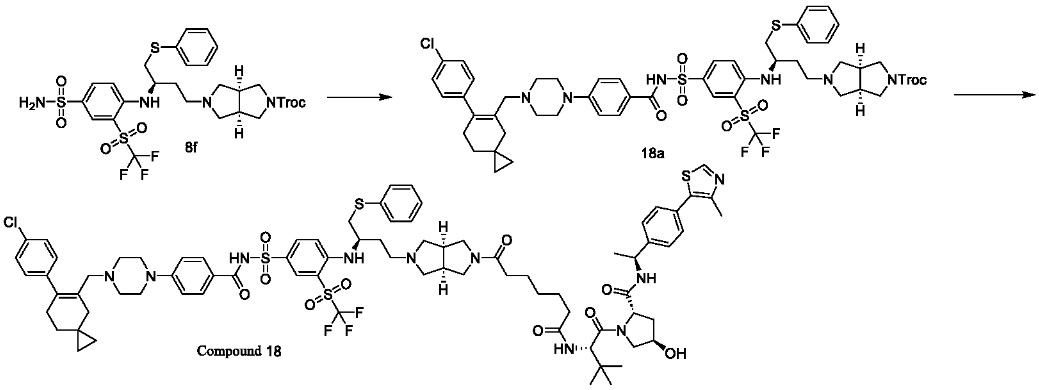


Step 1: Preparation of 18a

11a (170 mg, 0.39 mmol) was dissolved in 15 mL of DCM. 8f (300 mg, 0.40 mmol), DMAP (100 mg, 0.82 mmol) and EDCI (150 mg, 0.78 mmol) were sequentially added and the mixture was reacted at room temperature for 16 h. To the reaction system was slowly added 100 mL of water and the mixture was extracted with 100 mL of DCM. The organic phase was dried over anhydrous sodium sulphate and concentrated under reduced pressure and the resulting crude was separated and purified by silica gel column chromatography (dichloromethane/methanol (v/v) = 0 : 1-9 : 1) to afford 18a (300 mg, yield: 66%).

LCMS m/z = 587.2 $[M/2 +1]^+$.

Step 2: Preparation of compound 18

18a (300 mg, 0.26 mmol) was dissolved in 5 mL of tetrahydrofuran and 15 mL of methanol. Ammonium chloride (0.41 g, 7.66 mmol) and zinc powder (1.36 g, 20.9 mmol) were sequentially added and the mixture was reacted at room temperature for 16 h. The reaction system was filtered and to the filtrate were added 10 mL of water and 20 mL of ethyl acetate, followed by extraction. The organic phase was dried over anhydrous sodium sulphate and concentrated under reduced pressure. The resulting crude was separated and purified by chromatographic column on silica gel (dichloromethane : methanol : triethylamine (v/v) = 95 : 5 : 0-89 : 10 : 1) to afford a crude (220 mg). The above-mentioned crude (120 mg) was dissolved in 15 mL of DCM. Triethylamine (0.17 mL), intermediate 1 (70 mg, 0.12 mmol) and HATU (68 mg, 0.18 mmol) were sequentially added and the mixture was reacted at room temperature for 2 h. To the reaction system was added 100 mL of water. The mixture was extracted with 50 mL of DCM, dried over anhydrous sodium sulphate and concentrated under reduced pressure. The crude was separated and purified by silica gel column chromatography (methanol/dichloromethane (v/v) = 0 : 1-1 : 9) and then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water (containing 0.05% $NH_4HCO_3$). Gradient elution method: gradient elution with acetonitrile from 40% to 70% (elution time: 15 min). Lyophilisation was performed to afford compound 18 (35 mg, yield: 16%).

$^1H$ NMR (400 MHz, $CDCl_3$) δ 8.66 (s, 1H), 8.42 - 8.32 (m, 1H), 8.15 - 7.90 (m, 1H), 7.80 - 7.60 (m, 2H), 7.44 - 6.96 (m, 15H), 6.83 - 6.47 (m, 3H), 6.43 - 6.16 (m, 1H), 5.15 - 5.02 (m, 1H), 4.85 - 3.83 (m, 6H), 3.65 - 2.68 (m, 15H), 2.68 - 1.17 (m, 35H), 1.04 (s, 9H), 0.36 (s, 4H).

LCMS m/z = 783.9 $[M/2+1]^+$.

**Example 19:**

(2S,4R)-1-((S)-2-(7-((1R,4R)-5-((R)-3-((4-(N-(4-(4-((6-(4-chlorophenyl) spiro[2.5]oct-5-en-5-yl)methyl)piperazin-1-yl)benzoyl)sulphamoyl)-2-((trifluoromethyl)sulphonyl)phenyl)amino)-4-(phenylthio)butyl)-2,5-diazabicyclo [2.2.1]heptan-2-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 19)**

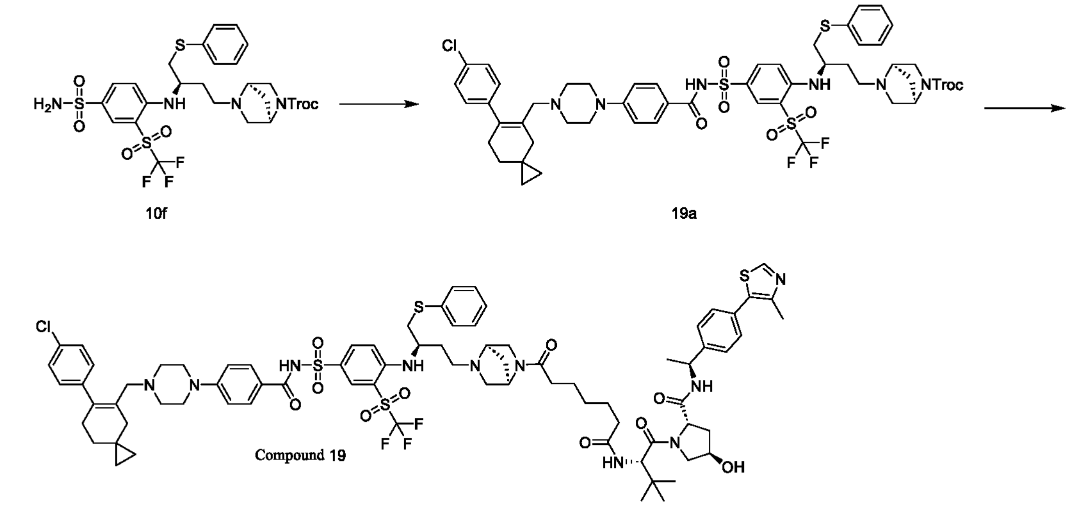

10f

19a

Compound 19

Step 1: Preparation of 19a

11a (150 mg, 0.34 mmol) was dissolved in 10 mL of DCM. 10f (250 mg, 0.34 mmol), DMAP (83 mg, 0.68 mmol) and EDCI (130 mg, 0.68 mmol) were sequentially added and the mixture was reacted at room temperature for 16 h. To the reaction system was slowly added 100 mL of water and the mixture was extracted with 100 mL of dichloromethane. The organic phase was dried over anhydrous sodium sulphate and concentrated under reduced pressure and the resulting crude was separated and purified by silica gel column chromatography (dichloromethane/methanol (v/v) = 0 : 1-9 : 1) to afford 19a (220 mg, yield: 56%).

LCMS m/z = 580.0 $[M/2+1]^+$.

Step 2: Preparation of compound 19

19a (220 mg, 0.19 mmol) was dissolved in 3 mL of tetrahydrofuran and 12 mL of methanol. Ammonium chloride (0.32 g, 5.98 mmol) and zinc powder (1.05 g, 16 mmol) were sequentially added and the mixture was reacted at room temperature for 16 h. The reaction system was filtered and to the filtrate were added 10 mL of water and 20 mL of ethyl acetate for extraction, followed by liquid separation. The organic phase was dried over anhydrous sodium sulphate and concentrated under reduced pressure and the resulting crude was separated and purified by chromatographic column on silica gel (dichloromethane : methanol : triethylamine (v/v/v) = 95 : 5 : 0-89 : 10 : 1) to afford a crude (0.16 g). The above-mentioned crude (120 mg) was dissolved in 15 mL of DCM. Triethylamine (0.17 mL), intermediate 1 (70 mg, 0.12 mmol) and HATU (68 mg, 0.18 mmol) were sequentially added and the mixture was reacted at room temperature for 2 h. To the reaction system was added 100 mL of water. The mixture was extracted with 50 mL of dichloromethane, dried over anhydrous sodium sulphate and concentrated under reduced pressure. The crude was separated and purified by silica gel column chromatography (methanol/dichloromethane (v/v) = 0 : 1-1 : 9) and then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water (containing 0.05% $NH_4HCO_3$). Gradient elution method: gradient elution with acetonitrile from 50% to 80% (elution time: 15 min). Lyophilisation was performed to afford compound 19 (40 mg, yield: 18%).

$^1H$ NMR (400 MHz, $CDCl_3$) δ 8.75 - 8.61 (m, 1H), 8.54 - 8.44 (m, 1H), 7.97 - 7.70 (m, 3H), 7.48 - 6.87 (m, 15H), 6.87 - 6.64 (m, 3H), 6.55 - 6.32 (m, 1H), 5.17 - 4.89 (m, 1H), 4.87 - 4.62 (m, 2H), 4.59 - 4.45 (m, 1H), 4.25 - 3.80 (m, 3H), 3.79 - 2.58 (m, 18H), 2.58 - 1.95 (m, 21H), 1.63 - 1.20 (m, 9H), 1.04 (s, 9H), 0.37 (s, 4H).

LCMS m/z = 776.5 [M/2+1]$^+$.

**Example 20:**

(2S,4R)-1-((S)-2-(7-((1S,4S)-5-((R)-3-((4-(N-(4-(4-((4'-chloro-2'-fluoro-4,4-dimethyl-3,4,5,6-tetrahydro-[1,1'-biphenyl]-2-yl)methyl)piperazin-1-yl)benzoyl) sulphamoyl)-2-((trifluoromethyl)sulphonyl)phenyl)amino)-4-(phenylthio)butyl)-2,5-diazabicyclo[2.2.1]heptan-2-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide (**compound 20**)

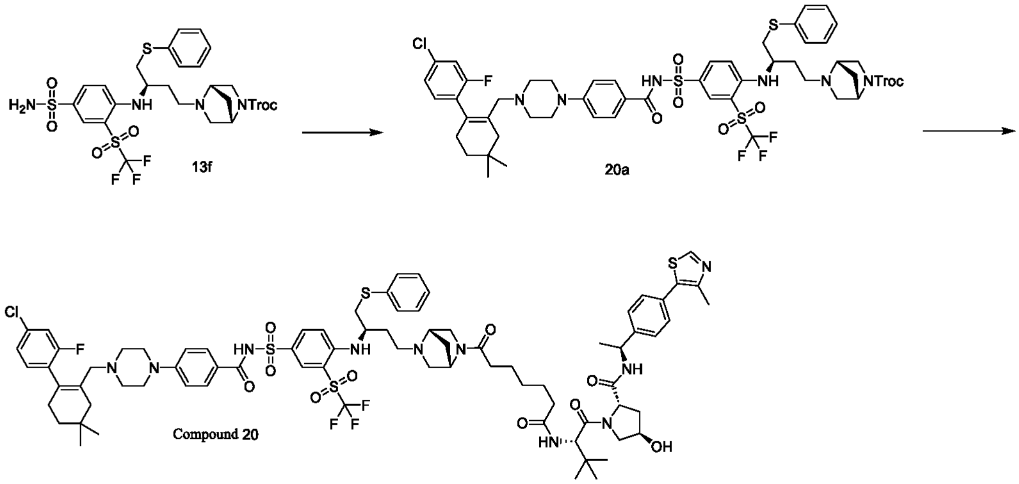

13f

20a

Compound 20

Step 1: Preparation of 20a

12c (1.4 g, 2.89 mmol) was dissolved in 10 mL of methanol. Water (1 mL) and sodium hydroxide (0.35 g, 8.75 mmol) were added and the mixture was reacted at 80°C for 10 h. The reaction solution was cooled to room temperature and concentrated under reduced pressure. The residue was dissolved with 10 mL of water and then washed with 20 mL of methyl tert-butyl ether. The aqueous phase was separated, adjusted to pH 6 with 1 mol/L hydrochloric acid, extracted with ethyl acetate (100 mL × 2), dried over anhydrous sodium sulphate and concentrated under reduced pressure to afford a crude (0.8 g). The above-mentioned crude (250 mg) was dissolved in 10 mL of DCM. 13f (410 mg, 0.55 mmol), DMAP (130 mg, 1.07 mmol) and EDCI (210 mg, 1.10 mmol) were added and the mixture was reacted at room temperature for 16 h. To the reaction system was slowly added 100 mL of water and the mixture was extracted with 50 mL of DCM. The organic phase was dried over anhydrous sodium sulphate and concentrated under reduced pressure and the resulting crude was separated and purified by silica gel column chromatography (dichloromethane/methanol (v/v) = 1 : 0-9 : 1) to afford 20a (310 mg, yield: 48%).

LCMS m/z = 590.2 [M/2+1]$^+$.

Step 2: Preparation of compound 20

20a (300 mg, 0.25 mmol) was dissolved in 3 mL of tetrahydrofuran and 12 mL of methanol. Ammonium chloride (0.4 g, 7.5 mmol) and zinc powder (1.31 g, 20.15 mmol) were added and the mixture was reacted at room temperature for 16 h. The reaction system was filtered and to the filtrate were added 10 mL of water and 20 mL of ethyl acetate, followed by extraction. The organic phase was dried over anhydrous sodium sulphate and concentrated under reduced pressure. The resulting crude was separated and purified by chromatographic column on silica gel (dichloromethane : methanol : triethylamine (v/v/v) = 95 : 5 : 0-89 : 10 : 1) to afford a crude (0.22 g). The above-mentioned crude (27 mg) was dissolved in 5 mL of DCM. Triethylamine (0.037 mL), intermediate 1 (16 mg, 0.027 mmol) and HATU (15 mg, 0.04 mmol) were sequentially added and the mixture was reacted at room temperature for 2 h. To the reaction system was added 100 mL of water. The mixture was extracted with 20 mL of DCM, dried over anhydrous sodium sulphate and concentrated under reduced pressure. The crude was separated and purified by silica gel column chromatography (methanol/dichloromethane (v/v) = 0 : 1-1 : 9) and then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide,

and filtered with a 0.45 μm filter membrane, to prepare into a sample liquid. Mobile phase system: acetonitrile/water (containing 0.05% $NH_4HCO_3$). Gradient elution method: gradient elution with acetonitrile from 40% to 70% (elution time: 15 min). Lyophilisation was performed to afford compound 20 (18 mg, yield: 37%).

$^1H$ NMR (400 MHz, $CDCl_3$) δ 8.72 - 8.60 (m, 1H), 8.50 - 8.34 (m, 1H), 8.08 - 7.90 (m, 1H), 7.80 - 7.64 (m, 2H), 7.48 - 6.88 (m, 14H), 6.85 - 6.55 (m, 3H), 6.49 - 6.25 (m, 1H), 5.15 - 4.90 (m, 1H), 4.85 - 3.85 (m, 6H), 3.68 - 2.90 (m, 10H), 2.86 - 1.94 (m, 22H), 1.90 - 1.18 (m, 16H), 1.12 - 0.88 (m, 15H).

LCMS m/z = 524.8 $[M/3+1]^+$.

**Example 21:**

cis-(2S,4R)-1-((S)-2-(7-(5-((R)-3-((4-(N-(4-(4-((6-(4-chloro-2-fluorophenyl)spiro[2.5]oct-5-en-5-yl)methyl)piperazin-1-yl)benzoyl)sulphamoyl)-2-((trifluoromethyl)sulphonyl)phenyl)amino)-4-(phenylthio)butyl)hexahydropyrrolo[3,4-c]pyrrol-2(1H)-yl)-7-oxoheptanamido)-3,3-dimethylbutanoyl)-4-hydroxy-N-((S)-1-(4-(4-methylthiazol-5 - yl)phenyl)ethyl)pyrrolidine-2-carboxamide (**compound 21**)

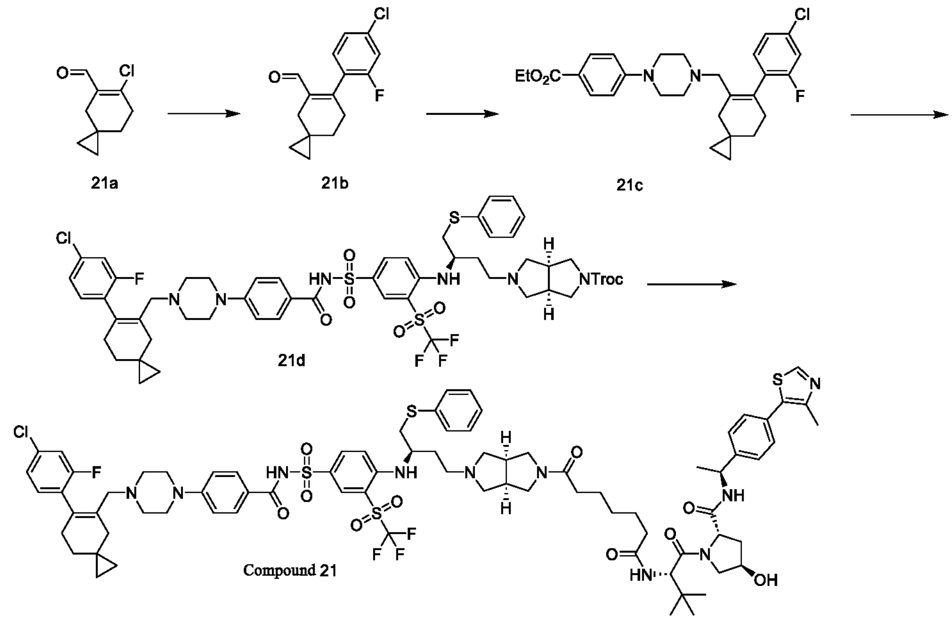

21a
21b
21c
21d
Compound 21

Step 1: Preparation of 21b

21a (see WO 2013185202 for the synthetic method) (1.00 g, 5.86 mmol) and (4-chloro-2-fluorophenyl)boronic acid (1.02 g, 5.85 mmol) were dissolved in 30 mL of dioxane and 3 mL of water. After nitrogen replacement was carried out three times, potassium acetate (1.73 g, 17.63 mmol) and $Pd(dppf)Cl_2$ (0.13 g, 0.18 mmol) were added under nitrogen protection and the mixture was reacted at 90°C for 7 h. The reaction solution was cooled to room temperature and to the reaction solution was slowly added 40 mL of water. The mixture was extracted with 40 mL of ethyl acetate, dried over anhydrous sodium sulphate and concentrated under reduced pressure and the resulting crude was separated and purified by silica gel column chromatography (ethyl acetate/petroleum ether (v/v) = 0 : 1-1 : 10) to afford 21b (0.78 g, yield: 50%).

Step 2: Preparation of 21c

21b (780 mg, 2.95 mmol) was dissolved in 35 mL of tetrahydrofuran. Ethyl 4-(piperazin-1-yl)benzoate (1.11 g, 4.74 mmol), 2 mL of acetic acid and sodium triacetoxyborohydride (2.01 g, 9.48 mmol) were sequentially added and the mixture was reacted at room temperature for 16 h. To the reaction solution was slowly added 50 mL of saturated sodium bicarbonate solution. The resulting mixture was extracted with 60 mL of ethyl acetate twice and the organic phase was washed with 50 mL of saturated sodium chloride, dried over anhydrous sodium sulphate and concentrated under reduced pressure. The resulting crude was separated and purified by silica gel column chromatography (ethyl acetate/petroleum ether (v/v) = 0 : 1-1 : 1) to afford 21c (1.27 g, yield: 89%).

LCMS m/z = 483.3 $[M+1]^+$.

Step 3: Preparation of 21d

Sodium hydroxide (0.26 g, 6.5 mmol) was dissolved in 5 mL of methanol and 3 mL of water. A solution of 21c (1.27 g, 2.63 mmol) in 10 mL of tetrahydrofuran was added and the resulting mixture was reacted at 80°C for 6 h. The reaction solution was cooled to room temperature and concentrated under reduced pressure. The residue was adjusted to pH 5 with 1 mol/L hydrochloric acid and extracted with ethyl acetate (15 mL × 3). The organic phase was washed with 20 mL of saturated sodium chloride solution, dried over anhydrous sodium sulphate and concentrated under reduced pressure to afford a crude (0.96 g). The above-mentioned crude (170 mg) was dissolved in 15 mL of DCM. 8f (300 mg, 0.40 mmol), DMAP (100 mg, 0.82 mmol) and EDCI (150 mg, 0.78 mmol) were added and the mixture was reacted at room temperature for 16 h. To the reaction system was slowly added 100 mL of water and the mixture was extracted with 100 mL of DCM. The organic phase was dried over anhydrous sodium sulphate and concentrated under reduced pressure and the resulting crude was separated and purified by silica gel column chromatography (dichloromethane/methanol (v/v) = 0 : 1-9 : 1) to afford 21d (350 mg, yield: 73%).
LCMS m/z = 596.1 $[M/2+1]^+$.

Step 4: Preparation of compound 21

21d (350 mg, 0.294 mmol) was dissolved in 5 mL of tetrahydrofuran and 15 mL of methanol. Ammonium chloride (0.48 g, 8.97 mmol) and zinc powder (1.57 g, 24.15 mmol) were added and the mixture was reacted at room temperature for 16 h. The reaction system was filtered and to the filtrate were added 10 mL of water and 20 mL of ethyl acetate, followed by extraction. The organic phase was separated, dried over anhydrous sodium sulphate and concentrated under reduced pressure. The residue was separated and purified by chromatographic column on silica gel (methanol/dichloromethane/triethylamine (v/v/v) = 1 : 19 : 0-10 : 89 : 1) to afford a crude (230 mg). The above-mentioned crude (100 mg) was dissolved in 15 mL of DCM. Triethylamine (0.14 mL), intermediate 1 (60 mg, 0.10 mmol) and HATU (57 mg, 0.15 mmol) were sequentially added and the mixture was reacted at room temperature for 2 h. To the reaction system was added 100 mL of water. The mixture was extracted with 50 mL of DCM, dried over anhydrous sodium sulphate and concentrated under reduced pressure. The residue was first separated and purified by silica gel column chromatography (methanol/dichloromethane (v/v) = 0 : 1-1 : 9) and subsequently, the resulting crude was passed through Pre-HPLC (instrument and preparative column: using SHIMADZU LC-20AP preparative liquid phase chromatographic instrument, preparative column model: Phenomenex C18). Mobile phase system: acetonitrile/water (containing 0.05% $NH_4HCO_3$). Gradient elution method: gradient elution with acetonitrile from 40% to 70% (elution time: 15 min). Lyophilisation was performed to afford compound 21 (35 mg, yield: 17%).
$^1$H NMR (400 MHz, $CDCl_3$) δ 8.73 - 8.60 (m, 1H), 8.55 - 8.45 (m, 1H), 7.95 - 7.70 (m, 3H), 7.44 - 7.14 (m, 10H), 7.13 - 6.90 (m, 4H), 6.86 - 6.62 (m, 3H), 6.52 - 6.32 (m, 1H), 5.15 - 4.88 (m, 1H), 4.87 - 4.63 (m, 2H), 4.57 - 4.43 (m, 1H), 4.27 - 3.82 (m, 2H), 3.80 - 3.36 (m, 3H), 3.35 - 2.93 (m, 8H), 2.91 - 1.95 (m, 25H), 1.68 - 1.39 (m, 11H), 1.38 - 1.20 (m, 4H), 1.15 - 0.95 (m, 9H), 0.37 (s, 4H).
LCMS m/z = 528.9 $[M/3+1]^+$.

**Example 22:**

cis-(2S,4R)-1-((S)-2-(8-(5-((R)-3-((4-(N-(4-(4-((6-(4-chlorophenyl)spiro[2.5]oct-5-en-5-yl)methyl)piperazin-1-yl)benzoyl)sulphamoyl)-2-((trifluoromethyl)sulphonyl) phenyl)amino)-4-(phenylthio)butyl)hexahydropyrrolo[3,4-c]pyrrol-2(1H)-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide (**compound 22**)

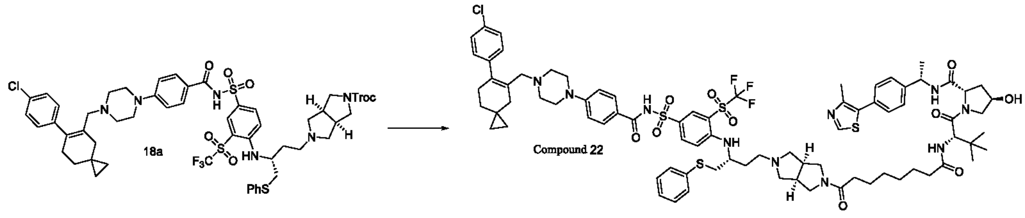


18a (300 mg, 0.26 mmol) was dissolved in 5 mL of tetrahydrofuran and 15 mL of methanol. Ammonium chloride

(0.41 g, 7.66 mmol) and zinc powder (1.36 g, 20.9 mmol) were sequentially added and the mixture was reacted at room temperature for 16 h. The reaction system was filtered and to the filtrate were added 10 mL of water and 20 mL of ethyl acetate, followed by extraction. The organic phase was dried over anhydrous sodium sulphate and concentrated under reduced pressure. The resulting crude was separated and purified by chromatographic column on silica gel (dichloromethane : methanol : triethylamine (v/v/v) = 95 : 5 : 0-89 : 10 : 1) to afford a crude (0.22 g). The above-mentioned crude (120 mg) was added to 15 mL of DCM. Intermediate 3 (70 mg, 0.11 mmol), triethylamine (0.17 mL, 1.2 mmol) and HATU (68 mg, 0.18 mmol) were sequentially added and the mixture was reacted at room temperature for 2 h. To the reaction system was added 100 mL of water. The mixture was extracted with 200 mL of DCM, dried over anhydrous sodium sulphate and concentrated under reduced pressure. The crude was separated and purified by silica gel column chromatography (methanol/dichloromethane (v/v) = 0 : 1-1 : 9) and then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water (containing 10 mmol/L $NH_4HCO_3$). Gradient elution method: gradient elution with acetonitrile from 35% to 65% (elution time: 15 min). Lyophilisation was performed to afford compound 22 (70 mg, yield: 31%).

$^1H$ NMR (400 MHz, $CDCl_3$) δ 8.67 (s, 1H), 8.54 - 8.38 (m, 1H), 8.04 - 7.70 (m, 3H), 7.57 - 7.14 (m, 12H), 7.10 - 6.92 (m, 3H), 6.90 - 6.59 (m, 3H), 6.52 - 6.18 (m, 1H), 5.16 - 4.92 (m, 1H), 4.85 - 4.62 (m, 2H), 4.59 - 4.42 (m, 1H), 4.20 - 3.82 (m, 2H), 3.68 - 3.40 (m, 3H), 3.38 - 2.93 (m, 8H), 2.93 - 1.95 (m, 26H), 1.80 - 1.42 (m, 11H), 1.41 - 1.16 (m, 5H), 1.12 - 0.94 (m, 9H), 0.36 (s, 4H).

LCMS m/z = 790.5 $[M/2+1]^+$.

**Example 23:**

cis-(2S,4R)-1-((S)-2-(8-(5-((R)-3-((4-(N-(4-(4-((6-(4-chloro-2-fluorophenyl)spiro[2.5]oct-5-en-5-yl)methyl)piperazin-1-yl)benzoyl)sulphamoyl)-2-((trifluoromethyl)sulphonyl)phenyl)amino)-4-(phenylthio)butyl)hexahydropyrrolo[3,4-c]pyrrol-2(1H)-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide (**compound 23**)

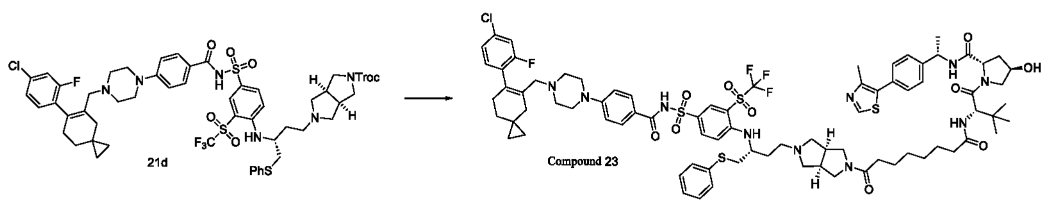

21d (350 mg, 0.294 mmol) was dissolved in 5 mL of tetrahydrofuran and 15 mL of methanol. Ammonium chloride (0.48 g, 8.97 mmol) and zinc powder (1.57 g, 24.15 mmol) were added and the mixture was reacted at room temperature for 16 h. The reaction system was filtered and to the filtrate were added 10 mL of water and 20 mL of ethyl acetate, followed by extraction. The organic phase was dried over anhydrous sodium sulphate and concentrated under reduced pressure. The residue was separated and purified by chromatographic column on silica gel (methanol/dichloromethane/triethylamine (v/v) = 1 : 19 : 0-10 : 89 : 1) to afford a crude (230 mg). The above-mentioned crude (120 mg) was added to 15 mL of DCM. Intermediate 3 (70 mg, 0.11 mmol), triethylamine (0.17 mL, 1.2 mmol) and HATU (68 mg, 0.18 mmol) were sequentially added and the mixture was reacted at room temperature for 2 h. To the reaction system was added 100 mL of water. The mixture was extracted with 200 mL of DCM, dried over anhydrous sodium sulphate and concentrated under reduced pressure. The crude was separated and purified by silica gel column chromatography (methanol/dichloromethane (v/v) = 0 : 1-1 : 9) and then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water (containing 10 mmol/L $NH_4HCO_3$). Gradient elution method: gradient elution with acetonitrile from 35% to 65% (elution time: 15 min). Lyophilisation was performed to afford compound 23 (65 mg, yield: 27%).

$^1H$ NMR (400 MHz, $CDCl_3$) δ 8.67 (s, 1H), 8.52 - 8.41 (m, 1H), 8.04 - 7.86 (m, 1H), 7.84 - 7.71 (m, 2H), 7.56 - 7.15 (m, 10H), 7.13 - 6.94 (m, 4H), 6.87 - 6.60 (m, 3H), 6.50 - 6.23 (m, 1H), 5.15 - 4.92 (m, 1H), 4.85 - 4.62 (m, 2H), 4.59 - 4.44 (m, 1H), 4.20 - 3.82 (m, 2H), 3.70 - 3.40 (m, 3H), 3.35 - 2.93 (m, 8H), 2.93 - 1.96 (m, 28H), 1.75 - 1.42 (m, 9H), 1.40 - 1.16 (m, 5H), 1.13 - 0.92 (m, 9H), 0.37 (s, 4H).

LCMS m/z = 799.5 $[M/2+1]^+$.

**Example 24:**

(2S,4R)-1-((S)-2-(8-(4-((R)-3-((4-(N-(4-(4-((4'-chloro-2'-fluoro-4,4-dimethyl-3,4,5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)piperazin-1-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide (**compound 24**)

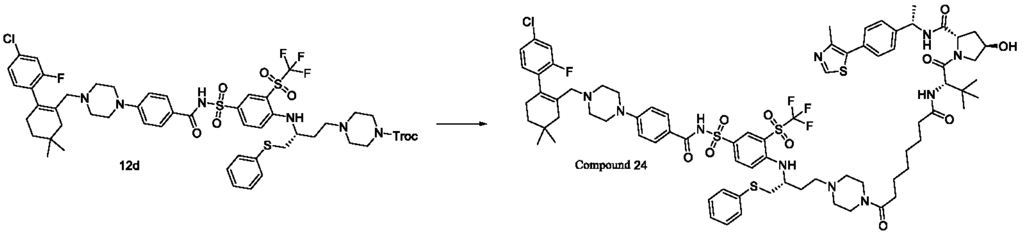


12d (5 g, 4.3 mmol) was dissolved in 200 mL of tetrahydrofuran. Acetic acid (16 g, 266.4 mmol) and zinc powder (16 g, 246.7 mmol) were sequentially added and the mixture was reacted at room temperature for 16 h. The reaction system was filtered and to the filtrate was added water (100 mL). The mixture was then extracted with 200 mL of ethyl acetate. The organic phase was separated, dried over anhydrous sodium sulphate and concentrated under reduced pressure to afford a crude (2 g). The above-mentioned crude (370 mg) was added to 8 mL of DCM. Intermediate 3 (220 mg, 0.35 mmol), triethylamine (0.51 mL, 3.6 mmol) and HATU (209 mg, 0.55 mmol) were sequentially added and the mixture was reacted at room temperature for 2 h. To the reaction system was added 20 mL of water and the mixture was extracted with 20 mL of DCM twice. The organic phase was dried over anhydrous sodium sulphate and concentrated under reduced pressure. Then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water (containing 0.05% $NH_4$ $HCO_3$). Gradient elution method: gradient elution with acetonitrile from 20% to 50% (elution time: 15 min). Lyophilisation was performed to afford compound 24 (221 mg, yield: 18%).

$^1H$ NMR (400 MHz, $CDCl_3$) δ 8.67 (s, 1H), 8.38 - 8.32 (m, 1H), 8.09 (dd, 1H), 7.68 (d, 2H), 7.48 - 7.22 (m, 10H), 7.16 - 6.90 (m, 4H), 6.82 - 6.71 (m, 2H), 6.62 (d, 1H), 6.26 (d, 1H), 5.15 - 5.00 (m, 1H), 4.80 - 4.67 (m, 1H), 4.60 (d, 1H), 4.55 - 4.45 (m, 1H), 4.15 - 4.07 (m, 1H), 3.97 - 3.82 (m, 1H), 3.73 - 3.54 (m, 2H), 3.48 - 3.20 (m, 7H), 3.18 - 2.92 (m, 2H), 2.75 (s, 2H), 2.57 - 1.90 (m, 24H), 1.76 - 1.40 (m, 10H), 1.36 - 1.23 (m, 4H), 1.09 - 0.93 (m, 15H).

LCMS m/z = 788.0 $[M/2+1]^+$.

**Example 25:**

(2S,4R)-1-((S)-2-(8-((1S,4S)-5-((R)-3-((4-(N-(4-(4-((4'-chloro-4,4-dimethyl-3,4, 5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)-2,5-diazabicyclo[2.2.1]heptan-2-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide (**compound 25**)

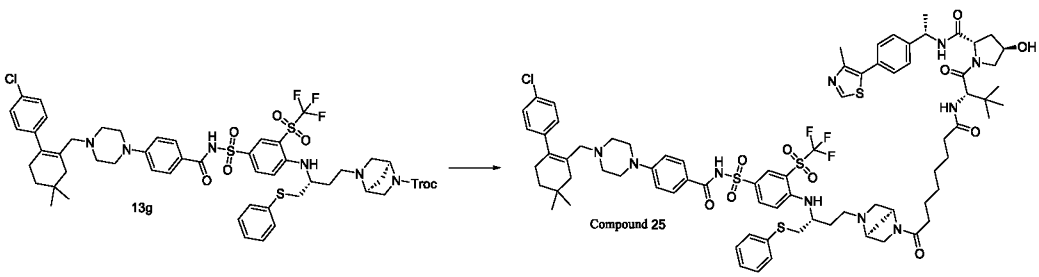

13g (0.5 g, 0.43 mmol) was added to 60 mL of tetrahydrofuran. Zinc powder (1.6 g, 24.46 mmol) and 1.6 mL of acetic acid were sequentially added and the mixture was reacted at room temperature for 4 h. To the reaction solution was added 25 mL of water to quench the reaction. The mixture was filtered over diatomaceous earth and the filtrate was adjusted to pH 8 with saturated aqueous sodium bicarbonate solution and extracted with 6 mL of dichloromethane twice. The organic phases were combined, dried over anhydrous sodium sulphate and concentrated under reduced pressure and the resulting crude was separated and purified by chromatographic column on silica gel (dichloromethane : methanol : triethylamine (v/v) = 100 : 10 : 1) to afford a crude (0.2 g). The above-mentioned crude (0.20 g) was added to 5 mL of DCM. Intermediate 3 (0.12 g, 0.2 mmol), triethylamine (0.28 mL, 2.0 mmol) and HATU (0.11 g, 0.29 mmol) were sequentially added and the mixture was reacted at room temperature for 3 h. To the reaction system was added 15 mL of water and the mixture was extracted with 20 mL of DCM, dried over anhydrous sodium sulphate and concentrated under reduced pressure. Then the resulting crude was passed through Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water (containing 0.05% $NH_4HCO_3$). Gradient elution method: gradient elution with acetonitrile from 50% to 80% (elution time: 15 min). Lyophilisation was performed to afford compound 25 (97 mg, yield: 14%).

$^1H$ NMR (400 MHz, $CDCl_3$) δ 8.66 (s, 1H), 8.44 - 8.32 (m, 1H), 8.07 - 7.95 (m, 1H), 7.80 - 7.67 (m, 2H), 7.54 - 7.16 (m, 13H), 7.03 - 6.94 (m, 2H), 6.83 - 6.70 (m, 2H), 6.69 - 6.55 (m, 1H), 6.39 - 6.25 (m, 1H), 5.15 - 4.96 (m, 1H), 4.80 - 4.18 (m, 4H), 4.16 - 4.05 (m, 1H), 4.03 - 3.87 (m, 1H), 3.63 - 3.53 (m, 1H), 3.53 - 3.35 (m, 2H), 3.33 - 2.72 (m, 10H), 2.70 - 1.95 (m, 21H), 1.95 - 1.20 (m, 16H), 1.15 - 0.86 (m, 15H).

LCMS m/z = 785.0 $[M/2+1]^+$.

**Example 26:**

cis-(2S,4R)-1-((S)-2-(7-(5-((R)-3-((4-(N-(4-(4-((4'-chloro-4,4-dimethyl-3,4, 5,6-tetrahydro-[1,1'-biphenyl]-2-yl)methyl)piperazin-1-yl)benzoyl)sulphamoyl)-2-((trifluoromethyl)sulphonyl)phenyl)amino)-4-(phenylthio)butyl)-3a,6a-dimethylhexahydropyrrolo[3,4-c]pyrrol-2(1H)-yl)-7-oxoheptanamido)-3,3-dimethylbutanoyl)-4-hydroxy-N-((S)-1-(4-(4-methylthiazol-5-yl)phenyl)ethyl) pyrrolidine-2-carboxamide **(compound 26)**

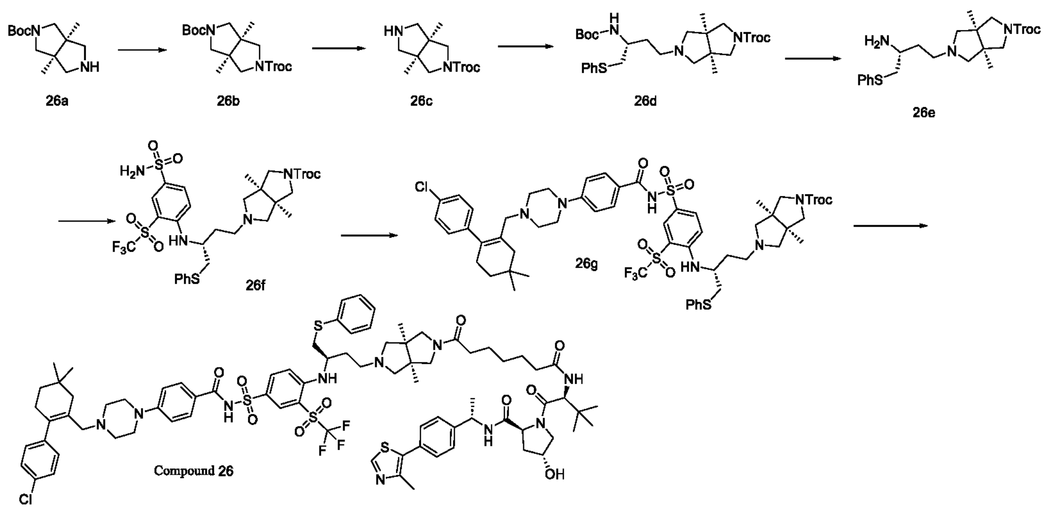

Step 1: Preparation of 26b

26a (1.0 g, 4.16 mmol) was dissolved in 10 mL of DCM and triethylamine (0.88 g, 8.70 mmol) was added. The mixture was cooled to 0°C and 2,2,2-trichloroethyl chloroformate (0.97 g, 4.58 mmol) was slowly added dropwise. Then the mixture was slowly warmed to room temperature and reacted for 2 h. After the reaction was completed, the reaction solution was diluted with 40 mL of DCM and then 50 mL of water was added. The organic phase was separated, dried over anhydrous sodium sulphate and concentrated under reduced pressure and the resulting crude was separated and purified by silica gel column chromatography (petroleum ether/ethyl acetate (v/v) =10 : 1-2 : 1) to afford 26b (1.7 g, yield: 98%).

Step 2: Preparation of 26c

26b (1.65 g, 3.97 mmol) was dissolved in 10 mL of DCM. Trifluoroacetic acid (2.9 mL) was added and the mixture was reacted at room temperature for 3 h. The reaction solution was concentrated under reduced pressure. To the residue were added 20 mL of DCM and 20 mL of water. The mixture was adjusted to pH 9 with saturated sodium bicarbonate solution. The organic phase was separated, dried over anhydrous sodium sulphate and concentrated under reduced pressure to afford crude 26c (1.5 g).

Step 3: Preparation of 26d

The above-mentioned crude 26c (1.5 g) was added to 10 mL of 1,2-dichloroethane. Tert-butyl (R)-(4-oxo-1-(phenylthio)butan-2-yl)carbamate (0.94 g, 3.18 mmol) and glacial acetic acid (0.18 mL) were added and the mixture was reacted at room temperature for 0.5 h. Sodium triacetoxyborohydride (2.2 g, 10.38 mmol) was added and the resulting mixture was reacted at room temperature for 19 h. DCM (50 mL) was added to dilute the reaction solution. Saturated sodium bicarbonate solution was used to adjust the reaction mixture to pH 9. The organic phase was separated, dried over anhydrous sodium sulphate and concentrated under reduced pressure. The resulting crude was separated and purified by chromatographic column on silica gel (dichloromethane : methanol (v/v) = 10 : 1) to afford 26d (0.68 g, yield over two steps from compound 26b: 29%).

Step 4: Preparation of trifluoroacetate of 26e

26d (0.68 g, 1.14 mmol) was dissolved in 4 mL of DCM. Trifluoroacetic acid (2 mL) was added and the mixture was reacted at room temperature for 3 h. The reaction solution was concentrated under reduced pressure to afford crude trifluoroacetate of 26e (0.6 g).

Step 5: Preparation of 26f

The above-mentioned crude trifluoroacetate of 26e (0.6 g) was added to 4 mL of acetonitrile. Triethylamine (0.79 mL, 5.67 mmol) and 4-fluoro-3-((trifluoromethyl)sulphonyl)benzenesulphonamide (0.35 g, 1.14 mmol) were added and the mixture was refluxed and reacted at 80°C for 3 h. The reaction mixture was cooled to room temperature. The reaction solution was concentrated under reduced pressure and the resulting crude was separated and purified by chromatographic column on silica gel (dichloromethane : methanol (v/v) = 10 : 1) to afford 26f (0.87 g, yield over two steps from compound 26d: 98%).

Step 6: 26g

26f (0.87 g, 1.11 mmol) was added to 10 mL of DCM. 4-(4-((4'-chloro-4,4-dimethyl-3,4,5,6-tetrahydro-[1,1'-biphenyl]-2-yl)methyl)piperazin-1-yl)benzoic acid (0.49 g, 1.11 mmol), DMAP (0.27 g, 2.21 mmol) and EDCI (0.43 g, 2.25 mmol) were sequentially added and the mixture was reacted at room temperature for 12 h. To the reaction system was added 20 mL of water. The organic phase was separated, dried over anhydrous sodium sulphate and concentrated under reduced pressure and the resulting crude was separated and purified by chromatographic column on silica gel (dichloromethane : methanol (v/v) = 10 : 1) to afford 26g (0.80 g, yield: 60%).

Step 7: Preparation of compound 26

26g (0.25 g, 0.208 mmol) was added to 5 mL of mixed solvents of methanol/tetrahydrofuran ((v/v) = 10 : 1). Zinc powder (1.1 g, 16.9 mmol) and ammonium chloride (0.34 g, 6.36 mmol) were sequentially added and the mixture was stirred at 30°C for 19 h. The reaction system was filtered and the filtrate was concentrated under reduced pressure. The crude was separated and purified by chromatographic column on silica gel (dichloromethane : methanol (v/v) = 10 : 1) to afford a crude (0.08 g). The above-mentioned crude (0.08 g) was added to 8 mL of DCM. Intermediate 1 (50 mg, 0.085 mmol), triethylamine (0.12 mL, 0.86 mmol) and HATU (48 mg, 0.126 mmol) were sequentially added and the mixture was reacted at room temperature for 2 h. To the reaction system was added 20 mL of water and the mixture was extracted with 30 mL of DCM twice. The organic phase was separated, dried over anhydrous sodium sulphate and concentrated under reduced pressure. Then the resulting crude was passed through Pre-HPLC (instrument and preparative column: using SHIMADZU LC-20AP preparative liquid phase chromatographic instrument, preparative column model: Phenomenex). Preparation method: the crude was dissolved with methanol and dimethyl sulphoxide, and filtered with a 0.45 μm filter membrane, to prepare into a sample liquid. Mobile phase system: acetonitrile/water. Gradient elution method: gradient elution with acetonitrile from 59% to 89% (elution time: 15 min). Lyophilisation was performed to afford

compound 26 (80 mg, yield: 24%).

$^1H$ NMR (400 MHz, $CDCl_3$) δ 8.73 - 8.62 (m, 1H), 8.55 - 8.45 (m, 1H), 8.00 - 7.70 (m, 3H), 7.44 - 7.15 (m, 12H), 7.05 - 6.70 (m, 6H), 6.63 - 6.34 (m, 1H), 5.17 - 4.60 (m, 3H), 4.58 - 4.45 (m, 1H), 4.25 - 3.90 (m, 2H), 3.75 - 2.62 (m, 15H), 2.60 - 1.91 (m, 20H), 1.65 - 0.75 (m, 35H).

LCMS m/z = 799.0 $[M/2+1]^+$.

**Example 27:**

(2S,4R)-1-((S)-2-(7-((1S,4S)-5-((R)-3-((4-(N-(4-(4-((6-(4-chlorophenyl) spiro[2.5]oct-5-en-5-yl)methyl)piperazin-1-yl)benzoyl)sulphamoyl)-2-((trifluoromethyl)sulphonyl)phenyl)amino)-4-(phenylthio)butyl)-2,5-diazabicyclo [2.2.1]heptan-2-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 27)**

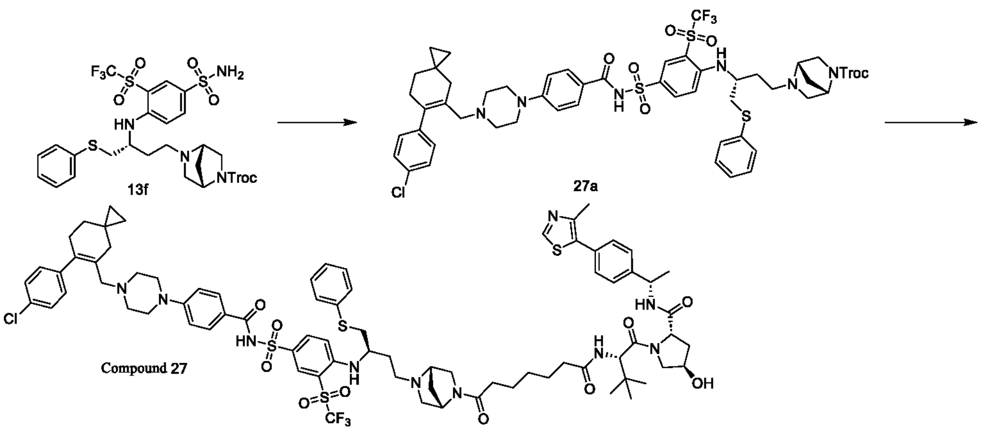
13f

27a

Compound 27

Step 1: Preparation of 27a

13f (0.50 g, 0.68 mmol) was added to 6 mL of DCM. 11a (0.30 g, 0.687 mmol), DMAP (0.17 g, 1.39 mmol) and EDCI (0.26 g, 1.36 mmol) were sequentially added and the mixture was reacted at 35°C for 12 h. The reaction system was cooled to room temperature and 5 mL of water and 3 mL of dichloromethane were added, followed by liquid separation. The aqueous phase was extracted with dichloromethane (5 mL × 3). The organic phases were combined, dried over anhydrous sodium sulphate and concentrated under reduced pressure and the resulting crude was separated and purified by chromatographic column on silica gel (dichloromethane : methanol (v/v) = 10 : 1) to afford 27a (0.30 g, yield: 38%).

LCMS m/z = 1159.1 $[M+1]^+$.

Step 2: Preparation of compound 27

27a (0.30 g, 0.26 mmol) was added to 3 mL of tetrahydrofuran and 15 mL of methanol. Zinc powder (1.36 g, 20.9 mmol) and ammonium chloride (0.42 g, 7.85 mmol) were sequentially added and the mixture was stirred at 27°C for 12 h. To the reaction solution was added 50 mL of ethyl acetate and 30 mL of water. The mixture was filtered over diatomaceous earth and the filtrate was extracted with ethyl acetate (15 mL × 2). The organic phases were combined, dried over anhydrous sodium sulphate and concentrated under reduced pressure to afford a crude (0.23 g). The above-mentioned crude (0.13 g) was added to 5 mL of DCM. Intermediate 1 (0.076 g, 0.13 mmol), triethylamine (0.066 g, 0.65 mmol) and HATU (0.074 g, 0.195 mmol) were sequentially added and the mixture was reacted at room temperature for 2 h. To the reaction solution was added 5 mL of water, followed by liquid separation. The aqueous phase was extracted with DCM (6 mL × 2) and the organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 0.05% $NH_4HCO_3$). Gradient elution method: gradient elution with acetonitrile from 50% to 80% (elution time: 15 min). Lyophilisation was performed to afford compound 27 (0.10 g, yield: 44%).

$^1H$ NMR (400 MHz, $CDCl_3$) δ 8.60 (s, 1H), 8.39 - 8.28 (m, 1H), 8.02 - 7.84 (m, 1H), 7.74 - 7.56 (m, 2H), 7.40 - 6.90 (m, 15H), 6.79 - 6.51 (m, 3H), 6.42 - 6.20 (m, 1H), 5.06 - 4.85 (m, 1H), 4.75 - 4.10 (m, 4H), 4.10 - 3.80 (m, 2H), 3.61 - 2.70 (m, 12H), 2.70 - 1.86 (m, 22H), 1.75 - 1.15 (m, 14H), 1.07 - 0.91 (m, 9H), 0.29 (s, 4H).
LCMS m/z = 776.5 $[M/2+1]^+$.

**Example 28:**

(2S,4R)-1-((S)-2-(8-((1S,4S)-5-((R)-3-((4-(N-(4-(4-((6-(4-chlorophenyl) spiro[2.5]oct-5-en-5-yl)methyl)piperazin-1-yl)benzoyl)sulphamoyl)-2-((trifluoromethyl)sulphonyl)phenyl)amino)-4-(phenylthio)butyl)-2,5-diazabicyclo[2.2.1]heptan-2-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide **(compound 28)**

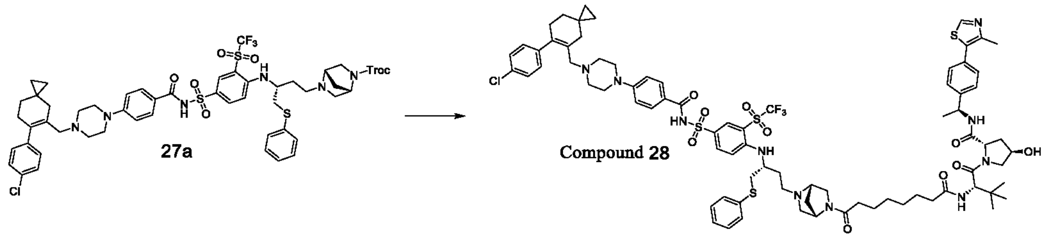

27a

Compound 28

27a (0.30 g, 0.26 mmol) was added to 3 mL of tetrahydrofuran and 15 mL of methanol. Zinc powder (1.36 g, 20.9 mmol) and ammonium chloride (0.42 g, 7.85 mmol) were sequentially added and the mixture was stirred at 27°C for 12 h. To the reaction solution was added 50 mL of ethyl acetate and 30 mL of water. The mixture was filtered over diatomaceous earth and the filtrate was extracted with ethyl acetate (15 mL × 2). The organic phases were combined, dried over anhydrous sodium sulphate and concentrated under reduced pressure to afford a crude (0.23 g). The above-mentioned crude (0.10 g) was added to 5 mL of DCM. Intermediate 3 (0.060 g, 0.10 mmol), triethylamine (0.051 g, 0.50 mmol) and HATU (0.057 g, 0.15 mmol) were sequentially added and the mixture was reacted at room temperature for 2 h. To the reaction solution was added 5 mL of water, followed by liquid separation. The aqueous phase was extracted with DCM (6 mL × 2) and the organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 0.05% $NH_4HCO_3$). Gradient elution method: gradient elution with acetonitrile from 50% to 80% (elution time: 15 min). Lyophilisation was performed to afford compound 28 (60 mg, yield: 34%).
$^1H$ NMR (400 MHz, $CDCl_3$) δ 8.67 (s, 1H), 8.38 (s, 1H), 8.10 - 7.92 (m, 1H), 7.80 - 7.66 (m, 2H), 7.49 - 7.12 (m, 13H), 7.07 - 6.98 (m, 2H), 6.83 - 6.58 (m, 3H), 6.38 - 6.24 (m, 1H), 5.15 - 4.97 (m, 1H), 4.83 - 4.20 (m, 4H), 4.16 - 3.90 (m, 2H), 3.68 - 2.75 (m, 13H), 2.75 - 1.97 (m, 21H), 1.97 - 1.17 (m, 16H), 1.14 - 0.90 (m, 9H), 0.36 (s, 4H).
LCMS m/z = 783.5 $[M/2+1]^+$.

**Example 29:**

(2S,4R)-1-((S)-2-(7-((1S,4S)-5-((R)-3-((4-(N-(4-(4-((6-(4-chloro-2-fluorophenyl)spiro[2.5]oct-5-en-5-yl)methyl)piperazin-1-yl)benzoyl)sulphamoyl)-2-((trifluoromethyl)sulphonyl)phenyl)amino)-4-(phenylthio)butyl)-2, 5-diazabicyclo [2.2.1]heptan-2-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 29)**

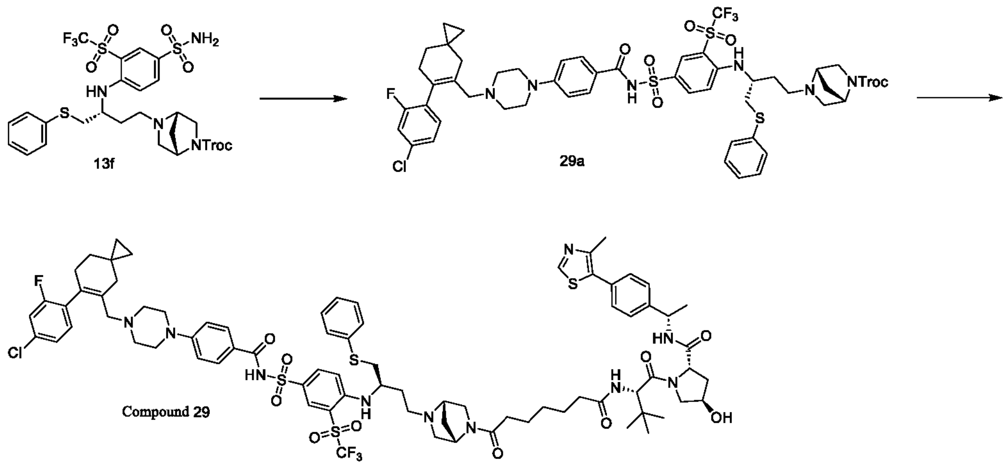
13f
29a
Compound 29

Step 1: Preparation of 29a

Sodium hydroxide (0.26 g, 6.5 mmol) was added to 5 mL of methanol and 3 mL of water. A solution of 21c (1.27 g, 2.63 mmol) in 10 mL of tetrahydrofuran was added and the resulting mixture was reacted at 80°C for 6 h. The reaction solution was cooled to room temperature and concentrated under reduced pressure. The residue was adjusted to pH 5 with 1 mol/L hydrochloric acid and extracted with ethyl acetate (15 mL × 3). The organic phase was washed with 20 mL of saturated sodium chloride solution, dried over anhydrous sodium sulphate and concentrated under reduced pressure to afford a crude (0.96 g). 13f (0.50 g, 0.68 mmol) was added to 6 mL of DCM. The above-mentioned crude (0.31 g), DMAP (0.17 g, 1.36 mmol) and EDCI (0.26, 1.36 mmol) were sequentially added and the mixture was warmed to 35°C and reacted for 12 h. The reaction system was cooled to room temperature and 5 mL of water and 3 mL of DCM were added, followed by liquid separation. The aqueous phase was extracted with DCM (5 mL × 3). The organic phases were combined, dried over anhydrous sodium sulphate and concentrated under reduced pressure and the resulting crude was separated and purified by chromatographic column on silica gel (dichloromethane : methanol (v/v) = 10 : 1) to afford 29a (0.35 g, yield: 44%).

LCMS m/z = 1177.3 $[M+1]^+$.

Step 2: Preparation of compound 29

29a (0.35 g, 0.30 mmol) was added to 3 mL of tetrahydrofuran and 15 mL of methanol. Zinc powder (1.57 g, 24.15 mmol) and ammonium chloride (0.48 g, 8.97 mmol) were sequentially added and the mixture was stirred at 27°C for 12 h. To the reaction solution was added 50 mL of ethyl acetate and 30 mL of water. The mixture was filtered over diatomaceous earth and the filtrate was extracted with ethyl acetate (15 mL × 2). The organic phases were combined, dried over anhydrous sodium sulphate and concentrated under reduced pressure to afford a crude (0.24 g). The above-mentioned crude (0.14 g) was added to 5 mL of DCM. Intermediate 1 (0.082 g, 0.14 mmol), triethylamine (0.071 g, 0.70 mmol) and HATU (0.080 g, 0.21 mmol) were sequentially added and the mixture was reacted at room temperature for 2 h. To the reaction solution was added 5 mL of water, followed by liquid separation. The aqueous phase was extracted with DCM (6 mL × 2) and the organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 0.05% $NH_4HCO_3$). Gradient elution method: gradient elution with acetonitrile from 50% to 80% (elution time: 15 min). Lyophilisation was performed to afford compound 29 (85 mg, yield: 31%).

$^1H$ NMR (400 MHz, $CDCl_3$) δ 8.67 (s, 1H), 8.45 - 8.35 (m, 1H), 8.08 - 7.92 (m, 1H), 7.84 - 7.64 (m, 2H), 7.44 - 6.94 (m, 14H), 6.82 - 6.58 (m, 3H), 6.50 - 6.30 (m, 1H), 5.17 - 4.90 (m, 1H), 4.85 - 4.15 (m, 4H), 4.15 - 3.87 (m, 2H), 3.80 - 2.92 (m, 10H), 2.90 - 1.80 (m, 24H), 1.80 - 1.20 (m, 14H), 1.04 (s, 9H), 0.37 (s, 4H).

LCMS m/z = 785.5 $[M/2+1]^+$.

**Example 30:**

(2S,4R)-1-((S)-2-(8-((1S,4S)-5-((R)-3-((4-(N-(4-(4-((6-(4-chloro-2-fluorophenyl)spiro[2.5]oct-5-en-5-yl)methyl)piperazin-1-yl)benzoyl)sulphamoyl)-2-((trifluoromethyl)sulphonyl)phenyl)amino)-4-(phenylthio)butyl)-2,5-diazabicyclo [2.2.1]heptan-2-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide **(compound 30)**

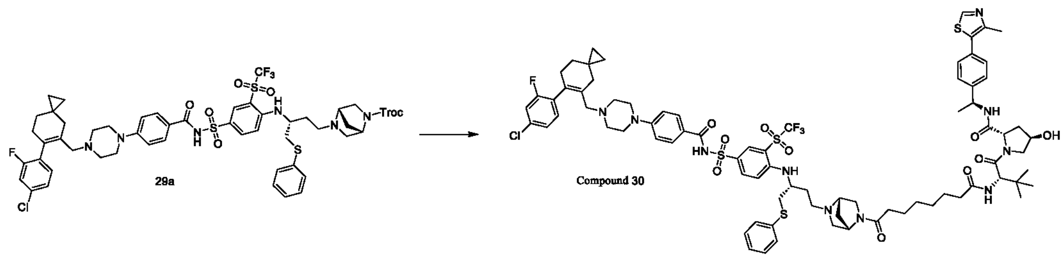


29a (0.35 g, 0.30 mmol) was added to 3 mL of tetrahydrofuran and 15 mL of methanol. Zinc powder (1.57 g, 24.15 mmol) and ammonium chloride (0.48 g, 8.97 mmol) were sequentially added and the mixture was stirred at 27°C for 12 h. To the reaction solution was added 50 mL of ethyl acetate and 30 mL of water. The mixture was filtered over diatomaceous earth and the filtrate was extracted with ethyl acetate (15 mL × 2). The organic phases were combined, dried over anhydrous sodium sulphate and concentrated under reduced pressure to afford a crude (0.24 g). The above-mentioned crude (0.10 g) was added to 5 mL of DCM. Intermediate 3 (0.060 g, 0.10 mmol), triethylamine (0.051 g, 0.50 mmol) and HATU (0.057 g, 0.15 mmol) were sequentially added and the mixture was reacted at room temperature for 2 h. To the reaction solution was added 5 mL of water, followed by liquid separation. The aqueous phase was extracted with DCM (6 mL × 2) and the organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 10 mmol/L $NH_4HCO_3$). Gradient elution method: gradient elution with acetonitrile from 35% to 65% (elution time: 15 min). Lyophilisation was performed to afford compound 30 (75 mg, yield: 38%).

$^1$H NMR (400 MHz, $CDCl_3$) δ 8.66 (s, 1H), 8.41 - 8.36 (m, 1H), 8.09 - 7.94 (m, 1H), 7.78 - 7.66 (m, 2H), 7.48 - 6.95 (m, 14H), 6.82 - 6.58 (m, 3H), 6.35 - 6.24 (m, 1H), 5.15 - 4.97 (m, 1H), 4.81 - 4.20 (m, 4H), 4.16 - 3.90 (m, 2H), 3.65 - 2.95 (m, 10H), 2.90 - 1.97 (m, 24H), 1.80 - 1.15 (m, 16H), 1.12 - 0.95 (m, 9H), 0.37 (s, 4H).

LCMS m/z = 792.5 $[M/2+1]^+$.

**Example 31:**

(2S,4R)-1-((S)-2-(7-(4-((R)-3-((4-(N-(4-(4-((6-(4-chloro-2-fluorophenyl)spiro [2.5]oct-5-en-5-yl)methyl)piperazin-1-yl)benzoyl)sulphamoyl)-2-((trifluoromethyl) sulphonyl)phenyl)amino)-4-(phenylthio)butyl)piperazin-1-yl)-7-oxoheptanamido)-3,3-dimethylbutanoyl)-4-hydroxy-N-((S)-1-(4-(4-methylthiazol-5-yl)phenyl)ethyl) pyrrolidine-2-carboxamide **(compound 31)**

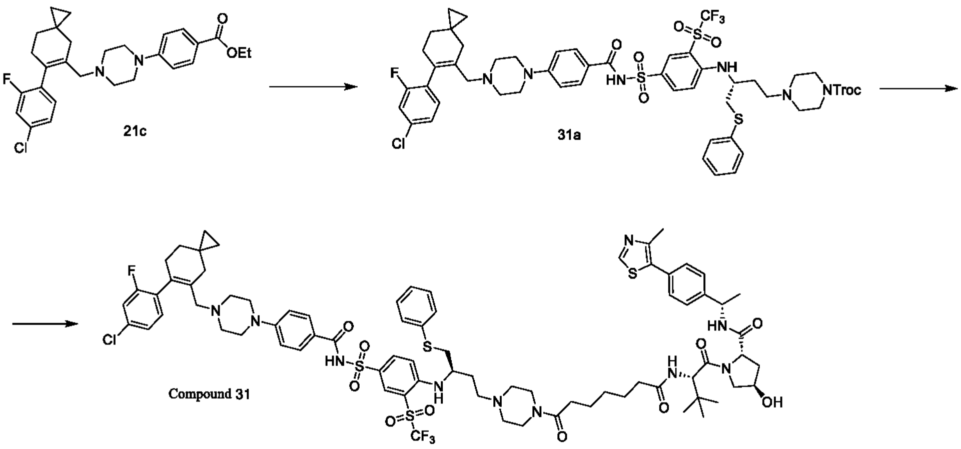

21c

31a

Compound 31

Step 1: Preparation of 31a

Sodium hydroxide (0.26 g, 6.5 mmol) was added to 5 mL of methanol and 3 mL of water. A solution of 21c (1.27 g, 2.63 mmol) in 10 mL of tetrahydrofuran was added and the resulting mixture was reacted at 80°C for 6 h. The reaction solution was cooled to room temperature and concentrated under reduced pressure. The residue was adjusted to pH 5 with 1 mol/L hydrochloric acid and extracted with ethyl acetate (15 mL × 3). The organic phase was washed with 20 mL of saturated sodium chloride solution, dried over anhydrous sodium sulphate and concentrated under reduced pressure to afford a crude (0.96 g). Intermediate 2 (0.62 g, 0.85 mmol) was added to 6 mL of DCM. The above-mentioned crude (0.43 g), DMAP (0.21 g, 1.72 mmol) and EDCI (0.33 g, 1.73 mmol) were sequentially added and the mixture was reacted at 35°C for 12 h. The reaction system was cooled to room temperature and 5 mL of water and 3 mL of DCM were added, followed by liquid separation. The aqueous phase was extracted with DCM (5 mL × 3). The organic phases were combined, dried over anhydrous sodium sulphate and concentrated under reduced pressure and the resulting crude was separated and purified by chromatographic column on silica gel (dichloromethane : methanol (v/v) = 10 : 1) to afford 31a (0.70 g, yield: 71%).

LCMS m/z = 583.2 $[M/2+1]^+$.

Step 2: **Preparation of** compound 31

31a (0.70 g, 0.6 mmol) was added to 5 mL of tetrahydrofuran and 15 mL of methanol. Zinc powder (3.14 g, 48.31 mmol) and ammonium chloride (0.96 g, 17.95 mmol) were sequentially added and the mixture was stirred at 27°C for 12 h. To the reaction solution was added 50 mL of ethyl acetate and 30 mL of water. The mixture was filtered over diatomaceous earth and the filtrate was extracted with ethyl acetate (15 mL × 2). The organic phases were combined, dried over anhydrous sodium sulphate and concentrated under reduced pressure to afford a crude (0.41 g). The above-mentioned crude (0.14 g) was added to 5 mL of DCM. Intermediate 1 (0.082 g, 0.14 mmol), triethylamine (0.071 g, 0.70 mmol) and HATU (0.080 g, 0.21 mmol) were sequentially added and the mixture was reacted at room temperature for 2 h. To the reaction solution was added 5 mL of water, followed by extraction using DCM (6 mL × 2). The organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18, mobile phase system: acetonitrile/water (containing 0.05% $NH_4HCO_3$), gradient elution method: gradient elution with acetonitrile from 50% to 80% (elution time: 15 min)). Lyophilisation was performed to afford compound 31 (50 mg, yield: 16%).

$^1$H NMR (400 MHz, $CDCl_3$) δ 8.67 (s, 1H), 8.40 - 8.32 (m, 1H), 8.09 (dd, 1H), 7.73 - 7.65 (m, 2H), 7.46 - 7.20 (m, 10H), 7.16 - 6.96 (m, 4H), 6.81 - 6.71 (m, 2H), 6.68 - 6.59 (m, 1H), 6.36 - 6.25 (m, 1H), 5.15 - 5.00 (m, 1H), 4.80 - 4.68 (m, 1H), 4.65 - 4.44 (m, 2H), 4.15 - 4.03 (m, 1H), 4.00 - 3.83 (m, 1H), 3.75 - 2.70 (m, 13H), 2.60 - 1.95 (m, 24H), 1.77 - 1.20 (m, 12H), 1.04 (s, 9H), 0.37 (s, 4H).

LCMS m/z = 779.5 $[M/2+1]^+$.

**Example 32:**

(2S,4R)-1-((S)-2-(8-(4-((R)-3-((4-(N-(4-(4-((6-(4-chloro-2-fluorophenyl) spiro[2.5]oct-5-en-5-yl)methyl)piperazin-1-yl)benzoyl)sulphamoyl)-2-((trifluoromethyl)sulphonyl)phenyl)amino)-4-(phenylthio)butyl)piperazin-1-yl)-8-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 32)**

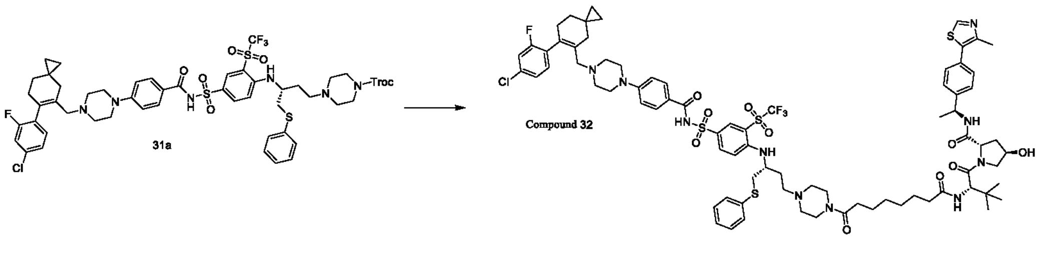

31a (0.70 g, 0.6 mmol) was added to 5 mL of tetrahydrofuran and 15 mL of methanol. Zinc powder (3.14 g, 48.31 mmol) and ammonium chloride (0.96 g, 17.95 mmol) were sequentially added and the mixture was stirred at 27°C for 12 h. To the reaction solution was added 50 mL of ethyl acetate and 30 mL of water. The mixture was filtered over diatomaceous earth and the filtrate was extracted with ethyl acetate (15 mL × 2). The organic phases were combined, dried over anhydrous sodium sulphate and concentrated under reduced pressure to afford a crude (0.41 g). The above-mentioned crude (0.10 g) was added to 5 mL of DCM. Intermediate 3 (0.060 g, 0.10 mmol), triethylamine (0.051 g, 0.504 mmol) and HATU (0.057 g, 0.15 mmol) were sequentially added and the mixture was reacted at room temperature for 2 h. To the reaction solution was added 5 mL of water, followed by extraction using DCM (6 mL × 2). The organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18, mobile phase system: acetonitrile/water (containing 0.05% $NH_4HCO_3$), gradient elution method: gradient elution with acetonitrile from 50% to 80% (elution time: 15 min)). Lyophilisation was performed to afford compound 32 (50 mg, yield: 22%).

$^1H$ NMR (400 MHz, $CDCl_3$) δ 8.68 (s, 1H), 8.37 - 8.32 (m, 1H), 8.09 (dd, 1H), 7.74 - 7.65 (m, 2H), 7.46 - 7.20 (m, 10H), 7.14 - 6.96 (m, 4H), 6.82 - 6.70 (m, 2H), 6.66 - 6.56 (m, 1H), 6.36 - 6.24 (m, 1H), 5.15 - 5.00 (m, 1H), 4.80 - 4.43 (m, 3H), 4.16 - 4.04 (m, 1H), 3.98 - 3.82 (m, 1H), 3.73 - 3.54 (m, 2H), 3.50 - 3.20 (m, 7H), 3.17 - 2.92 (m, 2H), 2.78 (s, 2H), 2.60 - 1.95 (m, 24H), 1.78 - 1.40 (m, 10H), 1.36 - 1.20 (m, 4H), 1.04 (s, 9H), 0.36 (s, 4H).

LCMS m/z = 786.5 $[M/2+1]^+$.

**Example 33:**

(2S,4R)-1-((S)-2-(7-((1S,4S)-5-((R)-3-((4-(N-(4-(4-((2-(4-chlorophenyl) cyclohept-1 -en-1 -yl)methyl)piperazin-1 -yl)benzoyl)sulphamoyl)-2-((trifluoromethyl) sulphonyl)phenyl)amino)-4-(phenylthio)butyl)-2,5-diazabicyclo[2.2.1]heptan-2-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 33)**

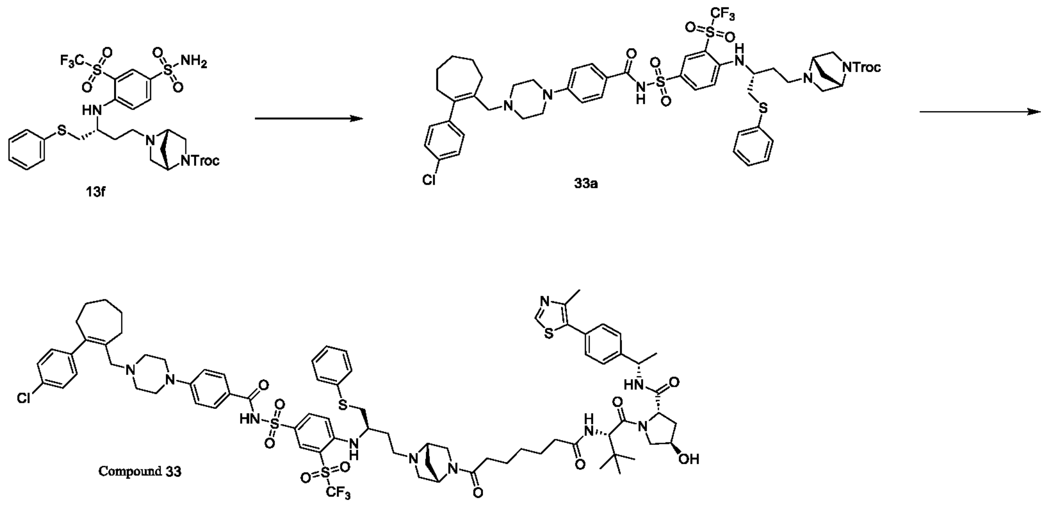

13f

33a

Compound 33

Step 1: Preparation of 33a

6c (4 g, 8.8 mmol) was dissolved in 100 mL of methanol. Water (10 mL) and sodium hydroxide (1.5 g, 37.5 mmol) were added and the mixture was reacted at 80°C for 10 h. The reaction solution was cooled to room temperature and concentrated under reduced pressure. The residue was dissolved with 100 mL of water and then extracted with 200 mL of methyl tert-butyl ether to remove the impurities. The aqueous phase was separated, adjusted to pH 6 with 1 mol/L hydrochloric acid, extracted with ethyl acetate (500 mL × 2), dried over anhydrous sodium sulphate and concentrated under reduced pressure to afford a crude (2.0 g). 13f (0.60 g, 0.82 mmol) was added to 10 mL of DCM. The above-mentioned crude (0.38 g), DMAP (0.20 g, 1.64 mmol) and EDCI (0.31 g, 1.62 mmol) were sequentially added and the mixture was reacted at 35°C for 12 h. To the reaction system were added 10 mL of water and 10 mL of DCM. Liquid separation was performed. The aqueous phase was extracted with 5 mL of DCM. The organic phases were combined, dried over anhydrous sodium sulphate and concentrated under reduced pressure and the crude was separated and purified by chromatographic column on silica gel (dichloromethane : methanol (v/v) = 10 : 1) to afford 33a (0.85 g, yield: 90%).

LCMS m/z = 1147.1 [M+1]$^+$.

Step 2: Preparation of compound 33

33a (0.85 g, 0.74 mmol) was added to 8 mL of tetrahydrofuran and 40 mL of methanol. Zinc powder (3.87 g, 59.54 mmol) and ammonium chloride (1.19 g, 22.25 mmol) were sequentially added and the mixture was reacted at 27°C for 12 h. The reaction system was filtered over diatomaceous earth. The filtrate was concentrated under reduced pressure and the resulting crude was separated and purified by chromatographic column on silica gel (dichloromethane : methanol (v/v) = 10 : 1) to afford a crude (0.46 g). The above-mentioned crude (0.22 g) was added to 10 mL of DCM. Intermediate 1 (0.15 g, 0.26 mmol), triethylamine (0.070 g, 0.69 mmol) and HATU (0.13 g, 0.34 mmol) were sequentially added and the mixture was reacted at room temperature for 2 h. To the reaction solution was added 10 mL of water, followed by liquid separation. The aqueous phase was extracted with DCM (6 mL × 2) and the organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 0.05% $NH_4HCO_3$). Gradient elution method: gradient elution with acetonitrile from 40% to 70% (elution time: 15 min). Lyophilisation was performed to afford compound 33 (165 mg, yield: 41%).

$^1$H NMR (400 MHz, $CDCl_3$) δ 8.67 (s, 1H), 8.47 - 8.35 (m, 1H), 8.06 - 7.91 (m, 1H), 7.82 - 7.64 (m, 2H), 7.44 - 7.08 (m, 13H), 7.03 - 6.92 (m, 2H), 6.83 - 6.57 (m, 3H), 6.50 - 6.31 (m, 1H), 5.15 - 4.90 (m, 1H), 4.84 - 3.86 (m, 6H), 3.67 - 2.94 (m, 10H), 2.94 - 2.71 (m, 3H), 2.71 - 1.98 (m, 22H), 1.98 - 1.42 (m, 14H), 1.40 - 1.22 (m, 3H), 1.13 - 0.98 (m, 9H).

LCMS m/z = 514.0 [M/3+1]$^+$.

**Example 34:**

(2S,4R)-1-((S)-2-(8-((1S,4S)-5-((R)-3-((4-(N-(4-(4-((2-(4-chlorophenyl) cyclohept-1 -en-1 -yl)methyl)piperazin-1 -yl)benzoyl)sulphamoyl)-2-((trifluoromethyl) sulphonyl)phenyl)amino)-4-(phenylthio)butyl)-2,5-diazabicyclo[2.2.1]heptan-2-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide **(compound 34)**

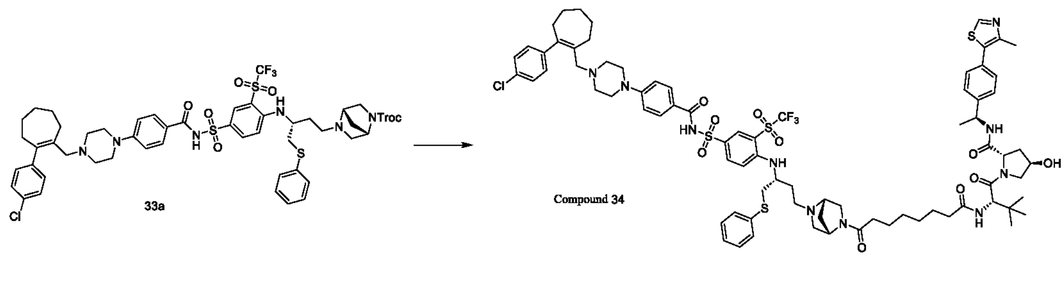


33a (0.85 g, 0.74 mmol) was added to 8 mL of tetrahydrofuran and 40 mL of methanol. Zinc powder (3.87 g, 59.54 mmol) and ammonium chloride (1.19 g, 22.25 mmol) were sequentially added and the mixture was reacted at 27°C for 12 h. The reaction system was filtered over diatomaceous earth. The filtrate was concentrated under reduced pressure and the resulting crude was separated and purified by chromatographic column on silica gel (dichloromethane : methanol (v/v) = 10 : 1) to afford a crude (0.46 g). The above-mentioned crude (0.22 g) was added to 5 mL of DCM. Intermediate 3 (0.15 g, 0.25 mmol), triethylamine (0.070 g, 0.69 mmol) and HATU (0.13 g, 0.34 mmol) were sequentially added and the mixture was reacted at room temperature for 2 h. To the reaction solution was added 10 mL of water, followed by liquid separation. The aqueous phase was extracted with DCM (6 mL × 2). The organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18, mobile phase system: acetonitrile/water (containing 0.05% $NH_4HCO_3$), gradient elution method: gradient elution with acetonitrile from 45% to 75% (elution time: 15 min)). Lyophilisation was performed to afford compound 34 (158 mg, yield: 41%).

$^1$H NMR (400 MHz, $CDCl_3$) δ 8.67 (s, 1H), 8.41 - 8.35 (m, 1H), 8.08 - 7.94 (m, 1H), 7.78 - 7.66 (m, 2H), 7.50 - 7.16 (m, 13H), 7.01 - 6.93 (m, 2H), 6.84 - 6.55 (m, 3H), 6.39 - 6.25 (m, 1H), 5.14 - 4.97 (m, 1H), 4.81 - 3.85 (m, 6H), 3.66 - 2.73 (m, 13H), 2.73 - 1.87 (m, 22H), 1.87 - 1.38 (m, 15H), 1.37 - 1.20 (m, 4H), 1.13 - 0.95 (m, 9H).

LCMS m/z = 777.5 $[M/2+1]^+$.

**Example 35:**

(2S,4R)-1-((S)-2-(7-(4-((R)-3-((4-(N-(4-(4-((2-(4-chloro-2-fluorophenyl) cyclohept-1 -en-1 -yl)methyl)piperazin-1 -yl)benzoyl)sulphamoyl)-2-((trifluoromethyl) sulphonyl)phenyl)amino)-4-(phenylthio)butyl)piperazin-1-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 35)**

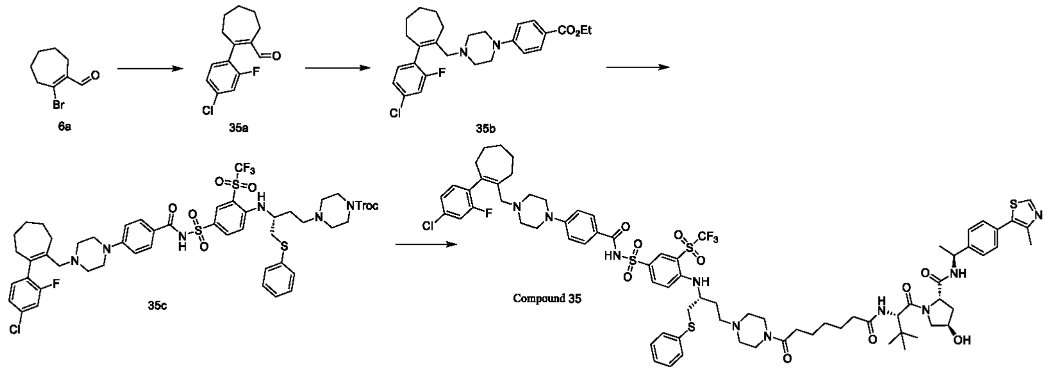

6a 35a 35b 35c Compound 35

Step 1: Preparation of 35a

6a (2.20 g, 10.84 mmol) and (4-chloro-2-fluorophenyl)boronic acid (2.83 g, 16.23 mmol) were dissolved in 15 mL of 1,4-dioxane and 1.5 mL of water. Under nitrogen protection, potassium acetate (3.19 g, 32.51 mmol) and Pd(dppf)$Cl_2$ (0.27 g, 0.36 mmol) were sequentially added and the mixture was reacted at 90°C for 1 h. The reaction solution was cooled to room temperature. To the reaction solution was slowly added 20 mL of ethyl acetate and 20 mL of water, followed by liquid separation. The aqueous phase was extracted with ethyl acetate (25 mL × 2). The organic phases were combined, washed with 25 mL of saturated aqueous sodium chloride solution, dried over anhydrous sodium sulphate and concentrated under reduced pressure and the resulting crude was separated and purified by silica gel column chromatography (petroleum ether/ethyl acetate (v/v) = 1 : 0-20 : 1) to afford 35a (2.5 g, yield: 92%).
LCMS m/z = 253.0 $[M+1]^+$.

Step 2: Preparation of 35b

35a (0.60 g, 2.38 mmol), ethyl 4-(piperazin-1-yl)benzoate (0.83 g, 3.54 mmol) and acetic acid (0.94 g, 15.67 mmol) were added to 50 mL of tetrahydrofuran. Sodium triacetoxyborohydride (1.51 g, 7.13 mmol) was added and the mixture was reacted at room temperature for 16 h. To the reaction solution was added 50 mL of saturated aqueous sodium bicarbonate solution, followed by extraction using ethyl acetate (50 mL × 2). The organic phases were combined, washed with 25 mL of saturated aqueous sodium chloride solution, dried over anhydrous sodium sulphate and concentrated under reduced pressure and the resulting crude was separated and purified by silica gel column chromatography (petroleum ether/ethyl acetate (v/v) = 1 : 0-5 : 1) to afford 35b (0.80 g, yield: 71%).
LCMS m/z = 471.2 $[M+1]^+$.

Step 3: Preparation of 35c

35b (0.80 g, 1.70 mmol) was dissolved in mixed solvents of 8 mL of tetrahydrofuran, 4 mL of ethanol and 2 mL of water. Sodium hydroxide (0.27 g, 6.75 mmol) was added and the mixture was stirred at 65°C for 16 h. The reaction solution was cooled to room temperature and concentrated under reduced pressure. To the residue was added 20 mL of water. Then the mixture was extracted with 20 mL of methyl tert-butyl ether to remove the impurities. The aqueous phase was adjusted to pH 5 with 1 mol/L hydrochloric acid and extracted with ethyl acetate (15 mL × 2). The organic phases were combined, washed with 25 mL of saturated sodium chloride solution, dried over anhydrous sodium sulphate and concentrated under reduced pressure to afford a crude (0.68 g). Intermediate 2 (0.60 g, 0.82 mmol) was added to 12 mL of DCM. The above-mentioned crude (0.40 g), DMAP (0.20 g, 1.64 mmol) and EDCI (0.31 g, 1.62 mmol) were sequentially added and the mixture was warmed to 35°C and reacted for 12 h. To the reaction system were added 5 mL of water and 3 mL of DCM. Liquid separation was performed. The aqueous phase was extracted with 5 mL of DCM. The organic phases were combined, dried over anhydrous sodium sulphate and concentrated under reduced pressure and the crude was separated and purified by chromatographic column on silica gel (dichloromethane : methanol (v/v) = 50 : 1-10 : 1) to afford 35c (0.62 g, yield: 66%).
LCMS m/z = 1153.1 $[M+1]^+$.

Step 4: Preparation of compound 35

35c (0.62 g, 0.54 mmol) was added to 5 mL of tetrahydrofuran and 30 mL of methanol. Zinc powder (2.83 g, 43.54 mmol) and ammonium chloride (0.87 g, 16.26 mmol) were sequentially added and the mixture was reacted at 27°C for 12 h. The reaction solution was filtered over diatomaceous earth. The filtrate was concentrated under reduced

pressure and the resulting crude was separated and purified by chromatographic column on silica gel (dichloromethane : methanol (v/v) = 10 : 1) to afford a crude (0.34 g). The above-mentioned crude (0.17 g) was added to 15 mL of DCM. Intermediate 1 (0.11 g, 0.19 mmol), triethylamine (0.17 g, 1.70 mmol) and HATU (0.097 g, 0.26 mmol) were sequentially added and the mixture was reacted at room temperature for 2 h. To the reaction solution was added 5 mL of water, followed by liquid separation. The aqueous phase was extracted with DCM (6 mL × 2) and the organic phases were combined, dried over anhydrous sodium sulphate and concentrated under reduced pressure. The resulting crude was separated and purified by chromatographic column on silica gel (dichloromethane : methanol (v/v) = 50 : 1-10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 0.05% $NH_4HCO_3$). Gradient elution method: gradient elution with acetonitrile from 40% to 70% (elution time: 15 min). Lyophilisation was performed to afford compound 35 (24.02 mg, yield: 8%).

$^1$H NMR (400 MHz, $CDCl_3$) δ 8.67 (s, 1H), 8.42 - 8.30 (m, 1H), 8.14 - 8.05 (m, 1H), 7.74 - 7.62 (m, 2H), 7.45 - 7.21 (m, 10H), 7.16 - 6.88 (m, 4H), 6.82 - 6.70 (m, 2H), 6.68 - 6.57 (m, 1H), 6.36 - 6.21 (m, 1H), 5.15 - 5.00 (m, 1H), 4.80 - 4.67 (m, 1H), 4.65 - 4.55 (m, 1H), 4.55 - 4.44 (m, 1H), 4.17 - 4.05 (m, 1H), 4.00 - 3.83 (m, 1H), 3.75 - 3.52 (m, 2H), 3.50 - 3.20 (m, 7H), 3.18 - 2.95 (m, 2H), 2.95 - 2.70 (m, 2H), 2.60 - 1.95 (m, 24H), 1.90 - 1.40 (m, 14H), 1.40 - 1.20 (m, 2H), 1.04 (s, 9H).

LCMS m/z = 516.1 $[M/3+1]^+$.

**Example 36:**

(2S,4R)-1-((S)-2-(8-(4-((R)-3-((4-(N-(4-(4-((2-(4-chloro-2-fluorophenyl) cyclohept-1 -en-1 -yl)methyl)piperazin-1 -yl)benzoyl)sulphamoyl)-2-((trifluoromethyl) sulphonyl)phenyl)amino)-4-(phenylthio)butyl)piperazin-1-yl)-8-oxooctanamido)-3,3-dimethylbutanoyl)-4-hydroxy-N-((S)-1-(4-(4-methylthiazol-5-yl)phenyl)ethyl) pyrrolidine-2-carboxamide **(compound 36)**

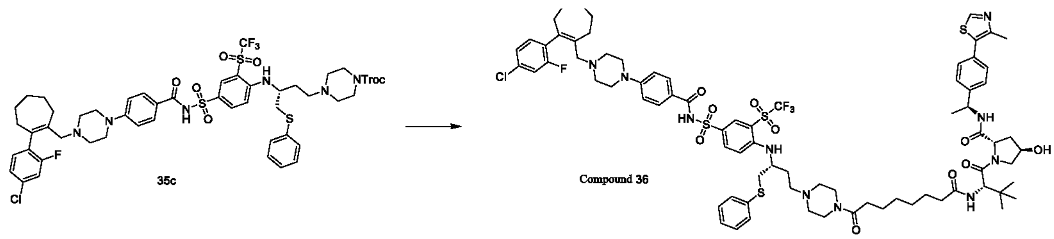


35c (0.62 g, 0.54 mmol) was added to 5 mL of tetrahydrofuran and 30 mL of methanol. Zinc powder (2.83 g, 43.54 mmol) and ammonium chloride (0.87 g, 16.26 mmol) were sequentially added and the mixture was reacted at 27°C for 12 h. The reaction solution was filtered over diatomaceous earth. The filtrate was concentrated under reduced pressure and the resulting crude was separated and purified by chromatographic column on silica gel (dichloromethane : methanol (v/v) = 10 : 1) to afford a crude (0.34 g). The above-mentioned crude (0.17 g) was added to 5 mL of DCM. Intermediate 3 (0.11 g, 0.18 mmol), triethylamine (0.17 g, 1.70 mmol) and HATU (0.097 g, 0.26 mmol) were sequentially added and the mixture was reacted at room temperature for 2 h. To the reaction solution was added 10 mL of water, followed by extraction using DCM (6 mL × 2). The organic phases were combined, dried over anhydrous sodium sulphate and concentrated under reduced pressure. The resulting crude was separated and purified by chromatographic column on silica gel (dichloromethane : methanol (v/v) = 50 : 1-10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 0.05% $NH_4HCO_3$). Gradient elution method: gradient elution with acetonitrile from 42% to 72% (elution time: 15 min). Lyophilisation was performed to afford compound 36 (25 mg, yield: 9%).

$^1$H NMR (400 MHz, $CDCl_3$) δ 8.67 (s, 1H), 8.40 - 8.32 (m, 1H), 8.13 - 8.05 (m, 1H), 7.75 - 7.61 (m, 2H), 7.45 - 7.21 (m, 10H), 7.14 - 7.02 (m, 3H), 6.98 - 6.90 (m, 1H), 6.84 - 6.58 (m, 3H), 6.32 - 6.15 (m, 1H), 5.15 - 5.00 (m, 1H), 4.78 - 4.68 (m, 1H), 4.64 - 4.46 (m, 2H), 4.17 - 4.06 (m, 1H), 4.02 - 3.84 (m, 1H), 3.80 - 3.20 (m, 9H), 3.18 - 2.72 (m, 4H), 2.70 - 2.00 (m, 24H), 1.95 - 1.20 (m, 18H), 1.04 (s, 9H).

LCMS m/z = 520.8 $[M/3+1]^+$.

**Example 37:**

(2S,4R)-1-((S)-2-(7-((1S,4S)-5-((R)-3-((4-(N-(4-(4-((2-(4-chloro-2-fluorophenyl)cyclohept-1-en-1-yl)methyl)piperazin-1-yl)benzoyl)sulphamoyl)-2-((trifluoromethyl)sulphonyl)phenyl)amino)-4-(phenylthio)butyl)-2,5-diazabicyclo [2.2.1]heptan-2-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 37)**

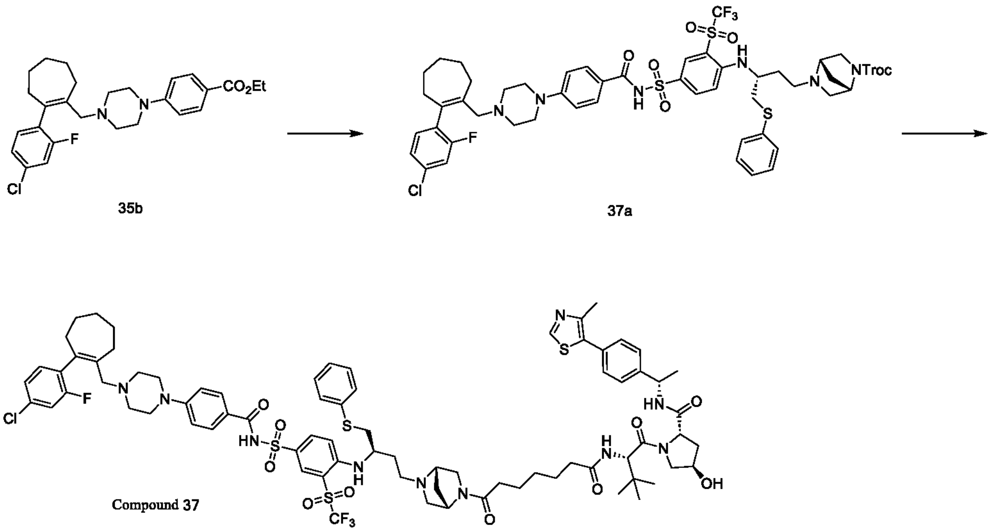

35b

37a

Compound 37

Step 1: Preparation of 37a

35b (0.80 g, 1.70 mmol) was dissolved in mixed solvents of 8 mL of tetrahydrofuran, 4 mL of ethanol and 2 mL of water. Sodium hydroxide (0.27 g, 6.75 mmol) was added and the mixture was stirred at 65°C for 16 h. The reaction solution was cooled to room temperature and concentrated under reduced pressure. To the residue was added 20 mL of water. Then the mixture was extracted with 20 mL of methyl tert-butyl ether to remove the impurities. The aqueous phase was adjusted to pH 5 with 1 mol/L hydrochloric acid and extracted with ethyl acetate (15 mL × 2). The organic phases were combined, washed with 25 mL of saturated sodium chloride solution, dried over anhydrous sodium sulphate and concentrated under reduced pressure to afford a crude (0.68 g). 13f (0.60 g, 0.82 mmol) was added to 12 mL of DCM. The above-mentioned crude (0.39 g), DMAP (0.20 g, 1.64 mmol) and EDCI (0.31 g, 1.62 mmol) were sequentially added and the mixture was reacted at 35°C for 12 h. To the reaction system were added 5 mL of water and 3 mL of DCM. Liquid separation was performed. The aqueous phase was extracted with 5 mL of DCM. The organic phases were combined, dried over anhydrous sodium sulphate and concentrated under reduced pressure and the crude was separated and purified by chromatographic column on silica gel (dichloromethane : methanol (v/v) = 50 : 1-10 : 1) to afford 37a (0.75 g, yield: 79%).

LCMS m/z = 1165.2 $[M+1]^+$.

Step 2: Preparation of compound 37

37a (0.75 g, 0.64 mmol) was added to 8 mL of tetrahydrofuran and 40 mL of methanol. Zinc powder (3.35 g, 51.54 mmol) and ammonium chloride (1.03 g, 19.26 mmol) were sequentially added and the mixture was reacted at 27°C for 12 h. The reaction solution was filtered over diatomaceous earth. The filtrate was concentrated under reduced pressure and the resulting crude was separated and purified by chromatographic column on silica gel (dichloromethane : methanol (v/v) = 50 : 1- 10 : 1) to afford a crude (0.46 g). The above-mentioned crude (0.23 g) was added to 10 mL of DCM. Intermediate 1 (0.15 g, 0.26 mmol), triethylamine (0.23 g, 2.30 mmol) and HATU (0.13 g, 0.34 mmol) were sequentially added and the mixture was reacted at room temperature for 2 h. To the reaction solution was added 10 mL of water, followed by liquid separation. The aqueous phase was extracted with DCM (6 mL × 2) and the organic phases were combined, dried over anhydrous sodium sulphate and concentrated under reduced pressure. The resulting crude was separated and purified by chromatographic column on silica gel (dichloromethane : methanol (v/v) = 50 : 1-10 : 1) to afford a crude, which was then passed through Pre-HPLC (instrument and preparative column: using SHIMADZU LC-20AP preparative liquid phase chromatographic instrument, preparative column model: Phenomenex C18). Mobile

phase system: acetonitrile/water (containing 10 mmol/L $NH_4HCO_3$). Gradient elution method: gradient elution with acetonitrile from 53% to 83% (elution time: 10 min). Lyophilisation was performed to afford compound 37 (68 mg, yield: 17%).

$^1$H NMR (400 MHz, $CDCl_3$) δ 8.67 (s, 1H), 8.45 - 8.35 (m, 1H), 8.08 - 7.92 (m, 1H), 7.80 - 7.65 (m, 2H), 7.45 - 7.10 (m, 11H), 7.09 - 7.01 (m, 2H), 6.98 - 6.87 (m, 1H), 6.82 - 6.58 (m, 3H), 6.51 - 6.32 (m, 1H), 5.14 - 4.92 (m, 1H), 4.84 - 3.85 (m, 6H), 3.68 - 2.92 (m, 10H), 2.87 - 1.95 (m, 25H), 1.92 - 1.42 (m, 14H), 1.40 - 1.21 (m, 3H), 1.09 - 0.99 (m, 9H).

LCMS m/z = 520.2 [M/3+1]$^+$.

**Example 38:**

(2S,4R)-1-((S)-2-(8-((1S,4S)-5-((R)-3-((4-(N-(4-(4-((2-(4-chloro-2-fluorophenyl)cyclohept-1-en-1-yl)methyl)piperazin-1-yl)benzoyl)sulphamoyl)-2-((trifluoromethyl)sulphonyl)phenyl)amino)-4-(phenylthio)butyl)-2,5-diazabicyclo[2.2.1]heptan-2-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide **(compound 38)**

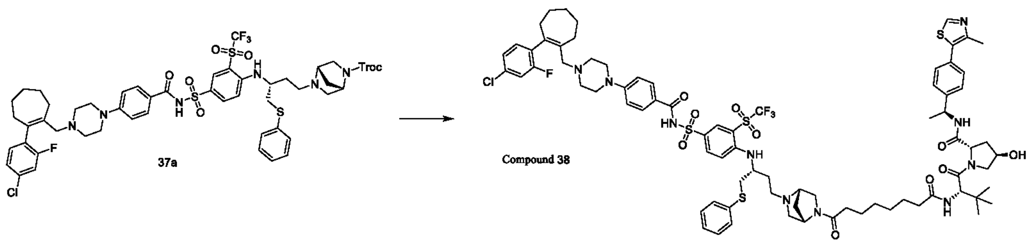


37a (0.75 g, 0.64 mmol) was added to 8 mL of tetrahydrofuran and 40 mL of methanol. Zinc powder (3.35 g, 51.54 mmol) and solid ammonium chloride (1.03 g, 19.26 mmol) were sequentially added and the mixture was reacted at 27°C for 12 h. The reaction solution was filtered over diatomaceous earth. The filtrate was concentrated under reduced pressure and the resulting crude was separated and purified by chromatographic column on silica gel (dichloromethane : methanol (v/v) = 50 : 1 -10 : 1) to afford a crude (0.46 g). The above-mentioned crude (0.23 g) was added to 10 mL of DCM. Intermediate 3 (0.15 g, 0.25 mmol), triethylamine (0.070 g, 0.69 mmol) and HATU (0.13 g, 0.34 mmol) were sequentially added and the mixture was reacted at room temperature for 2 h. To the reaction solution was added 10 mL of water, followed by liquid separation. The aqueous phase was extracted with DCM (6 mL × 2). The organic phases were combined, dried over anhydrous sodium sulphate and concentrated under reduced pressure. The resulting crude was separated and purified by chromatographic column on silica gel (dichloromethane : methanol (v/v) = 50 : 1-10 : 1) to afford a crude, which was then passed through Pre-HPLC (instrument and preparative column: using SHIMADZU LC-20AP preparative liquid phase chromatographic instrument, preparative column model: Phenomenex C18, mobile phase system: acetonitrile/water (containing 10 mmol/L $NH_4HCO_3$), gradient elution method: gradient elution with acetonitrile from 53% to 83% (elution time: 10 min)). Lyophilisation was performed to afford compound 38 (66 mg, yield: 17%).

$^1$H NMR (400 MHz, $CDCl_3$) δ 8.67 (s, 1H), 8.42 - 8.35 (m, 1H), 8.07 - 7.94 (m, 1H), 7.78 - 7.66 (m, 2H), 7.50 - 7.14 (m, 11H), 7.11 - 7.00 (m, 2H), 6.98 - 6.88 (m, 1H), 6.83 - 6.57 (m, 3H), 6.35 - 6.23 (m, 1H), 5.15 - 4.97 (m, 1H), 4.83 - 3.88 (m, 6H), 3.65 - 2.95 (m, 10H), 2.95 - 2.03 (m, 24H), 2.01 - 1.38 (m, 16H), 1.35 - 1.20 (m, 4H), 1.10 - 0.98 (m, 9H).

LCMS m/z = 524.8 [M/3+1]$^+$.

**Example 39:**

cis-(2S,4R)-1-((S)-2-(7-(5-((R)-3-((4-(N-(4-(4-((2-(4-chloro-2-fluorophenyl) cyclohept-1 -en-1 -yl)methyl)piperazin-1 -yl)benzoyl)sulphamoyl)-2-((trifluoromethyl) sulphonyl)phenyl)amino)-4-(phenylthio)butyl)hexahydropyrrolo[3,4-c]pyrrol-2(1H)-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 39)**

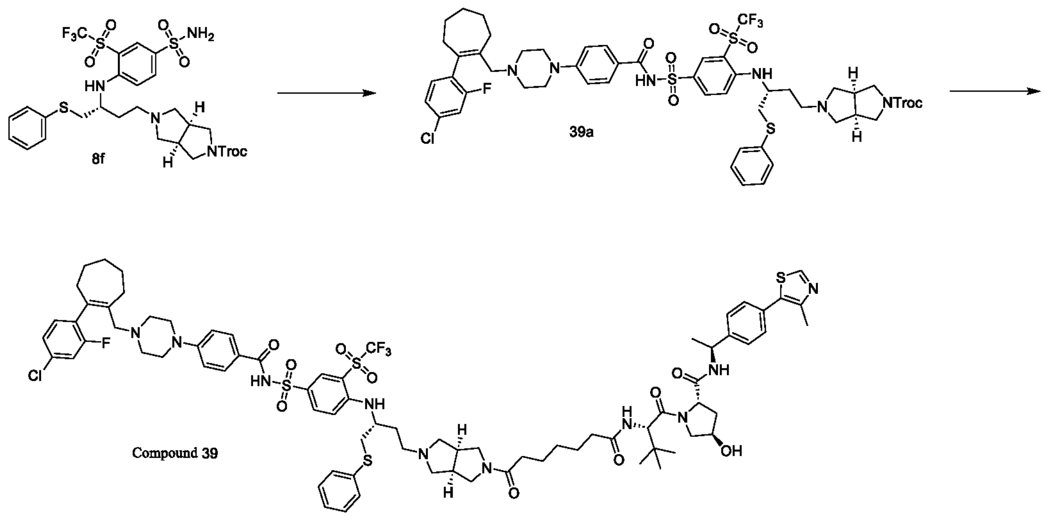

8f

39a

Compound 39

Step 1: Preparation of 39a

35b (0.80 g, 1.70 mmol) was dissolved in mixed solvents of 8 mL of tetrahydrofuran, 4 mL of ethanol and 2 mL of water. Sodium hydroxide (0.27 g, 6.75 mmol) was added and the mixture was stirred at 65°C for 16 h. The reaction solution was cooled to room temperature and concentrated under reduced pressure. To the residue was added 20 mL of water. Then the mixture was extracted with 20 mL of methyl tert-butyl ether to remove the impurities. The aqueous phase was adjusted to pH 5 with 1 mol/L hydrochloric acid and extracted with ethyl acetate (15 mL × 2). The organic phases were combined, washed with 25 mL of saturated sodium chloride solution, dried over anhydrous sodium sulphate and concentrated under reduced pressure to afford a crude (0.68 g). 8f (1.00 g, 1.33 mmol) was added to 12 mL of DCM. The above-mentioned crude (0.65 g), DMAP (0.32 g, 2.62 mmol) and EDCI (0.51 g, 2.67 mmol) were sequentially added and the mixture was reacted at 35°C for 12 h. To the reaction system were added 10 mL of water and 5 mL of DCM. Liquid separation was performed. The aqueous phase was extracted with 5 mL of DCM. The organic phases were combined, dried over anhydrous sodium sulphate and concentrated under reduced pressure and the crude was separated and purified by chromatographic column on silica gel (dichloromethane : methanol (v/v) = 20 : 1) to afford 39a (0.90 g, yield: 57%).

LCMS m/z = 1179.1 $[M+1]^+$.

Step 2: Preparation of compound 39

39a (0.90 g, 0.76 mmol) was added to 9 mL of tetrahydrofuran and 45 mL of methanol. Zinc powder (3.98 g, 61.23 mmol) and ammonium chloride (1.22 g, 22.81 mmol) were sequentially added and the mixture was reacted at 27°C for 12 h. The reaction solution was filtered over diatomaceous earth. The filtrate was concentrated under reduced pressure and the resulting crude was separated and purified by chromatographic column on silica gel (dichloromethane : methanol (v/v) = 10 : 1) to afford a crude (0.70 g). The above-mentioned crude (0.35 g) was added to 15 mL of DCM. Intermediate 1 (0.23 g, 0.40 mmol), triethylamine (0.11 g, 1.09 mmol) and HATU (0.20 g, 0.526 mmol) were sequentially added and the mixture was reacted at room temperature for 2 h. To the reaction solution was added 10 mL of water, followed by liquid separation. The aqueous phase was extracted with DCM (6 mL × 2) and the organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 10 mmol/L $NH_4HCO_3$). Gradient elution method: gradient elution with acetonitrile from 50% to 70% (elution time: 20 min). Lyophilisation was performed to afford compound 39 (280 mg, yield: 45%).

$^1H$ NMR (400 MHz, $CDCl_3$) δ 8.69 - 8.64 (m, 1H), 8.53 - 8.46 (m, 1H), 7.96 - 7.70 (m, 3H), 7.45 - 7.16 (m, 9H), 7.12 - 6.87 (m, 5H), 6.85 - 6.65 (m, 3H), 6.52 - 6.31 (m, 1H), 5.15 - 4.88 (m, 1H), 4.86 - 4.65 (m, 2H), 4.57 - 4.45 (m, 1H), 4.25 - 3.86 (m, 2H), 3.78 - 3.41 (m, 3H), 3.40 - 2.94 (m, 8H), 2.94 - 1.98 (m, 26H), 1.92 - 1.40 (m, 14H), 1.39 - 1.22 (m, 4H), 1.13 - 0.98 (m, 9H).

LCMS m/z = 786.6 $[M/2+1]^+$.

**Example 40:**

cis-(2S,4R)-1-((S)-2-(8-(5-((R)-3-((4-(N-(4-(4-((2-(4-chloro-2-fluorophenyl) cyclohept-1 -en-1 -yl)methyl)piperazin-1 -yl)benzoyl)sulphamoyl)-2-((trifluoromethyl) sulphonyl)phenyl)amino)-4-(phenylthio)butyl)hexahydropyrrolo[3,4-c]pyrrol-2(1H)-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide **(compound 40)**

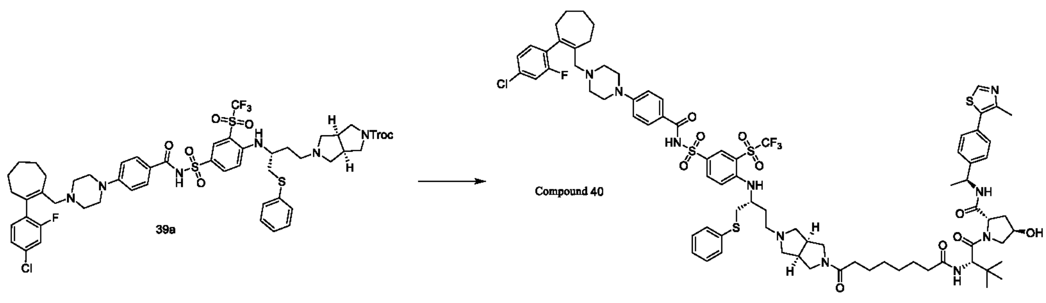


39a (0.90 g, 0.76 mmol) was added to 9 mL of tetrahydrofuran and 45 mL of methanol. Zinc powder (3.98 g, 61.23 mmol) and ammonium chloride (1.22 g, 22.81 mmol) were sequentially added and the mixture was reacted at 27°C for 12 h. The reaction solution was filtered over diatomaceous earth. The filtrate was concentrated under reduced pressure and the resulting crude was separated and purified by chromatographic column on silica gel (dichloromethane : methanol (v/v) = 10 : 1) to afford a crude (0.70 g). The above-mentioned crude (0.35 g) was added to 15 mL of DCM. Intermediate 3 (0.23 g, 0.38 mmol), triethylamine (0.11 g, 1.09 mmol) and HATU (0.20 g, 0.526 mmol) were sequentially added and the mixture was reacted at room temperature for 2 h. To the reaction solution was added 10 mL of water, followed by liquid separation. The aqueous phase was extracted with DCM (6 mL × 2). The organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18, mobile phase system: acetonitrile/water (containing 10 mmol/L $NH_4HCO_3$), gradient elution method: gradient elution with acetonitrile from 50% to 70% (elution time: 20 min)). Lyophilisation was performed to afford compound 40 (290 mg, yield: 48%).

$^1$H NMR (400 MHz, $CDCl_3$) δ 8.67 (s, 1H), 8.52 - 8.38 (m, 1H), 8.04 - 7.87 (m, 1H), 7.84 - 7.72 (m, 2H), 7.56 - 7.14 (m, 10H), 7.09 - 6.89 (m, 4H), 6.84 - 6.73 (m, 2H), 6.72 - 6.62 (m, 1H), 6.50 - 6.24 (m, 1H), 5.15 - 4.93 (m, 1H), 4.84 - 4.62 (m, 2H), 4.57 - 4.45 (m, 1H), 4.18 - 3.85 (m, 2H), 3.69 - 3.42 (m, 3H), 3.39 - 2.01 (m, 34H), 1.90 - 1.16 (m, 20H), 1.13 - 0.96 (m, 9H).

LCMS m/z = 793.9 $[M/2+1]^+$.

**Example 41:**

cis-(2S,4R)-1 -((S)-2-(7-(5-((R)-3-((4-(N-(4-(4-((2-(4-chlorophenyl)cyclohept-1 - en-1-yl)methyl)piperazin-1-yl)benzoyl)sulphamoyl)-2-((trifluoromethyl)sulphonyl) phenyl)amino)-4-(phenylthio)butyl)hexahydropyrrolo[3,4-c]pyrrol-2(1H)-yl)-7-oxoheptanamido)-3,3-dimethylbutanoyl)-4-hydroxy-N-((S)-1-(4-(4-methylthiazol-5-yl)phenyl)ethyl)pyrrolidine-2-carboxamide **(compound 41)**

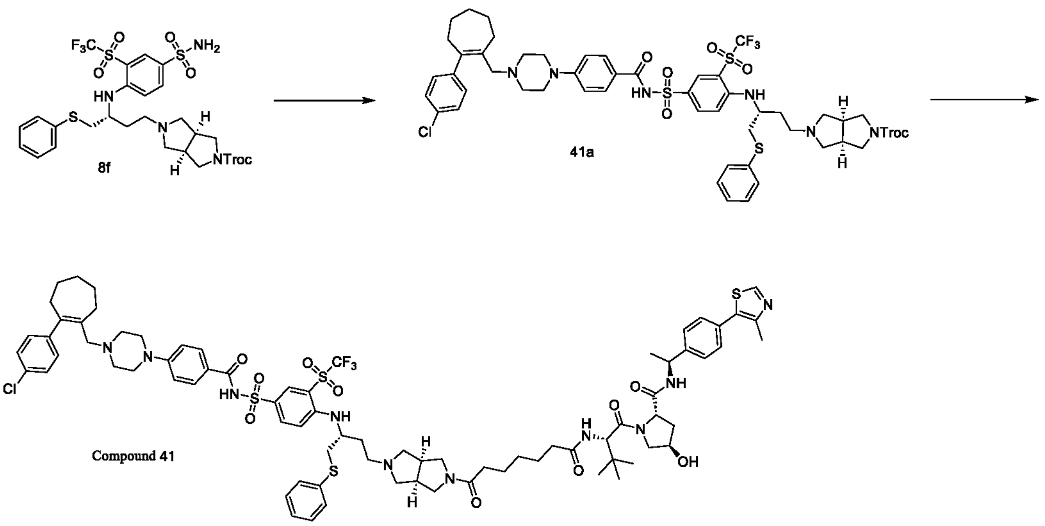

8f

41a

Compound 41

Step 1: Preparation of 41a

6c (0.40 g, 0.88 mmol) was dissolved in 10 mL of methanol. Water (1 mL) and sodium hydroxide (0.15 g, 3.75 mmol) were added and the mixture was reacted at 80°C for 10 h. The reaction solution was cooled to room temperature and concentrated under reduced pressure. The residue was dissolved with 10 mL of water and then extracted with 20 mL of methyl tert-butyl ether to remove the impurities. The aqueous phase was separated, adjusted to pH 6 with 1 mol/L hydrochloric acid, extracted with ethyl acetate (100 mL × 2), dried over anhydrous sodium sulphate and concentrated under reduced pressure to afford a crude (0.20 g). 8f (1.00 g, 1.33 mmol) was added to 12 mL of DCM. The above-mentioned crude (0.62 g), DMAP (0.32 g, 2.62 mmol) and EDCI (0.51 g, 2.67 mmol) were sequentially added and the mixture was reacted at 35°C for 12 h. To the reaction system were added 10 mL of water and 5 mL of dichloromethane. Liquid separation was performed. The aqueous phase was extracted with 5 mL of DCM. The organic phases were combined, dried over anhydrous sodium sulphate and concentrated under reduced pressure and the crude was separated and purified by chromatographic column on silica gel (dichloromethane : methanol (v/v) = 20 : 1) to afford 41a (1.00 g, yield: 65%).

LCMS m/z = 1161.2 $[M+1]^+$.

Step 2: **Preparation of** compound 41

41a (1.00 g, 0.86 mmol) was added to 10 mL of tetrahydrofuran and 50 mL of methanol. Zinc powder (4.50 g, 69.23 mmol) and ammonium chloride (1.38 g, 25.80 mmol) were sequentially added and the mixture was reacted at 27°C for 12 h. The reaction solution was filtered over diatomaceous earth. The filtrate was concentrated under reduced pressure and the resulting crude was separated and purified by chromatographic column on silica gel (dichloromethane : methanol (v/v) = 10 : 1) to afford a crude (0.80 g). The above-mentioned crude (0.40 g) was added to 15 mL of DCM. Intermediate 1 (0.26 g, 0.45 mmol), triethylamine (0.12 g, 1.19 mmol) and HATU (0.23 g, 0.605 mmol) were sequentially added and the mixture was reacted at room temperature for 2 h. To the reaction solution was added 10 mL of water, followed by liquid separation. The aqueous phase was extracted with DCM (6 mL × 2) and the organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 10 mmol/L $NH_4HCO_3$). Gradient elution method: gradient elution with acetonitrile from 45% to 75% (elution time: 20 min). Lyophilisation was performed to afford compound 41 (150 mg, yield: 21%).

$^1H$ NMR (400 MHz, $CDCl_3$) δ 8.70 - 8.62 (m, 1H), 8.52 - 8.44 (m, 1H), 7.96 - 7.70 (m, 3H), 7.44 - 6.92 (m, 15H), 6.83 - 6.65 (m, 3H), 6.55 - 6.35 (m, 1H), 5.15 - 4.90 (m, 1H), 4.85 - 4.65 (m, 2H), 4.55 - 4.45 (m, 1H), 4.22 - 3.86 (m, 2H), 3.80 - 3.40 (m, 3H), 3.37 - 1.96 (m, 34H), 1.90 - 1.20 (m, 18H), 1.10 - 0.98 (m, 9H).LCMS m/z = 777.3 $[M/2+1]^+$.

**Example 42:**

cis-(2S,4R)-1 -((S)-2-(8-(5-((R)-3-((4-(N-(4-(4-((2-(4-chlorophenyl)cyclohept-1 - en-1-yl)methyl)piperazin-1-yl)benzoyl)sulphamoyl)-2-((trifluoromethyl)sulphonyl) phenyl)amino)-4-(phenylthio)butyl)hexahydropyrrolo[3,4-c]pyrrol-2(1H)-yl)-8-oxooctanamido)-3,3-dimethylbutanoyl)-4-hydroxy-N-((S)-1-(4-(4-methylthiazol-5-yl)phenyl)ethyl)pyrrolidine-2-carboxamide **(compound 42)**

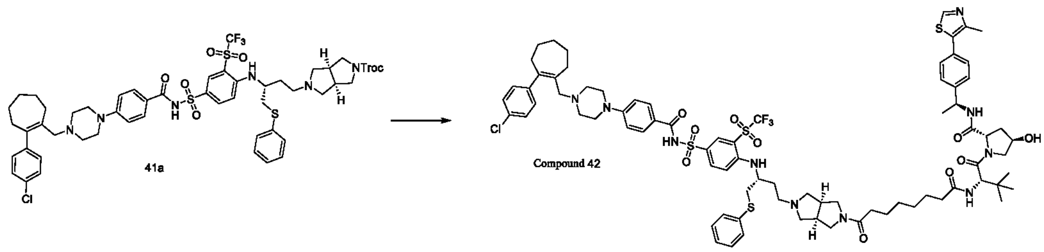


41a (1.00 g, 0.86 mmol) was added to 10 mL of tetrahydrofuran and 50 mL of methanol. Zinc powder (4.50 g, 69.23 mmol) and ammonium chloride (1.38 g, 25.80 mmol) were sequentially added and the mixture was reacted at 27°C for 12 h. The reaction solution was filtered over diatomaceous earth. The filtrate was concentrated under reduced pressure and the resulting crude was separated and purified by chromatographic column on silica gel (dichloromethane : methanol (v/v) = 10 : 1) to afford a crude (0.80 g). The above-mentioned crude (0.40 g) was added to 15 mL of DCM. Intermediate 3 (0.27 g, 0.45 mmol), triethylamine (0.12 g, 1.19 mmol) and HATU (0.23 g, 0.605 mmol) were sequentially added and the mixture was reacted at room temperature for 2 h. To the reaction solution was added 10 mL of water, followed by liquid separation. The aqueous phase was extracted with DCM (6 mL × 2). The organic phases were combined, dried over anhydrous sodium sulphate and concentrated under reduced pressure. The resulting crude was separated and purified by chromatographic column on silica gel (dichloromethane : methanol (v/v) = 10 : 1) to afford a crude, which was then passed through Pre-HPLC (instrument and preparative column: using SHIMADZU LC-20AP preparative liquid phase chromatographic instrument, preparative column model: Phenomenex C18, mobile phase system: acetonitrile/water (containing 10 mmol/L $NH_4HCO_3$), gradient elution method: gradient elution with acetonitrile from 45% to 75% (elution time: 20 min)). Lyophilisation was performed to afford compound 42 (270 mg, yield: 38%).

$^1H$ NMR (400 MHz, $CDCl_3$) δ 8.69 - 8.63 (m, 1H), 8.49 - 8.39 (m, 1H), 8.04 - 7.88 (m, 1H), 7.84 - 7.70 (m, 2H), 7.57 - 7.14 (m, 12H), 7.06 - 6.94 (m, 3H), 6.83 - 6.59 (m, 3H), 6.54 - 6.32 (m, 1H), 5.14 - 4.92 (m, 1H), 4.80 - 4.61 (m, 2H), 4.55 - 4.45 (m, 1H), 4.16 - 3.84 (m, 2H), 3.70 - 3.41 (m, 3H), 3.33 - 1.99 (m, 34H), 1.91 - 1.15 (m, 20H), 1.14 - 0.94 (m, 9H).LCMS m/z = 784.5 $[M/2+1]^+$.

**Example 43:**

(2S,4R)-1-((S)-2-(7-(4-((R)-3-((4-(N-(4-(4-((2-(4-chlorophenyl)cyclooct-1-en-1-yl)methyl)piperazin-1-yl)benzoyl)sulphamoyl)-2-((trifluoromethyl)sulphonyl) phenyl)amino)-4-(phenylthio)butyl)piperazin-1-yl)-7-oxoheptanamido)-3,3-dimethylbutanoyl)-4-hydroxy-N-((S)-1-(4-(4-methylthiazol-5-yl)phenyl)ethyl) pyrrolidine-2-carboxamide **(compound 43)**

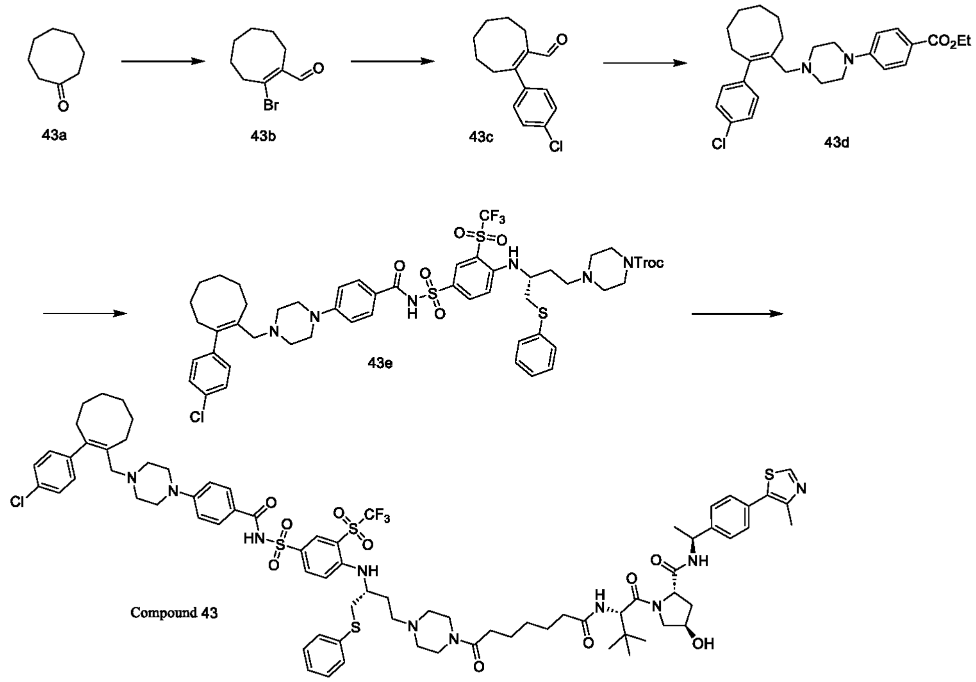

43a 43b 43c 43d

43e

Compound 43

Step 1: Preparation of 43b

DMF (17.12 g, 234.22 mmol) was added to 80 mL of dichloromethane. Under nitrogen protection, the temperature was controlled to be 0°C-5°C. $PBr_3$ (34.54 g, 127.6 mmol) was slowly added dropwise to the above-mentioned reaction solution. The ice-bath was removed and the mixture was slowly warmed to room temperature and stirred for 0.5 h. The reaction system was cooled to 0°C-5°C again. A solution of 43a (5.00 g, 39.62 mmol) in dichloromethane (40 mL) was added while the temperature was controlled in the range and then the mixture was warmed to room temperature and reacted for 16 h. The reaction solution was slowly added to 200 mL of ice water, followed by liquid separation. The aqueous phase was extracted with dichloromethane (100 mL × 2). The organic phases were combined, washed with 50 mL of saturated aqueous sodium chloride solution, dried over anhydrous sodium sulphate and concentrated under reduced pressure and the crude was separated and purified by chromatographic column on silica gel (pure petroleum ether) to afford 43b (2.20 g, yield: 26%).

Step 2: Preparation of 43c

43b (2.20 g, 10.13 mmol) and 4-chlorophenylboronic acid (2.38 g, 15.22 mmol) were added to 20 mL of 1,4-dioxane and 2 mL of water. Under nitrogen protection, potassium acetate (2.98 g, 30.36 mmol) and $Pd(dppf)Cl_2$ (0.25 g, 0.34 mmol) were sequentially added and the mixture was reacted at 90°C for 1 h. The reaction system was cooled to room temperature and 20 mL of ethyl acetate and 20 mL of water were added, followed by liquid separation. The aqueous phase was extracted with ethyl acetate (25 mL × 2). The organic phases were combined, washed with 30 mL of saturated aqueous sodium chloride solution, dried over anhydrous sodium sulphate and concentrated under reduced pressure and the resulting crude was separated and purified by silica gel column chromatography (petroleum ether/ethyl acetate (v/v) = 1 : 0-20 : 1) to afford 43c (1.60 g, yield: 64%).

LCMS m/z = 249.2 $[M+1]^+$.

Step 3: Preparation of 43d

43c (1.60 g, 6.45 mmol), ethyl (4-piperazin-1-yl)benzoate (2.26 g, 9.64 mmol) and acetic acid (2.56 g, 42.67 mmol) were added to 20 mL of tetrahydrofuran. Sodium triacetoxyborohydride (4.09 g, 19.30 mmol) was added and the mixture was reacted at room temperature for 16 h. To the reaction solution was added 100 mL of saturated sodium bicarbonate solution, followed by extraction using ethyl acetate (60 mL × 2). The organic phases were combined, washed with 50 mL of saturated aqueous sodium chloride solution, dried over anhydrous sodium sulphate and concentrated under reduced pressure and the resulting crude was separated and purified by silica gel column chromatography (petroleum ether/ethyl acetate (v/v) = 1 : 0-5 : 1) to afford 43d (2.20 g, yield: 73%).

LCMS m/z = 467.6 $[M+1]^+$.

Step 4: Preparation of 43e

43d (2.20 g, 4.71 mmol) was dissolved in mixed solvents of 32 mL of tetrahydrofuran, 16 mL of ethanol and 8 mL of water. Sodium hydroxide (0.75 g, 18.75 mmol) was added and the mixture was reacted at 65°C for 16 h. The reaction system was cooled to room temperature and concentrated under reduced pressure and 20 mL of water and 20 mL of methyl tert-butyl ether were added. The aqueous phase was separated, adjusted to pH 5 with 1 mol/L hydrochloric acid and extracted with ethyl acetate (15 mL × 2). The organic phases were combined, washed with 15 mL of saturated aqueous sodium chloride solution, dried over anhydrous sodium sulphate and concentrated under reduced pressure to afford a crude (0.80 g). Intermediate 2 (0.50 g, 0.68 mmol) was dissolved in 10 mL of dichloromethane. The above-mentioned crude (0.33 g), DMAP (0.17 g, 1.39 mmol) and EDCI (0.26 g, 1.36 mmol) were sequentially added and the mixture was reacted at 35°C for 12 h. To the reaction system was added 10 mL of saturated aqueous potassium dihydrogen phosphate solution, followed by liquid separation. The aqueous phase was extracted with 10 mL of dichloromethane and the organic phase was dried over anhydrous sodium sulphate and concentrated under reduced pressure. The resulting crude was separated and purified by chromatographic column on silica gel (dichloromethane : methanol (v/v) = 50 : 1-20 : 1) to afford 43e (0.75 g, yield: 96%).

LCMS m/z = 1149.3 $[M+1]^+$.

Step 5: Preparation of compound 43

43e (0.75 g, 0.65 mmol) was added to 8 mL of tetrahydrofuran and 40 mL of methanol. Zinc powder (3.42 g, 52.62 mmol) and ammonium chloride (1.04 g, 19.44 mmol) were sequentially added and the mixture was reacted at 27°C for 12 h. The reaction system was filtered over diatomaceous earth. The filtrate was concentrated under reduced pressure and the resulting crude was separated and purified by chromatographic column on silica gel (dichloromethane : methanol (v/v) = 10 : 1) to afford a crude (0.29 g). The above-mentioned crude (0.24 g) was added to 15 mL of DCM. Intermediate 1 (0.16 g, 0.28 mmol), triethylamine (0.076 g, 0.75 mmol) and HATU (0.14 g, 0.37 mmol) were sequentially added and the mixture was reacted at room temperature for 2 h. To the reaction solution was added 10 mL of water, followed by liquid separation. The aqueous phase was extracted with DCM (6 mL × 2) and the organic phase was dried over anhydrous sodium sulphate and concentrated under reduced pressure. The resulting crude was separated and purified by chromatographic column on silica gel (dichloromethane : methanol (v/v) = 50 : 1-10 : 1) to afford a crude, which was then passed through Pre-HPLC (instrument and preparative column: using SHIMADZU LC-20AP preparative liquid phase chromatographic instrument, preparative column model: Phenomenex C18). Mobile phase system: acetonitrile/water (containing 10 mmol/L $NH_4HCO_3$). Gradient elution method: gradient elution with acetonitrile from 50% to 80% (elution time: 15 min). Lyophilisation was performed to afford compound 43 (0.20 g, yield: 46%).

$^1H$ NMR (400 MHz, $CDCl_3$) δ 8.67 (s, 1H), 8.40 - 8.30 (m, 1H), 8.13 - 8.04 (m, 1H), 7.73 - 7.63 (m, 2H), 7.45 - 7.21 (m, 12H), 7.12 - 7.02 (m, 1H), 7.00 - 6.90 (m, 2H), 6.78 - 6.66 (m, 2H), 6.66 - 6.57 (m, 1H), 6.40 - 6.29 (m, 1H), 5.15 - 5.00 (m, 1H), 4.80 - 4.42 (m, 3H), 4.16 - 4.02 (m, 1H), 3.97 - 3.82 (m, 1H), 3.75 - 3.52 (m, 2H), 3.50 - 3.20 (m, 7H), 3.17 - 2.80 (m, 4H), 2.60 - 2.00 (m, 24H), 1.80 - 1.20 (m, 18H), 1.04 (s, 9H).

LCMS m/z = 514.8 $[M/3+1]^+$.

**Example 44:**

4-(4-((2-(4-chlorophenyl)cyclohept-1-en-1-yl)methyl)piperazin-1-yl)-N-((4-(((2R)-4-(4-((1-(2-(2,6-dioxopiperidin-3-yl)-1,3-dioxoisoindolin-5-yl)azetidin-3-yl)methyl)piperazin-1-yl)-1-(phenylthio)butan-2-yl)amino)-3-((trifluoromethyl)sulphonyl)phenyl)sulphonyl)benzamide **(compound 44)**

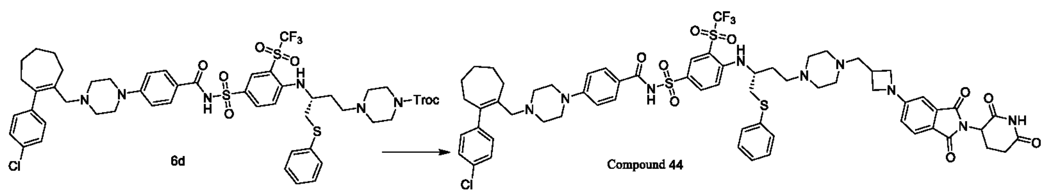


6d (3.00 g, 2.64 mmol) was added to 150 mL of methanol and 30 mL of tetrahydrofuran. Ammonium chloride (4.24 g, 79.27 mmol) and zinc powder (13.81 g, 212.46 mmol) were sequentially added and the mixture was reacted at 27°C for 19 h. The reaction system was filtered over diatomaceous earth. The filtrate was concentrated under reduced

pressure and the resulting crude was separated and purified by chromatographic column on silica gel (dichloromethane : methanol (v/v) = 10 : 1) to afford a crude (2.0 g). The above-mentioned crude (0.20 g) was added to 10 mL of dichloromethane. 1-(2-(2,6-dioxopiperidin-3-yl)-1,3-dioxoisoindolin-5-yl)azetidine-3-carbaldehyde (see CN114292270 for the synthetic method) (0.11 g, 0.32 mmol), acetic acid (0.05 g, 0.83 mmol) and sodium triacetoxyborohydride (0.13 g, 0.61 mmol) were sequentially added and the mixture was reacted at room temperature for 2 h. To the reaction solution was added 10 mL of saturated aqueous sodium bicarbonate solution, followed by liquid separation. The aqueous phase was extracted with DCM (6 mL × 2). The organic phases were combined, dried over anhydrous sodium sulphate and concentrated under reduced pressure and the crude was preliminarily separated and purified by chromatographic column on silica gel (dichloromethane : methanol (v/v) = 50 : 1-10 : 1). The resulting crude was passed through Pre-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude was dissolved with methanol and dimethyl sulphoxide, and filtered with a 0.45 μm filter membrane, to prepare into a sample liquid. Mobile phase system: acetonitrile/water (containing 0.1% TFA). Gradient elution method: gradient elution with acetonitrile from 5% to 60% (elution time: 15 min). The preparative solution was adjusted to pH 7 with saturated aqueous sodium bicarbonate solution and extracted with DCM (20 mL × 2). The organic phase was washed with 20 mL of saturated aqueous sodium chloride solution, dried over anhydrous sodium sulphate and concentrated under reduced pressure to afford compound 44 (68 mg, yield: 17%).

$^1$H NMR (400 MHz, $CDCl_3$) δ 8.33 (s, 1H), 8.18 - 8.01 (m, 1H), 7.84 - 7.45 (m, 3H), 7.40 - 7.15 (m, 7H), 7.03 - 6.90 (m, 3H), 6.80 - 6.55 (m, 4H), 6.50 - 6.35 (m, 1H), 4.95 - 4.80 (m, 1H), 4.20 - 4.00 (m, 2H), 3.98 - 3.79 (m, 1H), 3.76 - 3.55 (m, 2H), 3.30 - 2.90 (m, 6H), 2.85 - 2.57 (m, 6H), 2.55 - 2.25 (m, 18H), 2.15 - 1.95 (m, 3H), 1.90 - 1.75 (m, 3H), 1.67 - 1.45 (m, 5H).

LCMS m/z = 1284.3 $[M+1]^+$.

**Example 45:**

4-(4-((2-(4-chlorophenyl)cyclohept-1-en-1-yl)methyl)piperazin-1-yl)-N-((4-(((2R)-4-(4-((1-(2-(2,6-dioxopiperidin-3-yl)-1,3-dioxoisoindolin-5-yl)piperidin-4-yl)methyl)piperazin-1-yl)-1-(phenylthio)butan-2-yl)amino)-3-((trifluoromethyl)sulphonyl)phenyl)sulphonyl)benzamide **(compound 45)**

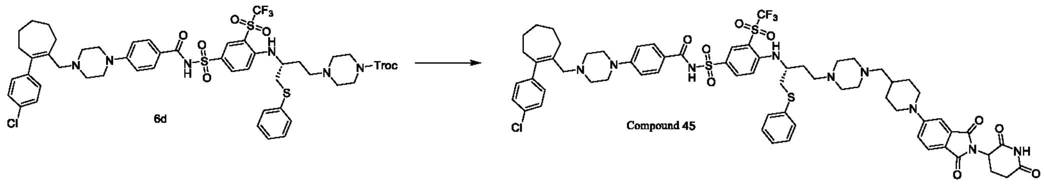

6d (3.00 g, 2.64 mmol) was added to 150 mL of methanol and 30 mL of tetrahydrofuran. Ammonium chloride (4.24 g, 79.27 mmol) and zinc powder (13.81 g, 212.46 mmol) were sequentially added and the mixture was reacted at 27°C for 19 h. The reaction system was filtered over diatomaceous earth. The filtrate was concentrated under reduced pressure and the resulting crude was separated and purified by chromatographic column on silica gel (dichloromethane : methanol (v/v) = 10 : 1) to afford a crude (2.0 g). The above-mentioned crude (0.20 g) was added to 10 mL of DCM. 1-(2-(2,6-dioxopiperidin-3-yl)-1,3-dioxoisoindolin-5-yl)piperidine-4-carbaldehyde (see CN 114292270 for the synthetic method) (0.12 g, 0.32 mmol), acetic acid (0.050 g, 0.83 mmol) and sodium triacetoxyborohydride (0.13 g, 0.61 mmol) were sequentially added and the mixture was reacted at room temperature for 2 h. To the reaction solution was added 10 mL of saturated aqueous sodium bicarbonate solution, followed by liquid separation. The aqueous phase was extracted with DCM (6 mL × 2). The organic phases were combined, dried over anhydrous sodium sulphate and concentrated under reduced pressure and the crude was preliminarily separated and purified by chromatographic column on silica gel (dichloromethane : methanol (v/v) = 50 : 1-10 : 1). The resulting crude was passed through Pre-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude was dissolved with methanol and dimethyl sulphoxide, and filtered with a 0.45 μm filter membrane, to prepare into a sample liquid. Mobile phase system: acetonitrile/water (containing 0.1% TFA). Gradient elution method: gradient elution with acetonitrile from 5% to 60% (elution time: 15 min). The preparative solution was adjusted to pH 7 with saturated aqueous sodium bicarbonate solution and extracted with DCM (20 mL × 2). The organic phase was washed with 20 mL of saturated aqueous sodium chloride solution, dried over anhydrous sodium sulphate and concentrated under reduced pressure to afford compound 45 (0.13 g, yield: 31%).

$^1$H NMR (400 MHz, $CDCl_3$) δ 8.31 (s, 1H), 8.16 - 7.98 (m, 1H), 7.82 - 7.55 (m, 3H), 7.40 - 7.15 (m, 8H), 7.05 - 6.83 (m, 4H), 6.80 - 6.50 (m, 3H), 4.98 - 4.84 (m, 1H), 4.00 - 3.75 (m, 3H), 3.30 - 2.60 (m, 13H), 2.60 - 2.15 (m, 20H), 2.15 - 1.95 (m, 3H), 1.92 - 1.75 (m, 4H), 1.68 - 1.46 (m, 7H).

LCMS m/z = 657.2 $[M/2+1]^+$.

**Example 46: Preparation of compound 46**

4-(4-((2-(4-chlorophenyl)cyclohept-1-en-1-yl)methyl)piperazin-1-yl)-N-((4-(((2R)-4-(4-((1-(4-(2,6-dioxopiperidin-3-yl)phenyl)azetidin-3-yl)methyl)piperazin-1-yl)-1-(phenylthio)butan-2-yl)amino)-3-((trifluoromethyl)sulphonyl)phenyl) sulphonyl)benzamide **(compound 46)**

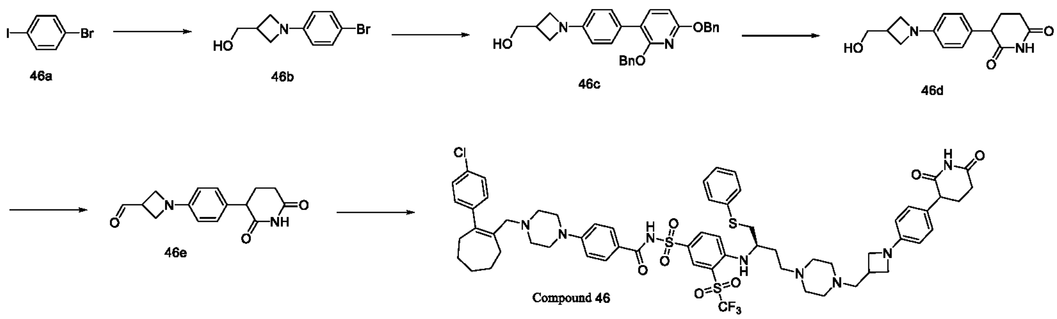

46a 46b 46c 46d 46e Compound 46

Step 1: Preparation of 46b

46a (15.00 g, 53.02 mmol) was added to 150 mL of DMSO and under nitrogen protection, (azetidin-3-yl)methanol hydrochloride (7.86 g, 63.60 mmol), L-proline (2.44 g, 21.19 mmol), potassium carbonate (21.98 g, 159.04 mmol) and CuI (2.02 g, 10.61 mmol) were added and the system was subjected to nitrogen replacement three times and reacted at 100°C for 16 h. The reaction system was cooled to room temperature and 100 mL of water was added. The mixture was extracted with ethyl acetate (60 mL × 3). The organic phase was washed with 50 mL of saturated aqueous sodium chloride solution, dried over anhydrous sodium sulphate and concentrated under reduced pressure and the resulting crude was separated and purified by chromatographic column on silica gel (petroleum ether : ethyl acetate (v/v) = 20 : 1-5 : 1) to afford 46b (5.60 g, yield: 44%).

LCMS m/z = 242.1 $[M+1]^+$.

Step 2: Preparation of 46c

46b (5.10 g, 21.15 mmol) was added to 60 mL of 1,4-dioxane and 15 mL of water. Under nitrogen protection, 2,6-bis(benzyloxy)-3-(4,4,5,5-tetramethyl-1,3,2-dioxaborolan-2-yl)pyridine (26.37 g, 63.19 mmol) (see WO 2021262812 for the synthetic method), caesium carbonate (20.59 g, 63.19 mmol) and $Pd(dppf)Cl_2$ (0.77 g, 1.05 mmol) were added and the system was subjected to nitrogen replacement three times and reacted at 100°C for 16 h. The reaction system was cooled to room temperature, filtered over diatomaceous earth, and washed with 50 mL of ethyl acetate. To the filtrate was added 50 mL of water and the mixture was extracted with ethyl acetate (50 mL × 3). The organic phase was washed with 50 mL of saturated aqueous sodium chloride solution, dried over anhydrous sodium sulphate and concentrated under reduced pressure and the resulting crude was separated and purified by chromatographic column on silica gel (petroleum ether : ethyl acetate (v/v) = 20 : 1-5 : 1) to afford 46c (9.10 g, yield: 95%).

LCMS m/z = 453.3 $[M+1]^+$.

Step 3: Preparation of 46d

46c (9.10 g, 20.12 mmol) was added to 100 mL of methanol. 10% Pd/C (5.00 g) was added and the system was subjected to nitrogen replacement three times and reacted at room temperature under the atmosphere of hydrogen balloon for 16 h. The reaction system was filtered over diatomaceous earth and then washed with mixed solvents of dichloromethane and methanol (v/v) = 10 : 1. The filtrate was concentrated under reduced pressure and to the resulting crude were added 40 mL of ethyl acetate and 40 mL of petroleum ether. The mixture was stirred at room temperature for 0.5 h and then filtered. The filter cake was dried under reduced pressure to afford crude 46d (3.10 g).

LCMS m/z = 275.2 $[M+1]^+$.

Step 4: Preparation of 46e

The above-mentioned crude 46d (0.20 g) was added to 5 mL of DMSO. 2-iodoxybenzoic acid (0.33 g, 1.18 mmol) was added and the mixture was reacted at 50°C for 1 h. The reaction system was cooled to room temperature and 10 mL of ethyl acetate and 10 mL of water were added, followed by liquid separation. The aqueous phase was extracted with ethyl acetate (5 mL × 2). The organic phases were combined, washed with 10 mL of saturated aqueous sodium chloride solution, dried over anhydrous sodium sulphate and concentrated under reduced pressure to afford crude 46e (0.15 g).

Step 5: Preparation of compound 46

6d (3.00 g, 2.64 mmol) was added to 150 mL of methanol and 30 mL of tetrahydrofuran. Ammonium chloride (4.24 g, 79.27 mmol) and zinc powder (13.81 g, 212.46 mmol) were sequentially added and the mixture was reacted at 27°C for 19 h. The reaction system was filtered over diatomaceous earth. The filtrate was concentrated under reduced pressure and the resulting crude was separated and purified by chromatographic column on silica gel (dichloromethane : methanol (v/v) = 10 : 1) to afford a crude (2.0 g). The above-mentioned crude (0.15 g) was added to 10 mL of dichloromethane. Crude 46e (0.15 g), acetic acid (0.038 g, 0.63 mmol) and sodium triacetoxyborohydride (0.10 g, 0.472 mmol) were sequentially added and the mixture was reacted at room temperature for 2 h. To the reaction solution was added 10 mL of saturated aqueous sodium bicarbonate solution, followed by liquid separation. The aqueous phase was extracted with dichloromethane (6 mL × 2). The organic phases were combined, dried over anhydrous sodium sulphate and concentrated under reduced pressure and the resulting crude was separated and purified by chromatographic column on silica gel (dichloromethane : methanol (v/v) = 50 : 1-10 : 1) to afford a crude, which was then separated and purified by preparative liquid phase chromatography to afford compound 46 (0.13 g, yield over three steps from compound 46c: 8%).

$^1$H NMR (400 MHz, $CDCl_3$) δ 8.40 - 8.29 (m, 1H), 8.10 (dd, 1H), 7.93 (s, 1H), 7.69 - 7.60 (m, 2H), 7.41 - 7.20 (m, 7H), 7.10 - 6.92 (m, 5H), 6.84 - 6.72 (m, 2H), 6.63 (d, 1H), 6.46 - 6.37 (m, 2H), 4.05 - 3.83 (m, 3H), 3.74 - 3.65 (m, 1H), 3.57 - 3.47 (m, 2H), 3.32 - 3.22 (m, 4H), 3.14 - 2.89 (m, 3H), 2.84 (s, 2H), 2.77 - 2.03 (m, 25H), 1.88 - 1.47 (m, 7H).

LCMS m/z = 608.3 $[M/2+1]^+$.

**Example 47: Preparation of compound 47**

4-(4-((2-(4-chlorophenyl)cyclohept-1-en-1-yl)methyl)piperazin-1-yl)-N-((4-(((2R)-4-(4-(2-(2,6-dioxopiperidin-3-yl)-1,3-dioxoisoindolin-5-yl)piperazin-1-yl)-1-(phenylthio)butan-2-yl)amino)-3-((trifluoromethyl)sulphonyl)phenyl)sulphonyl) benzamide **(compound 47)**

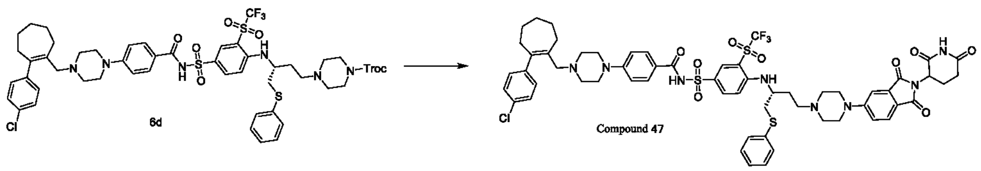

6d (3.00 g, 2.64 mmol) was added to 150 mL of methanol and 30 mL of tetrahydrofuran. Ammonium chloride (4.24 g, 79.27 mmol) and zinc powder (13.81 g, 212.46 mmol) were sequentially added and the mixture was reacted at 27°C for 19 h. The reaction system was filtered over diatomaceous earth. The filtrate was concentrated under reduced pressure and the resulting crude was separated and purified by chromatographic column on silica gel (dichloromethane : methanol (v/v) = 10 : 1) to afford a crude (2.0 g). The above-mentioned crude (0.10 g) was added to 5 mL of dimethyl sulphoxide. 2-(2,6-dioxopiperidin-3-yl)-5-fluoroisoindoline-1,3-dione (0.083 g, 0.30 mmol) and N,N-diisopropylethylamine (0.065 g, 0.50 mmol) were sequentially added and the mixture was reacted at 100°C for 16 h. The reaction system was cooled to room temperature and 10 mL of water and 10 mL of ethyl acetate were added, followed by liquid separation. The aqueous phase was extracted with ethyl acetate (6 mL × 2). The organic phases were combined, dried over anhydrous sodium sulphate and concentrated under reduced pressure and the resulting crude was separated and purified by chromatographic column on silica gel (dichloromethane : methanol (v/v) = 50 : 1-10 : 1) to afford a crude, which was then separated and purified by preparative liquid phase chromatography to afford compound 47 (20 mg, yield: 5%).

$^1$H NMR (400 MHz, $CDCl_3$) δ 8.40 - 8.35 (m, 1H), 8.13 (dd, 1H), 8.03 - 7.92 (m, 1H), 7.69 (d, 1H), 7.65 - 7.57

(m, 2H), 7.44 - 7.20 (m, 8H), 7.17 - 7.09 (m, 1H), 7.06 - 6.94 (m, 3H), 6.83 - 6.74 (m, 2H), 6.62 (d, 1H), 4.99 - 4.88 (m, 1H), 4.00 - 3.87 (m, 1H), 3.49 - 3.20 (m, 8H), 3.18 - 2.97 (m, 2H), 2.96 - 2.65 (m, 5H), 2.65 - 2.07 (m, 16H), 1.89 - 1.40 (m, 7H).

LCMS m/z = 608.8 $[M/2+1]^+$.

**Example 48: Preparation of compound 48**

(3R,5 S)-1-((S)-2-(7-(4-((R)-3-((4-(N-(4-(4-((2-(4-chlorophenyl)cyclohept-1-en-1-yl)methyl)piperazin-1-yl)benzoyl)sulphamoyl)-2-((trifluoromethyl)sulphonyl) phenyl)amino)-4-(phenylthio)butyl)piperazin-1-yl)-7-oxoheptanamido)-3,3-dimethylbutanoyl)-5-(((S)-1-(4-(4-methylthiazol-5-yl)phenyl)ethyl)carbamoyl) pyrrolidin-3-yl acetate **(compound 48)**

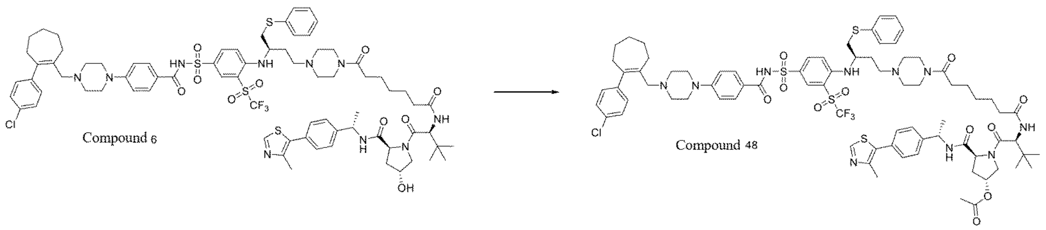

Free-form compound 6 (50 mg, 0.033 mmol) was dissolved in 3 mL of dichloromethane. 4-dimethylaminopyridine (8.1 mg, 0.066 mmol) and acetic anhydride (33 mg, 0.32 mmol) were added and the mixture was reacted at room temperature for 2 h. The reaction solution was concentrated under reduced pressure and the crude product was subjected to Pre-HPLC (instrument and preparative column: using SHIMADZU LC-20AP preparative liquid phase chromatographic instrument, preparative column model: Phenomenex C18). Preparation method: the crude was dissolved with methanol and dimethyl sulphoxide, and filtered with a 0.45 μm filter membrane, to prepare into a sample liquid. Mobile phase system: acetonitrile/water (containing 0.05% $NH_4$ $HCO_3$). Gradient elution method: gradient elution with acetonitrile from 60% to 90% (elution time: 15 min). Lyophilisation was performed to afford compound 48 (30 mg, yield: 58%).

$^1H$ NMR (400 MHz, $CDCl_3$) δ 8.68 (s, 1H), 8.40 - 8.32 (m, 1H), 8.16 - 8.06 (s, 1H), 7.69 - 7.58 (m, 2H), 7.46 - 7.20 (m, 12H), 7.17 - 7.08 (m, 1H), 7.02 - 6.93 (m, 2H), 6.84 - 6.71 (m, 2H), 6.64 - 6.54 (m, 1H), 6.18 - 6.08 (m, 1H), 5.43 - 5.27 (m, 1H), 5.15 - 4.97 (m, 1H), 4.77 - 4.67 (m, 1H), 4.62 - 4.54 (m, 1H), 4.13 - 4.00 (m, 1H), 3.97 - 3.56 (m, 3H), 3.54 - 3.17 (m, 7H), 3.16 - 2.95 (m, 2H), 2.94 - 2.65 (m, 3H), 2.55 - 1.97 (m, 26H), 1.90 - 1.30 (m, 16H), 1.02 (s, 9H).

LCMS m/z = 785.5 $[M/2+1]^+$.

## Experimental examples

### 1. BCL-xL and BCL-2 degradation activity studies in MOLT-4 cells

MOLT-4 cells are acute lymphoblastic leukaemia cell lines in humans. The cells were purchased from ATCC. Culture conditions: RPMI-1640 + 10% FBS + 1% penicillin-streptomycin solution. The cells were cultured in a 37°C, 5% $CO_2$ incubator. The cells were plated in a 12-well plate, with $2 \times 10^5$ cells/well. After plating, compounds at different concentrations were added and cultured in a 37°C, 5% $CO_2$ incubator for 16 h. After the completion of culture, the cells were harvested and RIPA lysis buffer (beyotime, Cat. P0013B) was added. The cells were lysed on ice for 15 minutes and centrifuged at 12000 rpm at 4°C for 10 minutes. The supernatant protein samples were collected and the protein was quantified by using the BCA kit (Beyotime, Cat. P0009). Then the protein was diluted to 0.8 mg/mL. The expressions of BCL-xL (CST, Cat.2764S), BCL-2 (CST, Cat.15071S) and the internal reference β-actin (CST, Cat. 3700S) were detected using a fully automated western blot quantitative analyser (Proteinsimple). The expression level of BCL-xL or BCL-2 relative to the internal reference was calculated by using Compass software, and the $DC_{50}$ value was calculated by using GraphPad Prism 8.0 software according to formula (1), wherein the $Protein_{administration}$ denoted the relative expression level of BCL-xL or BCL-2 in administration groups at different doses, and the $Protein_{vehicle}$ denoted the relative expression level of BCL-xL or BCL-2 in the vehicle control group.

$$Protein\% = Protein_{administration}/Protein_{vehicle} \times 100\% \quad \text{formula (1)}$$

The results of $DC_{50}$ values for the degradation of BCL-xL proteins in MOLT-4 cells are shown in Table 1.

Table 1 $DC_{50}$ values of the compounds according to the present invention for the degradation of BCL-xL proteins

| Serial No. | Compound No. | $DC_{50}$ (nM) |
|---|---|---|
| 1 | Control compound | >20 |
| 2 | Compound 6 | 8.4 |
| 3 | Compound 8 | 8.8 |
| 5 | Compound 10 | 3.7 |
| 6 | Compound 11 | 5.3 |
| 9 | Compound 14 | 8.1 |
| 10 | Compound 15 | 3.6 |
| 12 | Compound 19 | 10.7 |

Conclusion: the compounds of the present invention had a given degradation effect on the BCL-xL proteins in MOLT-4 cells.

Structure of the control compound (DT2216):

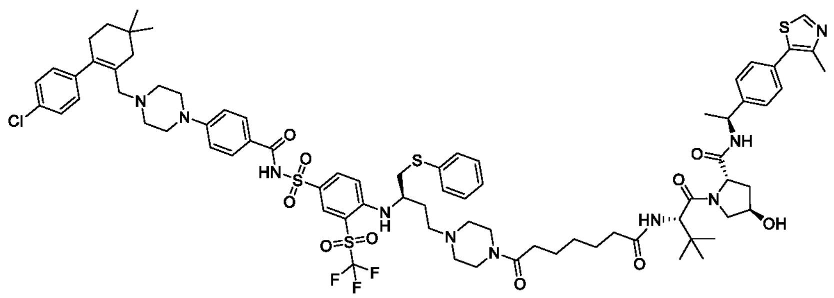

**2. Cell proliferation activity studies in MOLT-4 cells**

MOLT-4 cells are acute lymphoblastic leukaemia cell lines in humans. The cells were purchased from ATCC. Culture conditions: RPMI-1640 + 10% FBS + 1% penicillin-streptomycin solution. The cells were cultured in a 37°C, 5% $CO_2$ incubator. The cells were plated in a 96-well plate, with $5 \times 10^3$ cells/well. After plating, compounds at different concentrations were added and cultured in a 37°C, 5% $CO_2$ incubator for 72 h. After the completion of culture, a reagent (Promega, G7573) for detecting the cell viability was added. The mixture was uniformly mixed for 2 minutes and then incubated at room temperature for 10 minutes. The luminescence signal was detected with a multimode plate reader (BMG, PHERAstar FSX). The luminescence readings were processed using GraphPad Prism 8.0 software and the $IC_{50}$ values of the compounds for the inhibition of cell proliferation and the maximum inhibition rate were calculated according to formula (2) and formula (3), respectively, wherein $T_{administration}$ denotes the cell signal readings obtained after incubation with the compounds for 72 h, and $T_{vehicle}$ denotes the cell signal readings obtained after incubation with the vehicle control for 72 h.

$$Growth\% = T_{administration}/T_{vehicle} \times 100\% \quad \text{formula (2)}$$

Calculation of Max inhibition%: after the processing according to formula (2), the inhibition rate of the compounds at the highest concentration was calculated.

$$\text{Max inhi.}\% = 1 - Growth\% \quad \text{formula (3)}$$

The results of $IC_{50}$ values for the inhibition of MOLT-4 cell proliferation are shown in Table 2.

Table 2 IC50 values of the compounds according to the present invention for the inhibition of MOLT-4 cell proliferation

| Serial No. | Compound No. | $IC_{50}$ (nM) |
|---|---|---|
| 1 | Compound 2 | A |
| 2 | Trifluoroacetate of compound 4 | A |
| 3 | Compound 5 | A |
| 4 | Trifluoroacetate of compound 6 | A |
| 5 | Compound 7 | A |
| 6 | Compound 8 | A |
| 7 | Trifluoroacetate of compound 9 | A |
| 8 | Compound 10 | A |
| 9 | Compound 11 | A |
| 10 | Compound 12 | A |
| 11 | Compound 13 | A |
| 12 | Compound 14 | A |
| 13 | Compound 15 | A |
| 14 | Compound 16 | A |
| 15 | Compound 17 | A |
| 16 | Compound 18 | A |
| 17 | Compound 19 | A |
| 18 | Compound 20 | A |
| 19 | Compound 21 | A |
| 20 | Compound 22 | A |
| 21 | Compound 23 | A |
| 22 | Compound 24 | A |
| 23 | Compound 25 | A |
| 24 | Compound 26 | A |
| 25 | Compound 27 | A |
| 26 | Compound 28 | A |
| 27 | Compound 29 | A |
| 28 | Compound 30 | A |
| 29 | Compound 31 | A |
| 30 | Compound 32 | A |
| 31 | Compound 33 | A |
| 32 | Compound 34 | A |
| 33 | Compound 35 | A |
| 34 | Compound 36 | A |
| 35 | Compound 37 | A |
| 36 | Compound 38 | A |
| 37 | Compound 39 | A |

(continued)

| Serial No. | Compound No. | $IC_{50}$ (nM) |
|---|---|---|
| 38 | Compound 40 | A |
| 39 | Compound 41 | A |
| 40 | Compound 42 | A |
| 41 | Compound 43 | A |
| 42 | Compound 44 | A |
| 43 | Compound 45 | A |
| A < 500 nM; 500 nM ≤ B ≤ 5000 nM; C > 5000 nM. | | |

Conclusion: the compounds according to the present invention had a given inhibitory effect on the proliferation of MOLT-4 cells.

**3. Growth inhibition experiment in MOLT-4 xenograft tumour models in nude mice**

Cell inoculation and animal administration:

MOLT-4 cells are acute lymphoblastic leukaemia cell lines in humans. The cells were purchased from ATCC and cultured in RPMI-1640 + 10% FBS + 1% penicillin-streptomycin solution in a 37°C, 5% $CO_2$ incubator. When the cells were in the exponential growth phase, the cells were harvested and counted. An equal volume of matrigel was added. Female SCID Beige mice (4-6 weeks old, 14-16 g, Beijing Vital River Laboratory Animal Technology Co., Ltd.) were inoculated subcutaneously in the right flank with the cells (200 μL, containing $1 \times 10^7$ MOLT-4 cells and 50% matrigel). When the tumour volume reached 150-200 $mm^3$, the mice were randomly grouped for administration according to the tumour volume and body weight of the mice. Three administration dosages were provided: 5 mpk, 15 mpk and 50 mpk. The compounds were injected intraperitoneally (ip) weekly (qw). The mice in all the experimental groups (10 mice in each group) were continuously administered for 4 weeks.

Experiment observation and endpoint:

After inoculation, the body weights of the mice were measured three times a week and the long (a) and short (b) diameters of the tumours were measured. The tumour volume was calculated according to the formula: $V = ab^2/2$. At day 28 after the administration, the experiment was terminated.

Experimental results: in MOLT-4 xenograft tumour models, compound 6 had significant tumour-suppressing efficacy on MOLT-4 xenograft tumours in mice relative to the vehicle control group, and with the increase of the administration dosage, the tumour-suppressing effects grew stronger. See Figure 1.

**4. Pharmacokinetic test in rats**

**Experimental objective:** in this test, a single dose of test compounds was administered to SD rats intragastrically, and the concentrations of the test compounds in plasma of the rats were measured to evaluate the pharmacokinetic characteristics of the test compounds in the rats.

**Experimental animals:** male SD rats, 200-250 g, 6-8 weeks old, 3 rats/compound, purchased from Chengdu Ddossy Experimental Animals Co., Ltd.

**Experimental method:** on the day of the test, 3 SD rats were randomly grouped according to their body weight. The animals were fasted with water available for 12 to 14 h one day before the administration of a test compound, and were fed 4 hours after the administration.

Table 3

| Group | Quantity | Administration information | | | | | | |
|---|---|---|---|---|---|---|---|---|
| | Male | Test compound | Administrati on dosage* (mg/kg) | Administrati on concentration (mg/mL) | Administrati on volume (mL/kg) | Collected samples | Mode of administrati on | Vehicle |
| G1 | 3 | Compound according to the present invention | 5 | 0.5 | 10 | Plasma | Oral (intragastrically) | 5% DMSO+5 % Solutol+30% PEG400+ 60% (20% SBE-CD) |

*Dosage is calculated based on free base.

**Sampling:** before and after the administration, 0.1 mL of blood samples were drawn from the orbits of the animals under isoflurane anaesthesia, and placed in an EDTAK2 centrifuge tube. Centrifugation was carried out at 5000 rpm at 4°C for 10 min, and the plasma was collected.

Time points for plasma collection in PO group: 0, 5 min, 15 min, 30 min, 1 h, 2 h, 4 h, 6 h, 8 h, and 24 h.

Before analysis and detection, all samples were stored at -60°C. The samples were analysed quantitatively by LC-MS/MS.

Conclusion: the compound of the present invention had good oral absorption in rats.

**5. Pharmacokinetic test in mice**

**Experimental objective:** in this test, a single dose of test compounds was administered to C57 mice intragastrically, and the concentrations of the test compounds in plasma of the mice were measured to evaluate the pharmacokinetic characteristics of the test compounds in the mice.

**Experimental animals:** male C57 mice, 20-25 g, 6-8 weeks old, 3 mice/compound. purchased from Chengdu Ddossy Experimental Animals Co., Ltd.

**Experimental method:** on the day of the test, 3 C57 mice were randomly grouped according to their body weight. The animals were fasted with water available for 12 to 14 h one day before the administration of a test compound, and were fed 4 hours after the administration.

Table 4

| Group | Quantity | Administration information | | | | | | |
|---|---|---|---|---|---|---|---|---|
| | Male | Test compound | Administrati on dosage. (mg/kg) | Administration concentration (mg/mL) | Administrati on volume (mL/kg) | Collected sample | Mode of administra tion | Vehicle |
| G1 | 3 | Compound according to the present invention | 5 | 0.5 | 10 | Plasma | Oral (intragastr ically) | 5% DMSO+5% Solutol+30 % PEG400+60 % (20% SBE-β-CD) |
| *Dosage is calculated based on free base. | | | | | | | | |

**Sampling:** before and after the administration, 0.03 mL of blood samples were drawn from the orbits of the animals under isoflurane anaesthesia, and placed in an EDTAK2 centrifuge tube. Centrifugation was carried out at 5000 rpm at 4°C for 10 min, and the plasma was collected.

Time points for plasma collection in PO group: 0, 5 min, 15 min, 30 min, 1 h, 2 h, 4 h, 7 h, and 24 h.

Before analysis and detection, all samples were stored at -60°C. The samples were analysed quantitatively by LC-MS/MS.

Conclusion: the compound of the present invention had good oral absorption in mice.

**6. Pharmacokinetic test in monkeys**

**Experimental animals:** male cynomolgus monkeys, 3-5 kg, 3-6 years old, 2 monkeys/compound. Purchased from Suzhou Xishan Biotechnology Inc.

**Experimental method:** on the day of the test, 2 monkeys were randomly grouped according to their body weights. The animals were fasted with water available for 14 to 18 h one day before the administration of a test compound, and were fed 4 hours after the administration.

**Table 5. Administration information**

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| .G1 | 2 | Compound according to the present invention | 2 | 2 | 1 | Plasma | Intravenously |
| Notes: vehicle for intravenous administration: 5% DMSO+95% (3% Tween 80 in PBS);<br>*Dosage is calculated based on free base. | | | | | | | |

Before and after the administration, 1.0 mL of blood samples were drawn from the limb veins and placed in an EDTAK2 centrifuge tube. Centrifugation was carried out at 5000 rpm at 4°C for 10 min, and the plasma was collected. Blood collection time points for the intravenous group were: 0, 5 min, 15 min, 30 min, 1 h, 2 h, 4 h, 6 h, 8 h, 10 h, 12 h, 24 h, and 48 h. Before analysis and detection, all samples were stored at -80°C, and a quantitative analysis of samples was performed using LC-MS/MS.

**Table 6. Pharmacokinetic parameters of the compound according to the present invention in plasma of monkeys**

| Test compound | Mode of administration | $AUC_{0-t}$ (hr*ng/mL) | $T_{1/2}$ (h) |
|---|---|---|---|
| Compound 6 | i.v. (2 mg/kg) | 54628 ± 5909 | 9.83 ± 2.2 |

Conclusion: the compound according to the present invention exhibited good pharmacokinetic characteristics in monkeys after intravenous administration.

**7. Liver microsomal stability test**

In this experiment, liver microsomes of five species, including human, monkey, dog, rat, and mouse, were used as *in vitro* models to evaluate the metabolic stability of the test compound.
At 37°C, 1 μM of the test compound was co-incubated with microsomal protein and coenzyme NADPH. At given time points of the reaction (5 min, 10 min, 20 min, 30 min, and 60 min), the reaction was terminated by adding ice-cold acetonitrile containing an internal standard. The LC-MS/MS method was used to measure the concentration of the test compound in the sample. $T_{1/2}$ was calculated using the natural logarithm (ln) of the residual rate of the drug in the incubation system and the incubation time. In addition, the intrinsic clearance in liver microsomes $CL_{int(mic)}$ and the intrinsic clearance in liver $CL_{int(Liver)}$ were further calculated.
Conclusion: the compound according to the present invention had good stability in liver microsomes.

**8. CYP450 enzyme inhibition test**

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the completion of the reaction, the sample was treated and liquid chromatography-tandem mass spectrometry (LC-MS/MS) was used to quantitatively detect metabolites produced by specific substrates, changes in CYP enzyme activity were determined, and $IC_{50}$ value was calculated to evaluate the inhibitory potential of the test compound on each CYP enzyme subtype.
Conclusion: the compound according to the present invention did not exhibit significant inhibitory effects on the five isoforms of human liver microsomal cytochrome P450.

**9. Test for hERG potassium ion channel**

**Experimental platform:** electrophysiological manual patch-clamp system
**Cell line:** Chinese hamster ovary (CHO) cell lines stably expressing hERG potassium ion channel
**Experimental method:** in CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.
**Data processing:** data analysis processing was carried out by using pClamp 10, GraphPad Prism 5 and Excel software. The inhibition degree of hERG potassium current (peak value of hERG tail current induced at -50 mV) at

different compound concentrations was calculated by the following formula:

$$\text{Inhibition\%} = [1 - (I / I\text{o})] \times 100\%$$

wherein, Inhibition% represents the percentage of inhibition of hERG potassium current by the compound, and I and Io represent the amplitude of hERG potassium current after and before the administration, respectively.

Conclusion: the compound according to the present invention did not exhibit significant inhibitory activity on the hERG potassium current.

**10. Effect on platelet in mice after single intravenous injection**

Experimental animal: 78 SD rats, with an equal number of males and females, male (♂): 290-380 g, female (♀): 220-290 g, 8-10 weeks old, purchased from Zhejiang Vital River Laboratory Animal Technology Co., Ltd.

Experimental protocol: a clear solution was formulated using a vehicle (5% DMSO + 95% (3% Tween 80 in PBS)). The animals were dosed via tail vein a single time (administration volume: 20 mL/kg; bolus injection period: about 4 minutes; recovery period: 7 days).

| Group | Test compound | Administration dosage (mg/kg) | Administration volume (mL/kg) | Concentration (mg/mL) | Number of animal | |
|---|---|---|---|---|---|---|
| | | | | | Toxicology | Toxicokinetics |
| 1 | 5% DMSO+95% (3% Tween 80 in PBS) | 0 | 20 | 0 | 3♀/3♂ | - |
| 2 | Control compound | 6 | 20 | 0.3 | 3♀/3♂ | 3♀/3♂ |
| 3 | | 20 | 20 | 1 | 3♀/3♂ | 3♀/3♂ |
| 4 | | 60 | 20 | 3 | 3♀/3♂ | 3♀/3♂ |
| 5 | Compound 6 | 6 | 20 | 0.3 | 3♀/3♂ | 3♀/3♂ |
| 6 | | 20 | 20 | 1 | 3♀/3♂ | 3♀/3♂ |
| 7 | | 60 | 20 | 3 | 3♀/3♂ | 3♀/3♂ |

The experimental results are shown in Table 7 and Table 8:

Table 7 Effect of compound 6 and control compound on PLT in SD rats after single intravenous injection

| Detection Time point | Compound 6 (♂) | | Control compound (♂) | | Compound 6 (♀) | | Control compound (♀) | |
|---|---|---|---|---|---|---|---|---|
| | 6 mg/kg | 20 mg/kg | 6 mg/kg | 20 mg/kg | 6 mg/kg | 20 mg/kg | 6 mg/kg | 20 mg/kg |
| 4 h | ↓16% | ↓51% | ↓26% | ↓81% | -- | ↓61% | ↓24% | ↓89% |
| D2 | ↓56% | ↓71% | ↓69% | ↓91% | ↓60% | ↓84% | ↓78% | ↓96% |
| -- represents that no significant change was observed | | | | | | | | |

Table 8 Effect of compound 6 and control compound on PLT in SD rats after single intravenous injection

| Detection time point | Compound 6 (♂) | Compound 6 (♀) | Control compound (♂) | Control compound (♀) |
|---|---|---|---|---|
| Administration dosage | 60 mg/kg | 60 mg/kg | 60 mg/kg | 60 mg/kg |
| D8 | ↓17% | ↓53% | ↓73% | ↓87% |
| D2: day 2; D8: day 8 | | | | |

Conclusion: at 4 h and D2 after the administration via single intravenous injection, compound 6 exhibited less

platelet toxicity in rats at doses of 6 mg/kg and 20 mg/kg relative to the control compound. At D8, compound 6 exhibited less platelet toxicity in rats at the dose of 60 mg/kg relative to the control compound.

## Claims

1. A compound or a stereoisomer, deuterated compound, solvate, prodrug, metabolite, pharmaceutically acceptable salt, or co-crystal thereof, **characterised in that** the compound is selected from a compound of general formula (I),

B-L-K (I);

L is selected from a bond or $-C_{1-50}$ hydrocarbyl-, wherein the hydrocarbyl has 0 to 20 methylene units optionally further replaced by -Ak- or -Cy-;

each -Ak- is independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)_q-NR^LC(=O)-$, $-NR^L(CH_2)_qC(=O)-$, $-(CH_2)_q-C(=O)NR^L-$, -C(=O)-, $-C(=O)-(CH_2)_q-NR^L-$, $-(C\equiv C)_q-$, -CH=CH-, $-Si(R^L)_2-$, $-Si(OH)(R^L)-$, $-Si(OH)_2-$, $-P(=O)(OR^L)-$, $-P(=O)(R^L)-$, -S-, -S(=O)-, $-S(=O)_2-$ or a bond, wherein the $-CH_2-$ is optionally further substituted with 0 to 2 substituents selected from H, halogen, OH, CN, $NH_2$, $C_{1-6}$ alkyl, $C_{1-6}$ alkoxy, halogen-substituted $C_{1-6}$ alkyl, hydroxyl-substituted $C_{1-6}$ alkyl, or cyano-substituted $C_{1-6}$ alkyl;

each q is independently selected from 0, 1, 2, 3, 4, 5 or 6;

each $R^L$ is independently selected from H, $C_{1-6}$ alkyl, 3- to 7-membered heterocyclyl, 3- to 7-membered cycloalkyl, phenyl or 5- to 6-membered heteroaryl;

each -Cy- is independently selected from a bond, a 4- to 8-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally further substituted with 0, 1 or 2 =O when the heteroatom is selected from S;

B is selected from

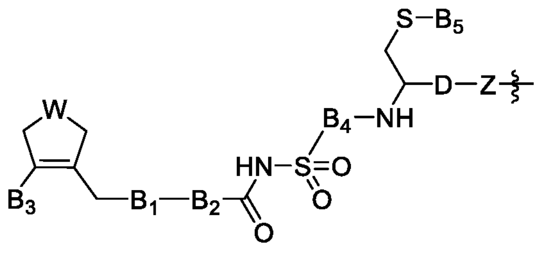

;

W is selected from $W_1$ or $W_2$;

$W_1$ is selected from $-CR^{w1}R^{w2}-$, $-(CR^{w1}R^{w2})_3-$, $-(CR^{w1}R^{w2})_4-$, $-CH_2CR^{w3}R^{w4}-$, $-CR^{w3}R^{w4}CH_2-$, $-CR^{w1}R^{w2}O-$, $-OCR^{w1}R^{w2}-$, $-CR^{w1}R^{w2}NR^{w5}-$, or $-NR^{w5}CR^{w1}R^{w2}-$;

$W_2$ is selected from $-(CR^{w1}R^{w2})_2-$;

D is selected from $C_{1-4}$ alkylene;

$B_1$ and Z are each independently selected from a 4- to 7-membered mono-heterocyclic ring, a 5- to 12-membered fused-heterocyclic ring, a 6- to 12-membered spiro-heterocyclic ring, or a 7- to 12-membered bridged-heterocyclic ring, the $B_1$ is optionally further substituted with 0 to 4 $R^{B1}$, and the Z is optionally further substituted with 0 to 4 $R^Q$, wherein the fused-heterocyclic ring, spiro-heterocyclic ring, or bridged-heterocyclic ring contains 1 to 3 heteroatoms selected from O, S or N, and is optionally further substituted with 0, 1 or 2 =O when the heteroatom is selected from S;

$B_2$, $B_3$, $B_4$, and $B_5$ are each independently selected from $C_{6-10}$ aryl or 5- to 10-membered heteroaryl, the $B_2$ is optionally further substituted with 0 to 4 $R^{B2}$, the $B_3$ is optionally further substituted with 0 to 5 $R^{B3}$, the $B_4$ is optionally further substituted with 0 to 4 $R^{B4}$, and the $B_5$ is optionally further substituted with 0 to 5 $R^{B5}$, wherein

the heteroaryl contains 1 to 3 heteroatoms selected from O, S or N;
$R^{B1}$, $R^{Q}$, $R^{B2}$, $R^{B3}$, and $R^{B5}$ are each independently selected from halogen, OH, oxo, CN, $C_{1-4}$ alkyl or $C_{1-4}$ alkoxy, wherein the alkyl or alkoxy is optionally further substituted with 0 to 4 substituents selected from H, halogen, OH, CN or $C_{1-4}$ alkyl;
each $R^{B4}$ is independently selected from $-SO_2-C_{1-4}$ alkyl, nitro, halogen, CN, OH, $C_{1-4}$ alkyl or $C_{1-4}$ alkoxy, wherein the alkyl or alkoxy is optionally further substituted with 0 to 4 substituents selected from H, halogen, OH, CN or $C_{1-4}$ alkyl;
$R^{w1}$, $R^{w2}$ and $R^{w5}$ are each independently selected from H, halogen, CN, OH, $C_{1-4}$ alkyl or $C_{1-4}$ alkoxy, wherein the alkyl or alkoxy is optionally further substituted with 0 to 4 substituents selected from H, halogen, OH, CN or $C_{1-4}$ alkyl;
alternatively, $R^{w1}$ and $R^{w2}$ are directly connected to form $C_{3-6}$ carbocycle or 3-to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;
$R^{w3}$ and $R^{w4}$ are directly connected to form $C_{3-6}$ carbocycle or 3- to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;
alternatively, $R^{B1}$ and $R^{B2}$ are directly connected to form $C_{5-7}$ carbocycle or 5-to 7-membered heterocycle, wherein the carbocycle or heterocycle is optionally further substituted with 0 to 3 $R^c$, and the heterocycle contains 1 to 3 heteroatoms selected from O, S or N;
each $R^c$ is independently selected from halogen, OH, CN, =O, $C_{1-4}$ alkyl, $C_{1-4}$ alkoxy, $C_{3-6}$ carbocycle or 3- to 7-membered heterocycle, wherein the alkyl, alkoxy, carbocycle or heterocycle is optionally further substituted with 0 to 4 substituents selected from H, halogen, OH, CN, $C_{1-4}$ alkyl, $C_{1-4}$ alkoxy, $C_{3-6}$ cycloalkyl or 3- to 7-membered heterocycloalkyl, and the heterocycle or heterocycloalkyl contains 1 to 3 heteroatoms selected from O, S or N,
provided that when $W_2$ is selected from $-(CR^{w1}R^{w2})_2-$, and $R^{w1}$ and $R^{w2}$ are each independently selected from F, methyl or methoxy, B at least satisfies any one of the following conditions:

1) $B_1$ is not piperazine;
2) Z is not piperazine, piperidine,

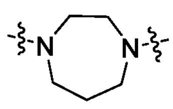 , 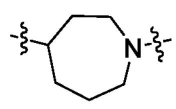 , 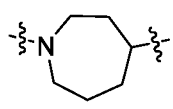 ,

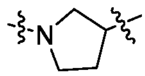 , 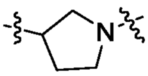 , 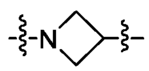 , or 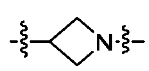 ;

3) $B_3$ is selected from phenyl substituted with 1 $R^{B3}$, when $R^{B3}$ is at the paraposition of the phenyl, $R^{B3}$ is not halogen, methyl or trifluoromethyl;
4) when $B_2$ is selected from phenyl, the phenyl is substituted with 1 to 4 $R^{B2}$;
5) $R^{B1}$ and $R^{B2}$ are directly connected to form $C_{5-7}$ carbocycle or 5- to 7-membered heterocycle, wherein the carbocycle or heterocycle is optionally further substituted with 0 to 3 $R^c$, and the heterocycle contains 1 to 3 heteroatoms selected from O, S or N;
6) when $B_5$ is selected from phenyl, the phenyl is substituted with 1 to 4 $R^{B5}$;

K is selected from

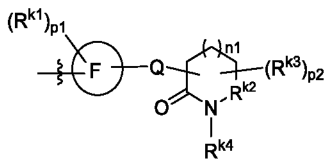 , 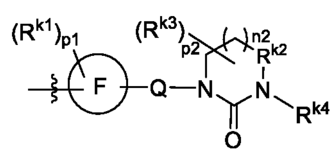 ,

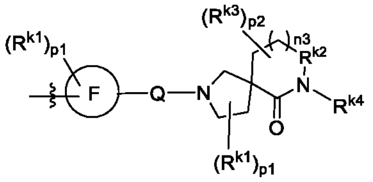 , 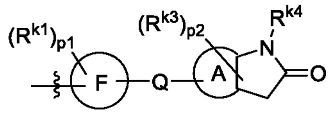 , 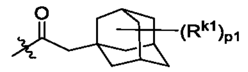 ,

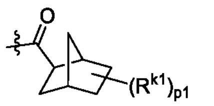 , 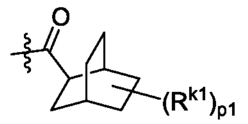 , 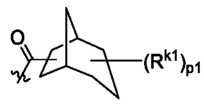 , 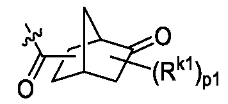 ,

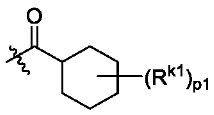 , 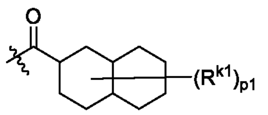 , 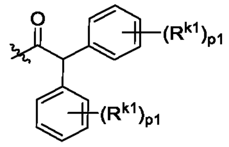 , 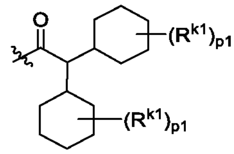 ,

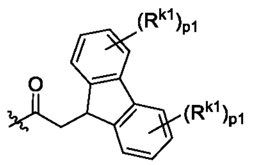 , 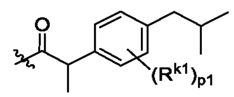 , 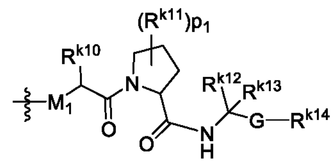 ,

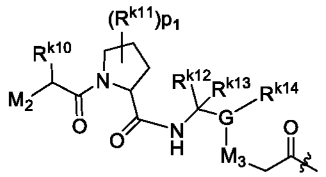 or 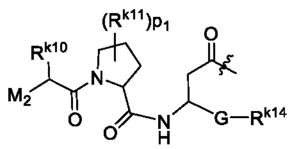 ;

each Q is independently selected from a bond, -O-, -S-, $-CH_2-$, $-NR^q-$, -CO-, - $NR^qCO-$, $-CONR^q-$ or 3- to 12-membered heterocyclyl, wherein the heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;
$R^q$ is selected from H or $C_{1-6}$ alkyl;
A is selected from $C_{3-10}$ carbocyclyl, $C_{6-10}$ aryl, 3- to 10-membered heterocyclyl or 5- to 10-membered heteroaryl, wherein the heterocycle or heteroaryl contains 1 to 4 heteroatoms selected from O, S or N;
each F is independently selected from $C_{3-20}$ carbocyclyl, $C_{6-20}$ aryl, 3- to 20-membered heterocyclyl or 5- to 20-membered heteroaryl, wherein the heterocyclyl or heteroaryl contains 1 to 4 heteroatoms selected from O, S or N;
each $R^{k2}$ is independently selected from a bond, -CO-, $-SO_2-$, -SO- or - $C(R^{k3})_2-$;
each $R^{k1}$ is independently selected from H, F, Cl, Br, I, OH, =O, $NH_2$, CN, COOH, $CONH_2$, $C_{1-6}$ alkyl or $C_{1-6}$ alkoxy, wherein the alkyl or alkoxy is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy;
each $R^{k3}$ is independently selected from H, F, Cl, Br, I, OH, =O, $NH_2$, CN, COOH, $CONH_2$, $C_{1-6}$ alkyl, $C_{1-6}$ alkoxy, $C_{3-8}$ cycloalkyl or 3- to 8-membered heterocyclyl, wherein the alkyl, alkoxy, cycloalkyl or heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;
or two $R^{k3}$ together with the carbon atoms or ring backbones to which they are directly attached form 3- to 8-membered carbocycle or 3- to 8-membered heterocycle, wherein the carbocycle or heterocycle is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;

each $R^{k4}$ is independently selected from H, OH, $NH_2$, CN, $CONH_2$, $C_{1-6}$ alkyl, $C_{3-8}$ cycloalkyl or 3- to 8-membered heterocyclyl, wherein the alkyl, cycloalkyl or heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

$M_1$ is selected from a bond, -C(=O)NH-, -NHC(=O)-, $-CH_2-C(=O)NH-$, - $C(=O)CH_2NH-$, or 5- to 6-membered heteroaryl, wherein the heteroaryl is optionally further substituted with 0 to 4 substituents selected from H, F, Cl, Br, I, OH, =O, $CF_3$, $NH_2$, CN, $C_{1-4}$ alkyl, halogen-substituted $C_{1-4}$ alkyl, hydroxyl-substituted $C_{1-4}$ alkyl or $C_{1-4}$ alkoxy, and the heteroaryl contains 1 to 4 heteroatoms selected from O, S or N;

$M_2$ is selected from $-NHC(=O)-C_{1-6}$ alkyl, $-NHC(=O)-C_{3-6}$ cycloalkyl or 4- to 10-membered heterocyclyl, wherein the alkyl, cycloalkyl or heterocyclyl is optionally further substituted with 0 to 4 substituents selected from H, F, Cl, Br, I, =O, OH, $NH_2$, $C_{1-4}$ alkyl or $C_{1-4}$ alkoxy, and the heterocyclyl contains 1 to 4 heteroatoms selected from O, S or N;

$M_3$ is selected from -NH- or -O-;

$R^{k10}$ is selected from $C_{1-6}$ alkyl, wherein the alkyl is optionally further substituted with 0 to 4 substituents selected from H, F, Cl, Br, I, =O, OH, $C_{1-6}$ alkyl or $C_{3-6}$ cycloalkyl;

each $R^{k11}$ is independently selected from H, F, Cl, Br, I, =O, OH, SH, $C_{1-6}$ alkyl, $C_{1-6}$ alkoxy, $C_{1-6}$ alkylthio or $-O-C(=O)-C_{1-6}$ alkyl, wherein the alkyl, alkoxy or alkylthio is optionally further substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, $C_{1-6}$ alkyl or $C_{3-6}$ cycloalkyl, wherein the alkyl or cycloalkyl is optionally further substituted with 0 to 4 substituents selected from H, F, Cl, Br, I, =O, OH, $NH_2$, $C_{1-4}$ alkyl or $C_{1-4}$ alkoxy;

$R^{k14}$ is selected from 5- to 6-membered heteroaryl, wherein the heteroaryl is optionally further substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 heteroatoms selected from N, O or S;

G is selected from 6- to 10-membered aryl or 5- to 10-membered heteroaryl, wherein the aryl or heteroaryl is optionally further substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 heteroatoms selected from N, O or S;

optionally, 1 to 20 H of the compound of general formula (I) are replaced by 1 to 20 deuterium;

n1, n2 and n3 are each independently selected from 0, 1, 2 or 3;

each p1 or p2 is independently selected from 0, 1, 2, 3, 4 or 5.

**2.** The compound or the stereoisomer, deuterated compound, solvate, prodrug, metabolite, pharmaceutically acceptable salt, or co-crystal thereof according to claim 1, **characterised in that**,

L is selected from -Cy1-Ak1-Cy2-Ak2-Cy3-Ak3-Cy4-Ak4-Cy5-Ak5-, -Cy1-Cy2-Cy3 -Cy4-Ak 1 -Ak2-Ak3 - Ak4-Ak5 -, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3 -Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, - Cy1-Ak1-Ak2-Cy2-Cy3-Cy4-Ak3-Ak4-Ak5-, -Cy1-Cy2-Ak1-Ak2-Cy3-Cy4-Ak3- Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3 -Ak1-Ak2-Ak3 -Cy4-Ak4-Ak5 -, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, -Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, - Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, - Ak1-Ak2-Cy1-Cy2-Cy3-Ak3-Ak4-Ak5-Cy4-, -Ak1-Ak2-Ak3-Cy1- Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, -Ak1-Ak2-Ak3-Ak4-Cy1-Cy2-Cy3-Ak5-Cy4-, -Ak1-, -Ak1-Ak2-, -Ak1-Ak2-Ak3-, -Ak1-Ak2-Ak3-Ak4-, - Ak1-Ak2-Ak3-Ak4-Ak5-, -Ak1-Ak2-Ak3-Ak4-Ak5-Ak6-, -Ak1-Ak2-Ak3-Ak4-Ak5-Ak6-Ak7-, -Ak1-Ak2-Ak3-Ak4-Ak5-Ak6-Ak7-Ak8-, or -Ak1-Ak2-Ak3-Ak4-Ak5-Ak6-Ak7-Ak8-Ak9;

Ak1, Ak2, Ak3, Ak4, Ak5, Ak6, Ak7, Ak8 and Ak9 are each independently selected from $-(CH_2)_q-$, $-(CH_2)_q-O-$, $-O-(CH_2)_q-$, $-(CH_2)_q-NR^L-$, $-NR^L-(CH_2)_q-$, - $(CH_2)q-NR^LC(=O)-$, $-(CH_2)_q-C(=O)NR^L-$, -C(=O)-, $-C(=O)-(CH_2)_q-NR^L-$, - $(C\equiv C)_q-$ or a bond, wherein the $-CH_2-$ is optionally further substituted with 0 to 2 substituents selected from H, halogen, OH, CN, $NH_2$, $C_{1-4}$ alkyl, $C_{1-4}$ alkoxy, halogen-substituted $C_{1-4}$ alkyl, hydroxyl-substituted $C_{1-4}$ alkyl or cyano-substituted $C_{1-4}$ alkyl;

each Cy1, Cy2, Cy3, Cy4 or Cy5 is independently selected from a bond, a 4-to 7-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally further substituted with 0, 1 or 2 =O when the heteroatom is selected from S;

each q is independently selected from 0, 1, 2, 3 or 4;

each $R^L$ is independently selected from H or $C_{1-6}$ alkyl.

**3.** The compound or the stereoisomer, deuterated compound, solvate, prodrug, metabolite, pharmaceutically acceptable salt, or co-crystal thereof according to claim 2, **characterised in that**,

Ak1, Ak2, Ak3, Ak4, Ak5, Ak6, Ak7, Ak8 and Ak9 are each independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)q-NR^LC(=O)-$, $-(CH_2)_q-C(=O)NR^L-$, $-C(=O)-$, $-C(=O)-(CH_2)_q-NR^L-$, $-(C\equiv C)_q-$, or a bond, wherein the $-CH_2-$ is optionally further substituted with 0 to 2 substituents selected from H, F, Cl, Br, I, OH, CN, $NH_2$, $CF_3$, hydroxymethyl, $C_{1-4}$ alkyl, or $C_{1-4}$ alkoxy;

each Cy1, Cy2, Cy3, Cy4 or Cy5 is independently selected from a bond, a 4-to 7-membered nitrogen-containing mono-heterocyclic ring, a 4- to 10-membered nitrogen-containing fused-heterocyclic ring, a 5- to 12-membered nitrogen-containing spiro-heterocyclic ring, a 7- to 10-membered nitrogen-containing bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally further substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused-heterocyclic ring, bridged-heterocyclic ring, spiro-heterocyclic ring or heteroaryl contains 1 to 4 heteroatoms selected from O, S or N, and is optionally further substituted with 0, 1 or 2 =O when the heteroatom is selected from S;

each $R^L$ is independently selected from H or $C_{1-4}$ alkyl;

K is selected from

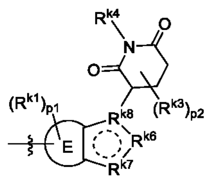
,
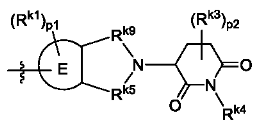
,
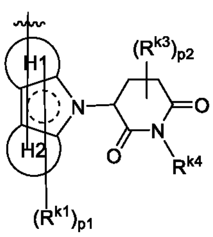
,

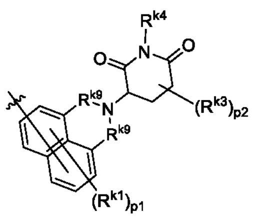
,
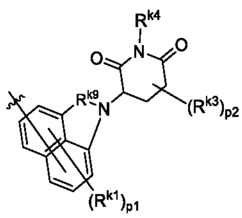
,
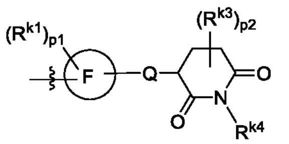
,

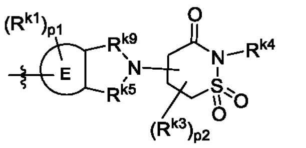,
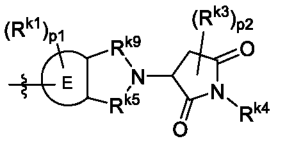,
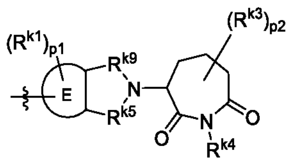,

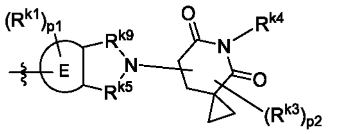,
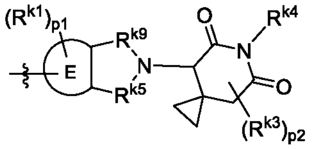,
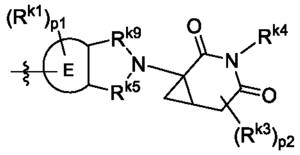,

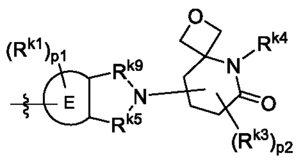,
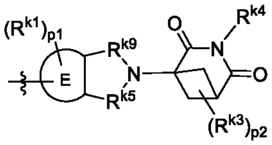,
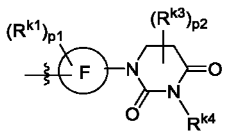,
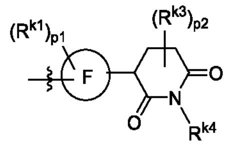

,
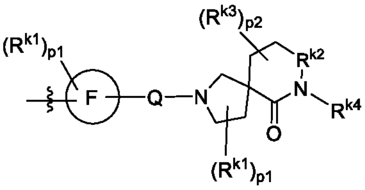,
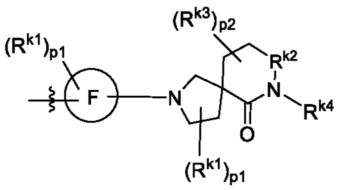,
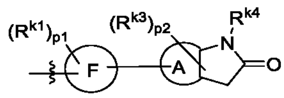,

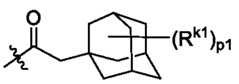,
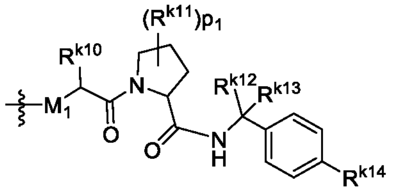,
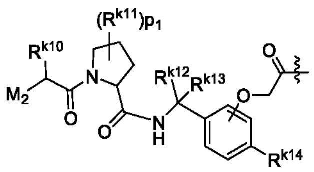

or

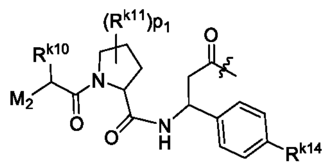;

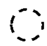

represents a ring selected from an aromatic ring or a non-aromatic ring;

$M_2$ is selected from -NHC(=O)-$C_{1-4}$ alkyl, -NHC(=O)-$C_{3-6}$ cycloalkyl or 4- to 10-membered heterocyclyl, wherein the alkyl, cycloalkyl or heterocyclyl is optionally further substituted with 0 to 4 substituents selected from H, F, Cl, Br, I, =O, OH, $NH_2$, $C_{1-4}$ alkyl or $C_{1-4}$ alkoxy, and the heterocyclyl contains 1 to 4 heteroatoms selected from O, S or N;

$R^{k10}$ is selected from $C_{1-4}$ alkyl, wherein the alkyl is optionally further substituted with 0 to 4 substituents selected from H, F, Cl, Br, I, =O, OH, $C_{1-4}$ alkyl or $C_{3-6}$ cycloalkyl;

each $R^{k11}$ is independently selected from H, F, Cl, Br, I, =O, OH, SH, $C_{1-4}$ alkyl, $C_{1-4}$ alkoxy or $C_{1-4}$ alkylthio or -O-C(=O)-$C_{1-4}$ alkyl, wherein the alkyl, alkoxy or alkylthio is optionally further substituted with 0 to 4 substituents

selected from H, F, Cl, Br, I, OH, $C_{1-4}$ alkyl or $C_{1-4}$ alkoxy;

$R^{k12}$ and $R^{k13}$ are each independently selected from H, $C_{1-4}$ alkyl or $C_{3-6}$ cycloalkyl, wherein the alkyl or cycloalkyl is optionally further substituted with 0 to 4 substituents selected from H, F, Cl, Br, I, =O, OH, $NH_2$, $C_{1-4}$ alkyl or $C_{1-4}$ alkoxy;

each Q is independently selected from -O-, -S-, $-CH_2-$, $-NR^q-$, -CO-, $-NR^qCO-$, $-CONR^q-$ or 4- to 7-membered heterocyclyl, wherein the heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

$R^q$ is selected from H or $C_{1-4}$ alkyl;

$R^{k1}$ and $R^{k3}$ are each independently selected from H, F, Cl, Br, I, OH, =O, $NH_2$, $CF_3$, CN, COOH, $CONH_2$, $C_{1-4}$ alkyl or $C_{1-4}$ alkoxy, wherein the alkyl or alkoxy is optionally further substituted with 0 to 4 substituents selected from H, F, Cl, Br, I, OH, or $NH_2$;

or two $R^{k3}$ together with the carbon atoms or ring backbones to which they are directly attached form 3- to 6-membered carbocycle or 3- to 7-membered heterocycle, wherein the carbocycle or heterocycle is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;

each $R^{k4}$ is independently selected from H, OH, $NH_2$, $CF_3$, CN or $C_{1-4}$ alkyl;

each $R^{k5}$ is independently selected from CO, $CH_2$, $SO_2$ or

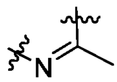
;

each $R^{k6}$ is independently selected from CO, CH, SO, $SO_2$, $CH_2$ or N;

each $R^{k7}$ is independently selected from CO, CH, N, $CH_2$, O, S, $N(CH_3)$ or NH;

each $R^{k8}$ is independently selected from C, N or CH;

each $R^{k9}$ is independently selected from CO, $CH_2$ or $SO_2$;

each A, H1 or H2 is independently selected from $C_{3-8}$ carbocycle, a benzene ring, 4- to 7-membered heterocycle or 5- to 6-membered heteroaryl, wherein the heterocycle or heteroaryl contains 1 to 4 heteroatoms selected from O, S or N;

each E is independently selected from $C_{3-8}$ carbocycle, a benzene ring, 4- to 7-membered heterocycle, 8- to 12-membered heterocycle, 7- to 12-membered heteroaryl or 5- to 6-membered heteroaryl, wherein the heterocycle or heteroaryl contains 1 to 4 heteroatoms selected from O, S or N;

each F is independently selected from 3- to 7-membered monocycloalkyl, 4-to 10-membered fused cycloalkyl, 5- to 12-membered spiro cycloalkyl, 5- to 10-membered bridged cycloalkyl, a 4- to 7-membered mono-heterocyclic ring, a 4- to 10-membered fused-heterocyclic ring, a 5- to 12-membered spiro-heterocyclic ring, a 5- to 10-membered bridged-heterocyclic ring, $C_{6-14}$ aryl or 5- to 10-membered heteroaryl, wherein the mono-heterocyclic ring, fused-heterocyclic ring, spiro-heterocyclic ring, bridged-heterocyclic ring or heteroaryl contains 1 to 4 heteroatoms selected from O, S or N.

**4.** The compound or the stereoisomer, deuterated compound, solvate, prodrug, metabolite, pharmaceutically acceptable salt, or co-crystal thereof according to claim 3, **characterised in that**,

Ak1, Ak2, Ak3, Ak4, Ak5, Ak6, Ak7, Ak8 and Ak9 are each independently selected from $-(CH_2)_q-$, $-(CH_2)_q-O-$, $-O-(CH_2)_q-$, $-(CH_2)_q-NR^L-$, $-NR^L-(CH_2)_q-$, - $(CH_2)q-NR^LC(=O)-$, $-(CH_2)_q-C(=O)NR^L-$, -C(=O)-, $-C(=O)-(CH_2)_q-NR^L-$, - $(C\equiv C)_q-$, or a bond, wherein the $-CH_2-$ is optionally further substituted with 0 to 2 substituents selected from H, F, Cl, Br, I, OH, CN, $NH_2$, $CF_3$, hydroxymethyl, methyl, ethyl, methoxy or ethoxy;

$R^L$ is selected from H, methyl or ethyl;

each q is independently selected from 0, 1, 2 or 3;

each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, azacyclopentyl, azacyclohexenyl, piperidyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclopropyl-fused-piperidyl, cyclobutyl-fused-azetidinyl, cyclobutyl-fused-azacyclopentyl, cyclobutyl-fused-azacyclohexyl, cyclobutyl-fused-piperidyl, cyclopentyl-fused-azetidinyl, cyclopentyl-fused-azacyclopentyl, cyclopentyl-fused-azacyclohexyl, cyclopentyl-fused-piperidyl, cyclohexyl-fused-azetidinyl, cyclohexyl-fused-azacyclopentyl, cyclohexyl-fused-azacyclohexyl, cyclohexyl-fused-piperidyl, azetidinyl-fused-azetidinyl, azetidinyl-fused-azacyclopentyl, azetidinyl-fused-azacyclohexyl, azetidinyl-fused-piperidyl, azacyclopentyl-fused-azetidinyl, azacyclopentyl-fused-azacyclopentyl, azacyclopentyl-fused-azacyclohexyl, azacyclopentyl-fused-piperidyl, azacyclohexyl-fused-azetidinyl, azacyclohexyl-fused-azacyclopentyl, azacyclohexyl-fused-azacyclohexyl, azacyclohexyl-fused-piperidyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidyl, cyclopentyl-spiro-piperidyl, cyclohexyl-spiro-piperidyl, azetidinyl-spiro-piperidyl, azacyclopentyl-spiro-piperidyl, azacyclohexyl-spiro-piperidyl,

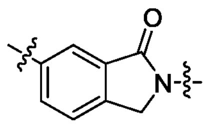 , 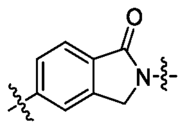 ,

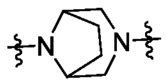 , 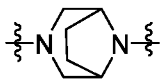 , 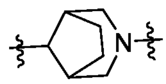 , 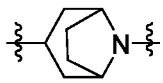 , 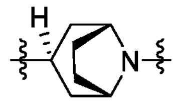 ,

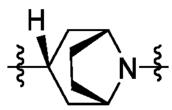 , 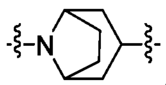 , 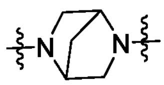 , 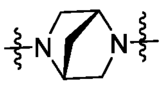 , 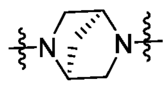 , 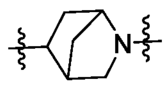

, 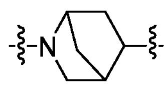 , 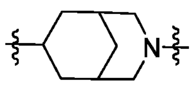 , 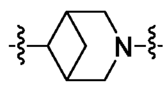 , 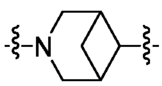 , 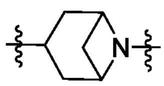 ,

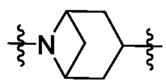 , 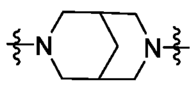 , 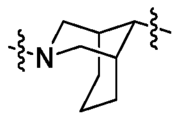 , 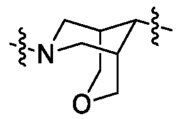 , 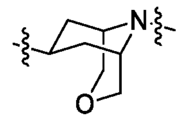 ,

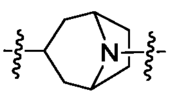 , 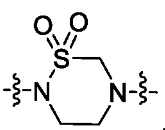 , or 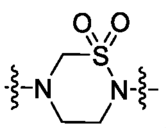 ,

which, when substituted, is optionally further substituted with 0 to 4 substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl, or $C_{1-4}$ alkoxy;

D is selected from ethylene;

$B_1$ and Z are each independently selected from azetidinyl, azacyclopentyl, piperazinyl, piperidyl, azacyclohexenyl, azepanyl, 1,4-diazepanyl, cyclobutyl-fused-azetidinyl, cyclobutyl-fused-azacyclopentyl, cyclobutyl-fused-azacyclohexyl, cyclobutyl-fused-piperidyl, cyclopentyl-fused-azetidinyl, cyclopentyl-fused-azacyclopentyl, cyclopentyl-fused-azacyclohexyl, cyclopentyl-fused-piperidyl, cyclohexyl-fused-azetidinyl, cyclohexyl-fused-azacyclopentyl, cyclohexyl-fused-azacyclohexyl, cyclohexyl-fused-piperidyl, azetidinyl-fused-azetidinyl, azacyclopentyl-fused-azetidinyl, azacyclopentyl-fused-azacyclopentyl, azacyclopentyl-fused-azacyclohexyl, azacyclopentyl-fused-piperidyl, azacyclohexyl-fused-azetidinyl, azacyclohexyl-fused-azacyclopentyl, azacyclohexyl-fused-azacyclohexyl, azacyclohexyl-fused-piperidyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidyl, cyclopentyl-spiro-piperidyl, cyclohexyl-spiro-piperidyl, azetidinyl-spiro-piperidyl, azacyclopentyl-spiro-piperidyl, azacyclohexyl-spiro-piperidyl,

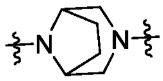, 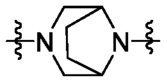, 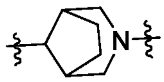,

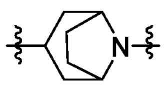, 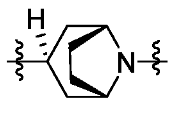, 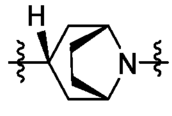, 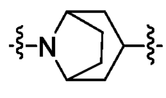, 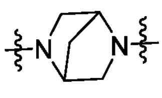,

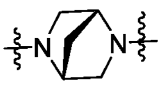, 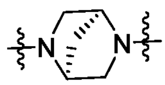, 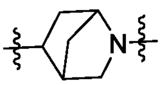, 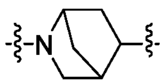, 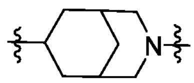,

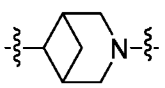, 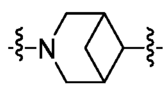, 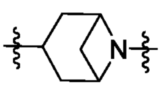, 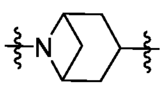, 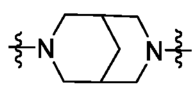,

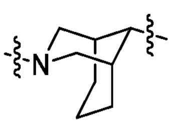, 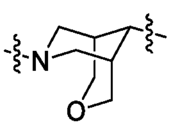, 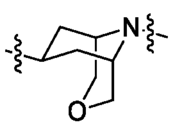, 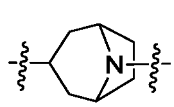, 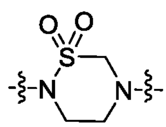,

or

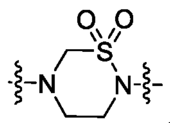,

the $B_1$ is optionally further substituted with 0 to 4 $R^{B1}$, and the Z is optionally further substituted with 0 to 4 $R^Q$; $B_2$ and $B_4$ are each independently selected from phenyl or 5- to 6-membered heteroaryl, $B_3$ and $B_5$ are each independently selected from phenyl, naphthyl, 5- to 6-membered heteroaryl or benzo 5- to 6-membered heteroaryl, the $B_2$ is optionally further substituted with 0 to 4 $R^{B2}$, the $B_3$ is optionally further substituted with 0 to 5 $R^{B3}$, the $B_4$ is optionally further substituted with 0 to 4 $R^{B4}$, and the $B_5$ is optionally further substituted with 0 to 5 $R^{B5}$, wherein the heteroaryl contains 1 to 3 heteroatoms selected from O, S or N;

$R^{B1}$, $R^Q$, $R^{B2}$, $R^{B3}$ and $R^{B5}$ are each independently selected from F, Cl, Br, I, oxo, OH, CN, methyl, ethyl, methoxy or ethoxy, wherein the methyl, ethyl, methoxy or ethoxy is optionally further substituted with 0 to 4 substituents selected from H, halogen, OH, CN or $C_{1-4}$ alkyl;

each $R^{B4}$ is independently selected from -$SO_2$-methyl, -$SO_2$-ethyl, nitro, F, Cl, Br, I, OH, CN, methyl, ethyl, methoxy or ethoxy, wherein the methyl, ethyl, methoxy or ethoxy is optionally further substituted with 0 to 4 substituents selected from H, halogen, OH, CN or $C_{1-4}$ alkyl;

$R^{w1}$, $R^{w2}$ and $R^{w5}$ are each independently selected from H, F, Cl, Br, I, OH, CN, methyl, ethyl, methoxy or ethoxy, wherein the methyl, ethyl, methoxy or ethoxy is optionally further substituted with 0 to 4 substituents

selected from H, halogen, OH, CN or $C_{1-4}$ alkyl;

alternatively, $R^{w1}$ and $R^{w2}$ are directly connected to form $C_{3-6}$ carbocycle or 3-to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;

$R^{w3}$ and $R^{w4}$ are directly connected to form $C_{3-6}$ carbocycle or 3- to 6-membered heterocycle, wherein the carbocycle or heterocycle is optionally further substituted with 0 to 4 substituents selected from H, halogen, OH, CN or $C_{1-4}$ alkyl, and the heterocycle contains 1 to 3 heteroatoms selected from O, S or N;

alternatively, $R^{B1}$ and $R^{B2}$ are directly connected to form $C_{5-7}$ carbocycle or 5-to 7-membered heterocycle, wherein the carbocycle or heterocycle is optionally further substituted with 0 to 3 $R^c$, and the heterocycle contains 1 to 3 heteroatoms selected from O, S or N;

each $R^c$ is independently selected from F, Cl, Br, I, OH, CN, =O, methyl, ethyl, methoxy, ethoxy, cyclopropyl, cyclobutyl, cyclopentyl, cyclohexyl, oxetanyl, oxacyclopentyl, oxacyclohexyl, azetidinyl, azacyclopentyl, azacyclohexyl, morpholinyl, or piperazinyl, wherein the methyl, ethyl, methoxy, ethoxy, cyclopropyl, cyclobutyl, cyclopentyl, cyclohexyl, oxetanyl, oxacyclopentyl, oxacyclohexyl, azetidinyl, azacyclopentyl, azacyclohexyl, morpholinyl, or piperazinyl is optionally further substituted with 0 to 4 substituents selected from H, halogen, OH, CN, $C_{1-4}$ alkyl, $C_{1-4}$ alkoxy, $C_{3-6}$ cycloalkyl, or 3- to 7-membered heterocycloalkyl, and the heterocycloalkyl contains 1 to 3 heteroatoms selected from O, S or N;

K is selected from

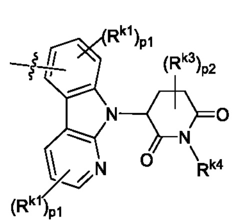 , 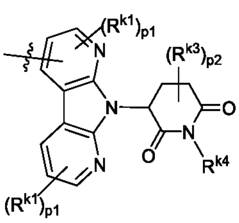 , 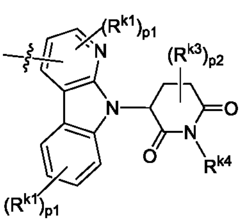 ,

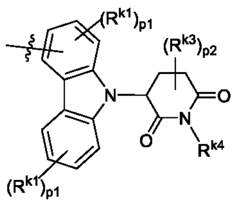 , 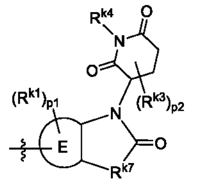 , 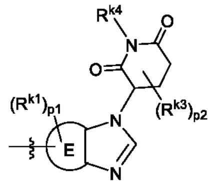 , 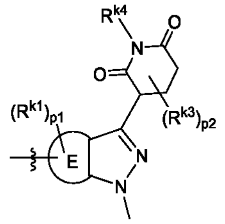 ,

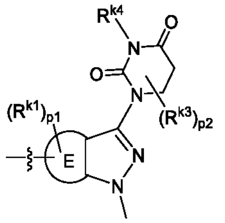 , 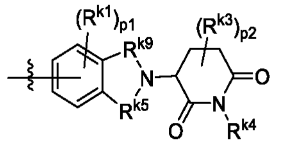 , 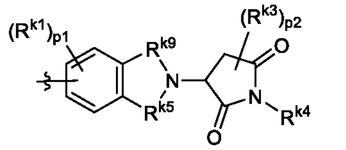 ,

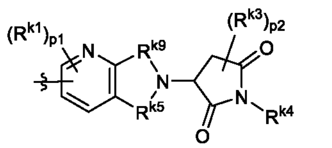 , 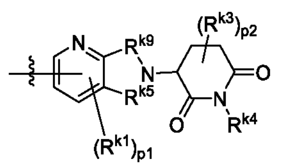 , 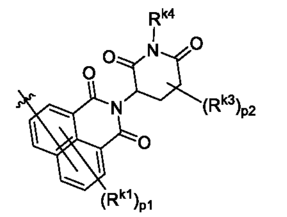 ,

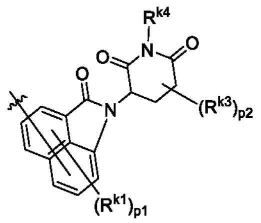 , 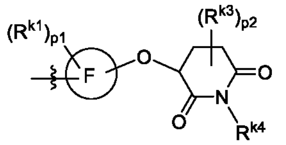 , 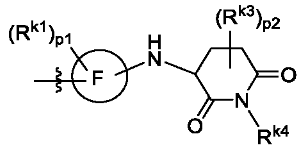 ,

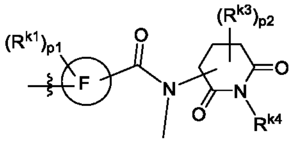 , 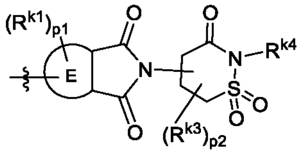 , 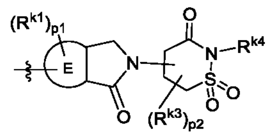 ,

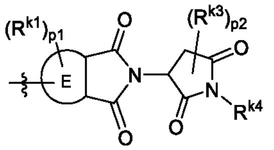 , 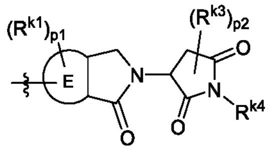 , 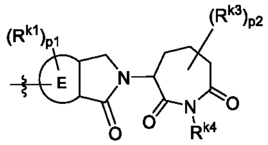 ,

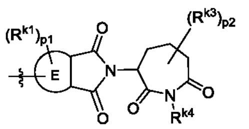 , 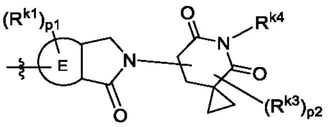 , 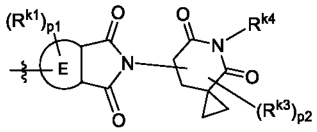 ,

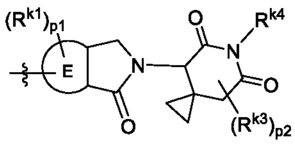 , 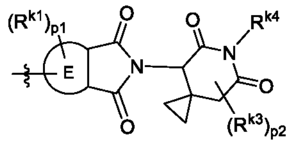 , 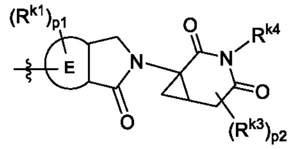 ,

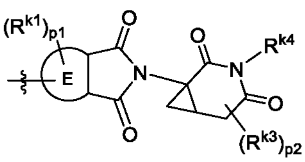 , 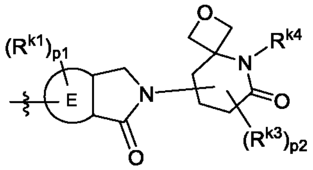 , 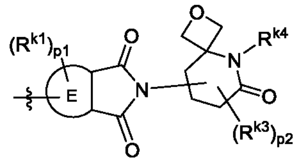 ,

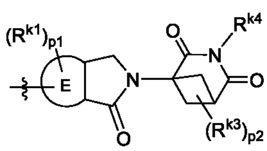 , 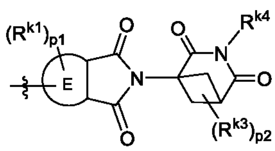 , 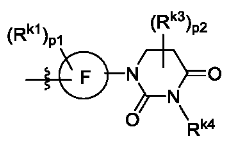 , 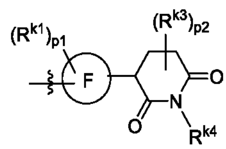 ,

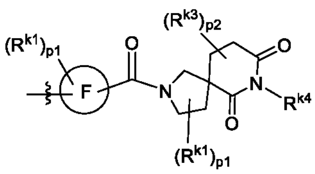 , 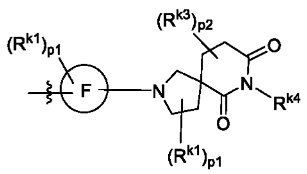 , 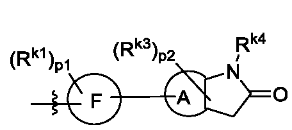 ,

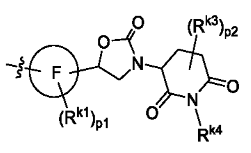,
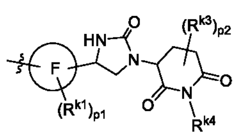,
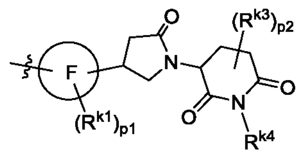,

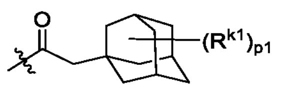,
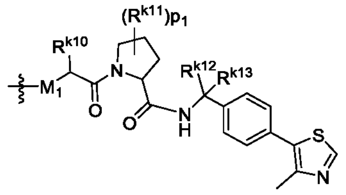,
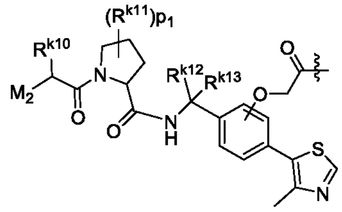,

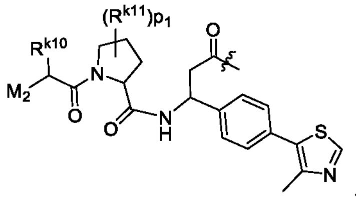,
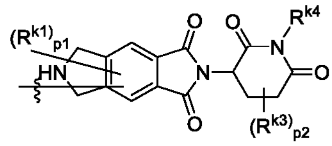,
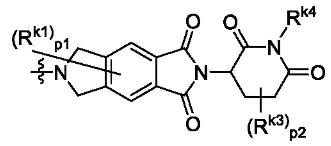,

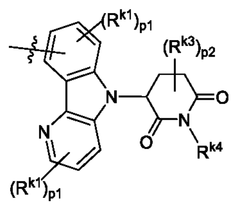 or 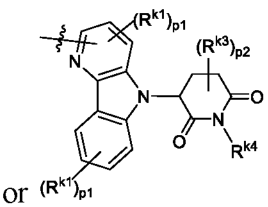;

$M_1$ is selected from a bond, -C(=O)NH-, $-CH_2-C(=O)NH-$, $-C(=O)CH_2NH-$, pyrrolyl, pyrazolyl, imidazolyl, triazolyl, oxazolyl, isoxazolyl, furyl, thienyl or thiazolyl;

$M_2$ is selected from $-NHC(=O)-CH_3$, -NHC(=O)-cyclopropyl, -NHC(=O)-cyclobutyl, azetidinyl, azacyclopentyl, benzo-azacyclopentyl or benzo-azacyclohexyl, wherein the cyclopropyl, cyclobutyl, azetidinyl, azacyclopentyl, benzo-azacyclopentyl or benzo-azacyclohexyl is optionally further substituted with 0 to 4 substituents selected from H, F, Cl, Br, I, =O, OH, $NH_2$, $C_{1-4}$ alkyl or $C_{1-4}$ alkoxy;

$R^{k10}$ is selected from methyl, ethyl, isopropyl, propyl or tert-butyl, wherein the methyl, ethyl, isopropyl, propyl or tert-butyl is optionally further substituted with 0 to 4 substituents selected from H, F, Cl, Br, I, =O, OH, $C_{1-4}$ alkyl or $C_{3-6}$ cycloalkyl;

each $R^{k11}$ is independently selected from H, F, Cl, Br, I, =O, OH, SH, methyl, ethyl, isopropyl, propyl, methoxy, ethoxy, propoxy, isopropyloxy, methylthio, ethylthio, propylthio or $-O-C(=O)-CH_3$, wherein the methyl, ethyl, isopropyl, propyl, methoxy, ethoxy, propoxy, isopropyloxy, methylthio, ethylthio, or propylthio is optionally further substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, methyl, ethyl, isopropyl, propyl, cyclopropyl or cyclobutyl, wherein the methyl, ethyl, isopropyl, propyl, cyclopropyl or cyclobutyl is optionally further substituted with 0 to 4 substituents selected from H, F, Cl, Br, I, =O, OH, $NH_2$, $C_{1-4}$ alkyl or $C_{1-4}$ alkoxy;

each E is independently selected from phenyl, pyridyl, pyridazinyl, pyrazinyl, pyrimidyl, pyrrolyl, pyrazolyl, imidazolyl, thiazolyl, furyl, thienyl or oxazolyl;

each A is independently selected from phenyl, pyridyl, pyridazinyl, pyrazinyl, pyrimidyl, pyrrolyl, pyrazolyl, imidazolyl, thiazolyl, furyl, thienyl or oxazolyl;

each F is independently selected from cyclopropyl, cyclobutyl, cyclopentyl, cyclohexyl, bicyclo[1.1.1]pentanyl, 6,7-dihydro-5H-cyclopenta[c]pyridyl, 2,3-dihydro-1H-indenyl, phenyl, naphthyl, anthryl, phenanthryl, azetidinyl, azacyclopentyl, piperidyl, morpholinyl, pyridyl, pyrimidyl, pyridazinyl, pyrazinyl, triazinyl, pyrrolyl, pyrazolyl, imidazolyl, triazolyl, oxazolyl, furyl, thienyl, thiazolyl, benzoimidazolyl, benzopyrazolyl, benzothiazolyl, benzothienyl, benzofuryl, benzopyrrolyl, benzopyridyl, benzopyrazinyl, benzopyrimidyl, benzopyridazinyl, pyrrolopyrrolyl, pyrrolopyridyl, pyrrolopyrimidyl, pyrrolopyridazinyl, pyrrolopyrazinyl, imidazopyrimidyl, imidazopyridyl, imidazopyrazinyl, imidazopyridazinyl, pyrazolopyridyl, pyrazolopyrimidyl, pyrazolopyridazinyl, pyrazolopyrazinyl, pyrimidopyridyl, pyrimidopyrazinyl, pyrimidopyridazinyl, pyrimidopyrimidyl, pyridopyridyl, pyridopyrazinyl, pyridopyridazinyl, pyridazinopyridazinyl, pyridazinopyrazinyl or pyrazinopyrazinyl;

each $R^{k7}$ is independently selected from $CH_2$, O, $N(CH_3)$ or NH;

each p1 or p2 is independently selected from 0, 1 or 2.

**5.** The compound or the stereoisomer, deuterated compound, solvate, prodrug, metabolite, pharmaceutically acceptable salt, or co-crystal thereof according to claim 4, **characterised in that**,

Ak1, Ak2, Ak3, Ak4, Ak5, Ak6, Ak7, Ak8 and Ak9 are each independently selected from a bond, -O-, $-OCH_2-$, $-CH_2O-$, $-OCH_2CH_2-$, $-CH_2CH_2O-$, -C=C-, - $C(CH_3)_2-$, $-CH_2-$, $-CH_2CH_2-$, $-CH_2CH_2CH_2-$, $-N(CH_3)-$, -NH-, $-CH_2N(CH_3)-$, - $CH_2NH-$, $-NHCH_2-$, $-CH_2CH_2N(CH_3)-$, $-CH_2CH_2NH-$, $-NHCH_2CH_2-$, -C(=O)-, - $C(=O)CH_2NH-$, $-CH_2C(=O)NH-$, -C(=O)NH- or -NHC(=O)-;

each Cy1, Cy2, Cy3, Cy4 or Cy5 is independently selected from a bond or one of the following substituted or unsubstituted groups:

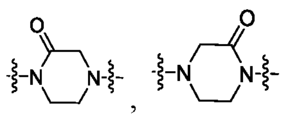, ,

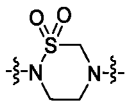, 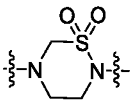, 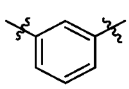, 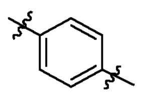, 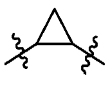, 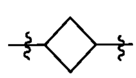, 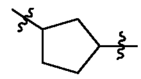,

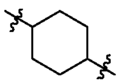, 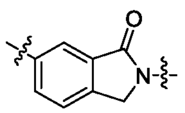, 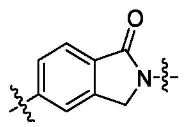, 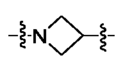, 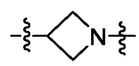, 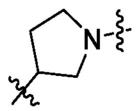,

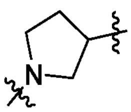, 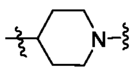, 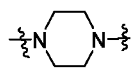, 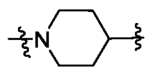, 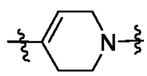, 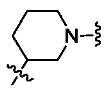, 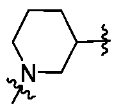,

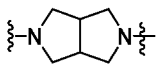, 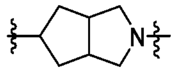, 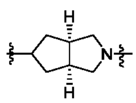, 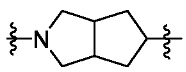, 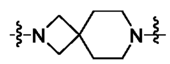,

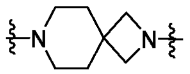, 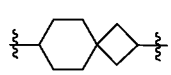, 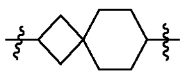, 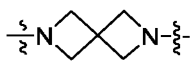, 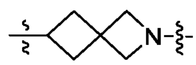,

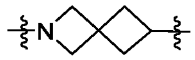, 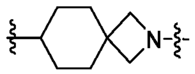, 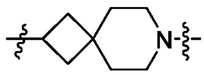, 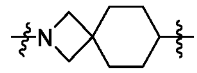, 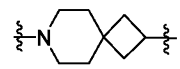,

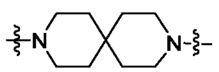, 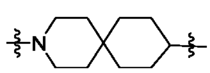, 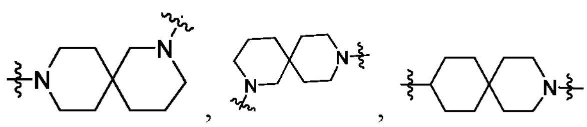, , ,

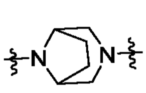, 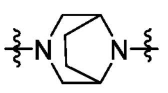, 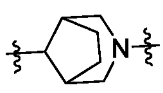, 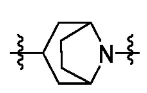, 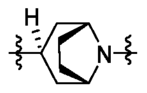, 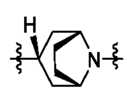,

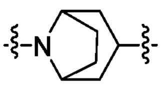, 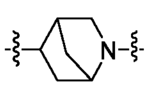, 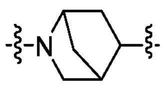, 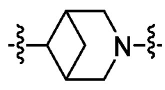, 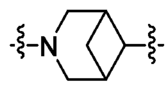, 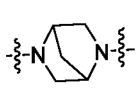,

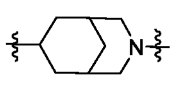, 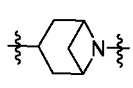, 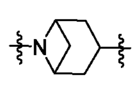, 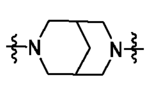, 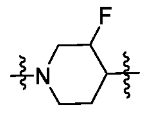, 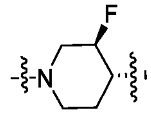,

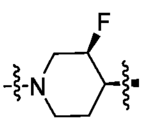, 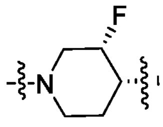, 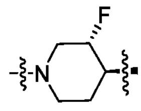, 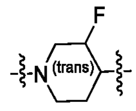, 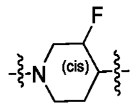, 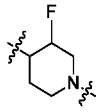, 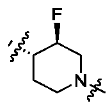,

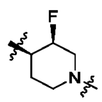, 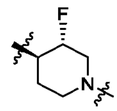, 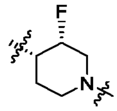, 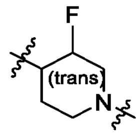 or 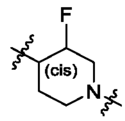,

which, when substituted, is optionally further substituted with 0 to 4 substituents selected from H, F, $CF_3$, methyl, =O, hydroxymethyl, COOH, CN or $NH_2$;
B is selected from one of the structural fragments shown in **Table B-a**;
K is selected from one of the structural fragments shown in **Table K-a**.

6. The compound or the stereoisomer, deuterated compound, solvate, prodrug, metabolite, pharmaceutically acceptable salt, or co-crystal thereof according to claim 5, **characterised in that**,
L is selected from a bond, -Ak1-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Cy1-, - Cy1-Ak1-, -Cy1-Ak1-Ak2-, -Cy1-Ak1-Ak2-Ak3-, -Cy1-Ak1-Ak2-Ak3-Ak4-, - Cy1-Cy2-, -Cy 1- Ak 1-Cy2-, -Cy1-Cy2-Ak2-, -Cy 1- Ak 1-Cy2- Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Ak2-Cy3-, -Cy1-Ak1-Ak2-Cy3-Ak3-, -Cy1-Cy2-Cy3-, -Cy 1- Ak 1-Cy2-Cy3 -, -Cy1-Cy2-Ak2-Cy3-, -Cy1-Cy2-Cy3-Ak3-, -Cy 1- Ak 1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cy1-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cy1-Ak1-Cy2-Ak2-Cy3-Cy4-, -Ak1-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Cy1-Cy2-Cy3-Ak3-Ak4-Ak5-, -Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-Ak2-Ak3-Ak4-, -Ak1-Cy2-Ak2-Ak3-, -Ak1-Ak2-Ak3-Ak4-Ak5 -, -Ak1-Ak2-Ak3-Ak4-Ak5-Ak6-, or -Ak1-Ak2-Ak3-Ak4-Ak5-Ak6-Ak7-.

7. The compound or the stereoisomer, deuterated compound, solvate, prodrug, metabolite, pharmaceutically acceptable salt, or co-crystal thereof according to any one of claims 1-6, **characterised in that**,
L is selected from a bond or a group shown in Table B-1, wherein the left side of the group is linked to B.

8. The compound or the stereoisomer, deuterated compound, solvate, prodrug, metabolite, pharmaceutically acceptable salt, or co-crystal thereof according to claim 7, **characterised in that**,
K is selected from one of the following structural fragments:

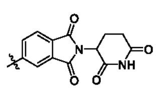 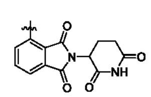 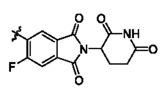 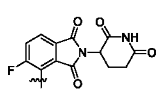 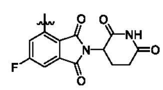 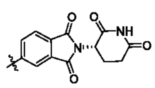

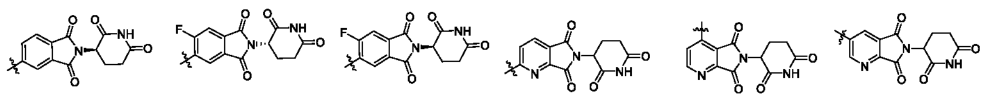

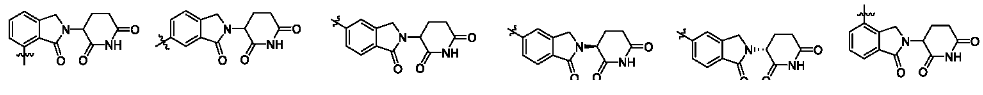

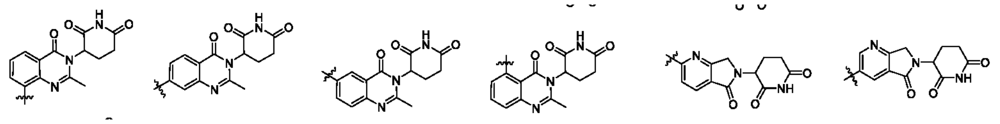

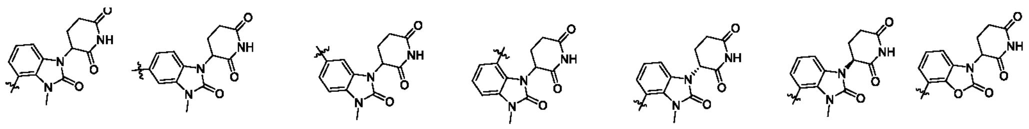

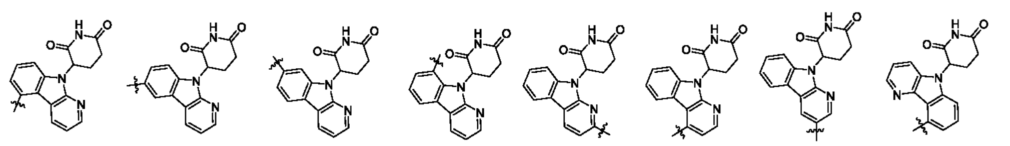

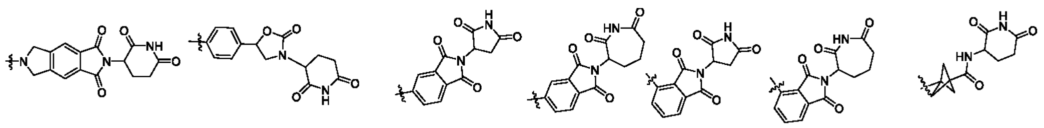

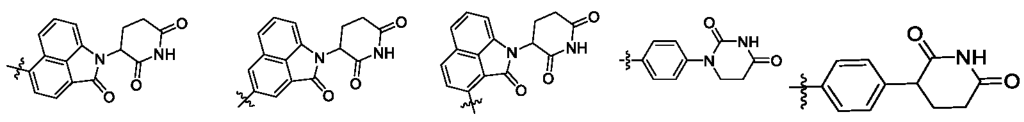

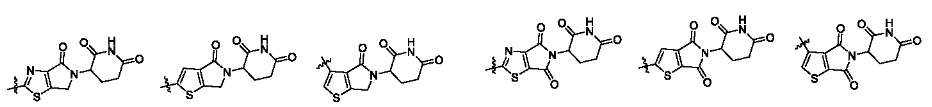

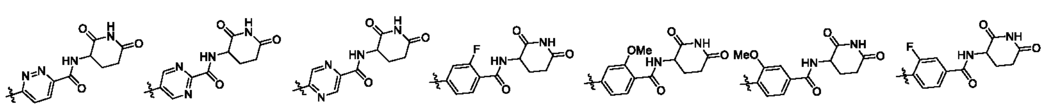

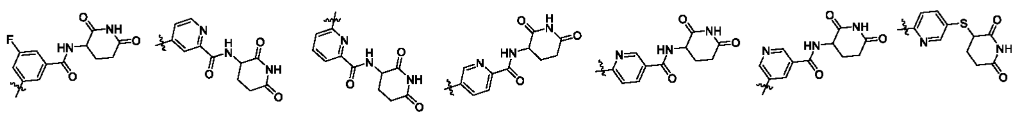

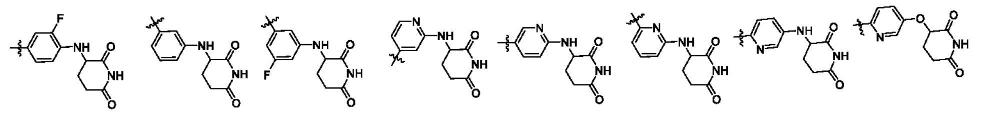

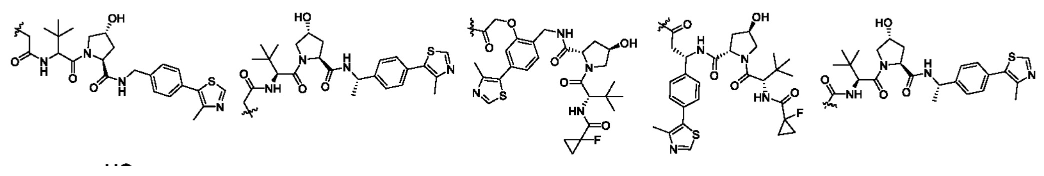

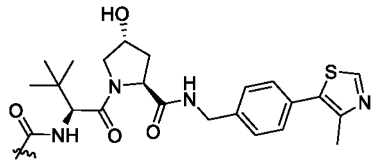 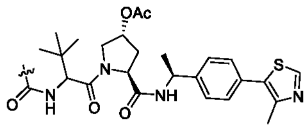 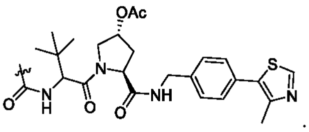.

**9.** The compound or the stereoisomer, deuterated compound, solvate, prodrug, metabolite, pharmaceutically acceptable salt, or co-crystal thereof according to claim 1, **characterised in that** the compound is selected from a compound of general formula (Ia)

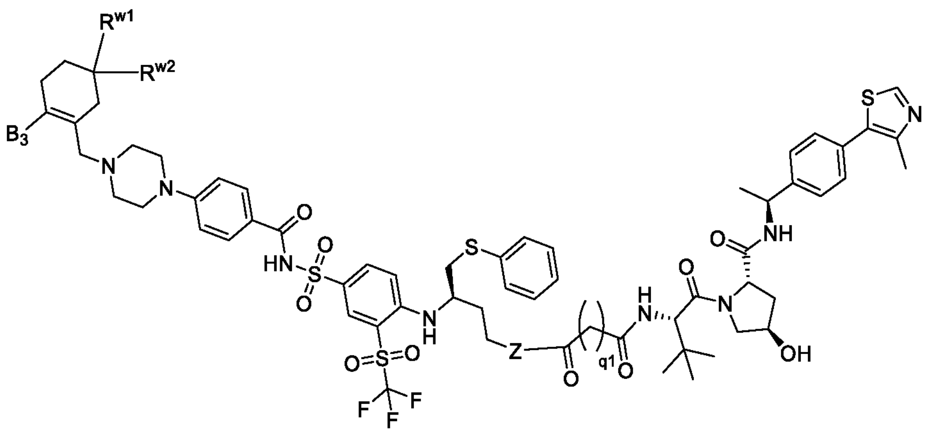

(Ia)

q1 is selected from 1, 2, 3, 4, 5, 6, 7 or 8;
$B_3$ is selected from

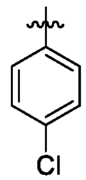 or 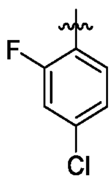;

$R^{w1}$ and $R^{w2}$ are selected from methyl;
or $R^{w1}$ and $R^{w2}$ are directly connected to form, together with the carbon atom to which they are attached, cyclopropyl;
Z is selected from

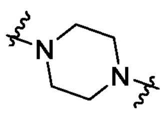, 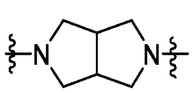, 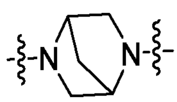,

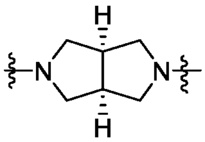, 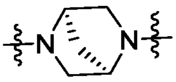, or 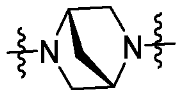;

when Z is selected from

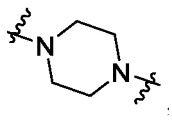

and $R^{w1}$ and $R^{w2}$ are selected from methyl, $B_3$ is selected from

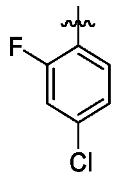.

**10.** The compound or the stereoisomer, deuterated compound, solvate, prodrug, metabolite, pharmaceutically acceptable salt, or co-crystal thereof according to claim 1, **characterised in that** the compound is selected from a compound of general formula (Ib)

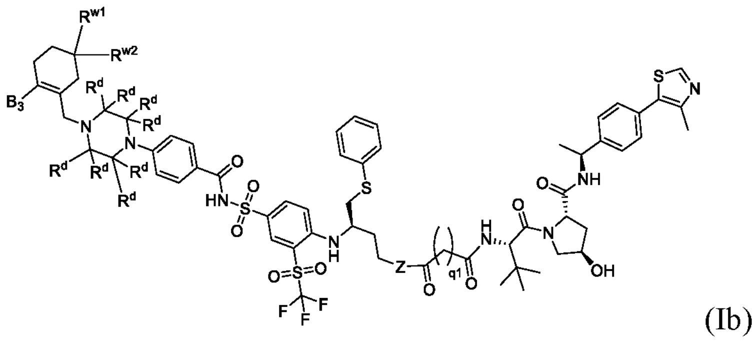

(Ib)

q1 is selected from 1, 2, 3, 4, 5, 6, 7 or 8;
$B_3$ is selected from

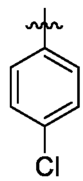 or 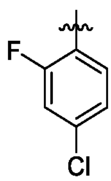;

$R^{w1}$ and $R^{w2}$ are selected from methyl;
or $R^{w1}$ and $R^{w2}$ are directly connected to form, together with the carbon atom to which they are attached, cyclopropyl;
Z is selected from

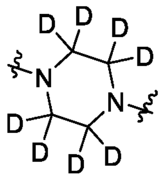, 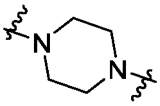, 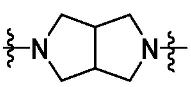, 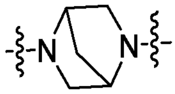,

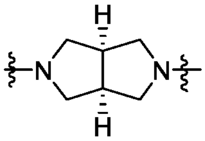, 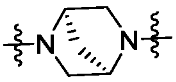, or 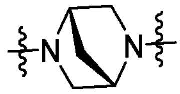;

each $R^d$ is independently selected from H or deuterium;
the compound of general formula (Ib) has at least one deuterium.

**11.** The compound or the stereoisomer, deuterated compound, solvate, prodrug, metabolite, pharmaceutically acceptable salt, or co-crystal thereof according to claim 1, **characterised in that** the compound is selected from one of the structures shown in Table P-1.

**12.** A pharmaceutical composition, **characterised in that** the pharmaceutical composition comprises the compound or the stereoisomer, deuterated compound, solvate, prodrug, metabolite, pharmaceutically acceptable salt, or co-crystal thereof according to any one of claims 1-11, and a pharmaceutically acceptable carrier, wherein preferably, the pharmaceutical composition comprises 1-1500 mg of the compound or the stereoisomer, deuterated compound, solvate, prodrug, metabolite, pharmaceutically acceptable salt, or co-crystal thereof according to any one of claims 1-11.

**13.** Use of the compound or the stereoisomer, deuterated compound, solvate, prodrug, metabolite, pharmaceutically acceptable salt, or co-crystal thereof according to any one of claims 1-11 in the preparation of a medicament for treating a disease related to Bcl-2 family protein activity or expression level.

**14.** Use of the compound or the stereoisomer, deuterated compound, solvate, prodrug, metabolite, pharmaceutically acceptable salt, or co-crystal thereof according to any one of claims 1-11 in the preparation of a medicament for treating a disease related to the inhibition or degradation of Bcl-2 family proteins.

**15.** The use according to claim 13 or 14, **characterised in that** the disease is selected from cancer.

**16.** A method for treating a disease in a mammal, **characterised in that** the method comprises administering to a subject a therapeutically effective amount of the compound or the stereoisomer, deuterated compound, solvate, prodrug, metabolite, pharmaceutically acceptable salt, or co-crystal thereof according to any one of claims 1-11, wherein the therapeutically effective amount is preferably 1-1500 mg, and the disease is preferably cancer.

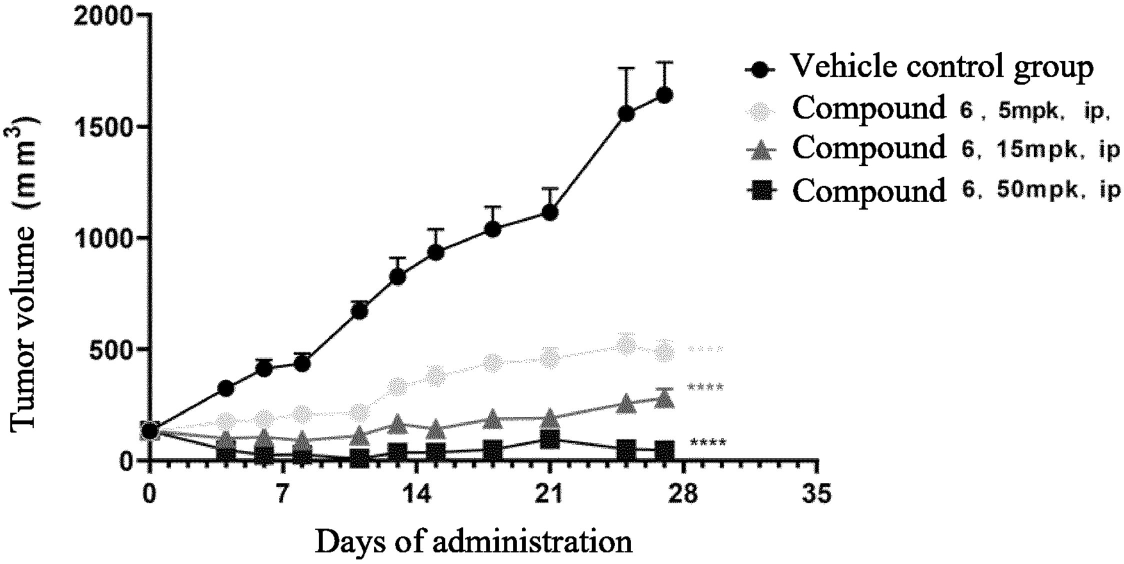
2000
1500
1000
500
0
Tumor volume (mm3)
0
7
14
21
28
35
Days of administration
Vehicle control group
Compound 6 , 5mpk, ip,
Compound 6, 15mpk, ip
Compound 6, 50mpk, ip
****
****
****

*FIG. 1*

**INTERNATIONAL SEARCH REPORT**

International application No.
**PCT/CN2022/116529**

**A. CLASSIFICATION OF SUBJECT MATTER**

C07D 487/04(2006.01)i； C07K 5/083(2006.01)i； A61K 31/5377(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D; C07K; A61K; A61P

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: E3泛素连接酶, 癌症, 蛋白水解靶向嵌合体, 海思科, E3 ligase , PROTAC, proteolysis targeting chimera, Bcl, cancer, HAISCO

**C. DOCUMENTS CONSIDERED TO BE RELEVANT**

| Category* | Citation of document, with indication, where appropriate, of the relevant passages | Relevant to claim No. |
|---|---|---|
| PX | NEGI, Arvind et al. "Strategies to Reduce the On-Target Platelet Toxicity of Bcl-xL Inhibitors: PROTACs, SNIPERs and Prodrug-Based Approaches" *ChemBioChem,* Vol. 23, 09 March 2022 (2022-03-09), pp. 1-19 | 1-16 |
| X | ZHANG, Xuan et al. "Discovery of PROTAC BCL-XL degraders as potent anticancer agents with low on-target platelet toxicity" *European Journal of Medicinal Chemistry,* Vol. 192, 27 February 2020 (2020-02-27), pp. 1-24 | 1-16 |
| X | ZHANG, Xuan et al. "Discovery of IAP-recruiting BCL-XL PROTACs as potent degraders across multiple cancer cell lines" *European Journal of Medicinal Chemistry,* Vol. 199, 04 May 2020 (2020-05-04), pp. 1-12 | 1-16 |
| X | HE, Yonghan et al. "Using proteolysis-targeting chimera technology to reduce navitoclax platelet toxicity and improve its senolytic activity" *Nature communications,* Vol. 11, No. 1, 24 April 2020 (2020-04-24), pp. 1-14 | 1-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 November 2022** | **30 November 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2022/116529

C. DOCUMENTS CONSIDERED TO BE RELEVANT

| Category* | Citation of document, with indication, where appropriate, of the relevant passages | Relevant to claim No. |
|---|---|---|
| X | WO 2020163823 A2 (UNIVERSITY OF FLORIDA RESEARCH FOUNDATION, INCORPORATED) 13 August 2020 (2020-08-13) claims 1-83 | 1-16 |
| A | WO 2017185023 A1 (DANA-FARBER CANCER INSTITUTE, INC.) 26 October 2017 (2017-10-26) table A | 1-16 |

Form PCT/ISA/210 (second sheet) (January 2015)

**INTERNATIONAL SEARCH REPORT**

International application No.
**PCT/CN2022/116529**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16**
because they relate to subject matter not required to be searched by this Authority, namely:

[1] Claim 16 sets forth a method for treating diseases in a mammal, which relates to a treatment method directly implemented on a human or animal body. Therefore, claim 16 does not comply with PCT Rule 39.1(iv). The search for claim 16 was conducted on the basis of a pharmaceutical use of the compound and the related product thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/116529**

| Patent document cited in search report | | | Publication date (day/month/year) | Patent family member(s) | | | Publication date (day/month/year) |
|---|---|---|---|---|---|---|---|
| WO | 2020163823 | A2 | 13 August 2020 | CN | 113660937 | A | 16 November 2021 |
| | | | | KR | 20210137025 | A | 17 November 2021 |
| | | | | CA | 3127501 | A1 | 13 August 2020 |
| | | | | AU | 2020218367 | A1 | 12 August 2021 |
| | | | | JP | 2022520061 | A | 28 March 2022 |
| | | | | EP | 3920923 | A2 | 15 December 2021 |
| | | | | US | 2022169628 | A1 | 02 June 2022 |
| WO | 2017185023 | A1 | 26 October 2017 | CN | 109153675 | A | 04 January 2019 |
| | | | | US | 2019111143 | A1 | 18 April 2019 |
| | | | | JP | 2019514882 | A | 06 June 2019 |
| | | | | EP | 3445760 | A1 | 27 February 2019 |
| | | | | CA | 3018429 | A1 | 26 October 2017 |
| | | | | AU | 2017254702 | A1 | 11 October 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019144117 A
- WO 2017184995 A
- WO 2020163823 A
- WO 2017101851 A
- WO 2020041406 A
- WO 2020212530 A
- CN 105985321
- WO 2013185202 A
- WO 2021066873 A
- CN 114292270
- WO 2021262812 A

### Non-patent literature cited in the description

- Synthetic Organic Chemistry. John Wiley & Sons, Inc,
- **S. R. SANDLER et al.** Organic Functional Group Preparations. Academic Press, 1983
- **H. O. HOUSE.** Modern Synthetic Reactions. W. A. Benjamin, Inc, 1972
- **T. L. GILCHRIST.** Heterocyclic Chemistry. John Wiley & Sons, 1992
- **J. MARCH.** Advanced Organic Chemistry: Reactions, Mechanisms and Structure. Wiley-Interscience, 1992
- **FUHRHOP, J. ; PENZLIN G.** Organic Synthesis: Concepts, Methods, Starting Materials. John Wiley & Sons, 1994
- **HOFFMAN, R.V.** Organic Chemistry, An Intermediate Text. Oxford University Press, 1996
- **LAROCK, R. C.** Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons, 1992
- Modern Carbonyl Chemistry. Wiley-VCH, 2000
- **PATAI, S.** Patai's 1992 Guide to the Chemistry of Functional Groups. Interscience, 1992
- **SOLOMONS, T. W. G.** Organic Chemistry. John Wiley & Sons, 2000
- **STOWELL, J.C.** Intermediate Organic Chemistry. Wiley-Interscience, 1993
- Industrial Organic Chemicals: Starting Materials and Intermediates: An Ullmann's Encyclopedia. John Wiley & Sons, 1999, vol. 8
- Organic Reactions. John Wiley & Sons, 1942, vol. 55
- Chemistry of Functional Groups. John Wiley & Sons, vol. 73
- *CHEMICAL ABSTRACTS,* 25952-53-8
- *CHEMICAL ABSTRACTS,* 2592-95-2
- *CHEMICAL ABSTRACTS,* 148893-10-1
- *Nature Communications,* 2020, vol. 11, 1996
- *European Journal of Medicinal Chemistry,* 2018, vol. 149, 79-89